# EUROPEAN PATENT APPLICATION

(11) **EP 1 439 393 A2**
(43) Date of publication of application: **21.07.2004**
(21) Application number: 03257868.4
(22) Date of filing: 15.12.2003
(51) Int. Cl.: G01N 33/574, G01N 33/53

(54) **Detection methods using TIMP 1 for colon cancer diagnosis**

(30) Priority: 13.12.2002 US 433554 P; 31.07.2003 US 491397 P
(71) Applicant: Bayer Healthcare LLC, Tarrytown, New York 10591-5097 (US); MAYO FOUNDATION FOR MEDICAL EDUCATION AND RESEARCH, Rochester, MN 55905 (US)
(72) Inventor: Astle, Jon H., Taunton, MA 02780 (US); Boardman, Lisa Allyn, Rochester, MN 55902 (US); Bugart, Lawrence J., Rochester, MN 55901 (US); Burgess, Christopher C., Westwood, MA 02090 (US); Catino, Theodore J., Attleboro, MA 02702 (US); Dwivedi, Poornima, Alamo, CA 94507 (US); Huntress, Maryanne, Attleboro, MA 02703 (US); Johnson, Karen Anne, Action, MA 01720 (US); Lewis, Marcia E., Cohasset, MA 02025 (US); Maimonis, Peter J., Westwood, MA 02090 (US); Myerow, Susan H., Lexington, MA 02421 (US); Brown-Shimer, Sheryl Lynn Andrea, Boston, MA 02118 (US); Thiagalingam, Arunthathi, Lexington, MA 02420 (US); Thibodeau, Stephen N., Rochester, MN 55906 (US); Molino, Gary A., Norfolk, MA 02056 (US)
(74) Representative: Grant, Anne Rosemary

(57) **Abstract**

The present invention relates to a method for detecting the presence of colorectal cancer in an individual, wherein colorectal cancer is detected by detecting the presence of Reg1α or TIMP1 nucleic acid or amino acid molecules in a clinical sample obtained from the patient, wherein Reg1α or TIMP1 expression is indicative of the presence of colorectal cancer. The invention further relates to a method for detecting the presence of colorectal cancer in an individual, wherein colorectal cancer is detected by detecting the presence of Reg1α or TIMP1 nucleic acid or amino acid molecules in a clinical sample, in addition to detecting the presence of one or more additional colorectal cancer associated markers.

SEQUENCE LISTING

<110> Bayer Healthcare, LLC et al.

<120> REG1A

<130> 1657/2011B

<160> 138

<170> FastSEQ for Windows Version 4.0

<210> 1
<211> 777
<212> DNA
<213> Homo sapiens

<400> 1
ttcttcaaac cctcctcttc cctgtgttct cctacagaga ttgctgattt ctccttaagc 60
aagagattca ctgccgctaa gcatggctca gaccaactcg ttcttcatgc tgatctcctc
120
cctgatgttc ctgtctctga gccaaggcca agaggcccag acagagttgc cccaggcccg
180
gatcagctgc ccagaaggca ccaatgccta tcgctcctac tgctactact ttaatgaaga
240
ccgtgagacc tgggttgatg cagatctcta ttgccagaac atgaattcgg gcaacctggt
300
gtctgtgctc acccaggccg agggtgcctt tgtggcctca ctgattaagg agagtggcac
360
tgatgacttc aatgtctgga ttggcctcca tgaccccaaa aagaaccgcc gctggcactg
420
gagcagtggg tccctggtct cctacaagtc ctggggcatt ggagccccaa gcagtgttaa
480

tcctggctac tgtgtgagcc tgacctcaag cacaggattc cagaaatgga aggatgtgcc
540

ttgtgaagac aagttctcct ttgtatgcaa gttcaaaaac tagaggcagc tggaaaatac
600

atgtctagaa ctgatccagc aattacaacg gagtcaaaaa ttaaaccgga ccatctctcc
660

aactcaactc aacctggaca ctctcttctc tgctgagttt gccttgttaa tcttcaatag
720

ttttacctac cccagtcttt ggaaccctaa ataataaaaa taaacatgtt ttccact
777

<210> 2

<211> 166

<212> PRT

<213> Homo sapiens

<400> 2

Met Ala Gln Thr Asn Ser Phe Phe Met Leu Ile Ser Ser Leu Met Phe

1               5                   10                  15

Leu Ser Leu Ser Gln Gly Gln Glu Ala Gln Thr Glu Leu Pro Gln Ala

            20                  25                  30

Arg Ile Ser Cys Pro Glu Gly Thr Asn Ala Tyr Arg Ser Tyr Cys Tyr

        35                  40                  45

Tyr Phe Asn Glu Asp Arg Glu Thr Trp Val Asp Ala Asp Leu Tyr Cys

    50                  55                  60

Gln Asn Met Asn Ser Gly Asn Leu Val Ser Val Leu Thr Gln Ala Glu

65                  70                  75                  80

Gly Ala Phe Val Ala Ser Leu Ile Lys Glu Ser Gly Thr Asp Asp Phe

                85                  90                  95

Asn Val Trp Ile Gly Leu His Asp Pro Lys Lys Asn Arg Arg Trp His

            100                 105                 110

Trp Ser Ser Gly Ser Leu Val Ser Tyr Lys Ser Trp Gly Ile Gly Ala

        115                 120                 125

Pro Ser Ser Val Asn Pro Gly Tyr Cys Val Ser Leu Thr Ser Ser Thr

    130                 135                 140

Gly Phe Gln Lys Trp Lys Asp Val Pro Cys Glu Asp Lys Phe Ser Phe

145                 150                 155                 160

Val Cys Lys Phe Lys Asn

                165

<210> 3

<211> 749

<212> DNA

<213> Homo sapiens

<400> 3

agccaacaga gattgttgat ttgcctctta agcaagagat tcattgcagc tcagcatggc 60

tcagaccagc tcatacttca tgctgatctc ctgcctgatg tttctgtctc agagccaagg
120

ccaagaggcc cagacagagt tgccccaggc ccggatcagc tgcccagaag gcaccaatgc
180

ctatcgctcc tactgctact actttaatga agaccgtgag acctgggttg atgcagatct
240

ctattgccag aacatgaatt cgggcaacct ggtgtctgtg ctcacccagg ccgagggtgc
300

ctttgtggcc tcactgatta aggagagtgg cactgatgac ttcaatgtct ggattggcct
360

ccatgacccc aaaaagaacc gccgctggca ctggagcagt gggtccctgg tctcctacaa
420

gtcctggggc attggagccc caagcagtgt taatcctggc tactgtgtga gcctgacctc
480

aagcacagga ttccagaaat ggaaggatgt gccttgtgaa gacaagttct cctttgtctg
540

caagttcaaa aactagaggc agctggaaaa tacatgtcta gaactgatcc agcaattaca
600

acggagtcaa aaattaaacc ggaccatctc tccaactcaa ctcaacctgg acactctctt
660

ctctgctgag tttgccttgt taatcttcaa tagttttacc taccccagtc tttggaacct
720

taaataataa aaataaacat gtttccact
749

<210> 4

<211> 166

<212> PRT

<213> Homo sapiens

<400> 4

Met Ala Gln Thr Ser Ser Tyr Phe Met Leu Ile Ser Cys Leu Met Phe

1               5                   10                  15

Leu Ser Gln Ser Gln Gly Gln Glu Ala Gln Thr Glu Leu Pro Gln Ala

            20                  25                  30

Arg Ile Ser Cys Pro Glu Gly Thr Asn Ala Tyr Arg Ser Tyr Cys Tyr

        35                  40                  45

Tyr Phe Asn Glu Asp Arg Glu Thr Trp Val Asp Ala Asp Leu Tyr Cys

    50                  55                  60

Gln Asn Met Asn Ser Gly Asn Leu Val Ser Val Leu Thr Gln Ala Glu

65                  70                  75                  80

Gly Ala Phe Val Ala Ser Leu Ile Lys Glu Ser Gly Thr Asp Asp Phe

                85                  90                  95

Asn Val Trp Ile Gly Leu His Asp Pro Lys Lys Asn Arg Arg Trp His

            100                 105                 110

Trp Ser Ser Gly Ser Leu Val Ser Tyr Lys Ser Trp Gly Ile Gly Ala

        115                 120                 125

Pro Ser Ser Val Asn Pro Gly Tyr Cys Val Ser Leu Thr Ser Ser Thr

    130                 135                 140

Gly Phe Gln Lys Trp Lys Asp Val Pro Cys Glu Asp Lys Phe Ser Phe

145                 150                 155                 160

Val Cys Lys Phe Lys Asn

                165

<210> 5

<211> 2974

<212> DNA

<213> Homo sapiens

<400> 5

ctcagggcag agggaggaag gacagcagac cagacagtca cagcagcctt gacaaaacgt 60

tcctggaact caagctcttc tccacagagg aggacagagc agacagcaga gaccatggag
120

tctccctcgg cccctcccca cagatggtgc atcccctggc agaggctcct gctcacagcc
180

tcacttctaa ccttctggaa cccgcccacc actgccaagc tcactattga atccacgccg
240

ttcaatgtcg cagaggggaa ggaggtgctt ctacttgtcc acaatctgcc ccagcatctt
300

tttggctaca gctggtacaa aggtgaaaga gtggatggca accgtcaaat tataggatat
360

gtaataggaa ctcaacaagc taccccaggg cccgcataca gtggtcgaga gataatatac
420

cccaatgcat ccctgctgat ccagaacatc atccagaatg acacaggatt ctacacccta
480

cacgtcataa agtcagatct tgtgaatgaa gaagcaactg gccagttccg ggtatacccg
540

gagctgccca agccctccat ctccagcaac aactccaaac ccgtggagga caaggatgct
600

gtggccttca cctgtgaacc tgagactcag gacgcaacct acctgtggtg ggtaaacaat
660

cagagcctcc cggtcagtcc caggctgcag ctgtccaatg gcaacaggac cctcactcta
720

ttcaatgtca caagaaatga cacagcaagc tacaaatgtg aaacccagaa cccagtgagt
780

gccaggcgca gtgattcagt catcctgaat gtcctctatg gcccggatgc ccccaccatt
840

tcccctctaa acacatctta cagatcaggg gaaaatctga acctctcctg ccacgcagcc
900

tctaacccac ctgcacagta ctcttggttt gtcaatggga ctttccagca atccacccaa
960

gagctcttta tccccaacat cactgtgaat aatagtggat cctatacgtg ccaagcccat
1020

aactcagaca ctggcctcaa taggaccaca gtcacgacga tcacagtcta tgcagagcca
1080

cccaaaccct tcatcaccag caacaactcc aaccccgtgg aggatgagga tgctgtagcc
1140

ttaacctgtg aacctgagat tcagaacaca acctacctgt ggtgggtaaa taatcagagc
1200

ctcccggtca gtcccaggct gcagctgtcc aatgacaaca ggaccctcac tctactcagt
1260

gtcacaagga atgatgtagg accctatgag tgtggaatcc agaacgaatt aagtgttgac
1320

cacagcgacc cagtcatcct gaatgtcctc tatggcccag acgaccccac catttccccc
1380

tcatacacct attaccgtcc aggggtgaac ctcagcctct cctgccatgc agcctctaac
1440

ccacctgcac agtattcttg gctgattgat gggaacatcc agcaacacac acaagagctc
1500

tttatctcca acatcactga gaagaacagc ggactctata cctgccaggc caataactca
1560

gccagtggcc acagcaggac tacagtcaag acaatcacag tctctgcgga gctgcccaag
1620

ccctccatct ccagcaacaa ctccaaaccc gtggaggaca aggatgctgt ggccttcacc
1680

tgtgaacctg aggctcagaa cacaacctac ctgtggtggg taaatggtca gagcctccca
1740

gtcagtccca ggctgcagct gtccaatggc aacaggaccc tcactctatt caatgtcaca
1800

agaaatgacg caagagccta tgtatgtgga atccagaact cagtgagtgc aaaccgcagt
1860

gacccagtca ccctggatgt cctctatggg ccggacaccc ccatcatttc ccccccagac
1920

tcgtcttacc tttcgggagc gaacctcaac ctctcctgcc actcggcctc taacccatcc
1980

ccgcagtatt cttggcgtat caatgggata ccgcagcaac acacacaagt tctctttatc
2040

gccaaaatca cgccaaataa taacgggacc tatgcctgtt ttgtctctaa cttggctact
2100

ggccgcaata attccatagt caagagcatc acagtctctg catctggaac ttctcctggt
2160

ctctcagctg gggccactgt cggcatcatg attggagtgc tggttggggt tgctctgata
2220

tagcagccct ggtgtagttt cttcatttca ggaagactga cagttgtttt gcttcttcct
2280

taaagcattt gcaacagcta cagtctaaaa ttgcttcttt accaaggata tttacagaaa
2340

agactctgac cagagatcga gaccatccta gccaacatcg tgaaacccca tctctactaa
2400

aaatacaaaa atgagctggg cttggtggcg cgcacctgta gtcccagtta ctcgggaggc
2460

tgaggcagga gaatcgcttg aacccgggag gtggagattg cagtgagccc agatcgcacc
2520

actgcactcc agtctggcaa cagagcaaga ctccatctca aaaagaaaag aaaagaagac
2580

tctgacctgt actcttgaat acaagtttct gataccactg cactgtctga gaatttccaa
2640

aactttaatg aactaactga cagcttcatg aaactgtcca ccaagatcaa gcagagaaaa
2700

taattaattt catgggacta aatgaactaa tgaggattgc tgattcttta aatgtcttgt
2760

ttcccagatt tcaggaaact ttttttcttt taagctatcc actcttacag caatttgata
2820

aaatatactt ttgtgaacaa aaattgagac atttacattt tctccctatg tggtcgctcc
2880

agacttggga aactattcat gaatatttat attgtatggt aatatagtta ttgcacaagt
2940

tcaataaaaa tctgctcttt gtataacaga aaaa
2974

<210> 6

<211> 2032

<212> DNA

<213> Homo sapiens

<400> 6

tccatattgt gcttccacca ctgccaataa caaaataact agcaaccatg aagtgggtgg 60

aatcaatttt tttaattttc ctactaaatt ttactgaatc cagaacactg catagaaatg
120

aatatggaat agcttccata ttggattctt accaatgtac tgcagagata agtttagctg
180

acctggctac catatttttt gcccagtttg ttcaagaagc cacttacaag gaagtaagca
240

aaatggtgaa agatgcattg actgcaattg agaaacccac tggagatgaa cagtcttcag
300

ggtgtttaga aaaccagcta cctgcctttc tggaagaact ttgccatgag aaagaaattt
360

tggagaagta cggacattca gactgctgca gccaaagtga agagggaaga cataactgtt
420

ttcttgcaca caaaaagccc actccagcat cgatcccact tttccaagtt ccagaacctg
480

tcacaagctg tgaagcatat gaagaagaca gggagacatt catgaacaaa ttcatttatg
540

agatagcaag aaggcatccc ttcctgtatg cacctacaat tcttctttgg gctgctcgct
600

atgacaaaat aattccatct tgctgcaaag ctgaaaatgc agttgaatgc ttccaaacaa
660

aggcagcaac agttacaaaa gaattaagag aaagcagctt gttaaatcaa catgcatgtg
720

cagtaatgaa aaattttggg acccgaactt tccaagccat aactgttact aaactgagtc
780

agaagtttac caaagttaat tttactgaaa tccagaaact agtcctggat gtggcccatg
840

tacatgagca ctgttgcaga ggagatgtgc tggattgtct gcaggatggg gaaaaaatca
900

tgtcctacat atgttctcaa caagacactc tgtcaaacaa aataacagaa tgctgcaaac
960

tgaccacgct ggaacgtggt caatgtataa ttcatgcaga aaatgatgaa aaacctgaag
1020

gtctatctcc aaatctaaac aggtttttag gagatagaga ttttaaccaa ttttcttcag
1080

gggaaaaaaa tatcttcttg gcaagttttg ttcatgaata ttcaagaaga catcctcagc
1140

ttgctgtctc agtaattcta agagttgcta aaggatacca ggagttattg gagaagtgtt
1200

tccagactga aaaccctctt gaatgccaag ataaaggaga agaagaatta cagaaataca
1260

tccaggagag ccaagcattg gcaaagcgaa gctgcggcct cttccagaaa ctaggagaat
1320

attacttaca aaatgcgttt ctcgttgctt acacaaagaa agccccccag ctgacctcgt
1380

cggagctgat ggccatcacc agaaaaatgg cagccacagc agccacttgt tgccaactca
1440

gtgaggacaa actattggcc tgtggcgagg gagcggctga cattattatc ggacacttat
1500

gtatcagaca tgaaatgact ccagtaaacc ctggtgttgg ccagtgctgc acttcttcat
1560

atgccaacag gaggccatgc ttcagcagct tggtggtgga tgaaacatat gtccctcctg
1620

cattctctga tgacaagttc attttccata aggatctgtg ccaagctcag ggtgtagcgc
1680

tgcaaacgat gaagcaagag tttctcatta accttgtgaa gcaaaagcca caaataacag
1740

aggaacaact tgaggctgtc attgcagatt tctcaggcct gttggagaaa tgctgccaag
1800

gccaggaaca ggaagtctgc tttgctgaag agggacaaaa actgatttca aaaactcgtg
1860

ctgctttggg agtttaaatt acttcagggg aagagaagac aaaacgagtc tttcattcgg
1920

tgtgaacttt tctctttaat tttaactgat ttaacacttt ttgtgaatta atgaaatgat
1980

aaagactttt atgtgagatt tccttatcac agaaataaaa tatctccaaa tg
2032

<210> 7

<211> 1639

<212> DNA

<213> Homo sapiens

<400> 7

agcagagcac acaagcttct aggacaagag ccaggaagaa accaccggaa ggaaccatct 60

cactgtgtgt aaacatgact tccaagctgg ccgtggctct cttggcagcc ttcctgattt
120

ctgcagctct gtgtgaaggt gcagttttgc caaggagtgc taaagaactt agatgtcagt
180

gcataaagac atactccaaa cctttccacc ccaaatttat caaagaactg agagtgattg
240

agagtggacc acactgcgcc aacacagaaa ttattgtaaa gctttctgat ggaagagagc
300

tctgtctgga ccccaaggaa aactgggtgc agagggttgt ggagaagttt ttgaagaggg
360

ctgagaattc ataaaaaaat tcattctctg tggtatccaa gaatcagtga agatgccagt
420

gaaacttcaa gcaaatctac ttcaacactt catgtattgt gtgggtctgt tgtagggttg
480

ccagatgcaa tacaagattc ctggttaaat ttgaatttca gtaaacaatg aatagttttt
540

cattgtacca tgaaatatcc agaacatact tatatgtaaa gtattattta tttgaatcta
600

caaaaaacaa caaataattt ttaaatataa ggattttcct agatattgca cgggagaata
660

tacaaatagc aaaattgagc caagggccaa gagaatatcc gaactttaat ttcaggaatt
720

gaatgggttt gctagaatgt gatatttgaa gcatcacata aaaatgatgg gacaataaat
780

tttgccataa agtcaaattt agctggaaat cctggatttt tttctgttaa atctggcaac
840

cctagtctgc tagccaggat ccacaagtcc ttgttccact gtgccttggt ttctccttta
900

tttctaagtg gaaaaagtat tagccaccat cttacctcac agtgatgttg tgaggacatg
960

tggaagcact ttaagttttt tcatcataac ataaattatt ttcaagtgta acttattaac
1020

ctatttatta tttatgtatt tatttaagca tcaaatattt gtgcaagaat ttggaaaaat
1080

agaagatgaa tcattgattg aatagttata aagatgttat agtaaattta ttttatttta
1140

gatattaaat gatgttttat tagataaatt tcaatcaggg tttttagatt aaacaaagaa
1200

acaattgggt acccagttaa attttcattt cagataaaca acaaataatt ttttagtata
1260

agtacattat tgtttatctg aaagttttaa ttgaactaac aatcctagtt tgatactccc
1320

agtcttgtca ttgccagctg tgttggtagt gctgtgttga attacggaat aatgagttag
1380

aactattaaa acagccaaaa ctccacagtc aatattagta atttcttgct ggttgaaact
1440

tgtttattat gtacaaatag attcttataa tattatttaa atgactgcat ttttaaatac
1500

aaggctttat atttttaact ttaagatgtt tttatgtgct ctccaaattt tttttactgt
1560

ttctgattgt atggaaatat aaaagtaaat atgaaacatt taaaatataa tttgttgtca
1620

aagtaaaaaa aaaaaaaaa
1639

<210> 8

<211> 1524

<212> DNA

<213> Homo sapiens

<400> 8

gcagagcaca gcatcgtcgg gaccagactc gtctcaggcc agttgcagcc ttctcagcca 60

aacgccgacc aaggaaaact cactaccatg agaattgcag tgatttgctt ttgcctccta
120

ggcatcacct gtgccatacc agttaaacag gctgattctg gaagttctga ggaaaagcag
180

ctttacaaca aatacccaga tgctgtggcc acatggctaa accctgaccc atctcagaag
240

cagaatctcc tagccccaca gacccttcca agtaagtcca acgaaagcca tgaccacatg
300

gatgatatgg atgatgaaga tgatgatgac catgtggaca gccaggactc cattgactcg
360

aacgactctg atgatgtaga tgacactgat gattctcacc agtctgatga gtctcaccat
420

tctgatgaat ctgatgaact ggtcactgat tttcccacgg acctgccagc aaccgaagtt
480

ttcactccag ttgtccccac agtagacaca tatgatggcc gaggtgatag tgtggtttat
540

ggactgaggt caaaatctaa gaagtttcgc agacctgaca tccagtaccc tgatgctaca
600

gacgaggaca tcacctcaca catggaaagc gaggagttga atggtgcata caaggccatc
660

cccgttgccc aggacctgaa cgcgccttct gattgggaca gccgtgggaa ggacagttat
720

gaaacgagtc agctggatga ccagagtgct gaaacccaca gccacaagca gtccagatta
780

tataagcgga aagccaatga tgagagcaat gagcattccg atgtgattga tagtcaggaa
840

ctttccaaag tcagccgtga attccacagc catgaatttc acagccatga agatatgctg
900

gttgtagacc ccaaaagtaa ggaagaagat aaacacctga aatttcgtat ttctcatgaa
960

ttagatagtg catcttctga ggtcaattaa aaggagaaaa aatacaattt ctcactttgc
1020

atttagtcaa aagaaaaaat gctttatagc aaaatgaaag agaacatgaa atgcttcttt
1080

ctcagtttat tggttgaatg tgtatctatt tgagtctgga aataactaat gtgtttgata
1140

attagtttag tttgtggctt catggaaact ccctgtaaac taaaagcttc agggttatgt
1200

ctatgttcat tctatagaag aaatgcaaac tatcactgta ttttaatatt tgttattctc
1260

tcatgaatag aaatttatgt agaagcaaac aaaatacttt tacccactta aaaagagaat
1320

ataacatttt atgtcactat aatcttttgt tttttaagtt agtgtatatt ttgttgtgat
1380

tatctttttg tggtgtgaat aaatctttta tcttgaatgt aataagaatt tggtggtgtc
1440

aattgcttat ttgttttccc acggttgtcc agcaattaat aaaacataac cttttttact
1500

gcctaaaaaa aaaaaaaaaa aaaa
1524

<210> 9

<211> 5500

<212> DNA

<213> Homo sapiens

<400> 9

gcagaccgtc gctaatgaat cttggggccg gtgtcgggcc ggggcggctt gatcggcaac 60

taggaaaccc caggcgcaga ggccaggagc gagggcagcg aggatcagag gccaggcctt
120

cccggctgcc ggcgctcctc ggaggtcagg gcagatgagg aacatgactc tcccccttcg
180

gaggaggaag gaagtcccgc tgccacctta tctctgctcc tctgcctcct ccctgttccc
240

agagcttttt ctctagagaa gattttgaag gcggcttttg tgctgacggc cacccaccat
300

catctaaaga agataaactt ggcaaatgac atgcaggttc ttcaaggcag aataattgca
360

gaaaatcttc aaaggaccct atctgcagat gttctgaata cctctgagaa tagagattga
420

ttattcaacc aggataccta attcaagaac tccagaaatc aggagacgga gacattttgt
480

cagttttgca acattggacc aaatacaatg aagtattctt gctgtgctct ggttttggct
540

gtcctgggca cagaattgct gggaagcctc tgttcgactg tcagatcccc gaggttcaga
600

ggacggatac agcaggaacg aaaaaacatc cgacccaaca ttattcttgt gcttaccgat
660

gatcaagatg tggagctggg gtccctgcaa gtcatgaaca aaacgagaaa gattatggaa
720

catggggggg ccaccttcat caatgccttt gtgactacac ccatgtgctg cccgtcacgg
780

tcctccatgc tcaccgggaa gtatgtgcac aatcacaatg tctacaccaa caacgagaac
840

tgctcttccc cctcgtggca ggccatgcat gagcctcgga cttttgctgt atatcttaac
900

aacactggct acagaacagc cttttttgga aaatacctca atgaatataa tggcagctac
960

atcccccctg ggtggcgaga atggcttgga ttaatcaaga attctcgctt ctataattac
1020

actgtttgtc gcaatggcat caaagaaaag catggatttg attatgcaaa ggactacttc
1080

acagacttaa tcactaacga gagcattaat tacttcaaaa tgtctaagag aatgtatccc
1140

cataggcccg ttatgatggt gatcagccac gctgcgcccc acggccccga ggactcagcc
1200

ccacagtttt ctaaactgta ccccaatgct tcccaacaca taactcctag ttataactat
1260

gcaccaaata tggataaaca ctggattatg cagtacacag gaccaatgct gcccatccac
1320

atggaattta caaacattct acagcgcaaa aggctccaga ctttgatgtc agtggatgat
1380

tctgtggaga ggctgtataa catgctcgtg gagacggggg agctggagaa tacttacatc
1440

atttacaccg ccgaccatgg ttaccatatt gggcagtttg gactggtcaa ggggaaatcc
1500

atgccatatg actttgatat tcgtgtgcct tttttattc gtggtccaag tgtagaacca
1560

ggatcaatag tcccacagat cgttctcaac attgacttgg cccccacgat cctggatatt
1620

gctgggctcg acacacctcc tgatgtggac ggcaagtctg tcctcaaact tctggaccca
1680

gaaaagccag gtaacaggtt tcgaacaaac aagaaggcca aaatttggcg tgatacattc
1740

ctagtggaaa gaggcaaatt tctacgtaag aaggaagaat ccagcaagaa tatccaacag
1800

tcaaatcact tgcccaaata tgaacgggtc aaagaactat gccagcaggc caggtaccag
1860

acagcctgtg aacaaccggg gcagaagtgg caatgcattg aggatacatc tggcaagctt
1920

cgaattcaca agtgtaaagg acccagtgac ctgctcacag tccggcagag cacgcggaac
1980

ctctacgctc gcggcttcca tgacaaagac aaagagtgca gttgtaggga gtctggttac
2040

cgtgccagca gaagccaaag aaagagtcaa cggcaattct tgagaaacca ggggactcca
2100

aagtacaagc ccagatttgt ccatactcgg cagacacgtt ccttgtccgt cgaatttgaa
2160

ggtgaaatat atgacataaa tctggaagaa gaagaagaat tgcaagtgtt gcaaccaaga
2220

aacattgcta agcgtcatga tgaaggccac aaggggccaa gagatctcca ggcttccagt
2280

ggtggcaaca ggggcaggat gctggcagat agcagcaacg ccgtgggccc acctaccact
2340

gtccgagtga cacacaagtg ttttattctt cccaatgact ctatccattg tgagagagaa
2400

ctgtaccaat cggccagagc gtggaaggac cataaggcat acattgacaa agagattgaa
2460

gctctgcaag ataaaattaa gaatttaaga gaagtgagag gacatctgaa gagaaggaag
2520

cctgaggaat gtagctgcag taaacaaagc tattacaata aagagaaagg tgtaaaaaag
2580

caagagaaat taaagagcca tcttcaccca ttcaaggagg ctgctcagga agtagatagc
2640

aaactgcaac ttttcaagga gaacaaccgt aggaggaaga aggagaggaa ggagaagaga
2700

cggcagagga aggggaaga gtgcagcctg cctggcctca cttgcttcac gcatgacaac
2760

aaccactggc agacagcccc gttctggaac ctgggatctt tctgtgcttg cacgagttct
2820

aacaataaca cctactggtg tttgcgtaca gttaatgaga cgcataattt tcttttctgt
2880

gagtttgcta ctggcttttt ggagtatttt gatatgaata cagatcctta tcagctcaca
2940

aatacagtgc acacggtaga acgaggcatt ttgaatcagc tacacgtaca actaatggag
3000

ctcagaagct gtcaaggata taagcagtgc aacccaagac ctaagaatct tgatgttgga
3060

aataaagatg gaggaagcta tgacctacac agaggacagt tatgggatgg atgggaaggt
3120

taatcagccc cgtctcactg cagacatcaa ctggcaaggc ctagaggagc tacacagtgt
3180

gaatgaaaac atctatgagt acagacaaaa ctacagactt agtctggtgg actggactaa
3240

ttacttgaag gatttagata gagtatttgc actgctgaag agtcactatg agcaaaataa
3300

aacaaataag actcaaactg ctcaaagtga cgggttcttg gttgtctctg ctgagcacgc
3360

tgtgtcaatg gagatggcct ctgctgactc agatgaagac ccaaggcata aggttgggaa
3420

aacacctcat ttgaccttgc cagctgacct tcaaaccctg catttgaacc gaccaacatt
3480

aagtccagag agtaaacttg aatggaataa cgacattcca gaagttaatc atttgaattc
3540

tgaacactgg agaaaaaccg aaaaatggac ggggcatgaa gagactaatc atctggaaac
3600

cgatttcagt ggcgatggca tgacagagct agagctcggg cccagcccca ggctgcagcc
3660

cattcgcagg cacccgaaag aacttcccca gtatggtggt cctggaaagg acatttttga
3720

agatcaacta tatcttcctg tgcattccga tggaatttca gttcatcaga tgttcaccat
3780

ggccaccgca gaacaccgaa gtaattccag catagcgggg aagatgttga ccaaggtgga
3840

gaagaatcac gaaaaggaga agtcacagca cctagaaggc agcgcctcct cttcactctc
3900

ctctgattag atgaaactgt taccttaccc taaacacagt atttcttttt aactttttta
3960

tttgtaaact aataaaggta atcacagcca ccaacattcc aagctaccct gggtaccttt
4020

gtgcagtaga agctagtgag catgtgagca agcggtgtgc acacggagac tcatcgttat
4080

aatttactat ctgccaagag tagaaagaaa ggctggggat atttgggttg gcttggtttt
4140

gattttttgc ttgtttgttt gttttgtact aaaacagtat tatcttttga atatcgtagg
4200

gacataagta tatacatgtt atccaatcaa gatggctaga atggtgcctt tctgagtgtc
4260

taaaacttga cacccctggt aaatctttca acacacttcc actgcctgcg taatgaagtt
4320

ttgattcatt tttaaccact ggaatttttc aatgccgtca ttttcagtta gatgattttg
4380

cactttgaga ttaaaatgcc atgtctattt gattagtctt atttttttat ttttacaggc
4440

ttatcagtct cactgttggc tgtcattgtg acaaagtcaa ataaaccccc aaggacgaca
4500

cacagtatgg atcacatatt gtttgacatt aagcttttgc cagaaaatgt tgcatgtgtt
4560

ttacctcgac ttgctaaaat cgattagcag aaaggcatgg ctaataatgt tggtggtgaa
4620

aataaataaa taagtaaaca aaatgaagat tgcctgctct ctctgtgcct agcctcaaag
4680

cgttcatcat acatcatacc tttaagattg ctatattttg ggttattttc ttgacaggag
4740

aaaaagatct aaagatcttt tattttcatc ttttttggtt ttcttggcat gactaagaag
4800

cttaaatgtt gataaaatat gactagtttt gaatttacac caagaacttc tcaataaaag
4860

aaaatcatga atgctccaca atttcaacat accacaagag aagttaattt cttaacattg
4920

tgttctatga ttatttgtaa gaccttcacc aagttctgat atcttttaaa gacatagttc
4980

aaaattgctt ttgaaaatct gtattcttga aaatatcctt gttgtgtatt aggtttttaa
5040

ataccagcta aaggattacc tcactgagtc atcagtaccc tcctattcag ctccccaaga
5100

tgatgtgttt ttgcttaccc taagagaggt tttcttctta tttttagata attcaagtgc
5160

ttagataaat tatgttttct ttaagtgttt atggtaaact cttttaaaga aaatttaata
5220

tgttatagct gaatcttttt ggtaacttta aatctttatc atagactctg tacatatgtt
5280

caaattagct gcttgcctga tgtgtgtatc atcggtggga tgacagaaca aacatattta
5340

tgatcatgaa taatgtgctt tgtaaaaaga tttcaagtta ttaggaagca tactctgttt
5400

tttaatcatg tataatattc catgatactt ttatagaaca attctggctt caggaaagtc
5460

tagaagcaat atttcttcaa ataaaaggtg tttaaacttt
5500

<210> 10

<211> 1778

<212> DNA

<213> Homo sapiens

<400> 10

tagaagttta caatgaagtt tcttctaata ctgctcctgc aggccactgc ttctggagct 60

cttcccctga acagctctac aagcctggaa aaaaataatg tgctatttgg tgagagatac
120

ttagaaaaat tttatggcct tgagataaac aaacttccag tgacaaaaat gaaatatagt
180

ggaaacttaa tgaaggaaaa aatccaagaa atgcagcact tcttgggtct gaaagtgacc
240

gggcaactgg acacatctac cctggagatg atgcacgcac ctcgatgtgg agtccccgat
300

ctccatcatt tcagggaaat gccagggggg cccgtatgga ggaaacatta tatcacctac
360

agaatcaata attacacacc tgacatgaac cgtgaggatg ttgactacgc aatccggaaa
420

gctttccaag tatggagtaa tgttaccccc ttgaaattca gcaagattaa cacaggcatg
480

gctgacattt tggtggtttt tgcccgtgga gctcatggag acttccatgc ttttgatggc
540

aaaggtggaa tcctagccca tgcttttgga cctggatctg gcattggagg ggatgcacat
600

ttcgatgagg acgaattctg gactacacat tcaggaggca caaacttgtt cctcactgct
660

gttcacgaga ttggccattc cttaggtctt ggccattcta gtgatccaaa ggctgtaatg
720

ttccccacct acaaatatgt cgacatcaac acatttcgcc tctctgctga tgacatacgt
780

ggcattcagt ccctgtatgg agacccaaaa gagaaccaac gcttgccaaa tcctgacaat
840

tcagaaccag ctctctgtga ccccaatttg agttttgatg ctgtcactac cgtgggaaat
900

aagatctttt tcttcaaaga caggttcttc tggctgaagg tttctgagag accaaagacc
960

agtgttaatt taatttcttc cttatggcca accttgccat ctggcattga agctgcttat
1020

gaaattgaag ccagaaatca agtttttctt tttaaagatg acaaatactg gttaattagc
1080

aatttaagac cagagccaaa ttatcccaag agcatacatt cttttggttt tcctaacttt
1140

gtgaaaaaaa ttgatgcagc tgtttttaac ccacgttttt ataggaccta cttctttgta
1200

gataaccagt attggaggta tgatgaaagg agacagatga tggaccctgg ttatcccaaa
1260

ctgattacca agaacttcca aggaatcggg cctaaaattg atgcagtctt ctattctaaa
1320

aacaaatact actatttctt ccaaggatct aaccaatttg aatatgactt cctactccaa
1380

cgtatcacca aaacactgaa aagcaatagc tggtttggtt gttagaaatg gtgtaattaa
1440

tggtttttgt tagttcactt cagcttaata agtatttatt gcatatttgc tatgtcctca
1500

gtgtaccact acttagagat atgtatcata aaaataaaat ctgtaaacca taggtaatga
1560

ttatataaaa tacataatat ttttcaattt tgaaaactct aattgtccat tcttgcttga
1620

ctctactatt aagtttgaaa atagttacct tcaaagcaag ataattctat ttgaagcatg
1680

ctctgtaagt tgcttcctaa catccttgga ctgagaaatt atacttactt ctggcataac
1740

taaaattaag tatatatatt ttggctcaaa taaaattg
1778

<210> 11

<211> 777

<212> DNA

<213> Homo sapiens

<400> 11

ggccccttgt ctgcagagat ggctcccaat gcttcctgcc tctgtgtgca tgtccgttcc 60

gaggaatggg atttaatgac ctttgatgcc aacccatatg acagcgtgaa aaaaatcaaa
120

gaacatgtcc ggtctaagac caaggttcct gtgcaggacc aggttctttt gctgggctcc
180

aagatcttaa agccacggag aagcctctca tcttatggca ttgacaaaga gaagaccatc
240

caccttaccc tgaaagtggt gaagcccagt gatgaggagc tgcccttgtt tcttgtggag
300

tcaggtgatg aggcaaagag gcacctcctc caggtgcgaa ggtccagctc agtggcacaa
360

gtgaaagcaa tgatcgagac taagacgggt ataatccctg agacccagat tgtgacttgc
420

aatggaaaga gactggaaga tgggaagatg atggcagatt acggcatcag aaagggcaac
480

ttactcttcc tggcatctta ttgtattgga gggtgaccac cctggggatg gggtgttggc
540

aggggtcaaa aagcttattt cttttaatct cttactcaac gaacacatct tctgatgatt
600

tcccaaaatt aatgagaatg agatgagtag agtaagattt gggtgggatg ggtaggatga
660

agtatattgc ccaactctat gtttctttga ttctaacaca attaattaag tgacatgatt
720

tttactaatg tattactgag actagtaaat aaatttttaa ggcaaaatag agcattc
777

<210> 12

<211> 5921

<212> DNA

<213> Homo sapiens

<400> 12

agcagacggg agtttctcct cggggtcgga gcaggaggca cgcggagtgt gaggccacgc 60

atgagcggac gctaaccccc tccccagcca caaagagtct acatgtctag ggtctagaca
120

tgttcagctt tgtggacctc cggctcctgc tcctcttagc ggccaccgcc ctcctgacgc
180

acggccaaga ggaaggccaa gtcgagggcc aagacgaaga catcccacca atcacctgcg
240

tacagaacgg cctcaggtac catgaccgag acgtgtggaa acccgagccc tgccggatct
300

gcgtctgcga caacggcaag gtgttgtgcg atgacgtgat ctgtgacgag accaagaact
360

gccccggcgc cgaagtcccc gagggcgagt gctgtcccgt ctgccccgac ggctcagagt
420

cacccaccga ccaagaaacc accggcgtcg agggacccaa gggagacact ggcccccgag
480

gcccaagggg acccgcaggc ccccctggcc gagatggcat ccctggacag cctggacttc
540

ccggaccccc cggacccccc ggacctcccg gaccccctgg cctcggagga aactttgctc
600

cccagctgtc ttatggctat gatgagaaat caaccggagg aatttccgtg cctggcccca
660

tgggtccctc tggtcctcgt ggtctccctg gcccccctgg tgcacctggt ccccaaggct
720

tccaaggtcc ccctggtgag cctggcgagc ctggagcttc aggtcccatg ggtccccgag
780

gtcccccagg tccccctgga aagaatggag atgatgggga agctggaaaa cctggtcgtc
840

ctggtgagcg tgggcctcct gggcctcagg gtgctcgagg attgcccgga acagctggcc
900

tccctggaat gaagggacac agaggtttca gtggtttgga tggtgccaag ggagatgctg
960

gtcctgctgg tcctaagggt gagcctggca gccctggtga aaatggagct cctggtcaga
1020

tgggcccccg tggcctgcct ggtgagagag gtcgccctgg agcccctggc cctgctggtg
1080

ctcgtggaaa tgatggtgct actggtgctg ccgggccccc tggtcccacc ggccccgctg
1140

gtcctcctgg cttccctggt gctgttggtg ctaagggtga agctggtccc caagggcccc
1200

gaggctctga aggtccccag ggtgtgcgtg gtgagcctgg cccccctggc cctgctggtg
1260

ctgctggccc tgctggaaac cctggtgctg atggacagcc tggtgctaaa ggtgccaatg
1320

gtgctcctgg tattgctggt gctcctggct tccctggtgc ccgaggcccc tctggacccc
1380

agggccccgg cggccctcct ggtcccaagg gtaacagcgg tgaacctggt gctcctggca
1440

gcaaaggaga cactggtgct aagggagagc ctggccctgt tggtgttcaa ggaccccctg
1500

gccctgctgg agaggaagga aagcgaggag ctcgaggtga acccggaccc actggcctgc
1560

ccggaccccc tggcgagcgt ggtggacctg gtagccgtgg tttccctggc gcagatggtg
1620

ttgctggtcc caagggtccc gctggtgaac gtggttctcc tggccccgct ggccccaaag
1680

gatctcctgg tgaagctggt cgtcccggtg aagctggtct gcctggtgcc aagggtctga
1740

ctggaagccc tggcagccct ggtcctgatg gcaaaactgg cccccctggt cccgccggtc
1800

aagatggtcg ccccggaccc ccaggcccac ctggtgcccg tggtcaggct ggtgtgatgg
1860

gattccctgg acctaaaggt gctgctggag agcccggcaa ggctggagag cgaggtgttc
1920

ccggaccccc tggcgctgtc ggtcctgctg gcaaagatgg agaggctgga gctcagggac
1980

cccctggccc tgctggtccc gctggcgaga gaggtgaaca aggccctgct ggctcccccg
2040

gattccaggg tctccctggt cctgctggtc ctccaggtga agcaggcaaa cctggtgaac
2100

agggtgttcc tggagacctt ggcgcccctg gcccctctgg agcaagaggc gagagaggtt
2160

tccctggcga gcgtggtgtg caaggtcccc ctggtcctgc tggaccccga ggggccaacg
2220

gtgctcccgg caacgatggt gctaagggtg atgctggtgc ccctggagct cccggtagcc
2280

agggcgcccc tggccttcag ggaatgcctg gtgaacgtgg tgcagctggt cttccagggc
2340

ctaagggtga cagaggtgat gctggtccca aaggtgctga tggctctcct ggcaaagatg
2400

gcgtccgtgg tctgaccggc cccattggtc ctcctggccc tgctggtgcc cctggtgaca
2460

agggtgaaag tggtcccagc ggccctgctg gtcccactgg agctcgtggt gcccccggag
2520

accgtggtga gcctggtccc cccggccctg ctggctttgc tggcccccct ggtgctgacg
2580

gccaacctgg tgctaaaggc gaacctggtg atgctggtgc caaaggcgat gctggtcccc
2640

ctgggcctgc cggacccgct ggaccccctg gccccattgg taatgttggt gctcctggag
2700

ccaaaggtgc tcgcggcagc gctggtcccc ctggtgctac tggtttccct ggtgctgctg
2760

gccgagtcgg tcctcctggc ccctctggaa atgctggacc ccctggccct cctggtcctg
2820

ctggcaaaga aggcggcaaa ggtccccgtg gtgagactgg ccctgctgga cgtcctggtg
2880

aagttggtcc ccctggtccc cctggccctg ctggcgagaa aggatcccct ggtgctgatg
2940

gtcctgctgg tgctcctggt actcccgggc ctcaaggtat tgctggacag cgtggtgtgg
3000

tcggcctgcc tggtcagaga ggagagagag gcttccctgg tcttcctggc ccctctggtg
3060

aacctggcaa acaaggtccc tctggagcaa gtggtgaacg tggtcccccc ggtcccatgg
3120

gcccccctgg attggctgga ccccctggtg aatctggacg tgagggggct cctgctgccg
3180

aaggttcccc tggacgagac ggttctcctg gcgccaaggg tgaccgtggt gagaccggcc
3240

ccgctggacc ccctggtgct cctggtgctc ctggtgcccc tggccccgtt ggccctgctg
3300

gcaagagtgg tgatcgtggt gagactggtc ctgctggtcc cgccggtccc gtcggccccg
3360

tcggcgcccg tggccccgcc ggaccccaag gcccccgtgg tgacaagggt gagacaggcg
3420

aacagggcga cagaggcata aagggtcacc gtggcttctc tggcctccag ggtccccctg
3480

gccctcctgg ctctcctggt gaacaaggtc cctctggagc ctctggtcct gctggtcccc
3540

gaggtccccc tggctctgct ggtgctcctg gcaaagatgg actcaacggt ctccctggcc
3600

ccattgggcc ccctggtcct cgcggtcgca ctggtgatgc tggtcctgtt ggtccccccg
3660

gccctcctgg acctcctggt ccccctggtc ctcccagcgc tggtttcgac ttcagcttcc
3720

tgccccagcc acctcaagag aaggctcacg atggtggccg ctactaccgg gctgatgatg
3780

ccaatgtggt tcgtgaccgt gacctcgagg tggacaccac cctcaagagc ctgagccagc
3840

agatcgagaa catccggagc ccagagggaa gccgcaagaa ccccgcccgc acctgccgtg
3900

acctcaagat gtgccactct gactggaaga gtggagagta ctggattgac cccaaccaag
3960

gctgcaacct ggatgccatc aaagtcttct gcaacatgga gactggtgag acctgcgtgt
4020

accccactca gcccagtgtg gcccagaaga actggtacat cagcaagaac cccaaggaca
4080

agaggcatgt ctggttcggc gagagcatga ccgatggatt ccagttcgag tatggcggcc
4140

agggctccga ccctgccgat gtggccatcc agctgacctt cctgcgcctg atgtccaccg
4200

aggcctccca gaacatcacc taccactgca agaacagcgt ggcctacatg gaccagcaga
4260

ctggcaacct caagaaggcc ctgctcctca agggctccaa cgagatcgag atccgcgccg
4320

agggcaacag ccgcttcacc tacagcgtca ctgtcgatgg ctgcacgagt cacaccggag
4380

cctggggcaa gacagtgatt gaatacaaaa ccaccaagtc ctcccgcctg cccatcatcg
4440

atgtggcccc cttggacgtt ggtgccccag accaggaatt cggcttcgac gttggccctg
4500

tctgcttcct gtaaactccc tccatcccaa cctggctccc tcccacccaa ccaactttcc
4560

ccccaacccg gaaacagaca agcaacccaa actgaacccc cccaaaagcc aaaaaatggg
4620

agacaatttc acatggactt tggaaaatat ttttttcctt tgcattcatc tctcaaactt
4680

agtttttatc tttgaccaac cgaacatgac caaaaaccaa aagtgcattc aaccttacca
4740

aaaaaaaaaa aaaaaaaaaa agaataaata aataagtttt taaaaaagga agcttggtcc
4800

acttgcttga agacccatgc gggggtaagt ccctttctgc ccgttgggtt atgaaacccc
4860

aatgctgccc tttctgctcc tttctccaca ccccccttgg cctcccctcc actccttccc
4920

aaatctgtct ccccagaaga cacaggaaac aatgtattgt ctgcccagca atcaaaggca
4980

atgctcaaac acccaagtgg cccccaccct cagcccgctc ctgcccgccc agcaccccca
5040

ggccctgggg acctggggtt ctcagactgc caaagaagcc ttgccatctg gcgctcccat
5100

ggctcttgca acatctcccc ttcgtttttg agggggtcat gccgggggag ccaccagccc
5160

ctcactgggt tcggaggaga gtcaggaagg gccacgacaa agcagaaaca tcggatttgg
5220

ggaacgcgtg tcatcccttg tgccgcaggc tgggcgggag agactgttct gttctgttcc
5280

ttgtgtaact gtgttgctga aagactacct cgttcttgtc ttgatgtgtc accggggcaa
5340

ctgcctgggg gcggggatgg gggcagggtg gaagcggctc cccattttta taccaaaggt
5400

gctacatcta tgtgatgggt ggggtgggga gggaatcact ggtgctatag aaattgagat
5460

gcccccccag gccagcaaat gttccttttt gttcaaagtc tatttttatt ccttgatatt
5520

ttttctttct tttttttttt ttttgtggat ggggacttgt gaatttttct aaaggtgcta
5580

tttaacatgg gaggagagcg tgtgcgctcc agcccagccc gctgctcact ttccaccctc
5640

tctccacctg cctctggctt ctcaggcctc tgctctccga cctctctcct ctgaaaccct
5700

cctccacagc tgcagcccat cctcccggct ccctcctagt ctgtcctgcg tcctctgtcc
5760

ccgggtttca gagacaactt cccaaagcac aaagcagttt ttccctaggg gtgggaggaa
5820

gcaaaagact ctgtacctat tttgtatgtg tataataatt tgagatgttt ttaattattt
5880

tgattgctgg aataaagcat gtggaaatga cccaaacata a
5921

<210> 13

<211> 1804

<212> DNA

<213> Homo sapiens

<400> 13
   gtatcactca gaatctggca gccagttccg tcctgacaga gttcacagca tatattggtg 60
   gattcttgtc catagtgcat ctgctttaag aattaacgaa agcagtgtca agacagtaag
   120
   gattcaaacc atttgccaaa aatgagtcta agtgcattta ctctcttcct ggcattgatt
   180
   ggtggtacca gtggccagta ctatgattat gatttttccc tatcaattta tgggcaatca
   240
   tcaccaaact gtgcaccaga atgtaactgc cctgaaagct acccaagtgc catgtactgt
   300
   gatgagctga aattgaaaag tgtaccaatg gtgcctcctg gaatcaagta tctttacctt
   360
   aggaataacc agattgacca tattgatgaa aaggcctttg agaatgtaac tgatctgcag
   420
   tggctcattc tagatcacaa ccttctagaa aactccaaga taaaagggag agttttctct
   480
   aaattgaaac aactgaagaa gctgcatata aaccacaaca acctgacaga gtctgtgggc
   540
   ccacttccca aatctctgga ggatctgcag cttactcata acaagatcac aaagctgggc
   600
   tcttttgaag gattggtaaa cctgaccttc atccatctcc agcacaatcg gctgaaagag
   660
   gatgctgttt cagctgcttt taaaggtctt aaatcactcg aataccttga cttgagcttc
   720
   aatcagatag ccagactgcc ttctggtctc cctgtctctc ttctaactct ctacttagac
   780
   aacaataaga tcagcaacat ccctgatgag tatttcaagc gttttaatgc attgcagtat
   840
   ctgcgtttat ctcacaacga actggctgat agtggaatac ctggaaattc tttcaatgtg
   900
   tcatccctgg ttgagctgga tctgtcctat aacaagctta aaaacatacc aactgtcaat
   960
   gaaaaccttg aaaactatta cctggaggtc aatcaacttg agaagtttga cataaagagc
   1020
   ttctgcaaga tcctggggcc attatcctac tccaagatca agcatttgcg tttggatggc
   1080
   aatcgcatct cagaaaccag tcttccaccg gatatgtatg aatgtctacg tgttgctaac
   1140
   gaagtcactc ttaattaata tctgtatcct ggaacaatat tttatggtta tgtttttctg
   1200
   tgtgtcagtt ttcatagtat ccatatttta ttactgttta ttacttccat gaattttaaa
   1260
   atctgaggga aatgttttgt aaacatttat tttttttaaa gaaaagatga aaggcaggcc
   1320
   tatttcatca caagaacaca cacatataca cgaatagaca tcaaactcaa tgctttattt
   1380
   gtaaatttag tgttttttta tttctactgt caaatgatgt gcaaaacctt ttactggttg
   1440
   catggaaatc agccaagttt tataatcctt aaatcttaat gttcctcaaa gcttggatta
   1500
   aatacatatg gatgttactc tcttgcacca aattatcttg atacattcaa atttgtctgg
   1560
   ttaaaaaata ggtggtagat attgaggcca agaatattgc aaaatacatg aagcttcatg
   1620
   cacttaaaga agtattttta gaataagaat ttgcatactt acctagtgaa acttttctag
   1680
   aattattttt cactctaagt catgtatgtt tctctttgat tatttgcatg ttatgtttaa
   1740
   taagctacta gcaaaataaa acatagcaaa tgaaaaaaaa aaaaaaaaaa aaaaaaaaaa
   1800
   aaaa
   1804

<210> 14

<211> 4827

<212> DNA

<213> Homo sapiens

<400> 14

gcggtggcgc tgcggagacc cggtccagac gcctggcggc cgccggcaca caaggcgctt 60

tctagctccc tcccccgagc gcacagcccg cctccttccg cggcgcctgc agtggcaggc
120

ttgctctgcc ctaccgtgac gcgctccgga gacgctctgc gggtcctgga caccgggtcc
180

gcggcgtggg gacgacagac ggaggcgaac gccatcggta gccggtccgc gagccatcgt
240

tcggggcgca gtcctctccc cggctggccc tcctttctcc ggggcattcg ccaccgcttc
300

cctgggctga gacgaccggt tcgtcgcctc cttgcccgtg accgtcgcta gaactcagtt
360

gtgcgttgcg gccagtcgcc actgctgagt ggaagcaaaa tgtcagtcag tgtgcatgag
420

aaccgcaagt ccagggccag cagcggctcc attaacatct atctgtttca caagtcctcc
480

tacgctgaca gcgtcctcac tcacctgaat cttttacgcc agcagcgtct cttcactgac
540

gtccttctcc atgccggaaa taggaccttc ccttgccacc gggcagtgct ggctgcatgc
600

agtcgctact ttgaggccat gttcagtggt ggcctgaaag agagccagga cagtgaggtc
660

aactttgaca attccatcca cccagaagtc ttggagctgc tgcttgacta tgcgtactcc
720

tcccgggtca tcatcaatga agaaaatgca gaatcgctcc tggaagctgg tgacatgctg
780

gagtttcaag acatccggga tgcatgtgca gagttcctgg aaaagaacct gcatcccacc
840

aactgcctgg gcatgctgct gctgtctgat gcacaccagt gcaccaagct gtacgaacta
900

tcttggagaa tgtgtctcag caacttccaa accatcagga agaatgaaga tttcctccag
960

ctgccccagg acatggtagt gcaactcttg tccagtgaag agctggagac agaggatgaa
1020

aggcttgtgt acgagtctgc aattaactgg atcagctatg acctgaagaa gcgctattgc
1080

tacctcccag aactgttgca gacagtaagg ctggcacttc tgccagccat ctatctcatg
1140

gagaatgtgg ccatggagga actcatcacc aagcagagaa agagtaagga aattgtggaa
1200

gaggccatca ggtgcaaact gaaaatcctg cagaatgacg gtgtggtaac cagcctctgt
1260

gcccgacctc ggaaaactgg ccatgccctc ttccttctgg gaggacagac tttcatgtgt
1320

gacaagttgt atctggtaga ccagaaggcc aaagaaatca ttcccaaggc tgacattccc
1380

agcccaagaa aagagtttag tgcatgtgcg attggctgca aagtgtacat tactgggggg
1440

cgggggtctg aaaatggggt ctcgaaagat gtctgggttt atgataccct gcacgaggag
1500

tggtccaagg ctgcccccat gctggtggcc aggtttggcc atggctctgc tgaactgaag
1560

cactgcctgt atgtggttgg ggggcacacg gccgcaactg gctgcctccc ggcctccccc
1620

tcagtctctc taaagcaggt agaacattat gaccccacaa tcaacaaatg gaccatggtg
1680

gccccactcc gagaaggcgt tagcaacgcc gcagtagtga gtgccaaact taagttattt
1740

gctttcggag gtaccagtgt cagtcatgac aagctcccca aagttcagtg ttacgatcag
1800

tgtgaaaaca ggtggactgt accggccacc tgtccccagc cctggcgtta cacagcagca
1860

gctgtgctgg ggaaccagat ttttattatg gggggtgata cagaattctc tgcctgctct
1920

gcttataaat tcaacagtga gacttaccag tggaccaaag tgggagatgt gacagcaaag
1980

cgcatgagct gccatgctgt ggcctctgga aacaaactct acgtggttgg aggatacttt
2040

ggcattcagc gatgcaagac tttggactgc tacgatccaa cattagacgt gtggaacagc
2100

atcaccactg tcccgtactc gctgattcct actgcatttg tcagcacctg gaaacatctg
2160

ccttcttaaa tgcagtacat tctaaagaga gtgagcatga gctcactcca tcactcgatg
2220

agataatatg agatttctac ttcggagagg ccaagtctaa tgaagagaaa aaaaggaaaa
2280

gaagttgcaa gactcgaata aaatctgctg caccttgtaa atgctctaac tggacatgaa
2340

ggaaaggggc gagggagggg ggtgggattt ttggtgcaag tagcacatgg tttaaatatg
2400

aatgaacaaa cctgtgatct agtccttgtc ttgtaattgt ggattaatgt caatgttaat
2460

cagcccctca aagggagaga aaagctggac cttttccctt gctgtaccat attcagcatt
2520

tgatttccat gggccccacc atttatgtgt agaatttgaa atggttgtca cctctctctg
2580

aggacagagc ttgaagcctc cacaccagct gctgctggag attcaaagcc caactgtggg
2640

tccgagaggg aagctggctg ggctggctga agaatgaaga ccactggact ctccgttaat
2700

ctctaagggg tctgctcccc aggaacgttt ctgaacaatg gggactttgt tggtagccat
2760

ttggtagatg ttcttttcta tttataagtg actttaaact ttcccttggc tgttaagaag
2820

tttgttatag atttagctat ttattgttcg atgcctgcat gctgaaacaa tgcctacagc
2880

tgtcttcaca tgtatggacg tgtgtgaatg gttgtacgtt ttgcacattt tgtggctgtt
2940

gagatgtgct ttgctgcaca aacatgaaaa tttttgagtt acaatttgga gcataactgg
3000

agggtgggct ggggaggggt ggatttttaa aatgtcaaga cagggaagga tgacaaaatg
3060

gaaatttaaa tgacatccta gaggtagaga aaccgtggag atcgcttttc tcagactcac
3120

caacttttaa tgggatttca tggggtttgg ttgtgctgat agggtaaggg gaggctgctt
3180

tctgcccttc tccccactcc catctgattt acttaattca gtctcagctg ctgaaatttg
3240

gaaaggacca aattgcttta cagttttttt ctttgtgtag tatcttgaaa tcctggaaaa
3300

ttctatggaa tagttctgta tatagggcac aagtaaaggc attgtccaaa gtttatttat
3360

ttatttatta ccctaagaat gctttgccat aaccacattt aatgggaaaa acggcatgta
3420

tcacagatgt aaattaactc accagattta ctgggcctga actcattctc ttcttgctat
3480

atgatttagc aagttctaga aggtctccaa gacaataatt acattggcac aatgtatact
3540

tcagtgctca cccgtagcaa atctcttttt aaaaaactct ttggtgcaca agtaacacat
3600

ttggccacaa aacaccaaag aattgtaggc agtggcccct attgagaagt tttccggtag
3660

agttggaaat cagttgtgaa tacattcttt gctagttgga gtgcttgttt actaagcatg
3720

tgccgtcgta ggtattagtg ctagtctcaa ataggtgctt cccctgaggt gcaggggaag
3780

accaaagttt gcaactcgaa ctgctttcgt ccatgtttct cacattgctg tattttagaa
3840

aataggggtt aagactgata acaacctttt acattgtgac tgtgtttgca ttgtctaatg
3900

acagataaat ccttaacatt tctctccacc ttagtacttt agactaattg tgtttgtccg
3960

tccatgccat gaatgagtgg gctgtagttg ggcctaaata aatgagctgt tggaagaaaa
4020

gaatcacagt actttccagc agtcagtccc tggttcctag atgtgttcta agcaatgcaa
4080

atgtctaatt gtcccccagt gggcatagtc agtgtcgttt atattgtagc agttacagct
4140

ctgtagttta tgatgcaaat ctgccaagag agatgtatgt gtcactgcat ggcttctgaa
4200

agcaggatga attttctgca gctgtttcaa agttggggtc tgttcttgaa tcctctatta
4260

<400> 16

atgcgcgatc tcccggagca tgcgcagcag cggcgccgac gcggggcggt gcctggtgac 60

cgcgcgcgct cccggaagtg tgccggcgtc gcgcgaaggt tcagcaggga gccgtgggcc
120

gggcgcgcgg ttcccggcac gtgtctcggc acgtggcagc gcgcctggcc ctgggcttgg
180

aggcgccggc gccctggatc cgccggccgt ggtcgccgag tcggtgtcgt ccttgaccat
240

cgccgacgcg ttcattgcag ccggcgagag ctcagctccg accccgccgc gccccgcgct
300

tcccaggagg ttcatctgct ccttccctga ctgcagcgcc aattacagca aagcctggaa
360

gcttgacgcg cacctgtgca agcacacggg ggagagacca tttgtttgtg actatgaagg
420

gtgtggcaag gccttcatca gggactacca tctgagccgc cacattctga ctcacacagg
480

agaaaagccg tttgtttgtg cagccaatgg ctgtgatcaa aaattcaaca caaaatcaaa
540

cttgaagaaa cattttgaac gcaaacatga aaatcaacaa aaacaatata tatgcagttt
600

tgaagactgt aagaagacct ttaagaaaca tcagcagctg aaaatccatc agtgccagaa
660

taccaatgaa cctctattca agtgtaccca ggaaggatgt gggaaacact ttgcatcacc
720

cagcaagctg aaacgacatg ccaaggccca cgagggctat gtatgtcaaa aaggatgttc
780

ctttgtggca aaaacatgga cggaacttct gaaacatgtg agagaaaccc ataaagagga
840

aatactatgt gaagtatgcc ggaaaacatt taaacgcaaa gattacctta agcaacacat
900

gaaaactcat gccccagaaa gggatgtatg tcgctgtcca agagaaggct gtggaagaac
960

ctatacaact gtgtttaatc tccaaagcca tatcctctcc ttccatgagg aaagccgccc
1020

ttttgtgtgt gaacatgctg gctgtggcaa aacatttgca atgaaacaaa gtctcactag
1080

actttaggcc acagtgtctt gttatgacag gtgctccaaa ctcacgtcca gctttacttc
2580

atcttgttca tgatttcaca aaaaatgttg gtttgatgat ctgtggccat gtacatatgg
2640

gtcctcgaag acaagccatg aaagagatgt ccatcgatca agccaaatat cagcgatggc
2700

ttattaagaa caaaatgaag gcattttatg ctccagtaca tgcagatgac ttgagagaag
2760

gtgcacagta tttgatgcag gctgctggtc ttggtcgtat gaagccaaac acacttgtcc
2820

ttggatttaa gaaagattgg ttgcaagcag atatgaggga tgtggatatg tatataaact
2880

tatttcatga tgcttttgac atacaatatg gagtagtggt tattcgccta aaagaaggtc
2940

tggatatatc tcatcttcaa ggacaagaag aattattgtc atcacaagag aaatctcctg
3000

gcaccaagga tgtggtagta agtgtggaat atagtaaaaa gtccgattta gatacttcca
3060

aaccactcag tgaaaaacca attacacaca aagttgagga agaggatggc aagactgcaa
3120

ctcaaccact gttgaaaaaa gaatccaaag gccctattgt gcctttaaat gtagctgacc
3180

aaaagcttct tgaagctagt acacagtttc agaaaaaaca aggaaagaat actattgatg
3240

tctggtggct ttttgatgat ggaggtttga ccttattgat accttacctt ctgacgacca
3300

agaaaaaatg gaaagactgt aagatcagag tattcattgg tggaaagata aacagaatag
3360

accatgaccg gagagcgatg gctactttgc ttagcaagtt ccggatagac ttttctgata
3420

tcatggttct aggagatatc aataccaaac caaagaaaga aaatattata gcttttgagg
3480

aaatcattga gccatacaga cttcatgaag atgataaaga gcaagatatt gcagataaaa
3540

tgaaagaaga tgaaccatgg cgaataacag ataatgagct tgaactttat aagaccaaga
3600

cataccggca gatcaggtta aatgagttat taaaggaaca ttcaagcaca gctaatatta
3660

ttgtcatgag tctcccagtt gcacgaaaag gtgctgtgtc tagtgctctc tacatggcat
3720

ggttagaagc tctatctaag gacctaccac caatcctcct agttcgtggg aatcatcaga
3780

gtgtccttac cttctattca taaatgttct atacagtgga cagccctcca gaatggtact
3840

tcagtgccta gtgtagtaac ctgaaatctt caatgacaca ttaacatcac aatggcgaat
3900

ggtgactttt ctttcacgat ttcattaatt tgaaagcaca caggaaagct tgctccattg
3960

ataacgtgta tggagacttc ggttttagtc aattccatat ctcaatctta atggtgattc
4020

ttctctgttg aactgaagtt tgtgagagta gttttccttt gctacttgaa tagcaataaa
4080

agcgtgttaa ctttttgg
4098

<210> 20

<211> 3120

<212> DNA

<213> Homo sapiens

<400> 20

aaaggaaaca caagttgctt ttgataacac atgatgcaaa gaaagaatta gaaagaatga 60

gcaatgaagc cggtataaat gacaaacaag tgtccaaagg cccaagaagt tactaccaaa
120

agctttcaaa caatattggt ttatctttaa agacacatcc atagcatact ttaaaaataa
180

ggaacttgaa caaggagaac cacaagaaaa actaaatctt agaggctgcg aagttgtgcc
240

cgatgtaaat gtagcaggaa gaaaatttgg aatcaagtta ctaatccctg ttgccgatgg
300

tatgaatgaa atgtatttga gatgtgacca tgagaatcaa tacgcccaat ggatggctgc
360

ctgcatgttg gcatcgaagg gcaaaaccat ggcagacagc tcctaccagc cagaggtcct
420

caacatcctt tcatttctga ggatgaaaaa caggaactct gcatctcagg tggcttccag
480

tctcgaaaac atggatatga acccagaatg ttttgtgtca ccacggtgtg caaaaagaca
540

caaatccaaa cagctggccg cccggatcct ggaggcgcac cagaacgtgg cccagatgcc
600

cctggtcgaa gccaagctgc ggttcatcca ggcgtggcag tcactgcctg agtttggcct
660

cacctactac cttgtcagat ttaaaggaag caaaaaagat gacattctgg gagtttcata
720

taacaggttg attaaaattg atgcagccac cgggattcca gtgacaacat ggagattcac
780

aaatatcaaa cagtggaatg taaactggga aacccggcag gtggtcatcg agtttgacca
840

aaacgtcttt actgctttca cctgcctgag tgcagattgc aagattgtgc acgagtacat
900

tggcggctac attttcttgt ccacccgctc caaggaccag aatgaaacac tcgatgagga
960

cttgttccac aaattgaccg gcggtcagga ttgaaacaag cacgcgtgct cggctcacac
1020

caacaaggca agccaaaggc gcccctcccc agagggatcc ctaacgtgcc cagcatgtag
1080

attctggact aacagacaac atacattcac cgctggtcac ccagatcctc attcaaaccc
1140

actgctggca catccctttc cttactttgc cctgtgctac cagccacgga aggagcctct
1200

cttgtttttt ctataaaatg ggtaggcagg agaaaagcag gtgccctaag attgctctaa
1260

ggcccagcat gtggttacag ttctctgact tgcagaacct gccaggtgta tggctacaag
1320

ttatcctcgt gctgatctgt ctcattacta agtcaatgga gaagacagaa aggtaaaaat
1380

cacgtgtagc aagaacaact cttatttcac aaactcaggt atgaaacgaa acgcctgtcc
1440

ttcatggaac tgcttttagc tcctgtcttt tcaaaatggc agagggagtt cctacacaca
1500

ctttttccct ggaggccaag gtctaggggt agaaagggga ggggtggggc taccaggtag
1560

cagttgacaa cccaaggtca gaggagtggc cctcagtgtc atctgtccac agtgatacct
1620

gccaagatga ccactgaccc acatctggtc ttagtcattg gtctcctcag atttctgggg
1680

ccacctgcaa gccccattcc attcctacag atctctcagc cacctgtaag tcctttgtga
1740

agatgtgggt gacacagggg gacaggaaaa cccatttctc aacccagatc catgtctcca
1800

ctgcttctac tctgggttgg gattcaggaa gacaggcaca gtcctctctg ttcatagaaa
1860

cacctgccag tgtcaaggat tccagtcagg tgtctatccc aactggtcag ggagagaagg
1920

gcagacccat tctcaaagac caccatgttc aaggtctgac agctccccac tggctgcccc
1980

cacaggggct ttaggctggt ctgggtcatg gggaagcgtc cctcttatcg ctggtctgtg
2040

ttctcctgga tttggtatct atgttggtac gactcctggc cttttatcta aaggactttg
2100

gcttttgtaa atcacaagcc aataatagac ttttttctcc ccctctgttt tttgctgtgt
2160

catctctgcc ttgagactgc cttgagacag tgcttgccct gagagagtga gccaattaac
2220

agctgcctga attgtcattt tccattttgg tttgttagag gtgggagggg tgggttttga
2280

gaaggtcaaa agcaatacca gaagtaaagg gaaatatcag acaatatttt attatttttt
2340

catagatgtt ctgccacaca aagaacttgg ggtgtaagga taaggcaaaa gctccaatcc
2400

catttttcag ttctcctagg atgcacccct cagggagcct ggccagagtt ccgaggcccg
2460

tgagcgtcag ctgttgcttt attttccatc aaagccctct gagaagtgag acctcagcaa
2520

ttccgggagc cacatagaga cagacttggc aagggacccc ctggttctga gccagtagct
2580

gccatctgga aattcctctt ttagcctctc cttagaggtg aatgtgaatg aagcctccca
2640

ggcacccgct gaatttctga ggccttgctt aaagctcaga agtggtttag gcatttggaa
2700

aatctggttc acatcataaa gaacttgatt tgaaatgttt tctatagaaa caagtgctaa
2760

gtgtaccgta ttatacttga tgttggtcat ttctcagtcc tatttctcag ttctattatt
2820

ttagaaccta gtcagttctt taagattata actggtccta cattaaaata atgcttctcg
2880

atgtcagatt ttacctgttt gctgctgaga acatctctgc ctaatttacc aaagccagac
2940

cttcagttca acatgcttcc ttagcttttc atagttgtct gacatttcca tgaaaacaaa
3000

ggaaccaact ttgttttaac caaactttgt ttggttacag ttttcagggg agcgtttctt
3060

ccatgacaca cagcaacatc ccaaagaaat aaacaagtgt gacaaaaaaa aaaaaaaaaa
3120

<210> 21

<211> 1337

<212> DNA

<213> Homo sapiens

<400> 21

ctggcgtccc ctttccggcc ggtccccatg gaggcgctgg ggaagctgaa gcagttcgat 60

gcctacccca agactttgga ggacttccgg gtcaagacct gcgggggcgc caccgtgacc
120

attgtcagtg gccttctcat gctgctactg ttcctgtccg agctgcagta ttacctcacc
180

acggaggtgc atcctgagct ctacgtggac aagtcgcggg gagataaact gaagatcaac
240

atcgatgtac tttttccgca catgccttgt gcctatctga gtattgatgc catggatgtg
300

gccggagaac agcagctgga tgtggaacac aacctgttca agcaacgact agataaagat
360

ggcatccccg tgagctcaga ggctgagcgg catgagcttg ggaaagtcga ggtgacggtg
420

tttgaccctg actccctgga ccctgatcgc tgtgagagct gctatggtgc tgaggcagaa
480

gatatcaagt gctgtaacac ctgtgaagat gtgcgggagg catatcgccg tagaggctgg
540

gccttcaaga acccagatac tattgagcag tgccggcgag agggcttcag ccagaagatg
600

caggagcaga agaatgaagg ctgccaggtg tatggcttct tggaagtcaa taaggtggcc
660

ggaaacttcc actttgcccc tgggaagagc ttccagcagt cccatgtgca cgtccatgac
720

ttgcagagct ttggccttga caacatcaac atgacccact acatccagca cctgtcattt
780

ggggaggact atccaggcat tgtgaacccc ctggaccaca ccaatgtcac tgcgccccaa
840

gcctccatga tgttccagta ctttgtgaag gtggtgccca ctgtgtacat gaaggtggac
900

ggagaggtac tgaggacaaa tcagttctct gtgaccagac atgagaaggt tgccaatggg
960

ctgttgggcg accaaggcct tcccggagtc ttcgtcctct atgagctctc gcccatgatg
1020

gtgaagctga cggagaagca caggtccttc acccacttcc tgacaggtgt gtgcgccatc
1080

attgggggca tgttcacagt ggctggactc atcgattcgc tcatctacca ctcagcacga
1140

gccatccaga agaaaattga tctagggaag acaacgtagt caccctcggt gcttcctctg
1200

tctcctcttt ctccctggcc tgtggttgtc ccccagcctc tgccaccctc cacctcctcg
1260

gtcagcccca gccccaggtt gataaatcta ttgattgatt gtgatagtaa aaaaaaaaaa
1320

aaaaaaaaaa aaaaaaa
1337

<210> 22

<211> 2908

<212> DNA

<213> Homo sapiens

<400> 22

ctgctcgcgg cgccgcctcc tgctcctccc gctgctgctg ccgctgccgc cctgagtcac 60

tgcctgcgca gctccggccg cctggctccc catactagtc gccgatattt ggagttctta
120

caacatggca gacattgaca acaaagaaca gtctgaactt gatcaagatt tggatgatgt
180

tgaagaagta gaagaagagg aaactggtga agaaacaaaa ctcaaagcac gtcagctaac
240

tgttcagatg atgcaaaatc ctcagattct tgcagccctt caagaaagac ttgatggtct
300

ggtagaaaca ccaacaggat acattgaaag cctgcctagg gtagttaaaa gacgagtgaa
360

tgctctcaaa aacctgcaag ttaaatgtgc acagatagaa gccaaattct atgaggaagt
420

tcacgatctt gaaaggaagt atgctgttct ctatcagcct ctatttgata agcgatttga
480

aattattaat gcaatttatg aacctacgga agaagaatgt gaatggaaac cagatgaaga
540

agatgagatt tcggaggaat tgaaagaaaa ggccaagatt gaagatgaga aaaaggatga
600

agaaaaagaa gaccccaaag gaattcctga attttggtta actgttttta agaatgttga
660

cttgctcagt gatatggttc aggaacacga tgaacctatt ctgaagcact tgaaagatat
720

taaagtgaag ttctcagatg ctggccagcc tatgagtttt gtcttagaat ttcactttga
780

acccaatgaa tattttacaa atgaagtgct gacaaagaca tacaggatga ggtcagaacc
840

agatgattct gatccctttt cttttgatgg accagaaatt atgggttgta cagggtgcca
900

gatagattgg aaaaaaggaa agaatgtcac tttgaaaact attaagaaga agcagaaaca
960

caagggacgt gggacagttc gtactgtgac taaaacagtt tccaatgact ctttctttaa
1020

cttttttgcc cctcctgaag ttcctgagag tggagatctg gatgatgatg ctgaagctat
1080

ccttgctgca gacttcgaaa ttggtcactt tttacgtgag cgtataatcc caagatcagt
1140

gttatatttt actggagaag ctattgaaga tgatgatgat gattatgatg aagaaggtga
1200

agaagcggat gaggaagggg aagaagaagg agatgaggaa aatgatccag actatgaccc
1260

aaagaaggat caaaacccag cagagtgcaa gcagcagtga agcaggatgt atgtggcctt
1320

gaggataacc tgcactggtc taccttctgc ttccctggaa aggatgaatt tacatcattt
1380

gacaagccta ttttcaagtt atttgttgtt tgtttgcttg tttttgtttt tgcagctaaa
1440

ataaaaattt caaatacaat tttagttctt acaagataat gtcttaattt tgtaccaatt
1500

caggtagaag tagaggccta ccttgaatta agggttatac tcagttttta acacattgtt
1560

gaagaaaagg taccagcttt ggaacgagat gctatactaa taagcaagtg taaaaaaaaa
1620

aaaaaaagag gaagaaaatc ttaagtgatt gatgctgttt tcttttaaaa aaaaaaaaaa
1680

aaattcattt tctttgggtt agagctagag agaaggcccc aagcttctat ggtttcttct
1740

aattcttatt gcttaaagta tgagtatgtc acttacccgt gcttctgttt actgtgtaat
1800

taaaatgggt agtactgttt acctaactac ctcatggatg tgttaaggca tattgagtta
1860

aatctcatat aatgtttctc aatcttgtta aaagctcaaa attttgggcc tatttgtaat
1920

gccagtgtga cactaagcat tttgttcaca ccacgctttg ataactaaac tggaaaacaa
1980

aggtgttaag tacctctgtt ctggatctgg gcagtcagca ctctttttag atctttgtgt
2040

ggctcctatt tttatagaag tggagggatg cactatttca caaggtccaa gatttgtttt
2100

cagatatttt tgatgactgt attgtaaata ctacagggat agcactatag tattgtagtc
2160

atgagactta aagtggaaat aagactattt ttgacaaaag atgccattaa atttcagact
2220

gtagagccac atttacaata cctcaggcta attactgtta attttggggt tgaacttttt
2280

ttgacagtga gggtggatta ttggattgtc attagaggaa ggtctagatt tcctgctctt
2340

aataaaatta cattgaattg atttttagag gtaatgaaaa cttcctttct gagaagttag
2400

tgttaaggtc ttggaatgtg aacacattgt ttgtagtgct atccattcct ctcctgagat
2460

tttaacttac tactggaaat ccttaaccaa ttataatagc tttttttctt tattttcaaa
2520

atgatttcct ttgctttgat tagacactat gtgctttttt tttttaacca tagttcatcg
2580

aaatgcagct ttttctgaac ttcaaagata gaatcccatt tttaatgaac tgaagtagca
2640

aaatcatctt tttcattctt taggaaatag ctattgccaa agtgaaggtg tagataatac
2700

ctagtcttgt tacataaagg ggatgtggtt tgcagaagaa ttttctttat aaaattgaag
2760

ttttaaggga cgtcagtgtt tatgccattt ttccagttcc aaaatgattc cattccattc
2820

tagaaatttg aagtatgtaa cctgaaatcc ttaataaaat ttggatttaa ttttataaaa
2880

aaaaaaaaaa aaaaaaaaaa aaaaaaaa
2908

<210> 23

<211> 3213

<212> DNA

<213> Homo sapiens

<400> 23

agagactcaa gatgattccc tttttaccca tgttttctct actattgctg cttattgtta 60

accctataaa cgccaacaat cattatgaca agatcttggc tcatagtcgt atcaggggtc
120

gggaccaagg cccaaatgtc tgtgcccttc aacagatttt gggcaccaaa aagaaatact
180

tcagcacttg taagaactgg tataaaaagt ccatctgtgg acagaaaacg actgttttat
240

atgaatgttg ccctggttat atgagaatgg aaggaatgaa aggctgccca gcagttttgc
300

ccattgacca tgtttatggc actctgggca tcgtgggagc caccacaacg cagcgctatt
360

ctgacgcctc aaaactgagg gaggagatcg agggaaaggg atccttcact tactttgcac
420

cgagtaatga ggcttgggac aacttggatt ctgatatccg tagaggtttg gagagcaacg
480

tgaatgttga attactgaat gctttacata gtcacatgat taataagaga atgttgacca
540

aggacttaaa aaatggcatg attattcctt caatgtataa caatttgggg cttttcatta
600

accattatcc taatggggtt gtcactgtta attgtgctcg aatcatccat gggaaccaga
660

ttgcaacaaa tggtgttgtc catgtcattg accgtgtgct tacacaaatt ggtacctcaa
720

ttcaagactt cattgaagca gaagatgacc tttcatcttt tagagcagct gccatcacat
780

cggacatatt ggaggccctt ggaagagacg gtcacttcac actctttgct cccaccaatg
840

aggcttttga gaaacttcca cgaggtgtcc tagaaaggtt catgggagac aaagtggctt
900

ccgaagctct tatgaagtac cacatcttaa atactctcca gtgttctgag tctattatgg
960

gaggagcagt ctttgagacg ctggaaggaa atacaattga gataggatgt gacggtgaca
1020

gtataacagt aaatggaatc aaaatggtga acaaaaagga tattgtgaca aataatggtg
1080

tgatccattt gattgatcag gtcctaattc ctgattctgc caaacaagtt attgagctgg
1140

ctggaaaaca gcaaaccacc ttcacggatc ttgtggccca attaggcttg gcatctgctc
1200

tgaggccaga tggagaatac actttgctgg cacctgtgaa taatgcattt tctgatgata
1260

ctctcagcat ggttcagcgc ctccttaaat taattctgca gaatcacata ttgaaagtaa
1320

aagttggcct taatgagctt tacaacgggc aaatactgga aaccatcgga ggcaaacagc
1380

tcagagtctt cgtatatcgt acagctgtct gcattgaaaa ttcatgcatg gagaaaggga
1440

gtaagcaagg gagaaacggt gcgattcaca tattccgcga gatcatcaag ccagcagaga
1500

aatccctcca tgaaaagtta aaacaagata agcgctttag caccttcctc agcctacttg
1560

aagctgcaga cttgaaagag ctcctgacac aacctggaga ctggacatta tttgtgccaa
1620

ccaatgatgc ttttaaggga atgactagtg aagaaaaaga aattctgata cgggacaaaa
1680

atgctcttca aaacatcatt ctttatcacc tgacaccagg agttttcatt ggaaaaggat
1740

ttgaacctgg tgttactaac attttaaaga ccacacaagg aagcaaaatc tttctgaaag
1800

aagtaaatga tacacttctg gtgaatgaat tgaaatcaaa agaatctgac atcatgacaa
1860

caaatggtgt aattcatgtt gtagataaac tcctctatcc agcagacaca cctgttggaa
1920

atgatcaact gctggaaata cttaataaat taatcaaata catccaaatt aagtttgttc
1980

gtggtagcac cttcaaagaa atccccgtga ctgtctatac aactaaaatt ataaccaaag
2040

ttgtggaacc aaaaattaaa gtgattgaag gcagtcttca gcctattatc aaaactgaag
2100

gacccacact aacaaaagtc aaaattgaag gtgaacctga attcagactg attaaagaag
2160

gtgaaacaat aactgaagtg atccatggag agccaattat taaaaaatac accaaaatca
2220

ttgatggagt gcctgtggaa ataactgaaa aagagacacg agaagaacga atcattacag
2280

gtcctgaaat aaaatacact aggatttcta ctggaggtgg agaaacagaa gaaactctga
2340

agaaattgtt acaagaagag gtcaccaagg tcaccaaatt cattgaaggt ggtgatggtc
2400

atttatttga agatgaagaa attaaaagac tgcttcaggg agacacaccc gtgaggaagt
2460

tgcaagccaa caaaaaagtt caaggttcta gaagacgatt aagggaaggt cgttctcagt
2520

gaaaatccaa aaaccagaaa aaaatgttta tacaacccta agtcaataac ctgaccttag
2580

aaaattgtga gagccaagtt gacttcagga actgaaacat cagcacaaag aagcaatcat
2640

caaataattc tgaacacaaa tttaatattt ttttttctga atgagaaaca tgagggaaat
2700

tgtggagtta gcctcctgtg gtaaaggaat tgaagaaaat ataacacctt acaccctttt
2760

tcatcttgac attaaaagtt ctggctaact ttggaatcca ttagagaaaa atccttgtca
2820

ccagattcat tacaattcaa atcgaagagt tgtgaactgt tatcccattg aaaagaccga
2880

gccttgtatg tatgttatgg atacataaaa tgcacgcaag ccattatctc tccatgggaa
2940

gctaagttat aaaaataggt gcttggtgta caaaactttt tatatcaaaa ggctttgcac
3000

atttctatat gagtgggttt actggtaaat tatgttattt tttacaacta attttgtact
3060

ctcagaatgt ttgtcatatg cttcttgcaa tgcatatttt ttaatctcaa acgtttcaat
3120

aaaaccattt ttcagatata aagagaatta cttcaaattg agtaattcag aaaaactcaa
3180

gatttaagtt aaaaagtggt ttggacttgg gaa
3213

<210> 24

<211> 10558

<212> DNA

<213> Homo sapiens

<400> 24
   cagtttggag ctcagtcttc caccaaaggc cgttcagttc tcctgggctc cagcctcctg 60
   caaggactgc aagagttttc ctccgcagct ctgagtctcc acttttttgg tggagaaagg
   120
   ctgcaaaaag aaaaagagac gcagtgagtg ggaaaagtat gcatcctatt caaacctaat
   180
   tgaatcgagg agcccaggga cacacgcctt caggtttgct caggggttca tatttggtgc
   240
   ttagacaaat tcaaaatgag gaaacatcgg cacttgccct tagtggccgt cttttgcctc
   300
   tttctctcag gctttcctac aactcatgcc cagcagcagc aagcagatgt caaaaatggt
   360
   gcggctgctg atataatatt tctagtggat tcctcttgga ccattggaga ggaacatttc
   420
   caacttgttc gagagtttct atatgatgtt gtaaaatcct tagctgtggg agaaaatgat
   480
   ttccattttg ctctggtcca gttcaacgga aacccacata ccgagttcct gttaaatacg
   540
   tatcgtacta aacaagaagt cctttctcat atttccaaca tgtcttatat tgggggaacc
   600
   aatcagactg gaaaaggatt agaatacata atgcaaagcc acctcaccaa ggctgctgga
   660
   agccgggccg gtgacggagt ccctcaggtt atcgtagtgt taactgatgg acactcgaag
   720
   gatggccttg ctctgccctc agcggaactt aagtctgctg atgttaacgt gtttgcaatt
   780
   ggagttgagg atgcagatga aggagcgtta aaagaaatag caagtgaacc gctcaatatg
   840
   catatgttca acctagagaa ttttacctca cttcatgaca tagtaggaaa cttagtgtcc
   900
   tgtgtgcatt catccgtgag tccagaaagg gctggggaca cggaaaccct taaagacatc
   960
   acagcacaag actctgctga cattattttc cttattgatg gatcaaacaa caccggaagt
   1020
   gtcaatttcg cagtcattct cgacttcctt gtaaatctcc ttgagaaact cccaattgga
   1080
   actcagcaga tccgagtggg ggtggtccag tttagcgatg agcccagaac catgttttcc
   1140
   ttggacacct actccaccaa ggcccaggtt ctgggtgcag tgaaagccct cgggtttgct
   1200
   ggtggggagt tggccaatat cggcctcgcc cttgatttcg tggtggagaa ccacttcacc
   1260
   cgggcagggg gcagccgcgt ggaggaaggg gttccccagg tgctggtcct cataagtgcc
   1320
   gggccttcta gtgacgagat tcgctacggg gtggtagcac tgaagcaggc tagcgtgttc
   1380
   tcattcggcc ttggagccca ggccgcctcc agggcagagc ttcagcacat agctaccgat
   1440
   gacaacttgg tgtttactgt cccggaattc cgtagctttg gggacctcca ggagaaatta
   1500
   ctgccgtaca ttgttggcgt ggcccaaagg cacattgtct tgaaaccgcc aaccattgtc
   1560
   acacaagtca ttgaagtcaa caagagagac atagtcttcc tggtggatgg ctcatctgca
   1620
   ctgggactgg ccaacttcaa tgccatccga gacttcattg ctaaagtcat ccagaggctg
   1680
   gaaatcggac aggatcttat ccaggtggca gtggcccagt atgcagacac tgtgaggcct
   1740
   gaattttatt tcaataccca tccaacaaaa agggaagtca taaccgctgt gcggaaaatg
   1800
   aagcccctgg acggctcggc cctgtacacg ggctctgctc tagactttgt tcgtaacaac
   1860
   ctattcacga gttcagccgg ctaccgggct gccgagggga ttcctaagct tttggtgctg
   1920
   atcacaggtg gtaagtccct agatgaaatc agccagcctg cccaggagct gaagagaagc
   1980
   agcataatgg cctttgccat tgggaacaag ggtgccgatc aggctgagct ggaagagatc
   2040
   gctttcgact cctccctggt gttcatccca gctgagttcc gagccgcccc attgcaaggc
   2100
   atgctgcctg gcttgctggc acctctcagg accctctctg gaacccctga agttcactca
   2160
   aacaaaagag atatcatctt tcttttggat ggatcagcca acgttggaaa aaccaatttc
   2220
   ccttatgtgc gcgactttgt aatgaaccta gttaacagcc ttgatattgg aaatgacaat
   2280
   attcgtgttg gtttagtgca atttagtgac actcctgtaa cggagttctc tttaaacaca
   2340
   taccagacca agtcagatat ccttggtcat ctgaggcagc tgcagctcca gggaggttcg
   2400
   ggcctgaaca caggctcagc cctaagctat gtctatgcca accacttcac ggaagctggc
   2460
   ggcagcagga tccgtgaaca cgtgccgcag ctcctgcttc tgctcacagc tgggcagtct
   2520
   gaggactcct atttgcaagc tgccaacgcc ttgacacgcg cgggcatcct gactttttgt
   2580
   gtgggagcta gccaggcgaa taaggcagag cttgagcaga ttgcttttaa cccaagcctg
   2640
   gtgtatctca tggatgattt cagctccctg ccagctttgc ctcagcagct gattcagccc
   2700
   ctaaccacat atgttagtgg aggtgtggag gaagtaccac tcgctcagcc agagagcaag
   2760
   cgagacattc tgttcctctt tgacggctca gccaatcttg tgggccagtt ccctgttgtc
   2820
   cgtgactttc tctacaagat tatcgatgag ctcaatgtga agccagaggg gacccgaatt
   2880
   gcggtggctc agtacagcga tgatgtcaag gtggagtccc gttttgatga gcaccagagt
   2940
   aagcctgaga tcctgaatct tgtgaagaga atgaagatca agacgggcaa agccctcaac
   3000
   ctgggctacg cgctggacta tgcacagagg tacatttttg tgaagtctgc tggcagccgg
   3060
   atcgaggatg gagtgcttca gttcctggtg ctgctggtcg caggaaggtc atctgaccgt
   3120
   gtggatgggc cagcaagtaa cctgaagcag agtggggttg tgcctttcat cttccaagcc
   3180
   aagaacgcag accctgctga gttagagcag atcgtgctgt ctccagcgtt tatcctggct
   3240
   gcagagtcgc ttcccaagat tggagatctt catccacaga tagtgaatct cttaaaatca
   3300
   gtgcacaacg gagcaccagc accagtttca ggtgaaaagg acgtggtgtt tctgcttgat
   3360
   ggctctgagg gcgtcaggag cggcttccct ctgttgaaag agtttgtcca gagagtggtg
   3420
   gaaagcctgg atgtgggcca ggaccgggtc cgcgtggccg tggtgcagta cagcgaccgg
   3480
   accaggcccg agttctacct gaattcatac atgaacaagc aggacgtcgt caacgctgtc
   3540
   cgccagctga ccctgctggg agggccgacc cccaacaccg gggccgccct ggagtttgtc
   3600
   ctgaggaaca tcctggtcag ctctgcggga agcaggataa cagaaggtgt gccccagctg
   3660
   ctgatcgtcc tcacggccga caggtctggg gatgatgtgc ggaacccctc cgtggtcgtg
   3720
   aagaggggtg gggctgtgcc cattggcatt ggcatcggga acgctgacat cacagagatg
   3780
   cagaccatct ccttcatccc ggactttgcc gtggccattc ccacctttcg ccagctgggg
   3840
   accgtccaac aggtcatctc tgagagggtg acccagctca cccgcgagga gctgagcagg
   3900
   ctgcagccgg tgttgcagcc tctaccgagc ccaggtgttg gtggcaagag ggacgtggtc
   3960
   tttctcatcg atgggtccca aagtgccggg cctgagttcc agtacgttcg caccctcata
   4020
   gagaggctgg ttgactacct ggacgtgggc tttgacacca cccgggtggc tgtcatccag
   4080
   ttcagcgatg accccaaggc ggagttcctg ctgaacgccc attccagcaa ggatgaagtg
   4140
   cagaacgcgg tgcagcggct gaggcccaag ggagggcggc agatcaacgt gggcaatgcc
   4200
   ctggagtacg tgtccaggaa catcttcaag aggcccctgg ggagccgcat tgaagagggc
   4260
   gtcccacagt tcctggtcct catctcgtct ggaaagtctg acgatgaggt ggtcgtcccg
   4320
   gcggtggagc tcaagcagtt tggcgtggcc cctttcacga tcgccaggaa cgcagaccag
   4380
   gaggagctgg tgaagatctc gctgagcccc gaatatgtgt tctcggtgag caccttccgg
   4440
   gagctgccca gcctggagca gaaactgctg acgcccatca cgaccctgac ctcagagcag
   4500
   atccagaagc tcttagccag cactcgctat ccacctccag cagttgagag tgatgctgca
   4560
   gacattgtct ttctgatcga cagctctgag ggagttaggc cagatggctt tgcacatatt
   4620
   cgagattttg ttagcaggat tgttcgaaga ctcaacatcg gccccagtaa agtgagagtt
   4680
   ggggtcgtgc agttcagcaa tgatgtcttc ccagaattct atctgaaaac ctacagatcc
   4740
   caggccccgg tgctggacgc catacggcgc ctgaggctca gaggggggtc cccactgaac
   4800
   actggcaagg ctctcgaatt tgtggcaaga aacctctttg ttaagtctgc ggggagtcgc
   4860
   atagaagacg gggtgcccca acacctggtc ctggtcctgg gtggaaaatc ccaggacgat
   4920
   gtgtccaggt tcgcccaggt gatccgttcc tcgggcattg tgagtttagg ggtaggagac
   4980
   cggaacatcg acagaacaga gctgcagacc atcaccaatg accccagact ggtcttcaca
   5040
   gtgcgagagt tcagagagct tcccaacata gaagaaagaa tcatgaactc gtttggaccc
   5100
   tccgcagcca ctcctgcacc tccaggggtg gacacccctc ctccttcacg gccagagaag
   5160
   aagaaagcag acattgtgtt cctgttggat ggttccatca acttcaggag ggacagtttc
   5220
   caggaagtgc ttcgttttgt gtctgaaata gtggacacag tttatgaaga tggcgactcc
   5280
   atccaagtgg ggcttgtcca gtacaactct gaccccactg acgaattctt cctgaaggac
   5340
   ttctctacca agaggcagat tattgacgcc atcaacaaag tggtctacaa agggggaaga
   5400
   cacgccaaca ctaaggtggg ccttgagcac ctgcgggtaa accactttgt gcctgaggca
   5460
   ggcagccgcc tggaccagcg ggtccctcag attgcctttg tgatcacggg aggaaagtcg
   5520
   gtggaagatg cacaggatgt gagcctggcc ctcacccaga ggggggtcaa agtgtttgct
   5580
   gttggagtga ggaatatcga ctcggaggag gttggaaaga tagcgtccaa cagcgccaca
   5640
   gcgttccgcg tgggcaacgt ccaggagctg tccgaactga gcgagcaagt tttggaaact
   5700
   ttgcatgatg cgatgcatga aaccctttgc cctggtgtaa ctgatgctgc caaagcttgt
   5760
   aatctggatg tgattctggg gtttgatggt tctagagacc agaatgtttt tgtggcccag
   5820
   aagggcttcg agtccaaggt ggacgccatc ttgaacagaa tcagccagat gcacagggtc
   5880
   agctgcagcg gtggccgctc gcccaccgtg cgtgtgtcag tggtggccaa cacgccctcg
   5940
   ggcccggtgg aggcctttga ctttgacgag taccagccag agatgctcga gaagttccgg
   6000
   aacatgcgca gccagcaccc ctacgtcctc acggaggaca ccctgaaggt ctacctgaac
   6060
   aagttcagac agtcctcgcc ggacagcgtg aaggtggtca ttcattttac tgatggagca
   6120
   gacggagatc tggctgattt acacagagca tctgagaacc tccgccaaga aggagtccgt
   6180
   gccttgatcc tggtgggcct tgaacgagtg gtcaacttgg agcggctaat gcatctggag
   6240
   tttgggcgag ggtttatgta tgacaggccc ctgaggctta acttgctgga cttggattat
   6300
   gaactagcgg agcagcttga caacattgcc gagaaagctt gctgtggggt tccctgcaag
   6360
   tgctctgggc agaggggaga ccgcgggccc atcggcagca tcgggccaaa gggtattcct
   6420
   ggagaagacg gctaccgagg ctatcctggt gatgagggtg gacccggtga gcgtggtccg
   6480
   cctggtgtga acggcactca aggtttccag ggctgcccgg gccagagagg agtaaagggc
   6540
   tctcggggat tcccaggaga gaagggcgaa gtaggagaaa ttggactgga tggtctggat
   6600
   ggtgaagatg gagacaaagg attgcctggt tcttctggag agaaagggaa tcctggaaga
   6660
   aggggtgata aaggacctcg aggagagaaa ggagaaagag gagatgttgg gattcgaggg
   6720
   gacccgggta acccaggaca agacagccag gagagaggac ccaaaggaga aaccggtgac
   6780
   ctcggcccca tgggtgtccc agggagagat ggagtacctg gaggacctgg agaaactggg
   6840
   aagaatggtg gctttggccg aaggggaccc cccggagcta agggcaacaa gggcggtcct
   6900
   ggccagccgg gctttgaggg agagcagggg accagaggtg cacagggccc agctggtcct
   6960
   gctggtcctc cagggctgat aggagaacaa ggcatttctg gacctagggg aagcggaggt
   7020
   gcccgtggcg ctcctggaga acgaggcaga accggtccac tgggaagaaa gggtgagccc
   7080
   ggagagccag gaccaaaagg aggaatcggg aacccgggcc ctcgtgggga gacgggagat
   7140
   gacgggagag acggagttgg cagtgaagga cgcagaggca aaaaaggaga aagaggattt
   7200
   cctggatacc caggaccaaa gggtaaccca ggtgaacctg ggctaaatgg aacaacagga
   7260
   cccaaaggca tcagaggccg aaggggaaat tcgggacctc cagggatagt tggacagaag
   7320
   gggagacctg gctacccagg accagctggt ccaaggggca acaggggcga ctccatcgat
   7380
   caatgtgccc tcatccaaag catcaaagat aaatgccctt gctgttacgg gcccctggag
   7440
   tgccccgtct tcccaacaga actagccttt gctttagaca cctctgaggg agtcaaccaa
   7500
   gacactttcg gccggatgcg agatgtggtc ttgagtattg tgaatgtcct gaccattgct
   7560
   gagagcaact gcccgacggg ggcccgggtg gctgtggtca cctacaacaa cgaggtgacc
   7620
   acggagatcc ggtttgctga ctccaagagg aagtcggtcc tcctggacaa gattaagaac
   7680
   cttcaggtgg ctctgacatc caaacagcag agtctggaga ctgccatgtc gtttgtggcc
   7740
   aggaacacat ttaagcgtgt gaggaacgga ttcctaatga ggaaagtggc tgttttcttc
   7800
   agcaacacac ccacaagagc atccccacag ctcagagagg ctgtgctcaa actctcagat
   7860
   gcggggatca cccccttgtt ccttacaagg caggaagacc ggcagctcat caacgctttg
   7920
   cagatcaata acacagcagt ggggcatgcg cttgtcctgc ctgcagggag agacctcaca
   7980
   gacttcctgg agaatgtcct cacgtgtcat gtttgcttgg acatctgcaa catcgaccca
   8040
   tcctgtggat ttggcagttg gaggccttcc ttcagggaca ggagagcggc agggagtgat
   8100
   gtggacatcg acatggcttt catcttagac agcgctgaga ccaccaccct gttccagttc
   8160
   aatgagatga agaagtacat agcgtacctg gtcagacaac tggacatgag cccagatccc
   8220
   aaggcctccc agcacttcgc cagagtggca gttgtgcagc acgcgccctc tgagtccgtg
   8280
   gacaatgcca gcatgccacc tgtgaaggtg gaattctccc tgactgacta tggctccaag
   8340
   gagaagctgg tggacttcct cagcagggga atgacacagt tgcagggaac cagggcctta
   8400
   ggcagtgcca ttgaatacac catagagaat gtctttgaaa gtgccccaaa cccacgggac
   8460
   ctgaaaattg tggtcctgat gctgacgggc gaggtgccgg agcagcagct ggaggaggcc
   8520
   cagagagtca tcctgcaggc caaatgcaag ggctacttct tcgtggtcct gggcattggc
   8580
   aggaaggtga acatcaagga ggtatacacc ttcgccagtg agccaaacga cgtcttcttc
   8640
   aaattagtgg acaagtccac cgagctcaac gaggagcctt tgatgcgctt cgggaggctg
   8700
   ttgccgtcct tcgtcagcag tgaaaatgct ttttacttgt ccccagatat caggaaacag
   8760
   tgtgattggt tccaagggga ccaacccaca aagaaccttg tgaagtttgg tcacaaacaa
   8820
   gtaaatgttc cgaataacgt tacttcaagt cctacatcca acccagtgac gacaacgaag
   8880
   ccggtgacta cgacgaagcc ggtgaccacc acaacaaagc ctgtaaccac cacaacaaag
   8940
   cctgtgacta ttataaatca gccatctgtg aagccagccg ctgcaaagcc ggcccctgcg
   9000
   aaacctgtgg ctgccaagcc tgtggccaca aagacggcca ctgttagacc cccagtggcg
   9060
   gtgaagccag caacagcagc gaagcctgta gcagcaaagc cagcagctgt aagacccccc
   9120
   gctgctgctg caaaaccagt ggcgaccaag cctgaggtcc ctaggccaca ggcagccaaa
   9180
   ccagctgcca ccaagccagc caccactaag cccgtggtta agatgctccg tgaagtccag
   9240
   gtgtttgaga taacagagaa cagcgccaaa ctccactggg agaggcctga gccccccggt
   9300
   ccttattttt atgacctcac cgtcacctca gcccatgatc agtccctggt tctgaagcag
   9360
   aacctcacgg tcacggaccg cgtcattgga ggcctgctcg ctgggcagac ataccatgtg
   9420
   gctgtggtct gctacctgag gtctcaggtc agagccacct accacggaag tttcagtaca
   9480
   aagaaatctc agcccccacc tccacagcca gcaaggtcag cttctagttc aaccatcaat
   9540
   ctaatggtga gcacagaacc attggctctc actgaaacag atatatgcaa gttgccgaaa
   9600
   gacgaaggaa cttgcaggga tttcatatta aaatggtact atgatccaaa caccaaaagc
   9660
   tgtgcaagat tctggtatgg aggttgtggt ggaaacgaaa acaaatttgg atcacagaaa
   9720
   gaatgtgaaa aggtttgcgc tcctgtgctc gccaaacccg gagtcatcag tgtgatggga
   9780
   acctaagcgt gggtggccaa catcatatac ctcttgaaga agaaggagtc agccatcgcc
   9840
   aacttgtctc tgtagaagct ccgggtgtag attcccttgc actgtatcat ttcatgcttt
   9900
   gatttacact cgaactcggg agggaacatc ctgctgcatg acctatcagt atggtgctaa
   9960
   tgtgtctgtg gaccctcgct ctctgtctcc agcagttctc tcgaatactt tgaatgttgt
   10020
   gtaacagtta gccactgctg gtgtttatgt gaacattcct atcaatccaa attccctctg
   10080
   gagtttcatg ttatgcctgt tgcaggcaaa tgtaaagtct agaaaataat gcaaatgtca
   10140
   cggctactct atatactttt gcttggttca ttttttttcc cttttagtta agcatgactt
   10200
   tagatgggaa gcctgtgtat cgtggagaaa caagagacca actttttcat tccctgcccc
   10260
   caatttccca gactagattt caagctaatt ttctttttct gaagcctcta acaaatgatc
   10320
   tagttcagaa ggaagcaaaa tcccttaatc tatgtgcacc gttgggacca atgccttaat
   10380
   taaagaattt aaaaaagttg taatagagaa tatttttggc attcctctca atgttgtgtg
   10440
   tttttttttt ttgtgtgctg gagggagggg atttaatttt aattttaaaa tgtttaggaa
   10500
   atttatacaa agaaactttt taataaagta tattgaaagt ttaaaaaaaa aaaaaaaa
   10558

<210> 25

<211> 2133

<212> DNA

<213> Homo sapiens

<400> 25

cgggagagcg cgctctgcct gccgcctgcc tgcctgccac tgagggttcc cagcaccatg 60

agggcctgga tcttctttct cctttgcctg gccgggaggg ccttggcagc ccctcagcaa
120

gaagccctgc ctgatgagac agaggtggtg gaagaaactg tggcagaggt gactgaggta
180

tctgtgggag ctaatcctgt ccaggtggaa gtaggagaat ttgatgatgg tgcagaggaa
240

accgaagagg aggtggtggc ggaaaatccc tgccagaacc accactgcaa acacggcaag
300

gtgtgcgagc tggatgagaa caacaccccc atgtgcgtgt gccaggaccc caccagctgc
360

gccccaacgt gtgtgctgtg cagaaggtta ttggcactaa taggaagtac ttcaccaact
240
gcaagcagtg gtaccaaagg aaaatctgtg gcaaatcaac agtcatcagc tacgagtgct
300
gtcctggata tgaaaaggtc cctggggaga agggctgtcc agcagcccta ccactctcaa
360
acctttacga gaccctggga gtcgttggat ccaccaccac tcagctgtac acggaccgca
420
cggagaagct gaggcctgag atggaggggc ccggcagctt caccatcttc gcccctagca
480
acgaggcctg ggcctccttg ccagctgaag tgctggactc cctggtcagc aatgtcaaca
540
ttgagctgct caatgccctc cgctaccata tggtgggcag gcgagtcctg actgatgagc
600
tgaaacacgg catgaccctc acctctatgt accagaattc caacatccag atccaccact
660
atcctaatgg gattgtaact gtgaactgtg cccggctcct gaaagccgac caccatgcaa
720
ccaacggggt ggtgcacctc atcgataagg tcatctccac catcaccaac aacatccagc
780
agatcattga gatcgaggac acctttgaga cccttcgggc tgctgtggct gcatcagggc
840
tcaacacgat gcttgaaggt aacggccagt acacgctttt ggccccgacc aatgaggcct
900
tcgagaagat ccctagtgag actttgaacc gtatcctggg cgacccagaa gccctgagag
960
acctgctgaa caaccacatc ttgaagtcag ctatgtgtgc tgaagccatc gttgcggggc
1020
tgtctgtaga gaccctggag ggcacgacac tggaggtggg ctgcagcggg gacatgctca
1080
ctatcaacgg gaaggcgatc atctccaata aagacatcct agccaccaac ggggtgatcc
1140
actacattga tgagctactc atcccagact cagccaagac actatttgaa ttggctgcag
1200
agtctgatgt gtccacagcc attgaccttt tcagacaagc cggcctcggc aatcatctct
1260
ctggaagtga gcggttgacc ctcctggctc ccctgaattc tgtattcaaa gatggaaccc
1320

ctccaattga tgcccataca aggaatttgc ttcggaacca cataattaaa gaccagctgg
1380

cctctaagta tctgtaccat ggacagaccc tggaaactct gggcggcaaa aaactgagag
1440

tttttgttta tcgtaatagc ctctgcattg agaacagctg catcgcggcc cacgacaaga
1500

gggggaggta cgggaccctg ttcacgatgg accgggtgct gaccccccca atggggactg
1560

tcatggatgt cctgaaggga gacaatcgct ttagcatgct ggtagctgcc atccagtctg
1620

caggactgac ggagaccctc aaccgggaag gagtctacac agtctttgct cccacaaatg
1680

aagccttccg agccctgcca ccaagagaac ggagcagact cttgggagat gccaaggaac
1740

ttgccaacat cctgaaatac cacattggtg atgaaatcct ggttagcgga ggcatcgggg
1800

ccctggtgcg gctaaagtct ctccaaggtg acaagctgga agtcagcttg aaaaacaatg
1860

tggtgagtgt caacaaggag cctgttgccg agcctgacat catggccaca aatggcgtgg
1920

tccatgtcat caccaatgtt ctgcagcctc cagccaacag acctcaggaa agaggggatg
1980

aacttgcaga ctctgcgctt gagatcttca aacaagcatc agcgttttcc agggcttccc
2040

agaggtctgt gcgactagcc cctgtctatc aaaagttatt agagaggatg aagcattagc
2100

ttgaagcact acaggaggaa tgcaccacgg cagctctccg ccaatttctc tcagatttcc
2160

acagagactg tttgaatgtt ttcaaaacca agtatcacac tttaatgtac atgggccgca
2220

ccataatgag atgtgagcct tgtgcatgtg ggggaggagg gagagagatg tactttttaa
2280

atcatgttcc ccctaaacat ggctgttaac ccactgcatg cagaaacttg gatgtcactg
2340

cctgacattc acttccagag aggacctatc ccaaatgtgg aattgactgc ctatgccaag
2400

tccctggaaa aggagcttca gtattgtggg gctcataaaa catgaatcaa gcaatccagc
2460

ctcatgggaa gtcctggcac agtttttgta aagcccttgc acagctggag aaatggcatc
2520

attataagct atgagttgaa atgttctgtc aaatgtgtct cacatctaca cgtggcttgg
2580

aggcttttat ggggccctgt ccaggtagaa aagaaatggt atgtagagct tagatttccc
2640

tattgtgaca gagccatggt gtgtttgtaa taataaaacc aaagaaacat a
2691

<210> 27

<211> 8027

<212> DNA

<213> Homo sapiens

<400> 27
   acgcccgcgc cggctgtgct gcacaggggg aggagaggga accccaggcg cgagcgggaa 60
   gaggggacct gcagccacaa cttctctggt cctctgcatc ccttctgtcc ctccacccgt
   120
   ccccttcccc accctctggc ccccaccttc ttggaggcga caacccccgg gaggcattag
   180
   aagggatttt tcccgcagtt gcgaagggaa gcaaacttgg tggcaacttg cctcccggtg
   240
   cgggcgtctc tcccccaccg tctcaacatg cttaggggtc cggggcccgg gctgctgctg
   300
   ctggccgtcc agtgcctggg gacagcggtg ccctccacgg gagcctcgaa gagcaagagg
   360
   caggctcagc aaatggttca gccccagtcc ccggtggctg tcagtcaaag caagcccggt
   420
   tgttatgaca atggaaaaca ctatcagata aatcaacagt gggagcggac ctacctaggc
   480
   aatgcgttgg tttgtacttg ttatggagga agccgaggtt ttaactgcga gagtaaacct
   540
   gaagctgaag agacttgctt tgacaagtac actgggaaca cttaccgagt gggtgacact
   600
   tatgagcgtc ctaaagactc catgatctgg gactgtacct gcatcggggc tgggcgaggg
   660
   agaataagct gtaccatcgc aaaccgctgc catgaagggg gtcagtccta caagattggt
   720
   gacacctgga ggagaccaca tgagactggt ggttacatgt tagagtgtgt gtgtcttggt
   780
   aatggaaaag gagaatggac ctgcaagccc atagctgaga agtgttttga tcatgctgct
   840
   gggacttcct atgtggtcgg agaaacgtgg gagaagccct accaaggctg gatgatggta
   900
   gattgtactt gcctgggaga aggcagcgga cgcatcactt gcacttctag aaatagatgc
   960
   aacgatcagg acacaaggac atcctataga attggagaca cctggagcaa gaaggataat
   1020
   cgaggaaacc tgctccagtg catctgcaca ggcaacggcc gaggagagtg gaagtgtgag
   1080
   aggcacacct ctgtgcagac cacatcgagc ggatctggcc ccttcaccga tgttcgtgca
   1140
   gctgtttacc aaccgcagcc tcacccccag cctcctccct atggccactg tgtcacagac
   1200
   agtggtgtgg tctactctgt ggggatgcag tggctgaaga cacaaggaaa taagcaaatg
   1260
   ctttgcacgt gcctgggcaa cggagtcagc tgccaagaga cagctgtaac ccagacttac
   1320
   ggtggcaact caaatggaga gccatgtgtc ttaccattca cctacaatgg caggacgttc
   1380
   tactcctgca ccacggaagg gcgacaggac ggacatcttt ggtgcagcac aacttcgaat
   1440
   tatgagcagg accagaaata ctctttctgc acagaccaca ctgttttggt tcagactcga
   1500
   ggaggaaatt ccaatggtgc cttgtgccac ttccccttcc tatacaacaa ccacaattac
   1560
   actgattgca cttctgaggg cagaagagac aacatgaagt ggtgtgggac cacacagaac
   1620
   tatgatgccg accagaagtt tgggttctgc cccatggctg cccacgagga aatctgcaca
   1680
   accaatgaag gggtcatgta ccgcattgga gatcagtggg ataagcagca tgacatgggt
   1740
   cacatgatga ggtgcacgtg tgttgggaat ggtcgtgggg aatggacatg cattgcctac
   1800
   tcgcagcttc gagatcagtg cattgttgat gacatcactt acaatgtgaa cgacacattc
   1860
   cacaagcgtc atgaagaggg gcacatgctg aactgtacat gcttcggtca gggtcggggc
   1920
   aggtggaagt gtgatcccgt cgaccaatgc caggattcag agactgggac gttttatcaa
   1980
   attggagatt catgggagaa gtatgtgcat ggtgtcagat accagtgcta ctgctatggc
   2040
   cgtggcattg gggagtggca ttgccaacct ttacagacct atccaagctc aagtggtcct
   2100
   gtcgaagtat ttatcactga gactccgagt cagcccaact cccaccccat ccagtggaat
   2160
   gcaccacagc catctcacat ttccaagtac attctcaggt ggagacctaa aaattctgta
   2220
   ggccgttgga aggaagctac cataccaggc cacttaaact cctacaccat caaaggcctg
   2280
   aagcctggtg tggtatacga gggccagctc atcagcatcc agcagtacgg ccaccaagaa
   2340
   gtgactcgct ttgacttcac caccaccagc accagcacac ctgtgaccag caacaccgtg
   2400
   acaggagaga cgactccctt ttctcctctt gtggccactt ctgaatctgt gaccgaaatc
   2460
   acagccagta gctttgtggt ctcctgggtc tcagcttccg acaccgtgtc gggattccgg
   2520
   gtggaatatg agctgagtga ggagggagat gagccacagt acctggatct tccaagcaca
   2580
   gccacttctg tgaacatccc tgacctgctt cctggccgaa aatacattgt aaatgtctat
   2640
   cagatatctg aggatgggga gcagagtttg atcctgtcta cttcacaaac aacagcgcct
   2700
   gatgcccctc ctgacccgac tgtggaccaa gttgatgaca cctcaattgt tgttcgctgg
   2760
   agcagacccc aggctcccat cacagggtac agaatagtct attcgccatc agtagaaggt
   2820
   agcagcacag aactcaacct tcctgaaact gcaaactccg tcaccctcag tgacttgcaa
   2880
   cctggtgttc agtataacat cactatctat gctgtggaag aaaatcaaga aagtacacct
   2940
   gttgtcattc aacaagaaac cactggcacc ccacgctcag atacagtgcc ctctcccagg
   3000
   gacctgcagt ttgtggaagt gacagacgtg aaggtcacca tcatgtggac accgcctgag
   3060
   agtgcagtga ccggctaccg tgtggatgtg atccccgtca acctgcctgg cgagcacggg
   3120
   cagaggctgc ccatcagcag gaacaccttt gcagaagtca ccgggctgtc ccctggggtc
   3180
   acctattact tcaaagtctt tgcagtgagc catgggaggg agagcaagcc tctgactgct
   3240
   caacagacaa ccaaactgga tgctcccact aacctccagt ttgtcaatga aactgattct
   3300
   actgtcctgg tgagatggac tccacctcgg gcccagataa caggataccg actgaccgtg
   3360
   ggccttaccc gaagaggcca gcccaggcag tacaatgtgg gtccctctgt ctccaagtac
   3420
   cccctgagga atctgcagcc tgcatctgag tacaccgtat ccctcgtggc cataaagggc
   3480
   aaccaagaga gccccaaagc cactggagtc tttaccacac tgcagcctgg gagctctatt
   3540
   ccaccttaca acaccgaggt gactgagacc accatcgtga tcacatggac gcctgctcca
   3600
   agaattggtt ttaagctggg tgtacgacca agccagggag gagaggcacc acgagaagtg
   3660
   acttcagact caggaagcat cgttgtgtcc ggcttgactc caggagtaga atacgtctac
   3720
   accatccaag tcctgagaga tggacaggaa agagatgcgc caattgtaaa caaagtggtg
   3780
   acaccattgt ctccaccaac aaacttgcat ctggaggcaa accctgacac tggagtgctc
   3840
   acagtctcct gggagaggag caccacccca gacattactg gttatagaat taccacaacc
   3900
   cctacaaacg gccagcaggg aaattctttg gaagaagtgg tccatgctga tcagagctcc
   3960
   tgcacttttg ataacctgag tcccggcctg gagtacaatg tcagtgttta cactgtcaag
   4020
   gatgacaagg aaagtgtccc tatctctgat accatcatcc cagctgttcc tcctcccact
   4080
   gacctgcgat tcaccaacat tggtccagac accatgcgtg tcacctgggc tccaccccca
   4140
   tccattgatt taaccaactt cctggtgcgt tactcacctg tgaaaaatga ggaagatgtt
   4200
   gcagagttgt caatttctcc ttcagacaat gcagtggtct taacaaatct cctgcctggt
   4260
   acagaatatg tagtgagtgt ctccagtgtc tacgaacaac atgagagcac acctcttaga
   4320
   ggaagacaga aaacaggtct tgattcccca actggcattg acttttctga tattactgcc
   4380
   aactctttta ctgtgcactg gattgctcct cgagccacca tcactggcta caggatccgc
   4440
   catcatcccg agcacttcag tgggagacct cgagaagatc gggtgcccca ctctcggaat
   4500
   tccatcaccc tcaccaacct cactccaggc acagagtatg tggtcagcat cgttgctctt
   4560
   aatggcagag aggaaagtcc cttattgatt ggccaacaat caacagtttc tgatgttccg
   4620
   agggacctgg aagttgttgc tgcgaccccc accagcctac tgatcagctg ggatgctcct
   4680
   gctgtcacag tgagatatta caggatcact tacggagaaa caggaggaaa tagccctgtc
   4740
   caggagttca ctgtgcctgg gagcaagtct acagctacca tcagcggcct taaacctgga
   4800
   gttgattata ccatcactgt gtatgctgtc actggccgtg gagacagccc cgcaagcagc
   4860
   aagccaattt ccattaatta ccgaacagaa attgacaaac catcccagat gcaagtgacc
   4920
   gatgttcagg acaacagcat tagtgtcaag tggctgcctt caagttcccc tgttactggt
   4980
   tacagagtaa ccaccactcc caaaaatgga ccaggaccaa caaaaactaa aactgcaggt
   5040
   ccagatcaaa cagaaatgac tattgaaggc ttgcagccca cagtggagta tgtggttagt
   5100
   gtctatgctc agaatccaag cggagagagt cagcctctgg ttcagactgc agtaaccaac
   5160
   attgatcgcc ctaaaggact ggcattcact gatgtggatg tcgattccat caaaattgct
   5220
   tgggaaagcc cacaggggca agtttccagg tacagggtga cctactcgag ccctgaggat
   5280
   ggaatccatg agctattccc tgcacctgat ggtgaagaag acactgcaga gctgcaaggc
   5340
   ctcagaccgg gttctgagta cacagtcagt gtggttgcct tgcacgatga tatggagagc
   5400
   cagcccctga ttggaaccca gtccacagct attcctgcac caactgacct gaagttcact
   5460
   caggtcacac ccacaagcct gagcgcccag tggacaccac ccaatgttca gctcactgga
   5520
   tatcgagtgc gggtgacccc caaggagaag accggaccaa tgaaagaaat caaccttgct
   5580
   cctgacagct catccgtggt tgtatcagga cttatggtgg ccaccaaata tgaagtgagt
   5640
   gtctatgctc ttaaggacac tttgacaagc agaccagctc agggtgttgt caccactctg
   5700
   gagaatgtca gcccaccaag aagggctcgt gtgacagatg ctactgagac caccatcacc
   5760
   attagctgga gaaccaagac tgagacgatc actggcttcc aagttgatgc cgttccagcc
   5820
   aatggccaga ctccaatcca gagaaccatc aagccagatg tcagaagcta caccatcaca
   5880
   ggtttacaac caggcactga ctacaagatc tacctgtaca ccttgaatga caatgctcgg
   5940
   agctcccctg tggtcatcga cgcctccact gccattgatg caccatccaa cctgcgtttc
   6000
   ctggccacca cacccaattc cttgctggta tcatggcagc cgccacgtgc caggattacc
   6060
   ggctacatca tcaagtatga gaagcctggg tctcctccca gagaagtggt ccctcggccc
   6120
   cgccctggtg tcacagaggc tactattact ggcctggaac cgggaaccga atatacaatt
   6180
   tatgtcattg ccctgaagaa taatcagaag agcgagcccc tgattggaag gaaaaagaca
   6240
   gacgagcttc cccaactggt aacccttcca caccccaatc ttcatggacc agagatcttg
   6300
   gatgttcctt ccacagttca aaagacccct ttcgtcaccc accctgggta tgacactgga
   6360
   aatggtattc agcttcctgg cacttctggt cagcaaccca gtgttgggca acaaatgatc
   6420
   tttgaggaac atggttttag gcggaccaca ccgcccacaa cggccacccc cataaggcat
   6480
   aggccaagac catacccgcc gaatgtagga caagaagctc tctctcagac aaccatctca
   6540
   tgggccccat tccaggacac ttctgagtac atcatttcat gtcatcctgt tggcactgat
   6600
   gaagaaccct tacagttcag ggttcctgga acttctacca gtgccactct gacaggcctc
   6660
   accagaggtg ccacctacaa catcatagtg gaggcactga aagaccagca gaggcataag
   6720
   gttcgggaag aggttgttac cgtgggcaac tctgtcaacg aaggcttgaa ccaacctacg
   6780
   gatgactcgt gctttgaccc ctacacagtt tcccattatg ccgttggaga tgagtgggaa
   6840
   cgaatgtctg aatcaggctt taaactgttg tgccagtgct taggctttgg aagtggtcat
   6900
   ttcagatgtg attcatctag atggtgccat gacaatggtg tgaactacaa gattggagag
   6960
   aagtgggacc gtcagggaga aaatggccag atgatgagct gcacatgtct tgggaacgga
   7020
   aaaggagaat tcaagtgtga ccctcatgag gcaacgtgtt acgatgatgg gaagacatac
   7080
   cacgtaggag aacagtggca gaaggaatat ctcggtgcca tttgctcctg cacatgcttt
   7140
   ggaggccagc ggggctggcg ctgtgacaac tgccgcagac ctgggggtga acccagtccc
   7200
   gaaggcacta ctggccagtc ctacaaccag tattctcaga gataccatca gagaacaaac
   7260
   actaatgtta attgcccaat tgagtgcttc atgcctttag atgtacaggc tgacagagaa
   7320
   gattcccgag agtaaatcat ctttccaatc cagaggaaca agcatgtctc tctgccaaga
   7380
   tccatctaaa ctggagtgat gttagcagac ccagcttaga gttcttcttt ctttcttaag
   7440
   ccctttgctc tggaggaagt tctccagctt cagctcaact cacagcttct ccaagcatca
   7500
   ccctgggagt ttcctgaggg ttttctcata aatgagggct gcacattgcc tgttctgctt
   7560
   cgaagtattc aataccgctc agtattttaa atgaagtgat tctaagattt ggtttgggat
   7620
   caataggaaa gcatatgcag ccaaccaaga tgcaaatgtt ttgaaatgat atgaccaaaa
   7680
   ttttaagtag gaaagtcacc caaacacttc tgctttcact taagtgtctg gcccgcaata
   7740
   ctgtaggaac aagcatgatc ttgttactgt gatattttaa atatccacag tactcacttt
   7800
   ttccaaatga tcctagtaat tgcctagaaa tatctttctc ttacctgtta tttatcaatt
   7860
   tttcccagta tttttatacg gaaaaaattg tattgaaaac acttagtatg cagttgataa
   7920
   gaggaatttg gtataattat ggtgggtgat tattttttat actgtatgtg ccaaagcttt
   7980
   actactgtgg aaagacaact gttttaataa aagatttaca ttccaca
   8027

<210> 28

<211> 5084

<212> DNA

<213> Homo sapiens

<400> 28

agcaccacgg cagcaggagg tttcggctaa gttggaggta ctggccacga ctgcatgccc 60

gcgcccgcca ggtgatacct ccgccggtga cccaggggct ctgcgacaca aggagtctgc
120

atgtctaagt gctagacatg ctcagctttg tggatacgcg gactttgttg ctgcttgcag
180

taaccttatg cctagcaaca tgccaatctt tacaagagga aactgtaaga aagggcccag
240

ccggagatag aggaccacgt ggagaaaggg gtccaccagg ccccccaggc agagatggtg
300

aagatggtcc cacaggccct cctggtccac ctggtcctcc tggcccccct ggtctcggtg
360

ggaactttgc tgctcagtat gatggaaaag gagttggact tggccctgga ccaatgggct
420

taatgggacc tagaggccca cctggtgcag ctggagcccc aggccctcaa ggtttccaag
480

gacctgctgg tgagcctggt gaacctggtc aaactggtcc tgcaggtgct cgtggtccag
540

ctggccctcc tggcaaggct ggtgaagatg gtcaccctgg aaaacccgga cgacctggtg
600

agagaggagt tgttggacca cagggtgctc gtggtttccc tggaactcct ggacttcctg
660

gcttcaaagg cattagggga cacaatggtc tggatggatt gaagggacag cccggtgctc
720

ctggtgtgaa gggtgaacct ggtgcccctg gtgaaaatgg aactccaggt caaacaggag
780

cccgtgggct tcctggtgag agaggacgtg ttggtgcccc tggcccagct ggtgcccgtg
840

gcagtgatgg aagtgtgggt cccgtgggtc ctgctggtcc cattgggtct gctggccctc
900

caggcttccc aggtgcccct ggccccaagg gtgaaattgg agctgttggt aacgctggtc
960

ctgctggtcc cgccggtccc cgtggtgaag tgggtcttcc aggcctctcc ggccccgttg
1020

gacctcctgg taatcctgga gcaaacggcc ttactggtgc caagggtgct gctggccttc
1080

ccggcgttgc tggggctccc ggcctccctg gaccccgcgg tattcctggc cctgttggtg
1140

ctgccggtgc tactggtgcc agaggacttg ttggtgagcc tggtccagct ggctccaaag
1200

gagagagcgg taacaagggt gagcccggct ctgctgggcc ccaaggtcct cctggtccca
1260

gtggtgaaga aggaaagaga ggccctaatg gggaagctgg atctgccggc cctccaggac
1320

ctcctgggct gagaggtagt cctggttctc gtggtcttcc tggagctgat ggcagagctg
1380

gcgtcatggg ccctcctggt agtcgtggtg caagtggccc tgctggagtc cgaggaccta
1440

atggagatgc tggtcgccct ggggagcctg gtctcatggg acccagaggt cttcctggtt
1500

cccctggaaa tatcggcccc gctggaaaag aaggtcctgt cggcctccct ggcatcgacg
1560

gcaggcctgg cccaattggc ccagctggag caagaggaga gcctggcaac attggattcc
1620

ctggacccaa aggccccact ggtgatcctg gcaaaaacgg tgataaaggt catgctggtc
1680

ttgctggtgc tcggggtgct ccaggtcctg atggaaacaa tggtgctcag ggacctcctg
1740

gaccacaggg tgttcaaggt ggaaaaggtg aacagggtcc cgctggtcct ccaggcttcc
1800

agggtctgcc tggcccctca ggtcccgctg gtgaagttgg caaaccagga gaaaggggtc
1860

tccatggtga gtttggtctc cctggtcctg ctggtccaag aggggaacgc ggtcccccag
1920

gtgagagtgg tgctgccggt cctactggtc ctattggaag ccgaggtcct tctggacccc
1980

cagggcctga tggaaacaag ggtgaacctg gtgtggttgg tgctgtgggc actgctggtc
2040

catctggtcc tagtggactc ccaggagaga ggggtgctgc tggcatacct ggaggcaagg
2100

gagaaaaggg tgaacctggt ctcagaggtg aaattggtaa ccctggcaga gatggtgctc
2160

gtggtgctca tggtgctgta ggtgcccctg gtcctgctgg agccacaggt gaccggggcg
2220

aagctggggc tgctggtcct gctggtcctg ctggtcctcg gggaagccct ggtgaacgtg
2280

gcgaggtcgg tcctgctggc cccaacggat ttgctggtcc ggctggtgct gctggtcaac
2340

cgggtgctaa aggagaaaga ggagccaaag ggcctaaggg tgaaaacggt gttgttggtc
2400

ccacaggccc cgttggagct gctggcccag ctggtccaaa tggtcccccc ggtcctgctg
2460

gaagtcgtgg tgatggaggc ccccctggta tgactggttt ccctggtgct gctggacgga
2520

ctggtccccc aggaccctct ggtatttctg gccctcctgg tccccctggt cctgctggga
2580

aagaagggct tcgtggtcct cgtggtgacc aaggtccagt tggccgaact ggagaagtag
2640

gtgcagttgg tccccctggc ttcgctggtg agaagggtcc ctctggagag gctggtactg
2700

ctggacctcc tggcactcca ggtcctcagg gtcttcttgg tgctcctggt attctgggtc
2760

tccctggctc gagaggtgaa cgtggtctac ctggtgttgc tggtgctgtg ggtgaacctg
2820

gtcctcttgg cattgccggc cctcctgggg cccgtggtcc tcctggtgct gtgggtagtc
2880

ctggagtcaa cggtgctcct ggtgaagctg gtcgtgatgg caaccctggg aacgatggtc
2940

ccccaggtcg cgatggtcaa cccggacaca agggagagcg cggttaccct ggcaatattg
3000

gtcccgttgg tgctgcaggt gcacctggtc ctcatggccc cgtgggtcct gctggcaaac
3060

atggaaaccg tggtgaaact ggtccttctg gtcctgttgg tcctgctggt gctgttggcc
3120

caagaggtcc tagtggccca caaggcattc gtggcgataa gggagagccc ggtgaaaagg
3180

ggcccagagg tcttcctggc ttaaagggac acaatggatt gcaaggtctg cctggtatcg
3240

ctggtcacca tggtgatcaa ggtgctcctg gctccgtggg tcctgctggt cctaggggcc
3300

ctgctggtcc ttctggccct gctggaaaag atggtcgcac tggacatcct ggtacggttg
3360

gacctgctgg cattcgaggc cctcagggtc accaaggccc tgctggcccc cctggtcccc
3420

ctggccctcc tggacctcca ggtgtaagcg gtggtggtta tgactttggt tacgatggag
3480

acttctacag ggctgaccag cctcgctcag caccttctct cagacccaag gactatgaag
3540

ttgatgctac tctgaagtct ctcaacaacc agattgagac ccttcttact cctgaaggct
3600

ctagaaagaa cccagctcgc acatgccgtg acttgagact cagccaccca gagtggagca
3660

gtggttacta ctggattgac cctaaccaag gatgcactat ggatgctatc aaagtatact
3720

gtgatttctc tactggcgaa acctgtatcc gggcccaacc tgaaaacatc ccagccaaga
3780

actggtatag gagctccaag gacaagaaac acgtctggct aggagaaact atcaatgctg
3840

gcagccagtt tgaatataat gtagaaggag tgacttccaa ggaaatggct acccaacttg
3900

ccttcatgcg cctgctggcc aactatgcct ctcagaacat cacctaccac tgcaagaaca
3960

gcattgcata catggatgag gagactggca acctgaaaaa ggctgtcatt ctacagggct
4020

ctaatgatgt tgaacttgtt gctgagggca acagcaggtt cacttacact gttcttgtag
4080

atggctgctc taaaaagaca aatgaatggg gaaagacaat cattgaatac aaaacaaata
4140

agccatcacg cctgcccttc cttgatattg cacctttgga catcggtggt gctgaccatg
4200

aattctttgt ggacattggc ccagtctgtt tcaaataaat gaactcaatc taaattaaaa
4260

aagaaagaaa tttgaaaaaa ctttctcttt gccatttctt cttcttcttt tttaactgaa
4320

agctgaatcc ttccatttct tctgcacatc tacttgctta aattgtgggc aaaagagaaa
4380

aagaaggatt gatcagagca ttgtgcaata cagtttcatt aactccttcc cccgctcccc
4440

caaaaatttg aatttttttt tcaacactct tacacctgtt atggaaaatg tcaacctttg
4500

taagaaaacc aaaataaaaa ttgaaaaata aaaaccataa acatttgcac cacttgtggc
4560

ttttgaatat cttccacaga gggaagttta aaacccaaac ttccaaaggt ttaaactacc
4620

tcaaaacact ttcccatgag tgtgatccac attgttaggt gctgacctag acagagatga
4680

actgaggtcc ttgttttgtt ttgttcataa tacaaaggtg ctaattaata gtatttcaga
4740

tacttgaaga atgttgatgg tgctagaaga atttgagaag aaatactcct gtattgagtt
4800

gtatcgtgtg gtgtattttt taaaaaattt gatttagcat tcatattttc catcttattc
4860

ccaattaaaa gtatgcagat tatttgccca aagttgtcct cttcttcaga ttcagcattt
4920

gttctttgcc agtctcattt tcatcttctt ccatggttcc acagaagctt tgtttcttgg
4980

gcaagcagaa aaattaaatt gtacctattt tgtatatgtg agatgtttaa ataaattgtg
5040

aaaaaaatga aataaagcat gtttggtttt ccaaaagaac atat
5084

<210> 29

<211> 595

<212> DNA

<213> Homo sapiens

<400> 29

gggcaaggct gggccgggaa gggcgtgggt tgaggagagg ctccagaccc gcacgccgcg 60

cgcacagagc tctcagcgcc gctcccagcc acagcctccc gcgcctcgct cagctccaac
120

atggcaaaaa tctccagccc tacagagact gagcggtgca tcgagtccct gattgctgtc
180

ttccagaagt atgctggaaa ggatggttat aactacactc tctccaagac agagttccta
240

agcttcatga atacagaact agctgccttc acaaagaacc agaaggaccc tggtgtcctt
300

gaccgcatga tgaagaaact ggacaccaac agtgatggtc agctagattt ctcagaattt
360

cttaatctga ttggtggcct agctatggct tgccatgact ccttcctcaa ggctgtccct
420

tcccagaagc ggacctgagg accccttggc cctggccttc aaacccaccc cctttccttc
480

cagcctttct gtcatcatct ccacagccca cccatcccct gagcacacta accacctcat
540

gcaggcccca cctgccaata gtaataaagc aatgtcactt ttttaaaaca tgaaa
595

<210> 30

<211> 2116

<212> DNA

<213> Homo sapiens

<400> 30

gtgaaggccg gcgcgctcgc cggccgaggt gggatcccga ggcctctcca gtccgccgag 60

ggcgcaccac cggcccgtct cgcccgccgc gccggggagg tggagcacga gcgcacgtgt
120

taggacccga aagatggtga actatgcctg ggcagggcga agccagagga aactctggtg
180

gaggtccgta gcggtcctga cgtgcaaatc ggtcgtccga cctgggtata ggggcgggct
240

ccaggcgagg cggtcgacgc tcctgaaaac ttgcgcgcgc gctcgcgcca ctgcgcccgg
300

agcgatgaag atggtcgcgc cctggacgcg gttctactcc aacagctgct gcttgtgctg
360

ccatgtccgc accggcacca tcctgctcgg cgtctggtat ctgatcatca atgctgtggt
420

actgttgatt ttattgagtg ccctggctga tccggatcag tataactttt caagttctga
480

actgggaggt gactttgagt tcatggatga tgccaacatg tgcattgcca ttgcgatttc
540

tcttctcatg atcctgatat gtgctatggc tacttacgga gcgtacaagc aacgcgcagc
600

ctggatcatc ccattcttct gttaccagat ctttgacttt gccctgaaca tgttggttgc
660

aatcactgtg cttatttatc caaactccat tcaggaatac atacggcaac tgcctcctaa
720

ttttccctac agagatgatg tcatgtcagt gaatcctacc tgtttggtcc ttattattct
780

tctgtttatt agcattatct tgacttttaa gggttacttg attagctgtg tttggaactg
840

ctaccgatac atcaatggta ggaactcctc tgatgtcctg gtttatgtta ccagcaatga
900

cactacggtg ctgctacccc cgtatgatga tgccactgtg aatggtgctg ccaaggagcc
960

accgccacct tacgtgtctg cctaagcctt caagtgggcg gagctgaggg cagcagcttg
1020

actttgcaga catctgagca atagttctgt tatttcactt ttgccatgag cctctctgag
1080

cttgtttgtt gctgaaatgc tactttttaa aatttagatg ttagattgaa aactgtagtt
1140

ttcaacatat gctttgctag aacactgtga tagattaact gtagaattct tcctgtacga
1200

ttggggatat aatgggcttc actaaccttc cctaggcatt gaaacttccc ccaaatctga
1260

tggacctaga agtctgcttt tgtacctgct gggccccaaa gttgggcatt tttctctctg
1320

ttccctctct tttgaaaatg taaaataaaa ccaaaaatag acaacttttt cttcagccat
1380

tccagcatag agaacaaaac cttatggaaa caggaatgtc aattgtgtaa tcattgttct
1440

aattaggtaa atagaagtcc ttatgtatgt gttacaagaa tttcccccac aacatccttt
1500

atgactgaag ttcaatgaca gtttgtgttt gggtggtaaa ggattttctc catggcctga
1560

attaagacca ttagaaagca ccaggccgtg ggagcagtga ccatctgctg actgttcttg
1620

tggatcttgt gtccagggac atggggtgac atgcctcgta tgtgttagag ggtggaatgg
1680

atgtgtttgg cgctgcatgg gatctggtgc ccctcttctc ctggattcac atccccaccc
1740

agggcccgct tttactaagt gttctgccct agattggttc aaggaggtca tccaactgac
1800

tttatcaagt ggaattggga tatatttgat atacttctgc ctaacaacat ggaaaagggt
1860

tttcttttcc ctgcaagcta catcctactg ctttgaactt ccaagtatgt ctagtcacct
1920

tttaaaatgt aaacattttc agaaaaatga ggattgcctt ccttgtatgc gctttttacc
1980

ttgactacct gaattgcaag ggatttttat atattcatat gttacaaagt cagcaactct
2040

cctgttggtt cattattgaa tgtgctgtaa attaagttgt ttgcaattaa aacaaggttt
2100

gcccacaaaa aaaaaa
2116

<210> 31

<211> 3583

<212> DNA

<213> Homo sapiens

<400> 31

cgcggacccg gccggcccag gcccgcgccc gccgcggccc tgagaggccc cggcaggtcc 60

cggcccggcg gcggcagcca tggccggggg gccgggcccg ggggagcccg cagcccccgg
120

cgcccagcac ttcttgtacg aggtgccgcc ctgggtcatg tgccgcttct acaaagtgat
180

ggacgccctg gagcccgccg actggtgcca gttcgccgcc ctgatcgtgc gcgaccagac
240

cgagctgcgg ctgtgcgagc gctccgggca gcgcacggcc agcgtcctgt ggccctggat
300

caaccgcaac gcccgtgtgg ccgacctcgt gcacatcctc acgcacctgc agctgctccg
360

tgcgcgggac atcatcacag cctggcaccc tcccgccccg cttccgtccc caggcaccac
420

tgccccgagg cccagcagca tccctgcacc cgccgaggcc gaggcctgga gcccccggaa
480

gttgccatcc tcagcctcca ccttcctctc cccagctttt ccaggctccc agacccattc
540

agggcctgag ctcggcctgg ttccaagccc tgcttccctg tggcctccac cgccatctcc
600

agccccttct tctaccaagc caggcccaga gagctcagtg tccctcctgc agggagcccg
660

cccctctccg ttttgctggc ccctctgtga gatttcccgg ggcacccaca acttctcgga
720

ggagctcaag atcggggagg gtggctttgg gtgcgtgtac cgggcggtga tgaggaacac
780

ggtgtatgct gtgaagaggc tgaaggagaa cgctgacctg gagtggactg cagtgaagca
840

gagcttcctg accgaggtgg agcagctgtc caggtttcgt cacccaaaca ttgtggactt
900

tgctggctac tgtgctcaga acggcttcta ctgcctggtg tacggcttcc tgcccaacgg
960

ctccctggag gaccgtctcc actgccagac ccaggcctgc ccacctctct cctggcctca
1020

gcgactggac atccttctgg gtacagcccg ggcaattcag tttctacatc aggacagccc
1080

cagcctcatc catggagaca tcaagagttc caacgtcctt ctggatgaga ggctgacacc
1140

caagctggga gactttggcc tggcccggtt cagccgcttt gccgggtcca gccccagcca
1200

gagcagcatg gtggcccgga cacagacagt gcggggcacc ctggcctacc tgcccgagga
1260

gtacatcaag acgggaaggc tggctgtgga cacggacacc ttcagctttg gggtggtagt
1320

gctagagacc ttggctggtc agagggctgt gaagacgcac ggtgccagga ccaagtatct
1380

gaaagacctg gtggaagagg aggctgagga ggctggagtg gctttgagaa gcacccagag
1440

cacactgcaa gcaggtctgg ctgcagatgc ctgggctgct cccatcgcca tgcagatcta
1500

caagaagcac ctggacccca ggcccgggcc ctgcccacct gagctgggcc tgggcctggg
1560

ccagctggcc tgctgctgcc tgcaccgccg ggccaaaagg aggcctccta tgacccaggt
1620

gtacgagagg ctagagaagc tgcaggcagt ggtggcgggg gtgcccgggc atttggaggc
1680

cgccagctgc atcccccctt ccccgcagga gaactcctac gtgtccagca ctggcagagc
1740

ccacagtggg gctgctccat ggcagcccct ggcagcgcca tcaggagcca gtgcccaggc
1800

agcagagcag ctgcagagag gccccaacca gcccgtggag agtgacgaga gcctaggcgg
1860

cctctctgct gccctgcgct cctggcactt gactccaagc tgccctctgg acccagcacc
1920

cctcagggag gccggctgtc ctcaggggga cacggcagga gaatcgagct gggggagtgg
1980

cccaggatcc cggcccacag ccgtggaagg actggccctt ggcagctctg catcatcgtc
2040

gtcagagcca ccgcagatta tcatcaaccc tgcccgacag aagatggtcc agaagctggc
2100

cctgtacgag gatggggccc tggacagcct gcagctgctg tcgtccagct ccctcccagg
2160

cttgggcctg gaacaggaca ggcaggggcc cgaagaaagt gatgaatttc agagctgatg
2220

tgttcacctg ggcagatccc ccaaatccgg aagtcaaagt tctcatggtc agaagttctc
2280

atggtgcacg agtcctcagc actctgccgg cagtgggggt gggggcccat gcccgcgggg
2340

gagagaagga ggtggccctg ctgttctagg ctctgtgggc ataggcaggc agagtggaac
2400

cctgcctcca tgccagcatc tgggggcaag gaaggctggc atcatccagt gaggaggctg
2460

gcgcatgttg ggaggctgct ggctgcacag acccgtgagg ggaggagagg ggctgctgtg
2520

caggggtgtg gagtagggag ctggctcccc tgagagccat gcagggcgtc tgcagcccag
2580

gcctctggca gcagctcttt gcccatctct ttggacagtg gccaccctgc acaatggggc
2640

cgacgaggcc tagggccctc ctacctgctt acaatttgga aaagtgtggc cgggtgcggt
2700

ggctcacgcc tgtaatccca gcactttggg aggccaaggc aggaggatcg ctggagccca
2760

gtaggtcaag accagccagg gcaacatgat gagaccctgt ctctgccaaa aaatttttta
2820

aactattagc ctggcgtggt agcgcacgcc tgtggtccca gctgctgggg aggctgaagt
2880

aggaggatca tttatgcttg ggaggtcgag gctgcagtga gtcatgattg tatgactgca
2940

ctccagcctg ggtgacagag caagaccctg tttcaaaaag aaaaaccctg ggaaaagtga
3000

agtatggctg taagtctcat ggttcagtcc tagcaagaag cgagaattct gagatcctcc
3060

agaaagtcga gcagcaccca cctccaacct cgggccagtg tcttcaggct ttactgggga
3120

cctgcgagct ggcctaatgt ggtggcctgc aagccaggcc atccctgggc gccacagacg
3180

agctccgagc caggtcaggc ttcggaggcc acaagctcag cctcaggccc aggcactgat
3240

tgtggcagag gggccactac ccaaggtcta gctaggccca agacctagtt acccagacag
3300

tgagaagccc ctggaaggca gaaaagttgg gagcatggca gacagggaag ggaaacattt
3360

tcagggaaaa gacatgtatc acatgtcttc agaagcaagt caggtttcat gtaaccgagt
3420

gtcctcttgc gtgtccaaaa gtagcccagg gctgtagcac aggcttcaca gtgattttgt
3480

gttcagccgt gagtcacact acatgccccc gtgaagctgg gcattggtga cgtccaggtt
3540

gtccttgagt aataaaaacg tatgttccct aaaaaaaaaa aaa
3583

<210> 32

<211> 905

<212> DNA

<213> Homo sapiens

<400> 32

caacacaggg gcagtctcca ggacctccac accattaaca agatgagcct tgtgctccct 60

tgggctctag agaggaagcc cctctgagcc ctcagcccct ctttcctccc tctcctaaag
120

taatttgatc ctcaggaatt tgttctgccc tcatctggcc ctggccagct ctgcatttga
180

caaatgccag gaagaggaaa ctgttgagaa aacggaacta ctggggaaag ggagggctca
240

ctgagaacca tcccggtaac ccgaccgccg ctggtcacca tgaaccacat tgtgcaaacc
300

ttctctcctg tcaacagcgg ccagcctccc aactacgaga tgctcaagga ggagcaggaa
360

gtggctatgc tgggggggcc ccacaaccct gctcccccga cgtccaccgt gatccacatc
420

cgcagcgaga cctccgtgcc tgaccatgtc gtctggtccc tgttcaacac cctcttcatg
480

aacacctgct gcctgggctt catagcattc gcctactccg tgaagtctag ggacaggaag
540

atggttggcg acgtgaccgg ggcccaggcc tatgcctcca ccgccaagtg cctgaacatc
600

tgggccctga ttttgggcat cttcatgacc attctgctcg tcatcatccc agtgttggtc
660

gtccaggccc agcgatagat caggaggcat cattgaggcc aggagctctg cccgtgacct
720

gtatcccacg tactctatct tccattcctc gccctgcccc cagaggccag gagctctgcc
780

cttgacctgt attccactta ctccaccttc cattcctcgc cctgtcccca cagccgagtc
840

ctgcatcagc cctttatcct cacacgcttt tctacaatgg cattcaataa agtgtatatg
900

tttct
905

<210> 33

<211> 782

<212> DNA

<213> Homo sapiens

<400> 33

aggggcctta gcgtgccgca tcgccgagat ccagcgccca gagagacacc agagaaccca 60

ccatggcccc ctttgagccc ctggcttctg gcatcctgtt gttgctgtgg ctgatagccc
120

ccagcagggc ctgcacctgt gtcccacccc acccacagac ggccttctgc aattccgacc
180

tcgtcatcag ggccaagttc gtggggacac cagaagtcaa ccagaccacc ttataccagc
240

gttatgagat caagatgacc aagatgtata aagggttcca agccttaggg gatgccgctg
300

acatccggtt cgtctacacc cccgccatgg agagtgtctg cggatacttc cacaggtccc
360

acaaccgcag cgaggagttt ctcattgctg gaaaactgca ggatggactc ttgcacatca
420

ctacctgcag tttcgtggct ccctggaaca gcctgagctt agctcagcgc cggggcttca
480

ccaagaccta cactgttggc tgtgaggaat gcacagtgtt tccctgttta tccatcccct
540

gcaaactgca gagtggcact cattgcttgt ggacggacca gctcctccaa ggctctgaaa
600

agggcttcca gtcccgtcac cttgcctgcc tgcctcggga gccagggctg tgcacctggc
660

agtccctgcg gtcccagata gcctgaatcc tgcccggagt ggaactgaag cctgcacagt
720

gtccaccctg ttcccactcc catctttctt ccggacaatg aaataaagag ttaccaccca
780

gc
782

<210> 34

<211> 1124

<212> DNA

<213> Homo sapiens

<400> 34

gccgctgcca ccgcaccccg ccatggagcg gccgtcgctg cgcgccctgc tcctcggcgc 60

cgctgggctg ctgctcctgc tcctgcccct ctcctcttcc tcctcttcgg acacctgcgg
120

cccctgcgag ccggcctcct gcccgcccct gcccccgctg ggctgcctgc tgggcgagac
180

ccgcgacgcg tgcggctgct gccctatgtg cgcccgcggc gagggcgagc cgtgcggggg
240

tggcggcgcc ggcagggggt actgcgcgcc gggcatggag tgcgtgaaga gccgcaagag
300

gcggaagggt aaagccgggg cagcagccgg cggtccgggt gtaagcggcg tgtgcgtgtg
360

caagagccgc tacccggtgt gcggcagcga cggcaccacc tacccgagcg gctgccagct
420

gcgcgccgcc agccagaggg ccgagagccg cggggagaag gccatcaccc aggtcagcaa
480

gggcacctgc gagcaaggtc cttccatagt gacgcccccc aaggacatct ggaatgtcac
540

tggtgcccag gtgtacttga gctgtgaggt catcggaatc ccgacacctg tcctcatctg
600

gaacaaggta aaaaggggtc actatggagt tcaaaggaca gaactcctgc ctggtgaccg
660

ggacaacctg gccattcaga cccggggtgg cccagaaaag catgaagtaa ctggctgggt
720

gctggtatct cctctaagta aggaagatgc tggagaatat gagtgccatg catccaattc
780

ccaaggacag gcttcagcat cagcaaaaat tacagtggtt gatgccttac atgaaatacc
840

agtgaaaaaa ggtgaaggtg ccgagctata aacctccaga atattattag tctgcatggt
900

taaaagtagt catggataac tacattacct gttcttgcct aataagtttc ttttaatcca
960

atccactaac actttagtta tattcactgg ttttacacag agaaatacaa aataaagatc
1020

acacatcaag actatctaca aaaatttatt atatatttac agaagaaaag catgcatatc
1080

attaaacaaa taaaatactt tttatcacaa aaaaaaaaaa aaaa
1124

<210> 35

<211> 647

<212> DNA

<213> Homo sapiens

<400> 35

gctcactgag caccgtccca gcatccggac accacagcgg cccttcgctc cacgcagaaa 60

accacacttc tcataccttc actcaacact tccttcccca aagccagaag atgcacaagg
120

aggaacatga ggtggctgtg ctgggggcac cccccagcac catccttcca aggtccaccg
180

tgattaacat ccacagcgag acctccgtgc ccgaccatgt cgtctggtcc ctgttcaaca
240

ccctcttctt gaactggtgc tgtctgggct tcatagcatt cgcctactcc gtaaagtcta
300

gggacaggaa gatggttggc gacgtgaccg gggcccaggc ctatgcctcc accgccaagt
360

gcctgaacat ctgggccctg attctgggca tcctcatgac cattggattc atcctgttac
420

tggtattcgg ctctgtaaca gtctaccata ttatgttaca gataatacag gaaaaacggg
480

gttactagta gccgcccata gcctgcaacc tttgcactcc actgtgcaat gctggccctg
540

cacgctgggg ctgttgcccc tgcccccttg gtcctgcccc tagatacagc agtttatacc
600

cacacacctg tctacagtgt cattcaataa agtgcacgtg cttgtga
647

<210> 36

<211> 5489

<212> DNA

<213> Homo sapiens

<400> 36

ggctgagttt tatgacgggc ccggtgctga agggcaggga acaacttgat ggtgctactt 60

tgaactgctt ttcttttctc ctttttgcac aaagagtctc atgtctgata tttagacatg
120

atgagctttg tgcaaaaggg gagctggcta cttctcgctc tgcttcatcc cactattatt
180

ttggcacaac aggaagctgt tgaaggagga tgttcccatc ttggtcagtc ctatgcggat
240

agagatgtct ggaagccaga accatgccaa atatgtgtct gtgactcagg atccgttctc
300

tgcgatgaca taatatgtga cgatcaagaa ttagactgcc ccaacccaga aattccattt
360

ggagaatgtt gtgcagtttg cccacagcct ccaactgctc ctactcgccc tcctaatggt
420

caaggacctc aaggccccaa gggagatcca ggccctcctg gtattcctgg gagaaatggt
480

gaccctggta ttccaggaca accagggtcc cctggttctc ctggcccccc tggaatctgt
540

gaatcatgcc ctactggtcc tcagaactat tctccccagt atgattcata tgatgtcaag
600

tctggagtag cagtaggagg actcgcaggc tatcctggac cagctggccc cccaggccct
660

cccggtcccc ctggtacatc tggtcatcct ggttcccctg gatctccagg ataccaagga
720

ccccctggtg aacctgggca agctggtcct tcaggccctc caggacctcc tggtgctata
780

ggtccatctg gtcctgctgg aaaagatgga gaatcaggta gacccggacg acctggagag
840

cgaggattgc ctggacctcc aggtatcaaa ggtccagctg ggatacctgg attccctggt
900

atgaaaggac acagaggctt cgatggacga aatggagaaa agggtgaaac aggtgctcct
960

ggattaaagg gtgaaaatgg tcttccaggc gaaaatggag ctcctggacc catgggtcca
1020

agaggggctc ctggtgagcg aggacggcca ggacttcctg gggctgcagg tgctcggggt
1080

aatgacggtg ctcgaggcag tgatggtcaa ccaggccctc ctggtcctcc tggaactgcc
1140

ggattccctg gatcccctgg tgctaagggt gaagttggac ctgcagggtc tcctggttca
1200

aatggtgccc ctggacaaag aggagaacct ggacctcagg gacacgctgg tgctcaaggt
1260

cctcctggcc ctcctgggat taatggtagt cctggtggta aaggcgaaat gggtcccgct
1320

ggcattcctg gagctcctgg actgatggga gcccggggtc ctccaggacc agccggtgct
1380

aatggtgctc ctggactgcg aggtggtgca ggtgagcctg gtaagaatgg tgccaaagga
1440

gagcccggac cacgtggtga acgcggtgag gctggtattc caggtgttcc aggagctaaa
1500

ggcgaagatg gcaaggatgg atcacctgga gaacctggtg caaatgggct tccaggagct
1560

gcaggagaaa ggggtgcccc tgggttccga ggacctgctg gaccaaatgg catcccagga
1620

gaaaagggtc ctgctggaga gcgtggtgct ccaggccctg cagggcccag aggagctgct
1680

ggagaacctg gcagagatgg cgtccctgga ggtccaggaa tgaggggcat gcccggaagt
1740

ccaggaggac caggaagtga tgggaaacca gggcctcccg gaagtcaagg agaaagtggt
1800

cgaccaggtc ctcctgggcc atctggtccc cgaggtcagc ctggtgtcat gggcttcccc
1860

ggtcctaaag gaaatgatgg tgctcctggt aagaatggag aacgaggtgg ccctggagga
1920

cctggccctc agggtcctcc tggaaagaat ggtgaaactg gacctcaagg acccccaggg
1980

cctactgggc ctggtggtga caaaggagac acaggacccc ctggtccaca aggattacaa
2040

ggcttgcctg gtacaggtgg tcctccagga gaaaatggaa aacctgggga accaggtcca
2100

aagggtgatg ccggtgcacc tggagctcca ggaggcaagg gtgatgctgg tgcccctggt
2160

gaacgtggac ctcctggatt ggcaggggcc ccaggactta gaggtggagc tggtccccct
2220

ggtcccgaag gaggaaaggg tgctgctggt cctcctgggc cacctggtgc tgctggtact
2280

cctggtctgc aaggaatgcc tggagaaaga ggaggtcttg gaagtcctgg tccaaagggt
2340

gacaagggtg aaccaggcgg cccaggtgct gatggtgtcc cagggaaaga tggcccaagg
2400

ggtcctactg gtcctattgg tcctcctggc ccagctggcc agcctggaga taagggtgaa
2460

ggtggtgccc ccggacttcc aggtatagct ggacctcgtg gtagccctgg tgagagaggt
2520

gaaactggcc ctccaggacc tgctggtttc cctggtgctc ctggacagaa tggtgaacct
2580

ggtggtaaag gagaaagagg ggctccgggt gagaaaggtg aaggaggccc tcctggagtt
2640

gcaggacccc ctggaggttc tggacctgct ggtcctcctg gtccccaagg tgtcaaaggt
2700

gaacgtggca gtcctggtgg acctggtgct gctggcttcc ctggtgctcg tggtcttcct
2760

ggtcctcctg gtagtaatgg taacccagga cccccaggtc ccagcggttc tccaggcaag
2820

gatgggcccc caggtcctgc gggtaacact ggtgctcctg gcagccctgg agtgtctgga
2880

ccaaaaggtg atgctggcca accaggagag aagggatcgc ctggtgccca gggcccacca
2940

ggagctccag gcccacttgg gattgctggg atcactggag cacggggtct tgcaggacca
3000

ccaggcatgc caggtcctag gggaagccct ggccctcagg gtgtcaaggg tgaaagtggg
3060

aaaccaggag ctaacggtct cagtggagaa cgtggtcccc ctggacccca gggtcttcct
3120

ggtctggctg gtacagctgg tgaacctgga agagatggaa accctggatc agatggtctt
3180

ccaggccgag atggatctcc tggtggcaag ggtgatcgtg gtgaaaatgg ctctcctggt
3240

gcccctggcg ctcctggtca tccaggccca cctggtcctg tcggtccagc tggaaagagt
3300

ggtgacagag gagaaagtgg ccctgctggc cctgctggtg ctcccggtcc tgctggttcc
3360

cgaggtgctc ctggtcctca aggcccacgt ggtgacaaag gtgaaacagg tgaacgtgga
3420

gctgctggca tcaaaggaca tcgaggattc cctggtaatc caggtgcccc aggttctcca
3480

ggccctgctg gtcagcaggg tgcaatcggc agtccaggac ctgcaggccc cagaggacct
3540

gttggaccca gtggacctcc tggcaaagat ggaaccagtg gacatccagg tcccattgga
3600

ccaccagggc ctcgaggtaa cagaggtgaa agaggatctg agggctcccc aggccaccca
3660

gggcaaccag gccctcctgg acctcctggt gcccctggtc cttgctgtgg tggtgttgga
3720

gccgctgcca ttgctgggat tggaggtgaa aaagctggcg gttttgcccc gtattatgga
3780

gatgaaccaa tggatttcaa aatcaacacc gatgagatta tgacttcact caagtctgtt
3840

aatggacaaa tagaaagcct cattagtcct gatggttctc gtaaaaaccc cgctagaaac
3900

tgcagagacc tgaaattctg ccatcctgaa ctcaagagtg gagaatactg ggttgaccct
3960

aaccaaggat gcaaattgga tgctatcaag gtattctgta atatggaaac tggggaaaca
4020

tgcataagtg ccaatccttt gaatgttcca cggaaacact ggtggacaga ttctagtgct
4080

gagaagaaac acgtttggtt tggagagtcc atggatggtg gttttcagtt tagctacggc
4140

aatcctgaac ttcctgaaga tgtccttgat gtgcagctgg cattccttcg acttctctcc
4200

agccgagctt cccagaacat cacatatcac tgcaaaaata gcattgcata catggatcag
4260

gccagtggaa atgtaaagaa ggccctgaag ctgatggggt caaatgaagg tgaattcaag
4320

gctgaaggaa atagcaaatt cacctacaca gttctggagg atggttgcac gaaacacact
4380

ggggaatgga gcaaaacagt ctttgaatat cgaacacgca aggctgtgag actacctatt
4440

gtagatattg caccctatga cattggtggt cctgatcaag aatttggtgt ggacgttggc
4500

cctgtttgct ttttataaac caaactctat ctgaaatccc aacaaaaaaa atttaactcc
4560

atatgtgttc ctcttgttct aatcttgtca accagtgcaa gtgaccgaca aaattccagt
4620

tatttatttc caaaatgttt ggaaacagta taatttgaca aagaaaaatg atacttctct
4680

ttttttgctg ttccaccaaa tacaattcaa atgctttttg ttttattttt ttaccaattc
4740

caatttcaaa atgtctcaat ggtgctataa taaataaact tcaacactct ttatgataac
4800

aacactgtgt tatattcttt gaatcctagc ccatctgcag agcaatgact gtgctcacca
4860

gtaaaagata acctttcttt ctgaaatagt caaatacgaa attagaaaag ccctccctat
4920

tttaactacc tcaactggtc agaaacacag attgtattct atgagtccca gaagatgaaa
4980

aaaattttat acgttgataa aacttataaa tttcattgat taatctcctg gaagattggt
5040

ttaaaaagaa aagtgtaatg caagaattta aagaaatatt tttaaagcca caattatttt
5100

aatattggat atcaactgct tgtaaaggtg ctcctctttt ttcttgtcat tgctggtcaa
5160

gattactaat atttgggaag gctttaaaga cgcatgttat ggtgctaatg tactttcact
5220

tttaaactct agatcagaat tgttgacttg cattcagaac ataaatgcac aaaatctgta
5280

catgtctccc atcagaaaga ttcattggca tgccacaggg attctcctcc ttcatcctgt
5340

aaaggtcaac aataaaaacc aaattatggg gctgcttttg tcacactagc atagagaatg
5400

tgttgaaatt taactttgta agcttgtatg tggttgttga tctttttttt ccttacagac
5460

acccataata aaatatcata ttaaaattc
5489

<210> 37

<211> 1722

<212> DNA

<213> Homo sapiens

<400> 37

ggggaaaaga gctaggaaag agctgcaaag cagtgtgggc tttttccctt tttttgctcc 60

ttttcattac ccctcctccg ttttcaccct tctccggact tcgcgtagaa cctgcgaatt
120

tcgaagagga ggtggcaaag tgggagaaaa gaggtgttag ggtttggggt ttttttgttt
180

ttgtttttgt tttttaattt cttgatttca acattttctc ccaccctctc ggctgcagcc
240

aacgcctctt acctgttctg cggcgccgcg caccgctggc agctgagggt tagaaagcgg
300

ggtgtatttt agattttaag caaaaatttt aaagataaat ccatttttct ctcccacccc
360

caacgccatc tccactgcat ccgatctcat tatttcggtg gttgcttggg ggtgaacaat
420

tttgtggctt tttttcccct ataattctga cccgctcagg cttgagggtt tctccggcct
480

ccgctcactg cgtgcacctg gcgctgccct gcttccccca acctgttgca aggctttaat
540

tcttgcaact gggacctgct cgcaggcacc ccagccctcc acctctctct acatttttgc
600

aagtgtctgg gggagggcac ctgctctacc tgccagaaat tttaaaacaa aaacaaaaac
660

aaaaaaatct ccgggggccc tcttggcccc tttatccctg cactctcgct ctcctgcccc
720

accccgaggt aaagggggcg actaagagaa gatggtgttg ctcaccgcgg tcctcctgct
780

gctggccgcc tatgcggggc cggcccagag cctgggctcc ttcgtgcact gcgagccctg
840

cgacgagaaa gccctctcca tgtgcccccc cagccccctg ggctgcgagc tggtcaagga
900

gccgggctgc ggctgctgca tgacctgcgc cctggccgag gggcagtcgt gcggcgtcta
960

caccgagcgc tgcgcccagg ggctgcgctg cctcccccgg caggacgagg agaagccgct
1020

gcacgccctg ctgcacggcc gcggggtttg cctcaacgaa aagagctacc gcgagcaagt
1080

caagatcgag agagactccc gtgagcacga ggagcccacc acctctgaga tggccgagga
1140

gacctactcc cccaagatct tccggcccaa acacacccgc atctccgagc tgaaggctga
1200

agcagtgaag aaggaccgca gaaagaagct gacccagtcc aagtttgtcg ggggagccga
1260

gaacactgcc cacccccgga tcatctctgc acctgagatg agacaggagt ctgagcaggg
1320

cccctgccgc agacacatgg aggcttccct gcaggagctc aaagccagcc cacgcatggt
1380

gccccgtgct gtgtacctgc ccaattgtga ccgcaaagga ttctacaaga gaaagcagtg
1440

caaaccttcc cgtggccgca agcgtggcat ctgctggtgc gtggacaagt acgggatgaa
1500

gctgccaggc atggagtacg ttgacgggga ctttcagtgc cacaccttcg acagcagcaa
1560

cgttgagtga tgcgtccccc cccaaccttt ccctcacccc ctcccacccc cagccccgac
1620

tccagccagc gcctccctcc accccaggac gccactcatt tcatctcatt taagggaaaa
1680

atatatatct atctatttga ggaaaaaaaa aaaaaaaaaa aa
1722

<210> 38

<211> 1200

<212> DNA

<213> Homo sapiens

<400> 38

aagatataaa agctccagaa acgttgactg ggaccactgg agacactgaa gaaggcaggg 60

gcccttagag tcttggttgc caaacagatt tgcagatcaa ggagaaccca ggagtttcaa
120

agaagcgcta gtaaggtctc tgagatcctt gcactagcta catcctcagg gtaggaggaa
180

gatggcttcc agaagcatgc ggctgctcct attgctgagc tgcctggcca aaacaggagt
240

cctgggtgat atcatcatga gacccagctg tgctcctgga tggttttacc acaagtccaa
300

ttgctatggt tacttcagga agctgaggaa ctggtctgat gccgagctcg agtgtcagtc
360

ttacggaaac ggagcccacc tggcatctat cctgagttta aaggaagcca gcaccatagc
420

agagtacata agtggctatc agagaagcca gccgatatgg attggcctgc acgacccaca
480

gaagaggcag cagtggcagt ggattgatgg ggccatgtat ctgtacagat cctggtctgg
540

caagtccatg ggtgggaaca agcactgtgc tgagatgagc tccaataaca actttttaac
600

ttggagcagc aacgaatgca acaagcgcca acacttcctg tgcaagtacc gaccatagag
660

caagaatcaa gattctgcta actcctgcac agccccgtcc tcttcctttc tgctagcctg
720

gctaaatctg ctcattattt cagaggggaa acctagcaaa ctaagagtga taagggccct
780

actacactgg cttttttagg cttagagaca gaaactttag cattggccca gtagtggctt
840

ctagctctaa atgtttgccc cgccatccct ttccacagta tccttcttcc ctcctcccct
900

gtctctggct gtctcgagca gtctagaaga gtgcatctcc agcctatgaa acagctgggt
960

ctttggccat aagaagtaaa gatttgaaga cagaaggaag aaactcagga gtaagcttct
1020

agaccccttc agcttctaca cccttctgcc ctctctccat tgcctgcacc ccaccccagc
1080

cactcaactc ctgcttgttt ttcctttggc cataggaagg tttaccagta gaatccttgc
1140

taggttgatg tgggccatac attcctttaa taaaccattg tgtacataag aaaaaaaaaa
1200

<210> 39

<211> 1701

<212> DNA

<213> Homo sapiens

<400> 39

ccgcatccta gccgccgact cacacaaggc aggtgggtga ggaaatccag agttgccatg 60

gagaaaattc cagtgtcagc attcttgctc cttgtggccc tctcctacac tctggccaga
120

gataccacag tcaaacctgg agccaaaaag gacacaaagg actctcgacc caaactgccc
180

cagaccctct ccagaggttg gggtgaccaa ctcatctgga ctcagacata tgaagaagct
240

ctatataaat ccaagacaag caacaaaccc ttgatgatta ttcatcactt ggatgagtgc
300

ccacacagtc aagctttaaa gaaagtgttt gctgaaaata aagaaatcca gaaattggca
360

gagcagtttg tcctcctcaa tctggtttat gaaacaactg acaaacacct ttctcctgat
420

ggccagtatg tccccaggat tatgtttgtt gacccatctc tgacagttag agccgatatc
480

actggaagat attcaaatcg tctctatgct tacgaacctg cagatacagc tctgttgctt
540

gacaacatga agaaagctct caagttgctg aagactgaat tgtaaagaaa aaaaatctcc
600

aagcccttct gtctgtcagg ccttgagact tgaaaccaga agaagtgtga gaagactggc
660

tagtgtggaa gcatagtgaa cacactgatt aggttatggt ttaatgttac aacaactatt
720

ttttaagaaa aacaagtttt agaaatttgg tttcaagtgt acatgtgtga aaacaatatt
780

gtatactacc atagtgagcc atgattttct aaaaaaaaaa ataaatgttt tgggggtgtt
840

ctgttttctc caacttggtc tttcacagtg gttcgtttac caaataggat taaacacaca
900

caaaatgctc aaggaaggga caagacaaaa ccaaaactag ttcaaatgat gaagaccaaa
960

gaccaagtta tcatctcacc acaccacagg ttctcactag atgactgtaa gtagacacga
1020

gcttaatcaa cagaagtatc aagccatgtg ctttagcata aaagaatatt tagaaaaaca
1080

tcccaagaaa atcacatcac tacctagagt caactctggc caggaactct aaggtacaca
1140

ctttcattta gtaattaaat tttagtcaga ttttgcccaa cctaatgctc tcagggaaag
1200

cctctggcaa gtagctttct ccttcagagg tctaatttag tagaaaggtc atccaaagaa
1260

catctgcact cctgaacaca ccctgaagaa atcctgggaa ttgaccttgt aatcgatttg
1320

tctgtcaagg tcctaaagta ctggagtgaa ataaattcag ccaacatgtg actaattgga
1380

agaagagcaa agggtggtga cgtgttgatg aggcagatgg agatcagagg ttactagggt
1440

ttaggaaacg tgaaaggctg tggcatcagg gtaggggagc attctgccta acagaaatta
1500

gaattgtgtg ttaatgtctt cactctatac ttaatctcac attcattaat atatggaatt
1560

cctctactgc ccagcccctc ctgatttctt tggcccctgg actatggtgc tgtatataat
1620

gctttgcagt atctgttgct tgtcttgatt aacttttttg gataaaacct tttttgaaca
1680

gaaaaaaaaa aaaaaaaaaa a
1701

<210> 40

<211> 2259

<212> DNA

<213> Homo sapiens

<400> 40

cagttgcttc agcgtcccgg tgtggctgtg ccgttggtcc tgtgcggtca cttagccaag 60

atgcctgagg aaacccagac ccaagaccaa ccgatggagg aggaggaggt tgagacgttc
120

gcctttcagg cagaaattgc ccagttgatg tcattgatca tcaatacttt ctactcgaac
180

aaagagatct ttctgagaga gctcatttca aattcatcag atgcattgga caaaatccgg
240

tatgaaagct tgacagatcc cagtaaatta gactctggga aagagctgca tattaacctt
300

ataccgaaca aacaagatcg aactctcact attgtggata ctggaattgg aatgaccaag
360

gctgacttga tcaataacct tggtactatc gccaagtctg ggaccaaagc gttcatggaa
420

gctttgcagg ctggtgcaga tatctctatg attggccagt tcggtgttgg tttttattct
480

gcttatttgg ttgctgagaa agtaactgtg atcaccaaac ataacgatga tgagcagtac
540

gcttgggagt cctcagcagg gggatcattc acagtgagga cagacacagg tgaacctatg
600

ggtcgtggaa caaaagttat cctacacctg aaagaagacc aaactgagta cttggaggaa
660

cgaagaataa aggagattgt gaagaaacat tctcagttta ttggatatcc cattactctt
720

tttgtggaga aggaacgtga taaagaagta agcgatgatg aggctgaaga aaaggaagac
780

aaagaagaag aaaaagaaaa agaagagaaa gagtcggaag acaaacctga aattgaagat
840

gttggttctg atgaggaaga agaaaagaag gatggtgaca agaagaagaa gaagaagatt
900

aaggaaaagt acatcgatca agaagagctc aacaaaacaa agcccatctg gaccagaaat
960

cccgacgata ttactaatga ggagtacgga gaattctata agagcttgac caatgactgg
1020

gaagatcact tggcagtgaa gcatttttca gttgaaggac agttggaatt cagagccctt
1080

ctatttgtcc cacgacgtgc tccttttgat ctgtttgaaa acagaaagaa aaagaacaac
1140

atcaaattgt atgtacgcag agttttcatc atggataact gtgaggagct aatccctgaa
1200

tatctgaact tcattagagg ggtggtagac tcggaggatc tccctctaaa catatcccgt
1260

gagatgttgc aacaaagcaa aattttgaaa gttatcagga agaatttggt caaaaaatgc
1320

ttagaactct ttactgaact ggcggaagat aaagagaact acaagaaatt ctatgagcag
1380

ttctctaaaa acataaagct tggaatacac gaagactctc aaaatcggaa gaagctttca
1440

gagctgttaa ggtactacac atctgcctct ggtgatgaga tggtttctct caaggactac
1500

tgcaccagaa tgaaggagaa ccagaaacat atctattata tcacaggtga gaccaaggac
1560

caggtagcta actcagcctt tgtggaacgt cttcggaaac atggcttaga agtgatctat
1620

atgattgagc ccattgatga gtactgtgtc caacagctga aggaatttga ggggaagact
1680

ttagtgtcag tcaccaaaga aggcctggaa cttccagagg atgaagaaga gaaaaagaag
1740

caggaagaga aaaaaacaaa gtttgagaac ctctgcaaaa tcatgaaaga catattggag
1800

aaaaaagttg aaaaggtggt tgtgtcaaac cgattggtga catctccatg ctgtattgtc
1860

acaagcacat atggctggac agcaaacatg gagagaatca tgaaagctca agccctaaga
1920

gacaactcaa caatgggtta catggcagca aagaaacacc tggagataaa ccctgaccat
1980

tccattattg agaccttaag gcaaaaggca gaggctgata agaacgacaa gtctgtgaag
2040

gatctggtca tcttgcttta tgaaactgcg ctcctgtctt ctggcttcag tctggaagat
2100

ccccagacac atgctaacag gatctacagg atgatcaaac ttggtctggg tattgatgaa
2160

gatgacccta ctgctgatga taccagtgct gctgtaactg aagaaatgcc accccttgaa
2220

ggagatgacg acacatcacg catggaagaa gtagactaa
2259

<210> 41

<211> 7080

<212> DNA

<213> Homo sapiens

<400> 41
   gagctagcgc tcaagcagag cccagcgcgg tgctatcgga cagagcctgg cgagcgcaag 60
   cggcgcgggg agccagcggg gctgagcgcg gccagggtct gaacccagat ttcccagact
   120
   agctaccact ccgcttgccc acgccccggg agctcgcggc gcctggcggt cagcgaccag
   180
   acgtccgggg ccgctgcgct cctggcccgc gaggcgtgac actgtctcgg ctacagaccc
   240
   agagggagca cactgccagg atgggagctg ctgggaggca ggacttcctc ttcaaggcca
   300
   tgctgaccat cagctggctc actctgacct gcttccctgg ggccacatcc acagtggctg
   360
   ctgggtgccc tgaccagagc cctgagttgc aaccctggaa ccctggccat gaccaagacc
   420
   accatgtgca tatcggccag ggcaagacac tgctgctcac ctcttctgcc acggtctatt
   480
   ccatccacat ctcagaggga ggcaagctgg tcattaaaga ccacgacgag ccgattgttt
   540
   tgcgaacccg gcacatcctg attgacaacg gaggagagct gcatgctggg agtgccctct
   600
   gccctttcca gggcaatttc accatcattt tgtatggaag ggctgatgaa ggtattcagc
   660
   cggatcctta ctatggtctg aagtacattg gggttggtaa aggaggcgct cttgagttgc
   720
   atggacagaa aaagctctcc tggacatttc tgaacaagac ccttcaccca ggtggcatgg
   780
   cagaaggagg ctattttttt gaaaggagct ggggccaccg tggagttatt gttcatgtca
   840
   tcgaccccaa atcaggcaca gtcatccatt ctgaccggtt tgacacctat agatccaaga
   900
   aagagagtga acgtctggtc cagtatttga acgcggtgcc cgatggcagg atcctttctg
   960
   ttgcagtgaa tgatgaaggt tctcgaaatc tggatgacat ggccaggaag gcgatgacca
   1020
   aattgggaag caaacacttc ctgcaccttg gatttagaca cccttggagt tttctaactg
   1080
   tgaaaggaaa tccatcatct tcagtggaag accatattga atatcatgga catcgaggct
   1140
   ctgctgctgc ccgggtattc aaattgttcc agacagagca tggcgaatat ttcaatgttt
   1200
   ctttgtccag tgagtgggtt caagacgtgg agtggacgga gtggttcgat catgataaag
   1260
   tatctcagac taaaggtggg gagaaaattt cagacctctg gaaagctcac ccaggaaaaa
   1320
   tatgcaatcg tcccattgat atacaggcca ctacaatgga tggagttaac ctcagcaccg
   1380
   aggttgtcta caaaaaaggc caggattata ggtttgcttg ctacgaccgg ggcagagcct
   1440
   gccggagcta ccgtgtacgg ttcctctgtg ggaagcctgt gaggcccaaa ctcacagtca
   1500
   ccattgacac caatgtgaac agcaccattc tgaacttgga ggataatgta cagtcatgga
   1560
   aacctggaga taccctggtc attgccagta ctgattactc catgtaccag gcagaagagt
   1620
   tccaggtgct tccctgcaga tcctgcgccc ccaaccaggt caaagtggca gggaaaccaa
   1680
   tgtacctgca catcggggag gagatagacg gcgtggacat gcgggcggag gttgggcttc
   1740
   tgagccggaa catcatagtg atgggggaga tggaggacaa atgctacccc tacagaaacc
   1800
   acatctgcaa tttctttgac ttcgatacct ttgggggcca catcaagttt gctctgggat
   1860
   ttaaggcagc acacttggag ggcacggagc tgaagcatat gggacagcag ctggtgggtc
   1920
   agtacccgat tcacttccac ctggccggtg atgtagacga aaggggaggt tatgacccac
   1980
   ccacatacat cagggacctc tccatccatc atacattctc tcgctgcgtc acagtccatg
   2040
   gctccaatgg cttgttgatc aaggacgttg tgggctataa ctctttgggc cactgcttct
   2100
   tcacggaaga tgggccggag gaacgcaaca cttttgacca ctgtcttggc ctccttgtca
   2160
   agtctggaac cctcctcccc tcggaccgtg acagcaagat gtgcaagatg atcacagagg
   2220
   actcctaccc ggggtacatc cccaagccca ggcaagactg caatgctgtg tccaccttct
   2280
   ggatggccaa tcccaacaac aacctcatca actgtgccgc tgcaggatct gaggaaactg
   2340
   gattttggtt tatttttcac cacgtaccaa cgggcccctc cgtgggaatg tactccccag
   2400
   gttattcaga gcacattcca ctgggaaaat tctataacaa ccgagcacat tccaactacc
   2460
   gggctggcat gatcatagac aacggagtca aaaccaccga ggcctctgcc aaggacaagc
   2520
   ggccgttcct ctcaatcatc tctgccagat acagccctca ccaggacgcc gacccgctga
   2580
   agccccggga gccggccatc atcagacact tcattgccta caagaaccag gaccacgggg
   2640
   cctggctgcg cggcggggat gtgtggctgg acagctgccg gtttgctgac aatggcattg
   2700
   gcctgaccct ggccagtggt ggaaccttcc cgtatgacga cggctccaag caagagataa
   2760
   agaacagctt gtttgttggc gagagtggca acgtggggac ggaaatgatg gacaatagga
   2820
   tctgggggcc tggcggcttg gaccatagcg gaaggaccct ccctataggc cagaattttc
   2880
   caattagagg aattcagtta tatgatggcc ccatcaacat ccaaaactgc actttccgaa
   2940
   agtttgtggc cctggagggc cggcacacca gcgccctggc cttccgcctg aataatgcct
   3000
   ggcagagctg cccccataac aacgtgaccg gcattgcctt tgaggacgtt ccgattactt
   3060
   ccagagtgtt cttcggagag cctgggccct ggttcaacca gctggacatg gatggggata
   3120
   agacatctgt gttccatgac gtcgacggct ccgtgtccga gtaccctggc tcctacctca
   3180
   cgaagaatga caactggctg gtccggcacc cagactgcat caatgttccc gactggagag
   3240
   gggccatttg cagtgggtgc tatgcacaga tgtacattca agcctacaag accagtaacc
   3300
   tgcgaatgaa gatcatcaag aatgacttcc ccagccaccc tctttacctg gagggggcgc
   3360
   tcaccaggag cacccattac cagcaatacc aaccggttgt caccctgcag aagggctaca
   3420
   ccatccactg ggaccagacg gcccccgccg aactcgccat ctggctcatc aacttcaaca
   3480
   agggcgactg gatccgagtg gggctctgct acccgcgagg caccacattc tccatcctct
   3540
   cggatgttca caatcgcctg ctgaagcaaa cgtccaagac gggcgtcttc gtgaggacct
   3600
   tgcagatgga caaagtggag cagagctacc ctggcaggag ccactactac tgggacgagg
   3660
   actcagggct gttgttcctg aagctgaaag ctcagaacga gagagagaag tttgctttct
   3720
   gctccatgaa aggctgtgag aggataaaga ttaaagctct gattccaaag aacgcaggcg
   3780
   tcagtgactg cacagccaca gcttacccca agttcaccga gagggctgtc gtagacgtgc
   3840
   cgatgcccaa gaagctcttt ggttctcagc tgaaaacaaa ggaccatttc ttggaggtga
   3900
   agatggagag ttccaagcag cacttcttcc acctctggaa cgacttcgct tacattgaag
   3960
   tggatgggaa gaagtacccc agttcggagg atggcatcca ggtggtggtg attgacggga
   4020
   accaagggcg cgtggtgagc cacacgagct tcaggaactc cattctgcaa ggcataccat
   4080
   ggcagctttt caactatgtg gcgaccatcc ctgacaattc catagtgctt atggcatcaa
   4140
   agggaagata cgtctccaga ggcccatgga ccagagtgct ggaaaagctt ggggcagaca
   4200
   ggggtctcaa gttgaaagag caaatggcat tcgttggctt caaaggcagc ttccggccca
   4260
   tctgggtgac actggacact gaggatcaca aagccaaaat cttccaagtt gtgcccatcc
   4320
   ctgtggtgaa gaagaagaag ttgtgaggac agctgccgcc cggtgccacc tcgtggtaga
   4380
   ctatgacggt gactcttggc agcagaccag tgggggatgg ctgggtcccc cagcccctgc
   4440
   cagcagctgc ctgggaaggc cgtgtttcag ccctgatggg ccaagggaag gctatcagag
   4500
   accctggtgc tgccacctgc ccctactcaa gtgtctacct ggagcccctg gggcggtgct
   4560
   ggccaatgct ggaaacattc actttcctgc agcctcttgg gtgcttctct cctatctgtg
   4620
   cctcttcagt gggggtttgg ggaccatatc aggagacctg ggttgtgctg acagcaaaga
   4680
   tccactttgg caggagccct gacccagcta ggaggtagtc tggagggctg gtcattcaca
   4740
   gatccccatg gtcttcagca gacaagtgag ggtggtaaat gtaggagaaa gagccttggc
   4800
   cttaaggaaa tctttactcc tgtaagcaag agccaacctc acaggattag gagctggggt
   4860
   agaactggct atccttgggg aagaggcaag ccctgcctct ggccgtgtcc acctttcagg
   4920
   agactttgag tggcaggttt ggacttggac tagatgactc tcaaaggccc ttttagttct
   4980
   gagattccag aaatctgctg catttcacat ggtacctgga acccaacagt tcatggatat
   5040
   ccactgatat ccatgatgct gggtgcccca gcgcacacgg gatggagagg tgagaactaa
   5100
   tgcctagctt gaggggtctg cagtccagta gggcaggcag tcaggtccat gtgcactgca
   5160
   atgccaggtg gagaaatcac agagaggtaa aatggaggcc agtgccattt cagaggggag
   5220
   gctcaggaag gcttcttgct tacaggaatg aaggctgggg gcattttgct ggggggagat
   5280
   gaggcagcct ctggaatggc tcagggattc agccctccct gccgctgcct gctgaagctg
   5340
   gtgactacgg ggtcgccctt tgctcacgtc tctctggccc actcatgatg gagaagtgtg
   5400
   gtcagagggg agcaatgggc tttgctgctt atgagcacag aggaattcag tccccaggca
   5460
   gccctgcctc tgactccaag agggtgaagt ccacagaagt gagctcctgc cttagggcct
   5520
   catttgctct tcatccaggg aactgagcac agggggcctc caggagaccc tagatgtgct
   5580
   cgtactccct cggcctggga tttcagagct ggaaatatag aaaatatcta gcccaaagcc
   5640
   ttcattttaa cagatgggga aagtgagccc ccaagatggg aaagaaccac acagctaagg
   5700
   gagggcctgg ggagccccac cctagccctt gctgccacac cacattgcct caacaaccgg
   5760
   ccccagagtg cccaggcact cctgaggtag cttctggaaa tggggacaag tcccctcgaa
   5820
   ggaaaggaaa tgactagagt agaatgacag ctagcagatc tcttccctcc tgctcccagc
   5880
   gcacacaaac ccgccctccc cttggtgttg gcggtccctg tggccttcac tttgttcact
   5940
   acctgtcagc ccagcctggg tgcacagtag ctgcaactcc ccattggtgc tacctggctc
   6000
   tcctgtctct gcagctctac aggtgaggcc cagcagaggg agtagggctc gccatgtttc
   6060
   tggtgagcca atttggctga tcttgggtgt ctgaacagct attgggtcca ccccagtccc
   6120
   tttcagctgc tgcttaatgc cctgctctct ccctggccca ccttatagag agcccaaaga
   6180
   gctcctgtaa gagggagaac tctatctgtg gtttataatc ttgcacgagg caccagagtc
   6240
   tccctgggtc ttgtgatgaa ctacatttat cccctttcct gccccaacca caaactcttt
   6300
   ccttcaaaga gggcctgcct ggctccctcc acccaactgc acccatgaga ctcggtccaa
   6360
   gagtccattc cccaggtggg agccaactgt cagggaggtc tttcccacca aacatctttc
   6420
   agctgctggg aggtgaccat agggctctgc ttttaaagat atggctgctt caaaggccag
   6480
   agtcacagga aggacttctt ccagggagat tagtggtgat ggagaggaga gttaaaatga
   6540
   cctcatgtcc ttcttgtcca cggttttgtt gagttttcac tcttctaatg caagggtctc
   6600
   acactgtgaa ccacttagga tgtgatcact ttcaggtggc caggaatgtt gaatgtcttt
   6660
   ggctcagttc atttaaaaaa gatatctatt tgaaagttct cagagttgta catatgtttc
   6720
   acagtacagg atctgtacat aaaagtttct ttcctaaacc attcaccaag agccaatatc
   6780
   taggcatttt cttggtagca caaattttct tattgcttag aaaattgtcc tccttgttat
   6840
   ttctgtttgt aagacttaag tgagttaggt ctttaaggaa agcaacgctc ctctgaaatg
   6900
   cttgtctttt ttctgttgcc gaaatagctg gtcctttttc gggagttaga tgtatagagt
   6960
   gtttgtatgt aaacatttct tgtaggcatc accatgaaca aagatatatt ttctatttat
   7020
   ttattatatg tgcacttcaa gaagtcactg tcagagaaat aaagaattgt cttaaatgtc
   7080

<210> 42

<211> 1973

<212> DNA

<213> Homo sapiens

<400> 42

gggatattgg agtagcaaga ggctgggaag ccatcactta ccttgcactg agaaagaaga 60

caaaggccag tatgcacagc tttcctccac tgctgctgct gctgttctgg ggtgtggtgt
120

ctcacagctt cccagcgact ctagaaacac aagagcaaga tgtggactta gtccagaaat
180

acctggaaaa atactacaac ctgaagaatg atgggaggca agttgaaaag cggagaaata
240

gtggcccagt ggttgaaaaa ttgaagcaaa tgcaggaatt ctttgggctg aaagtgactg
300

ggaaaccaga tgctgaaacc ctgaaggtga tgaagcagcc cagatgtgga gtgcctgatg
360

tggctcagtt tgtcctcact gaggggaacc ctcgctggga gcaaacacat ctgacctaca
420

ggattgaaaa ttacacgcca gatttgccaa gagcagatgt ggaccatgcc attgagaaag
480

ccttccaact ctggagtaat gtcacacctc tgacattcac caaggtctct gagggtcaag
540

cagacatcat gatatctttt gtcaggggag atcatcggga caactctcct tttgatggac
600

ctggaggaaa tcttgctcat gcttttcaac caggcccagg tattggaggg gatgctcatt
660

ttgatgaaga tgaaaggtgg accaacaatt tcagagagta caacttacat cgtgttgcgg
720

ctcatgaact cggccattct cttggactct cccattctac tgatatcggg gctttgatgt
780

accctagcta caccttcagt ggtgatgttc agctagctca ggatgacatt gatggcatcc
840

aagccatata tggacgttcc caaaatcctg tccagcccat cggcccacaa accccaaaag
900

cgtgtgacag taagctaacc tttgatgcta taactacgat tcggggagaa gtgatgttct
960

ttaaagacag attctacatg cgcacaaatc ccttctaccc ggaagttgag ctcaatttca
1020

tttctgtttt ctggccacaa ctgccaaatg ggcttgaagc tgcttacgaa tttgccgaca
1080

gagatgaagt ccggtttttc aaagggaata agtactgggc tgttcaggga cagaatgtgc
1140

tacacggata ccccaaggac atctacagct cctttggctt ccctagaact gtgaagcata
1200

tcgatgctgc tctttctgag gaaaacactg gaaaaaccta cttctttgtt gctaacaaat
1260

actggaggta tgatgaatat aaacgatcta tggatccagg ttatcccaaa atgatagcac
1320

atgactttcc tggaattggc cacaaagttg atgcagtttt catgaaagat ggatttttct
1380

atttctttca tggaacaaga caatacaaat ttgatcctaa aacgaagaga attttgactc
1440

tccagaaagc taatagctgg ttcaactgca ggaaaaattg aacattacta atttgaatgg
1500

aaaacacatg gtgtgagtcc aaagaaggtg ttttcctgaa gaactgtcta ttttctcagt
1560

catttttaac ctctagagtc actgatacac agaatataat cttatttata cctcagtttg
1620

catatttttt tactatttag aatgtagccc tttttgtact gatataattt agttccacaa
1680

atggtgggta caaaaagtca agtttgtggc ttatggattc atataggcca gagttgcaaa
1740

gatcttttcc agagtatgca actctgacgt tgatcccaga gagcagcttc agtgacaaac
1800

atatcctttc aagacagaaa gagacaggag acatgagtct ttgccggagg aaaagcagct
1860

caagaacaca tgtgcagtca ctggtgtcac cctggatagg caagggataa ctcttctaac
1920

acaaaataag tgttttatgt ttggaataaa gtcaaccttg tttctactgt ttt
1973

<210> 43

<211> 1127

<212> DNA

<213> Homo sapiens

<400> 43

accaaatcaa ccataggtcc aagaacaatt gtctctggac ggcagctatg cgactcaccg 60

tgctgtgtgc tgtgtgcctg ctgcctggca gcctggccct gccgctgcct caggaggcgg
120

gaggcatgag tgagctacag tgggaacagg ctcaggacta tctcaagaga ttttatctct
180

atgactcaga aacaaaaaat gccaacagtt tagaagccaa actcaaggag atgcaaaaat
240

tctttggcct acctataact ggaatgttaa actcccgcgt catagaaata atgcagaagc
300

ccagatgtgg agtgccagat gttgcagaat actcactatt tccaaatagc ccaaaatgga
360

cttccaaagt ggtcacctac aggatcgtat catatactcg agacttaccg catattacag
420

tggatcgatt agtgtcaaag gctttaaaca tgtggggcaa agagatcccc ctgcatttca
480

ggaaagttgt atggggaact gctgacatca tgattggctt tgcgcgagga gctcatgggg
540

actcctaccc atttgatggg ccaggaaaca cgctggctca tgcctttgcg cctgggacag
600

gtctcggagg agatgctcac ttcgatgagg atgaacgctg gacggatggt agcagtctag
660

ggattaactt cctgtatgct gcaactcatg aacttggcca ttctttgggt atgggacatt
720

cctctgatcc taatgcagtg atgtatccaa cctatggaaa tggagatccc caaaatttta
780

aactttccca ggatgatatt aaaggcattc agaaactata tggaaagaga agtaattcaa
840

gaaagaaata gaaacttcag gcagaacatc cattcattca ttcattggat tgtatatcat
900

tgttgcacaa tcagaattga taagcactgt tcctccactc catttagcaa ttatgtcacc
960

cttttttatt gcagttggtt tttgaatgtc tttcactcct tttattggtt aaactccttt
1020

atggtgtgac tgtgtcttat tccatctatg agctttgtca gtgcgcgtag atgtcaataa
1080

atgttacata cacaaataaa taaaatgttt attccatggt aaattta
1127

<210> 44

<211> 1163

<212> DNA

<213> Homo sapiens

<400> 44
   agatccagca ggtgcgcgaa accgcggcgc gccgccctcc gccgttatat gaggccccgc 60
   tccggcccca cgcggaaccc gcggctccga gccttcgccg gcgtcccgac ccgaggccgg
   120
   acccgaggcc agtcccgccg ctgcgcagcc gaagccagtg cggggcctga gagggacgcg
   180
   cgccccgggg cccccgccgc gggcaccatg ggcgctgccc actccgcgtc tgaggaggtg
   240
   cgggagctcg agggcaagac cggcttctca tcggatcaga tcgagcagct ccatcggaga
   300
   tttaagcagc tgagtggaga tcagcctacc attcgcaagg agaacttcaa caatgtcccg
   360
   gacctggagc tcaaccccat ccgatccaaa attgttcgtg ccttcttcga caacaggaac
   420
   ctgcgcaagg gacccagtgg cctggctgat gagatcaatt tcgaggactt cctgaccatc
   480
   atgtcctact tccggcccat cgacaccacc atggacgagg aacaggtgga gctgtcccgg
   540
   aaggagaagc tgagatttct gttccacatg tacgactcgg acagcgacgg ccgcatcact
   600
   ctggaagaat atcgaaatgt aaagtggtcg aggagctgct gtcgggaaac cctcacatct
   660
   agaaggagtc cgctcgctcc atcgccgacg gggccatgat ggaggcggcc agcgtgtgca
   720
   tggggcagat ggagcctgat caggtgtacg aggggatcac cttcgaggac ttcctgaaga
   780
   tctggcaggg gatcgacatt gagaccaaga tgcacgtccg cttccttaac atggaaacca
   840
   tggccctctg ccactgaccc accgccacct ccgcggagag actgcacttt gcaatggggc
   900
   cgcctccccg cgtagctgga gcagcccagg cccggcggac agcctcttcc tgcagcgccg
   960
   gtacatagcc aaggctcgtc tgcgcacctt gtgtcttgta gggtatggta tgtgggactt
   1020
   cgctgttttt atctccaata aaaaaaaaaa aaaaaggttt gttaattaaa aaaaaaaaaa
   1080
   aaaagaaaaa aaaaaaaaaa aaaaaaaaaa aaaaagaaaa aaaaaaaaaa aaaaaaaaaa
   1140
   aaaaaaaaaa aaaaaaaaaa aaa
   1163

<210> 45

<211> 2007

<212> DNA

<213> Homo sapiens

<400> 45

agcggagggg gaaatcctga gcgcaggcca gggttgtttg gttttgaggt gtgctgggat 60

gaaaggcacc ctggaagtgg aaggtaaatg agcaatggaa aaacttcacg gcaagattag
120

aaagatacct gagcccaata cccgcctgat gtcgtgggcc acacctccgg gttaccaggg
180

gaagggagga agcaaactgt catattgatg tggctctaaa caacaacagt gtgcgaaggc
240

ccaggggcac tttgggattg accaagagga aacacaagtt gcacaatgat acaatcttgt
300

tggtacaatt gtcagagaag ggaactccca cagcaaaggc cataaaacca tccagggcag
360

tctggggcgg ctcagttctg cggtgccagg gagtggagca gagctcagcc ccgtcccaaa
420

cacagatggg accatgaact ccggacacag cttcagccag accccctcgg cctccttcca
480

tggcgccgga ggtggctggg gccggcccag gagcttcccc agggctccca ccgtccatgg
540

cggtgcgggg ggagcccgca tctccctgtc cttcaccacg cggagctgcc caccccctgg
600

agggtcttgg ggttctggaa gaagcagccc cctactaggc ggaaatggga aggccaccat
660

gcagaatctc aacgaccgcc tggcctccta cgtggagaag gttcgcgccc tggaggaggc
720

caacatgaag ctggaaagcc gcatcctgaa atggcaccag cagagagatc ctggcagtaa
780

gaaagattat tcacagtatg aggaaaacat cacacacctg caggagcaga tagtggatgg
840

taagatgacc aatgctcaga ttattcttct cattgacaat gccaggatgg cagtggatga
900

cttcaacctc aagtatgaaa atgaacactc ctttaagaaa gacttggaaa ttgaagtcga
960

gggcctccga aggaccttag acaacctgac cattgtcaca acagacctag aacaggaggt
1020

ggaaggaatg aggaaagagc tcattctcat gaagaagcac catgagcagg aaatggagaa
1080

gcatcatgtg ccaagtgact tcaatgtcaa tgtgaaggtg gatacgggtc ccagggaaga
1140

tctgattaag gtcctggagg atatgagaca agaatatgag cttataataa agaagaagca
1200

tcgagacttg gacacttggt ataaagaaca gtctgcagcc atgtcccagg aggcagccag
1260

tccagccact gtgcagagca gacaaggtga catccacgaa ctgaagcgca cattccaggc
1320

cctggagatt gacctgcaga cacagtacag cacgaaatct gctttggaaa acatgttatc
1380

cgagacccag tctcggtact cctgcaagct ccaggacatg caagagatca tctcccacta
1440

tgaggaggaa ctgacgcagc tacgccatga actggagcgg cagaacaatg aataccaagt
1500

gctgctgggc atcaaaaccc acctggagaa ggaaatcacc acgtaccgac ggctcctgga
1560

gggagagagt gaagggacac gggaagaatc aaagtcgagc atgaaagtgt ttgcaactcc
1620

aaagatcaag gccataaccc aggagaccat caacggaaga ttagttcttt gtcaagtgaa
1680

tgaaatccaa aagcacgcat gagaccaatg aaagtttccg cctgttgtaa aatctatttt
1740

cccccaagga aagtccttgc acagacacca gtgagtgagt tctaaaagat acccttggaa
1800

ttatcagact cagaaacttt tatttttttt ttctgtaact gtctcaccag acttctcata
1860

atgctcttaa tatattgcac ttttctaatc aaagtgcgag tttatgaggg taaagctcta
1920

ctttcctact gcagccttca gattctcatc attttgcatc tattttgtag ccaataaaac
1980

tccgcactag caaaaaaaaa aaaaaaa
2007

<210> 46

<211> 1650

<212> DNA

<213> Homo sapiens

<400> 46

gccttttttg cagtctcagg acgggcgctt tggagccggc cccaggcagc gtgtgtcggt 60

cgcctagtct ggagaactag tcctcgactc acggtgaggg aatggaccga cacgggtatt
120

gtaccgctga gggaaaggag cgggactccg gacctccagg agtgcaagga tgatgctgaa
180

aggaataaca aggcttatct ctaggatcca taagttggac cctgggcgtt ttttacacat
240

ggggacccag gctcgccaaa gcattgctgc tcacctagat aaccaggttc cagttgagag
300

tccgagagct atttcccgca ccaatgagaa tgacccggcc aagcatgggg atcagcacga
360

gctctggacc tgcattaaaa tgggtttcac tgtgaatgcg tgacaataag atattccctt
1560

gttcctaaaa ctttatatca gtttattgga tgtggttttt cacatttaag ataattatgg
1620

ctcttttcct aaaaaataaa atatctttct
1650

<210> 47

<211> 2566

<212> DNA

<213> Homo sapiens

<400> 47

cgcttttttt ttttttttct ttttttgaga tggagcttgc tctgtcaccc aggctggagt 60

gcagtggcac gatcacagct cactgcaact tccgccacct gggttcaagc aattttcctg
120

cttcaacctc ccgagtagtt gagattacag gcacgtgcca ccacacctga ctgatttttg
180

tatttttagt agagacaggg tttcaccatg ttggccaggc tggtcttgaa ctcctgacct
240

caagtgatcc acccacctca gtctaccaaa gtgctgggat tacaggttgt gagccactgc
300

acccggcctc tgtctgactt ttaaatgatg gagtatccca gaggtcaggc tttagagctc
360

ttctttcttc catctacaga gtgctcattt ctttggtgat ctcattcagt ctcacggctt
420

tgaatgttat atgctgataa gtcccaactt tctctgtctc gcctaaactt cccctaatag
480

cagaacctga tatcaaagtg cctatttgac atctccactt agatatctaa taagcatctt
540

taatgtaaaa tttccaaaac tgagctccta caattccctc ccacacttgt tcagttttcc
600

ccatctaagc taatggcaac tccatccctc cagttgcttt ggccaaaact tttgagttgt
660

ctttgactgc tttttttctc ccttcttaca ttgtgctcat cctttctttc aggcaaattc
720

tgtttggtat accttcaaat atatctagaa ttctatactt ctcactaccc ctcattccca
780

ttatctttga aacacactcc aatgaggttt ttacccccac tattccacca aaaacactct
840

tgtgaaagtc accagatgat ggttcttcag gctatcatct gtctcctgaa ttattttaag
900

agcctcccta actggtctcc ttgttccagc ccctgctccc ttcactctat tctcaataca
960

acagtcagag ggatcttata aaaccgtaag tcagatcata taattctgtg tgaaaccctc
1020

caatgttatc ccacttcaga gtaaaagtta aagggtttat tttgatctct aaatattttg
1080

atccctatga tctagtctct tcttacccaa agtatgggcc tcataccagc agaactggca
1140

ttctctggga gcttgttaga aatgtagaat ctctggtcct aagattttct aaatcagaat
1200

ctgcattttc atcagctctc ttggcaattc atatgtttga gaaggactga tgtagtcctc
1260

tactgcctct cctgctgcta tgtgctgctc tagtcacact gtcctcctgg ctatttcgat
1320

aagatggaaa ctctcctgcc tcagggcttt tgcacctact gctccctctg tctaggataa
1380

tcttccccag aaaactgcat gccagttgcc ttatttcctt caggtcttta ttgaagtcac
1440

tttctcatta aacactttct ggcccatcct gtctaaaact gcatccccaa acccttccta
1500

tcccttttct ccactttttt ctttttttaa actatagttc ttatcaacat agtatatgtt
1560

ttgcttattt atcctgttta ttgtctgact caacaagtat aatttgacaa agaaaaatga
1620

tacttctctt tttttgctgt tccaccaaat acaattcaaa tgctttttgt tttatttttt
1680

taccaattcc aatttcaaaa tgtctcaatg gtgctataat aaataaactt caacactctt
1740

tatgataaca acactgtgtt atattctttg aatcctagcc catctgcaga gcaatgactg
1800

tgctcaccag taaaagataa cctttctttc tgaaatagtc aaatacgaaa ttagaaaagc
1860

cctccctatt ttaactacct caactggtca gaaacacaga ttgtattcta tgagtcccag
1920

aagatgaaaa aaattttata cgttgataaa acttataaat ttcattgatt aatctcctgg
1980

aagattggtt taaaaagaaa agtgtaatgc aagaatttaa agaaatattt ttaaagccac
2040

aattatttta atattggata tcaactgctt gtaaaggtgc tcctcctttt tcttgtcatt
2100

gctggtcaag attactaata tttgggaagg ctttaaagac gcatgttatg gtgctaatgt
2160

actttcactt ttaaactcta gatcagaatt gttgacttgc attcagaaca taaatgcaca
2220

aaatctgtac atgtctccca tcagaaagat tcattggcat gccacagggg attctcctcc
2280

ttcatcctgt aaaggtcaac aataaaaacc aaattatggg gctgcttttg tcacactagc
2340

atagagaatg tgttgaaatt taactttgta agcttgtatg tggttgttga tctttttttt
2400

ccttacagac acccataata aaatatcata ttaaaaaaaa aaaaaaaaaa aaaaaaaaaa
2460

aaaaaaaaaa aaaaaaaaaa aaaaaaaaaa aaaaaaaaaa aaaaaaaaaa aaaaaaaaaa
2520

aaaaaaaaaa aaaaaaaaaa aaaaaaaaaa aaaaaaaaaa aaaaaa
2566

<210> 48

<211> 2067

<212> DNA

<213> Homo sapiens

<400> 48

cgcttttttt tttttttttt atgaataaat tgtatgtgtt tctagctgaa actactcacc 60

aatatgcatg ttaaattcaa tcctctctta cctattcttt gatataactc cttgaagttt
120

tcccattctc ctacatcatc tcccccaccc ccactgagtc atttctataa tacaaacata
180

ttatcttttt attaaattaa aaatctacct gtacaccttg aatgacaatg ctcggagctc
240

ccctgtggtc atcgacgcct ccactgccat tgatgcacca tccaacctgc gtttcctggc
300

caccacaccc aattccttgc tggtatcatg gcagccgcca cgtgccagga ttaccggcta
360

catcatcaag tatgagaagc ctgggtctcc tcccagagaa gtggtccctc ggccccgccc
420

tggtgtcaca gaggctacta ttactggcct ggaaccggga accgaatata caatttatgt
480

cattgccctg aagaataatc agaagagcga gcccctgatt ggaaggaaaa agacaggaca
540

agaagctctc tctcagacaa ccatctcatg ggccccattc caggacactt ctgagtacat
600

catttcatgt catcctgttg gcactgatga agaaccctta cagttcaggg ttcctggaac
660

ttctaccagt gccactctga caggcctcac cagaggtgcc acctacaaca tcatagtgga
720

ggcactgaaa gaccagcaga ggcataaggt tcgggaagag gttgttaccg tgggcaactc
780

tgtcaacgaa ggcttgaacc aacctacgga tgactcgtgc tttgacccct acacagtttc
840

ccattatgcc gttggagatg agtgggaacg aatgtctgaa tcaggcttta aactgttgtg
900

ccagtgctta ggctttggaa gtggtcattt cagatgtgat tcatctagat ggtgccatga
960

caatggtgtg aactacaaga ttggagagaa gtgggaccgt cagggagaaa atggccagat
1020

gatgagctgc acatgtcttg ggaacggaaa aggagaattc aagtgtgacc ctcatgaggc
1080

aacgtgttat gatgatggga agacatacca cgtaggagaa cagtggcaga aggaatatct
1140

cggtgccatt tgctcctgca catgctttgg aggccagcgg ggctggcgct gtgacaactg
1200

ccgcagacct gggggtgaac ccagtcccga aggcactact ggccagtcct acaaccagta
1260

ttctcagaga taccatcaga gaacaaacac taatgttaat tgcccaattg agtgcttcat
1320

gcctttagat gtacaggctg acagagaaga ttcccgagag taaatcatct ttccaatcca
1380

gaggaacaag catgtctctc tgccaagatc catctaaact ggagtgatgt tagcagaccc
1440

agcttagagt tcttctttct ttcttaagcc ctttgctctg gaggaagttc tccagcttca
1500

gctcaactca cagcttctcc aagcatcacc ctgggagttt cctgagggtt ttctcataaa
1560

tgagggctgc acattgcctg ttctgcttcg aagtattcaa taccgctcag tattttaaat
1620

gaagtgattc taagatttgg tttgggatca ataggaaagc atatgcagcc aaccaagatg
1680

caaatgtttt gaaatgatat gaccaaaatt ttaagtagga aagtcaccca aacacttctg
1740

ctttcactta agtgtctggc ccgcaatact gtaggaacaa gcatgatctt gttactgtga
1800

tattttaaat atccacagta ctcacttttt ccaaatgatc ctagtaattg cctagaaata
1860

tctttctctt acctgttatt tatcaatttt tcccagtatt tttatacgga aaaaattgta
1920

ttgaaaacac ttagtatgca gttgataaga ggaatttggt ataattatgg tgggtgatta
1980

ttttttatac tgtatgtgcc aaagctttac tactgtggaa agacaactgt tttaataaaa
2040

gatttacatt ccaaaaaaaa aaaaaaa
2067

<210> 49

<211> 1752

<212> DNA

<213> Homo sapiens

<400> 49

ctgctccttc taggatctcc gcctggttcg gcccgcctgc ctccactcct gcctccacca 60

tgtccatcag ggtgacccag aagtcctaca aggtgtccac ctctggcccc cgggccttca
120

gcagccgctc ctacacgagt gggcccggtt cccgcatcag ctcctcgagc ttctcccgag
180

tgggcagcag caactttcgc ggtggcctgg gcggcggcta tggtggggcc agcggcatgg
240

gaggcatcac cgcagttacg gtcaaccaga gcctgctgag cccccttgtc ctggaggtgg
300

accccaacat ccaggccgtg cgcacccagg agaaggagca gatcaagacc ctcaacaaca
360

agtttgcctc cttcatagac aaggtacggt tcctggagca gcagaacaag atgctggaga
420

ccaagtggag cctcctgcag cagcagaaga cggctcgaag caacatggac aacatgttcg
480

agagctacat caacaacctt aggcggcagc tggagactct gggccaggag aagctgaagc
540

tggaggcgga gcttggcaac atgcaggggc tggtggagga cttcaagaac aagtatgagg
600

atgagatcaa taagcgtaca gagatggaga acgaatttgt cctcatcaag aaggatgtgg
660

atgaagctta catgaacaag gtagagctgg agtctcgcct ggaagggctg accgacgaga
720

tcaacttcct caggcagcta tatgaagagg agatccggga gctgcagtcc cagatctcgg
780

acacatctgt ggtgctgtcc atggacaaca gccgctccct ggacatggac agcatcattg
840

ctgaggtcaa ggcacagtac gaggatattg ccaaccgcag ccgggctgag gctgagagca
900

tgtaccagat caagtatgag gagctgcaga gcctggctgg gaagcacggg gatgacctgc
960

ggcgcacaaa gactgagatc tctgagatga accggaacat cagccggctc caggctgaga
1020

ttgagggcct caaaggccag agggcttccc tggaggccgc cattgcagat gccgagcagc
1080

gtggagagct ggccattaag gatgccaacg ccaagttgtc cgagctggag gccgccctgc
1140

agcgggccaa gcaggacatg gcgcggcagc tgcgtgagta ccaggagctg atgaacgtca
1200

agctggccct ggacatcgag atcgccacct acaggaagct gctggagggc gaggagagcc
1260

ggctggagtc tgggatgcag aacatgagta ttcatacgaa gaccaccagc ggctatgcag
1320

gtggtctgag ctcggcctat gggggcctca caagccccgg cctcagctac agcctgggct
1380

ccagctttgg ctctggcgcg ggctccagct ccttcagccg caccagctcc tccagggccg
1440

tggttgtgaa gaagatcgag acacgtgatg ggaagctggt gtctgagtcc tctgacgtcc
1500

tgcccaagtg aacagctgcg gcagcccctc ccagcctacc cctcctgcgc tgccccagag
1560

cctgggaagg aggccgctat gcagggtagc actgggaaca ggagacccac ctgaggctca
1620

gccctagccc tcagcccacc tggggagttt actacctggg gacccccctt gcccatgcct
1680

ccagctacaa aacaattcaa ttgctttttt tttttggtcc aaaataaaac ctcagctagc
1740

tctgccaaac cc
1752

<210> 50

<211> 1412

<212> DNA

<213> Homo sapiens

<400> 50

cggggtcgtc cgcaaagcct gagtcctgtc ctttctctct ccccggacag catgagcttc 60

accactcgct ccaccttctc caccaactac cggtccctgg gctctgtcca ggcgcccagc
120

tacggcgccc ggccggtcag cagcgcggcc agcgtctatg caggcgctgg gggctctggt
180

tcccggatct ccgtgtcccg ctccaccagc ttcaggggcg gcatggggtc cgggggcctg
240

gccaccggga tagccggggg tctggcagga atgggaggca tccagaacga gaaggagacc
300

atgcaaagcc tgaacgaccg cctggcctct tacctggaca gagtgaggag cctggagacc
360

gagaaccgga ggctggagag caaaatccgg gagcacttgg agaagaaggg accccaggtc
420

agagactgga gccattactt caagatcatc gaggacctga gggctcagat cttcgcaaat
480

actgtggaca atgcccgcat cgttctgcag attgacaatg cccgtcttgc tgctgatgac
540

tttagagtca agtatgagac agagctggcc atgcgccagt ctgtggagaa cgacatccat
600

gggctccgca aggtcattga tgacaccaat atcacacgac tgcagctgga gacagagatc
660

gaggctctca aggaggagct gctcttcatg aagaagaacc acgaagagga agtaaaaggc
720

ctacaagccc agattgccag ctctgggttg accgtggagg tagatgcccc caaatctcag
780

gacctcgcca agatcatggc agacatccgg gcccaatatg acgagctggc tcggaagaac
840

cgagaggagc tagacaagta ctggtctcag cagattgagg agagcaccac agtggtcacc
900

acacagtctg ctgaggttgg agctgctgag acgacgctca cagagctgag acgtacagtc
960

cagtccttgg agatcgacct ggactccatg agaaatctga aggccagctt ggagaacagc
1020

ctgagggagg tggaggcccg ctacgcccta cagatggagc agctcaacgg gatcctgctg
1080

caccttgagt cagagctggc acagacccgg gcagagggac agcgccaggc ccaggagtat
1140

gaggccctgc tgaacatcaa ggtcaagctg gaggctgaga tcgccaccta ccgccgcctg
1200

ctggaagatg gcgaggactt taatcttggt gatgccttgg acagcagcaa ctccatgcaa
1260

accatccaaa agaccaccac ccgccggata gtggatggca aagtggtgtc tgagaccaat
1320

gacaccaaag ttctgaggca ttaagccagc agaagcaggg taccctttgg ggagcaggag
1380

gccaataaaa agttcagagt tcattggatg tc
1412

<210> 51

<211> 1407

<212> DNA

<213> Homo sapiens

<400> 51

cgcgaatcgc agcttctgag accagggttg ctccgtccgt gctccgcctc gccatgactt 60

cctacagcta tcgccagtcg tcggccacgt cgtccttcgg aggcctgggc ggcggctccg
120

tgcgttttgg gccgggggtc gcctttcgcg cgcccagcat tcacgggggc tccggcggcc
180

gcggcgtatc cgtgtcctcc gcccgctttg tgtcctcgtc ctcctcgggg gcctacggcg
240

gcggctacgg cggcgtcctg accgcgtccg acgggctgct ggcgggcaac gagaagctaa
300

ccatgcagaa cctcaacgac cgcctggcct cctacctgga caaggtgcgc gccctggagg
360

cggccaacgg cgagctagag gtgaagatcc gcgactggta ccagaagcag gggcctgggc
420

cctcccgcga ctacagccac tactacacga ccatccagga cctgcgggac aagattcttg
480

gtgccaccat tgagaactcc aggattgtcc tgcagatcga caatgcccgt ctggctgcag
540

atgacttccg aaccaagttt gagacggaac aggctctgcg catgagcgtg gaggccgaca
600

tcaacggcct gcgcagggtg ctggatgagc tgaccctggc caggaccgac ctggagatgc
660

agatcgaagg cctgaaggaa gagctggcct acctgaagaa gaaccatgag gaggaaatca
720

gtacgctgag gggccaagtg ggaggccagg tcagtgtgga ggtggattcc gctccgggca
780

ccgatctcgc caagatcctg agtgacatgc gaagccaata tgaggtcatg gccgagcaga
840

accggaagga tgctgaagcc tggttcacca gccggactga agaattgaac cgggaggtcg
900

ctggccacac ggagcagctc cagatgagca ggtccgaggt tactgacctg cggcgcaccc
960

ttcagggtct tgagattgag ctgcagtcac agctgagcat gaaagctgcc ttggaagaca
1020

cactggcaga aacggaggcg cgctttggag cccagctggc gcatatccag gcgctgatca
1080

gcggtattga agcccagctg ggcgatgtgc gagctgatag tgagcggcag aatcaggagt
1140

accagcggct catggacatc aagtcgcggc tggagcagga gattgccacc taccgcagcc
1200

tgctcgaggg acaggaagat cactacaaca atttgtctgc ctccaaggtc ctctgaggca
1260

gcaggctctg gggcttctgc tgtcctttgg agggtgtctt ctgggtagag ggatgggaag
1320

gaagggaccc ttacccccgg ctcttctcct gacctgccaa taaaaattta tggtccaagg
1380

gaaaaaaaaa aaaaaaaaaa aaaaaaa
1407

<210> 52

<211> 1723

<212> DNA

<213> Homo sapiens

<400> 52

caaccatcct gaagctacag gtgctccctc ctggaatctc caatggattt cagtcgcaga 60

agcttccaca gaagcctgag ctcctccttg caggcccctg tagtcagtac agtgggcatg
120

cagcgcctcg ggacgacacc cagcgtttat gggggtgctg gaggccgggg catccgcatc
180

tccaactcca gacacacggt gaactatggg agcgatctca caggcggcgg ggacctgttt
240

gttggcaatg agaaaatggc catgcagaac ctaaatgacc gtctagcgag ctacctagaa
300

aaggtgcgga ccctggagca gtccaactcc aaacttgaag tgcaaatcaa gcagtggtac
360

gaaaccaacg ccccgagggc tggtcgcgac tacagtgcat attacagaca aattgaagag
420

ctgcgaagtc agattaagga tgctcaactg caaaatgctc ggtgtgtcct gcaaattgat
480

aatgctaaac tggctgctga ggacttcaga ctgaagtatg agactgagag aggaatacgt
540

ctaacagtgg aagctgatct ccaaggcctg aataaggtct ttgatgacct aaccctacat
600

aaaacagatt tggagattca aattgaagaa ctgaataaag acctagctct cctcaaaaag
660

gagcatcagg aggaagtcga tggcctacac aagcatctgg gcaacactgt caatgtggag
720

gttgatgctg ctccaggcct gaaccttggc gtcatcatga atgaaatgag gcagaagtat
780

gaagtcatgg cccagaagaa ccttcaagag gccaaagaac agtttgagag acagactgca
840

gttctgcagc aacaggtcac agtgaatact gaagaattaa aaggaactga ggttcaacta
900

acggagctga gacgcacctc ccagagcctt gagatagaac tccagtccca tctcagcatg
960

aaagagtctt tggagcacac tctagaggag accaaggccc gttacagcag ccagttagcc
1020

aacctccagt cgctgttgag ctctctggag gcccaactga tgcagattcg gagtaacatg
1080

gaacgccaga acaacgaata ccatatcctt cttgacataa agactcgact tgaacaggaa
1140

attgctactt accgccgcct tctggaagga gaagacgtaa aaactacaga atatcagtta
1200

agcaccctgg aagagagaga tataaagaaa accaggaaga ttaagacagt cgtgcaagaa
1260

gtagtggatg gcaaggtcgt gtcatctgaa gtcaaagagg tggaagaaaa tatctaaata
1320

gctaccagaa ggagatgctg ctgaggtttt gaaagaaatt tggctataat cttatctttg
1380

ctccctgcaa gaaatcagcc ataagaaagc actattaata ctctgcagtg attagaaggg
1440

gtggggtggc gggaatccta tttatcagac tctgtaattg aatataaatg ttttactcag
1500

aggagctgca aattgcctgc aaaaatgaaa tccagtgagc actagaatat ttaaaacatc
1560

attactgcca tctttatcat gaagcacatc aattacaagc tgtagaccac ctaatatcaa
1620

tttgtaggta atgttcctga aaattgcaat acatttcaat tatactaaac ctcacaaagt
1680

agaggaatcc atgtaaattg caaataaacc actttctaat ttt
1723

<210> 53

<211> 4139

<212> DNA

<213> Homo sapiens

<400> 53

ccgctccacc tctcaagcag ccagcgcctg cctgaatctg ttctgccccc tccccaccca 60

tttcaccacc accatgacac cgggcaccca gtctcctttc ttcctgctgc tgctcctcac
120

agtgcttaca gttgttacag gttctggtca tgcaagctct accccaggtg gagaaaagga
180

gacttcggct acccagagaa gttcagtgcc cagctctact gagaagaatg ctgtgagtat
240

gaccagcagc gtactctcca gccacagccc cggttcaggc tcctccacca ctcagggaca
300

ggatgtcact ctggccccgg ccacggaacc agcttcaggt tcagctgcca cctggggaca
360

ggatgtcacc tcggtcccag tcaccaggcc agccctgggc tccaccaccc cgccagccca
420

cgatgtcacc tcagccccgg acaacaagcc agccccgggc tccaccgccc ccccagccca
480

cggtgtcacc tcggccccgg acaccaggcc ggccccgggc tccaccgccc ccccagccca
540

cggtgtcacc tcggccccgg acaccaggcc ggccccgggc tccaccgccc ccccagccca
600

cggtgtcacc tcggccccgg acaccaggcc ggccccgggc tccaccgccc ccccagccca
660

cggtgtcacc tcggccccgg acaccaggcc ggccccgggc tccaccgccc ccccagccca
720

cggtgtcacc tcggccccgg acaccaggcc ggccccgggc tccaccgccc ccccagccca
780

cggtgtcacc tcggccccgg acaccaggcc ggccccgggc tccaccgccc ccccagccca
840

cggtgtcacc tcggccccgg acaccaggcc ggccccgggc tccaccgccc ccccagccca
900

cggtgtcacc tcggccccgg acaccaggcc ggccccgggc tccaccgccc ccccagccca
960

cggtgtcacc tcggccccgg acaccaggcc ggccccgggc tccaccgccc ccccagccca
1020

cggtgtcacc tcggccccgg acaccaggcc ggccccgggc tccaccgccc ccccagccca
1080

cggtgtcacc tcggccccgg acaccaggcc ggccccgggc tccaccgccc ccccagccca
1140

cggtgtcacc tcggccccgg acaccaggcc ggccccgggc tccaccgccc ccccagccca
1200

cggtgtcacc tcggccccgg acaccaggcc ggccccgggc tccaccgccc ccccagccca
1260

cggtgtcacc tcggccccgg acaccaggcc ggccccgggc tccaccgccc ccccagccca
1320

cggtgtcacc tcggccccgg acaccaggcc ggccccgggc tccaccgccc ccccagccca
1380

cggtgtcacc tcggccccgg acaccaggcc ggccccgggc tccaccgccc ccccagccca
1440

cggtgtcacc tcggccccgg acaccaggcc ggccccgggc tccaccgccc ccccagccca
1500

cggtgtcacc tcggccccgg acaccaggcc ggccccgggc tccaccgccc ccccagccca
1560

cggtgtcacc tcggccccgg acaccaggcc ggccccgggc tccaccgccc ccccagccca
1620

cggtgtcacc tcggccccgg acaccaggcc ggccccgggc tccaccgccc ccccagccca
1680

cggtgtcacc tcggccccgg acaccaggcc ggccccgggc tccaccgccc ccccagccca
1740

cggtgtcacc tcggccccgg acaccaggcc ggccccgggc tccaccgccc ccccagccca
1800

cggtgtcacc tcggccccgg acaccaggcc ggccccgggc tccaccgccc ccccagccca
1860

cggtgtcacc tcggccccgg acaccaggcc ggccccgggc tccaccgccc ccccagccca
1920

cggtgtcacc tcggccccgg acaccaggcc ggccccgggc tccaccgccc ccccagccca
1980

cggtgtcacc tcggccccgg acaccaggcc ggccccgggc tccaccgccc ccccagccca
2040

cggtgtcacc tcggccccgg acaccaggcc ggccccgggc tccaccgccc ccccagccca
2100

cggtgtcacc tcggccccgg acaccaggcc ggccccgggc tccaccgccc ccccagccca
2160

cggtgtcacc tcggccccgg acaccaggcc ggccccgggc tccaccgccc ccccagccca
2220

cggtgtcacc tcggccccgg acaccaggcc ggccccgggc tccaccgccc ccccagccca
2280

cggtgtcacc tcggccccgg acaccaggcc ggccccgggc tccaccgccc ccccagccca
2340

cggtgtcacc tcggccccgg acaccaggcc ggccccgggc tccaccgccc ccccagccca
2400

cggtgtcacc tcggccccgg acaccaggcc ggccccgggc tccaccgccc ccccagccca
2460

cggtgtcacc tcggccccgg acaccaggcc ggccccgggc tccaccgccc ccccagccca
2520

cggtgtcacc tcggccccgg acaccaggcc ggccccgggc tccaccgccc ccccagccca
2580

cggtgtcacc tcggccccgg acaccaggcc ggccccgggc tccaccgccc ccccagccca
2640

cggtgtcacc tcggccccgg acaccaggcc ggccccgggc tccaccgccc ccccagccca
2700

cggtgtcacc tcggccccgg acaccaggcc ggccccgggc tccaccgccc ccccagccca
2760

cggtgtcacc tcggccccgg acaccaggcc ggccccgggc tccaccgccc ccccagccca
2820

cggtgtcacc tcggccccgg acaccaggcc ggccccgggc tccaccgccc ccccagccca
2880

tggtgtcacc tcggccccgg acaacaggcc cgccttgggc tccaccgccc ctccagtcca
2940

caatgtcacc tcggcctcag gctctgcatc aggctcagct tctactctgg tgcacaacgg
3000

cacctctgcc agggctacca caaccccagc cagcaagagc actccattct caattcccag
3060

ccaccactct gatactccta ccacccttgc cagccatagc accaagactg atgccagtag
3120

cactcaccat agctcggtac ctcctctcac ctcctccaat cacagcactt ctccccagtt
3180

gtctactggg gtctctttct ttttcctgtc ttttcacatt tcaaacctcc agtttaattc
3240

ctctctggaa gatcccagca ccgactacta ccaagagctg cagagagaca tttctgaaat
3300

gtttttgcag atttataaac aagggggttt tctgggcctc tccaatatta agttcaggcc
3360

aggatctgtg gtggtacaat tgactctggc cttccgagaa ggtaccatca atgtccacga
3420

cgtggagaca cagttcaatc agtataaaac ggaagcagcc tctcgatata acctgacgat
3480

ctcagacgtc agcgtgagtg atgtgccatt tcctttctct gcccagtctg gggctggggt
3540

gccaggctgg ggcatcgcgc tgctggtgct ggtctgtgtt ctggttgcgc tggccattgt
3600

ctatctcatt gccttggctg tctgtcagtg ccgccgaaag aactacgggc agctggacat
3660

ctttccagcc cgggatacct accatcctat gagcgagtac cccacctacc acacccatgg
3720

gcgctatgtg ccccctagca gtaccgatcg tagcccctat gagaaggttt ctgcaggtaa
3780

cggtggcagc agcctctctt acacaaaccc agcagtggca gccgcttctg ccaacttgta
3840

gggcacgtcg ccgctgagct gagtggccag ccagtgccat tccactccac tcaggttctt
3900

caggccagag cccctgcacc ctgtttgggc tggtgagctg ggagttcagg tgggctgctc
3960

acagcctcct tcagaggccc caccaatttc tcggacactt ctcagtgtgt ggaagctcat
4020

gtgggcccct gaggctcatg cctgggaagt gttgtggggg ctcccaggag gactggccca
4080

gagagccctg agatagcggg gatcctgaac tggactgaat aaaacgtggt ctcccactg
4139

<210> 54

<211> 15720

<212> DNA

<213> Homo sapiens

<400> 54
   caacccacac cgcccctgcc agccaccatg gggctgccac tagcccgcct ggcggctgtg 60
   tgcctggccc tgtctttggc agggggctcg gagctccaga cagagggcag aacccgatac
   120
   cacggccgca acgtctgcag cacctggggc aacttccact acaagacctt cgacggggac
   180
   gtcttccgct tccccggcct ctgcgactac aacttcgcct ccgactgccg aggctcctac
   240
   aaggaatttg ctgtgcacct gaagcggggt ccgggccagg ctgaggcccc cgccggggtg
   300
   gagtccatcc tgctgaccat caaggatgac accatctacc tcacccgcca cctggctgtg
   360
   cttaacgggg ccgtggtcag caccccgcac tacagccccg ggctgctcat tgagaagagc
   420
   gatgcctaca ccaaagtcta ctcccgcgcc ggcctcaccc tcatgtggaa ccgggaggat
   480
   gcactcatgc tggagctgga cactaagttc cggaaccaca cctgtggcct ctgcggggac
   540
   tacaacggcc tgcagagcta ttcagaattc ctctctgacg gcgtgctctt cagtcccctg
   600
   gagtttggga acatgcagaa gatcaaccag cccgatgtgg tgtgtgagga tcccgaggag
   660
   gaggtggccc ccgcatcctg ctccgagcac cgcgccgagt gtgagaggct gctgaccgcc
   720
   gaggccttcg cggactgtca ggacctggtg ccgctggagc cgtatctgcg cgcctgccag
   780
   caggaccgct gccggtgccc gggcggtgac acctgcgtct gcagcaccgt ggccgagttc
   840
   tcccgccagt gctcccacgc cggcggccgg cccgggaact ggaggaccgc cacgctctgc
   900
   cccaagacct gccccgggaa cctggtgtac ctggagagcg gctcgccctg catggacacc
   960
   tgctcacacc tggaggtgag cagcctgtgc gaggagcacc gcatggacgg ctgtttctgc
   1020
   ccagaaggca ccgtatatga cgacatcggg gacagtggct gcgttcctgt gagccagtgc
   1080
   cactgcaggc tgcacggaca cctgtacaca ccgggccagg agatcaccaa tgactgcgag
   1140
   cagtgtgtct gtaacgctgg ccgctgggtg tgcaaagacc tgccctgccc cggcacctgt
   1200
   gccctggaag gcggctccca catcaccacc ttcgatggga agacgtacac cttccacggg
   1260
   gactgctact atgtcctggc caagggtgac cacaacgatt cctacgctct cctgggcgag
   1320
   ctggccccct gtggctccac agacaagcag acctgcctga agacggtggt gctgctggct
   1380
   gacaagaaga agaatgcggt ggtcttcaag tccgatggca gtgtactgct caaccagctg
   1440
   caggtgaacc tgccccacgt gaccgcgagc ttctctgtct tccgcccgtc ttcctaccac
   1500
   atcatggtga gcatggccat tggcgtccgg ctgcaggtgc agctggcccc agtcatgcaa
   1560
   ctctttgtga cactggacca ggcctcccag gggcaggtgc agggcctctg cgggaacttc
   1620
   aacggcctgg aaggtgacga cttcaagacg gccagcgggc tggtggaggc cacgggggcc
   1680
   ggctttgcca acacctggaa ggcacagtca acctgccatg acaagctgga ctggttggac
   1740
   gatccctgct ccctgaacat cgagagcgcc aactacgccg agcactggtg ctccctcctg
   1800
   aagaagacag agaccccctt tggcaggtgc cactcggctg tggaccctgc tgagtattac
   1860
   aagaggtgca aatatgacac gtgtaactgt cagaacaatg aggactgcct gtgcgccgcc
   1920
   ctgtcctcct acgcgcgcgc ctgcaccgcc aagggcgtca tgctgtgggg ctggcgggag
   1980
   catgtctgca acaaggatgt gggctcctgc cccaactcgc aggtcttcct gtacaacctg
   2040
   accacctgcc agcagacctg ccgctccctc tccgaggccg acagccactg tctcgagggc
   2100
   tttgcgcctg tggacggctg cggctgccct gaccacacct tcctggacga gaagggccgc
   2160
   tgcgtacccc tggccaagtg ctcctgttac caccgcggtc tctacctgga ggcgggggat
   2220
   gtggtcgtca ggcaggaaga acgatgtgtg tgccgggatg ggcggctgca ctgtaggcag
   2280
   atccggctga tcggccagag ctgcacggcc ccaaagatcc acatggactg cagcaacctg
   2340
   actgcactgg ccacctcgaa gccccgagcc ctcagctgcc agacgctggc cgccggctat
   2400
   taccacacag agtgtgtcag tggctgtgtg tgccccgacg ggctgatgga tgacggccgg
   2460
   ggtggctgcg tggtggagaa ggaatgccct tgcgtccata acaacgacct gtattcttcc
   2520
   ggcgccaaga tcaaggtgga ctgcaatacc tgcacctgca agagaggacg ctgggtgtgc
   2580
   acccaggctg tgtgccatgg cacctgctcc atttacggga gtggccacta catcaccttt
   2640
   gatgggaagt actacgactt tgacggacac tgctcctacg tggctgttca ggactactgc
   2700
   ggccagaact cctcactggg ctcattcagc atcatcaccg agaacgtccc ctgtggcact
   2760
   acgggcgtca cctgctccaa ggccatcaag atcttcatgg ggaggacgga gctgaagttg
   2820
   gaagacaagc accgtgtggt gatccagcgt gatgagggtc accacgtggc ctacaccacg
   2880
   cgggaggtgg gccagtacct ggtggtggag tccagcacgg gcatcatcgt catctgggac
   2940
   aagaggacca ccgtgttcat caagctggct ccctcctaca agggcaccgt gtgtggcctg
   3000
   tgtgggaact ttgaccaccg ctccaacaac gacttcacca cgcgggacca catggtggtg
   3060
   agcagcgagc tggacttcgg gaacagctgg aaggaggccc ccacctgccc agatgtgagc
   3120
   accaaccccg agccctgcag cctgaacccg caccgccgct cctgggccga gaagcagtgc
   3180
   agcatcctca aaagcagcgt gttcagcatc tgccacagca aggtggaccc caagcccttc
   3240
   tacgaggcct gtgtgcacga ctcgtgctcc tgtgacacgg gtggggactg tgagtgcttc
   3300
   tgctctgccg tggcctccta cgcccaggag tgtaccaaag agggggcctg cgtgttctgg
   3360
   aggacgccgg acctgtgccc catattctgc gactactaca accctccgca tgagtgtgag
   3420
   tggcactatg agccatgtgg gaaccggagc ttcgagacct gcaggaccat caacggcatc
   3480
   cactccaaca tctccgtgtc ctacctggag ggctgctacc cccggtgccc caaggacagg
   3540
   cccatctatg aggaggatct gaagaagtgt gtcactgcag acaagtgtgg ctgctatgtc
   3600
   gaggacaccc actacccacc tggagcatcg gttcccaccg aggagacctg caagtcctgc
   3660
   gtgtgtacca actcctccca agtcgtctgc aggccggagg aaggaaagat tcttaaccag
   3720
   acccaggatg gcgccttctg ctactgggag atctgtggcc ccaacgggac ggtggagaag
   3780
   cacttcaaca tctgttccat tacgacacgc ccgtccaccc tgaccacctt caccaccatc
   3840
   accctcccca ccacccccac ctccttcacc actaccacca ccaccaccac cccgacctcc
   3900
   agcacagttt tatcaacaac tccgaagctg tgctgcctct ggtctgactg gatcaatgag
   3960
   gaccacccca gcagtggcag cgacgacggt gaccgagaac catttgatgg ggtctgcggg
   4020
   gcccctgagg acatcgagtg caggtcggtc aaggatcccc acctcagctt ggagcagcat
   4080
   ggccagaagg tgcagtgtga tgtctctgtt gggttcattt gcaagaatga agaccagttt
   4140
   ggaaatggac catttggact gtgttacgac tacaagatac gtgtcaattg ttgctggccc
   4200
   atggataagt gtatcaccac tcccagccct ccaactacca ctcccagccc tccaccaacc
   4260
   acgacgacca cccttccacc aaccaccacc cccagccctc caaccaccac cacaaccacc
   4320
   cctccaccaa ccaccacccc cagccctcca ataaccacca cgaccacccc tctaccaacc
   4380
   accactccca gccctccaat aagcaccaca accacccctc caccaaccac cactcccagc
   4440
   cctccaacca ccactcccag ccctccaacc accactccca gccctccaac aaccaccaca
   4500
   accacccctc caccaaccac cactcccagc cctccaatga ctacgcccat cactccacca
   4560
   gccagcacta ccacccttcc accaaccacc actcccagcc ctccaacaac caccacaacc
   4620
   acccctccac caaccaccac tcccagtcct ccaacgacta cgcccatcac tccaccaacc
   4680
   agcactacta cccttccacc aaccaccact cccagccctc caccaaccac cacaaccacc
   4740
   cctccaccaa ccaccactcc cagccctcca acaaccacca ctcccagtcc tccaacaatc
   4800
   accacaacca cccctccacc aaccaccact cccagccctc caacaacgac cacaaccacc
   4860
   cctccaccaa ccaccactcc cagccctcca acgactacac ccatcactcc accaaccagc
   4920
   actaccaccc ttccaccaac caccactccc agccctccac caaccaccac aaccacccct
   4980
   ccaccaacca ccactcccag ccctccaaca accaccactc ccagccctcc aataaccacc
   5040
   acaaccaccc ctccaccaac caccactccc agctctccaa taaccaccac tcccagccct
   5100
   ccaacaacca ccatgaccac cccttcacca accaccaccc ccagctctcc aataaccacc
   5160
   acaaccaccc cttcctcaac taccactccc agccctccac caaccaccat gaccacccct
   5220
   tcaccaacca ccactcccag ccctccaaca accaccatga ccacccttcc accaaccacc
   5280
   acttccagcc ctctaacaac tactcctcta cctccatcaa taactcctcc tacattttca
   5340
   ccattctcaa cgacaacccc tactacccca tgcgtgcctc tctgcaattg gactggctgg
   5400
   ctggattctg gaaaacccaa ctttcacaaa ccaggtggag acacagaatt gattggagac
   5460
   gtctgtggac caggctgggc agctaacatc tcttgcagag ccaccatgta tcctgatgtt
   5520
   cccattggac agcttggaca aacagtggtg tgtgatgtct ctgtggggct gatatgcaaa
   5580
   aatgaagacc aaaagccagg tggggtcatc cctatggcct tctgcctcaa ctacgagatc
   5640
   aacgttcagt gctgtgagtg tgtcacccaa cccaccacca tgacaaccac caccacagag
   5700
   aacccaactc cgccaaccac gacacccatc accaccacca ctacggtgac cccaacccca
   5760
   acacccaccg gcacacagac cccaaccacg acacccatca ccaccaccac tacggtgacc
   5820
   ccaaccccaa cacccaccgg cacacagacc ccaaccacga cacccatcac caccaccact
   5880
   acggtgaccc caaccccaac acccaccggc acacagaccc caaccacgac acccatcacc
   5940
   accaccacta cggtgacccc aaccccaaca cccaccggca cacagacccc aaccacgaca
   6000
   cccatcacca ccaccactac ggtgacccca accccaacac ccaccggcac acagacccca
   6060
   accacgacac ccatcaccac caccactacg gtgaccccaa ccccaacacc caccggcaca
   6120
   cagaccccaa ccacgacacc catcaccacc accactacgg tgaccccaac cccaacaccc
   6180
   accggcacac agaccccaac cacgacaccc atcaccacca ccactacggt gaccccaacc
   6240
   ccaacaccca ccggcacaca gaccccaacc acgacaccca tcaccaccac cactacggtg
   6300
   accccaaccc caacacccac cggcacacag accccaacca cgacacccat caccaccacc
   6360
   actacggtga ccccaacccc aacacccacc ggcacacaga ccccaaccac gacacccatc
   6420
   accaccacca ctacggtgac cccaacccca acacccaccg gcacacagac cccaaccacg
   6480
   acacccatca ccaccaccac tacggtgacc ccaaccccaa cacccaccgg cacacagacc
   6540
   ccaaccacga cacccatcac caccaccact acggtgaccc caaccccaac acccaccggc
   6600
   acacagaccc caaccacgac acccatcacc accaccacta cggtgacccc aaccccaaca
   6660
   cccaccggca cacagacccc aaccacgaca cccatcacca ccaccactac ggtgacccca
   6720
   accccaacac ccaccggcac acagacccca accacgacac ccatcaccac caccactacg
   6780
   gtgaccccaa ccccaacacc caccggcaca cagaccccaa ccacgacacc catcaccacc
   6840
   accactacgg tgaccccaac cccaacaccc accggcacac agaccccaac cacgacaccc
   6900
   atcaccacca ccactacggt gaccccaacc ccaacaccca ccggcacaca gaccccaacc
   6960
   acgacaccca tcaccaccac cactacggtg accccaaccc caacacccac cggcacacag
   7020
   accccaacca cgacacccat caccaccacc actacggtga ccccaacccc aacacccacc
   7080
   ggcacacaga ccccaaccac gacacccatc accaccacca ctacggtgac cccaacccca
   7140
   acacccaccg gcacacagac cccaaccacg acacccatca ccaccaccac tacggtgacc
   7200
   ccaaccccaa cacccaccgg cacacagacc ccaaccacga cacccatcac caccaccact
   7260
   acggtgaccc caaccccaac acccaccggc acacagaccc caaccacgac acccatcacc
   7320
   accaccacta cggtgacccc aaccccaaca cccaccggca cacagacccc aaccacgaca
   7380
   cccatcacca ccaccactac ggtgacccca accccaacac ccaccggcac acagacccca
   7440
   accacgacac ccatcaccac caccactacg gtgaccccaa ccccaacacc caccggcaca
   7500
   cagaccccaa ccacgacacc catcaccacc accactacgg tgaccccaac cccaacaccc
   7560
   accggcacac agaccccaac cacgacaccc atcaccacca ccactacggt gaccccaacc
   7620
   ccaacaccca ccggcacaca gaccccaacc acgacaccca tcaccaccac cactacggtg
   7680
   accccaaccc caacacccac cggcacacag accccaacca cgacacccat caccaccacc
   7740
   actacggtga ccccaacccc aacacccacc ggcacacaga ccccaaccac gacacccatc
   7800
   accaccacca ctacggtgac cccaacccca acacccaccg gcacacagac cccaaccacg
   7860
   acacccatca ccaccaccac tacggtgacc ccaaccccaa cacccaccgg cacacagacc
   7920
   ccaaccacga cacccatcac caccaccact acggtgaccc caaccccaac acccaccggc
   7980
   acacagaccc caaccacgac acccatcacc accaccacta cggtgacccc aaccccaaca
   8040
   cccaccggca cacagacccc aaccacgaca cccatcacca ccaccactac ggtgacccca
   8100
   accccaacac ccaccggcac acagacccca accacgacac ccatcaccac caccactacg
   8160
   gtgaccccaa ccccaacacc caccggcaca cagaccccaa ccacgacacc catcaccacc
   8220
   accactacgg tgaccccaac cccaacaccc accggcacac agaccccaac cacgacaccc
   8280
   atcaccacca ccactacggt gaccccaacc ccaacaccca ccggcacaca gaccccaacc
   8340
   acgacaccca tcaccaccac cactacggtg accccaaccc caacacccac cggcacacag
   8400
   accccaacca cgacacccat caccaccacc actacggtga ccccaacccc aacacccacc
   8460
   ggcacacaga ccccaaccac gacacccatc accaccacca ctacggtgac cccaacccca
   8520
   acacccaccg gcacacagac cccaaccacg acacccatca ccaccaccac tacggtgacc
   8580
   ccaaccccaa cacccaccgg cacacagacc ccaaccacga cacccatcac caccaccact
   8640
   acggtgaccc caaccccaac acccaccggc acacagaccc caaccacgac acccatcacc
   8700
   accaccacta cggtgacccc aaccccaaca cccaccggca cacagacccc aaccacgaca
   8760
   cccatcacca ccaccactac ggtgacccca accccaacac ccaccggcac acagacccca
   8820
   accacgacac ccatcaccac caccactacg gtgaccccaa ccccaacacc caccggcaca
   8880
   cagaccccaa ccacgacacc catcaccacc accactacgg tgaccccaac cccaacaccc
   8940
   accggcacac agaccccaac cacgacaccc atcaccacca ccactacggt gaccccaacc
   9000
   ccaacaccca ccggcacaca gaccccaacc acgacaccca tcaccaccac cactacggtg
   9060
   accccaaccc caacacccac cggcacacag accccaacca cgacacccat caccaccacc
   9120
   actacggtga ccccaacccc aacacccacc ggcacacaga ccccaaccac gacacccatc
   9180
   accaccacca ctacggtgac cccaacccca acacccaccg gcacacagac cccaaccacg
   9240
   acacccatca ccaccaccac tacggtgacc ccaaccccaa cacccaccgg cacacagacc
   9300
   ccaaccacga cacccatcac caccaccact acggtgaccc caaccccaac acccaccggc
   9360
   acacagaccc caaccacgac acccatcacc accaccacta cggtgacccc aaccccaaca
   9420
   cccaccggca cacagacccc aaccacgaca cccatcacca ccaccactac ggtgacccca
   9480
   accccaacac ccaccggcac acagacccca accacgacac ccatcaccac caccactacg
   9540
   gtgaccccaa ccccaacacc caccggcaca cagaccccaa ccacgacacc catcaccacc
   9600
   accactacgg tgaccccaac cccaacaccc accggcacac agaccccaac cacgacaccc
   9660
   atcaccacca ccactacggt gaccccaacc ccaacaccca ccggcacaca gaccccaacc
   9720
   acgacaccca tcaccaccac cactacggtg accccaaccc caacacccac cggcacacag
   9780
   accccaacca cgacacccat caccaccacc actacggtga ccccaacccc aacacccacc
   9840
   ggcacacaga ccccaaccac gacacccatc accaccacca ctacggtgac cccaacccca
   9900
   acacccaccg gcacacagac cccaaccacg acacccatca ccaccaccac tacggtgacc
   9960
   ccaaccccaa cacccaccgg cacacagacc ccaaccacga cacccatcac caccaccact
   10020
   acggtgaccc caaccccaac acccaccggc acacagaccc caaccacgac acccatcacc
   10080
   accaccacta cggtgacccc aaccccaaca cccaccggca cacagacccc aaccacgaca
   10140
   cccatcacca ccaccactac ggtgacccca accccaacac ccaccggcac acagacccca
   10200
   accacgacac ccatcaccac caccactacg gtgaccccaa ccccaacacc caccggcaca
   10260
   cagaccccaa ccacgacacc catcaccacc accactacgg tgaccccaac cccaacaccc
   10320
   accggcacac agaccccaac cacgacaccc atcaccacca ccactacggt gaccccaacc
   10380
   ccaacaccca ccggcacaca gaccccaacc acgacaccca tcaccaccac cactacggtg
   10440
   accccaaccc caacacccac cggcacacag accccaacca cgacacccat caccaccacc
   10500
   actacggtga ccccaacccc aacacccacc ggcacacaga ccccaaccac gacacccatc
   10560
   accaccacca ctacggtgac cccaacccca acacccaccg gcacacagac cccaaccacg
   10620
   acacccatca ccaccaccac tacggtgacc ccaaccccaa cacccaccgg cacacagacc
   10680
   ccaaccacga cacccatcac caccaccact acggtgaccc caaccccaac acccaccggc
   10740
   acacagaccc caaccacgac acccatcacc accaccacta cggtgacccc aaccccaaca
   10800
   cccaccggca cacagacccc aaccacgaca cccatcacca ccaccactac ggtgacccca
   10860
   accccaacac ccaccggcac acagacccca accacgacac ccatcaccac caccactacg
   10920
   gtgaccccaa ccccaacacc caccggcaca cagaccccaa ccacgacacc catcaccacc
   10980
   accactacgg tgaccccaac cccaacaccc accggcacac agaccccaac cacgacaccc
   11040
   atcaccacca ccactacggt gaccccaacc ccaacaccca ccggcacaca gaccccaacc
   11100
   acgacaccca tcaccaccac cactacggtg accccaaccc caacacccac cggcacacag
   11160
   accccaacca cgacacccat caccaccacc actacggtga ccccaacccc aacacccacc
   11220
   ggcacacaga ccccaaccac gacacccatc accaccacca ctacggtgac cccaacccca
   11280
   acacccaccg gcacacagac cccaaccacg acacccatca ccaccaccac tacggtgacc
   11340
   ccaaccccaa cacccaccgg cacacagacc ccaaccacga cacccatcac caccaccact
   11400
   acggtgaccc caaccccaac acccaccggc acacagaccc caaccacgac acccatcacc
   11460
   accaccacta cggtgacccc aaccccaaca cccaccggca cacagacccc aaccacgaca
   11520
   cccatcacca ccaccactac ggtgacccca accccaacac ccaccggcac acagacccca
   11580
   accacgacac ccatcaccac caccactacg gtgaccccaa ccccaacacc caccggcaca
   11640
   cagaccccaa ccacgacacc catcaccacc accactacgg tgaccccaac cccaacaccc
   11700
   accggcacac agaccccaac cacgacaccc atcaccacca ccactacggt gaccccaacc
   11760
   ccaacaccca ccggcacaca gaccccaacc acgacaccca tcaccaccac cactacggtg
   11820
   accccaaccc caacacccac cggcacacag accccaacca cgacacccat caccaccacc
   11880
   actacggtga ccccaacccc aacacccacc ggcacacaga ccccaaccac gacacccatc
   11940
   accaccacca ctacggtgac cccaacccca acacccaccg gcacacagac cccaaccacg
   12000
   acacccatca ccaccaccac tacggtgacc ccaaccccaa cacccaccgg cacacagacc
   12060
   ccaaccacga cacccatcac caccaccact acggtgaccc caaccccaac acccaccggc
   12120
   acacagaccc caaccacgac acccatcacc accaccacta cggtgacccc aaccccaaca
   12180
   cccaccggca cacagacccc aaccacgaca cccatcacca ccaccactac ggtgacccca
   12240
   accccaacac ccaccggcac acagacccca accacgacac ccatcaccac caccactacg
   12300
   gtgaccccaa ccccaacacc caccggcaca cagaccccaa ccacgacacc catcaccacc
   12360
   accactacgg tgaccccaac cccaacaccc accggcacac agaccccaac cacgacaccc
   12420
   atcaccacca ccactacggt gaccccaacc ccaacaccca ccggcacaca gaccccaacc
   12480
   acgacaccca tcaccaccac cactacggtg accccaaccc caacacccac cggcacacag
   12540
   accccaacca cgacacccat caccaccacc actacggtga ccccaacccc aacacccacc
   12600
   ggcacacaga ccgggccccc cacccacaca agcacagcac cgattgctga gttgaccaca
   12660
   tccaatcctc cgcctgagtc ctcaacccct cagacctctc ggtccacctc ttcccctctc
   12720
   acggagtcaa ccacccttct gagtacccta ccacctgcca ttgagatgac cagcacggcc
   12780
   ccaccctcca cacccacggc acccacgacc acgagcggag gccacacact gtctccaccg
   12840
   cccagcacca ccacgtcccc tccaggcacc cccactcgcg gtaccacgac cgggtcatct
   12900
   tcagccccca cccccagcac tgtgcagacg accaccacca gtgcctggac cccaacgccg
   12960
   accccactct ccacacccag catcatcagg accacaggcc tgaggcccta cccttcctct
   13020
   gtgcttatct gctgtgtcct gaacgacacc tactacgcac caggtgagga ggtgtacaac
   13080
   ggcacatacg gagacacctg ttatttcgtc aactgctcac tgagctgtac gttggagttc
   13140
   tataactggt cctgcccatc cacgccctcc ccaacaccca cgccctccaa gtcgacgccc
   13200
   acgccttcca agccatcgtc cacgccctcc aagccgacgc ccggcaccaa gccccccgag
   13260
   tgcccagact ttgatcctcc cagacaggag aacgagactt ggtggctgtg cgactgcttc
   13320
   atggccacgt gcaagtacaa caacacggtg gagatcgtga aggtggagtg tgagccgccg
   13380
   cccatgccca cctgctccaa cggcctccaa cccgtgcgcg tcgaggaccc cgacggctgc
   13440
   tgctggcact gggagtgcga ctgctactgc acgggctggg gcgacccgca ctatgtcacc
   13500
   ttcgacggac tctactacag ctaccagggc aactgcacct acgtgctggt ggaggagatc
   13560
   agcccctccg tggacaactt cggagtttac atcgacaact accactgcga tcccaacgac
   13620
   aaggtgtcct gtccccgcac cctcatcgtg cgccacgaga cccaggaggt gctgatcaag
   13680
   accgtgcata tgatgcccat gcaggtgcag gtgcaggtga acaggcaggc ggtggcactg
   13740
   ccctacaaga agtacgggct ggaggtgtac cagtctggca tcaactacgt ggtggacatc
   13800
   cccgagctgg gtgtcctcgt ctcctacaat ggcctgtcct tctccgtcag gctgccctac
   13860
   caccggtttg gcaacaacac caagggccag tgtggcacct gcaccaacac cacctccgac
   13920
   gactgcattc tgcccagcgg ggagatcgtc tccaactgtg aggctgcggc tgaccagtgg
   13980
   ctggtgaacg acccctccaa gccacactgc ccccacagca gctccacgac caagcgcccg
   14040
   gccgtcactg tgcccggggg cggtaaaacg accccacaca aggactgcac cccatctccc
   14100
   ctctgccagc tcatcaagga cagcctgttt gcccagtgcc acgcactggt gcccccgcag
   14160
   cactactacg atgcctgcgt gttcgacagc tgcttcatgc cgggctcgag cctggagtgc
   14220
   gccagtctgc aggcctacgc agccctctgt gcccagcaga acatctgcct cgactggcgg
   14280
   aaccacacgc atggggcctg cttggtggag tgcccatctc acagggagta ccaggcctgt
   14340
   ggccctgcag aagagcccac gtgcaaatcc agctcctccc agcagaacaa cacagtcctg
   14400
   gtggaaggct gcttctgtcc tgagggcacc atgaactacg ctcctggctt tgatgtctgc
   14460
   gtgaagacct gcggctgtgt gggacctgac aatgtgccca gagagtttgg ggagcacttc
   14520
   gagttcgact gcaagaactg tgtctgcctg gagggtggaa gtggcatcat ctgccaaccc
   14580
   aagaggtgca gccagaagcc cgttacccac tgcgtggaag acggcaccta cctcgccacg
   14640
   gaggtcaacc ctgccgacac ctgctgcaac attaccgtct gcaagtgcaa caccagcctg
   14700
   tgcaaagaga agccctccgt gtgcccgctg ggattcgaag tgaagagcaa gatggtgcct
   14760
   ggaaggtgct gtcccttcta ctggtgtgag tccaaggggg tgtgtgttca cgggaatgct
   14820
   gagtaccagc ccggttctcc agtttattcc tccaagtgcc aggactgcgt gtgcacggac
   14880
   aaggtggaca acaacaccct gctcaacgtc atcgcctgca cccacgtgcc ctgcaacacc
   14940
   tcctgcagcc ctggcttcga actcatggag gcccccgggg agtgctgtaa gaagtgtgaa
   15000
   cagacgcact gtatcatcaa acggcccgac aaccagcacg tcatcctgaa gcccggggac
   15060
   ttcaagagcg acccgaagaa caactgcaca ttcttcagct gcgtgaagat ccacaaccag
   15120
   ctcatctcgt ccgtctccaa catcacctgc cccaactttg atgccagcat ttgcatcccg
   15180
   ggctccatca cattcatgcc caatggatgc tgcaagacct gcacccctcg caatgagacc
   15240
   agggtgccct gctccaccgt ccccgtcacc acggaggttt cgtacgccgg ctgcaccaag
   15300
   accgtcctca tgaatcattg ctccgggtcc tgcgggacat ttgtcatgta ctcggccaag
   15360
   gcccaggccc tggaccacag ctgctcctgc tgcaaagagg agaaaaccag ccagcgtgag
   15420
   gtggtcctga gctgccccaa tggcggctcg ctgacacaca cctacaccca catcgagagc
   15480
   tgccagtgcc aggacaccgt ctgcgggctc cccaccggca cctcccgccg ggcccggcgc
   15540
   tcccctaggc atctggggag cgggtgagcg gggtgggcac agcccccttc actgccctcg
   15600
   acagctttac ctcccccgga ccctctgagc ctcctaagct cggcttcctc tcttcagata
   15660
   tttattgtct gagtctttgt tcagtccttg ctttccaata ataaactcag ggggacatgc
   15720

<210> 55

<211> 4707

<212> DNA

<213> Homo sapiens

<400> 55
   gatcaccatc accgagacca cctcacacag tactcccagc tacactacct caatcaccac 60
   caccgagacc ccctcacaca gtactcccag ctacactacc tcaatcacca ccaccgagac
   120
   cccatcacac agtactccca gcttcacttc ttcaatcacc accaccgaga ccacatccca
   180
   cagtactccc agcttcactt cttcaatcag gaccaccgag accacatcct acagtactcc
   240
   cagcttcact tcttcaaata ccatcactga gaccacctca cacagtactc ccagctacat
   300
   tacctcaatc accaccaccg agaccccctc aagcagtact cccagcttca gttcttcgat
   360
   caccaccact gagaccacat cccacagtac tcccggcttc acttcttcaa tcaccaccac
   420
   tgagactaca tcccacagta ctcccagctt cacttcttcg atcaccacca ctgagaccac
   480
   ctcacatgat actcccagct tcacttcttc aatcaccacc agtgagaccc cctcacacag
   540
   tactcccagc tccacttctt taatcaccac caccaagacc acctcacaca gtactcccag
   600
   cttcacttct tcgatcacca ccaccgagac cacctcacac agtgctcgca gcttcacttc
   660
   ttcgatcacc accaccgaga ccacctcaca caatactcgg agcttcactt cttcgatcac
   720
   caccaccgag accaactctc acagtactac cagcttcact tcttcgatca ccaccaccga
   780
   gaccacctca cacagtactc ccagcttcag ttcttcaatc accaccactg agaccccctt
   840
   acacagtact cctggcctac cttcgtgggt caccaccacc aagaccacct cacacattac
   900
   tcctggcctc acttcttcaa tcaccaccac tgagactacc tcacacagta ctcccggctt
   960
   cacttcttca atcaccacca ctgagaccac ctcagagagt actcccagcc tcagttcttc
   1020
   aaccatctac tccacagtca gcacatccac aactgccatc acctcacatt ttactacctc
   1080
   agagactgcg gtgactccca cacctgtaac cccatcttct ctgagtacag acatcccgac
   1140
   cacaagccta cgaactctca ccccttcgtc tgtgggcacc agcacttcat tgactacaac
   1200
   cacagacttt ccctctatac ccactgatat cagtacctta ccaactcgaa cacacatcat
   1260
   ttcatcttct ccctccatcc aaagtacaga aacctcatcc cttgtgggca ccacctctcc
   1320
   caccatgtcc actgtgagaa tgaccctcag aattactgag aacaccccaa tcagttcctt
   1380
   tagcacaagt attgttgtta tacctgaaac cccaacacag acccctcctg tactgacgtc
   1440
   agccactggg acccaaacat ctcctgcacc tactactgtc acctttggaa gtacggattc
   1500
   ctccacgtcc actcttcata ctcttactcc atcaacagcc ttgagcacga tcgtgtcaac
   1560
   atcacaggtt cctattccta gcacacattc ctccaccctt caaacaactc cttctactcc
   1620
   ctcattgcaa acttcactca catctacaag tgagttcact acagaatctt tcactagggg
   1680
   aagtacgtct acaaatgcaa tcttgacttc ttttagtacc atcatctggt cctcaacacc
   1740
   cactattatc atgtcctctt ctccatcttc tgccagcata actccagtgt tctccactac
   1800
   cattcattct gttccttctt caccatacat tttcagtaca gaaaatgtgg gctccgcttc
   1860
   tatcacaggc tttcctagtc tctcttcctc tgcaactacc agcacttctt caaccagctc
   1920
   ctctctgacc acagctctca ctgaaataac ccccttttct tatatttccc ttccctccac
   1980
   cacaccctgt ccaggaacta taacaattac catagtccct gcctctccca ctgatccatg
   2040
   tgttgaaatg gatcccagca ctgaagctac ttctcctccc accaccccat taacagtctt
   2100
   tccctttact accgaaatgg tcacctgtcc tacctccatc agtatccaaa ctactcttac
   2160
   tacatatatg gacacttctt ccatgatgcc agaaagtgag tccagcatct cacccaatgc
   2220
   ttccagttcc actggcactg ggactgtacc cacaaacaca gttttcacaa gtactcgact
   2280
   gcccaccagt gagacctggc tgagcaacag ttctgtgatc cccctacctc ttcctggcgt
   2340
   ctctaccatc ccgctcacca tgaaaccaag cagtagcctc ccgaccatcc tgaggacttc
   2400
   aagcaagtca acacacccat ccccacccac cactaggact tcagagacac cagtggccac
   2460
   tacccagact cctaccaccc ttacatcacg caggacaact cgcatcactt ctcagatgac
   2520
   cacacagtcc acgttgacca ccactgcagg cacctgtgac aatggtggca cctgggaaca
   2580
   gggccagtgt gcttgccttc cggggttttc tggggaccgc tgtcagctcc agaccagatg
   2640
   ccagaatggg ggtcagtggg atggcctcaa atgccagtgc cccagcacct tctatggttc
   2700
   cagttgtgag tttgctgtgg aacaggtgga tctagatgca gaagattttt gcagacatgc
   2760
   agggcttcac cttcaagggt gtggagatcc tgtccctgag gaatggcagc atcgtggtgg
   2820
   actacctggt cctgctggag atgcccttca gcccccagct ggagagcgag tatgagcagg
   2880
   tgaagaccac gctgaaggag gggctgcaga acgccagcca ggatgtgaac agctgccagg
   2940
   actcccagac cctgtgtttt aagcctgact ccatcaaggt gaacaacaac agcaagacag
   3000
   agctgacccc ggcagccatc tgccgcgcgc cgctcccacg ggctatgaag agttctactt
   3060
   ccccttggtg gaggccaccc ggctccgctg tgtcaccaaa tgcacgtctg gggtggacaa
   3120
   cgccatcgac tgtcaccagg gccagtgcgt tctggagacg agcggtccca cgtgtcgctg
   3180
   ctactccacc gacacgcact ggttctctgg cccgcgctgc gaggtggccg tccactggag
   3240
   ggcgctggtc ggggcctgac ggccggcgcg cgctgctggt gctgctgctc gtggcgctgg
   3300
   gcgtccgggc ggtgcgctcc ggatggtggg gcggccagcg ccgaggccgg tcctgggacc
   3360
   aggacaggaa atggttcgag acctgggatg aggaagtcgt gggcactttt tcaaactggg
   3420
   gtttcgagga cgacggaaca gacaaggata caaatttcta tgtggccttg gagaacgtga
   3480
   caccactatg aaggtgcaca tcaagagacc cgagatgacc tcgtcctcag tgtgagcctg
   3540
   cggggcccct tcaccacccc ctccgccctg ccccggacac aagggtctgc attgcgtcca
   3600
   tttcaagagg tgaccccagg acgcgggcag cccaggctcc tgctgttctt gggcaagatg
   3660
   agactgttcc cccaaatccc atccttctcc ttccaacttg gctgaaaccc acctggagac
   3720
   gcagttcacg tccaggctct tccactgtgg aatcttgggc aagtcagtaa cgagcctcag
   3780
   tttcctcacc tgcaaaacgg gtacagcatt cctgtatgat acgtcacgcc gttgttgtga
   3840
   aaaccacata gacttggtca attctcggtc ctactctgcc ctcccgtctc agccctcgtg
   3900
   ttgccattgc ctctctcgga tcctccaatc ctcacgtcct tcacctggtc tctggccctg
   3960
   gttcttattt tctctcaatt ccctactgcc tgtttcttac tttgaacctg gaggcagcct
   4020
   gcagccccat cccatctcct gccctctcct gatctaactc cctgctgcat ctcttgctcc
   4080
   cattccttag acgtcctccc cttttgaccc cgttccttca tccatcctgc accccagtcc
   4140
   cccagcccta aatcctccct cctctcctca catcctggcc cctagcaagg tatagatagc
   4200
   ctctgtgtct taggataccc cgggtgctgt tccctcggtc atcctgttgc ccagttcccc
   4260
   gtttctcttg ctctcattcc tgtatccttt ccccttttga gcccgtccat tcatcggttc
   4320
   tgcccccgac tcccccagcc ctaaataccc cagctgctgt tccccccatc accctgctgc
   4380
   ccaattcttt attctccacc cctttctctc acccctggag ccctgcgggt gggggcaggg
   4440
   catgagttcc ccagtcccca aggaaaggca gccccctcag tctccctcct cctcattccc
   4500
   ttccatctcc ctcccctctg ccttttaaac ccatcccctc cgattcccct cctcccccct
   4560
   ctctccctgg tgtcacctgg attcctgcag taattctgag cccttgaaat cctcagtcgc
   4620
   cttggcgggg aagattggct ttggggacag gaggtcggca catctccagg tcttcatgtg
   4680
   cgcaatatag agtttattgt aaaaagc
   4707

<210> 56

<211> 4151

<212> DNA

<213> Homo sapiens

<400> 56

gggacagggc actcttcccc gccgtccaca caatgagtgt tggccggagg aagctggccc 60

tgctctgggc cctggctctc gctctggcct gcacccggca tacaggccat gcccaggatg
120

gctcctccga atccagctac aagcaccacc ctgccctctc tcctatcgcc cgggggccca
180

tcggggtccc gctccgtggg gcgactgtct tcccatctct gaggaccatc cctgtggtac
240

gagcctccaa cccggcgcac aacgggcggg tgtgcagcac ctggggcagc ttccactaca
300

agaccttcga cggcgacgtc ttccgcttcc ccggcctctg caactacgtg ttctccgagc
360

actgcggtgc cgcctacgag gattttaaca tcccagctac gccgcagcca ggagtcagcg
420

gcccccacgc tgagcagggt cctcatgaag gtggatggcg tggtcatcca gctgaccaag
480

ggctccgtcc tggtcaacgg ccacccggtc ctgctgccct tcagccagtc tggggtcctc
540

attcagcaag agcagcagct acaccaaggt ggaagccagg ctgggccttg tcctcatgtg
600

gaaccacgat gacagcctgc tgctggaagc tggacaccaa atacgccaac aagaacctgt
660

gggctctgtg gggacttcaa cgggatgccc gtggtcagcg agctcctctc ccacaacacc
720

aagctgacac ccatggaatt cgggaacctg ccgaaagatg gacgaaccca cggagcagtg
780

tcaggaccct gtccctgaac ccccgaagaa ctgctccact ggctttggca tcctgtgagg
840

agctcctgca cggccagctg ttctctggct gcgtggccct ggtggacgtc ggcagctacc
900

ttgccacgcg gagccggtct gtggtggggg acgtgctgga gtttgggaac agctggaagc
3240

tctccccctc ctgcccagat gccctggcgc ccaaggaccc ctgcacggcc aaccccttcc
3300

gcaagtcctg ggcccagaag cagtgcagca tcctccacgg ccccaccttc gccgcctgcc
3360

acgcacacgt ggagccggcc aggtactacg aggcctgcgt gaacgacgcg tgcgcctgcg
3420

actccggggg tgactgcgag tgcttctgca cggctgtggc ccgctacgcc caggcctgcc
3480

atgaagtagg cacctgtgtg tgtctgcgga ccccaagcat ctgccctctg ttctgcgact
3540

actacaaccc cgaaggccag tgcgagtggc actaccagcc ctgcggggtg ccctgcctgc
3600

gcacctgccg gaacccccgt ggagactgcc tgcgggacgt ccggggcctg gaaggctgct
3660

accccaagtg cccaccagag gctcccatct ttgatgagga caagatgcag tgtgtggcca
3720

cctgcccaac cccgcctctg ccaccacggt gccacgtcca tgggaagtcc taccggccag
3780

gtgcagtggt gccctcggac aagaactgcc agtcctgcct ttgtacggag cgcggcgtgg
3840

agtgcaccta caaagctgag gcctgtgtct gcacctacaa tggacagcgc ttccacccag
3900

gggacgtcat ctaccacacg acggatggca cgggtggctg catctccgcc cgctgcgggg
3960

ccaacggcac cattgagagg agggtctacc cctgcagccc caccacccct gtccccccaa
4020

ccaccttctc cttctccaca cccccgcttg tcgtgagctc cacgcacacc cccagcaatg
4080

gcccaagcag cgcgcacaca ggccctccga gcagcgcctg gcccaccaca gcaggcactt
4140

ctcccaggac g
4151

<210> 57

<211> 880

<211> 5532

<212> DNA

<213> Homo sapiens

<400> 58

gccgcgctgc gccggagtcc cgagctagcc ccggcgccgc cgccgcccag accggacgac 60

aggccacctc gtcggcgtcc gcccgagtcc ccgcctcgcc gccaacgcca caaccaccgc
120

gcacggcccc ctgactccgt ccagtattga tcgggagagc cggagcgagc tcttcgggga
180

gcagcgatgc gaccctccgg gacggccggg gcagcgctcc tggcgctgct ggctgcgctc
240

tgcccggcga gtcgggctct ggaggaaaag aaagtttgcc aaggcacgag taacaagctc
300

acgcagttgg gcacttttga agatcatttt ctcagcctcc agaggatgtt caataactgt
360

gaggtggtcc ttgggaattt ggaaattacc tatgtgcaga ggaattatga tctttccttc
420

ttaaagacca tccaggaggt ggctggttat gtcctcattg ccctcaacac agtggagcga
480

attcctttgg aaaacctgca gatcatcaga ggaaatatgt actacgaaaa ttcctatgcc
540

ttagcagtct tatctaacta tgatgcaaat aaaaccggac tgaaggagct gcccatgaga
600

aatttacagg aaatcctgca tggcgccgtg cggttcagca acaaccctgc cctgtgcaac
660

gtggagagca tccagtggcg ggacatagtc agcagtgact ttctcagcaa catgtcgatg
720

gacttccaga accacctggg cagctgccaa aagtgtgatc caagctgtcc caatgggagc
780

tgctggggtg caggagagga gaactgccag aaactgacca aaatcatctg tgcccagcag
840

tgctccgggc gctgccgtgg caagtccccc agtgactgct gccacaacca gtgtgctgca
900

ggctgcacag gcccccggga gagcgactgc ctggtctgcc gcaaattccg agacgaagcc
960

acgtgcaagg acacctgccc cccactcatg ctctacaacc ccaccacgta ccagatggat
1020

gtgaaccccg agggcaaata cagctttggt gccacctgcg tgaagaagtg tccccgtaat
1080

tatgtggtga cagatcacgg ctcgtgcgtc cgagcctgtg gggccgacag ctatgagatg
1140

gaggaagacg gcgtccgcaa gtgtaagaag tgcgaagggc cttgccgcaa agtgtgtaac
1200

ggaataggta ttggtgaatt taaagactca ctctccataa atgctacgaa tattaaacac
1260

ttcaaaaact gcacctccat cagtggcgat ctccacatcc tgccggtggc atttaggggt
1320

gactccttca cacatactcc tcctctggat ccacaggaac tggatattct gaaaaccgta
1380

aaggaaatca cagggttttt gctgattcag gcttggcctg aaaacaggac ggacctccat
1440

gcctttgaga acctagaaat catacgcggc aggaccaagc aacatggtca gttttctctt
1500

gcagtcgtca gcctgaacat aacatccttg ggattacgct ccctcaagga gataagtgat
1560

ggagatgtga taatttcagg aaacaaaaat ttgtgctatg caaatacaat aaactggaaa
1620

aaactgtttg ggacctccgg tcagaaaacc aaaattataa gcaacagagg tgaaaacagc
1680

tgcaaggcca caggccaggt ctgccatgcc ttgtgctccc ccgagggctg ctggggcccg
1740

gagcccaggg actgcgtctc ttgccggaat gtcagccgag gcagggaatg cgtggacaag
1800

tgcaagcttc tggagggtga gccaagggag tttgtggaga actctgagtg catacagtgc
1860

cacccagagt gcctgcctca ggccatgaac atcacctgca caggacgggg accagacaac
1920

tgtatccagt gtgcccacta cattgacggc ccccactgcg tcaagacctg cccggcagga
1980

gtcatgggag aaaacaacac cctggtctgg aagtacgcag acgccggcca tgtgtgccac
2040

ctgtgccatc caaactgcac ctacggatgc actgggccag gtcttgaagg ctgtccaacg
2100

aatgggccta agatcccgtc catcgccact gggatggtgg gggccctcct cttgctgctg
2160

gtggtggccc tggggatcgg cctcttcatg cgaaggcgcc acatcgttcg gaagcgcacg
2220

ctgcggaggc tgctgcagga gagggagctt gtggagcctc ttacacccag tggagaagct
2280

cccaaccaag ctctcttgag gatcttgaag gaaactgaat tcaaaaagat caaagtgctg
2340

ggctccggtg cgttcggcac ggtgtataag ggactctgga tcccagaagg tgagaaagtt
2400

aaaattcccg tcgctatcaa ggaattaaga gaagcaacat ctccgaaagc caacaaggaa
2460

atcctcgatg aagcctacgt gatggccagc gtggacaacc cccacgtgtg ccgcctgctg
2520

ggcatctgcc tcacctccac cgtgcaactc atcacgcagc tcatgccctt cggctgcctc
2580

ctggactatg tccgggaaca caaagacaat attggctccc agtacctgct caactggtgt
2640

gtgcagatcg caaagggcat gaactacttg gaggaccgtc gcttggtgca ccgcgacctg
2700

gcagccagga acgtactggt gaaaacaccg cagcatgtca agatcacaga ttttgggctg
2760

gccaaactgc tgggtgcgga agagaaagaa taccatgcag aaggaggcaa agtgcctatc
2820

aagtggatgg cattggaatc aattttacac agaatctata cccaccagag tgatgtctgg
2880

agctacgggg tgaccgtttg ggagttgatg acctttggat ccaagccata tgacggaatc
2940

cctgccagcg agatctcctc catcctggag aaaggagaac gcctccctca gccacccata
3000

tgtaccatcg atgtctacat gatcatggtc aagtgctgga tgatagacgc agatagtcgc
3060

ccaaagttcc gtgagttgat catcgaattc tccaaaatgg cccgagaccc ccagcgctac
3120

cttgtcattc agggggatga aagaatgcat ttgccaagtc ctacagactc caacttctac
3180

cgtgccctga tggatgaaga agacatggac gacgtggtgg atgccgacga gtacctcatc
3240

ccacagcagg gcttcttcag cagcccctcc acgtcacgga ctcccctcct gagctctctg
3300

agtgcaacca gcaacaattc caccgtggct tgcattgata gaaatgggct gcaaagctgt
3360

cccatcaagg aagacagctt cttgcagcga tacagctcag accccacagg cgccttgact
3420

gaggacagca tagacgacac cttcctccca gtgcctgaat acataaacca gtccgttccc
3480

aaaaggcccg ctggctctgt gcagaatcct gtctatcaca atcagcctct gaaccccgcg
3540

cccagcagag acccacacta ccaggacccc cacagcactg cagtgggcaa ccccgagtat
3600

ctcaacactg tccagcccac ctgtgtcaac agcacattcg acagccctgc ccactgggcc
3660

cagaaaggca gccaccaaat tagcctggac aaccctgact accagcagga cttctttccc
3720

aaggaagcca agccaaatgg catctttaag ggctccacag ctgaaaatgc agaataccta
3780

agggtcgcgc cacaaagcag tgaatttatt ggagcatgac cacggaggat agtatgagcc
3840

ctaaaaatcc agactctttc gatacccagg accaagccac agcaggtcct ccatcccaac
3900

agccatgccc gcattagctc ttagacccac agactggttt tgcaacgttt acaccgacta
3960

gccaggaagt acttccacct cgggcacatt ttgggaagtt gcattccttt gtcttcaaac
4020

tgtgaagcat ttacagaaac gcatccagca agaatattgt ccctttgagc agaaatttat
4080

ctttcaaaga ggtatatttg aaaaaaaaaa aaaaagtata tgtgaggatt tttattgatt
4140

ggggatcttg gagtttttca ttgtcgctat tgatttttac ttcaatgggc tcttccaaca
4200

aggaagaagc ttgctggtag cacttgctac cctgagttca tccaggccca actgtgagca
4260

aggagcacaa gccacaagtc ttccagagga tgcttgattc cagtggttct gcttcaaggc
4320

ttccactgca aaacactaaa gatccaagaa ggccttcatg gccccagcag gccggatcgg
4380

tactgtatca agtcatggca ggtacagtag gataagccac tctgtccctt cctgggcaaa
4440

gaagaaacgg aggggatgaa ttcttcctta gacttacttt tgtaaaaatg tccccacggt
4500

acttactccc cactgatgga ccagtggttt ccagtcatga gcgttagact gacttgtttg
4560

tcttccattc cattgttttg aaactcagta tgccgcccct gtcttgctgt catgaaatca
4620

gcaagagagg atgacacatc aaataataac tcggattcca gcccacattg gattcatcag
4680

catttggacc aatagcccac agctgagaat gtggaatacc taaggataac accgcttttg
4740

ttctcgcaaa aacgtatctc ctaatttgag gctcagatga aatgcatcag gtcctttggg
4800

gcatagatca gaagactaca aaaatgaagc tgctctgaaa tctcctttag ccatcacccc
4860

aaccccccaa aattagtttg tgttacttat ggaagatagt tttctccttt tacttcactt
4920

caaaagcttt ttactcaaag agtatatgtt ccctccaggt cagctgcccc caaacccct
4980

ccttacgctt tgtcacacaa aaagtgtctc tgccttgagt catctattca agcacttaca
5040

gctctggcca caacagggca ttttacaggt gcgaatgaca gtagcattat gagtagtgtg
5100

aattcaggta gtaaatatga aactagggtt tgaaattgat aatgctttca caacatttgc
5160

agatgtttta gaaggaaaaa agttccttcc taaaataatt tctctacaat tggaagattg
5220

gaagattcag ctagttagga gcccattttt tcctaatctg tgtgtgccct gtaacctgac
5280

tggttaacag cagtcctttg taaacagtgt tttaaactct cctagtcaat atccacccca
5340

tccaatttat caaggaagaa atggttcaga aaatattttc agcctacagt tatgttcagt
5400

cacacacaca tacaaaatgt tccttttgct tttaaagtaa tttttgactc ccagatcagt
5460

cagagcccct acagcattgt taagaaagta tttgattttt gtctcaatga aaataaaact
5520

atattcattt cc
5532

<210> 59

<211> 4530

<212> DNA

<213> Homo sapiens

<400> 59

aattctcgag ctcgtcgacc ggtcgacgag ctcgagggtc gacgagctcg agggcgcgcg 60

cccggccccc acccctcgca gcaccccgcg ccccgcgccc tcccagccgg gtccagccgg
120

agccatgggg ccggagccgc agtgagcacc atggagctgg cggccttgtg ccgctggggg
180

ctcctcctcg ccctcttgcc ccccggagcc gcgagcaccc aagtgtgcac cggcacagac
240

atgaagctgc ggctccctgc cagtcccgag acccacctgg acatgctccg ccacctctac
300

cagggctgcc aggtggtgca gggaaacctg gaactcacct acctgcccac caatgccagc
360

ctgtccttcc tgcaggatat ccaggaggtg cagggctacg tgctcatcgc tcacaaccaa
420

gtgaggcagg tcccactgca gaggctgcgg attgtgcgag gcacccagct ctttgaggac
480

aactatgccc tggccgtgct agacaatgga gacccgctga acaataccac ccctgtcaca
540

ggggcctccc caggaggcct gcgggagctg cagcttcgaa gcctcacaga gatcttgaaa
600

ggaggggtct tgatccagcg gaacccccag ctctgctacc aggacacgat tttgtggaag
660

gacatcttcc acaagaacaa ccagctggct ctcacactga tagacaccaa ccgctctcgg
720

gcctgccacc cctgttctcc gatgtgtaag ggctcccgct gctggggaga gagttctgag
780

gattgtcaga gcctgacgcg cactgtctgt gccggtggct gtgcccgctg caaggggcca
840

ctgcccactg actgctgcca tgagcagtgt gctgccggct gcacgggccc caagcactct
900

gactgcctgg cctgcctcca cttcaaccac agtggcatct gtgagctgca ctgcccagcc
960

ctggtcacct acaacacaga cacgtttgag tccatgccca atcccgaggg ccggtataca
1020

ttcggcgcca gctgtgtgac tgcctgtccc tacaactacc tttctacgga cgtgggatcc
1080

tgcaccctcg tctgccccct gcacaaccaa gaggtgacag cagaggatgg aacacagcgg
1140

tgtgagaagt gcagcaagcc ctgtgcccga gtgtgctatg gtctgggcat ggagcacttg
1200

cgagaggtga gggcagttac cagtgccaat atccaggagt ttgctggctg caagaagatc
1260

tttgggagcc tggcatttct gccggagagc tttgatgggg acccagcctc caacactgcc
1320

ccgctccagc cagagcagct ccaagtgttt gagactctgg aagagatcac aggttaccta
1380

tacatctcag catggccgga cagcctgcct gacctcagcg tcttccagaa cctgcaagta
1440

atccggggac gaattctgca caatggcgcc tactcgctga ccctgcaagg gctgggcatc
1500

agctggctgg ggctgcgctc actgagggaa ctgggcagtg gactggccct catccaccat
1560

aacacccacc tctgcttcgt gcacacggtg ccctgggacc agctctttcg gaacccgcac
1620

caagctctgc tccacactgc caaccggcca gaggacgagt gtgtgggcga gggcctggcc
1680

tgccaccagc tgtgcgcccg agggcactgc tggggtccag ggcccaccca gtgtgtcaac
1740

tgcagccagt tccttcgggg ccaggagtgc gtggaggaat gccgagtact gcaggggctc
1800

cccagggagt atgtgaatgc caggcactgt ttgccgtgcc accctgagtg tcagccccag
1860

aatggctcag tgacctgttt tggaccggag gctgaccagt gtgtggcctg tgcccactat
1920

aaggaccctc ccttctgcgt ggcccgctgc cccagcggtg tgaaacctga cctctcctac
1980

atgcccatct ggaagtttcc agatgaggag ggcgcatgcc agccttgccc catcaactgc
2040

acccactcct gtgtggacct ggatgacaag ggctgccccg ccgagcagag agccagccct
2100

ctgacgtcca tcgtctctgc ggtggttggc attctgctgg tcgtggtctt gggggtggtc
2160

tttgggatcc tcatcaagcg acggcagcag aagatccgga agtacacgat gcggagactg
2220

ctgcaggaaa cggagctggt ggagccgctg acacctagcg gagcgatgcc caaccaggcg
2280

cagatgcgga tcctgaaaga gacggagctg aggaaggtga aggtgcttgg atctggcgct
2340

tttggcacag tctacaaggg catctggatc cctgatgggg agaatgtgaa aattccagtg
2400

gccatcaaag tgttgaggga aaacacatcc cccaaagcca acaaagaaat cttagacgaa
2460

gcatacgtga tggctggtgt gggctcccca tatgtctccc gccttctggg catctgcctg
2520

acatccacgg tgcagctggt gacacagctt atgccctatg gctgcctctt agaccatgtc
2580

cgggaaaacc gcggacgcct gggctcccag gacctgctga actggtgtat gcagattgcc
2640

aaggggatga gctacctgga ggatgtgcgg ctcgtacaca gggacttggc cgctcggaac
2700

gtgctggtca agagtcccaa ccatgtcaaa attacagact tcgggctggc tcggctgctg
2760

gacattgacg agacagagta ccatgcagat gggggcaagg tgcccatcaa gtggatggcg
2820

ctggagtcca ttctccgccg gcggttcacc caccagagtg atgtgtggag ttatggtgtg
2880

actgtgtggg agctgatgac ttttggggcc aaaccttacg atgggatccc agcccgggag
2940

atccctgacc tgctggaaaa gggggagcgg ctgccccagc cccccatctg caccattgat
3000

gtctacatga tcatggtcaa atgttggatg attgactctg aatgtcggcc aagattccgg
3060

gagttggtgt ctgaattctc ccgcatggcc agggaccccc agcgctttgt ggtcatccag
3120

aatgaggact tgggcccagc cagtcccttg gacagcacct tctaccgctc actgctggag
3180

gacgatgaca tgggggacct ggtggatgct gaggagtatc tggtacccca gcagggcttc
3240

ttctgtccag accctgcccc gggcgctggg ggcatggtcc accacaggca ccgcagctca
3300

tctaccagga gtggcggtgg ggacctgaca ctagggctgg agccctctga agaggaggcc
3360

cccaggtctc cactggcacc ctccgaaggg gctggctccg atgtatttga tggtgacctg
3420

ggaatggggg cagccaaggg gctgcaaagc ctccccacac atgaccccag ccctctacag
3480

cggtacagtg aggaccccac agtacccctg ccctctgaga ctgatggcta cgttgccccc
3540

ctgacctgca gcccccagcc tgaatatgtg aaccagccag atgttcggcc ccagccccct
3600

tcgccccgag agggccctct gcctgctgcc cgacctgctg gtgccactct ggaaagggcc
3660

aagactctct ccccagggaa gaatggggtc gtcaaagacg tttttgcctt tgggggtgcc
3720

gtggagaacc ccgagtactt gacaccccag ggaggagctg cccctcagcc ccaccctcct
3780

cctgccttca gcccagcctt cgacaacctc tattactggg accaggaccc accagagcgg
3840

ggggctccac ccagcacctt caaagggaca cctacggcag agaacccaga gtacctgggt
3900

ctggacgtgc cagtgtgaac cagaaggcca agtccgcaga agccctgatg tgtcctcagg
3960

gagcagggaa ggcctgactt ctgctggcat caagaggtgg gagggccctc cgaccacttc
4020

caggggaacc tgccatgcca ggaacctgtc ctaaggaacc ttccttcctg cttgagttcc
4080

cagatggctg gaaggggtcc agcctcgttg gaagaggaac agcactgggg agtctttgtg
4140

gattctgagg ccctgcccaa tgagactcta gggtccagtg gatgccacag cccagcttgg
4200

ccctttcctt ccagatcctg ggtactgaaa gccttaggga agctggcctg agaggggaag
4260

cggccctaag ggagtgtcta agaacaaaag cgacccattc agagactgtc cctgaaacct
4320

agtactgccc cccatgagga aggaacagca atggtgtcag tatccaggct ttgtacagag
4380

tgcttttctg tttagttttt actttttttg ttttgttttt ttaaagacga aataaagacc
4440

caggggagaa tgggtgttgt atggggaggc aagtgtgggg ggtccttctc cacacccact
4500

ttgtccattt gcaaatatat tttggaaaac
4530

<210> 60

<211> 801

<212> DNA

<213> Homo sapiens

<400> 60

caccgcaccc tcggactgcc ccaaggcccc cgccgccgct ccagcgccgc gcagccaccg 60

ccgccgccgc cgcctctcct tagtcgccgc catgacgacc gcgtccacct cgcaggtgcg
120

ccagaactac caccaggact cagaggccgc catcaaccgc cagatcaacc tggagctcta
180

cgcctcctac gtttacctgt ccatgtctta ctactttgac cgcgatgatg tggctttgaa
240

gaactttgcc aaatactttc ttcaccaatc tcatgaggag agggaacatg ctgagaaact
300

gatgaagctg cagaaccaac gaggtggccg aatcttcctt caggatatca agaaaccaga
360

ctgtgatgac tgggagagcg ggctgaatgc aatggagtgt gcattacatt tggaaaaaaa
420

tgtgaatcag tcactactgg aactgcacaa actggccact gacaaaaatg acccccattt
480

gtgtgacttc attgagacac attacctgaa tgagcaggtg aaagccatca aagaattggg
540

tgaccacgtg accaacttgc gcaagatggg agcgcccgaa tctggcttgg cggaatatct
600

ctttgacaag cacacctggg agacagtgat aatgaaagct aagcctcggg ctaatttccc
660

atagccgtgg ggtgacttcc tggtcaccaa ggcagtgcat gcatgttggg gtttccttta
720

ccttttctat aagttgtacc aaaacatcca cttaagttct ttgatttgta ccattccttc
780

aaataaagaa atttggtacc c
801

<210> 61

<211> 878

<212> DNA

<213> Homo sapiens

<400> 61

gtcccgcggg tctgtctctt gcttcaacag tgtttggacg gaacagatcc ggggactctc 60

ttccagcctc cgaccgccct ccgatttcct ctccgcttgc aacctccggg accatcttct
120

cggccatctc ctgcttctgg gacctgccag caccgttttt gtggttagct ccttcttgcc
180

aaccaaccat gagctcccag attcgtcaga attattccac cgacgtggag gcagccgtca
240

acagcctggt caatttgtac ctgcaggcct cctacaccta cctctctctg ggcttctatt
300

tcgaccgcga tgatgtggct ctggaaggcg tgagccactt cttccgcgaa ttggccgagg
360

agaagcgcga gggctacgag cgtctcctga agatgcaaaa ccagcgtggc ggccgcgctc
420

tcttccagga catcaagaag ccagctgaag atgagtgggg taaaacccca gacgccatga
480

aagctgccat ggccctggag aaaaagctga accaggccct tttggatctt catgccctgg
540

gttctgcccg cacggacccc catctctgtg acttcctgga gactcacttc ctagatgagg
600

aagtgaagct tatcaagaag atgggtgacc acctgaccaa cctccacagg ctgggtggcc
660

cggaggctgg gctgggcgag tatctcttcg aaaggctcac tctcaagcac gactaagagc
720

cttctgagcc cagcgacttc tgaagggccc cttgcaaagt aatagggctt ctgcctaagc
780

ctctccctcc agccaatagg cagctttctt aactatccta acaagccttg gaccaaatgg
840

aaataaagct tttgatgca aaaaaaaaa aaaaaaa
878

<210> 62

<211> 2747

<212> DNA

<213> Homo sapiens

<400> 62
   catactccat gcccagaatt cctgcctcgc cactgtcctg ctgccctcca gacatgctgg 60
   ggccctgcat gctgctgctg ctgctgctgc tgggcctgag gctacagctc tccctgggca
   120
   tcatcctagt tgaggaggag aacccggact tctggaaccg cgaggcagcc gaggccctgg
   180
   gtgccgccaa gaagctgcag cctgcacaga cagccgccaa gaacctcatc atcttcctgg
   240
   gcgatgggat gggggtgtct acggtgacag ctgccaggat cctaaaaggg cagaagaagg
   300
   acaaactggg gcctgagata cccctggcca tggaccgctt cccatatgtg gctctgtcca
   360
   agacatacaa tgtagacaaa catgtgccag acagtggagc cacagccacg gcctacctgt
   420
   gcggggtcaa gggcaacttc cagaccattg gcttgagtgc agccgcccgc tttaaccagt
   480
   gcaacacgac acgcggcaac gaggtcatct ccgtgatgaa tcgggccaag aaagcaggga
   540
   agtcagtggg agtggtaacc accacacgag tgcagcacgc ctcgccagcc ggcacctacg
   600
   cccacacggt gaaccgcaac tggtactcgg acgccgacgt gcctgcctcg gcccgccagg
   660
   aggggtgcca ggacatcgct acgcagctca tctccaacat ggacattgac gtgatcctag
   720
   gtgggggccg aaagtacatg tttcgcatgg gaaccccaga ccctgagtac ccagatgact
   780
   acagccaagg tgggaccagg ctggacggga agaatctggt gcaggaatgg ctggcgaagc
   840
   gccagggtgc ccggtacgtg tggaaccgca ctgagctcat gcaggcttcc ctggacccgt
   900
   ctgtggccca tctcatgggt ctctttgagc ctggagacat gaaatacgag atccaccgag
   960
   actccacact ggacccctcc ctgatggaga tgacagaggc tgccctgcgc ctgctgagca
   1020
   ggaacccccg cggcttcttc ctcttcgtgg agggtggtcg catcgaccat ggtcatcatg
   1080
   aaagcagggc ttaccgggca ctgactgaga cgatcatgtt cgacgacgcc attgagaggg
   1140
   cgggccagct caccagcgag gaggacacgc tgagcctcgt cactgccgac cactcccacg
   1200
   tcttctcctt cggaggctac cccctgcgag ggagctccat cttcgggctg gcccctggca
   1260
   aggcccggga caggaaggcc tacacggtcc tcctatacgg aaacggtcca ggctatgtgc
   1320
   tcaaggacgg cgcccggccg gatgttaccg agagcgagag cgggagcccc gagtatcggc
   1380
   agcagtcagc agtgcccctg gacgaagaga cccacgcagg cgaggacgtg gcggtgttcg
   1440
   cgcgcggccc gcaggcgcac ctggttcacg gcgtgcagga gcagaccttc atagcgcacg
   1500
   tcatggcctt cgccgcctgc ctggagccct acaccgcctg cgacctggcg ccccccgccg
   1560
   gcaccaccga cgccgcgcac ccggggcggt ccgtggtccc cgcgttgctt cctctgctgg
   1620
   ccgggaccct gctgctgctg gagacggcca ctgctccctg agtgtcccgt ccctggggct
   1680
   cctgcttccc catcccggag ttctcctgct ccccgcctcc tgtcgtcctg cctggcctcc
   1740
   agcccgagtc gtcatccccg gagtccctat acagaggtcc tgccatggaa ccttcccctc
   1800
   cccgtgcgct ctggggactg agcccatgac accaaacctg ccccttggct gctctcggac
   1860
   tccctacccc aaccccaggg actgcaggtt gtgccctgtg gctgcctgca ccccaggaaa
   1920
   ggagggggct caggccatcc agccaccacc tacagcccag tgggtaccag gcaggctccc
   1980
   ttcctgggga aaagaagcac ccagaccccg cgccccgctg atctttgctt cagtccttga
   2040
   atcacctgtg ggacttgagg actcgggatc ttcaggacgc ctggagaagg gtggtttcct
   2100
   gccaccctgc tggccaagga ggctcctggg gtggggatca ccagggggat tttgacacag
   2160
   ccttcggctg ccccccacta agttaattcc acacccctgt accccccagg gggccctctg
   2220
   cctcatggca aaggcttgcc ccaaatctca acttctcaga cgttccatac ccccacatgc
   2280
   caatttcagc acccaactga gatccgagga gctcctggga agccctgggt gcaggacact
   2340
   ggtcgagagc caaaggtccc tccccagaca tctggacact gggcatagat ttctcaagaa
   2400
   ggaagactcc cctgcctccc cagggcctct gctctcctgg gagacaaagc aataataaaa
   2460
   ggaagtgttt gtaatcccag cactttggga ggccgaggtg ggcggatcac gaggtcagga
   2520
   gatggagacc atcctggcta acacggtgaa accccttatc tatgcgcctg tagtcccagc
   2580
   tacccaggag gctgaagcag gataatcgct tgaacccggg cggcggagat tgcagtgagc
   2640
   cgaggtcatg ccactgcact gcagcctggg cgacagagcg agattctgcc tcaaaaataa
   2700
   acaaataaat tttaaaaata aataaataat aaaaggaagt gttagac
   2747

<210> 63

<211> 2062

<212> DNA

<213> Homo sapiens

<400> 63

gtcagtccct cctgtagccg ccgccgccgc cgcccgccgc ccctctgcca gcagctccgg 60

cgccacctcg ggccggcgtc tccggcgggc gggagccagg cgctgacggg cgcggcgggg
120

gcggccgagc gctcctgcgg ctgcgactca ggctccggcg tctgcgcttc cccatggggc
180

tggcctgcgg cgcctgggcg ctctgagatt gtcactgctg ttccaagggc acacgcagag
240

ggatttggaa ttcctggaga gttgcctttg tgagaagctg gaaatatttc tttcaattcc
300

atctcttagt tttccatagg aacatcaaga aatcatgaac aactttggta atgaagagtt
360

tgactgccac ttcctcgatg aaggttttac tgccaaggac attctggacc agaaaattaa
420

tgaagtttct tcttctgatg ataaggatgc cttctatgtg gcagacctgg gagacattct
480

aaagaaacat ctgaggtggt taaaagctct ccctcgtgtc accccctttt atgcagtcaa
540

atgtaatgat agcaaagcca tcgtgaagac ccttgctgct accgggacag gatttgactg
600

tgctagcaag actgaaatac agttggtgca gagtctgggg gtgcctccag agaggattat
660

ctatgcaaat ccttgtaaac aagtatctca aattaagtat gctgctaata atggagtcca
720

gatgatgact tttgatagtg aagttgagtt gatgaaagtt gccagagcac atcccaaagc
780

aaagttggtt ttgcggattg ccactgatga ttccaaagca gtctgtcgtc tcagtgtgaa
840

attcggtgcc acgctcagaa ccagcaggct ccttttggaa cgggcgaaag agctaaatat
900

cgatgttgtt ggtgtcagct tccatgtagg aagcggctgt accgatcctg agaccttcgt
960

gcaggcaatc tctgatgccc gctgtgtttt tgacatgggg gctgaggttg gtttcagcat
1020

gtatctgctt gatattggcg gtggctttcc tggatctgag gatgtgaaac ttaaatttga
1080

agagatcacc ggcgtaatca acccagcgtt ggacaaatac tttccgtcag actctggagt
1140

gagaatcata gctgagcccg gcagatacta tgttgcatca gctttcacgc ttgcagttaa
1200

tatcattgcc aagaaaattg tattaaagga acagacgggc tctgatgacg aagatgagtc
1260

gagtgagcag acctttatgt attatgtgaa tgatggcgtc tatggatcat ttaattgcat
1320

actctatgac cacgcacatg taaagcccct tctgcaaaag agacctaaac cagatgagaa
1380

gtattattca tccagcatat ggggaccaac atgtgatggc ctcgatcgga ttgttgagcg
1440

ctgtgacctg cctgaaatgc atgtgggtga ttggatgctc tttgaaaaca tgggcgctta
1500

cactgttgct gctgcctcta cgttcaatgg cttccagagg ccgacgatct actatgtgat
1560

gtcagggcct gcgtggcaac tcatgcagca attccagaac cccgacttcc cacccgaagt
1620

agaggaacag gatgccagca ccctgcctgt gtcttgtgcc tgggagagtg ggatgaaacg
1680

ccacagagca gcctgtgctt cggctagtat taatgtgtag atagcactct ggtagctgtt
1740

aactgcaagt ttagcttgaa ttaagggatt tggggggacc atgtaactta attactgcta
1800

gttttgaaat gtctttgtaa gagtagggtc gccatgatgc agccatatgg aagactagga
1860

tatgggtcac acttatctgt gttcctatgg aaactatttg aatatttgtt ttatatggat
1920

ttttattcac tcttcagaca cgctactcaa gagtgcccct cagctgctga acaagcattt
1980

gtagcttgta caatggcaga atgggccaaa agcttagtgt tgtgacctgt ttttaaaata
2040

aagtatcttg aaataattag gc
2062

<210> 64

<211> 3557

<212> DNA

<213> Homo sapiens

<400> 64

gagagggtcc ttcagggtct gcttatgccc ttgttcaaga acaccagtgt cagctctctg 60

tactctggtt gcagactgac cttgctcagg cctgagaagg atggggcagc caccagagtg
120

gatgctgtct gcacccatcg tcctgacccc aaaagccctg gactggacag agagcggctg
180

tactggaagc tgagccagct gacccacggc atcactgagc tgggccccta caccctggac
240

aggcacagtc tctatgtcaa tggtttcacc catcagagct ctatgacgac caccagaact
300

cctgatacct ccacaatgca cctggcaacc tcgagaactc cagcctccct gtctggacct
360

acgaccgcca gccctctcct ggtgctattc acaattaact tcaccatcac taacctgcgg
420

tatgaggaga acatgcatca ccctggctct agaaagttta acaccacgga gagagtcctt
480

cagggtctgc tcaggcctgt gttcaagaac accagtgttg gccctctgta ctctggctgc
540

agactgacct tgctcaggcc caagaaggat ggggcagcca ccaaagtgga tgccatctgc
600

acctaccgcc ctgatcccaa aagccctgga ctggacagag agcagctata ctgggagctg
660

agccagctaa cccacagcat cactgagctg ggcccctaca ccctggacag ggacagtctc
720

tatgtcaatg gtttcacaca gcggagctct gtgcccacca ctagcattcc tgggaccccc
780

acagtggacc tgggaacatc tgggactcca gtttctaaac ctggtccctc ggctgccagc
840

cctctcctgg tgctattcac tctcaacttc accatcacca acctgcggta tgaggagaac
900

atgcagcacc ctggctccag gaagttcaac accacggaga gggtccttca gggcctgctc
960

aggtccctgt tcaagagcac cagtgttggc cctctgtact ctggctgcag actgactttg
1020

ctcaggcctg aaaaggatgg gacagccact ggagtggatg ccatctgcac ccaccaccct
1080

gaccccaaaa gccctaggct ggacagagag cagctgtatt gggagctgag ccagctgacc
1140

cacaatatca ctgagctggg ccactatgcc ctggacaacg acagcctctt tgtcaatggt
1200

ttcactcatc ggagctctgt gtccaccacc agcactcctg ggacccccac agtgtatctg
1260

ggagcatcta agactccagc ctcgatattt ggcccttcag ctgccagcca tctcctgata
1320

ctattcaccc tcaacttcac catcactaac ctgcggtatg aggagaacat gtggcctggc
1380

tccaggaagt tcaacactac agagagggtc cttcagggcc tgctaaggcc cttgttcaag
1440

aacaccagtg ttggccctct gtactctggc tccaggctga ccttgctcag gccagagaaa
1500

gatggggaag ccaccggagt ggatgccatc tgcacccacc gccctgaccc cacaggccct
1560

gggctggaca gagagcagct gtatttggag ctgagccagc tgacccacag catcactgag
1620

ctgggcccct acacactgga cagggacagt ctctatgtca atggtttcac ccatcggagc
1680

tctgtaccca ccaccagcac cggggtggtc agcgaggagc cattcacact gaacttcacc
1740

atcaacaacc tgcgctacat ggcggacatg ggccaacccg gctccctcaa gttcaacatc
1800

acagacaacg tcatgaagca cctgctcagt cctttgttcc agaggagcag cctgggtgca
1860

cggtacacag gctgcagggt catcgcacta aggtctgtga agaacggtgc tgagacacgg
1920

gtggacctcc tctgcaccta cctgcagccc ctcagcggcc caggtctgcc tatcaagcag
1980

gtgttccatg agctgagcca gcagacccat ggcatcaccc ggctgggccc ctactctctg
2040

gacaaagaca gcctctacct taacggttac aatgaacctg gtctagatga gcctcctaca
2100

actcccaagc cagccaccac attcctgcct cctctgtcag aagccacaac agccatgggg
2160

taccacctga agaccctcac actcaacttc accatctcca atctccagta ttcaccagat
2220

atgggcaagg gctcagctac attcaactcc accgaggggg tccttcagca cctgctcaga
2280

cccttgttcc agaagagcag catgggcccc ttctacttgg gttgccaact gatctccctc
2340

aggcctgaga aggatggggc agccactggt gtggacacca cctgcaccta ccaccctgac
2400

cctgtgggcc ccgggctgga catacagcag ctttactggg agctgagtca gctgacccat
2460

ggtgtcaccc aactgggctt ctatgtcctg gacagggata gcctcttcat caatggctat
2520

gcaccccaga atttatcaat ccggggcgag taccagataa atttccacat tgtcaactgg
2580

aacctcagta atccagaccc cacatcctca gagtacatca ccctgctgag ggacatccag
2640

gacaaggtca ccacactcta caaaggcagt caactacatg acacattccg cttctgcctg
2700

gtcaccaact tgacgatgga ctccgtgttg gtcactgtca aggcattgtt ctcctccaat
2760

ttggacccca gcctggtgga gcaagtcttt ctagataaga ccctgaatgc ctcattccat
2820

tggctgggct ccacctacca gttggtggac atccatgtga cagaaatgga gtcatcagtt
2880

tatcaaccaa caagcagctc cagcacccag cacttctacc cgaatttcac catcaccaac
2940

ctaccatatt cccaggacaa agcccagcca ggcaccacca attaccagag gaacaaaagg
3000

aatattgagg atgcgctcaa ccaactcttc cgaaacagca gcatcaagag ttatttttct
3060

gactgtcaag tttcaacatt caggtctgtc cccaacaggc accacaccgg ggtggactcc
3120

ctgtgtaact tctcgccact ggctcggaga gtagacagag ttgccatcta tgaggaattt
3180

ctgcggatga cccggaatgg tacccagctg cagaacttca ccctggacag gagcagtgtc
3240

cttgtggatg ggtattctcc caacagaaat gagcccttaa ctgggaattc tgaccttccc
3300

ttctgggctg tcatcttcat cggcttggca ggactcctgg gactcatcac atgcctgatc
3360

tgcggtgtcc tggtgaccac ccgccggcgg aagaaggaag gagaatacaa cgtccagcaa
3420

cagtgcccag gctactacca gtcacaccta gacctggagg atctgcaatg actggaactt
3480

gccggtgcct ggggtgcctt tcccccagcc agggtccaaa gaagcttggc tggggcagaa
3540

ataaaccata ttggtcg
3557

<210> 65

<211> 3464

<212> DNA

<213> Homo sapiens

<400> 65
   cagccgtgct cgaagcgttc ctggagccca agctctcctc cacaggtgaa gacagggcca 60
   gcaggagaca ccatggggca cctctcagcc ccacttcaca gagtgcgtgt accctggcag
   120
   gggcttctgc tcacagcctc acttctaacc ttctggaacc cgcccaccac tgcccagctc
   180
   actactgaat ccatgccatt caatgttgca gaggggaagg aggttcttct ccttgtccac
   240
   aatctgcccc agcaactttt tggctacagc tggtacaaag gggaaagagt ggatggcaac
   300
   cgtcaaattg taggatatgc aataggaact caacaagcta ccccagggcc cgcaaacagc
   360
   ggtcgagaga caatataccc caatgcatcc ctgctgatcc agaacgtcac ccagaatgac
   420
   acaggattct acaccctaca agtcataaag tcagatcttg tgaatgaaga agcaactgga
   480
   cagttccatg tataccggga gctgcccaag ccctccatct ccagcaacaa ctccaaccct
   540
   gtggaggaca aggatgctgt ggccttcacc tgtgaacctg agactcagga cacaacctac
   600
   ctgtggtgga taaacaatca gagcctcccg gtcagtccca ggctgcagct gtccaatggc
   660
   aacaggaccc tcactctact cagtgtcaca aggaatgaca caggacccta tgagtgtgaa
   720
   atacagaacc cagtgagtgc gaaccgcagt gacccagtca ccttgaatgt cacctatggc
   780
   ccggacaccc ccaccatttc cccttcagac acctattacc gtccaggggc aaacctcagc
   840
   ctctcctgct atgcagcctc taacccacct gcacagtact cctggcttat caatggaaca
   900
   ttccagcaaa gcacacaaga gctctttatc cctaacatca ctgtgaataa tagtggatcc
   960
   tatacctgcc acgccaataa ctcagtcact ggctgcaaca ggaccacagt caagacgatc
   1020
   atagtcactg agctaagtcc agtagtagca aagccccaaa tcaaagccag caagaccaca
   1080
   gtcacaggag ataaggactc tgtgaacctg acctgctcca caaatgacac tggaatctcc
   1140
   atccgttggt tcttcaaaaa ccagagtctc ccgtcctcgg agaggatgaa gctgtcccag
   1200
   ggcaacacca ccctcagcat aaaccctgtc aagagggagg atgctgggac gtattggtgt
   1260
   gaggtcttca acccaatcag taagaaccaa agcgacccca tcatgctgaa cgtaaactat
   1320
   aatgctctac cacaagaaaa tggcctctca cctggggcca ttgctggcat tgtgattgga
   1380
   gtagtggccc tggttgctct gatagcagta gccctggcat gttttctgca tttcgggaag
   1440
   accggcaggg caagcgacca gcgtgatctc acagagcaca aaccctcagt ctccaaccac
   1500
   actcaggacc actccaatga cccacctaac aagatgaatg aagttactta ttctaccctg
   1560
   aactttgaag cccagcaacc cacacaacca acttcagcct ccccatccct aacagccaca
   1620
   gaaataattt attcagaagt aaaaaagcag taatgaaacc tgtcctgctc actgcagtgc
   1680
   tgatgtattt caagtctctc accctcatca ctaggagatt cctttcccct ctagggtaga
   1740
   ggggtgggga cagaaacaac tttctcctac tcttccttcc taataggcat ctccaggctg
   1800
   cctggtcact gcccctctct cagtgtcaat agatgaaagt acattgggag tctgtaggaa
   1860
   acccaacctt cttgtcattg aaatttggca aagctgactt tgggaaagag ggaccagaac
   1920
   ttcccctccc ttcccctttt cccaacctgg acttgtttta aacttgcctg ttcagagcac
   1980
   tcattccttc ccacccccag tcctgtccta tcactctaat tcggatttgc catagccttg
   2040
   aggttatgtc cttttccatt aagtacatgt gccaggaaac agcgagagag agaaagtaaa
   2100
   cggcagtaat gcttctccta tttctccaaa gccttgtgtg aactagcaaa gagaagaaaa
   2160
   ccaaatatat aaccaatagt gaaatgccac aggtttgtcc actgtcaggg ttgtctacct
   2220
   gtaggatcag ggtctaagca ccttggtgct tagctagaat accacctaat ccttctggca
   2280
   agcctgtctt cagagaaccc actagaagca actaggaaaa atcacttgcc aaaatccaag
   2340
   gcaattcctg atggaaaatg caaaagcaca tatatgtttt aatatcttta tgggctctgt
   2400
   tcaaggcagt gctgagaggg aggggttata gcttcaggag ggaaccagct tctgataaac
   2460
   acaatctgct aggaacttgg gaaaggaatc agagagctgc ccttcagcga ttatttaaat
   2520
   tattgttaaa gaatacacaa tttggggtat tgggattttt ctccttttct ctgagacatt
   2580
   ccaccatttt aatttttgta actgcttatt tatgtgaaaa gggttatttt tacttagctt
   2640
   agctatgtca gccaatccga ttgccttagg tgaaagaaac caccgaaatc cctcaggtcc
   2700
   cttggtcagg agcctctcaa gatttttttt gtcagaggct ccaaatagaa aataagaaaa
   2760
   ggttttcttc attcatggct agagctagat ttaactcagt ttctaggcac ctcagaccaa
   2820
   tcatcaacta ccattctatt ccatgtttgc acctgtgcat tttctgtttg cccccattca
   2880
   ctttgtcagg aaaccttggc ctctgctaag gtgtatttgg tccttgagaa gtgggagcac
   2940
   cctacaggga cactatcact catgctggtg gcattgttta cagctagaaa gctgcactgg
   3000
   tgctaatgcc ccttgggaaa tggggctgtg aggaggagga ttataactta ggcctagcct
   3060
   cttttaacag cctctgaaat ttatcttttc ttctatgggg cttataaatg tatcttataa
   3120
   taaaaaggaa ggacaggagg aagacaggca aatgtacttc tcacccagtc ttctacacag
   3180
   atggaatctc tttggggcta agagaaaggt tttattctat attgcttacc tgatctcatg
   3240
   ttaggcctaa gaggctttct ccaggaggat tagcttggag ttctctatac tcaggtacct
   3300
   ctttcagggt tttctaaccc tgacacggac tgtgcatact ttccctcatc catgctgtgc
   3360
   tgtgttattt aatttttcct ggctaagatc atgtctgaat tatgtatgaa aattattcta
   3420
   tgtttttata ataaaaataa tatatcagac atcgaaaaaa aaaa
   3464

<210> 66

<211> 1022

<212> DNA

<213> Homo sapiens

<400> 66

tcctggagcc caggctcttt tccacagagg aggaaagagc aggcagcaga gaccatgggg 60

cccccctcag cccctcccca cagagaatgc atcccctggc aggggcttct gctcacagcc
120

tcacttctaa acttctggaa cccgcccacc actgccaagc tcactattga atccatgccg
180

ctcagtgtcg cagaggggaa ggaggtgctt ctacttgtcc acaatctgcc ccagcatctt
240

tttggctaca gctggtacaa aggggaaaga gtggatggca acagtctaat tgtgggatat
300

gtaataggaa ctcaacaagc taccccaggg gccgcataca gcggtcgaga gacaatatac
360

accaatgcat ccctgctgat ccagaatgtc acccagaatg acataggatt ctacacccta
420

caagtcataa agtcagatct tgtgaatgaa gaagcaactg gacagttcca tgtataccaa
480

gaaaatgccc caggccttcc tgtgggggcc gtcgccggca tcgtgaccgg ggtcctggtc
540

ggagtggcgc tggtggccgc gctggtgtgt ttcctgctcc ttgccaaaac tggaaggccg
600

tggtccctcc cacagctctg ccttctcgat gtcccctctc tccactgcct aggcccccct
660

acccaacccc aggacagcag cttccatcta tgagaagtgg cttcttagct tcctccagga
720

gctgctcctg tgggttgatg gagagtcccc aaggccccca gccctgggga tggggaagga
780

catgaagcct gagccagaga accagctata agtcctgaga agacactggt gtctggggac
840

agggagggat ggggtccctg atgaatatct ggagacctcg acagcctgcc ctaggccctg
900

ggtgggtcag gacaaaggcc tctcatcacc gcagaaagcg ggggcttgca gggaaagtga
960

atgggcctgt ggcccacctg gggtcacttg gaaaggatct gaataaaggg gacccttcct
1020

cg
1022

<210> 67

<211> 1190

<212> DNA

<213> Homo sapiens

<400> 67

gtcagcagcc ccgacagccg acagtcacag cagctctgac aagagcgttc ctggagccca 60

gctcctctcc acagaccaca agcacccagc agagaccatg ggccccccct cagccgctcc
120

ccgtggaggg cacaggccct ggcaggggct cctgatcaca gcctcacttt taaccttctg
180

ggacccgccc accactgtcc agttcactat tgaagccctg ccatccagtg ctgcagaggg
240

aaaggatgtt cttctactgg cctgcaatat ttcagaaact attcaagcct attattggca
300

caaggggaaa acggcagaag ggagccctct cattgctggt tatataacag acattcaagc
360

aaatatccca ggggccgcat acagtggtcg agagcaagta taccccaatg gatccctgct
420

gttccaaaac atcaccctgg aggacgcagg atcctacacc ctacgaacca taaatgccag
480

ttacgactct gaccaagcaa ctggccagct ccacgtacac caaaacaacg tcccaggcct
540

tcctgtgggg gccgtcgctg gcatcgtgac tggggtcctg gttggggtgg ctctggtggc
600

cgccctggtg tgttttctgc ttctctccag gactggaagg gccagcatcc agcgtgacct
660

cagggagcag ccgcccccag cctccacccc tggccatggt ccctctcaca gatccacctt
720

ctcggcccct ctacccagcc ccagaacagc cactcccatc tatgtggaat ttctatactc
780

tgatgcaaac atttactgcc agatcgacca caaagcagat gtggtctctt aggttcctct
840

gggagctgct cttgtgggtt gatggagcgt cctcgaagct ccagccctgg ggacggggaa
900

ggacatggag cctgagccag agaaccagct ctgagtcctg aggagacaca ggcctgggga
960

cagggaggga tgggagtccc tgctgaatat ctggagaccc tgacaggttg ccctgggctc
1020

cgggtgggcc gggacaaagg cctctcatca ccacaggaag cgggggcttg caaggaaagt
1080

gaatgggcct gtggcccacc cggggtcacc aggaaaggat ctgaataaag aggacccttc
1140

ctctcattgg ctctttttct gctcacggga acttagcaga aactcacctg
1190

<210> 68

<211> 2249

<212> DNA

<213> Homo sapiens

<400> 68

ctcctctaca aagaggtgga cagagaagac agcagagacc atgggacccc cctcagcccc 60

tccctgcaga ttgcatgtcc cctggaagga ggtcctgctc acagcctcac ttctaacctt
120

ctggaaccca cccaccactg ccaagctcac tattgaatcc acgccattca atgtcgcaga
180

ggggaaggag gttcttctac tcgcccacaa cctgccccag aatcgtattg gttacagctg
240

gtacaaaggc gaaagagtgg atggcaacag tctaattgta ggatatgtaa taggaactca
300

acaagctacc ccagggcccg catacagtgg tcgagagaca atatacccca atgcatccct
360

gctgatccag aacgtcaccc agaatgacac aggattctat accctacaag tcataaagtc
420

agatcttgtg aatgaagaag caaccggaca gttccatgta tacccggagc tgcccaagcc
480

ctccatctcc agcaacaact ccaaccccgt ggaggacaag gatgctgtgg ccttcacctg
540

tgaacctgag gttcagaaca caacctacct gtggtgggta aatggtcaga gcctcccggt
600

cagtcccagg ctgcagctgt ccaatggcaa catgaccctc actctactca gcgtcaaaag
660

gaacgatgca ggatcctatg aatgtgaaat acagaaccca gcgagtgcca accgcagtga
720

cccagtcacc ctgaatgtcc tctatggccc agatgtcccc accatttccc cctcaaaggc
780

caattaccgt ccaggggaaa atctgaacct ctcctgccac gcagcctcta acccacctgc
840

acagtactct tggtttatca atgggacgtt ccagcaatcc acacaagagc tctttatccc
900

caacatcact gtgaataata gcggatccta tatgtgccaa gcccataact cagccactgg
960

cctcaatagg accacagtca cgatgatcac agtctctgga agtgctcctg tcctctcagc
1020

tgtggccacc gtcggcatca cgattggagt gctggccagg gtggctctga tatagcagcc
1080

ctggtgtatt ttcgatattt caggaagact ggcagattgg accagaccct gaattcttct
1140

agctcctcca atcccatttt atcccatgga accactaaaa acaaggtctg ctctgctcct
1200

gaagccctat atgctggaga tggacaactc aatgaaaatt taaagggaaa accctcaggc
1260

ctgaggtgtg tgccactcag agacttcacc taactagaga cagtcaaact gcaaaccatg
1320

gtgagaaatt gacgacttca cactatggac agcttttccc aagatgtcaa aacaagactc
1380

ctcatcatga taaggctctt accccctttt aatttgtcct tgcttatgcc tgcctctttc
1440

gcttggcagg atgatgctgt cattagtatt tcacaagaag tagcttcaga gggtaactta
1500

acagagtgtc agatctatct tgtcaatccc aacgttttac ataaaataag agatccttta
1560

gtgcacccag tgactgacat tagcagcatc tttaacacag ccgtgtgttc aaatgtacag
1620

tggtcctttt cagagttgga cttctagact cacctgttct cactccctgt tttaattcaa
1680

cccagccatg caatgccaaa taatagaatt gctccctacc agctgaacag ggaggagtct
1740

gtgcagtttc tgacacttgt tgttgaacat ggctaaatac aatgggtatc gctgagacta
1800

agttgtagaa attaacaaat gtgctgcttg gttaaaatgg ctacactcat ctgactcatt
1860

ctttattcta ttttagttgg tttgtatctt gcctaaggtg cgtagtccaa ctcttggtat
1920

taccctccta atagtcatac tagtagtcat actccctggt gtagtgtatt ctctaaaagc
1980

tttaaatgtc tgcatgcagc cagccatcaa atagtgaatg gtctctcttt ggctggaatt
2040

acaaaactca gagaaatgtg tcatcaggag aacatcataa cccatgaagg ataaaagccc
2100

caaatggtgg taactgataa tagcactaat gctttaagat ttggtcacac tctcacctag
2160

gtgagcgcat tgagccagtg gtgctaaatg ctacatactc caactgaaat gttaaggaag
2220

aagatagatc caaaaaaaaa aaaaaaaaa
2249

<210> 69

<211> 2292

<212> DNA

<213> Homo sapiens

<400> 69

ccatgggttc cccttcagcc tgtccataca gagtgtgcat tccctggcag gggctcctgc 60

tcacagcctc gcttttaacc ttctggaacc tgccaaacag tgcccagacc aatattgatg
120

tcgtgccgtt caatgtcgca gaagggaagg aggtccttct agtagtccat aatgagtccc
180

agaatcttta tggctacaac tggtacaaag gggaaagggt gcatgccaac tatcgaatta
240

taggatatgt aaaaaatata agtcaagaaa atgccccagg gcccgcacac aacggtcgag
300

agacaatata ccccaatgga accctgctga tccagaacgt tacccacaat gacgcaggat
360

tctataccct acacgttata aaagaaaatc ttgtgaatga agaagtaacc agacaattct
420

acgtattctc ggagccaccc aagccctcca tcaccagcaa caacttcaat ccggtggaga
480

acaaagatat tgtggtttta acctgtcaac ctgagactca gaacacaacc tacctgtggt
540

gggtaaacaa tcagagcctc ctggtcagtc ccaggctgct gctctccact gacaacagga
600

ccctcgttct actcagcgcc acaaagaatg acataggacc ctatgaatgt gaaatacaga
660

acccagtggg tgccagccgc agtgacccag tcaccctgaa tgtccgctat gagtcagtac
720

aagcaagttc acctgacctc tcagctggga ccgctgtcag catcatgatt ggagtactgg
780

ctgggatggc tctgatatag cagccttggt gtagtttctg catttcggga agagtgtttt
840

tattatccac ctgcagactg gactggattc ttctagctcc ttcaatccca ttttctcctg
900

tggcatcact aagtataaga cctgctctct tcctgaagac ctataagctg gaggtggaca
960

actcaatgta aatttcaagg aaaaaccctc atgcctgaga tgtgggccac tcagagctaa
1020

ccaaaatgtt caacaccata actagagaca ctcaaattgc caaccaggac aagaagttga
1080

tgacttcatg ctgtggacag tttttcccaa gatgtcccaa gcctcatcgt gacgaggctc
1140

ttatcccact ccatttttcc ctgctcatgc ctgcctcttt aatttggtaa gataatgctg
1200

taactagaat ttcacaatca gcgccttgtg caggcaattt gacagagtgt tggatgtgtc
1260

atgtcatcat gtcaaaccca aatatttgac ctaagggatc ctttattctg cccagtggct
1320

aactttaaca acatccctaa tacaactgtt tattcaaatg cacggtggtc cctgttagag
1380

ttagacctct agactcacct gttctcacgc cctgttttaa tttaacccag ctatgggatg
1440

ccagataaca gaattgctgc ctacgagctg aacagggagg agtttgtgca gttgctgaca
1500

cttcttgttg cacataaata aatacagtgg gtactataga gactcagttg caaaaattaa
1560

caaatatgct gcttgattaa aatgggtagg cttctcatgt ggctcattct ttaatctatt
1620

ctcttttatt tggtttggtt catggggtct ctgcctatgg atcatacttc aaactcttgg
1680

tgtgatcctc ctgattgtca caatattagt taccctggtg tgctgtattc tctaaaacct
1740

ttaaatgttt gcatgcagcc attcgtcaaa tgtcaaatat tctctctttg gctggaatga
1800

caaaaactca aataaatgta tgattaggag gacatcataa cctatgaatg atggaagtcc
1860

aaaatgatgg taactgacag tagtgttaat gccttatgtt tagtcaaact ctcatttagg
1920

tgacagcctg gtgactccag aatggagcca gtcatgctaa atgccatata ctcacactga
1980

aacatgagga agcaggtaga tcccagaaca gacaaaactt tcctaaaaac atgagagtcc
2040

aggctgtctg agtcagcaca gtaagaaagt cctttctgct ttaactctta gaaaaaagta
2100

atatgaagta ttctgaaatt aaccaatcag tttatttaaa tcaatttatt tatattcttc
2160

tgttcctgga ttcccatttt acaaaaccca ctgttctact gttgtattgc ccagtaggag
2220

ctatcactat attttgcaga atggaaactg ccctgactct tgaatcacaa ataaaagcca
2280

attgtatctg tt
2292

<210> 70

<211> 2297

<212> DNA

<213> Homo sapiens

<400> 70
   agccgtgctc agaaagtttc tggatcccag gctcatctcc acagaggaga acacgcaggc 60
   agcagagacc atggggccca tctcagcccc ttcctgcaga tggcgcatcc cctggcaggg
   120
   gctcctgctc acagcctcac ttttcacctt ctggaacccg cccaccactg ctcagctcac
   180
   tattgaagct gtgccatcca atgctgcaga ggggaaggag gttcttctac ttgtccacaa
   240
   tctgccccag gaccctcgtg gctacaactg gtacaaaggg gaaacagtgg atgccaaccg
   300
   tcgaattata ggatatgtaa tatcaaatca acagattacc ccagggcctg catacagcaa
   360
   tcgagagaca atatacccca atgcatccct gctgatgcgg aacgtcacca gaaatgacac
   420
   aggatcctac accctacaag tcataaagct aaatcttatg agtgaagaag taactggcca
   480
   gttcagcgta catccggaga ctcccaagcc ctccatctcc agcaacaact ccaaccccgt
   540
   ggaggacaag gatgctgtgg ccttcacctg tgaacctgag actcagaaca caacctacct
   600
   gtggtgggta aatggtcaga gtctcccggt cagtcccagg ctgcagctgt ccaatggcaa
   660
   caggaccctc actctactca gtgtcacaag gaatgacgta ggaccctatg aatgtgaaat
   720
   acagaaccca gcgagtgcaa acttcagtga cccagtcacc ctgaatgtcc tctatggccc
   780
   agatgccccc accatttccc cttcagacac ctattaccat gcaggggtaa atctcaacct
   840
   ctcctgccat gcggcctcta atccaccctc acagtattct tggtctgtca atggcacatt
   900
   ccagcaatac acacaaaagc tctttatccc caacatcact acaaagaaca gcggatccta
   960
   tgcctgccac accactaact cagccactgg ccgcaacagg accacagtca ggatgatcac
   1020
   agtctctgat gctttagtac aaggaagttc tcctggcctc tcagctagag ccactgtcag
   1080
   catcatgatt ggagtactgg ccagggtggc tctgatatag tagctctggt gtagtttctg
   1140
   catttcaaga agactggcag acagttgttt ttattcttcc tcaaagcatt tgcaatcagc
   1200
   taccattcaa aattgcttct tcttcaagat ttatggaaaa tactctgacg agtactcttg
   1260
   aacacaagtt cctgataact ttaagatcac gccactggac tgtctatgaa cttgcaaaca
   1320
   ggctgatacc tttgtgaagt tgcccaccaa aacacagaag gaaaaaaaca tgaatttcat
   1380
   tgaactaaat aataatgagg ataatgtttt taagattttt tttttttttt gagatggaat
   1440
   ctcgctctgt cgcccaggct ggagtgcagt ggcacgatct caactcactg caagctccgc
   1500
   ctcctgggtt cacaccattc tcctgcctca gcctcctgag tagctgggac tacaggcgcc
   1560
   tgccacaacg cccggctaat tttttgtatt tttagtagag acggggtttc actgtggtct
   1620
   caatctcctg acttcatggt ccgcctgcct cagcctccca aagttctggg attacaggtg
   1680
   tgagccaccg cgcccagccc gtttttaaga ttttttattt gaaaaattgc caattcttta
   1740
   agtgttttct ttttcagatt tatgaatttc tttatctttt aagctatcta taccttactg
   1800
   caatttggta aagcagactt ttgtgaacaa aaattataac atttactttt gctccctacc
   1860
   tgactgccac agaactgggc aactattcat gagtattcat atgtttatgg taattcagtt
   1920
   atttgcacaa gttcagtgag aatctgctgt ctttataatg ggatatagtt taaaacattg
   1980
   gttatattac caaggctttg attgggatgt tatatttgag aaaatacaga gaatgataga
   2040
   ttaacggagt gtctaatcta tcgtgtcaac cccaaatttt tacgtatgag atcctttagt
   2100
   ccacccaatg gctgacagta acagcatctt taacacaact ctttgttcaa atgtactatg
   2160
   gtctctttta gagtcagact cctagactca cttgttctca ctgtctgttt taatttaacc
   2220
   caggcatgca atgctagata ataaaattgc tccctattgg ctgatcaaaa aaaaaaaaaa
   2280
   aaaaaaaaaa aaaaaaa
   2297

<210> 71

<211> 1552

<212> DNA

<213> Homo sapiens

<400> 71

gcccgtacac accgtgtgct gggacacccc acagtcagcc gcatggctcc cctgtgcccc 60

agcccctggc tccctctgtt gatcccggcc cctgctccag gcctcactgt gcaactgctg
120

ctgtcactgc tgcttctgat gcctgtccat ccccagaggt tgccccggat gcaggaggat
180

tcccccttgg gaggaggctc ttctggggaa gatgacccac tgggcgagga ggatctgccc
240

agtgaagagg attcacccag agaggaggat ccacccggag aggaggatct acctggagag
300

gaggatctac ctggagagga ggatctacct gaagttaagc ctaaatcaga agaagagggc
360

tccctgaagt tagaggatct acctactgtt gaggctcctg gagatcctca agaaccccag
420

aataatgccc acagggacaa agaaggggat gaccagagtc attggcgcta tggaggcgac
480

ccgccctggc cccgggtgtc cccagcctgc gcgggccgct tccagtcccc ggtggatatc
540

cgcccccagc tcgccgcctt ctgcccggcc ctgcgccccc tggaactcct gggcttccag
600

ctcccgccgc tcccagaact gcgcctgcgc aacaatggcc acagtgtgca actgaccctg
660

cctcctgggc tagagatggc tctgggtccc gggcgggagt accgggctct gcagctgcat
720

ctgcactggg gggctgcagg tcgtccgggc tcggagcaca ctgtggaagg ccaccgtttc
780

cctgccgaga tccacgtggt tcacctcagc accgcctttg ccagagttga cgaggccttg
840

gggcgcccgg gaggcctggc cgtgttggcc gcctttctgg aggagggccc ggaagaaaac
900

agtgcctatg agcagttgct gtctcgcttg gaagaaatcg ctgaggaagg ctcagagact
960

caggtcccag gactggacat atctgcactc ctgccctctg acttcagccg ctacttccaa
1020

tatgaggggt ctctgactac accgccctgt gcccagggtg tcatctggac tgtgtttaac
1080

cagacagtga tgctgagtgc taagcagctc cacaccctct ctgacaccct gtggggacct
1140

ggtgactctc ggctacagct gaacttccga gcgacgcagc ctttgaatgg gcgagtgatt
1200

gaggcctcct tccctgctgg agtggacagc agtcctcggg ctgctgagcc agtccagctg
1260

aattcctgcc tggctgctgg tgacatccta gccctggttt ttggcctcct ttttgctgtc
1320

accagcgtcg cgttccttgt gcagatgaga aggcagcaca gaaggggaac caaagggggt
1380

gtgagctacc gcccagcaga ggtagccgag actggagcct agaggctgga tcttggagaa
1440

tgtgagaagc cagccagagg catctgaggg ggagccggta actgtcctgt cctgctcatt
1500

atgccacttc cttttaactg ccaagaaatt ttttaaaata aatatttata at
1552

<210> 72

<211> 702

<212> PRT

<213> Homo sapiens

<400> 72

Met Glu Ser Pro Ser Ala Pro Pro His Arg Trp Cys Ile Pro Trp Gln

1               5                   10                  15

Arg Leu Leu Leu Thr Ala Ser Leu Leu Thr Phe Trp Asn Pro Pro Thr

            20                  25                  30

Thr Ala Lys Leu Thr Ile Glu Ser Thr Pro Phe Asn Val Ala Glu Gly

        35                  40                  45

Lys Glu Val Leu Leu Leu Val His Asn Leu Pro Gln His Leu Phe Gly

    50                  55                  60

Tyr Ser Trp Tyr Lys Gly Glu Arg Val Asp Gly Asn Arg Gln Ile Ile

65                  70                  75                  80

Gly Tyr Val Ile Gly Thr Gln Gln Ala Thr Pro Gly Pro Ala Tyr Ser

                85                  90                  95

Gly Arg Glu Ile Ile Tyr Pro Asn Ala Ser Leu Leu Ile Gln Asn Ile

            100                 105                 110

Ile Gln Asn Asp Thr Gly Phe Tyr Thr Leu His Val Ile Lys Ser Asp

        115                 120                 125

Leu Val Asn Glu Glu Ala Thr Gly Gln Phe Arg Val Tyr Pro Glu Leu

    130                 135                 140

Pro Lys Pro Ser Ile Ser Ser Asn Asn Ser Lys Pro Val Glu Asp Lys

145                 150                 155                 160

Asp Ala Val Ala Phe Thr Cys Glu Pro Glu Thr Gln Asp Ala Thr Tyr

                165                 170                 175

Leu Trp Trp Val Asn Asn Gln Ser Leu Pro Val Ser Pro Arg Leu Gln
            180                 185                 190
Leu Ser Asn Gly Asn Arg Thr Leu Thr Leu Phe Asn Val Thr Arg Asn
        195                 200                 205
Asp Thr Ala Ser Tyr Lys Cys Glu Thr Gln Asn Pro Val Ser Ala Arg
    210                 215                 220
Arg Ser Asp Ser Val Ile Leu Asn Val Leu Tyr Gly Pro Asp Ala Pro
225                 230                 235                 240
Thr Ile Ser Pro Leu Asn Thr Ser Tyr Arg Ser Gly Glu Asn Leu Asn
                245                 250                 255
Leu Ser Cys His Ala Ala Ser Asn Pro Pro Ala Gln Tyr Ser Trp Phe
            260                 265                 270
Val Asn Gly Thr Phe Gln Gln Ser Thr Gln Glu Leu Phe Ile Pro Asn
        275                 280                 285
Ile Thr Val Asn Asn Ser Gly Ser Tyr Thr Cys Gln Ala His Asn Ser
    290                 295                 300
Asp Thr Gly Leu Asn Arg Thr Thr Val Thr Thr Ile Thr Val Tyr Ala
305                 310                 315                 320
Glu Pro Pro Lys Pro Phe Ile Thr Ser Asn Asn Ser Asn Pro Val Glu
                325                 330                 335
Asp Glu Asp Ala Val Ala Leu Thr Cys Glu Pro Glu Ile Gln Asn Thr
            340                 345                 350
Thr Tyr Leu Trp Trp Val Asn Asn Gln Ser Leu Pro Val Ser Pro Arg
        355                 360                 365
Leu Gln Leu Ser Asn Asp Asn Arg Thr Leu Thr Leu Leu Ser Val Thr
    370                 375                 380
Arg Asn Asp Val Gly Pro Tyr Glu Cys Gly Ile Gln Asn Glu Leu Ser
385                 390                 395                 400
Val Asp His Ser Asp Pro Val Ile Leu Asn Val Leu Tyr Gly Pro Asp

                405                 410                 415

Asp Pro Thr Ile Ser Pro Ser Tyr Thr Tyr Tyr Arg Pro Gly Val Asn

            420                 425                 430

Leu Ser Leu Ser Cys His Ala Ala Ser Asn Pro Pro Ala Gln Tyr Ser

        435                 440                 445

Trp Leu Ile Asp Gly Asn Ile Gln Gln His Thr Gln Glu Leu Phe Ile

    450                 455                 460

Ser Asn Ile Thr Glu Lys Asn Ser Gly Leu Tyr Thr Cys Gln Ala Asn

465                 470                 475                 480

Asn Ser Ala Ser Gly His Ser Arg Thr Thr Val Lys Thr Ile Thr Val

                485                 490                 495

Ser Ala Glu Leu Pro Lys Pro Ser Ile Ser Ser Asn Asn Ser Lys Pro

            500                 505                 510

Val Glu Asp Lys Asp Ala Val Ala Phe Thr Cys Glu Pro Glu Ala Gln

        515                 520                 525

Asn Thr Thr Tyr Leu Trp Trp Val Asn Gly Gln Ser Leu Pro Val Ser

    530                 535                 540

Pro Arg Leu Gln Leu Ser Asn Gly Asn Arg Thr Leu Thr Leu Phe Asn

545                 550                 555                 560

Val Thr Arg Asn Asp Ala Arg Ala Tyr Val Cys Gly Ile Gln Asn Ser

                565                 570                 575

Val Ser Ala Asn Arg Ser Asp Pro Val Thr Leu Asp Val Leu Tyr Gly

            580                 585                 590

Pro Asp Thr Pro Ile Ile Ser Pro Pro Asp Ser Ser Tyr Leu Ser Gly

        595                 600                 605

Ala Asn Leu Asn Leu Ser Cys His Ser Ala Ser Asn Pro Ser Pro Gln

    610                 615                 620

Tyr Ser Trp Arg Ile Asn Gly Ile Pro Gln Gln His Thr Gln Val Leu

625                 630                 635                 640

Phe Ile Ala Lys Ile Thr Pro Asn Asn Asn Gly Thr Tyr Ala Cys Phe
                645                 650                 655
Val Ser Asn Leu Ala Thr Gly Arg Asn Asn Ser Ile Val Lys Ser Ile
            660                 665                 670
Thr Val Ser Ala Ser Gly Thr Ser Pro Gly Leu Ser Ala Gly Ala Thr
        675                 680                 685
Val Gly Ile Met Ile Gly Val Leu Val Gly Val Ala Leu Ile
    690                 695                 700

<210> 73
<211> 609
<212> PRT
<213> Homo sapiens

<400> 73

<210> 74

<211> 99

<212> PRT

<213> Homo sapiens

<400> 74

Met Thr Ser Lys Leu Ala Val Ala Leu Leu Ala Ala Phe Leu Ile Ser

1               5                   10                  15

Ala Ala Leu Cys Glu Gly Ala Val Leu Pro Arg Ser Ala Lys Glu Leu

            20                  25                  30

Arg Cys Gln Cys Ile Lys Thr Tyr Ser Lys Pro Phe His Pro Lys Phe

        35                  40                  45

Ile Lys Glu Leu Arg Val Ile Glu Ser Gly Pro His Cys Ala Asn Thr

    50                  55                  60

Glu Ile Ile Val Lys Leu Ser Asp Gly Arg Glu Leu Cys Leu Asp Pro

65                  70                  75                  80

Lys Glu Asn Trp Val Gln Arg Val Val Glu Lys Phe Leu Lys Arg Ala

                85                  90                  95

Glu Asn Ser

165 170 175

Ser His Met Glu Ser Glu Glu Leu Asn Gly Ala Tyr Lys Ala Ile Pro

180 185 190

Val Ala Gln Asp Leu Asn Ala Pro Ser Asp Trp Asp Ser Arg Gly Lys

195 200 205

Asp Ser Tyr Glu Thr Ser Gln Leu Asp Asp Gln Ser Ala Glu Thr His

210 215 220

Ser His Lys Gln Ser Arg Leu Tyr Lys Arg Lys Ala Asn Asp Glu Ser

225 230 235 240

Asn Glu His Ser Asp Val Ile Asp Ser Gln Glu Leu Ser Lys Val Ser

245 250 255

Arg Glu Phe His Ser His Glu Phe His Ser His Glu Asp Met Leu Val

260 265 270

Val Asp Pro Lys Ser Lys Glu Glu Asp Lys His Leu Lys Phe Arg Ile

275 280 285

Ser His Glu Leu Asp Ser Ala Ser Ser Glu Val Asn

290 295 300

<210> 76

<211> 871

<212> PRT

<213> Homo sapiens

<400> 76

Met Lys Tyr Ser Cys Cys Ala Leu Val Leu Ala Val Leu Gly Thr Glu

1               5                   10                  15

Leu Leu Gly Ser Leu Cys Ser Thr Val Arg Ser Pro Arg Phe Arg Gly

            20                  25                  30

Arg Ile Gln Gln Glu Arg Lys Asn Ile Arg Pro Asn Ile Ile Leu Val
        35                  40                  45
Leu Thr Asp Asp Gln Asp Val Glu Leu Gly Ser Leu Gln Val Met Asn
    50                  55                  60
Lys Thr Arg Lys Ile Met Glu His Gly Gly Ala Thr Phe Ile Asn Ala
65                  70                  75                  80
Phe Val Thr Thr Pro Met Cys Cys Pro Ser Arg Ser Ser Met Leu Thr
                85                  90                  95
Gly Lys Tyr Val His Asn His Asn Val Tyr Thr Asn Asn Glu Asn Cys
            100                 105                 110
Ser Ser Pro Ser Trp Gln Ala Met His Glu Pro Arg Thr Phe Ala Val
        115                 120                 125
Tyr Leu Asn Asn Thr Gly Tyr Arg Thr Ala Phe Phe Gly Lys Tyr Leu
    130                 135                 140
Asn Glu Tyr Asn Gly Ser Tyr Ile Pro Pro Gly Trp Arg Glu Trp Leu
145                 150                 155                 160
Gly Leu Ile Lys Asn Ser Arg Phe Tyr Asn Tyr Thr Val Cys Arg Asn
                165                 170                 175
Gly Ile Lys Glu Lys His Gly Phe Asp Tyr Ala Lys Asp Tyr Phe Thr
            180                 185                 190
Asp Leu Ile Thr Asn Glu Ser Ile Asn Tyr Phe Lys Met Ser Lys Arg
        195                 200                 205
Met Tyr Pro His Arg Pro Val Met Met Val Ile Ser His Ala Ala Pro
    210                 215                 220
His Gly Pro Glu Asp Ser Ala Pro Gln Phe Ser Lys Leu Tyr Pro Asn
225                 230                 235                 240
Ala Ser Gln His Ile Thr Pro Ser Tyr Asn Tyr Ala Pro Asn Met Asp
                245                 250                 255
Lys His Trp Ile Met Gln Tyr Thr Gly Pro Met Leu Pro Ile His Met

            260                 265                 270

Glu Phe Thr Asn Ile Leu Gln Arg Lys Arg Leu Gln Thr Leu Met Ser

        275                 280                 285

Val Asp Asp Ser Val Glu Arg Leu Tyr Asn Met Leu Val Glu Thr Gly

    290                 295                 300

Glu Leu Glu Asn Thr Tyr Ile Ile Tyr Thr Ala Asp His Gly Tyr His

305                 310                 315                 320

Ile Gly Gln Phe Gly Leu Val Lys Gly Lys Ser Met Pro Tyr Asp Phe

                325                 330                 335

Asp Ile Arg Val Pro Phe Phe Ile Arg Gly Pro Ser Val Glu Pro Gly

            340                 345                 350

Ser Ile Val Pro Gln Ile Val Leu Asn Ile Asp Leu Ala Pro Thr Ile

        355                 360                 365

Leu Asp Ile Ala Gly Leu Asp Thr Pro Pro Asp Val Asp Gly Lys Ser

    370                 375                 380

Val Leu Lys Leu Leu Asp Pro Glu Lys Pro Gly Asn Arg Phe Arg Thr

385                 390                 395                 400

Asn Lys Lys Ala Lys Ile Trp Arg Asp Thr Phe Leu Val Glu Arg Gly

                405                 410                 415

Lys Phe Leu Arg Lys Lys Glu Glu Ser Ser Lys Asn Ile Gln Gln Ser

            420                 425                 430

Asn His Leu Pro Lys Tyr Glu Arg Val Lys Glu Leu Cys Gln Gln Ala

        435                 440                 445

Arg Tyr Gln Thr Ala Cys Glu Gln Pro Gly Gln Lys Trp Gln Cys Ile

    450                 455                 460

Glu Asp Thr Ser Gly Lys Leu Arg Ile His Lys Cys Lys Gly Pro Ser

465                 470                 475                 480

Asp Leu Leu Thr Val Arg Gln Ser Thr Arg Asn Leu Tyr Ala Arg Gly

                485                 490                 495

Phe His Asp Lys Asp Lys Glu Cys Ser Cys Arg Glu Ser Gly Tyr Arg
            500                 505                 510
Ala Ser Arg Ser Gln Arg Lys Ser Gln Arg Gln Phe Leu Arg Asn Gln
        515                 520                 525
Gly Thr Pro Lys Tyr Lys Pro Arg Phe Val His Thr Arg Gln Thr Arg
    530                 535                 540
Ser Leu Ser Val Glu Phe Glu Gly Glu Ile Tyr Asp Ile Asn Leu Glu
545                 550                 555                 560
Glu Glu Glu Glu Leu Gln Val Leu Gln Pro Arg Asn Ile Ala Lys Arg
                565                 570                 575
His Asp Glu Gly His Lys Gly Pro Arg Asp Leu Gln Ala Ser Ser Gly
            580                 585                 590
Gly Asn Arg Gly Arg Met Leu Ala Asp Ser Ser Asn Ala Val Gly Pro
        595                 600                 605
Pro Thr Thr Val Arg Val Thr His Lys Cys Phe Ile Leu Pro Asn Asp
    610                 615                 620
Ser Ile His Cys Glu Arg Glu Leu Tyr Gln Ser Ala Arg Ala Trp Lys
625                 630                 635                 640
Asp His Lys Ala Tyr Ile Asp Lys Glu Ile Glu Ala Leu Gln Asp Lys
                645                 650                 655
Ile Lys Asn Leu Arg Glu Val Arg Gly His Leu Lys Arg Arg Lys Pro
            660                 665                 670
Glu Glu Cys Ser Cys Ser Lys Gln Ser Tyr Tyr Asn Lys Glu Lys Gly
        675                 680                 685
Val Lys Lys Gln Glu Lys Leu Lys Ser His Leu His Pro Phe Lys Glu
    690                 695                 700
Ala Ala Gln Glu Val Asp Ser Lys Leu Gln Leu Phe Lys Glu Asn Asn
705                 710                 715                 720
Arg Arg Arg Lys Lys Glu Arg Lys Glu Lys Arg Arg Gln Arg Lys Gly

                725                 730                 735

Glu Glu Cys Ser Leu Pro Gly Leu Thr Cys Phe Thr His Asp Asn Asn

            740                 745                 750

His Trp Gln Thr Ala Pro Phe Trp Asn Leu Gly Ser Phe Cys Ala Cys

        755                 760                 765

Thr Ser Ser Asn Asn Asn Thr Tyr Trp Cys Leu Arg Thr Val Asn Glu

    770                 775                 780

Thr His Asn Phe Leu Phe Cys Glu Phe Ala Thr Gly Phe Leu Glu Tyr

785                 790                 795                 800

Phe Asp Met Asn Thr Asp Pro Tyr Gln Leu Thr Asn Thr Val His Thr

                805                 810                 815

Val Glu Arg Gly Ile Leu Asn Gln Leu His Val Gln Leu Met Glu Leu

            820                 825                 830

Arg Ser Cys Gln Gly Tyr Lys Gln Cys Asn Pro Arg Pro Lys Asn Leu

        835                 840                 845

Asp Val Gly Asn Lys Asp Gly Gly Ser Tyr Asp Leu His Arg Gly Gln

    850                 855                 860

Leu Trp Asp Gly Trp Glu Gly

865                 870

<210> 77

<211> 470

<212> PRT

<213> Homo sapiens

<400> 77

Met Lys Phe Leu Leu Ile Leu Leu Leu Gln Ala Thr Ala Ser Gly Ala

1               5                   10                  15

Leu Pro Leu Asn Ser Ser Thr Ser Leu Glu Lys Asn Asn Val Leu Phe
            20                  25                  30
Gly Glu Arg Tyr Leu Glu Lys Phe Tyr Gly Leu Glu Ile Asn Lys Leu
        35                  40                  45
Pro Val Thr Lys Met Lys Tyr Ser Gly Asn Leu Met Lys Glu Lys Ile
    50                  55                  60
Gln Glu Met Gln His Phe Leu Gly Leu Lys Val Thr Gly Gln Leu Asp
65                  70                  75                  80
Thr Ser Thr Leu Glu Met Met His Ala Pro Arg Cys Gly Val Pro Asp
                85                  90                  95
Leu His His Phe Arg Glu Met Pro Gly Gly Pro Val Trp Arg Lys His
            100                 105                 110
Tyr Ile Thr Tyr Arg Ile Asn Asn Tyr Thr Pro Asp Met Asn Arg Glu
        115                 120                 125
Asp Val Asp Tyr Ala Ile Arg Lys Ala Phe Gln Val Trp Ser Asn Val
    130                 135                 140
Thr Pro Leu Lys Phe Ser Lys Ile Asn Thr Gly Met Ala Asp Ile Leu
145                 150                 155                 160
Val Val Phe Ala Arg Gly Ala His Gly Asp Phe His Ala Phe Asp Gly
                165                 170                 175
Lys Gly Gly Ile Leu Ala His Ala Phe Gly Pro Gly Ser Gly Ile Gly
            180                 185                 190
Gly Asp Ala His Phe Asp Glu Asp Glu Phe Trp Thr Thr His Ser Gly
        195                 200                 205
Gly Thr Asn Leu Phe Leu Thr Ala Val His Glu Ile Gly His Ser Leu
    210                 215                 220
Gly Leu Gly His Ser Ser Asp Pro Lys Ala Val Met Phe Pro Thr Tyr
225                 230                 235                 240
Lys Tyr Val Asp Ile Asn Thr Phe Arg Leu Ser Ala Asp Asp Ile Arg

                245                 250                 255

Gly Ile Gln Ser Leu Tyr Gly Asp Pro Lys Glu Asn Gln Arg Leu Pro

            260                 265                 270

Asn Pro Asp Asn Ser Glu Pro Ala Leu Cys Asp Pro Asn Leu Ser Phe

        275                 280                 285

Asp Ala Val Thr Thr Val Gly Asn Lys Ile Phe Phe Phe Lys Asp Arg

    290                 295                 300

Phe Phe Trp Leu Lys Val Ser Glu Arg Pro Lys Thr Ser Val Asn Leu

305                 310                 315                 320

Ile Ser Ser Leu Trp Pro Thr Leu Pro Ser Gly Ile Glu Ala Ala Tyr

                325                 330                 335

Glu Ile Glu Ala Arg Asn Gln Val Phe Leu Phe Lys Asp Asp Lys Tyr

            340                 345                 350

Trp Leu Ile Ser Asn Leu Arg Pro Glu Pro Asn Tyr Pro Lys Ser Ile

        355                 360                 365

His Ser Phe Gly Phe Pro Asn Phe Val Lys Lys Ile Asp Ala Ala Val

    370                 375                 380

Phe Asn Pro Arg Phe Tyr Arg Thr Tyr Phe Phe Val Asp Asn Gln Tyr

385                 390                 395                 400

Trp Arg Tyr Asp Glu Arg Arg Gln Met Met Asp Pro Gly Tyr Pro Lys

                405                 410                 415

Leu Ile Thr Lys Asn Phe Gln Gly Ile Gly Pro Lys Ile Asp Ala Val

            420                 425                 430

Phe Tyr Ser Lys Asn Lys Tyr Tyr Tyr Phe Phe Gln Gly Ser Asn Gln

        435                 440                 445

Phe Glu Tyr Asp Phe Leu Leu Gln Arg Ile Thr Lys Thr Leu Lys Ser

    450                 455                 460

Asn Ser Trp Phe Gly Cys

465                 470

Pro Gln Leu Ser Tyr Gly Tyr Asp Glu Lys Ser Thr Gly Gly Ile Ser
                165                 170                 175
Val Pro Gly Pro Met Gly Pro Ser Gly Pro Arg Gly Leu Pro Gly Pro
            180                 185                 190
Pro Gly Ala Pro Gly Pro Gln Gly Phe Gln Gly Pro Pro Gly Glu Pro
        195                 200                 205
Gly Glu Pro Gly Ala Ser Gly Pro Met Gly Pro Arg Gly Pro Pro Gly
    210                 215                 220
Pro Pro Gly Lys Asn Gly Asp Asp Gly Glu Ala Gly Lys Pro Gly Arg
225                 230                 235                 240
Pro Gly Glu Arg Gly Pro Pro Gly Pro Gln Gly Ala Arg Gly Leu Pro
                245                 250                 255
Gly Thr Ala Gly Leu Pro Gly Met Lys Gly His Arg Gly Phe Ser Gly
            260                 265                 270
Leu Asp Gly Ala Lys Gly Asp Ala Gly Pro Ala Gly Pro Lys Gly Glu
        275                 280                 285
Pro Gly Ser Pro Gly Glu Asn Gly Ala Pro Gly Gln Met Gly Pro Arg
    290                 295                 300
Gly Leu Pro Gly Glu Arg Gly Arg Pro Gly Ala Pro Gly Pro Ala Gly
305                 310                 315                 320
Ala Arg Gly Asn Asp Gly Ala Thr Gly Ala Ala Gly Pro Pro Gly Pro
                325                 330                 335
Thr Gly Pro Ala Gly Pro Pro Gly Phe Pro Gly Ala Val Gly Ala Lys
            340                 345                 350
Gly Glu Ala Gly Pro Gln Gly Pro Arg Gly Ser Glu Gly Pro Gln Gly
        355                 360                 365
Val Arg Gly Glu Pro Gly Pro Pro Gly Pro Ala Gly Ala Ala Gly Pro
    370                 375                 380
Ala Gly Asn Pro Gly Ala Asp Gly Gln Pro Gly Ala Lys Gly Ala Asn

850 855 860

Arg Gly Ser Ala Gly Pro Pro Gly Ala Thr Gly Phe Pro Gly Ala Ala

865 870 875 880

Gly Arg Val Gly Pro Pro Gly Pro Ser Gly Asn Ala Gly Pro Pro Gly

885 890 895

Pro Pro Gly Pro Ala Gly Lys Glu Gly Gly Lys Gly Pro Arg Gly Glu

900 905 910

Thr Gly Pro Ala Gly Arg Pro Gly Glu Val Gly Pro Pro Gly Pro Pro

915 920 925

Gly Pro Ala Gly Glu Lys Gly Ser Pro Gly Ala Asp Gly Pro Ala Gly

930 935 940

Ala Pro Gly Thr Pro Gly Pro Gln Gly Ile Ala Gly Gln Arg Gly Val

945 950 955 960

Val Gly Leu Pro Gly Gln Arg Gly Glu Arg Gly Phe Pro Gly Leu Pro

965 970 975

Gly Pro Ser Gly Glu Pro Gly Lys Gln Gly Pro Ser Gly Ala Ser Gly

980 985 990

Glu Arg Gly Pro Pro Gly Pro Met Gly Pro Pro Gly Leu Ala Gly Pro

995 1000 1005

Pro Gly Glu Ser Gly Arg Glu Gly Ala Pro Ala Ala Glu Gly Ser Pro

1010 1015 1020

Gly Arg Asp Gly Ser Pro Gly Ala Lys Gly Asp Arg Gly Glu Thr Gly

1025 1030 1035 1040

Pro Ala Gly Pro Pro Gly Ala Pro Gly Ala Pro Gly Ala Pro Gly Pro

1045 1050 1055

Val Gly Pro Ala Gly Lys Ser Gly Asp Arg Gly Glu Thr Gly Pro Ala

1060 1065 1070

Gly Pro Ala Gly Pro Val Gly Pro Val Gly Ala Arg Gly Pro Ala Gly

1075 1080 1085

Pro Gln Gly Pro Arg Gly Asp Lys Gly Glu Thr Gly Glu Gln Gly Asp
1090 1095 1100
Arg Gly Ile Lys Gly His Arg Gly Phe Ser Gly Leu Gln Gly Pro Pro
1105 1110 1115 1120
Gly Pro Pro Gly Ser Pro Gly Glu Gln Gly Pro Ser Gly Ala Ser Gly
1125 1130 1135
Pro Ala Gly Pro Arg Gly Pro Pro Gly Ser Ala Gly Ala Pro Gly Lys
1140 1145 1150
Asp Gly Leu Asn Gly Leu Pro Gly Pro Ile Gly Pro Pro Gly Pro Arg
1155 1160 1165
Gly Arg Thr Gly Asp Ala Gly Pro Val Gly Pro Pro Gly Pro Pro Gly
1170 1175 1180
Pro Pro Gly Pro Pro Gly Pro Pro Ser Ala Gly Phe Asp Phe Ser Phe
1185 1190 1195 1200
Leu Pro Gln Pro Pro Gln Glu Lys Ala His Asp Gly Gly Arg Tyr Tyr
1205 1210 1215
Arg Ala Asp Asp Ala Asn Val Val Arg Asp Arg Asp Leu Glu Val Asp
1220 1225 1230
Thr Thr Leu Lys Ser Leu Ser Gln Gln Ile Glu Asn Ile Arg Ser Pro
1235 1240 1245
Glu Gly Ser Arg Lys Asn Pro Ala Arg Thr Cys Arg Asp Leu Lys Met
1250 1255 1260
Cys His Ser Asp Trp Lys Ser Gly Glu Tyr Trp Ile Asp Pro Asn Gln
1265 1270 1275 1280
Gly Cys Asn Leu Asp Ala Ile Lys Val Phe Cys Asn Met Glu Thr Gly
1285 1290 1295
Glu Thr Cys Val Tyr Pro Thr Gln Pro Ser Val Ala Gln Lys Asn Trp
1300 1305 1310
Tyr Ile Ser Lys Asn Pro Lys Asp Lys Arg His Val Trp Phe Gly Glu

1315                1320                1325

Ser Met Thr Asp Gly Phe Gln Phe Glu Tyr Gly Gly Gln Gly Ser Asp

    1330                1335                1340

Pro Ala Asp Val Ala Ile Gln Leu Thr Phe Leu Arg Leu Met Ser Thr

1345                1350                1355                1360

Glu Ala Ser Gln Asn Ile Thr Tyr His Cys Lys Asn Ser Val Ala Tyr

                1365                1370                1375

Met Asp Gln Gln Thr Gly Asn Leu Lys Lys Ala Leu Leu Leu Lys Gly

            1380                1385                1390

Ser Asn Glu Ile Glu Ile Arg Ala Glu Gly Asn Ser Arg Phe Thr Tyr

        1395                1400                1405

Ser Val Thr Val Asp Gly Cys Thr Ser His Thr Gly Ala Trp Gly Lys

    1410                1415                1420

Thr Val Ile Glu Tyr Lys Thr Thr Lys Ser Ser Arg Leu Pro Ile Ile

1425                1430                1435                1440

Asp Val Ala Pro Leu Asp Val Gly Ala Pro Asp Gln Glu Phe Gly Phe

                1445                1450                1455

Asp Val Gly Pro Val Cys Phe Leu

            1460

<210> 80

<211> 338

<212> PRT

<213> Homo sapiens

<400> 80

Met Ser Leu Ser Ala Phe Thr Leu Phe Leu Ala Leu Ile Gly Gly Thr

 1               5                  10                  15

245                 250                 255

Val Glu Leu Asp Leu Ser Tyr Asn Lys Leu Lys Asn Ile Pro Thr Val

            260                 265                 270

Asn Glu Asn Leu Glu Asn Tyr Tyr Leu Glu Val Asn Gln Leu Glu Lys

        275                 280                 285

Phe Asp Ile Lys Ser Phe Cys Lys Ile Leu Gly Pro Leu Ser Tyr Ser

    290                 295                 300

Lys Ile Lys His Leu Arg Leu Asp Gly Asn Arg Ile Ser Glu Thr Ser

305                 310                 315                 320

Leu Pro Pro Asp Met Tyr Glu Cys Leu Arg Val Ala Asn Glu Val Thr

                325                 330                 335

Leu Asn

<210> 81

<211> 589

<212> PRT

<213> Homo sapiens

<400> 81

Met Ser Val Ser Val His Glu Asn Arg Lys Ser Arg Ala Ser Ser Gly

1               5                   10                  15

Ser Ile Asn Ile Tyr Leu Phe His Lys Ser Ser Tyr Ala Asp Ser Val

            20                  25                  30

Leu Thr His Leu Asn Leu Leu Arg Gln Gln Arg Leu Phe Thr Asp Val

        35                  40                  45

Leu Leu His Ala Gly Asn Arg Thr Phe Pro Cys His Arg Ala Val Leu

    50                  55                  60

290                 295                 300

Phe Met Cys Asp Lys Leu Tyr Leu Val Asp Gln Lys Ala Lys Glu Ile

305                 310                 315                 320

Ile Pro Lys Ala Asp Ile Pro Ser Pro Arg Lys Glu Phe Ser Ala Cys

                325                 330                 335

Ala Ile Gly Cys Lys Val Tyr Ile Thr Gly Gly Arg Gly Ser Glu Asn

            340                 345                 350

Gly Val Ser Lys Asp Val Trp Val Tyr Asp Thr Leu His Glu Glu Trp

        355                 360                 365

Ser Lys Ala Ala Pro Met Leu Val Ala Arg Phe Gly His Gly Ser Ala

    370                 375                 380

Glu Leu Lys His Cys Leu Tyr Val Val Gly Gly His Thr Ala Ala Thr

385                 390                 395                 400

Gly Cys Leu Pro Ala Ser Pro Ser Val Ser Leu Lys Gln Val Glu His

                405                 410                 415

Tyr Asp Pro Thr Ile Asn Lys Trp Thr Met Val Ala Pro Leu Arg Glu

            420                 425                 430

Gly Val Ser Asn Ala Ala Val Val Ser Ala Lys Leu Lys Leu Phe Ala

        435                 440                 445

Phe Gly Gly Thr Ser Val Ser His Asp Lys Leu Pro Lys Val Gln Cys

    450                 455                 460

Tyr Asp Gln Cys Glu Asn Arg Trp Thr Val Pro Ala Thr Cys Pro Gln

465                 470                 475                 480

Pro Trp Arg Tyr Thr Ala Ala Ala Val Leu Gly Asn Gln Ile Phe Ile

                485                 490                 495

Met Gly Gly Asp Thr Glu Phe Ser Ala Cys Ser Ala Tyr Lys Phe Asn

            500                 505                 510

Ser Glu Thr Tyr Gln Trp Thr Lys Val Gly Asp Val Thr Ala Lys Arg

        515                 520                 525

Met Ser Cys His Ala Val Ala Ser Gly Asn Lys Leu Tyr Val Val Gly
    530                 535                 540
Gly Tyr Phe Gly Ile Gln Arg Cys Lys Thr Leu Asp Cys Tyr Asp Pro
545                 550                 555                 560
Thr Leu Asp Val Trp Asn Ser Ile Thr Thr Val Pro Tyr Ser Leu Ile
                565                 570                 575
Pro Thr Ala Phe Val Ser Thr Trp Lys His Leu Pro Ser
            580                 585

<210> 82
<211> 193
<212> PRT
<213> Homo sapiens

<400> 82
Met Ile Arg Cys Gly Leu Ala Cys Glu Arg Cys Arg Trp Ile Leu Pro
 1               5                  10                  15
Leu Leu Leu Leu Ser Ala Ile Ala Phe Asp Ile Ile Ala Leu Ala Gly
            20                  25                  30
Arg Gly Trp Leu Gln Ser Ser Asp His Gly Gln Thr Ser Ser Leu Trp
        35                  40                  45
Trp Lys Cys Ser Gln Glu Gly Gly Gly Ser Gly Ser Tyr Glu Glu Gly
    50                  55                  60
Cys Gln Ser Leu Met Glu Tyr Ala Trp Gly Arg Ala Ala Ala Ala Met
65                  70                  75                  80
Leu Phe Cys Gly Phe Ile Ile Leu Val Ile Cys Phe Ile Leu Ser Phe
                85                  90                  95
Phe Ala Leu Cys Gly Pro Gln Met Leu Val Phe Leu Arg Val Ile Gly

            100                 105                 110

Gly Leu Leu Ala Leu Ala Ala Val Phe Gln Ile Ile Ser Leu Val Ile

        115                 120                 125

Tyr Pro Val Lys Tyr Thr Gln Thr Phe Thr Leu His Ala Asn Pro Ala

    130                 135                 140

Val Thr Tyr Ile Tyr Asn Trp Ala Tyr Gly Phe Gly Trp Ala Ala Thr

145                 150                 155                 160

Ile Ile Leu Ile Gly Cys Ala Phe Phe Phe Cys Cys Leu Leu Asn Tyr

                165                 170                 175

Glu Asp Asp Leu Leu Gly Asn Ala Lys Pro Arg Tyr Phe Tyr Thr Ser

            180                 185                 190

Ala

<210> 83

<211> 423

<212> PRT

<213> Homo sapiens

<400> 83

Met Arg Ser Ser Gly Ala Asp Ala Gly Arg Cys Leu Val Thr Ala Arg

1               5                   10                  15

Ala Pro Gly Ser Val Pro Ala Ser Arg Glu Gly Ser Ala Gly Ser Arg

            20                  25                  30

Gly Pro Gly Ala Arg Phe Pro Ala Arg Val Ser Ala Arg Gly Ser Ala

        35                  40                  45

Pro Gly Pro Gly Leu Gly Gly Ala Gly Ala Leu Asp Pro Pro Ala Val

    50                  55                  60

Val Ala Glu Ser Val Ser Ser Leu Thr Ile Ala Asp Ala Phe Ile Ala
65                  70                  75                  80
Ala Gly Glu Ser Ser Ala Pro Thr Pro Pro Arg Pro Ala Leu Pro Arg
                85                  90                  95
Arg Phe Ile Cys Ser Phe Pro Asp Cys Ser Ala Asn Tyr Ser Lys Ala
            100                 105                 110
Trp Lys Leu Asp Ala His Leu Cys Lys His Thr Gly Glu Arg Pro Phe
        115                 120                 125
Val Cys Asp Tyr Glu Gly Cys Gly Lys Ala Phe Ile Arg Asp Tyr His
    130                 135                 140
Leu Ser Arg His Ile Leu Thr His Thr Gly Glu Lys Pro Phe Val Cys
145                 150                 155                 160
Ala Ala Asn Gly Cys Asp Gln Lys Phe Asn Thr Lys Ser Asn Leu Lys
                165                 170                 175
Lys His Phe Glu Arg Lys His Glu Asn Gln Gln Lys Gln Tyr Ile Cys
            180                 185                 190
Ser Phe Glu Asp Cys Lys Lys Thr Phe Lys Lys His Gln Gln Leu Lys
        195                 200                 205
Ile His Gln Cys Gln Asn Thr Asn Glu Pro Leu Phe Lys Cys Thr Gln
    210                 215                 220
Glu Gly Cys Gly Lys His Phe Ala Ser Pro Ser Lys Leu Lys Arg His
225                 230                 235                 240
Ala Lys Ala His Glu Gly Tyr Val Cys Gln Lys Gly Cys Ser Phe Val
                245                 250                 255
Ala Lys Thr Trp Thr Glu Leu Leu Lys His Val Arg Glu Thr His Lys
            260                 265                 270
Glu Glu Ile Leu Cys Glu Val Cys Arg Lys Thr Phe Lys Arg Lys Asp
        275                 280                 285
Tyr Leu Lys Gln His Met Lys Thr His Ala Pro Glu Arg Asp Val Cys

290                 295                 300

Arg Cys Pro Arg Glu Gly Cys Gly Arg Thr Tyr Thr Thr Val Phe Asn

305                 310                 315                 320

Leu Gln Ser His Ile Leu Ser Phe His Glu Glu Ser Arg Pro Phe Val

                325                 330                 335

Cys Glu His Ala Gly Cys Gly Lys Thr Phe Ala Met Lys Gln Ser Leu

            340                 345                 350

Thr Arg His Ala Val Val His Asp Pro Asp Lys Lys Lys Met Lys Leu

        355                 360                 365

Lys Val Lys Lys Ser Arg Glu Lys Arg Glu Phe Gly Leu Ser Ser Gln

    370                 375                 380

Trp Ile Tyr Pro Pro Lys Arg Lys Gln Gly Gln Gly Leu Ser Leu Cys

385                 390                 395                 400

Gln Asn Gly Glu Ser Pro Asn Cys Val Glu Asp Lys Met Leu Ser Thr

                405                 410                 415

Val Ala Val Leu Thr Leu Gly

            420

<210> 84

<211> 339

<212> PRT

<213> Homo sapiens

<400> 84

Met Trp Gln Leu Trp Ala Ser Leu Cys Cys Leu Leu Val Leu Ala Asn

 1               5                  10                  15

Ala Arg Ser Arg Pro Ser Phe His Pro Leu Ser Asp Glu Leu Val Asn

            20                  25                  30

Tyr Val Asn Lys Arg Asn Thr Thr Trp Gln Ala Gly His Asn Phe Tyr
        35                  40                  45
Asn Val Asp Met Ser Tyr Leu Lys Arg Leu Cys Gly Thr Phe Leu Gly
    50                  55                  60
Gly Pro Lys Pro Pro Gln Arg Val Met Phe Thr Glu Asp Leu Lys Leu
65                  70                  75                  80
Pro Ala Ser Phe Asp Ala Arg Glu Gln Trp Pro Gln Cys Pro Thr Ile
                85                  90                  95
Lys Glu Ile Arg Asp Gln Gly Ser Cys Gly Ser Cys Trp Ala Phe Gly
            100                 105                 110
Ala Val Glu Ala Ile Ser Asp Arg Ile Cys Ile His Thr Asn Ala His
        115                 120                 125
Val Ser Val Glu Val Ser Ala Glu Asp Leu Leu Thr Cys Cys Gly Ser
    130                 135                 140
Met Cys Gly Asp Gly Cys Asn Gly Gly Tyr Pro Ala Glu Ala Trp Asn
145                 150                 155                 160
Phe Trp Thr Arg Lys Gly Leu Val Ser Gly Gly Leu Tyr Glu Ser His
                165                 170                 175
Val Gly Cys Arg Pro Tyr Ser Ile Pro Pro Cys Glu His His Val Asn
            180                 185                 190
Gly Ser Arg Pro Pro Cys Thr Gly Glu Gly Asp Thr Pro Lys Cys Ser
        195                 200                 205
Lys Ile Cys Glu Pro Gly Tyr Ser Pro Thr Tyr Lys Gln Asp Lys His
    210                 215                 220
Tyr Gly Tyr Asn Ser Tyr Ser Val Ser Asn Ser Glu Lys Asp Ile Met
225                 230                 235                 240
Ala Glu Ile Tyr Lys Asn Gly Pro Val Glu Gly Ala Phe Ser Val Tyr
                245                 250                 255
Ser Asp Phe Leu Leu Tyr Lys Ser Gly Val Tyr Gln His Val Thr Gly

            260                 265                 270

Glu Met Met Gly Gly His Ala Ile Arg Ile Leu Gly Trp Gly Val Glu

        275                 280                 285

Asn Gly Thr Pro Tyr Trp Leu Val Ala Asn Ser Trp Asn Thr Asp Trp

    290                 295                 300

Gly Asp Asn Gly Phe Phe Lys Ile Leu Arg Gly Gln Asp His Cys Gly

305                 310                 315                 320

Ile Glu Ser Glu Val Val Ala Gly Ile Pro Arg Thr Asp Gln Tyr Trp

                325                 330                 335

Glu Lys Ile

<210> 85

<211> 150

<212> PRT

<213> Homo sapiens

<400> 85

Met Ala Ala Arg Gly Val Ile Ala Pro Val Gly Glu Ser Leu Arg Tyr

1               5                   10                  15

Ala Glu Tyr Leu Gln Pro Ser Ala Lys Arg Pro Asp Ala Asp Val Asp

            20                  25                  30

Gln Gln Gly Leu Val Arg Ser Leu Ile Ala Val Gly Leu Gly Val Ala

        35                  40                  45

Ala Leu Ala Phe Ala Gly Arg Tyr Ala Phe Arg Ile Trp Lys Pro Leu

    50                  55                  60

Glu Gln Val Ile Thr Glu Thr Ala Lys Lys Ile Ser Thr Pro Ser Phe

65                  70                  75                  80

Ser Ser Tyr Tyr Lys Gly Gly Phe Glu Gln Lys Met Ser Arg Arg Glu
                85                  90                  95
Ala Gly Leu Ile Leu Gly Val Ser Pro Ser Ala Gly Lys Ala Lys Ile
            100                 105                 110
Arg Thr Ala His Arg Arg Val Met Ile Leu Asn His Pro Asp Lys Gly
        115                 120                 125
Gly Ser Pro Tyr Val Ala Ala Lys Ile Asn Glu Ala Lys Asp Leu Leu
    130                 135                 140
Glu Thr Thr Thr Lys His
145                 150

<210> 86
<211> 1212
<212> PRT
<213> Homo sapiens

<400> 86

<210> 87

<211> 230

<212> PRT

<213> Homo sapiens

<400> 87

Met Asn Glu Met Tyr Leu Arg Cys Asp His Glu Asn Gln Tyr Ala Gln
1               5                   10                  15

Trp Met Ala Ala Cys Met Leu Ala Ser Lys Gly Lys Thr Met Ala Asp
            20                  25                  30

Ser Ser Tyr Gln Pro Glu Val Leu Asn Ile Leu Ser Phe Leu Arg Met
        35                  40                  45

Lys Asn Arg Asn Ser Ala Ser Gln Val Ala Ser Ser Leu Glu Asn Met
    50                  55                  60

Asp Met Asn Pro Glu Cys Phe Val Ser Pro Arg Cys Ala Lys Arg His
65                  70                  75                  80

Lys Ser Lys Gln Leu Ala Ala Arg Ile Leu Glu Ala His Gln Asn Val
                85                  90                  95

Ala Gln Met Pro Leu Val Glu Ala Lys Leu Arg Phe Ile Gln Ala Trp
            100                 105                 110

Gln Ser Leu Pro Glu Phe Gly Leu Thr Tyr Tyr Leu Val Arg Phe Lys
        115                 120                 125

Gly Ser Lys Lys Asp Asp Ile Leu Gly Val Ser Tyr Asn Arg Leu Ile
    130                 135                 140

Lys Ile Asp Ala Ala Thr Gly Ile Pro Val Thr Thr Trp Arg Phe Thr
145                 150                 155                 160

Asn Ile Lys Gln Trp Asn Val Asn Trp Glu Thr Arg Gln Val Val Ile
                165                 170                 175

Glu Phe Asp Gln Asn Val Phe Thr Ala Phe Thr Cys Leu Ser Ala Asp
            180                 185                 190

Cys Lys Ile Val His Glu Tyr Ile Gly Gly Tyr Ile Phe Leu Ser Thr

195                 200                 205

Arg Ser Lys Asp Gln Asn Glu Thr Leu Asp Glu Asp Leu Phe His Lys

    210                 215                 220

Leu Thr Gly Gly Gln Asp

225                 230

<210> 88

<211> 383

<212> PRT

<213> Homo sapiens

<400> 88

Met Glu Ala Leu Gly Lys Leu Lys Gln Phe Asp Ala Tyr Pro Lys Thr

 1               5                  10                  15

Leu Glu Asp Phe Arg Val Lys Thr Cys Gly Gly Ala Thr Val Thr Ile

            20                  25                  30

Val Ser Gly Leu Leu Met Leu Leu Leu Phe Leu Ser Glu Leu Gln Tyr

        35                  40                  45

Tyr Leu Thr Thr Glu Val His Pro Glu Leu Tyr Val Asp Lys Ser Arg

    50                  55                  60

Gly Asp Lys Leu Lys Ile Asn Ile Asp Val Leu Phe Pro His Met Pro

65                  70                  75                  80

Cys Ala Tyr Leu Ser Ile Asp Ala Met Asp Val Ala Gly Glu Gln Gln

                85                  90                  95

Leu Asp Val Glu His Asn Leu Phe Lys Gln Arg Leu Asp Lys Asp Gly

            100                 105                 110

Ile Pro Val Ser Ser Glu Ala Glu Arg His Glu Leu Gly Lys Val Glu

        115                 120                 125

Val Thr Val Phe Asp Pro Asp Ser Leu Asp Pro Asp Arg Cys Glu Ser
    130                 135                 140
Cys Tyr Gly Ala Glu Ala Glu Asp Ile Lys Cys Cys Asn Thr Cys Glu
145                 150                 155                 160
Asp Val Arg Glu Ala Tyr Arg Arg Arg Gly Trp Ala Phe Lys Asn Pro
                165                 170                 175
Asp Thr Ile Glu Gln Cys Arg Arg Glu Gly Phe Ser Gln Lys Met Gln
            180                 185                 190
Glu Gln Lys Asn Glu Gly Cys Gln Val Tyr Gly Phe Leu Glu Val Asn
        195                 200                 205
Lys Val Ala Gly Asn Phe His Phe Ala Pro Gly Lys Ser Phe Gln Gln
    210                 215                 220
Ser His Val His Val His Asp Leu Gln Ser Phe Gly Leu Asp Asn Ile
225                 230                 235                 240
Asn Met Thr His Tyr Ile Gln His Leu Ser Phe Gly Glu Asp Tyr Pro
                245                 250                 255
Gly Ile Val Asn Pro Leu Asp His Thr Asn Val Thr Ala Pro Gln Ala
            260                 265                 270
Ser Met Met Phe Gln Tyr Phe Val Lys Val Val Pro Thr Val Tyr Met
        275                 280                 285
Lys Val Asp Gly Glu Val Leu Arg Thr Asn Gln Phe Ser Val Thr Arg
    290                 295                 300
His Glu Lys Val Ala Asn Gly Leu Leu Gly Asp Gln Gly Leu Pro Gly
305                 310                 315                 320
Val Phe Val Leu Tyr Glu Leu Ser Pro Met Met Val Lys Leu Thr Glu
                325                 330                 335
Lys His Arg Ser Phe Thr His Phe Leu Thr Gly Val Cys Ala Ile Ile
            340                 345                 350
Gly Gly Met Phe Thr Val Ala Gly Leu Ile Asp Ser Leu Ile Tyr His

355                 360                 365

Ser Ala Arg Ala Ile Gln Lys Lys Ile Asp Leu Gly Lys Thr Thr

    370                 375                 380

<210> 89

<211> 391

<212> PRT

<213> Homo sapiens

<400> 89

Met Ala Asp Ile Asp Asn Lys Glu Gln Ser Glu Leu Asp Gln Asp Leu

 1               5                  10                  15

Asp Asp Val Glu Glu Val Glu Glu Glu Glu Thr Gly Glu Glu Thr Lys

            20                  25                  30

Leu Lys Ala Arg Gln Leu Thr Val Gln Met Met Gln Asn Pro Gln Ile

        35                  40                  45

Leu Ala Ala Leu Gln Glu Arg Leu Asp Gly Leu Val Glu Thr Pro Thr

    50                  55                  60

Gly Tyr Ile Glu Ser Leu Pro Arg Val Val Lys Arg Arg Val Asn Ala

65                  70                  75                  80

Leu Lys Asn Leu Gln Val Lys Cys Ala Gln Ile Glu Ala Lys Phe Tyr

                85                  90                  95

Glu Glu Val His Asp Leu Glu Arg Lys Tyr Ala Val Leu Tyr Gln Pro

            100                 105                 110

Leu Phe Asp Lys Arg Phe Glu Ile Ile Asn Ala Ile Tyr Glu Pro Thr

        115                 120                 125

Glu Glu Glu Cys Glu Trp Lys Pro Asp Glu Glu Asp Glu Ile Ser Glu

    130                 135                 140

Glu Leu Lys Glu Lys Ala Lys Ile Glu Asp Glu Lys Lys Asp Glu Glu
145                 150                 155                 160
Lys Glu Asp Pro Lys Gly Ile Pro Glu Phe Trp Leu Thr Val Phe Lys
                165                 170                 175
Asn Val Asp Leu Leu Ser Asp Met Val Gln Glu His Asp Glu Pro Ile
            180                 185                 190
Leu Lys His Leu Lys Asp Ile Lys Val Lys Phe Ser Asp Ala Gly Gln
        195                 200                 205
Pro Met Ser Phe Val Leu Glu Phe His Phe Glu Pro Asn Glu Tyr Phe
    210                 215                 220
Thr Asn Glu Val Leu Thr Lys Thr Tyr Arg Met Arg Ser Glu Pro Asp
225                 230                 235                 240
Asp Ser Asp Pro Phe Ser Phe Asp Gly Pro Glu Ile Met Gly Cys Thr
                245                 250                 255
Gly Cys Gln Ile Asp Trp Lys Lys Gly Lys Asn Val Thr Leu Lys Thr
            260                 265                 270
Ile Lys Lys Lys Gln Lys His Lys Gly Arg Gly Thr Val Arg Thr Val
        275                 280                 285
Thr Lys Thr Val Ser Asn Asp Ser Phe Phe Asn Phe Phe Ala Pro Pro
    290                 295                 300
Glu Val Pro Glu Ser Gly Asp Leu Asp Asp Asp Ala Glu Ala Ile Leu
305                 310                 315                 320
Ala Ala Asp Phe Glu Ile Gly His Phe Leu Arg Glu Arg Ile Ile Pro
                325                 330                 335
Arg Ser Val Leu Tyr Phe Thr Gly Glu Ala Ile Glu Asp Asp Asp
            340                 345                 350
Asp Tyr Asp Glu Glu Gly Glu Glu Ala Asp Glu Glu Gly Glu Glu Glu
        355                 360                 365
Gly Asp Glu Glu Asn Asp Pro Asp Tyr Asp Pro Lys Lys Asp Gln Asn

370                 375                 380

Pro Ala Glu Cys Lys Gln Gln

385                 390

<210> 90

<211> 836

<212> PRT

<213> Homo sapiens

<400> 90

Met Ile Pro Phe Leu Pro Met Phe Ser Leu Leu Leu Leu Leu Ile Val

1               5                   10                  15

Asn Pro Ile Asn Ala Asn Asn His Tyr Asp Lys Ile Leu Ala His Ser

            20                  25                  30

Arg Ile Arg Gly Arg Asp Gln Gly Pro Asn Val Cys Ala Leu Gln Gln

        35                  40                  45

Ile Leu Gly Thr Lys Lys Lys Tyr Phe Ser Thr Cys Lys Asn Trp Tyr

    50                  55                  60

Lys Lys Ser Ile Cys Gly Gln Lys Thr Thr Val Leu Tyr Glu Cys Cys

65                  70                  75                  80

Pro Gly Tyr Met Arg Met Glu Gly Met Lys Gly Cys Pro Ala Val Leu

                85                  90                  95

Pro Ile Asp His Val Tyr Gly Thr Leu Gly Ile Val Gly Ala Thr Thr

            100                 105                 110

Thr Gln Arg Tyr Ser Asp Ala Ser Lys Leu Arg Glu Glu Ile Glu Gly

        115                 120                 125

Lys Gly Ser Phe Thr Tyr Phe Ala Pro Ser Asn Glu Ala Trp Asp Asn

    130                 135                 140

Leu Asp Ser Asp Ile Arg Arg Gly Leu Glu Ser Asn Val Asn Val Glu
145                 150                 155                 160
Leu Leu Asn Ala Leu His Ser His Met Ile Asn Lys Arg Met Leu Thr
                165                 170                 175
Lys Asp Leu Lys Asn Gly Met Ile Ile Pro Ser Met Tyr Asn Asn Leu
            180                 185                 190
Gly Leu Phe Ile Asn His Tyr Pro Asn Gly Val Val Thr Val Asn Cys
        195                 200                 205
Ala Arg Ile Ile His Gly Asn Gln Ile Ala Thr Asn Gly Val Val His
    210                 215                 220
Val Ile Asp Arg Val Leu Thr Gln Ile Gly Thr Ser Ile Gln Asp Phe
225                 230                 235                 240
Ile Glu Ala Glu Asp Asp Leu Ser Ser Phe Arg Ala Ala Ala Ile Thr
                245                 250                 255
Ser Asp Ile Leu Glu Ala Leu Gly Arg Asp Gly His Phe Thr Leu Phe
            260                 265                 270
Ala Pro Thr Asn Glu Ala Phe Glu Lys Leu Pro Arg Gly Val Leu Glu
        275                 280                 285
Arg Phe Met Gly Asp Lys Val Ala Ser Glu Ala Leu Met Lys Tyr His
    290                 295                 300
Ile Leu Asn Thr Leu Gln Cys Ser Glu Ser Ile Met Gly Gly Ala Val
305                 310                 315                 320
Phe Glu Thr Leu Glu Gly Asn Thr Ile Glu Ile Gly Cys Asp Gly Asp
                325                 330                 335
Ser Ile Thr Val Asn Gly Ile Lys Met Val Asn Lys Lys Asp Ile Val
            340                 345                 350
Thr Asn Asn Gly Val Ile His Leu Ile Asp Gln Val Leu Ile Pro Asp
        355                 360                 365
Ser Ala Lys Gln Val Ile Glu Leu Ala Gly Lys Gln Gln Thr Thr Phe

    370                 375                 380

Thr Asp Leu Val Ala Gln Leu Gly Leu Ala Ser Ala Leu Arg Pro Asp

385                 390                 395                 400

Gly Glu Tyr Thr Leu Leu Ala Pro Val Asn Asn Ala Phe Ser Asp Asp

                405                 410                 415

Thr Leu Ser Met Val Gln Arg Leu Leu Lys Leu Ile Leu Gln Asn His

            420                 425                 430

Ile Leu Lys Val Lys Val Gly Leu Asn Glu Leu Tyr Asn Gly Gln Ile

        435                 440                 445

Leu Glu Thr Ile Gly Gly Lys Gln Leu Arg Val Phe Val Tyr Arg Thr

    450                 455                 460

Ala Val Cys Ile Glu Asn Ser Cys Met Glu Lys Gly Ser Lys Gln Gly

465                 470                 475                 480

Arg Asn Gly Ala Ile His Ile Phe Arg Glu Ile Ile Lys Pro Ala Glu

                485                 490                 495

Lys Ser Leu His Glu Lys Leu Lys Gln Asp Lys Arg Phe Ser Thr Phe

            500                 505                 510

Leu Ser Leu Leu Glu Ala Ala Asp Leu Lys Glu Leu Leu Thr Gln Pro

        515                 520                 525

Gly Asp Trp Thr Leu Phe Val Pro Thr Asn Asp Ala Phe Lys Gly Met

    530                 535                 540

Thr Ser Glu Glu Lys Glu Ile Leu Ile Arg Asp Lys Asn Ala Leu Gln

545                 550                 555                 560

Asn Ile Ile Leu Tyr His Leu Thr Pro Gly Val Phe Ile Gly Lys Gly

                565                 570                 575

Phe Glu Pro Gly Val Thr Asn Ile Leu Lys Thr Thr Gln Gly Ser Lys

            580                 585                 590

Ile Phe Leu Lys Glu Val Asn Asp Thr Leu Leu Val Asn Glu Leu Lys

        595                 600                 605

Ser Lys Glu Ser Asp Ile Met Thr Thr Asn Gly Val Ile His Val Val
    610                 615                 620
Asp Lys Leu Leu Tyr Pro Ala Asp Thr Pro Val Gly Asn Asp Gln Leu
625                 630                 635                 640
Leu Glu Ile Leu Asn Lys Leu Ile Lys Tyr Ile Gln Ile Lys Phe Val
                645                 650                 655
Arg Gly Ser Thr Phe Lys Glu Ile Pro Val Thr Val Tyr Thr Thr Lys
            660                 665                 670
Ile Ile Thr Lys Val Val Glu Pro Lys Ile Lys Val Ile Glu Gly Ser
        675                 680                 685
Leu Gln Pro Ile Ile Lys Thr Glu Gly Pro Thr Leu Thr Lys Val Lys
    690                 695                 700
Ile Glu Gly Glu Pro Glu Phe Arg Leu Ile Lys Glu Gly Glu Thr Ile
705                 710                 715                 720
Thr Glu Val Ile His Gly Glu Pro Ile Ile Lys Lys Tyr Thr Lys Ile
                725                 730                 735
Ile Asp Gly Val Pro Val Glu Ile Thr Glu Lys Glu Thr Arg Glu Glu
            740                 745                 750
Arg Ile Ile Thr Gly Pro Glu Ile Lys Tyr Thr Arg Ile Ser Thr Gly
        755                 760                 765
Gly Gly Glu Thr Glu Glu Thr Leu Lys Lys Leu Leu Gln Glu Glu Val
    770                 775                 780
Thr Lys Val Thr Lys Phe Ile Glu Gly Gly Asp Gly His Leu Phe Glu
785                 790                 795                 800
Asp Glu Glu Ile Lys Arg Leu Leu Gln Gly Asp Thr Pro Val Arg Lys
                805                 810                 815
Leu Gln Ala Asn Lys Lys Val Gln Gly Ser Arg Arg Arg Leu Arg Glu
            820                 825                 830
Gly Arg Ser Gln

        835

<210> 91

<211> 3176

<212> PRT

<213> Homo sapiens

<400> 91

Met Arg Lys His Arg His Leu Pro Leu Val Ala Val Phe Cys Leu Phe
1               5                   10                  15

Leu Ser Gly Phe Pro Thr Thr His Ala Gln Gln Gln Gln Ala Asp Val
            20                  25                  30

Lys Asn Gly Ala Ala Ala Asp Ile Ile Phe Leu Val Asp Ser Ser Trp
        35                  40                  45

Thr Ile Gly Glu Glu His Phe Gln Leu Val Arg Glu Phe Leu Tyr Asp
    50                  55                  60

Val Val Lys Ser Leu Ala Val Gly Glu Asn Asp Phe His Phe Ala Leu
65                  70                  75                  80

Val Gln Phe Asn Gly Asn Pro His Thr Glu Phe Leu Leu Asn Thr Tyr
                85                  90                  95

Arg Thr Lys Gln Glu Val Leu Ser His Ile Ser Asn Met Ser Tyr Ile
            100                 105                 110

Gly Gly Thr Asn Gln Thr Gly Lys Gly Leu Glu Tyr Ile Met Gln Ser
        115                 120                 125

His Leu Thr Lys Ala Ala Gly Ser Arg Ala Gly Asp Gly Val Pro Gln
    130                 135                 140

Val Ile Val Val Leu Thr Asp Gly His Ser Lys Asp Gly Leu Ala Leu
145                 150                 155                 160

Pro Ser Ala Glu Leu Lys Ser Ala Asp Val Asn Val Phe Ala Ile Gly
                165                 170                 175
Val Glu Asp Ala Asp Glu Gly Ala Leu Lys Glu Ile Ala Ser Glu Pro
            180                 185                 190
Leu Asn Met His Met Phe Asn Leu Glu Asn Phe Thr Ser Leu His Asp
        195                 200                 205
Ile Val Gly Asn Leu Val Ser Cys Val His Ser Ser Val Ser Pro Glu
    210                 215                 220
Arg Ala Gly Asp Thr Glu Thr Leu Lys Asp Ile Thr Ala Gln Asp Ser
225                 230                 235                 240
Ala Asp Ile Ile Phe Leu Ile Asp Gly Ser Asn Asn Thr Gly Ser Val
                245                 250                 255
Asn Phe Ala Val Ile Leu Asp Phe Leu Val Asn Leu Leu Glu Lys Leu
            260                 265                 270
Pro Ile Gly Thr Gln Gln Ile Arg Val Gly Val Val Gln Phe Ser Asp
        275                 280                 285
Glu Pro Arg Thr Met Phe Ser Leu Asp Thr Tyr Ser Thr Lys Ala Gln
    290                 295                 300
Val Leu Gly Ala Val Lys Ala Leu Gly Phe Ala Gly Gly Glu Leu Ala
305                 310                 315                 320
Asn Ile Gly Leu Ala Leu Asp Phe Val Val Glu Asn His Phe Thr Arg
                325                 330                 335
Ala Gly Gly Ser Arg Val Glu Glu Gly Val Pro Gln Val Leu Val Leu
            340                 345                 350
Ile Ser Ala Gly Pro Ser Ser Asp Glu Ile Arg Tyr Gly Val Val Ala
        355                 360                 365
Leu Lys Gln Ala Ser Val Phe Ser Phe Gly Leu Gly Ala Gln Ala Ala
    370                 375                 380
Ser Arg Ala Glu Leu Gln His Ile Ala Thr Asp Asp Asn Leu Val Phe

385                 390                 395                 400

Thr Val Pro Glu Phe Arg Ser Phe Gly Asp Leu Gln Glu Lys Leu Leu

                405                 410                 415

Pro Tyr Ile Val Gly Val Ala Gln Arg His Ile Val Leu Lys Pro Pro

            420                 425                 430

Thr Ile Val Thr Gln Val Ile Glu Val Asn Lys Arg Asp Ile Val Phe

        435                 440                 445

Leu Val Asp Gly Ser Ser Ala Leu Gly Leu Ala Asn Phe Asn Ala Ile

    450                 455                 460

Arg Asp Phe Ile Ala Lys Val Ile Gln Arg Leu Glu Ile Gly Gln Asp

465                 470                 475                 480

Leu Ile Gln Val Ala Val Ala Gln Tyr Ala Asp Thr Val Arg Pro Glu

                485                 490                 495

Phe Tyr Phe Asn Thr His Pro Thr Lys Arg Glu Val Ile Thr Ala Val

            500                 505                 510

Arg Lys Met Lys Pro Leu Asp Gly Ser Ala Leu Tyr Thr Gly Ser Ala

        515                 520                 525

Leu Asp Phe Val Arg Asn Asn Leu Phe Thr Ser Ser Ala Gly Tyr Arg

    530                 535                 540

Ala Ala Glu Gly Ile Pro Lys Leu Leu Val Leu Ile Thr Gly Gly Lys

545                 550                 555                 560

Ser Leu Asp Glu Ile Ser Gln Pro Ala Gln Glu Leu Lys Arg Ser Ser

                565                 570                 575

Ile Met Ala Phe Ala Ile Gly Asn Lys Gly Ala Asp Gln Ala Glu Leu

            580                 585                 590

Glu Glu Ile Ala Phe Asp Ser Ser Leu Val Phe Ile Pro Ala Glu Phe

        595                 600                 605

Arg Ala Ala Pro Leu Gln Gly Met Leu Pro Gly Leu Leu Ala Pro Leu

    610                 615                 620

Arg Thr Leu Ser Gly Thr Pro Glu Val His Ser Asn Lys Arg Asp Ile
625                 630                 635                 640
Ile Phe Leu Leu Asp Gly Ser Ala Asn Val Gly Lys Thr Asn Phe Pro
                645                 650                 655
Tyr Val Arg Asp Phe Val Met Asn Leu Val Asn Ser Leu Asp Ile Gly
            660                 665                 670
Asn Asp Asn Ile Arg Val Gly Leu Val Gln Phe Ser Asp Thr Pro Val
        675                 680                 685
Thr Glu Phe Ser Leu Asn Thr Tyr Gln Thr Lys Ser Asp Ile Leu Gly
    690                 695                 700
His Leu Arg Gln Leu Gln Leu Gln Gly Gly Ser Gly Leu Asn Thr Gly
705                 710                 715                 720
Ser Ala Leu Ser Tyr Val Tyr Ala Asn His Phe Thr Glu Ala Gly Gly
                725                 730                 735
Ser Arg Ile Arg Glu His Val Pro Gln Leu Leu Leu Leu Leu Thr Ala
            740                 745                 750
Gly Gln Ser Glu Asp Ser Tyr Leu Gln Ala Ala Asn Ala Leu Thr Arg
        755                 760                 765
Ala Gly Ile Leu Thr Phe Cys Val Gly Ala Ser Gln Ala Asn Lys Ala
    770                 775                 780
Glu Leu Glu Gln Ile Ala Phe Asn Pro Ser Leu Val Tyr Leu Met Asp
785                 790                 795                 800
Asp Phe Ser Ser Leu Pro Ala Leu Pro Gln Gln Leu Ile Gln Pro Leu
                805                 810                 815
Thr Thr Tyr Val Ser Gly Gly Val Glu Glu Val Pro Leu Ala Gln Pro
            820                 825                 830
Glu Ser Lys Arg Asp Ile Leu Phe Leu Phe Asp Gly Ser Ala Asn Leu
        835                 840                 845
Val Gly Gln Phe Pro Val Val Arg Asp Phe Leu Tyr Lys Ile Ile Asp

    850                 855                 860

Glu Leu Asn Val Lys Pro Glu Gly Thr Arg Ile Ala Val Ala Gln Tyr

865                 870                 875                 880

Ser Asp Asp Val Lys Val Glu Ser Arg Phe Asp Glu His Gln Ser Lys

                885                 890                 895

Pro Glu Ile Leu Asn Leu Val Lys Arg Met Lys Ile Lys Thr Gly Lys

            900                 905                 910

Ala Leu Asn Leu Gly Tyr Ala Leu Asp Tyr Ala Gln Arg Tyr Ile Phe

        915                 920                 925

Val Lys Ser Ala Gly Ser Arg Ile Glu Asp Gly Val Leu Gln Phe Leu

    930                 935                 940

Val Leu Leu Val Ala Gly Arg Ser Ser Asp Arg Val Asp Gly Pro Ala

945                 950                 955                 960

Ser Asn Leu Lys Gln Ser Gly Val Val Pro Phe Ile Phe Gln Ala Lys

                965                 970                 975

Asn Ala Asp Pro Ala Glu Leu Glu Gln Ile Val Leu Ser Pro Ala Phe

            980                 985                 990

Ile Leu Ala Ala Glu Ser Leu Pro Lys Ile Gly Asp Leu His Pro Gln

        995                 1000                1005

Ile Val Asn Leu Leu Lys Ser Val His Asn Gly Ala Pro Ala Pro Val

    1010                1015                1020

Ser Gly Glu Lys Asp Val Val Phe Leu Leu Asp Gly Ser Glu Gly Val

1025                1030                1035                1040

Arg Ser Gly Phe Pro Leu Leu Lys Glu Phe Val Gln Arg Val Val Glu

                1045                1050                1055

Ser Leu Asp Val Gly Gln Asp Arg Val Arg Val Ala Val Val Gln Tyr

            1060                1065                1070

Ser Asp Arg Thr Arg Pro Glu Phe Tyr Leu Asn Ser Tyr Met Asn Lys

        1075                1080                1085

Gln Asp Val Val Asn Ala Val Arg Gln Leu Thr Leu Leu Gly Gly Pro
    1090                1095                1100
Thr Pro Asn Thr Gly Ala Ala Leu Glu Phe Val Leu Arg Asn Ile Leu
1105                1110                1115                1120
Val Ser Ser Ala Gly Ser Arg Ile Thr Glu Gly Val Pro Gln Leu Leu
                1125                1130                1135
Ile Val Leu Thr Ala Asp Arg Ser Gly Asp Asp Val Arg Asn Pro Ser
            1140                1145                1150
Val Val Val Lys Arg Gly Gly Ala Val Pro Ile Gly Ile Gly Ile Gly
        1155                1160                1165
Asn Ala Asp Ile Thr Glu Met Gln Thr Ile Ser Phe Ile Pro Asp Phe
    1170                1175                1180
Ala Val Ala Ile Pro Thr Phe Arg Gln Leu Gly Thr Val Gln Gln Val
1185                1190                1195                1200
Ile Ser Glu Arg Val Thr Gln Leu Thr Arg Glu Glu Leu Ser Arg Leu
                1205                1210                1215
Gln Pro Val Leu Gln Pro Leu Pro Ser Pro Gly Val Gly Gly Lys Arg
            1220                1225                1230
Asp Val Val Phe Leu Ile Asp Gly Ser Gln Ser Ala Gly Pro Glu Phe
        1235                1240                1245
Gln Tyr Val Arg Thr Leu Ile Glu Arg Leu Val Asp Tyr Leu Asp Val
    1250                1255                1260
Gly Phe Asp Thr Thr Arg Val Ala Val Ile Gln Phe Ser Asp Asp Pro
1265                1270                1275                1280
Lys Ala Glu Phe Leu Leu Asn Ala His Ser Ser Lys Asp Glu Val Gln
                1285                1290                1295
Asn Ala Val Gln Arg Leu Arg Pro Lys Gly Gly Arg Gln Ile Asn Val
            1300                1305                1310
Gly Asn Ala Leu Glu Tyr Val Ser Arg Asn Ile Phe Lys Arg Pro Leu

1315                1320                1325

Gly Ser Arg Ile Glu Glu Gly Val Pro Gln Phe Leu Val Leu Ile Ser

    1330                1335                1340

Ser Gly Lys Ser Asp Asp Glu Val Val Val Pro Ala Val Glu Leu Lys

1345                1350                1355                1360

Gln Phe Gly Val Ala Pro Phe Thr Ile Ala Arg Asn Ala Asp Gln Glu

                1365                1370                1375

Glu Leu Val Lys Ile Ser Leu Ser Pro Glu Tyr Val Phe Ser Val Ser

            1380                1385                1390

Thr Phe Arg Glu Leu Pro Ser Leu Glu Gln Lys Leu Leu Thr Pro Ile

        1395                1400                1405

Thr Thr Leu Thr Ser Glu Gln Ile Gln Lys Leu Leu Ala Ser Thr Arg

    1410                1415                1420

Tyr Pro Pro Pro Ala Val Glu Ser Asp Ala Ala Asp Ile Val Phe Leu

1425                1430                1435                1440

Ile Asp Ser Ser Glu Gly Val Arg Pro Asp Gly Phe Ala His Ile Arg

                1445                1450                1455

Asp Phe Val Ser Arg Ile Val Arg Arg Leu Asn Ile Gly Pro Ser Lys

            1460                1465                1470

Val Arg Val Gly Val Val Gln Phe Ser Asn Asp Val Phe Pro Glu Phe

        1475                1480                1485

Tyr Leu Lys Thr Tyr Arg Ser Gln Ala Pro Val Leu Asp Ala Ile Arg

    1490                1495                1500

Arg Leu Arg Leu Arg Gly Gly Ser Pro Leu Asn Thr Gly Lys Ala Leu

1505                1510                1515                1520

Glu Phe Val Ala Arg Asn Leu Phe Val Lys Ser Ala Gly Ser Arg Ile

                1525                1530                1535

Glu Asp Gly Val Pro Gln His Leu Val Leu Val Leu Gly Gly Lys Ser

            1540                1545                1550

Gln Asp Asp Val Ser Arg Phe Ala Gln Val Ile Arg Ser Ser Gly Ile
        1555                1560                1565
Val Ser Leu Gly Val Gly Asp Arg Asn Ile Asp Arg Thr Glu Leu Gln
    1570                1575                1580
Thr Ile Thr Asn Asp Pro Arg Leu Val Phe Thr Val Arg Glu Phe Arg
1585                1590                1595                1600
Glu Leu Pro Asn Ile Glu Glu Arg Ile Met Asn Ser Phe Gly Pro Ser
                1605                1610                1615
Ala Ala Thr Pro Ala Pro Pro Gly Val Asp Thr Pro Pro Pro Ser Arg
            1620                1625                1630
Pro Glu Lys Lys Lys Ala Asp Ile Val Phe Leu Leu Asp Gly Ser Ile
        1635                1640                1645
Asn Phe Arg Arg Asp Ser Phe Gln Glu Val Leu Arg Phe Val Ser Glu
    1650                1655                1660
Ile Val Asp Thr Val Tyr Glu Asp Gly Asp Ser Ile Gln Val Gly Leu
1665                1670                1675                1680
Val Gln Tyr Asn Ser Asp Pro Thr Asp Glu Phe Phe Leu Lys Asp Phe
                1685                1690                1695
Ser Thr Lys Arg Gln Ile Ile Asp Ala Ile Asn Lys Val Val Tyr Lys
            1700                1705                1710
Gly Gly Arg His Ala Asn Thr Lys Val Gly Leu Glu His Leu Arg Val
        1715                1720                1725
Asn His Phe Val Pro Glu Ala Gly Ser Arg Leu Asp Gln Arg Val Pro
    1730                1735                1740
Gln Ile Ala Phe Val Ile Thr Gly Gly Lys Ser Val Glu Asp Ala Gln
1745                1750                1755                1760
Asp Val Ser Leu Ala Leu Thr Gln Arg Gly Val Lys Val Phe Ala Val
                1765                1770                1775
Gly Val Arg Asn Ile Asp Ser Glu Glu Val Gly Lys Ile Ala Ser Asn

1780                1785                1790

Ser Ala Thr Ala Phe Arg Val Gly Asn Val Gln Glu Leu Ser Glu Leu

        1795                1800                1805

Ser Glu Gln Val Leu Glu Thr Leu His Asp Ala Met His Glu Thr Leu

    1810                1815                1820

Cys Pro Gly Val Thr Asp Ala Ala Lys Ala Cys Asn Leu Asp Val Ile

1825                1830                1835                1840

Leu Gly Phe Asp Gly Ser Arg Asp Gln Asn Val Phe Val Ala Gln Lys

                1845                1850                1855

Gly Phe Glu Ser Lys Val Asp Ala Ile Leu Asn Arg Ile Ser Gln Met

            1860                1865                1870

His Arg Val Ser Cys Ser Gly Gly Arg Ser Pro Thr Val Arg Val Ser

        1875                1880                1885

Val Val Ala Asn Thr Pro Ser Gly Pro Val Glu Ala Phe Asp Phe Asp

    1890                1895                1900

Glu Tyr Gln Pro Glu Met Leu Glu Lys Phe Arg Asn Met Arg Ser Gln

1905                1910                1915                1920

His Pro Tyr Val Leu Thr Glu Asp Thr Leu Lys Val Tyr Leu Asn Lys

                1925                1930                1935

Phe Arg Gln Ser Ser Pro Asp Ser Val Lys Val Val Ile His Phe Thr

            1940                1945                1950

Asp Gly Ala Asp Gly Asp Leu Ala Asp Leu His Arg Ala Ser Glu Asn

        1955                1960                1965

Leu Arg Gln Glu Gly Val Arg Ala Leu Ile Leu Val Gly Leu Glu Arg

    1970                1975                1980

Val Val Asn Leu Glu Arg Leu Met His Leu Glu Phe Gly Arg Gly Phe

1985                1990                1995                2000

Met Tyr Asp Arg Pro Leu Arg Leu Asn Leu Leu Asp Leu Asp Tyr Glu

                2005                2010                2015

Leu Ala Glu Gln Leu Asp Asn Ile Ala Glu Lys Ala Cys Cys Gly Val
            2020                2025                2030
Pro Cys Lys Cys Ser Gly Gln Arg Gly Asp Arg Gly Pro Ile Gly Ser
        2035                2040                2045
Ile Gly Pro Lys Gly Ile Pro Gly Glu Asp Gly Tyr Arg Gly Tyr Pro
    2050                2055                2060
Gly Asp Glu Gly Gly Pro Gly Glu Arg Gly Pro Pro Gly Val Asn Gly
2065                2070                2075                2080
Thr Gln Gly Phe Gln Gly Cys Pro Gly Gln Arg Gly Val Lys Gly Ser
                2085                2090                2095
Arg Gly Phe Pro Gly Glu Lys Gly Glu Val Gly Glu Ile Gly Leu Asp
            2100                2105                2110
Gly Leu Asp Gly Glu Asp Gly Asp Lys Gly Leu Pro Gly Ser Ser Gly
        2115                2120                2125
Glu Lys Gly Asn Pro Gly Arg Arg Gly Asp Lys Gly Pro Arg Gly Glu
    2130                2135                2140
Lys Gly Glu Arg Gly Asp Val Gly Ile Arg Gly Asp Pro Gly Asn Pro
2145                2150                2155                2160
Gly Gln Asp Ser Gln Glu Arg Gly Pro Lys Gly Glu Thr Gly Asp Leu
                2165                2170                2175
Gly Pro Met Gly Val Pro Gly Arg Asp Gly Val Pro Gly Gly Pro Gly
            2180                2185                2190
Glu Thr Gly Lys Asn Gly Gly Phe Gly Arg Arg Gly Pro Pro Gly Ala
        2195                2200                2205
Lys Gly Asn Lys Gly Gly Pro Gly Gln Pro Gly Phe Glu Gly Glu Gln
    2210                2215                2220
Gly Thr Arg Gly Ala Gln Gly Pro Ala Gly Pro Ala Gly Pro Pro Gly
2225                2230                2235                2240
Leu Ile Gly Glu Gln Gly Ile Ser Gly Pro Arg Gly Ser Gly Gly Ala

Thr Ser Lys Gln Gln Ser Leu Glu Thr Ala Met Ser Phe Val Ala Arg
2485 2490 2495
Asn Thr Phe Lys Arg Val Arg Asn Gly Phe Leu Met Arg Lys Val Ala
2500 2505 2510
Val Phe Phe Ser Asn Thr Pro Thr Arg Ala Ser Pro Gln Leu Arg Glu
2515 2520 2525
Ala Val Leu Lys Leu Ser Asp Ala Gly Ile Thr Pro Leu Phe Leu Thr
2530 2535 2540
Arg Gln Glu Asp Arg Gln Leu Ile Asn Ala Leu Gln Ile Asn Asn Thr
2545 2550 2555 2560
Ala Val Gly His Ala Leu Val Leu Pro Ala Gly Arg Asp Leu Thr Asp
2565 2570 2575
Phe Leu Glu Asn Val Leu Thr Cys His Val Cys Leu Asp Ile Cys Asn
2580 2585 2590
Ile Asp Pro Ser Cys Gly Phe Gly Ser Trp Arg Pro Ser Phe Arg Asp
2595 2600 2605
Arg Arg Ala Ala Gly Ser Asp Val Asp Ile Asp Met Ala Phe Ile Leu
2610 2615 2620
Asp Ser Ala Glu Thr Thr Thr Leu Phe Gln Phe Asn Glu Met Lys Lys
2625 2630 2635 2640
Tyr Ile Ala Tyr Leu Val Arg Gln Leu Asp Met Ser Pro Asp Pro Lys
2645 2650 2655
Ala Ser Gln His Phe Ala Arg Val Ala Val Val Gln His Ala Pro Ser
2660 2665 2670
Glu Ser Val Asp Asn Ala Ser Met Pro Pro Val Lys Val Glu Phe Ser
2675 2680 2685
Leu Thr Asp Tyr Gly Ser Lys Glu Lys Leu Val Asp Phe Leu Ser Arg
2690 2695 2700
Gly Met Thr Gln Leu Gln Gly Thr Arg Ala Leu Gly Ser Ala Ile Glu

2705                2710                2715                2720

Tyr Thr Ile Glu Asn Val Phe Glu Ser Ala Pro Asn Pro Arg Asp Leu

                2725                2730                2735

Lys Ile Val Val Leu Met Leu Thr Gly Glu Val Pro Glu Gln Gln Leu

            2740                2745                2750

Glu Glu Ala Gln Arg Val Ile Leu Gln Ala Lys Cys Lys Gly Tyr Phe

        2755                2760                2765

Phe Val Val Leu Gly Ile Gly Arg Lys Val Asn Ile Lys Glu Val Tyr

    2770                2775                2780

Thr Phe Ala Ser Glu Pro Asn Asp Val Phe Phe Lys Leu Val Asp Lys

2785                2790                2795                2800

Ser Thr Glu Leu Asn Glu Glu Pro Leu Met Arg Phe Gly Arg Leu Leu

                2805                2810                2815

Pro Ser Phe Val Ser Ser Glu Asn Ala Phe Tyr Leu Ser Pro Asp Ile

            2820                2825                2830

Arg Lys Gln Cys Asp Trp Phe Gln Gly Asp Gln Pro Thr Lys Asn Leu

        2835                2840                2845

Val Lys Phe Gly His Lys Gln Val Asn Val Pro Asn Asn Val Thr Ser

    2850                2855                2860

Ser Pro Thr Ser Asn Pro Val Thr Thr Thr Lys Pro Val Thr Thr Thr

2865                2870                2875                2880

Lys Pro Val Thr Thr Thr Thr Lys Pro Val Thr Thr Thr Thr Lys Pro

                2885                2890                2895

Val Thr Ile Ile Asn Gln Pro Ser Val Lys Pro Ala Ala Ala Lys Pro

            2900                2905                2910

Ala Pro Ala Lys Pro Val Ala Ala Lys Pro Val Ala Thr Lys Thr Ala

        2915                2920                2925

Thr Val Arg Pro Pro Val Ala Val Lys Pro Ala Thr Ala Ala Lys Pro

    2930                2935                2940

Val Ala Ala Lys Pro Ala Ala Val Arg Pro Pro Ala Ala Ala Ala Lys
2945                2950                2955                2960
Pro Val Ala Thr Lys Pro Glu Val Pro Arg Pro Gln Ala Ala Lys Pro
                2965                2970                2975
Ala Ala Thr Lys Pro Ala Thr Thr Lys Pro Val Val Lys Met Leu Arg
            2980                2985                2990
Glu Val Gln Val Phe Glu Ile Thr Glu Asn Ser Ala Lys Leu His Trp
        2995                3000                3005
Glu Arg Pro Glu Pro Pro Gly Pro Tyr Phe Tyr Asp Leu Thr Val Thr
    3010                3015                3020
Ser Ala His Asp Gln Ser Leu Val Leu Lys Gln Asn Leu Thr Val Thr
3025                3030                3035                3040
Asp Arg Val Ile Gly Gly Leu Leu Ala Gly Gln Thr Tyr His Val Ala
                3045                3050                3055
Val Val Cys Tyr Leu Arg Ser Gln Val Arg Ala Thr Tyr His Gly Ser
            3060                3065                3070
Phe Ser Thr Lys Lys Ser Gln Pro Pro Pro Pro Gln Pro Ala Arg Ser
        3075                3080                3085
Ala Ser Ser Ser Thr Ile Asn Leu Met Val Ser Thr Glu Pro Leu Ala
    3090                3095                3100
Leu Thr Glu Thr Asp Ile Cys Lys Leu Pro Lys Asp Glu Gly Thr Cys
3105                3110                3115                3120
Arg Asp Phe Ile Leu Lys Trp Tyr Tyr Asp Pro Asn Thr Lys Ser Cys
                3125                3130                3135
Ala Arg Phe Trp Tyr Gly Gly Cys Gly Gly Asn Glu Asn Lys Phe Gly
            3140                3145                3150
Ser Gln Lys Glu Cys Glu Lys Val Cys Ala Pro Val Leu Ala Lys Pro
        3155                3160                3165
Gly Val Ile Ser Val Met Gly Thr

3170                3175

<210> 92

<211> 303

<212> PRT

<213> Homo sapiens

<400> 92

Met Arg Ala Trp Ile Phe Phe Leu Leu Cys Leu Ala Gly Arg Ala Leu

1               5                   10                  15

Ala Ala Pro Gln Gln Glu Ala Leu Pro Asp Glu Thr Glu Val Val Glu

            20                  25                  30

Glu Thr Val Ala Glu Val Thr Glu Val Ser Val Gly Ala Asn Pro Val

        35                  40                  45

Gln Val Glu Val Gly Glu Phe Asp Asp Gly Ala Glu Glu Thr Glu Glu

    50                  55                  60

Glu Val Val Ala Glu Asn Pro Cys Gln Asn His His Cys Lys His Gly

65                  70                  75                  80

Lys Val Cys Glu Leu Asp Glu Asn Asn Thr Pro Met Cys Val Cys Gln

                85                  90                  95

Asp Pro Thr Ser Cys Pro Ala Pro Ile Gly Glu Phe Glu Lys Val Cys

            100                 105                 110

Ser Asn Asp Asn Lys Thr Phe Asp Ser Ser Cys His Phe Phe Ala Thr

        115                 120                 125

Lys Cys Thr Leu Glu Gly Thr Lys Lys Gly His Lys Leu His Leu Asp

    130                 135                 140

Tyr Ile Gly Pro Cys Lys Tyr Ile Pro Pro Cys Leu Asp Ser Glu Leu

145                 150                 155                 160

Thr Glu Phe Pro Leu Arg Met Arg Asp Trp Leu Lys Asn Val Leu Val
                165                 170                 175
Thr Leu Tyr Glu Arg Asp Glu Asp Asn Asn Leu Leu Thr Glu Lys Gln
            180                 185                 190
Lys Leu Arg Val Lys Lys Ile His Glu Asn Glu Lys Arg Leu Glu Ala
        195                 200                 205
Gly Asp His Pro Val Glu Leu Leu Ala Arg Asp Phe Glu Lys Asn Tyr
    210                 215                 220
Asn Met Tyr Ile Phe Pro Val His Trp Gln Phe Gly Gln Leu Asp Gln
225                 230                 235                 240
His Pro Ile Asp Gly Tyr Leu Ser His Thr Glu Leu Ala Pro Leu Arg
                245                 250                 255
Ala Pro Leu Ile Pro Met Glu His Cys Thr Thr Arg Phe Phe Glu Thr
            260                 265                 270
Cys Asp Leu Asp Asn Asp Lys Tyr Ile Ala Leu Asp Glu Trp Ala Gly
        275                 280                 285
Cys Phe Gly Ile Lys Gln Lys Asp Ile Asp Lys Asp Leu Val Ile
    290                 295                 300

<210> 93
<211> 683
<212> PRT
<213> Homo sapiens

<400> 93

<210> 94

<211> 2355

<212> PRT

<213> Homo sapiens

<400> 94

Met Leu Arg Gly Pro Gly Pro Gly Leu Leu Leu Leu Ala Val Gln Cys
1               5                   10                  15

Leu Gly Thr Ala Val Pro Ser Thr Gly Ala Ser Lys Ser Lys Arg Gln
            20                  25                  30

Ala Gln Gln Met Val Gln Pro Gln Ser Pro Val Ala Val Ser Gln Ser
        35                  40                  45

Lys Pro Gly Cys Tyr Asp Asn Gly Lys His Tyr Gln Ile Asn Gln Gln
    50                  55                  60

Trp Glu Arg Thr Tyr Leu Gly Asn Ala Leu Val Cys Thr Cys Tyr Gly
65                  70                  75                  80

Gly Ser Arg Gly Phe Asn Cys Glu Ser Lys Pro Glu Ala Glu Glu Thr
                85                  90                  95

Cys Phe Asp Lys Tyr Thr Gly Asn Thr Tyr Arg Val Gly Asp Thr Tyr
            100                 105                 110

Glu Arg Pro Lys Asp Ser Met Ile Trp Asp Cys Thr Cys Ile Gly Ala
        115                 120                 125

Gly Arg Gly Arg Ile Ser Cys Thr Ile Ala Asn Arg Cys His Glu Gly
    130                 135                 140

Gly Gln Ser Tyr Lys Ile Gly Asp Thr Trp Arg Arg Pro His Glu Thr
145                 150                 155                 160

Gly Gly Tyr Met Leu Glu Cys Val Cys Leu Gly Asn Gly Lys Gly Glu
                165                 170                 175

Trp Thr Cys Lys Pro Ile Ala Glu Lys Cys Phe Asp His Ala Ala Gly
            180                 185                 190

Thr Ser Tyr Val Val Gly Glu Thr Trp Glu Lys Pro Tyr Gln Gly Trp

195                 200                 205

Met Met Val Asp Cys Thr Cys Leu Gly Glu Gly Ser Gly Arg Ile Thr

    210                 215                 220

Cys Thr Ser Arg Asn Arg Cys Asn Asp Gln Asp Thr Arg Thr Ser Tyr

225                 230                 235                 240

Arg Ile Gly Asp Thr Trp Ser Lys Lys Asp Asn Arg Gly Asn Leu Leu

                245                 250                 255

Gln Cys Ile Cys Thr Gly Asn Gly Arg Gly Glu Trp Lys Cys Glu Arg

            260                 265                 270

His Thr Ser Val Gln Thr Thr Ser Ser Gly Ser Gly Pro Phe Thr Asp

        275                 280                 285

Val Arg Ala Ala Val Tyr Gln Pro Gln Pro His Pro Gln Pro Pro Pro

    290                 295                 300

Tyr Gly His Cys Val Thr Asp Ser Gly Val Val Tyr Ser Val Gly Met

305                 310                 315                 320

Gln Trp Leu Lys Thr Gln Gly Asn Lys Gln Met Leu Cys Thr Cys Leu

                325                 330                 335

Gly Asn Gly Val Ser Cys Gln Glu Thr Ala Val Thr Gln Thr Tyr Gly

            340                 345                 350

Gly Asn Ser Asn Gly Glu Pro Cys Val Leu Pro Phe Thr Tyr Asn Gly

        355                 360                 365

Arg Thr Phe Tyr Ser Cys Thr Thr Glu Gly Arg Gln Asp Gly His Leu

    370                 375                 380

Trp Cys Ser Thr Thr Ser Asn Tyr Glu Gln Asp Gln Lys Tyr Ser Phe

385                 390                 395                 400

Cys Thr Asp His Thr Val Leu Val Gln Thr Arg Gly Gly Asn Ser Asn

                405                 410                 415

Gly Ala Leu Cys His Phe Pro Phe Leu Tyr Asn Asn His Asn Tyr Thr

            420                 425                 430

Asp Cys Thr Ser Glu Gly Arg Arg Asp Asn Met Lys Trp Cys Gly Thr
        435                 440                 445
Thr Gln Asn Tyr Asp Ala Asp Gln Lys Phe Gly Phe Cys Pro Met Ala
    450                 455                 460
Ala His Glu Glu Ile Cys Thr Thr Asn Glu Gly Val Met Tyr Arg Ile
465                 470                 475                 480
Gly Asp Gln Trp Asp Lys Gln His Asp Met Gly His Met Met Arg Cys
                485                 490                 495
Thr Cys Val Gly Asn Gly Arg Gly Glu Trp Thr Cys Ile Ala Tyr Ser
            500                 505                 510
Gln Leu Arg Asp Gln Cys Ile Val Asp Asp Ile Thr Tyr Asn Val Asn
        515                 520                 525
Asp Thr Phe His Lys Arg His Glu Glu Gly His Met Leu Asn Cys Thr
    530                 535                 540
Cys Phe Gly Gln Gly Arg Gly Arg Trp Lys Cys Asp Pro Val Asp Gln
545                 550                 555                 560
Cys Gln Asp Ser Glu Thr Gly Thr Phe Tyr Gln Ile Gly Asp Ser Trp
                565                 570                 575
Glu Lys Tyr Val His Gly Val Arg Tyr Gln Cys Tyr Cys Tyr Gly Arg
            580                 585                 590
Gly Ile Gly Glu Trp His Cys Gln Pro Leu Gln Thr Tyr Pro Ser Ser
        595                 600                 605
Ser Gly Pro Val Glu Val Phe Ile Thr Glu Thr Pro Ser Gln Pro Asn
    610                 615                 620
Ser His Pro Ile Gln Trp Asn Ala Pro Gln Pro Ser His Ile Ser Lys
625                 630                 635                 640
Tyr Ile Leu Arg Trp Arg Pro Lys Asn Ser Val Gly Arg Trp Lys Glu
                645                 650                 655
Ala Thr Ile Pro Gly His Leu Asn Ser Tyr Thr Ile Lys Gly Leu Lys

            660                 665                 670
Pro Gly Val Val Tyr Glu Gly Gln Leu Ile Ser Ile Gln Gln Tyr Gly
        675                 680                 685
His Gln Glu Val Thr Arg Phe Asp Phe Thr Thr Thr Ser Thr Ser Thr
    690                 695                 700
Pro Val Thr Ser Asn Thr Val Thr Gly Glu Thr Thr Pro Phe Ser Pro
705                 710                 715                 720
Leu Val Ala Thr Ser Glu Ser Val Thr Glu Ile Thr Ala Ser Ser Phe
                725                 730                 735
Val Val Ser Trp Val Ser Ala Ser Asp Thr Val Ser Gly Phe Arg Val
            740                 745                 750
Glu Tyr Glu Leu Ser Glu Glu Gly Asp Glu Pro Gln Tyr Leu Asp Leu
        755                 760                 765
Pro Ser Thr Ala Thr Ser Val Asn Ile Pro Asp Leu Leu Pro Gly Arg
    770                 775                 780
Lys Tyr Ile Val Asn Val Tyr Gln Ile Ser Glu Asp Gly Glu Gln Ser
785                 790                 795                 800
Leu Ile Leu Ser Thr Ser Gln Thr Thr Ala Pro Asp Ala Pro Pro Asp
                805                 810                 815
Pro Thr Val Asp Gln Val Asp Asp Thr Ser Ile Val Val Arg Trp Ser
            820                 825                 830
Arg Pro Gln Ala Pro Ile Thr Gly Tyr Arg Ile Val Tyr Ser Pro Ser
        835                 840                 845
Val Glu Gly Ser Ser Thr Glu Leu Asn Leu Pro Glu Thr Ala Asn Ser
    850                 855                 860
Val Thr Leu Ser Asp Leu Gln Pro Gly Val Gln Tyr Asn Ile Thr Ile
865                 870                 875                 880
Tyr Ala Val Glu Glu Asn Gln Glu Ser Thr Pro Val Val Ile Gln Gln
                885                 890                 895

Glu Thr Thr Gly Thr Pro Arg Ser Asp Thr Val Pro Ser Pro Arg Asp
            900                 905                 910
Leu Gln Phe Val Glu Val Thr Asp Val Lys Val Thr Ile Met Trp Thr
        915                 920                 925
Pro Pro Glu Ser Ala Val Thr Gly Tyr Arg Val Asp Val Ile Pro Val
    930                 935                 940
Asn Leu Pro Gly Glu His Gly Gln Arg Leu Pro Ile Ser Arg Asn Thr
945                 950                 955                 960
Phe Ala Glu Val Thr Gly Leu Ser Pro Gly Val Thr Tyr Tyr Phe Lys
                965                 970                 975
Val Phe Ala Val Ser His Gly Arg Glu Ser Lys Pro Leu Thr Ala Gln
            980                 985                 990
Gln Thr Thr Lys Leu Asp Ala Pro Thr Asn Leu Gln Phe Val Asn Glu
        995                 1000                1005
Thr Asp Ser Thr Val Leu Val Arg Trp Thr Pro Pro Arg Ala Gln Ile
    1010                1015                1020
Thr Gly Tyr Arg Leu Thr Val Gly Leu Thr Arg Arg Gly Gln Pro Arg
1025                1030                1035                1040
Gln Tyr Asn Val Gly Pro Ser Val Ser Lys Tyr Pro Leu Arg Asn Leu
                1045                1050                1055
Gln Pro Ala Ser Glu Tyr Thr Val Ser Leu Val Ala Ile Lys Gly Asn
            1060                1065                1070
Gln Glu Ser Pro Lys Ala Thr Gly Val Phe Thr Thr Leu Gln Pro Gly
        1075                1080                1085
Ser Ser Ile Pro Pro Tyr Asn Thr Glu Val Thr Glu Thr Thr Ile Val
    1090                1095                1100
Ile Thr Trp Thr Pro Ala Pro Arg Ile Gly Phe Lys Leu Gly Val Arg
1105                1110                1115                1120
Pro Ser Gln Gly Gly Glu Ala Pro Arg Glu Val Thr Ser Asp Ser Gly

1125                1130                1135

Ser Ile Val Val Ser Gly Leu Thr Pro Gly Val Glu Tyr Val Tyr Thr

        1140                1145                1150

Ile Gln Val Leu Arg Asp Gly Gln Glu Arg Asp Ala Pro Ile Val Asn

    1155                1160                1165

Lys Val Val Thr Pro Leu Ser Pro Pro Thr Asn Leu His Leu Glu Ala

1170                1175                1180

Asn Pro Asp Thr Gly Val Leu Thr Val Ser Trp Glu Arg Ser Thr Thr

1185                1190                1195                1200

Pro Asp Ile Thr Gly Tyr Arg Ile Thr Thr Thr Pro Thr Asn Gly Gln

                1205                1210                1215

Gln Gly Asn Ser Leu Glu Glu Val Val His Ala Asp Gln Ser Ser Cys

        1220                1225                1230

Thr Phe Asp Asn Leu Ser Pro Gly Leu Glu Tyr Asn Val Ser Val Tyr

    1235                1240                1245

Thr Val Lys Asp Asp Lys Glu Ser Val Pro Ile Ser Asp Thr Ile Ile

1250                1255                1260

Pro Ala Val Pro Pro Pro Thr Asp Leu Arg Phe Thr Asn Ile Gly Pro

1265                1270                1275                1280

Asp Thr Met Arg Val Thr Trp Ala Pro Pro Pro Ser Ile Asp Leu Thr

                1285                1290                1295

Asn Phe Leu Val Arg Tyr Ser Pro Val Lys Asn Glu Glu Asp Val Ala

        1300                1305                1310

Glu Leu Ser Ile Ser Pro Ser Asp Asn Ala Val Val Leu Thr Asn Leu

    1315                1320                1325

Leu Pro Gly Thr Glu Tyr Val Val Ser Val Ser Ser Val Tyr Glu Gln

1330                1335                1340

His Glu Ser Thr Pro Leu Arg Gly Arg Gln Lys Thr Gly Leu Asp Ser

1345                1350                1355                1360

Pro Thr Gly Ile Asp Phe Ser Asp Ile Thr Ala Asn Ser Phe Thr Val
                1365                1370                1375
His Trp Ile Ala Pro Arg Ala Thr Ile Thr Gly Tyr Arg Ile Arg His
            1380                1385                1390
His Pro Glu His Phe Ser Gly Arg Pro Arg Glu Asp Arg Val Pro His
        1395                1400                1405
Ser Arg Asn Ser Ile Thr Leu Thr Asn Leu Thr Pro Gly Thr Glu Tyr
    1410                1415                1420
Val Val Ser Ile Val Ala Leu Asn Gly Arg Glu Glu Ser Pro Leu Leu
1425                1430                1435                1440
Ile Gly Gln Gln Ser Thr Val Ser Asp Val Pro Arg Asp Leu Glu Val
                1445                1450                1455
Val Ala Ala Thr Pro Thr Ser Leu Leu Ile Ser Trp Asp Ala Pro Ala
            1460                1465                1470
Val Thr Val Arg Tyr Tyr Arg Ile Thr Tyr Gly Glu Thr Gly Gly Asn
        1475                1480                1485
Ser Pro Val Gln Glu Phe Thr Val Pro Gly Ser Lys Ser Thr Ala Thr
    1490                1495                1500
Ile Ser Gly Leu Lys Pro Gly Val Asp Tyr Thr Ile Thr Val Tyr Ala
1505                1510                1515                1520
Val Thr Gly Arg Gly Asp Ser Pro Ala Ser Ser Lys Pro Ile Ser Ile
                1525                1530                1535
Asn Tyr Arg Thr Glu Ile Asp Lys Pro Ser Gln Met Gln Val Thr Asp
            1540                1545                1550
Val Gln Asp Asn Ser Ile Ser Val Lys Trp Leu Pro Ser Ser Ser Pro
        1555                1560                1565
Val Thr Gly Tyr Arg Val Thr Thr Thr Pro Lys Asn Gly Pro Gly Pro
    1570                1575                1580
Thr Lys Thr Lys Thr Ala Gly Pro Asp Gln Thr Glu Met Thr Ile Glu

1585                1590                1595                1600

Gly Leu Gln Pro Thr Val Glu Tyr Val Val Ser Val Tyr Ala Gln Asn

                1605                1610                1615

Pro Ser Gly Glu Ser Gln Pro Leu Val Gln Thr Ala Val Thr Asn Ile

            1620                1625                1630

Asp Arg Pro Lys Gly Leu Ala Phe Thr Asp Val Asp Val Asp Ser Ile

        1635                1640                1645

Lys Ile Ala Trp Glu Ser Pro Gln Gly Gln Val Ser Arg Tyr Arg Val

    1650                1655                1660

Thr Tyr Ser Ser Pro Glu Asp Gly Ile His Glu Leu Phe Pro Ala Pro

1665                1670                1675                1680

Asp Gly Glu Glu Asp Thr Ala Glu Leu Gln Gly Leu Arg Pro Gly Ser

                1685                1690                1695

Glu Tyr Thr Val Ser Val Val Ala Leu His Asp Asp Met Glu Ser Gln

            1700                1705                1710

Pro Leu Ile Gly Thr Gln Ser Thr Ala Ile Pro Ala Pro Thr Asp Leu

        1715                1720                1725

Lys Phe Thr Gln Val Thr Pro Thr Ser Leu Ser Ala Gln Trp Thr Pro

    1730                1735                1740

Pro Asn Val Gln Leu Thr Gly Tyr Arg Val Arg Val Thr Pro Lys Glu

1745                1750                1755                1760

Lys Thr Gly Pro Met Lys Glu Ile Asn Leu Ala Pro Asp Ser Ser Ser

                1765                1770                1775

Val Val Val Ser Gly Leu Met Val Ala Thr Lys Tyr Glu Val Ser Val

            1780                1785                1790

Tyr Ala Leu Lys Asp Thr Leu Thr Ser Arg Pro Ala Gln Gly Val Val

        1795                1800                1805

Thr Thr Leu Glu Asn Val Ser Pro Pro Arg Arg Ala Arg Val Thr Asp

    1810                1815                1820

Ala Thr Glu Thr Thr Ile Thr Ile Ser Trp Arg Thr Lys Thr Glu Thr
1825                1830                1835                1840
Ile Thr Gly Phe Gln Val Asp Ala Val Pro Ala Asn Gly Gln Thr Pro
                1845                1850                1855
Ile Gln Arg Thr Ile Lys Pro Asp Val Arg Ser Tyr Thr Ile Thr Gly
            1860                1865                1870
Leu Gln Pro Gly Thr Asp Tyr Lys Ile Tyr Leu Tyr Thr Leu Asn Asp
        1875                1880                1885
Asn Ala Arg Ser Ser Pro Val Val Ile Asp Ala Ser Thr Ala Ile Asp
    1890                1895                1900
Ala Pro Ser Asn Leu Arg Phe Leu Ala Thr Thr Pro Asn Ser Leu Leu
1905                1910                1915                1920
Val Ser Trp Gln Pro Pro Arg Ala Arg Ile Thr Gly Tyr Ile Ile Lys
                1925                1930                1935
Tyr Glu Lys Pro Gly Ser Pro Pro Arg Glu Val Val Pro Arg Pro Arg
            1940                1945                1950
Pro Gly Val Thr Glu Ala Thr Ile Thr Gly Leu Glu Pro Gly Thr Glu
        1955                1960                1965
Tyr Thr Ile Tyr Val Ile Ala Leu Lys Asn Asn Gln Lys Ser Glu Pro
    1970                1975                1980
Leu Ile Gly Arg Lys Lys Thr Asp Glu Leu Pro Gln Leu Val Thr Leu
1985                1990                1995                2000
Pro His Pro Asn Leu His Gly Pro Glu Ile Leu Asp Val Pro Ser Thr
                2005                2010                2015
Val Gln Lys Thr Pro Phe Val Thr His Pro Gly Tyr Asp Thr Gly Asn
            2020                2025                2030
Gly Ile Gln Leu Pro Gly Thr Ser Gly Gln Gln Pro Ser Val Gly Gln
        2035                2040                2045
Gln Met Ile Phe Glu Glu His Gly Phe Arg Arg Thr Thr Pro Pro Thr

2050                2055                2060

Thr Ala Thr Pro Ile Arg His Arg Pro Arg Pro Tyr Pro Pro Asn Val

2065                2070                2075                2080

Gly Gln Glu Ala Leu Ser Gln Thr Thr Ile Ser Trp Ala Pro Phe Gln

                2085                2090                2095

Asp Thr Ser Glu Tyr Ile Ile Ser Cys His Pro Val Gly Thr Asp Glu

            2100                2105                2110

Glu Pro Leu Gln Phe Arg Val Pro Gly Thr Ser Thr Ser Ala Thr Leu

        2115                2120                2125

Thr Gly Leu Thr Arg Gly Ala Thr Tyr Asn Ile Ile Val Glu Ala Leu

    2130                2135                2140

Lys Asp Gln Gln Arg His Lys Val Arg Glu Glu Val Val Thr Val Gly

2145                2150                2155                2160

Asn Ser Val Asn Glu Gly Leu Asn Gln Pro Thr Asp Asp Ser Cys Phe

                2165                2170                2175

Asp Pro Tyr Thr Val Ser His Tyr Ala Val Gly Asp Glu Trp Glu Arg

            2180                2185                2190

Met Ser Glu Ser Gly Phe Lys Leu Leu Cys Gln Cys Leu Gly Phe Gly

        2195                2200                2205

Ser Gly His Phe Arg Cys Asp Ser Ser Arg Trp Cys His Asp Asn Gly

    2210                2215                2220

Val Asn Tyr Lys Ile Gly Glu Lys Trp Asp Arg Gln Gly Glu Asn Gly

2225                2230                2235                2240

Gln Met Met Ser Cys Thr Cys Leu Gly Asn Gly Lys Gly Glu Phe Lys

                2245                2250                2255

Cys Asp Pro His Glu Ala Thr Cys Tyr Asp Asp Gly Lys Thr Tyr His

            2260                2265                2270

Val Gly Glu Gln Trp Gln Lys Glu Tyr Leu Gly Ala Ile Cys Ser Cys

        2275                2280                2285

Thr Cys Phe Gly Gly Gln Arg Gly Trp Arg Cys Asp Asn Cys Arg Arg
    2290                2295                2300
Pro Gly Gly Glu Pro Ser Pro Glu Gly Thr Thr Gly Gln Ser Tyr Asn
2305                2310                2315                2320
Gln Tyr Ser Gln Arg Tyr His Gln Arg Thr Asn Thr Asn Val Asn Cys
                2325                2330                2335
Pro Ile Glu Cys Phe Met Pro Leu Asp Val Gln Ala Asp Arg Glu Asp
            2340                2345                2350
Ser Arg Glu
        2355

<210> 95
<211> 1366
<212> PRT
<213> Homo sapiens

<400> 95
Met Leu Ser Phe Val Asp Thr Arg Thr Leu Leu Leu Leu Ala Val Thr
1               5                   10                  15
Leu Cys Leu Ala Thr Cys Gln Ser Leu Gln Glu Glu Thr Val Arg Lys
            20                  25                  30
Gly Pro Ala Gly Asp Arg Gly Pro Arg Gly Glu Arg Gly Pro Pro Gly
        35                  40                  45
Pro Pro Gly Arg Asp Gly Glu Asp Gly Pro Thr Gly Pro Pro Gly Pro
    50                  55                  60
Pro Gly Pro Pro Gly Pro Pro Gly Leu Gly Gly Asn Phe Ala Ala Gln
65                  70                  75                  80
Tyr Asp Gly Lys Gly Val Gly Leu Gly Pro Gly Pro Met Gly Leu Met

85                  90                  95
Gly Pro Arg Gly Pro Pro Gly Ala Ala Gly Ala Pro Gly Pro Gln Gly
            100                 105                 110
Phe Gln Gly Pro Ala Gly Glu Pro Gly Glu Pro Gly Gln Thr Gly Pro
        115                 120                 125
Ala Gly Ala Arg Gly Pro Ala Gly Pro Pro Gly Lys Ala Gly Glu Asp
    130                 135                 140
Gly His Pro Gly Lys Pro Gly Arg Pro Gly Glu Arg Gly Val Val Gly
145                 150                 155                 160
Pro Gln Gly Ala Arg Gly Phe Pro Gly Thr Pro Gly Leu Pro Gly Phe
                165                 170                 175
Lys Gly Ile Arg Gly His Asn Gly Leu Asp Gly Leu Lys Gly Gln Pro
            180                 185                 190
Gly Ala Pro Gly Val Lys Gly Glu Pro Gly Ala Pro Gly Glu Asn Gly
        195                 200                 205
Thr Pro Gly Gln Thr Gly Ala Arg Gly Leu Pro Gly Glu Arg Gly Arg
    210                 215                 220
Val Gly Ala Pro Gly Pro Ala Gly Ala Arg Gly Ser Asp Gly Ser Val
225                 230                 235                 240
Gly Pro Val Gly Pro Ala Gly Pro Ile Gly Ser Ala Gly Pro Pro Gly
                245                 250                 255
Phe Pro Gly Ala Pro Gly Pro Lys Gly Glu Ile Gly Ala Val Gly Asn
            260                 265                 270
Ala Gly Pro Ala Gly Pro Ala Gly Pro Arg Gly Glu Val Gly Leu Pro
        275                 280                 285
Gly Leu Ser Gly Pro Val Gly Pro Pro Gly Asn Pro Gly Ala Asn Gly
    290                 295                 300
Leu Thr Gly Ala Lys Gly Ala Ala Gly Leu Pro Gly Val Ala Gly Ala
305                 310                 315                 320

Pro Gly Leu Pro Gly Pro Arg Gly Ile Pro Gly Pro Val Gly Ala Ala
                325                 330                 335
Gly Ala Thr Gly Ala Arg Gly Leu Val Gly Glu Pro Gly Pro Ala Gly
            340                 345                 350
Ser Lys Gly Glu Ser Gly Asn Lys Gly Glu Pro Gly Ser Ala Gly Pro
        355                 360                 365
Gln Gly Pro Pro Gly Pro Ser Gly Glu Glu Gly Lys Arg Gly Pro Asn
    370                 375                 380
Gly Glu Ala Gly Ser Ala Gly Pro Pro Gly Pro Pro Gly Leu Arg Gly
385                 390                 395                 400
Ser Pro Gly Ser Arg Gly Leu Pro Gly Ala Asp Gly Arg Ala Gly Val
                405                 410                 415
Met Gly Pro Pro Gly Ser Arg Gly Ala Ser Gly Pro Ala Gly Val Arg
            420                 425                 430
Gly Pro Asn Gly Asp Ala Gly Arg Pro Gly Glu Pro Gly Leu Met Gly
        435                 440                 445
Pro Arg Gly Leu Pro Gly Ser Pro Gly Asn Ile Gly Pro Ala Gly Lys
    450                 455                 460
Glu Gly Pro Val Gly Leu Pro Gly Ile Asp Gly Arg Pro Gly Pro Ile
465                 470                 475                 480
Gly Pro Ala Gly Ala Arg Gly Glu Pro Gly Asn Ile Gly Phe Pro Gly
                485                 490                 495
Pro Lys Gly Pro Thr Gly Asp Pro Gly Lys Asn Gly Asp Lys Gly His
            500                 505                 510
Ala Gly Leu Ala Gly Ala Arg Gly Ala Pro Gly Pro Asp Gly Asn Asn
        515                 520                 525
Gly Ala Gln Gly Pro Pro Gly Pro Gln Gly Val Gln Gly Gly Lys Gly
    530                 535                 540
Glu Gln Gly Pro Ala Gly Pro Pro Gly Phe Gln Gly Leu Pro Gly Pro

545                 550                 555                 560

Ser Gly Pro Ala Gly Glu Val Gly Lys Pro Gly Glu Arg Gly Leu His

                565                 570                 575

Gly Glu Phe Gly Leu Pro Gly Pro Ala Gly Pro Arg Gly Glu Arg Gly

            580                 585                 590

Pro Pro Gly Glu Ser Gly Ala Ala Gly Pro Thr Gly Pro Ile Gly Ser

        595                 600                 605

Arg Gly Pro Ser Gly Pro Pro Gly Pro Asp Gly Asn Lys Gly Glu Pro

    610                 615                 620

Gly Val Val Gly Ala Val Gly Thr Ala Gly Pro Ser Gly Pro Ser Gly

625                 630                 635                 640

Leu Pro Gly Glu Arg Gly Ala Ala Gly Ile Pro Gly Gly Lys Gly Glu

                645                 650                 655

Lys Gly Glu Pro Gly Leu Arg Gly Glu Ile Gly Asn Pro Gly Arg Asp

            660                 665                 670

Gly Ala Arg Gly Ala His Gly Ala Val Gly Ala Pro Gly Pro Ala Gly

        675                 680                 685

Ala Thr Gly Asp Arg Gly Glu Ala Gly Ala Ala Gly Pro Ala Gly Pro

    690                 695                 700

Ala Gly Pro Arg Gly Ser Pro Gly Glu Arg Gly Glu Val Gly Pro Ala

705                 710                 715                 720

Gly Pro Asn Gly Phe Ala Gly Pro Ala Gly Ala Ala Gly Gln Pro Gly

                725                 730                 735

Ala Lys Gly Glu Arg Gly Ala Lys Gly Pro Lys Gly Glu Asn Gly Val

            740                 745                 750

Val Gly Pro Thr Gly Pro Val Gly Ala Ala Gly Pro Ala Gly Pro Asn

        755                 760                 765

Gly Pro Pro Gly Pro Ala Gly Ser Arg Gly Asp Gly Gly Pro Pro Gly

    770                 775                 780

Met Thr Gly Phe Pro Gly Ala Ala Gly Arg Thr Gly Pro Pro Gly Pro
785                 790                 795                 800
Ser Gly Ile Ser Gly Pro Pro Gly Pro Pro Gly Pro Ala Gly Lys Glu
                805                 810                 815
Gly Leu Arg Gly Pro Arg Gly Asp Gln Gly Pro Val Gly Arg Thr Gly
            820                 825                 830
Glu Val Gly Ala Val Gly Pro Pro Gly Phe Ala Gly Glu Lys Gly Pro
        835                 840                 845
Ser Gly Glu Ala Gly Thr Ala Gly Pro Pro Gly Thr Pro Gly Pro Gln
    850                 855                 860
Gly Leu Leu Gly Ala Pro Gly Ile Leu Gly Leu Pro Gly Ser Arg Gly
865                 870                 875                 880
Glu Arg Gly Leu Pro Gly Val Ala Gly Ala Val Gly Glu Pro Gly Pro
                885                 890                 895
Leu Gly Ile Ala Gly Pro Pro Gly Ala Arg Gly Pro Pro Gly Ala Val
            900                 905                 910
Gly Ser Pro Gly Val Asn Gly Ala Pro Gly Glu Ala Gly Arg Asp Gly
        915                 920                 925
Asn Pro Gly Asn Asp Gly Pro Pro Gly Arg Asp Gly Gln Pro Gly His
    930                 935                 940
Lys Gly Glu Arg Gly Tyr Pro Gly Asn Ile Gly Pro Val Gly Ala Ala
945                 950                 955                 960
Gly Ala Pro Gly Pro His Gly Pro Val Gly Pro Ala Gly Lys His Gly
                965                 970                 975
Asn Arg Gly Glu Thr Gly Pro Ser Gly Pro Val Gly Pro Ala Gly Ala
            980                 985                 990
Val Gly Pro Arg Gly Pro Ser Gly Pro Gln Gly Ile Arg Gly Asp Lys
        995                 1000                1005
Gly Glu Pro Gly Glu Lys Gly Pro Arg Gly Leu Pro Gly Leu Lys Gly

1010                1015                1020

His Asn Gly Leu Gln Gly Leu Pro Gly Ile Ala Gly His His Gly Asp

1025                1030                1035                1040

Gln Gly Ala Pro Gly Ser Val Gly Pro Ala Gly Pro Arg Gly Pro Ala

                1045                1050                1055

Gly Pro Ser Gly Pro Ala Gly Lys Asp Gly Arg Thr Gly His Pro Gly

            1060                1065                1070

Thr Val Gly Pro Ala Gly Ile Arg Gly Pro Gln Gly His Gln Gly Pro

        1075                1080                1085

Ala Gly Pro Pro Gly Pro Pro Gly Pro Pro Gly Pro Pro Gly Val Ser

    1090                1095                1100

Gly Gly Gly Tyr Asp Phe Gly Tyr Asp Gly Asp Phe Tyr Arg Ala Asp

1105                1110                1115                1120

Gln Pro Arg Ser Ala Pro Ser Leu Arg Pro Lys Asp Tyr Glu Val Asp

                1125                1130                1135

Ala Thr Leu Lys Ser Leu Asn Asn Gln Ile Glu Thr Leu Leu Thr Pro

            1140                1145                1150

Glu Gly Ser Arg Lys Asn Pro Ala Arg Thr Cys Arg Asp Leu Arg Leu

        1155                1160                1165

Ser His Pro Glu Trp Ser Ser Gly Tyr Tyr Trp Ile Asp Pro Asn Gln

    1170                1175                1180

Gly Cys Thr Met Asp Ala Ile Lys Val Tyr Cys Asp Phe Ser Thr Gly

1185                1190                1195                1200

Glu Thr Cys Ile Arg Ala Gln Pro Glu Asn Ile Pro Ala Lys Asn Trp

                1205                1210                1215

Tyr Arg Ser Ser Lys Asp Lys Lys His Val Trp Leu Gly Glu Thr Ile

            1220                1225                1230

Asn Ala Gly Ser Gln Phe Glu Tyr Asn Val Glu Gly Val Thr Ser Lys

        1235                1240                1245

Glu Met Ala Thr Gln Leu Ala Phe Met Arg Leu Leu Ala Asn Tyr Ala
    1250                1255                1260
Ser Gln Asn Ile Thr Tyr His Cys Lys Asn Ser Ile Ala Tyr Met Asp
1265                1270                1275                1280
Glu Glu Thr Gly Asn Leu Lys Lys Ala Val Ile Leu Gln Gly Ser Asn
                1285                1290                1295
Asp Val Glu Leu Val Ala Glu Gly Asn Ser Arg Phe Thr Tyr Thr Val
            1300                1305                1310
Leu Val Asp Gly Cys Ser Lys Lys Thr Asn Glu Trp Gly Lys Thr Ile
        1315                1320                1325
Ile Glu Tyr Lys Thr Asn Lys Pro Ser Arg Leu Pro Phe Leu Asp Ile
    1330                1335                1340
Ala Pro Leu Asp Ile Gly Gly Ala Asp His Glu Phe Phe Val Asp Ile
1345                1350                1355                1360
Gly Pro Val Cys Phe Lys
                1365

<210> 96
<211> 105
<212> PRT
<213> Homo sapiens

<400> 96
Met Ala Lys Ile Ser Ser Pro Thr Glu Thr Glu Arg Cys Ile Glu Ser
 1               5                  10                  15
Leu Ile Ala Val Phe Gln Lys Tyr Ala Gly Lys Asp Gly Tyr Asn Tyr
            20                  25                  30
Thr Leu Ser Lys Thr Glu Phe Leu Ser Phe Met Asn Thr Glu Leu Ala

        35                  40                  45

Ala Phe Thr Lys Asn Gln Lys Asp Pro Gly Val Leu Asp Arg Met Met

    50                  55                  60

Lys Lys Leu Asp Thr Asn Ser Asp Gly Gln Leu Asp Phe Ser Glu Phe

65                  70                  75                  80

Leu Asn Leu Ile Gly Gly Leu Ala Met Ala Cys His Asp Ser Phe Leu

                85                  90                  95

Lys Ala Val Pro Ser Gln Lys Arg Thr

            100                 105

<210> 97

<211> 283

<212> PRT

<213> Homo sapiens

<400> 97

Met Val Asn Tyr Ala Trp Ala Gly Arg Ser Gln Arg Lys Leu Trp Trp

1               5                   10                  15

Arg Ser Val Ala Val Leu Thr Cys Lys Ser Val Val Arg Pro Gly Tyr

            20                  25                  30

Arg Gly Gly Leu Gln Ala Arg Arg Ser Thr Leu Leu Lys Thr Cys Ala

        35                  40                  45

Arg Ala Arg Ala Thr Ala Pro Gly Ala Met Lys Met Val Ala Pro Trp

    50                  55                  60

Thr Arg Phe Tyr Ser Asn Ser Cys Cys Leu Cys Cys His Val Arg Thr

65                  70                  75                  80

Gly Thr Ile Leu Leu Gly Val Trp Tyr Leu Ile Ile Asn Ala Val Val

                85                  90                  95

Leu Leu Ile Leu Leu Ser Ala Leu Ala Asp Pro Asp Gln Tyr Asn Phe
            100                 105                 110
Ser Ser Ser Glu Leu Gly Gly Asp Phe Glu Phe Met Asp Asp Ala Asn
        115                 120                 125
Met Cys Ile Ala Ile Ala Ile Ser Leu Leu Met Ile Leu Ile Cys Ala
    130                 135                 140
Met Ala Thr Tyr Gly Ala Tyr Lys Gln Arg Ala Ala Trp Ile Ile Pro
145                 150                 155                 160
Phe Phe Cys Tyr Gln Ile Phe Asp Phe Ala Leu Asn Met Leu Val Ala
                165                 170                 175
Ile Thr Val Leu Ile Tyr Pro Asn Ser Ile Gln Glu Tyr Ile Arg Gln
            180                 185                 190
Leu Pro Pro Asn Phe Pro Tyr Arg Asp Asp Val Met Ser Val Asn Pro
        195                 200                 205
Thr Cys Leu Val Leu Ile Ile Leu Leu Phe Ile Ser Ile Ile Leu Thr
    210                 215                 220
Phe Lys Gly Tyr Leu Ile Ser Cys Val Trp Asn Cys Tyr Arg Tyr Ile
225                 230                 235                 240
Asn Gly Arg Asn Ser Ser Asp Val Leu Val Tyr Val Thr Ser Asn Asp
                245                 250                 255
Thr Thr Val Leu Leu Pro Pro Tyr Asp Asp Ala Thr Val Asn Gly Ala
            260                 265                 270
Ala Lys Glu Pro Pro Pro Pro Tyr Val Ser Ala
        275                 280

<210> 98
<211> 712
<212> PRT

<213> Homo sapiens

<400> 98

Met Ala Gly Gly Pro Gly Pro Gly Glu Pro Ala Ala Pro Gly Ala Gln
1               5                   10                  15

His Phe Leu Tyr Glu Val Pro Pro Trp Val Met Cys Arg Phe Tyr Lys
            20                  25                  30

Val Met Asp Ala Leu Glu Pro Ala Asp Trp Cys Gln Phe Ala Ala Leu
        35                  40                  45

Ile Val Arg Asp Gln Thr Glu Leu Arg Leu Cys Glu Arg Ser Gly Gln
    50                  55                  60

Arg Thr Ala Ser Val Leu Trp Pro Trp Ile Asn Arg Asn Ala Arg Val
65                  70                  75                  80

Ala Asp Leu Val His Ile Leu Thr His Leu Gln Leu Leu Arg Ala Arg
                85                  90                  95

Asp Ile Ile Thr Ala Trp His Pro Pro Ala Pro Leu Pro Ser Pro Gly
            100                 105                 110

Thr Thr Ala Pro Arg Pro Ser Ser Ile Pro Ala Pro Ala Glu Ala Glu
        115                 120                 125

Ala Trp Ser Pro Arg Lys Leu Pro Ser Ser Ala Ser Thr Phe Leu Ser
    130                 135                 140

Pro Ala Phe Pro Gly Ser Gln Thr His Ser Gly Pro Glu Leu Gly Leu
145                 150                 155                 160

Val Pro Ser Pro Ala Ser Leu Trp Pro Pro Pro Pro Ser Pro Ala Pro
                165                 170                 175

Ser Ser Thr Lys Pro Gly Pro Glu Ser Ser Val Ser Leu Leu Gln Gly
            180                 185                 190

Ala Arg Pro Ser Pro Phe Cys Trp Pro Leu Cys Glu Ile Ser Arg Gly
        195                 200                 205

Thr His Asn Phe Ser Glu Glu Leu Lys Ile Gly Glu Gly Gly Phe Gly
    210                 215                 220
Cys Val Tyr Arg Ala Val Met Arg Asn Thr Val Tyr Ala Val Lys Arg
225                 230                 235                 240
Leu Lys Glu Asn Ala Asp Leu Glu Trp Thr Ala Val Lys Gln Ser Phe
                245                 250                 255
Leu Thr Glu Val Glu Gln Leu Ser Arg Phe Arg His Pro Asn Ile Val
            260                 265                 270
Asp Phe Ala Gly Tyr Cys Ala Gln Asn Gly Phe Tyr Cys Leu Val Tyr
        275                 280                 285
Gly Phe Leu Pro Asn Gly Ser Leu Glu Asp Arg Leu His Cys Gln Thr
    290                 295                 300
Gln Ala Cys Pro Pro Leu Ser Trp Pro Gln Arg Leu Asp Ile Leu Leu
305                 310                 315                 320
Gly Thr Ala Arg Ala Ile Gln Phe Leu His Gln Asp Ser Pro Ser Leu
                325                 330                 335
Ile His Gly Asp Ile Lys Ser Ser Asn Val Leu Leu Asp Glu Arg Leu
            340                 345                 350
Thr Pro Lys Leu Gly Asp Phe Gly Leu Ala Arg Phe Ser Arg Phe Ala
        355                 360                 365
Gly Ser Ser Pro Ser Gln Ser Ser Met Val Ala Arg Thr Gln Thr Val
    370                 375                 380
Arg Gly Thr Leu Ala Tyr Leu Pro Glu Glu Tyr Ile Lys Thr Gly Arg
385                 390                 395                 400
Leu Ala Val Asp Thr Asp Thr Phe Ser Phe Gly Val Val Val Leu Glu
                405                 410                 415
Thr Leu Ala Gly Gln Arg Ala Val Lys Thr His Gly Ala Arg Thr Lys
            420                 425                 430
Tyr Leu Lys Asp Leu Val Glu Glu Glu Ala Glu Glu Ala Gly Val Ala

        435                 440                 445

Leu Arg Ser Thr Gln Ser Thr Leu Gln Ala Gly Leu Ala Ala Asp Ala

    450                 455                 460

Trp Ala Ala Pro Ile Ala Met Gln Ile Tyr Lys Lys His Leu Asp Pro

465                 470                 475                 480

Arg Pro Gly Pro Cys Pro Pro Glu Leu Gly Leu Gly Leu Gly Gln Leu

                485                 490                 495

Ala Cys Cys Cys Leu His Arg Arg Ala Lys Arg Arg Pro Pro Met Thr

            500                 505                 510

Gln Val Tyr Glu Arg Leu Glu Lys Leu Gln Ala Val Val Ala Gly Val

        515                 520                 525

Pro Gly His Leu Glu Ala Ala Ser Cys Ile Pro Pro Ser Pro Gln Glu

    530                 535                 540

Asn Ser Tyr Val Ser Ser Thr Gly Arg Ala His Ser Gly Ala Ala Pro

545                 550                 555                 560

Trp Gln Pro Leu Ala Ala Pro Ser Gly Ala Ser Ala Gln Ala Ala Glu

                565                 570                 575

Gln Leu Gln Arg Gly Pro Asn Gln Pro Val Glu Ser Asp Glu Ser Leu

            580                 585                 590

Gly Gly Leu Ser Ala Ala Leu Arg Ser Trp His Leu Thr Pro Ser Cys

        595                 600                 605

Pro Leu Asp Pro Ala Pro Leu Arg Glu Ala Gly Cys Pro Gln Gly Asp

    610                 615                 620

Thr Ala Gly Glu Ser Ser Trp Gly Ser Gly Pro Gly Ser Arg Pro Thr

625                 630                 635                 640

Ala Val Glu Gly Leu Ala Leu Gly Ser Ser Ala Ser Ser Ser Ser Glu

                645                 650                 655

Pro Pro Gln Ile Ile Ile Asn Pro Ala Arg Gln Lys Met Val Gln Lys

            660                 665                 670

Leu Ala Leu Tyr Glu Asp Gly Ala Leu Asp Ser Leu Gln Leu Leu Ser
        675                 680                 685
Ser Ser Ser Leu Pro Gly Leu Gly Leu Glu Gln Asp Arg Gln Gly Pro
    690                 695                 700
Glu Glu Ser Asp Glu Phe Gln Ser
705                 710

<210> 99
<211> 132
<212> PRT
<213> Homo sapiens

<400> 99
Met Asn His Ile Val Gln Thr Phe Ser Pro Val Asn Ser Gly Gln Pro
1               5                   10                  15
Pro Asn Tyr Glu Met Leu Lys Glu Glu Gln Glu Val Ala Met Leu Gly
            20                  25                  30
Gly Pro His Asn Pro Ala Pro Pro Thr Ser Thr Val Ile His Ile Arg
        35                  40                  45
Ser Glu Thr Ser Val Pro Asp His Val Val Trp Ser Leu Phe Asn Thr
    50                  55                  60
Leu Phe Met Asn Thr Cys Cys Leu Gly Phe Ile Ala Phe Ala Tyr Ser
65                  70                  75                  80
Val Lys Ser Arg Asp Arg Lys Met Val Gly Asp Val Thr Gly Ala Gln
                85                  90                  95
Ala Tyr Ala Ser Thr Ala Lys Cys Leu Asn Ile Trp Ala Leu Ile Leu
            100                 105                 110
Gly Ile Phe Met Thr Ile Leu Leu Val Ile Ile Pro Val Leu Val Val

115                 120                 125

Gln Ala Gln Arg

    130

<210> 100

<211> 207

<212> PRT

<213> Homo sapiens

<400> 100

Met Ala Pro Phe Glu Pro Leu Ala Ser Gly Ile Leu Leu Leu Leu Trp

1               5                   10                  15

Leu Ile Ala Pro Ser Arg Ala Cys Thr Cys Val Pro Pro His Pro Gln

            20                  25                  30

Thr Ala Phe Cys Asn Ser Asp Leu Val Ile Arg Ala Lys Phe Val Gly

        35                  40                  45

Thr Pro Glu Val Asn Gln Thr Thr Leu Tyr Gln Arg Tyr Glu Ile Lys

    50                  55                  60

Met Thr Lys Met Tyr Lys Gly Phe Gln Ala Leu Gly Asp Ala Ala Asp

65                  70                  75                  80

Ile Arg Phe Val Tyr Thr Pro Ala Met Glu Ser Val Cys Gly Tyr Phe

                85                  90                  95

His Arg Ser His Asn Arg Ser Glu Glu Phe Leu Ile Ala Gly Lys Leu

            100                 105                 110

Gln Asp Gly Leu Leu His Ile Thr Thr Cys Ser Phe Val Ala Pro Trp

        115                 120                 125

Asn Ser Leu Ser Leu Ala Gln Arg Arg Gly Phe Thr Lys Thr Tyr Thr

    130                 135                 140

Val Gly Cys Glu Glu Cys Thr Val Phe Pro Cys Leu Ser Ile Pro Cys
145                 150                 155                 160
Lys Leu Gln Ser Gly Thr His Cys Leu Trp Thr Asp Gln Leu Leu Gln
                165                 170                 175
Gly Ser Glu Lys Gly Phe Gln Ser Arg His Leu Ala Cys Leu Pro Arg
            180                 185                 190
Glu Pro Gly Leu Cys Thr Trp Gln Ser Leu Arg Ser Gln Ile Ala
        195                 200                 205

<210> 101
<211> 282
<212> PRT
<213> Homo sapiens

<400> 101
Met Glu Arg Pro Ser Leu Arg Ala Leu Leu Leu Gly Ala Ala Gly Leu
1               5                   10                  15
Leu Leu Leu Leu Leu Pro Leu Ser Ser Ser Ser Ser Ser Asp Thr Cys
            20                  25                  30
Gly Pro Cys Glu Pro Ala Ser Cys Pro Pro Leu Pro Pro Leu Gly Cys
        35                  40                  45
Leu Leu Gly Glu Thr Arg Asp Ala Cys Gly Cys Cys Pro Met Cys Ala
    50                  55                  60
Arg Gly Glu Gly Glu Pro Cys Gly Gly Gly Gly Ala Gly Arg Gly Tyr
65                  70                  75                  80
Cys Ala Pro Gly Met Glu Cys Val Lys Ser Arg Lys Arg Arg Lys Gly
                85                  90                  95
Lys Ala Gly Ala Ala Ala Gly Gly Pro Gly Val Ser Gly Val Cys Val

            100                 105                 110
Cys Lys Ser Arg Tyr Pro Val Cys Gly Ser Asp Gly Thr Thr Tyr Pro
        115                 120                 125
Ser Gly Cys Gln Leu Arg Ala Ala Ser Gln Arg Ala Glu Ser Arg Gly
    130                 135                 140
Glu Lys Ala Ile Thr Gln Val Ser Lys Gly Thr Cys Glu Gln Gly Pro
145                 150                 155                 160
Ser Ile Val Thr Pro Pro Lys Asp Ile Trp Asn Val Thr Gly Ala Gln
                165                 170                 175
Val Tyr Leu Ser Cys Glu Val Ile Gly Ile Pro Thr Pro Val Leu Ile
            180                 185                 190
Trp Asn Lys Val Lys Arg Gly His Tyr Gly Val Gln Arg Thr Glu Leu
        195                 200                 205
Leu Pro Gly Asp Arg Asp Asn Leu Ala Ile Gln Thr Arg Gly Gly Pro
    210                 215                 220
Glu Lys His Glu Val Thr Gly Trp Val Leu Val Ser Pro Leu Ser Lys
225                 230                 235                 240
Glu Asp Ala Gly Glu Tyr Glu Cys His Ala Ser Asn Ser Gln Gly Gln
                245                 250                 255
Ala Ser Ala Ser Ala Lys Ile Thr Val Val Asp Ala Leu His Glu Ile
            260                 265                 270
Pro Val Lys Lys Gly Glu Gly Ala Glu Leu
        275                 280

<210> 102
<211> 125
<212> PRT
<213> Homo sapiens

<400> 102

Met His Lys Glu Glu His Glu Val Ala Val Leu Gly Ala Pro Pro Ser

1               5                   10                  15

Thr Ile Leu Pro Arg Ser Thr Val Ile Asn Ile His Ser Glu Thr Ser

            20                  25                  30

Val Pro Asp His Val Val Trp Ser Leu Phe Asn Thr Leu Phe Leu Asn

        35                  40                  45

Trp Cys Cys Leu Gly Phe Ile Ala Phe Ala Tyr Ser Val Lys Ser Arg

    50                  55                  60

Asp Arg Lys Met Val Gly Asp Val Thr Gly Ala Gln Ala Tyr Ala Ser

65                  70                  75                  80

Thr Ala Lys Cys Leu Asn Ile Trp Ala Leu Ile Leu Gly Ile Leu Met

                85                  90                  95

Thr Ile Gly Phe Ile Leu Leu Leu Val Phe Gly Ser Val Thr Val Tyr

            100                 105                 110

His Ile Met Leu Gln Ile Ile Gln Glu Lys Arg Gly Tyr

        115                 120                 125

<210> 103

<211> 1466

<212> PRT

<213> Homo sapiens

<400> 103

Met Met Ser Phe Val Gln Lys Gly Ser Trp Leu Leu Leu Ala Leu Leu

1               5                   10                  15

His Pro Thr Ile Ile Leu Ala Gln Gln Glu Ala Val Glu Gly Gly Cys

            20                  25                  30
Ser His Leu Gly Gln Ser Tyr Ala Asp Arg Asp Val Trp Lys Pro Glu
        35                  40                  45
Pro Cys Gln Ile Cys Val Cys Asp Ser Gly Ser Val Leu Cys Asp Asp
    50                  55                  60
Ile Ile Cys Asp Asp Gln Glu Leu Asp Cys Pro Asn Pro Glu Ile Pro
65                  70                  75                  80
Phe Gly Glu Cys Cys Ala Val Cys Pro Gln Pro Pro Thr Ala Pro Thr
                85                  90                  95
Arg Pro Pro Asn Gly Gln Gly Pro Gln Gly Pro Lys Gly Asp Pro Gly
            100                 105                 110
Pro Pro Gly Ile Pro Gly Arg Asn Gly Asp Pro Gly Ile Pro Gly Gln
        115                 120                 125
Pro Gly Ser Pro Gly Ser Pro Gly Pro Pro Gly Ile Cys Glu Ser Cys
    130                 135                 140
Pro Thr Gly Pro Gln Asn Tyr Ser Pro Gln Tyr Asp Ser Tyr Asp Val
145                 150                 155                 160
Lys Ser Gly Val Ala Val Gly Gly Leu Ala Gly Tyr Pro Gly Pro Ala
                165                 170                 175
Gly Pro Pro Gly Pro Pro Gly Pro Pro Gly Thr Ser Gly His Pro Gly
            180                 185                 190
Ser Pro Gly Ser Pro Gly Tyr Gln Gly Pro Pro Gly Glu Pro Gly Gln
        195                 200                 205
Ala Gly Pro Ser Gly Pro Pro Gly Pro Pro Gly Ala Ile Gly Pro Ser
    210                 215                 220
Gly Pro Ala Gly Lys Asp Gly Glu Ser Gly Arg Pro Gly Arg Pro Gly
225                 230                 235                 240
Glu Arg Gly Leu Pro Gly Pro Pro Gly Ile Lys Gly Pro Ala Gly Ile
                245                 250                 255

Pro Gly Phe Pro Gly Met Lys Gly His Arg Gly Phe Asp Gly Arg Asn
            260                 265                 270
Gly Glu Lys Gly Glu Thr Gly Ala Pro Gly Leu Lys Gly Glu Asn Gly
        275                 280                 285
Leu Pro Gly Glu Asn Gly Ala Pro Gly Pro Met Gly Pro Arg Gly Ala
    290                 295                 300
Pro Gly Glu Arg Gly Arg Pro Gly Leu Pro Gly Ala Ala Gly Ala Arg
305                 310                 315                 320
Gly Asn Asp Gly Ala Arg Gly Ser Asp Gly Gln Pro Gly Pro Pro Gly
                325                 330                 335
Pro Pro Gly Thr Ala Gly Phe Pro Gly Ser Pro Gly Ala Lys Gly Glu
            340                 345                 350
Val Gly Pro Ala Gly Ser Pro Gly Ser Asn Gly Ala Pro Gly Gln Arg
        355                 360                 365
Gly Glu Pro Gly Pro Gln Gly His Ala Gly Ala Gln Gly Pro Pro Gly
    370                 375                 380
Pro Pro Gly Ile Asn Gly Ser Pro Gly Gly Lys Gly Glu Met Gly Pro
385                 390                 395                 400
Ala Gly Ile Pro Gly Ala Pro Gly Leu Met Gly Ala Arg Gly Pro Pro
                405                 410                 415
Gly Pro Ala Gly Ala Asn Gly Ala Pro Gly Leu Arg Gly Gly Ala Gly
            420                 425                 430
Glu Pro Gly Lys Asn Gly Ala Lys Gly Glu Pro Gly Pro Arg Gly Glu
        435                 440                 445
Arg Gly Glu Ala Gly Ile Pro Gly Val Pro Gly Ala Lys Gly Glu Asp
    450                 455                 460
Gly Lys Asp Gly Ser Pro Gly Glu Pro Gly Ala Asn Gly Leu Pro Gly
465                 470                 475                 480
Ala Ala Gly Glu Arg Gly Ala Pro Gly Phe Arg Gly Pro Ala Gly Pro

                485                 490                 495
Asn Gly Ile Pro Gly Glu Lys Gly Pro Ala Gly Glu Arg Gly Ala Pro
            500                 505                 510
Gly Pro Ala Gly Pro Arg Gly Ala Ala Gly Glu Pro Gly Arg Asp Gly
        515                 520                 525
Val Pro Gly Gly Pro Gly Met Arg Gly Met Pro Gly Ser Pro Gly Gly
    530                 535                 540
Pro Gly Ser Asp Gly Lys Pro Gly Pro Pro Gly Ser Gln Gly Glu Ser
545                 550                 555                 560
Gly Arg Pro Gly Pro Pro Gly Pro Ser Gly Pro Arg Gly Gln Pro Gly
                565                 570                 575
Val Met Gly Phe Pro Gly Pro Lys Gly Asn Asp Gly Ala Pro Gly Lys
            580                 585                 590
Asn Gly Glu Arg Gly Gly Pro Gly Gly Pro Gly Pro Gln Gly Pro Pro
        595                 600                 605
Gly Lys Asn Gly Glu Thr Gly Pro Gln Gly Pro Pro Gly Pro Thr Gly
    610                 615                 620
Pro Gly Gly Asp Lys Gly Asp Thr Gly Pro Pro Gly Pro Gln Gly Leu
625                 630                 635                 640
Gln Gly Leu Pro Gly Thr Gly Gly Pro Pro Gly Glu Asn Gly Lys Pro
                645                 650                 655
Gly Glu Pro Gly Pro Lys Gly Asp Ala Gly Ala Pro Gly Ala Pro Gly
            660                 665                 670
Gly Lys Gly Asp Ala Gly Ala Pro Gly Glu Arg Gly Pro Pro Gly Leu
        675                 680                 685
Ala Gly Ala Pro Gly Leu Arg Gly Gly Ala Gly Pro Pro Gly Pro Glu
    690                 695                 700
Gly Gly Lys Gly Ala Ala Gly Pro Pro Gly Pro Pro Gly Ala Ala Gly
705                 710                 715                 720

Thr Pro Gly Leu Gln Gly Met Pro Gly Glu Arg Gly Gly Leu Gly Ser
                725                 730                 735
Pro Gly Pro Lys Gly Asp Lys Gly Glu Pro Gly Gly Pro Gly Ala Asp
            740                 745                 750
Gly Val Pro Gly Lys Asp Gly Pro Arg Gly Pro Thr Gly Pro Ile Gly
        755                 760                 765
Pro Pro Gly Pro Ala Gly Gln Pro Gly Asp Lys Gly Glu Gly Gly Ala
    770                 775                 780
Pro Gly Leu Pro Gly Ile Ala Gly Pro Arg Gly Ser Pro Gly Glu Arg
785                 790                 795                 800
Gly Glu Thr Gly Pro Pro Gly Pro Ala Gly Phe Pro Gly Ala Pro Gly
                805                 810                 815
Gln Asn Gly Glu Pro Gly Gly Lys Gly Glu Arg Gly Ala Pro Gly Glu
            820                 825                 830
Lys Gly Glu Gly Gly Pro Pro Gly Val Ala Gly Pro Pro Gly Gly Ser
        835                 840                 845
Gly Pro Ala Gly Pro Pro Gly Pro Gln Gly Val Lys Gly Glu Arg Gly
    850                 855                 860
Ser Pro Gly Gly Pro Gly Ala Ala Gly Phe Pro Gly Ala Arg Gly Leu
865                 870                 875                 880
Pro Gly Pro Pro Gly Ser Asn Gly Asn Pro Gly Pro Pro Gly Pro Ser
                885                 890                 895
Gly Ser Pro Gly Lys Asp Gly Pro Pro Gly Pro Ala Gly Asn Thr Gly
            900                 905                 910
Ala Pro Gly Ser Pro Gly Val Ser Gly Pro Lys Gly Asp Ala Gly Gln
        915                 920                 925
Pro Gly Glu Lys Gly Ser Pro Gly Ala Gln Gly Pro Pro Gly Ala Pro
    930                 935                 940
Gly Pro Leu Gly Ile Ala Gly Ile Thr Gly Ala Arg Gly Leu Ala Gly

945                 950                 955                 960

Pro Pro Gly Met Pro Gly Pro Arg Gly Ser Pro Gly Pro Gln Gly Val

                965                 970                 975

Lys Gly Glu Ser Gly Lys Pro Gly Ala Asn Gly Leu Ser Gly Glu Arg

            980                 985                 990

Gly Pro Pro Gly Pro Gln Gly Leu Pro Gly Leu Ala Gly Thr Ala Gly

        995                 1000                1005

Glu Pro Gly Arg Asp Gly Asn Pro Gly Ser Asp Gly Leu Pro Gly Arg

    1010                1015                1020

Asp Gly Ser Pro Gly Gly Lys Gly Asp Arg Gly Glu Asn Gly Ser Pro

1025                1030                1035                1040

Gly Ala Pro Gly Ala Pro Gly His Pro Gly Pro Pro Gly Pro Val Gly

                1045                1050                1055

Pro Ala Gly Lys Ser Gly Asp Arg Gly Glu Ser Gly Pro Ala Gly Pro

            1060                1065                1070

Ala Gly Ala Pro Gly Pro Ala Gly Ser Arg Gly Ala Pro Gly Pro Gln

        1075                1080                1085

Gly Pro Arg Gly Asp Lys Gly Glu Thr Gly Glu Arg Gly Ala Ala Gly

    1090                1095                1100

Ile Lys Gly His Arg Gly Phe Pro Gly Asn Pro Gly Ala Pro Gly Ser

1105                1110                1115                1120

Pro Gly Pro Ala Gly Gln Gln Gly Ala Ile Gly Ser Pro Gly Pro Ala

                1125                1130                1135

Gly Pro Arg Gly Pro Val Gly Pro Ser Gly Pro Pro Gly Lys Asp Gly

            1140                1145                1150

Thr Ser Gly His Pro Gly Pro Ile Gly Pro Pro Gly Pro Arg Gly Asn

        1155                1160                1165

Arg Gly Glu Arg Gly Ser Glu Gly Ser Pro Gly His Pro Gly Gln Pro

    1170                1175                1180

Gly Pro Pro Gly Pro Pro Gly Ala Pro Gly Pro Cys Cys Gly Gly Val
1185                1190                1195                1200
Gly Ala Ala Ala Ile Ala Gly Ile Gly Gly Glu Lys Ala Gly Gly Phe
                1205                1210                1215
Ala Pro Tyr Tyr Gly Asp Glu Pro Met Asp Phe Lys Ile Asn Thr Asp
            1220                1225                1230
Glu Ile Met Thr Ser Leu Lys Ser Val Asn Gly Gln Ile Glu Ser Leu
        1235                1240                1245
Ile Ser Pro Asp Gly Ser Arg Lys Asn Pro Ala Arg Asn Cys Arg Asp
    1250                1255                1260
Leu Lys Phe Cys His Pro Glu Leu Lys Ser Gly Glu Tyr Trp Val Asp
1265                1270                1275                1280
Pro Asn Gln Gly Cys Lys Leu Asp Ala Ile Lys Val Phe Cys Asn Met
                1285                1290                1295
Glu Thr Gly Glu Thr Cys Ile Ser Ala Asn Pro Leu Asn Val Pro Arg
            1300                1305                1310
Lys His Trp Trp Thr Asp Ser Ser Ala Glu Lys Lys His Val Trp Phe
        1315                1320                1325
Gly Glu Ser Met Asp Gly Gly Phe Gln Phe Ser Tyr Gly Asn Pro Glu
    1330                1335                1340
Leu Pro Glu Asp Val Leu Asp Val Gln Leu Ala Phe Leu Arg Leu Leu
1345                1350                1355                1360
Ser Ser Arg Ala Ser Gln Asn Ile Thr Tyr His Cys Lys Asn Ser Ile
                1365                1370                1375
Ala Tyr Met Asp Gln Ala Ser Gly Asn Val Lys Lys Ala Leu Lys Leu
            1380                1385                1390
Met Gly Ser Asn Glu Gly Glu Phe Lys Ala Glu Gly Asn Ser Lys Phe
        1395                1400                1405
Thr Tyr Thr Val Leu Glu Asp Gly Cys Thr Lys His Thr Gly Glu Trp

1410                1415                1420

Ser Lys Thr Val Phe Glu Tyr Arg Thr Arg Lys Ala Val Arg Leu Pro

1425                1430                1435                1440

Ile Val Asp Ile Ala Pro Tyr Asp Ile Gly Gly Pro Asp Gln Glu Phe

                1445                1450                1455

Gly Val Asp Val Gly Pro Val Cys Phe Leu

            1460                1465

<210> 104

<211> 272

<212> PRT

<213> Homo sapiens

<400> 104

Met Val Leu Leu Thr Ala Val Leu Leu Leu Leu Ala Ala Tyr Ala Gly

1               5                   10                  15

Pro Ala Gln Ser Leu Gly Ser Phe Val His Cys Glu Pro Cys Asp Glu

            20                  25                  30

Lys Ala Leu Ser Met Cys Pro Pro Ser Pro Leu Gly Cys Glu Leu Val

        35                  40                  45

Lys Glu Pro Gly Cys Gly Cys Cys Met Thr Cys Ala Leu Ala Glu Gly

    50                  55                  60

Gln Ser Cys Gly Val Tyr Thr Glu Arg Cys Ala Gln Gly Leu Arg Cys

65                  70                  75                  80

Leu Pro Arg Gln Asp Glu Glu Lys Pro Leu His Ala Leu Leu His Gly

                85                  90                  95

Arg Gly Val Cys Leu Asn Glu Lys Ser Tyr Arg Glu Gln Val Lys Ile

            100                 105                 110

Glu Arg Asp Ser Arg Glu His Glu Glu Pro Thr Thr Ser Glu Met Ala
        115                 120                 125
Glu Glu Thr Tyr Ser Pro Lys Ile Phe Arg Pro Lys His Thr Arg Ile
    130                 135                 140
Ser Glu Leu Lys Ala Glu Ala Val Lys Lys Asp Arg Arg Lys Lys Leu
145                 150                 155                 160
Thr Gln Ser Lys Phe Val Gly Gly Ala Glu Asn Thr Ala His Pro Arg
                165                 170                 175
Ile Ile Ser Ala Pro Glu Met Arg Gln Glu Ser Glu Gln Gly Pro Cys
            180                 185                 190
Arg Arg His Met Glu Ala Ser Leu Gln Glu Leu Lys Ala Ser Pro Arg
        195                 200                 205
Met Val Pro Arg Ala Val Tyr Leu Pro Asn Cys Asp Arg Lys Gly Phe
    210                 215                 220
Tyr Lys Arg Lys Gln Cys Lys Pro Ser Arg Gly Arg Lys Arg Gly Ile
225                 230                 235                 240
Cys Trp Cys Val Asp Lys Tyr Gly Met Lys Leu Pro Gly Met Glu Tyr
                245                 250                 255
Val Asp Gly Asp Phe Gln Cys His Thr Phe Asp Ser Ser Asn Val Glu
            260                 265                 270

<210> 105
<211> 158
<212> PRT
<213> Homo sapiens

<400> 105
Met Ala Ser Arg Ser Met Arg Leu Leu Leu Leu Leu Ser Cys Leu Ala

1                   5                   10                  15

Lys Thr Gly Val Leu Gly Asp Ile Ile Met Arg Pro Ser Cys Ala Pro

            20                  25                  30

Gly Trp Phe Tyr His Lys Ser Asn Cys Tyr Gly Tyr Phe Arg Lys Leu

        35                  40                  45

Arg Asn Trp Ser Asp Ala Glu Leu Glu Cys Gln Ser Tyr Gly Asn Gly

    50                  55                  60

Ala His Leu Ala Ser Ile Leu Ser Leu Lys Glu Ala Ser Thr Ile Ala

65                  70                  75                  80

Glu Tyr Ile Ser Gly Tyr Gln Arg Ser Gln Pro Ile Trp Ile Gly Leu

                85                  90                  95

His Asp Pro Gln Lys Arg Gln Gln Trp Gln Trp Ile Asp Gly Ala Met

            100                 105                 110

Tyr Leu Tyr Arg Ser Trp Ser Gly Lys Ser Met Gly Gly Asn Lys His

        115                 120                 125

Cys Ala Glu Met Ser Ser Asn Asn Asn Phe Leu Thr Trp Ser Ser Asn

    130                 135                 140

Glu Cys Asn Lys Arg Gln His Phe Leu Cys Lys Tyr Arg Pro

145                 150                 155

<210> 106

<211> 175

<212> PRT

<213> Homo sapiens

<400> 106

Met Glu Lys Ile Pro Val Ser Ala Phe Leu Leu Leu Val Ala Leu Ser

1               5                   10                  15

Tyr Thr Leu Ala Arg Asp Thr Thr Val Lys Pro Gly Ala Lys Lys Asp
            20                  25                  30
Thr Lys Asp Ser Arg Pro Lys Leu Pro Gln Thr Leu Ser Arg Gly Trp
        35                  40                  45
Gly Asp Gln Leu Ile Trp Thr Gln Thr Tyr Glu Glu Ala Leu Tyr Lys
    50                  55                  60
Ser Lys Thr Ser Asn Lys Pro Leu Met Ile Ile His His Leu Asp Glu
65                  70                  75                  80
Cys Pro His Ser Gln Ala Leu Lys Lys Val Phe Ala Glu Asn Lys Glu
                85                  90                  95
Ile Gln Lys Leu Ala Glu Gln Phe Val Leu Leu Asn Leu Val Tyr Glu
            100                 105                 110
Thr Thr Asp Lys His Leu Ser Pro Asp Gly Gln Tyr Val Pro Arg Ile
        115                 120                 125
Met Phe Val Asp Pro Ser Leu Thr Val Arg Ala Asp Ile Thr Gly Arg
    130                 135                 140
Tyr Ser Asn Arg Leu Tyr Ala Tyr Glu Pro Ala Asp Thr Ala Leu Leu
145                 150                 155                 160
Leu Asp Asn Met Lys Lys Ala Leu Lys Leu Leu Lys Thr Glu Leu
                165                 170                 175

<210> 107
<211> 732
<212> PRT
<213> Homo sapiens

<400> 107
Met Pro Glu Glu Thr Gln Thr Gln Asp Gln Pro Met Glu Glu Glu Glu

1                   5                   10                  15

Val Glu Thr Phe Ala Phe Gln Ala Glu Ile Ala Gln Leu Met Ser Leu

            20                  25                  30

Ile Ile Asn Thr Phe Tyr Ser Asn Lys Glu Ile Phe Leu Arg Glu Leu

        35                  40                  45

Ile Ser Asn Ser Ser Asp Ala Leu Asp Lys Ile Arg Tyr Glu Ser Leu

    50                  55                  60

Thr Asp Pro Ser Lys Leu Asp Ser Gly Lys Glu Leu His Ile Asn Leu

65                  70                  75                  80

Ile Pro Asn Lys Gln Asp Arg Thr Leu Thr Ile Val Asp Thr Gly Ile

                85                  90                  95

Gly Met Thr Lys Ala Asp Leu Ile Asn Asn Leu Gly Thr Ile Ala Lys

            100                 105                 110

Ser Gly Thr Lys Ala Phe Met Glu Ala Leu Gln Ala Gly Ala Asp Ile

        115                 120                 125

Ser Met Ile Gly Gln Phe Gly Val Gly Phe Tyr Ser Ala Tyr Leu Val

    130                 135                 140

Ala Glu Lys Val Thr Val Ile Thr Lys His Asn Asp Asp Glu Gln Tyr

145                 150                 155                 160

Ala Trp Glu Ser Ser Ala Gly Gly Ser Phe Thr Val Arg Thr Asp Thr

                165                 170                 175

Gly Glu Pro Met Gly Arg Gly Thr Lys Val Ile Leu His Leu Lys Glu

            180                 185                 190

Asp Gln Thr Glu Tyr Leu Glu Glu Arg Arg Ile Lys Glu Ile Val Lys

        195                 200                 205

Lys His Ser Gln Phe Ile Gly Tyr Pro Ile Thr Leu Phe Val Glu Lys

    210                 215                 220

Glu Arg Asp Lys Glu Val Ser Asp Asp Glu Ala Glu Glu Lys Glu Asp

225                 230                 235                 240

Lys Glu Glu Glu Lys Glu Lys Glu Glu Lys Glu Ser Glu Asp Lys Pro
                245                 250                 255
Glu Ile Glu Asp Val Gly Ser Asp Glu Glu Glu Glu Lys Lys Asp Gly
            260                 265                 270
Asp Lys Lys Lys Lys Lys Lys Ile Lys Glu Lys Tyr Ile Asp Gln Glu
        275                 280                 285
Glu Leu Asn Lys Thr Lys Pro Ile Trp Thr Arg Asn Pro Asp Asp Ile
    290                 295                 300
Thr Asn Glu Glu Tyr Gly Glu Phe Tyr Lys Ser Leu Thr Asn Asp Trp
305                 310                 315                 320
Glu Asp His Leu Ala Val Lys His Phe Ser Val Glu Gly Gln Leu Glu
                325                 330                 335
Phe Arg Ala Leu Leu Phe Val Pro Arg Arg Ala Pro Phe Asp Leu Phe
            340                 345                 350
Glu Asn Arg Lys Lys Lys Asn Asn Ile Lys Leu Tyr Val Arg Arg Val
        355                 360                 365
Phe Ile Met Asp Asn Cys Glu Glu Leu Ile Pro Glu Tyr Leu Asn Phe
    370                 375                 380
Ile Arg Gly Val Val Asp Ser Glu Asp Leu Pro Leu Asn Ile Ser Arg
385                 390                 395                 400
Glu Met Leu Gln Gln Ser Lys Ile Leu Lys Val Ile Arg Lys Asn Leu
                405                 410                 415
Val Lys Lys Cys Leu Glu Leu Phe Thr Glu Leu Ala Glu Asp Lys Glu
            420                 425                 430
Asn Tyr Lys Lys Phe Tyr Glu Gln Phe Ser Lys Asn Ile Lys Leu Gly
        435                 440                 445
Ile His Glu Asp Ser Gln Asn Arg Lys Lys Leu Ser Glu Leu Leu Arg
    450                 455                 460
Tyr Tyr Thr Ser Ala Ser Gly Asp Glu Met Val Ser Leu Lys Asp Tyr

465                 470                 475                 480

Cys Thr Arg Met Lys Glu Asn Gln Lys His Ile Tyr Tyr Ile Thr Gly

                485                 490                 495

Glu Thr Lys Asp Gln Val Ala Asn Ser Ala Phe Val Glu Arg Leu Arg

            500                 505                 510

Lys His Gly Leu Glu Val Ile Tyr Met Ile Glu Pro Ile Asp Glu Tyr

        515                 520                 525

Cys Val Gln Gln Leu Lys Glu Phe Glu Gly Lys Thr Leu Val Ser Val

    530                 535                 540

Thr Lys Glu Gly Leu Glu Leu Pro Glu Asp Glu Glu Glu Lys Lys Lys

545                 550                 555                 560

Gln Glu Glu Lys Lys Thr Lys Phe Glu Asn Leu Cys Lys Ile Met Lys

                565                 570                 575

Asp Ile Leu Glu Lys Lys Val Glu Lys Val Val Val Ser Asn Arg Leu

            580                 585                 590

Val Thr Ser Pro Cys Cys Ile Val Thr Ser Thr Tyr Gly Trp Thr Ala

        595                 600                 605

Asn Met Glu Arg Ile Met Lys Ala Gln Ala Leu Arg Asp Asn Ser Thr

    610                 615                 620

Met Gly Tyr Met Ala Ala Lys Lys His Leu Glu Ile Asn Pro Asp His

625                 630                 635                 640

Ser Ile Ile Glu Thr Leu Arg Gln Lys Ala Glu Ala Asp Lys Asn Asp

                645                 650                 655

Lys Ser Val Lys Asp Leu Val Ile Leu Leu Tyr Glu Thr Ala Leu Leu

            660                 665                 670

Ser Ser Gly Phe Ser Leu Glu Asp Pro Gln Thr His Ala Asn Arg Ile

        675                 680                 685

Tyr Arg Met Ile Lys Leu Gly Leu Gly Ile Asp Glu Asp Asp Pro Thr

    690                 695                 700

Ala Asp Asp Thr Ser Ala Ala Val Thr Glu Glu Met Pro Pro Leu Glu
705                 710                 715                 720
Gly Asp Asp Asp Thr Ser Arg Met Glu Glu Val Asp
                725                 730

<210> 108
<211> 1361
<212> PRT
<213> Homo sapiens

<400> 108
Met Gly Ala Ala Gly Arg Gln Asp Phe Leu Phe Lys Ala Met Leu Thr
1               5                   10                  15
Ile Ser Trp Leu Thr Leu Thr Cys Phe Pro Gly Ala Thr Ser Thr Val
            20                  25                  30
Ala Ala Gly Cys Pro Asp Gln Ser Pro Glu Leu Gln Pro Trp Asn Pro
        35                  40                  45
Gly His Asp Gln Asp His His Val His Ile Gly Gln Gly Lys Thr Leu
    50                  55                  60
Leu Leu Thr Ser Ser Ala Thr Val Tyr Ser Ile His Ile Ser Glu Gly
65                  70                  75                  80
Gly Lys Leu Val Ile Lys Asp His Asp Glu Pro Ile Val Leu Arg Thr
                85                  90                  95
Arg His Ile Leu Ile Asp Asn Gly Gly Glu Leu His Ala Gly Ser Ala
            100                 105                 110
Leu Cys Pro Phe Gln Gly Asn Phe Thr Ile Ile Leu Tyr Gly Arg Ala
        115                 120                 125
Asp Glu Gly Ile Gln Pro Asp Pro Tyr Tyr Gly Leu Lys Tyr Ile Gly

    130                 135                 140

Val Gly Lys Gly Gly Ala Leu Glu Leu His Gly Gln Lys Lys Leu Ser

145                 150                 155                 160

Trp Thr Phe Leu Asn Lys Thr Leu His Pro Gly Gly Met Ala Glu Gly

                165                 170                 175

Gly Tyr Phe Phe Glu Arg Ser Trp Gly His Arg Gly Val Ile Val His

            180                 185                 190

Val Ile Asp Pro Lys Ser Gly Thr Val Ile His Ser Asp Arg Phe Asp

        195                 200                 205

Thr Tyr Arg Ser Lys Lys Glu Ser Glu Arg Leu Val Gln Tyr Leu Asn

    210                 215                 220

Ala Val Pro Asp Gly Arg Ile Leu Ser Val Ala Val Asn Asp Glu Gly

225                 230                 235                 240

Ser Arg Asn Leu Asp Asp Met Ala Arg Lys Ala Met Thr Lys Leu Gly

                245                 250                 255

Ser Lys His Phe Leu His Leu Gly Phe Arg His Pro Trp Ser Phe Leu

            260                 265                 270

Thr Val Lys Gly Asn Pro Ser Ser Ser Val Glu Asp His Ile Glu Tyr

        275                 280                 285

His Gly His Arg Gly Ser Ala Ala Ala Arg Val Phe Lys Leu Phe Gln

    290                 295                 300

Thr Glu His Gly Glu Tyr Phe Asn Val Ser Leu Ser Ser Glu Trp Val

305                 310                 315                 320

Gln Asp Val Glu Trp Thr Glu Trp Phe Asp His Asp Lys Val Ser Gln

                325                 330                 335

Thr Lys Gly Gly Glu Lys Ile Ser Asp Leu Trp Lys Ala His Pro Gly

            340                 345                 350

Lys Ile Cys Asn Arg Pro Ile Asp Ile Gln Ala Thr Thr Met Asp Gly

        355                 360                 365

Val Asn Leu Ser Thr Glu Val Val Tyr Lys Lys Gly Gln Asp Tyr Arg
    370                 375                 380
Phe Ala Cys Tyr Asp Arg Gly Arg Ala Cys Arg Ser Tyr Arg Val Arg
385                 390                 395                 400
Phe Leu Cys Gly Lys Pro Val Arg Pro Lys Leu Thr Val Thr Ile Asp
                405                 410                 415
Thr Asn Val Asn Ser Thr Ile Leu Asn Leu Glu Asp Asn Val Gln Ser
            420                 425                 430
Trp Lys Pro Gly Asp Thr Leu Val Ile Ala Ser Thr Asp Tyr Ser Met
        435                 440                 445
Tyr Gln Ala Glu Glu Phe Gln Val Leu Pro Cys Arg Ser Cys Ala Pro
    450                 455                 460
Asn Gln Val Lys Val Ala Gly Lys Pro Met Tyr Leu His Ile Gly Glu
465                 470                 475                 480
Glu Ile Asp Gly Val Asp Met Arg Ala Glu Val Gly Leu Leu Ser Arg
                485                 490                 495
Asn Ile Ile Val Met Gly Glu Met Glu Asp Lys Cys Tyr Pro Tyr Arg
            500                 505                 510
Asn His Ile Cys Asn Phe Phe Asp Phe Asp Thr Phe Gly Gly His Ile
        515                 520                 525
Lys Phe Ala Leu Gly Phe Lys Ala Ala His Leu Glu Gly Thr Glu Leu
    530                 535                 540
Lys His Met Gly Gln Gln Leu Val Gly Gln Tyr Pro Ile His Phe His
545                 550                 555                 560
Leu Ala Gly Asp Val Asp Glu Arg Gly Gly Tyr Asp Pro Pro Thr Tyr
                565                 570                 575
Ile Arg Asp Leu Ser Ile His His Thr Phe Ser Arg Cys Val Thr Val
            580                 585                 590
His Gly Ser Asn Gly Leu Leu Ile Lys Asp Val Val Gly Tyr Asn Ser

        595                 600                 605

Leu Gly His Cys Phe Phe Thr Glu Asp Gly Pro Glu Glu Arg Asn Thr

    610                 615                 620

Phe Asp His Cys Leu Gly Leu Leu Val Lys Ser Gly Thr Leu Leu Pro

625                 630                 635                 640

Ser Asp Arg Asp Ser Lys Met Cys Lys Met Ile Thr Glu Asp Ser Tyr

                645                 650                 655

Pro Gly Tyr Ile Pro Lys Pro Arg Gln Asp Cys Asn Ala Val Ser Thr

            660                 665                 670

Phe Trp Met Ala Asn Pro Asn Asn Asn Leu Ile Asn Cys Ala Ala Ala

        675                 680                 685

Gly Ser Glu Glu Thr Gly Phe Trp Phe Ile Phe His His Val Pro Thr

    690                 695                 700

Gly Pro Ser Val Gly Met Tyr Ser Pro Gly Tyr Ser Glu His Ile Pro

705                 710                 715                 720

Leu Gly Lys Phe Tyr Asn Asn Arg Ala His Ser Asn Tyr Arg Ala Gly

                725                 730                 735

Met Ile Ile Asp Asn Gly Val Lys Thr Thr Glu Ala Ser Ala Lys Asp

            740                 745                 750

Lys Arg Pro Phe Leu Ser Ile Ile Ser Ala Arg Tyr Ser Pro His Gln

        755                 760                 765

Asp Ala Asp Pro Leu Lys Pro Arg Glu Pro Ala Ile Ile Arg His Phe

    770                 775                 780

Ile Ala Tyr Lys Asn Gln Asp His Gly Ala Trp Leu Arg Gly Gly Asp

785                 790                 795                 800

Val Trp Leu Asp Ser Cys Arg Phe Ala Asp Asn Gly Ile Gly Leu Thr

                805                 810                 815

Leu Ala Ser Gly Gly Thr Phe Pro Tyr Asp Asp Gly Ser Lys Gln Glu

            820                 825                 830

Ile Lys Asn Ser Leu Phe Val Gly Glu Ser Gly Asn Val Gly Thr Glu
        835                 840                 845
Met Met Asp Asn Arg Ile Trp Gly Pro Gly Gly Leu Asp His Ser Gly
    850                 855                 860
Arg Thr Leu Pro Ile Gly Gln Asn Phe Pro Ile Arg Gly Ile Gln Leu
865                 870                 875                 880
Tyr Asp Gly Pro Ile Asn Ile Gln Asn Cys Thr Phe Arg Lys Phe Val
                885                 890                 895
Ala Leu Glu Gly Arg His Thr Ser Ala Leu Ala Phe Arg Leu Asn Asn
            900                 905                 910
Ala Trp Gln Ser Cys Pro His Asn Asn Val Thr Gly Ile Ala Phe Glu
        915                 920                 925
Asp Val Pro Ile Thr Ser Arg Val Phe Phe Gly Glu Pro Gly Pro Trp
    930                 935                 940
Phe Asn Gln Leu Asp Met Asp Gly Asp Lys Thr Ser Val Phe His Asp
945                 950                 955                 960
Val Asp Gly Ser Val Ser Glu Tyr Pro Gly Ser Tyr Leu Thr Lys Asn
                965                 970                 975
Asp Asn Trp Leu Val Arg His Pro Asp Cys Ile Asn Val Pro Asp Trp
            980                 985                 990
Arg Gly Ala Ile Cys Ser Gly Cys Tyr Ala Gln Met Tyr Ile Gln Ala
        995                 1000                1005
Tyr Lys Thr Ser Asn Leu Arg Met Lys Ile Ile Lys Asn Asp Phe Pro
    1010                1015                1020
Ser His Pro Leu Tyr Leu Glu Gly Ala Leu Thr Arg Ser Thr His Tyr
1025                1030                1035                1040
Gln Gln Tyr Gln Pro Val Val Thr Leu Gln Lys Gly Tyr Thr Ile His
                1045                1050                1055
Trp Asp Gln Thr Ala Pro Ala Glu Leu Ala Ile Trp Leu Ile Asn Phe

1060                1065                1070

Asn Lys Gly Asp Trp Ile Arg Val Gly Leu Cys Tyr Pro Arg Gly Thr

        1075                1080                1085

Thr Phe Ser Ile Leu Ser Asp Val His Asn Arg Leu Leu Lys Gln Thr

    1090                1095                1100

Ser Lys Thr Gly Val Phe Val Arg Thr Leu Gln Met Asp Lys Val Glu

1105                1110                1115                1120

Gln Ser Tyr Pro Gly Arg Ser His Tyr Tyr Trp Asp Glu Asp Ser Gly

                1125                1130                1135

Leu Leu Phe Leu Lys Leu Lys Ala Gln Asn Glu Arg Glu Lys Phe Ala

            1140                1145                1150

Phe Cys Ser Met Lys Gly Cys Glu Arg Ile Lys Ile Lys Ala Leu Ile

        1155                1160                1165

Pro Lys Asn Ala Gly Val Ser Asp Cys Thr Ala Thr Ala Tyr Pro Lys

    1170                1175                1180

Phe Thr Glu Arg Ala Val Val Asp Val Pro Met Pro Lys Lys Leu Phe

1185                1190                1195                1200

Gly Ser Gln Leu Lys Thr Lys Asp His Phe Leu Glu Val Lys Met Glu

                1205                1210                1215

Ser Ser Lys Gln His Phe Phe His Leu Trp Asn Asp Phe Ala Tyr Ile

            1220                1225                1230

Glu Val Asp Gly Lys Lys Tyr Pro Ser Ser Glu Asp Gly Ile Gln Val

        1235                1240                1245

Val Val Ile Asp Gly Asn Gln Gly Arg Val Val Ser His Thr Ser Phe

    1250                1255                1260

Arg Asn Ser Ile Leu Gln Gly Ile Pro Trp Gln Leu Phe Asn Tyr Val

1265                1270                1275                1280

Ala Thr Ile Pro Asp Asn Ser Ile Val Leu Met Ala Ser Lys Gly Arg

                1285                1290                1295

Tyr Val Ser Arg Gly Pro Trp Thr Arg Val Leu Glu Lys Leu Gly Ala
            1300                1305                1310
Asp Arg Gly Leu Lys Leu Lys Glu Gln Met Ala Phe Val Gly Phe Lys
        1315                1320                1325
Gly Ser Phe Arg Pro Ile Trp Val Thr Leu Asp Thr Glu Asp His Lys
    1330                1335                1340
Ala Lys Ile Phe Gln Val Val Pro Ile Pro Val Val Lys Lys Lys Lys
1345                1350                1355                1360
Leu

<210> 109
<211> 469
<212> PRT
<213> Homo sapiens

<400> 109
Met His Ser Phe Pro Pro Leu Leu Leu Leu Leu Phe Trp Gly Val Val
1               5                   10                  15
Ser His Ser Phe Pro Ala Thr Leu Glu Thr Gln Glu Gln Asp Val Asp
            20                  25                  30
Leu Val Gln Lys Tyr Leu Glu Lys Tyr Tyr Asn Leu Lys Asn Asp Gly
        35                  40                  45
Arg Gln Val Glu Lys Arg Arg Asn Ser Gly Pro Val Val Glu Lys Leu
    50                  55                  60
Lys Gln Met Gln Glu Phe Phe Gly Leu Lys Val Thr Gly Lys Pro Asp
65                  70                  75                  80
Ala Glu Thr Leu Lys Val Met Lys Gln Pro Arg Cys Gly Val Pro Asp

                85                  90                  95
Val Ala Gln Phe Val Leu Thr Glu Gly Asn Pro Arg Trp Glu Gln Thr
            100                 105                 110
His Leu Thr Tyr Arg Ile Glu Asn Tyr Thr Pro Asp Leu Pro Arg Ala
        115                 120                 125
Asp Val Asp His Ala Ile Glu Lys Ala Phe Gln Leu Trp Ser Asn Val
    130                 135                 140
Thr Pro Leu Thr Phe Thr Lys Val Ser Glu Gly Gln Ala Asp Ile Met
145                 150                 155                 160
Ile Ser Phe Val Arg Gly Asp His Arg Asp Asn Ser Pro Phe Asp Gly
                165                 170                 175
Pro Gly Gly Asn Leu Ala His Ala Phe Gln Pro Gly Pro Gly Ile Gly
            180                 185                 190
Gly Asp Ala His Phe Asp Glu Asp Glu Arg Trp Thr Asn Asn Phe Arg
        195                 200                 205
Glu Tyr Asn Leu His Arg Val Ala Ala His Glu Leu Gly His Ser Leu
    210                 215                 220
Gly Leu Ser His Ser Thr Asp Ile Gly Ala Leu Met Tyr Pro Ser Tyr
225                 230                 235                 240
Thr Phe Ser Gly Asp Val Gln Leu Ala Gln Asp Asp Ile Asp Gly Ile
                245                 250                 255
Gln Ala Ile Tyr Gly Arg Ser Gln Asn Pro Val Gln Pro Ile Gly Pro
            260                 265                 270
Gln Thr Pro Lys Ala Cys Asp Ser Lys Leu Thr Phe Asp Ala Ile Thr
        275                 280                 285
Thr Ile Arg Gly Glu Val Met Phe Phe Lys Asp Arg Phe Tyr Met Arg
    290                 295                 300
Thr Asn Pro Phe Tyr Pro Glu Val Glu Leu Asn Phe Ile Ser Val Phe
305                 310                 315                 320

Trp Pro Gln Leu Pro Asn Gly Leu Glu Ala Ala Tyr Glu Phe Ala Asp
                325                 330                 335
Arg Asp Glu Val Arg Phe Phe Lys Gly Asn Lys Tyr Trp Ala Val Gln
            340                 345                 350
Gly Gln Asn Val Leu His Gly Tyr Pro Lys Asp Ile Tyr Ser Ser Phe
        355                 360                 365
Gly Phe Pro Arg Thr Val Lys His Ile Asp Ala Ala Leu Ser Glu Glu
    370                 375                 380
Asn Thr Gly Lys Thr Tyr Phe Phe Val Ala Asn Lys Tyr Trp Arg Tyr
385                 390                 395                 400
Asp Glu Tyr Lys Arg Ser Met Asp Pro Gly Tyr Pro Lys Met Ile Ala
                405                 410                 415
His Asp Phe Pro Gly Ile Gly His Lys Val Asp Ala Val Phe Met Lys
            420                 425                 430
Asp Gly Phe Phe Tyr Phe Phe His Gly Thr Arg Gln Tyr Lys Phe Asp
        435                 440                 445
Pro Lys Thr Lys Arg Ile Leu Thr Leu Gln Lys Ala Asn Ser Trp Phe
    450                 455                 460
Asn Cys Arg Lys Asn
465

<210> 110
<211> 267
<212> PRT
<213> Homo sapiens

<400> 110
Met Arg Leu Thr Val Leu Cys Ala Val Cys Leu Leu Pro Gly Ser Leu

1                   5                   10                  15

Ala Leu Pro Leu Pro Gln Glu Ala Gly Gly Met Ser Glu Leu Gln Trp

                20                  25                  30

Glu Gln Ala Gln Asp Tyr Leu Lys Arg Phe Tyr Leu Tyr Asp Ser Glu

            35                  40                  45

Thr Lys Asn Ala Asn Ser Leu Glu Ala Lys Leu Lys Glu Met Gln Lys

    50                  55                  60

Phe Phe Gly Leu Pro Ile Thr Gly Met Leu Asn Ser Arg Val Ile Glu

65                  70                  75                  80

Ile Met Gln Lys Pro Arg Cys Gly Val Pro Asp Val Ala Glu Tyr Ser

                85                  90                  95

Leu Phe Pro Asn Ser Pro Lys Trp Thr Ser Lys Val Val Thr Tyr Arg

            100                 105                 110

Ile Val Ser Tyr Thr Arg Asp Leu Pro His Ile Thr Val Asp Arg Leu

        115                 120                 125

Val Ser Lys Ala Leu Asn Met Trp Gly Lys Glu Ile Pro Leu His Phe

    130                 135                 140

Arg Lys Val Val Trp Gly Thr Ala Asp Ile Met Ile Gly Phe Ala Arg

145                 150                 155                 160

Gly Ala His Gly Asp Ser Tyr Pro Phe Asp Gly Pro Gly Asn Thr Leu

                165                 170                 175

Ala His Ala Phe Ala Pro Gly Thr Gly Leu Gly Gly Asp Ala His Phe

            180                 185                 190

Asp Glu Asp Glu Arg Trp Thr Asp Gly Ser Ser Leu Gly Ile Asn Phe

        195                 200                 205

Leu Tyr Ala Ala Thr His Glu Leu Gly His Ser Leu Gly Met Gly His

    210                 215                 220

Ser Ser Asp Pro Asn Ala Val Met Tyr Pro Thr Tyr Gly Asn Gly Asp

225                 230                 235                 240

Pro Gln Asn Phe Lys Leu Ser Gln Asp Asp Ile Lys Gly Ile Gln Lys
                245                 250                 255
Leu Tyr Gly Lys Arg Ser Asn Ser Arg Lys Lys
            260                 265

<210> 111
<211> 216
<212> PRT
<213> Homo sapiens

<400> 111
Met Arg Pro Arg Ser Gly Pro Thr Arg Asn Pro Arg Leu Arg Ala Phe
1               5                   10                  15
Ala Gly Val Pro Thr Arg Gly Arg Thr Arg Gly Gln Ser Arg Arg Cys
            20                  25                  30
Ala Ala Glu Ala Ser Ala Gly Pro Glu Arg Asp Ala Arg Pro Gly Ala
        35                  40                  45
Pro Ala Ala Gly Thr Met Gly Ala Ala His Ser Ala Ser Glu Glu Val
    50                  55                  60
Arg Glu Leu Glu Gly Lys Thr Gly Phe Ser Ser Asp Gln Ile Glu Gln
65                  70                  75                  80
Leu His Arg Arg Phe Lys Gln Leu Ser Gly Asp Gln Pro Thr Ile Arg
                85                  90                  95
Lys Glu Asn Phe Asn Asn Val Pro Asp Leu Glu Leu Asn Pro Ile Arg
            100                 105                 110
Ser Lys Ile Val Arg Ala Phe Phe Asp Asn Arg Asn Leu Arg Lys Gly
        115                 120                 125
Pro Ser Gly Leu Ala Asp Glu Ile Asn Phe Glu Asp Phe Leu Thr Ile

130                 135                 140

Met Ser Tyr Phe Arg Pro Ile Asp Thr Thr Met Asp Glu Glu Gln Val

145                 150                 155                 160

Glu Leu Ser Arg Lys Glu Lys Leu Arg Phe Leu Phe His Met Tyr Asp

                165                 170                 175

Ser Asp Ser Asp Gly Arg Ile Thr Leu Glu Glu Tyr Arg Asn Val Lys

            180                 185                 190

Trp Ser Arg Ser Cys Cys Arg Glu Thr Leu Thr Ser Arg Arg Ser Pro

        195                 200                 205

Leu Ala Pro Ser Pro Thr Gly Pro

    210                 215

<210> 112

<211> 422

<212> PRT

<213> Homo sapiens

<400> 112

<210> 113

<211> 398

<212> PRT

<213> Homo sapiens

<400> 113

Met Met Leu Lys Gly Ile Thr Arg Leu Ile Ser Arg Ile His Lys Leu

 1               5                  10                  15

Asp Pro Gly Arg Phe Leu His Met Gly Thr Gln Ala Arg Gln Ser Ile

            20                  25                  30

Ala Ala His Leu Asp Asn Gln Val Pro Val Glu Ser Pro Arg Ala Ile

        35                  40                  45

Ser Arg Thr Asn Glu Asn Asp Pro Ala Lys His Gly Asp Gln His Glu
    50                  55                  60
Gly Gln His Tyr Asn Ile Ser Pro Gln Asp Leu Glu Thr Val Phe Pro
65                  70                  75                  80
His Gly Leu Pro Pro Arg Phe Val Met Gln Val Lys Thr Phe Ser Glu
                85                  90                  95
Ala Cys Leu Met Val Arg Lys Pro Ala Leu Glu Leu Leu His Tyr Leu
            100                 105                 110
Lys Asn Thr Ser Phe Ala Tyr Pro Ala Ile Arg Tyr Leu Leu Tyr Gly
        115                 120                 125
Glu Lys Gly Thr Gly Lys Thr Leu Ser Leu Cys His Val Ile His Phe
    130                 135                 140
Cys Ala Lys Gln Asp Trp Leu Ile Leu His Ile Pro Asp Ala His Leu
145                 150                 155                 160
Trp Val Lys Asn Cys Arg Asp Leu Leu Gln Ser Ser Tyr Asn Lys Gln
                165                 170                 175
Arg Phe Asp Gln Pro Leu Glu Ala Ser Thr Trp Leu Lys Asn Phe Lys
            180                 185                 190
Thr Thr Asn Glu Arg Phe Leu Asn Gln Ile Lys Val Gln Glu Lys Tyr
        195                 200                 205
Val Trp Asn Lys Arg Glu Ser Thr Glu Lys Gly Ser Pro Leu Gly Glu
    210                 215                 220
Val Val Glu Gln Gly Ile Thr Arg Val Arg Asn Ala Thr Asp Ala Val
225                 230                 235                 240
Gly Ile Val Leu Lys Glu Leu Lys Arg Gln Ser Ser Leu Gly Met Phe
                245                 250                 255
His Leu Leu Val Ala Val Asp Gly Ile Asn Ala Leu Trp Gly Arg Thr
            260                 265                 270
Thr Leu Lys Arg Glu Asp Lys Ser Pro Ile Ala Pro Glu Glu Leu Ala

        275                 280                 285

Leu Val His Asn Leu Arg Lys Met Met Lys Asn Asp Trp His Gly Gly

    290                 295                 300

Ala Ile Val Ser Ala Leu Ser Gln Thr Gly Ser Leu Phe Lys Pro Arg

305                 310                 315                 320

Lys Ala Tyr Leu Pro Gln Glu Leu Leu Gly Lys Glu Gly Phe Asp Ala

                325                 330                 335

Leu Asp Pro Phe Ile Pro Ile Leu Val Ser Asn Tyr Asn Pro Lys Glu

            340                 345                 350

Phe Glu Ser Cys Ile Gln Tyr Tyr Leu Glu Asn Asn Trp Leu Gln His

        355                 360                 365

Glu Lys Ala Pro Thr Glu Glu Gly Lys Lys Glu Leu Leu Phe Leu Ser

    370                 375                 380

Asn Ala Asn Pro Ser Leu Leu Glu Arg His Cys Ala Tyr Leu

385                 390                 395

<210> 114

<211> 75

<212> PRT

<213> Homo sapiens

<400> 114

Met Leu Ser His Phe Arg Val Lys Val Lys Gly Phe Ile Leu Ile Ser

1               5                   10                  15

Lys Tyr Phe Asp Pro Tyr Asp Leu Val Ser Ser Tyr Pro Lys Tyr Gly

            20                  25                  30

Pro His Thr Ser Arg Thr Gly Ile Leu Trp Glu Leu Val Arg Asn Val

        35                  40                  45

Glu Ser Leu Val Leu Arg Phe Ser Lys Ser Glu Ser Ala Phe Ser Ser
    50                  55                  60
Ala Leu Leu Ala Ile His Met Phe Glu Lys Asp
65                  70                  75

<210> 115
<211> 163
<212> PRT
<213> Homo sapiens

<400> 115
Met Ser Glu Ser Gly Phe Lys Leu Leu Cys Gln Cys Leu Gly Phe Gly
1               5                   10                  15
Ser Gly His Phe Arg Cys Asp Ser Ser Arg Trp Cys His Asp Asn Gly
            20                  25                  30
Val Asn Tyr Lys Ile Gly Glu Lys Trp Asp Arg Gln Gly Glu Asn Gly
        35                  40                  45
Gln Met Met Ser Cys Thr Cys Leu Gly Asn Gly Lys Gly Glu Phe Lys
    50                  55                  60
Cys Asp Pro His Glu Ala Thr Cys Tyr Asp Asp Gly Lys Thr Tyr His
65                  70                  75                  80
Val Gly Glu Gln Trp Gln Lys Glu Tyr Leu Gly Ala Ile Cys Ser Cys
                85                  90                  95
Thr Cys Phe Gly Gly Gln Arg Gly Trp Arg Cys Asp Asn Cys Arg Arg
            100                 105                 110
Pro Gly Gly Glu Pro Ser Pro Glu Gly Thr Thr Gly Gln Ser Tyr Asn
        115                 120                 125
Gln Tyr Ser Gln Arg Tyr His Gln Arg Thr Asn Thr Asn Val Asn Cys

130                 135                 140

Pro Ile Glu Cys Phe Met Pro Leu Asp Val Gln Ala Asp Arg Glu Asp

145                 150                 155                 160

Ser Arg Glu

<210> 116

<211> 483

<212> PRT

<213> Homo sapiens

<400> 116

Met Ser Ile Arg Val Thr Gln Lys Ser Tyr Lys Val Ser Thr Ser Gly

1               5                   10                  15

Pro Arg Ala Phe Ser Ser Arg Ser Tyr Thr Ser Gly Pro Gly Ser Arg

            20                  25                  30

Ile Ser Ser Ser Ser Phe Ser Arg Val Gly Ser Ser Asn Phe Arg Gly

        35                  40                  45

Gly Leu Gly Gly Gly Tyr Gly Gly Ala Ser Gly Met Gly Gly Ile Thr

    50                  55                  60

Ala Val Thr Val Asn Gln Ser Leu Leu Ser Pro Leu Val Leu Glu Val

65                  70                  75                  80

Asp Pro Asn Ile Gln Ala Val Arg Thr Gln Glu Lys Glu Gln Ile Lys

                85                  90                  95

Thr Leu Asn Asn Lys Phe Ala Ser Phe Ile Asp Lys Val Arg Phe Leu

            100                 105                 110

Glu Gln Gln Asn Lys Met Leu Glu Thr Lys Trp Ser Leu Leu Gln Gln

        115                 120                 125

Gln Lys Thr Ala Arg Ser Asn Met Asp Asn Met Phe Glu Ser Tyr Ile
    130                 135                 140
Asn Asn Leu Arg Arg Gln Leu Glu Thr Leu Gly Gln Glu Lys Leu Lys
145                 150                 155                 160
Leu Glu Ala Glu Leu Gly Asn Met Gln Gly Leu Val Glu Asp Phe Lys
                165                 170                 175
Asn Lys Tyr Glu Asp Glu Ile Asn Lys Arg Thr Glu Met Glu Asn Glu
            180                 185                 190
Phe Val Leu Ile Lys Lys Asp Val Asp Glu Ala Tyr Met Asn Lys Val
        195                 200                 205
Glu Leu Glu Ser Arg Leu Glu Gly Leu Thr Asp Glu Ile Asn Phe Leu
    210                 215                 220
Arg Gln Leu Tyr Glu Glu Glu Ile Arg Glu Leu Gln Ser Gln Ile Ser
225                 230                 235                 240
Asp Thr Ser Val Val Leu Ser Met Asp Asn Ser Arg Ser Leu Asp Met
                245                 250                 255
Asp Ser Ile Ile Ala Glu Val Lys Ala Gln Tyr Glu Asp Ile Ala Asn
            260                 265                 270
Arg Ser Arg Ala Glu Ala Glu Ser Met Tyr Gln Ile Lys Tyr Glu Glu
        275                 280                 285
Leu Gln Ser Leu Ala Gly Lys His Gly Asp Asp Leu Arg Arg Thr Lys
    290                 295                 300
Thr Glu Ile Ser Glu Met Asn Arg Asn Ile Ser Arg Leu Gln Ala Glu
305                 310                 315                 320
Ile Glu Gly Leu Lys Gly Gln Arg Ala Ser Leu Glu Ala Ala Ile Ala
                325                 330                 335
Asp Ala Glu Gln Arg Gly Glu Leu Ala Ile Lys Asp Ala Asn Ala Lys
            340                 345                 350
Leu Ser Glu Leu Glu Ala Ala Leu Gln Arg Ala Lys Gln Asp Met Ala

        355                 360                 365

Arg Gln Leu Arg Glu Tyr Gln Glu Leu Met Asn Val Lys Leu Ala Leu

    370                 375                 380

Asp Ile Glu Ile Ala Thr Tyr Arg Lys Leu Leu Glu Gly Glu Glu Ser

385                 390                 395                 400

Arg Leu Glu Ser Gly Met Gln Asn Met Ser Ile His Thr Lys Thr Thr

                405                 410                 415

Ser Gly Tyr Ala Gly Gly Leu Ser Ser Ala Tyr Gly Gly Leu Thr Ser

            420                 425                 430

Pro Gly Leu Ser Tyr Ser Leu Gly Ser Ser Phe Gly Ser Gly Ala Gly

        435                 440                 445

Ser Ser Ser Phe Ser Arg Thr Ser Ser Ser Arg Ala Val Val Val Lys

    450                 455                 460

Lys Ile Glu Thr Arg Asp Gly Lys Leu Val Ser Glu Ser Ser Asp Val

465                 470                 475                 480

Leu Pro Lys

<210> 117

<211> 430

<212> PRT

<213> Homo sapiens

<400> 117

Met Ser Phe Thr Thr Arg Ser Thr Phe Ser Thr Asn Tyr Arg Ser Leu

1               5                   10                  15

Gly Ser Val Gln Ala Pro Ser Tyr Gly Ala Arg Pro Val Ser Ser Ala

            20                  25                  30

Ala Ser Val Tyr Ala Gly Ala Gly Gly Ser Gly Ser Arg Ile Ser Val
        35                  40                  45
Ser Arg Ser Thr Ser Phe Arg Gly Gly Met Gly Ser Gly Gly Leu Ala
    50                  55                  60
Thr Gly Ile Ala Gly Gly Leu Ala Gly Met Gly Gly Ile Gln Asn Glu
65                  70                  75                  80
Lys Glu Thr Met Gln Ser Leu Asn Asp Arg Leu Ala Ser Tyr Leu Asp
                85                  90                  95
Arg Val Arg Ser Leu Glu Thr Glu Asn Arg Arg Leu Glu Ser Lys Ile
            100                 105                 110
Arg Glu His Leu Glu Lys Lys Gly Pro Gln Val Arg Asp Trp Ser His
        115                 120                 125
Tyr Phe Lys Ile Ile Glu Asp Leu Arg Ala Gln Ile Phe Ala Asn Thr
    130                 135                 140
Val Asp Asn Ala Arg Ile Val Leu Gln Ile Asp Asn Ala Arg Leu Ala
145                 150                 155                 160
Ala Asp Asp Phe Arg Val Lys Tyr Glu Thr Glu Leu Ala Met Arg Gln
                165                 170                 175
Ser Val Glu Asn Asp Ile His Gly Leu Arg Lys Val Ile Asp Asp Thr
            180                 185                 190
Asn Ile Thr Arg Leu Gln Leu Glu Thr Glu Ile Glu Ala Leu Lys Glu
        195                 200                 205
Glu Leu Leu Phe Met Lys Lys Asn His Glu Glu Glu Val Lys Gly Leu
    210                 215                 220
Gln Ala Gln Ile Ala Ser Ser Gly Leu Thr Val Glu Val Asp Ala Pro
225                 230                 235                 240
Lys Ser Gln Asp Leu Ala Lys Ile Met Ala Asp Ile Arg Ala Gln Tyr
                245                 250                 255
Asp Glu Leu Ala Arg Lys Asn Arg Glu Glu Leu Asp Lys Tyr Trp Ser

            260                 265                 270

Gln Gln Ile Glu Glu Ser Thr Thr Val Val Thr Thr Gln Ser Ala Glu

        275                 280                 285

Val Gly Ala Ala Glu Thr Thr Leu Thr Glu Leu Arg Arg Thr Val Gln

    290                 295                 300

Ser Leu Glu Ile Asp Leu Asp Ser Met Arg Asn Leu Lys Ala Ser Leu

305                 310                 315                 320

Glu Asn Ser Leu Arg Glu Val Glu Ala Arg Tyr Ala Leu Gln Met Glu

                325                 330                 335

Gln Leu Asn Gly Ile Leu Leu His Leu Glu Ser Glu Leu Ala Gln Thr

            340                 345                 350

Arg Ala Glu Gly Gln Arg Gln Ala Gln Glu Tyr Glu Ala Leu Leu Asn

        355                 360                 365

Ile Lys Val Lys Leu Glu Ala Glu Ile Ala Thr Tyr Arg Arg Leu Leu

    370                 375                 380

Glu Asp Gly Glu Asp Phe Asn Leu Gly Asp Ala Leu Asp Ser Ser Asn

385                 390                 395                 400

Ser Met Gln Thr Ile Gln Lys Thr Thr Thr Arg Arg Ile Val Asp Gly

                405                 410                 415

Lys Val Val Ser Glu Thr Asn Asp Thr Lys Val Leu Arg His

            420                 425                 430

<210> 118

<211> 400

<212> PRT

<213> Homo sapiens

<400> 118

Met Thr Ser Tyr Ser Tyr Arg Gln Ser Ser Ala Thr Ser Ser Phe Gly
1               5                   10                  15
Gly Leu Gly Gly Gly Ser Val Arg Phe Gly Pro Gly Val Ala Phe Arg
            20                  25                  30
Ala Pro Ser Ile His Gly Gly Ser Gly Gly Arg Gly Val Ser Val Ser
        35                  40                  45
Ser Ala Arg Phe Val Ser Ser Ser Ser Ser Gly Ala Tyr Gly Gly Gly
    50                  55                  60
Tyr Gly Gly Val Leu Thr Ala Ser Asp Gly Leu Leu Ala Gly Asn Glu
65                  70                  75                  80
Lys Leu Thr Met Gln Asn Leu Asn Asp Arg Leu Ala Ser Tyr Leu Asp
                85                  90                  95
Lys Val Arg Ala Leu Glu Ala Ala Asn Gly Glu Leu Glu Val Lys Ile
            100                 105                 110
Arg Asp Trp Tyr Gln Lys Gln Gly Pro Gly Pro Ser Arg Asp Tyr Ser
        115                 120                 125
His Tyr Tyr Thr Thr Ile Gln Asp Leu Arg Asp Lys Ile Leu Gly Ala
    130                 135                 140
Thr Ile Glu Asn Ser Arg Ile Val Leu Gln Ile Asp Asn Ala Arg Leu
145                 150                 155                 160
Ala Ala Asp Asp Phe Arg Thr Lys Phe Glu Thr Glu Gln Ala Leu Arg
                165                 170                 175
Met Ser Val Glu Ala Asp Ile Asn Gly Leu Arg Arg Val Leu Asp Glu
            180                 185                 190
Leu Thr Leu Ala Arg Thr Asp Leu Glu Met Gln Ile Glu Gly Leu Lys
        195                 200                 205
Glu Glu Leu Ala Tyr Leu Lys Lys Asn His Glu Glu Glu Ile Ser Thr
    210                 215                 220
Leu Arg Gly Gln Val Gly Gly Gln Val Ser Val Glu Val Asp Ser Ala

225                 230                 235                 240

Pro Gly Thr Asp Leu Ala Lys Ile Leu Ser Asp Met Arg Ser Gln Tyr

                245                 250                 255

Glu Val Met Ala Glu Gln Asn Arg Lys Asp Ala Glu Ala Trp Phe Thr

            260                 265                 270

Ser Arg Thr Glu Glu Leu Asn Arg Glu Val Ala Gly His Thr Glu Gln

        275                 280                 285

Leu Gln Met Ser Arg Ser Glu Val Thr Asp Leu Arg Arg Thr Leu Gln

    290                 295                 300

Gly Leu Glu Ile Glu Leu Gln Ser Gln Leu Ser Met Lys Ala Ala Leu

305                 310                 315                 320

Glu Asp Thr Leu Ala Glu Thr Glu Ala Arg Phe Gly Ala Gln Leu Ala

                325                 330                 335

His Ile Gln Ala Leu Ile Ser Gly Ile Glu Ala Gln Leu Gly Asp Val

            340                 345                 350

Arg Ala Asp Ser Glu Arg Gln Asn Gln Glu Tyr Gln Arg Leu Met Asp

        355                 360                 365

Ile Lys Ser Arg Leu Glu Gln Glu Ile Ala Thr Tyr Arg Ser Leu Leu

    370                 375                 380

Glu Gly Gln Glu Asp His Tyr Asn Asn Leu Ser Ala Ser Lys Val Leu

385                 390                 395                 400

<210> 119

<211> 424

<212> PRT

<213> Homo sapiens

<400> 119

Met Asp Phe Ser Arg Arg Ser Phe His Arg Ser Leu Ser Ser Ser Leu
1               5                   10                  15
Gln Ala Pro Val Val Ser Thr Val Gly Met Gln Arg Leu Gly Thr Thr
            20                  25                  30
Pro Ser Val Tyr Gly Gly Ala Gly Gly Arg Gly Ile Arg Ile Ser Asn
        35                  40                  45
Ser Arg His Thr Val Asn Tyr Gly Ser Asp Leu Thr Gly Gly Gly Asp
    50                  55                  60
Leu Phe Val Gly Asn Glu Lys Met Ala Met Gln Asn Leu Asn Asp Arg
65                  70                  75                  80
Leu Ala Ser Tyr Leu Glu Lys Val Arg Thr Leu Glu Gln Ser Asn Ser
                85                  90                  95
Lys Leu Glu Val Gln Ile Lys Gln Trp Tyr Glu Thr Asn Ala Pro Arg
            100                 105                 110
Ala Gly Arg Asp Tyr Ser Ala Tyr Tyr Arg Gln Ile Glu Glu Leu Arg
        115                 120                 125
Ser Gln Ile Lys Asp Ala Gln Leu Gln Asn Ala Arg Cys Val Leu Gln
    130                 135                 140
Ile Asp Asn Ala Lys Leu Ala Ala Glu Asp Phe Arg Leu Lys Tyr Glu
145                 150                 155                 160
Thr Glu Arg Gly Ile Arg Leu Thr Val Glu Ala Asp Leu Gln Gly Leu
                165                 170                 175
Asn Lys Val Phe Asp Asp Leu Thr Leu His Lys Thr Asp Leu Glu Ile
            180                 185                 190
Gln Ile Glu Glu Leu Asn Lys Asp Leu Ala Leu Leu Lys Lys Glu His
        195                 200                 205
Gln Glu Glu Val Asp Gly Leu His Lys His Leu Gly Asn Thr Val Asn
    210                 215                 220
Val Glu Val Asp Ala Ala Pro Gly Leu Asn Leu Gly Val Ile Met Asn

225                 230                 235                 240

Glu Met Arg Gln Lys Tyr Glu Val Met Ala Gln Lys Asn Leu Gln Glu

                245                 250                 255

Ala Lys Glu Gln Phe Glu Arg Gln Thr Ala Val Leu Gln Gln Gln Val

            260                 265                 270

Thr Val Asn Thr Glu Glu Leu Lys Gly Thr Glu Val Gln Leu Thr Glu

        275                 280                 285

Leu Arg Arg Thr Ser Gln Ser Leu Glu Ile Glu Leu Gln Ser His Leu

    290                 295                 300

Ser Met Lys Glu Ser Leu Glu His Thr Leu Glu Glu Thr Lys Ala Arg

305                 310                 315                 320

Tyr Ser Ser Gln Leu Ala Asn Leu Gln Ser Leu Leu Ser Ser Leu Glu

                325                 330                 335

Ala Gln Leu Met Gln Ile Arg Ser Asn Met Glu Arg Gln Asn Asn Glu

            340                 345                 350

Tyr His Ile Leu Leu Asp Ile Lys Thr Arg Leu Glu Gln Glu Ile Ala

        355                 360                 365

Thr Tyr Arg Arg Leu Leu Glu Gly Glu Asp Val Lys Thr Thr Glu Tyr

    370                 375                 380

Gln Leu Ser Thr Leu Glu Glu Arg Asp Ile Lys Lys Thr Arg Lys Ile

385                 390                 395                 400

Lys Thr Val Val Gln Glu Val Val Asp Gly Lys Val Val Ser Ser Glu

                405                 410                 415

Val Lys Glu Val Glu Glu Asn Ile

            420

<210> 120

<211> 1255

<212> PRT

<213> Homo sapiens

<400> 120

Met Thr Pro Gly Thr Gln Ser Pro Phe Phe Leu Leu Leu Leu Leu Thr
1               5                   10                  15

Val Leu Thr Val Val Thr Gly Ser Gly His Ala Ser Ser Thr Pro Gly
            20                  25                  30

Gly Glu Lys Glu Thr Ser Ala Thr Gln Arg Ser Ser Val Pro Ser Ser
        35                  40                  45

Thr Glu Lys Asn Ala Val Ser Met Thr Ser Ser Val Leu Ser Ser His
    50                  55                  60

Ser Pro Gly Ser Gly Ser Ser Thr Thr Gln Gly Gln Asp Val Thr Leu
65                  70                  75                  80

Ala Pro Ala Thr Glu Pro Ala Ser Gly Ser Ala Ala Thr Trp Gly Gln
                85                  90                  95

Asp Val Thr Ser Val Pro Val Thr Arg Pro Ala Leu Gly Ser Thr Thr
            100                 105                 110

Pro Pro Ala His Asp Val Thr Ser Ala Pro Asp Asn Lys Pro Ala Pro
        115                 120                 125

Gly Ser Thr Ala Pro Pro Ala His Gly Val Thr Ser Ala Pro Asp Thr
    130                 135                 140

Arg Pro Ala Pro Gly Ser Thr Ala Pro Pro Ala His Gly Val Thr Ser
145                 150                 155                 160

Ala Pro Asp Thr Arg Pro Ala Pro Gly Ser Thr Ala Pro Pro Ala His
                165                 170                 175

Gly Val Thr Ser Ala Pro Asp Thr Arg Pro Ala Pro Gly Ser Thr Ala
            180                 185                 190

Pro Pro Ala His Gly Val Thr Ser Ala Pro Asp Thr Arg Pro Ala Pro

        195                 200                 205

Gly Ser Thr Ala Pro Pro Ala His Gly Val Thr Ser Ala Pro Asp Thr

    210                 215                 220

Arg Pro Ala Pro Gly Ser Thr Ala Pro Pro Ala His Gly Val Thr Ser

225                 230                 235                 240

Ala Pro Asp Thr Arg Pro Ala Pro Gly Ser Thr Ala Pro Pro Ala His

                245                 250                 255

Gly Val Thr Ser Ala Pro Asp Thr Arg Pro Ala Pro Gly Ser Thr Ala

            260                 265                 270

Pro Pro Ala His Gly Val Thr Ser Ala Pro Asp Thr Arg Pro Ala Pro

        275                 280                 285

Gly Ser Thr Ala Pro Pro Ala His Gly Val Thr Ser Ala Pro Asp Thr

    290                 295                 300

Arg Pro Ala Pro Gly Ser Thr Ala Pro Pro Ala His Gly Val Thr Ser

305                 310                 315                 320

Ala Pro Asp Thr Arg Pro Ala Pro Gly Ser Thr Ala Pro Pro Ala His

                325                 330                 335

Gly Val Thr Ser Ala Pro Asp Thr Arg Pro Ala Pro Gly Ser Thr Ala

            340                 345                 350

Pro Pro Ala His Gly Val Thr Ser Ala Pro Asp Thr Arg Pro Ala Pro

        355                 360                 365

Gly Ser Thr Ala Pro Pro Ala His Gly Val Thr Ser Ala Pro Asp Thr

    370                 375                 380

Arg Pro Ala Pro Gly Ser Thr Ala Pro Pro Ala His Gly Val Thr Ser

385                 390                 395                 400

Ala Pro Asp Thr Arg Pro Ala Pro Gly Ser Thr Ala Pro Pro Ala His

                405                 410                 415

Gly Val Thr Ser Ala Pro Asp Thr Arg Pro Ala Pro Gly Ser Thr Ala

            420                 425                 430

Pro Pro Ala His Gly Val Thr Ser Ala Pro Asp Thr Arg Pro Ala Pro
        435                 440                 445
Gly Ser Thr Ala Pro Pro Ala His Gly Val Thr Ser Ala Pro Asp Thr
    450                 455                 460
Arg Pro Ala Pro Gly Ser Thr Ala Pro Pro Ala His Gly Val Thr Ser
465                 470                 475                 480
Ala Pro Asp Thr Arg Pro Ala Pro Gly Ser Thr Ala Pro Pro Ala His
                485                 490                 495
Gly Val Thr Ser Ala Pro Asp Thr Arg Pro Ala Pro Gly Ser Thr Ala
            500                 505                 510
Pro Pro Ala His Gly Val Thr Ser Ala Pro Asp Thr Arg Pro Ala Pro
        515                 520                 525
Gly Ser Thr Ala Pro Pro Ala His Gly Val Thr Ser Ala Pro Asp Thr
    530                 535                 540
Arg Pro Ala Pro Gly Ser Thr Ala Pro Pro Ala His Gly Val Thr Ser
545                 550                 555                 560
Ala Pro Asp Thr Arg Pro Ala Pro Gly Ser Thr Ala Pro Pro Ala His
                565                 570                 575
Gly Val Thr Ser Ala Pro Asp Thr Arg Pro Ala Pro Gly Ser Thr Ala
            580                 585                 590
Pro Pro Ala His Gly Val Thr Ser Ala Pro Asp Thr Arg Pro Ala Pro
        595                 600                 605
Gly Ser Thr Ala Pro Pro Ala His Gly Val Thr Ser Ala Pro Asp Thr
    610                 615                 620
Arg Pro Ala Pro Gly Ser Thr Ala Pro Pro Ala His Gly Val Thr Ser
625                 630                 635                 640
Ala Pro Asp Thr Arg Pro Ala Pro Gly Ser Thr Ala Pro Pro Ala His
                645                 650                 655
Gly Val Thr Ser Ala Pro Asp Thr Arg Pro Ala Pro Gly Ser Thr Ala

            660                 665                 670

Pro Pro Ala His Gly Val Thr Ser Ala Pro Asp Thr Arg Pro Ala Pro

        675                 680                 685

Gly Ser Thr Ala Pro Pro Ala His Gly Val Thr Ser Ala Pro Asp Thr

    690                 695                 700

Arg Pro Ala Pro Gly Ser Thr Ala Pro Pro Ala His Gly Val Thr Ser

705                 710                 715                 720

Ala Pro Asp Thr Arg Pro Ala Pro Gly Ser Thr Ala Pro Pro Ala His

                725                 730                 735

Gly Val Thr Ser Ala Pro Asp Thr Arg Pro Ala Pro Gly Ser Thr Ala

            740                 745                 750

Pro Pro Ala His Gly Val Thr Ser Ala Pro Asp Thr Arg Pro Ala Pro

        755                 760                 765

Gly Ser Thr Ala Pro Pro Ala His Gly Val Thr Ser Ala Pro Asp Thr

    770                 775                 780

Arg Pro Ala Pro Gly Ser Thr Ala Pro Pro Ala His Gly Val Thr Ser

785                 790                 795                 800

Ala Pro Asp Thr Arg Pro Ala Pro Gly Ser Thr Ala Pro Pro Ala His

                805                 810                 815

Gly Val Thr Ser Ala Pro Asp Thr Arg Pro Ala Pro Gly Ser Thr Ala

            820                 825                 830

Pro Pro Ala His Gly Val Thr Ser Ala Pro Asp Thr Arg Pro Ala Pro

        835                 840                 845

Gly Ser Thr Ala Pro Pro Ala His Gly Val Thr Ser Ala Pro Asp Thr

    850                 855                 860

Arg Pro Ala Pro Gly Ser Thr Ala Pro Pro Ala His Gly Val Thr Ser

865                 870                 875                 880

Ala Pro Asp Thr Arg Pro Ala Pro Gly Ser Thr Ala Pro Pro Ala His

                885                 890                 895

Gly Val Thr Ser Ala Pro Asp Thr Arg Pro Ala Pro Gly Ser Thr Ala
            900                 905                 910
Pro Pro Ala His Gly Val Thr Ser Ala Pro Asp Thr Arg Pro Ala Pro
        915                 920                 925
Gly Ser Thr Ala Pro Pro Ala His Gly Val Thr Ser Ala Pro Asp Asn
    930                 935                 940
Arg Pro Ala Leu Gly Ser Thr Ala Pro Pro Val His Asn Val Thr Ser
945                 950                 955                 960
Ala Ser Gly Ser Ala Ser Gly Ser Ala Ser Thr Leu Val His Asn Gly
                965                 970                 975
Thr Ser Ala Arg Ala Thr Thr Thr Pro Ala Ser Lys Ser Thr Pro Phe
            980                 985                 990
Ser Ile Pro Ser His His Ser Asp Thr Pro Thr Thr Leu Ala Ser His
        995                 1000                1005
Ser Thr Lys Thr Asp Ala Ser Ser Thr His His Ser Ser Val Pro Pro
    1010                1015                1020
Leu Thr Ser Ser Asn His Ser Thr Ser Pro Gln Leu Ser Thr Gly Val
1025                1030                1035                1040
Ser Phe Phe Phe Leu Ser Phe His Ile Ser Asn Leu Gln Phe Asn Ser
                1045                1050                1055
Ser Leu Glu Asp Pro Ser Thr Asp Tyr Tyr Gln Glu Leu Gln Arg Asp
            1060                1065                1070
Ile Ser Glu Met Phe Leu Gln Ile Tyr Lys Gln Gly Gly Phe Leu Gly
        1075                1080                1085
Leu Ser Asn Ile Lys Phe Arg Pro Gly Ser Val Val Val Gln Leu Thr
    1090                1095                1100
Leu Ala Phe Arg Glu Gly Thr Ile Asn Val His Asp Val Glu Thr Gln
1105                1110                1115                1120
Phe Asn Gln Tyr Lys Thr Glu Ala Ala Ser Arg Tyr Asn Leu Thr Ile

1125                    1130                    1135

Ser Asp Val Ser Val Ser Asp Val Pro Phe Pro Phe Ser Ala Gln Ser

        1140                    1145                    1150

Gly Ala Gly Val Pro Gly Trp Gly Ile Ala Leu Leu Val Leu Val Cys

    1155                    1160                    1165

Val Leu Val Ala Leu Ala Ile Val Tyr Leu Ile Ala Leu Ala Val Cys

1170                    1175                    1180

Gln Cys Arg Arg Lys Asn Tyr Gly Gln Leu Asp Ile Phe Pro Ala Arg

1185                    1190                    1195                    1200

Asp Thr Tyr His Pro Met Ser Glu Tyr Pro Thr Tyr His Thr His Gly

                1205                    1210                    1215

Arg Tyr Val Pro Pro Ser Ser Thr Asp Arg Ser Pro Tyr Glu Lys Val

            1220                    1225                    1230

Ser Ala Gly Asn Gly Gly Ser Ser Leu Ser Tyr Thr Asn Pro Ala Val

        1235                    1240                    1245

Ala Ala Ala Ser Ala Asn Leu

    1250                    1255

<210> 121

<211> 5179

<212> PRT

<213> Homo sapiens

<400> 121

Met Gly Leu Pro Leu Ala Arg Leu Ala Ala Val Cys Leu Ala Leu Ser
1               5                   10                  15

Leu Ala Gly Gly Ser Glu Leu Gln Thr Glu Gly Arg Thr Arg Tyr His

            20                  25                  30

Gly Arg Asn Val Cys Ser Thr Trp Gly Asn Phe His Tyr Lys Thr Phe
        35                  40                  45
Asp Gly Asp Val Phe Arg Phe Pro Gly Leu Cys Asp Tyr Asn Phe Ala
    50                  55                  60
Ser Asp Cys Arg Gly Ser Tyr Lys Glu Phe Ala Val His Leu Lys Arg
65                  70                  75                  80
Gly Pro Gly Gln Ala Glu Ala Pro Ala Gly Val Glu Ser Ile Leu Leu
                85                  90                  95
Thr Ile Lys Asp Asp Thr Ile Tyr Leu Thr Arg His Leu Ala Val Leu
            100                 105                 110
Asn Gly Ala Val Val Ser Thr Pro His Tyr Ser Pro Gly Leu Leu Ile
        115                 120                 125
Glu Lys Ser Asp Ala Tyr Thr Lys Val Tyr Ser Arg Ala Gly Leu Thr
    130                 135                 140
Leu Met Trp Asn Arg Glu Asp Ala Leu Met Leu Glu Leu Asp Thr Lys
145                 150                 155                 160
Phe Arg Asn His Thr Cys Gly Leu Cys Gly Asp Tyr Asn Gly Leu Gln
                165                 170                 175
Ser Tyr Ser Glu Phe Leu Ser Asp Gly Val Leu Phe Ser Pro Leu Glu
            180                 185                 190
Phe Gly Asn Met Gln Lys Ile Asn Gln Pro Asp Val Val Cys Glu Asp
        195                 200                 205
Pro Glu Glu Glu Val Ala Pro Ala Ser Cys Ser Glu His Arg Ala Glu
    210                 215                 220
Cys Glu Arg Leu Leu Thr Ala Glu Ala Phe Ala Asp Cys Gln Asp Leu
225                 230                 235                 240
Val Pro Leu Glu Pro Tyr Leu Arg Ala Cys Gln Gln Asp Arg Cys Arg
                245                 250                 255
Cys Pro Gly Gly Asp Thr Cys Val Cys Ser Thr Val Ala Glu Phe Ser

            260                 265                 270
Arg Gln Cys Ser His Ala Gly Gly Arg Pro Gly Asn Trp Arg Thr Ala
        275                 280                 285
Thr Leu Cys Pro Lys Thr Cys Pro Gly Asn Leu Val Tyr Leu Glu Ser
    290                 295                 300
Gly Ser Pro Cys Met Asp Thr Cys Ser His Leu Glu Val Ser Ser Leu
305                 310                 315                 320
Cys Glu Glu His Arg Met Asp Gly Cys Phe Cys Pro Glu Gly Thr Val
                325                 330                 335
Tyr Asp Asp Ile Gly Asp Ser Gly Cys Val Pro Val Ser Gln Cys His
            340                 345                 350
Cys Arg Leu His Gly His Leu Tyr Thr Pro Gly Gln Glu Ile Thr Asn
        355                 360                 365
Asp Cys Glu Gln Cys Val Cys Asn Ala Gly Arg Trp Val Cys Lys Asp
    370                 375                 380
Leu Pro Cys Pro Gly Thr Cys Ala Leu Glu Gly Gly Ser His Ile Thr
385                 390                 395                 400
Thr Phe Asp Gly Lys Thr Tyr Thr Phe His Gly Asp Cys Tyr Tyr Val
                405                 410                 415
Leu Ala Lys Gly Asp His Asn Asp Ser Tyr Ala Leu Leu Gly Glu Leu
            420                 425                 430
Ala Pro Cys Gly Ser Thr Asp Lys Gln Thr Cys Leu Lys Thr Val Val
        435                 440                 445
Leu Leu Ala Asp Lys Lys Lys Asn Ala Val Val Phe Lys Ser Asp Gly
    450                 455                 460
Ser Val Leu Leu Asn Gln Leu Gln Val Asn Leu Pro His Val Thr Ala
465                 470                 475                 480
Ser Phe Ser Val Phe Arg Pro Ser Ser Tyr His Ile Met Val Ser Met
                485                 490                 495

Ala Ile Gly Val Arg Leu Gln Val Gln Leu Ala Pro Val Met Gln Leu
            500                 505                 510
Phe Val Thr Leu Asp Gln Ala Ser Gln Gly Gln Val Gln Gly Leu Cys
        515                 520                 525
Gly Asn Phe Asn Gly Leu Glu Gly Asp Asp Phe Lys Thr Ala Ser Gly
    530                 535                 540
Leu Val Glu Ala Thr Gly Ala Gly Phe Ala Asn Thr Trp Lys Ala Gln
545                 550                 555                 560
Ser Thr Cys His Asp Lys Leu Asp Trp Leu Asp Asp Pro Cys Ser Leu
                565                 570                 575
Asn Ile Glu Ser Ala Asn Tyr Ala Glu His Trp Cys Ser Leu Leu Lys
            580                 585                 590
Lys Thr Glu Thr Pro Phe Gly Arg Cys His Ser Ala Val Asp Pro Ala
        595                 600                 605
Glu Tyr Tyr Lys Arg Cys Lys Tyr Asp Thr Cys Asn Cys Gln Asn Asn
    610                 615                 620
Glu Asp Cys Leu Cys Ala Ala Leu Ser Ser Tyr Ala Arg Ala Cys Thr
625                 630                 635                 640
Ala Lys Gly Val Met Leu Trp Gly Trp Arg Glu His Val Cys Asn Lys
                645                 650                 655
Asp Val Gly Ser Cys Pro Asn Ser Gln Val Phe Leu Tyr Asn Leu Thr
            660                 665                 670
Thr Cys Gln Gln Thr Cys Arg Ser Leu Ser Glu Ala Asp Ser His Cys
        675                 680                 685
Leu Glu Gly Phe Ala Pro Val Asp Gly Cys Gly Cys Pro Asp His Thr
    690                 695                 700
Phe Leu Asp Glu Lys Gly Arg Cys Val Pro Leu Ala Lys Cys Ser Cys
705                 710                 715                 720
Tyr His Arg Gly Leu Tyr Leu Glu Ala Gly Asp Val Val Val Arg Gln

725                 730                 735

Glu Glu Arg Cys Val Cys Arg Asp Gly Arg Leu His Cys Arg Gln Ile

            740                 745                 750

Arg Leu Ile Gly Gln Ser Cys Thr Ala Pro Lys Ile His Met Asp Cys

        755                 760                 765

Ser Asn Leu Thr Ala Leu Ala Thr Ser Lys Pro Arg Ala Leu Ser Cys

    770                 775                 780

Gln Thr Leu Ala Ala Gly Tyr Tyr His Thr Glu Cys Val Ser Gly Cys

785                 790                 795                 800

Val Cys Pro Asp Gly Leu Met Asp Asp Gly Arg Gly Gly Cys Val Val

                805                 810                 815

Glu Lys Glu Cys Pro Cys Val His Asn Asn Asp Leu Tyr Ser Ser Gly

            820                 825                 830

Ala Lys Ile Lys Val Asp Cys Asn Thr Cys Thr Cys Lys Arg Gly Arg

        835                 840                 845

Trp Val Cys Thr Gln Ala Val Cys His Gly Thr Cys Ser Ile Tyr Gly

    850                 855                 860

Ser Gly His Tyr Ile Thr Phe Asp Gly Lys Tyr Tyr Asp Phe Asp Gly

865                 870                 875                 880

His Cys Ser Tyr Val Ala Val Gln Asp Tyr Cys Gly Gln Asn Ser Ser

                885                 890                 895

Leu Gly Ser Phe Ser Ile Ile Thr Glu Asn Val Pro Cys Gly Thr Thr

            900                 905                 910

Gly Val Thr Cys Ser Lys Ala Ile Lys Ile Phe Met Gly Arg Thr Glu

        915                 920                 925

Leu Lys Leu Glu Asp Lys His Arg Val Val Ile Gln Arg Asp Glu Gly

    930                 935                 940

His His Val Ala Tyr Thr Thr Arg Glu Val Gly Gln Tyr Leu Val Val

945                 950                 955                 960

Glu Ser Ser Thr Gly Ile Ile Val Ile Trp Asp Lys Arg Thr Thr Val
                965                 970                 975
Phe Ile Lys Leu Ala Pro Ser Tyr Lys Gly Thr Val Cys Gly Leu Cys
            980                 985                 990
Gly Asn Phe Asp His Arg Ser Asn Asn Asp Phe Thr Thr Arg Asp His
        995                 1000                1005
Met Val Val Ser Ser Glu Leu Asp Phe Gly Asn Ser Trp Lys Glu Ala
    1010                1015                1020
Pro Thr Cys Pro Asp Val Ser Thr Asn Pro Glu Pro Cys Ser Leu Asn
1025                1030                1035                1040
Pro His Arg Arg Ser Trp Ala Glu Lys Gln Cys Ser Ile Leu Lys Ser
                1045                1050                1055
Ser Val Phe Ser Ile Cys His Ser Lys Val Asp Pro Lys Pro Phe Tyr
            1060                1065                1070
Glu Ala Cys Val His Asp Ser Cys Ser Cys Asp Thr Gly Gly Asp Cys
        1075                1080                1085
Glu Cys Phe Cys Ser Ala Val Ala Ser Tyr Ala Gln Glu Cys Thr Lys
    1090                1095                1100
Glu Gly Ala Cys Val Phe Trp Arg Thr Pro Asp Leu Cys Pro Ile Phe
1105                1110                1115                1120
Cys Asp Tyr Tyr Asn Pro Pro His Glu Cys Glu Trp His Tyr Glu Pro
                1125                1130                1135
Cys Gly Asn Arg Ser Phe Glu Thr Cys Arg Thr Ile Asn Gly Ile His
            1140                1145                1150
Ser Asn Ile Ser Val Ser Tyr Leu Glu Gly Cys Tyr Pro Arg Cys Pro
        1155                1160                1165
Lys Asp Arg Pro Ile Tyr Glu Glu Asp Leu Lys Lys Cys Val Thr Ala
    1170                1175                1180
Asp Lys Cys Gly Cys Tyr Val Glu Asp Thr His Tyr Pro Pro Gly Ala

1185                1190                1195                1200

Ser Val Pro Thr Glu Glu Thr Cys Lys Ser Cys Val Cys Thr Asn Ser

                1205                1210                1215

Ser Gln Val Val Cys Arg Pro Glu Glu Gly Lys Ile Leu Asn Gln Thr

            1220                1225                1230

Gln Asp Gly Ala Phe Cys Tyr Trp Glu Ile Cys Gly Pro Asn Gly Thr

        1235                1240                1245

Val Glu Lys His Phe Asn Ile Cys Ser Ile Thr Thr Arg Pro Ser Thr

    1250                1255                1260

Leu Thr Thr Phe Thr Thr Ile Thr Leu Pro Thr Thr Pro Thr Ser Phe

1265                1270                1275                1280

Thr Thr Thr Thr Thr Thr Thr Thr Pro Thr Ser Ser Thr Val Leu Ser

                1285                1290                1295

Thr Thr Pro Lys Leu Cys Cys Leu Trp Ser Asp Trp Ile Asn Glu Asp

            1300                1305                1310

His Pro Ser Ser Gly Ser Asp Asp Gly Asp Arg Glu Pro Phe Asp Gly

        1315                1320                1325

Val Cys Gly Ala Pro Glu Asp Ile Glu Cys Arg Ser Val Lys Asp Pro

    1330                1335                1340

His Leu Ser Leu Glu Gln His Gly Gln Lys Val Gln Cys Asp Val Ser

1345                1350                1355                1360

Val Gly Phe Ile Cys Lys Asn Glu Asp Gln Phe Gly Asn Gly Pro Phe

                1365                1370                1375

Gly Leu Cys Tyr Asp Tyr Lys Ile Arg Val Asn Cys Cys Trp Pro Met

            1380                1385                1390

Asp Lys Cys Ile Thr Thr Pro Ser Pro Pro Thr Thr Thr Pro Ser Pro

        1395                1400                1405

Pro Pro Thr Thr Thr Thr Thr Leu Pro Pro Thr Thr Thr Pro Ser Pro

    1410                1415                1420

Pro Thr Thr Thr Thr Thr Thr Pro Pro Pro Thr Thr Thr Pro Ser Pro
1425                1430                1435                1440
Pro Ile Thr Thr Thr Thr Thr Pro Leu Pro Thr Thr Thr Pro Ser Pro
                1445                1450                1455
Pro Ile Ser Thr Thr Thr Thr Pro Pro Pro Thr Thr Thr Pro Ser Pro
            1460                1465                1470
Pro Thr Thr Thr Pro Ser Pro Pro Thr Thr Thr Pro Ser Pro Pro Thr
        1475                1480                1485
Thr Thr Thr Thr Thr Pro Pro Pro Thr Thr Thr Pro Ser Pro Pro Met
    1490                1495                1500
Thr Thr Pro Ile Thr Pro Pro Ala Ser Thr Thr Thr Leu Pro Pro Thr
1505                1510                1515                1520
Thr Thr Pro Ser Pro Pro Thr Thr Thr Thr Thr Thr Pro Pro Pro Thr
                1525                1530                1535
Thr Thr Pro Ser Pro Pro Thr Thr Thr Pro Ile Thr Pro Pro Thr Ser
            1540                1545                1550
Thr Thr Thr Leu Pro Pro Thr Thr Thr Pro Ser Pro Pro Pro Thr Thr
        1555                1560                1565
Thr Thr Thr Pro Pro Pro Thr Thr Thr Pro Ser Pro Pro Thr Thr Thr
    1570                1575                1580
Thr Pro Ser Pro Pro Thr Ile Thr Thr Thr Thr Pro Pro Pro Thr Thr
1585                1590                1595                1600
Thr Pro Ser Pro Pro Thr Thr Thr Thr Thr Thr Pro Pro Pro Thr Thr
                1605                1610                1615
Thr Pro Ser Pro Pro Thr Thr Thr Pro Ile Thr Pro Pro Thr Ser Thr
            1620                1625                1630
Thr Thr Leu Pro Pro Thr Thr Thr Pro Ser Pro Pro Pro Thr Thr Thr
        1635                1640                1645
Thr Thr Pro Pro Pro Thr Thr Thr Pro Ser Pro Pro Thr Thr Thr Thr

1650                1655                1660

Pro Ser Pro Pro Ile Thr Thr Thr Thr Thr Pro Pro Pro Thr Thr Thr

1665                1670                1675                1680

Pro Ser Ser Pro Ile Thr Thr Thr Pro Ser Pro Pro Thr Thr Thr Met

                1685                1690                1695

Thr Thr Pro Ser Pro Thr Thr Thr Pro Ser Ser Pro Ile Thr Thr Thr

            1700                1705                1710

Thr Thr Pro Ser Ser Thr Thr Thr Pro Ser Pro Pro Pro Thr Thr Met

        1715                1720                1725

Thr Thr Pro Ser Pro Thr Thr Thr Pro Ser Pro Pro Thr Thr Thr Met

    1730                1735                1740

Thr Thr Leu Pro Pro Thr Thr Thr Ser Ser Pro Leu Thr Thr Thr Pro

1745                1750                1755                1760

Leu Pro Pro Ser Ile Thr Pro Pro Thr Phe Ser Pro Phe Ser Thr Thr

                1765                1770                1775

Thr Pro Thr Thr Pro Cys Val Pro Leu Cys Asn Trp Thr Gly Trp Leu

            1780                1785                1790

Asp Ser Gly Lys Pro Asn Phe His Lys Pro Gly Gly Asp Thr Glu Leu

        1795                1800                1805

Ile Gly Asp Val Cys Gly Pro Gly Trp Ala Ala Asn Ile Ser Cys Arg

    1810                1815                1820

Ala Thr Met Tyr Pro Asp Val Pro Ile Gly Gln Leu Gly Gln Thr Val

1825                1830                1835                1840

Val Cys Asp Val Ser Val Gly Leu Ile Cys Lys Asn Glu Asp Gln Lys

                1845                1850                1855

Pro Gly Gly Val Ile Pro Met Ala Phe Cys Leu Asn Tyr Glu Ile Asn

            1860                1865                1870

Val Gln Cys Cys Glu Cys Val Thr Gln Pro Thr Thr Met Thr Thr Thr

        1875                1880                1885

Thr Thr Glu Asn Pro Thr Pro Pro Thr Thr Thr Pro Ile Thr Thr Thr
    1890                1895                1900
Thr Thr Val Thr Pro Thr Pro Thr Pro Thr Gly Thr Gln Thr Pro Thr
1905                1910                1915                1920
Thr Thr Pro Ile Thr Thr Thr Thr Thr Val Thr Pro Thr Pro Thr Pro
                1925                1930                1935
Thr Gly Thr Gln Thr Pro Thr Thr Thr Pro Ile Thr Thr Thr Thr Thr
            1940                1945                1950
Val Thr Pro Thr Pro Thr Pro Thr Gly Thr Gln Thr Pro Thr Thr Thr
        1955                1960                1965
Pro Ile Thr Thr Thr Thr Thr Val Thr Pro Thr Pro Thr Pro Thr Gly
    1970                1975                1980
Thr Gln Thr Pro Thr Thr Thr Pro Ile Thr Thr Thr Thr Thr Val Thr
1985                1990                1995                2000
Pro Thr Pro Thr Pro Thr Gly Thr Gln Thr Pro Thr Thr Thr Pro Ile
                2005                2010                2015
Thr Thr Thr Thr Thr Val Thr Pro Thr Pro Thr Pro Thr Gly Thr Gln
            2020                2025                2030
Thr Pro Thr Thr Thr Pro Ile Thr Thr Thr Thr Thr Val Thr Pro Thr
        2035                2040                2045
Pro Thr Pro Thr Gly Thr Gln Thr Pro Thr Thr Thr Pro Ile Thr Thr
    2050                2055                2060
Thr Thr Thr Val Thr Pro Thr Pro Thr Pro Thr Gly Thr Gln Thr Pro
2065                2070                2075                2080
Thr Thr Thr Pro Ile Thr Thr Thr Thr Thr Val Thr Pro Thr Pro Thr
                2085                2090                2095
Pro Thr Gly Thr Gln Thr Pro Thr Thr Thr Pro Ile Thr Thr Thr Thr
            2100                2105                2110
Thr Val Thr Pro Thr Pro Thr Pro Thr Gly Thr Gln Thr Pro Thr Thr

2115                2120                2125

Thr Pro Ile Thr Thr Thr Thr Thr Val Thr Pro Thr Pro Thr Pro Thr

    2130                2135                2140

Gly Thr Gln Thr Pro Thr Thr Thr Pro Ile Thr Thr Thr Thr Thr Val

2145                2150                2155                2160

Thr Pro Thr Pro Thr Pro Thr Gly Thr Gln Thr Pro Thr Thr Thr Pro

                2165                2170                2175

Ile Thr Thr Thr Thr Thr Val Thr Pro Thr Pro Thr Pro Thr Gly Thr

            2180                2185                2190

Gln Thr Pro Thr Thr Thr Pro Ile Thr Thr Thr Thr Thr Val Thr Pro

        2195                2200                2205

Thr Pro Thr Pro Thr Gly Thr Gln Thr Pro Thr Thr Thr Pro Ile Thr

    2210                2215                2220

Thr Thr Thr Thr Val Thr Pro Thr Pro Thr Pro Thr Gly Thr Gln Thr

2225                2230                2235                2240

Pro Thr Thr Thr Pro Ile Thr Thr Thr Thr Thr Val Thr Pro Thr Pro

                2245                2250                2255

Thr Pro Thr Gly Thr Gln Thr Pro Thr Thr Thr Pro Ile Thr Thr Thr

            2260                2265                2270

Thr Thr Val Thr Pro Thr Pro Thr Pro Thr Gly Thr Gln Thr Pro Thr

        2275                2280                2285

Thr Thr Pro Ile Thr Thr Thr Thr Thr Val Thr Pro Thr Pro Thr Pro

    2290                2295                2300

Thr Gly Thr Gln Thr Pro Thr Thr Thr Pro Ile Thr Thr Thr Thr Thr

2305                2310                2315                2320

Val Thr Pro Thr Pro Thr Pro Thr Gly Thr Gln Thr Pro Thr Thr Thr

                2325                2330                2335

Pro Ile Thr Thr Thr Thr Thr Val Thr Pro Thr Pro Thr Pro Thr Gly

            2340                2345                2350

Thr Gln Thr Pro Thr Thr Thr Pro Ile Thr Thr Thr Thr Thr Val Thr
        2355                2360                2365
Pro Thr Pro Thr Pro Thr Gly Thr Gln Thr Pro Thr Thr Thr Pro Ile
    2370                2375                2380
Thr Thr Thr Thr Thr Val Thr Pro Thr Pro Thr Pro Thr Gly Thr Gln
2385                2390                2395                2400
Thr Pro Thr Thr Thr Pro Ile Thr Thr Thr Thr Thr Val Thr Pro Thr
                2405                2410                2415
Pro Thr Pro Thr Gly Thr Gln Thr Pro Thr Thr Thr Pro Ile Thr Thr
            2420                2425                2430
Thr Thr Thr Val Thr Pro Thr Pro Thr Pro Thr Gly Thr Gln Thr Pro
        2435                2440                2445
Thr Thr Thr Pro Ile Thr Thr Thr Thr Thr Val Thr Pro Thr Pro Thr
    2450                2455                2460
Pro Thr Gly Thr Gln Thr Pro Thr Thr Thr Pro Ile Thr Thr Thr Thr
2465                2470                2475                2480
Thr Val Thr Pro Thr Pro Thr Pro Thr Gly Thr Gln Thr Pro Thr Thr
                2485                2490                2495
Thr Pro Ile Thr Thr Thr Thr Thr Val Thr Pro Thr Pro Thr Pro Thr
            2500                2505                2510
Gly Thr Gln Thr Pro Thr Thr Thr Pro Ile Thr Thr Thr Thr Thr Val
        2515                2520                2525
Thr Pro Thr Pro Thr Pro Thr Gly Thr Gln Thr Pro Thr Thr Thr Pro
    2530                2535                2540
Ile Thr Thr Thr Thr Thr Val Thr Pro Thr Pro Thr Pro Thr Gly Thr
2545                2550                2555                2560
Gln Thr Pro Thr Thr Thr Pro Ile Thr Thr Thr Thr Thr Val Thr Pro
                2565                2570                2575
Thr Pro Thr Pro Thr Gly Thr Gln Thr Pro Thr Thr Thr Pro Ile Thr

            2580                2585                2590
Thr Thr Thr Thr Val Thr Pro Thr Pro Thr Pro Thr Gly Thr Gln Thr
        2595                2600                2605
Pro Thr Thr Thr Pro Ile Thr Thr Thr Thr Thr Val Thr Pro Thr Pro
    2610                2615                2620
Thr Pro Thr Gly Thr Gln Thr Pro Thr Thr Thr Pro Ile Thr Thr Thr
2625                2630                2635                2640
Thr Thr Val Thr Pro Thr Pro Thr Pro Thr Gly Thr Gln Thr Pro Thr
                2645                2650                2655
Thr Thr Pro Ile Thr Thr Thr Thr Thr Val Thr Pro Thr Pro Thr Pro
            2660                2665                2670
Thr Gly Thr Gln Thr Pro Thr Thr Thr Pro Ile Thr Thr Thr Thr Thr
        2675                2680                2685
Val Thr Pro Thr Pro Thr Pro Thr Gly Thr Gln Thr Pro Thr Thr Thr
    2690                2695                2700
Pro Ile Thr Thr Thr Thr Thr Val Thr Pro Thr Pro Thr Pro Thr Gly
2705                2710                2715                2720
Thr Gln Thr Pro Thr Thr Thr Pro Ile Thr Thr Thr Thr Thr Val Thr
                2725                2730                2735
Pro Thr Pro Thr Pro Thr Gly Thr Gln Thr Pro Thr Thr Thr Pro Ile
            2740                2745                2750
Thr Thr Thr Thr Thr Val Thr Pro Thr Pro Thr Pro Thr Gly Thr Gln
        2755                2760                2765
Thr Pro Thr Thr Thr Pro Ile Thr Thr Thr Thr Thr Val Thr Pro Thr
    2770                2775                2780
Pro Thr Pro Thr Gly Thr Gln Thr Pro Thr Thr Thr Pro Ile Thr Thr
2785                2790                2795                2800
Thr Thr Thr Val Thr Pro Thr Pro Thr Pro Thr Gly Thr Gln Thr Pro
                2805                2810                2815

Thr Thr Thr Pro Ile Thr Thr Thr Thr Thr Val Thr Pro Thr Pro Thr
            2820                2825                2830
Pro Thr Gly Thr Gln Thr Pro Thr Thr Thr Pro Ile Thr Thr Thr Thr
        2835                2840                2845
Thr Val Thr Pro Thr Pro Thr Pro Thr Gly Thr Gln Thr Pro Thr Thr
    2850                2855                2860
Thr Pro Ile Thr Thr Thr Thr Thr Val Thr Pro Thr Pro Thr Pro Thr
2865                2870                2875                2880
Gly Thr Gln Thr Pro Thr Thr Thr Pro Ile Thr Thr Thr Thr Thr Val
                2885                2890                2895
Thr Pro Thr Pro Thr Pro Thr Gly Thr Gln Thr Pro Thr Thr Thr Pro
            2900                2905                2910
Ile Thr Thr Thr Thr Thr Val Thr Pro Thr Pro Thr Pro Thr Gly Thr
        2915                2920                2925
Gln Thr Pro Thr Thr Thr Pro Ile Thr Thr Thr Thr Thr Val Thr Pro
    2930                2935                2940
Thr Pro Thr Pro Thr Gly Thr Gln Thr Pro Thr Thr Thr Pro Ile Thr
2945                2950                2955                2960
Thr Thr Thr Thr Val Thr Pro Thr Pro Thr Pro Thr Gly Thr Gln Thr
                2965                2970                2975
Pro Thr Thr Thr Pro Ile Thr Thr Thr Thr Thr Val Thr Pro Thr Pro
            2980                2985                2990
Thr Pro Thr Gly Thr Gln Thr Pro Thr Thr Thr Pro Ile Thr Thr Thr
        2995                3000                3005
Thr Thr Val Thr Pro Thr Pro Thr Pro Thr Gly Thr Gln Thr Pro Thr
    3010                3015                3020
Thr Thr Pro Ile Thr Thr Thr Thr Thr Val Thr Pro Thr Pro Thr Pro
3025                3030                3035                3040
Thr Gly Thr Gln Thr Pro Thr Thr Thr Pro Ile Thr Thr Thr Thr Thr

                3045                3050                3055
Val Thr Pro Thr Pro Thr Pro Thr Gly Thr Gln Thr Pro Thr Thr Thr
            3060                3065                3070
Pro Ile Thr Thr Thr Thr Thr Val Thr Pro Thr Pro Thr Pro Thr Gly
        3075                3080                3085
Thr Gln Thr Pro Thr Thr Thr Pro Ile Thr Thr Thr Thr Thr Val Thr
    3090                3095                3100
Pro Thr Pro Thr Pro Thr Gly Thr Gln Thr Pro Thr Thr Thr Pro Ile
3105                3110                3115                3120
Thr Thr Thr Thr Thr Val Thr Pro Thr Pro Thr Pro Thr Gly Thr Gln
                3125                3130                3135
Thr Pro Thr Thr Thr Pro Ile Thr Thr Thr Thr Thr Val Thr Pro Thr
            3140                3145                3150
Pro Thr Pro Thr Gly Thr Gln Thr Pro Thr Thr Thr Pro Ile Thr Thr
        3155                3160                3165
Thr Thr Thr Val Thr Pro Thr Pro Thr Pro Thr Gly Thr Gln Thr Pro
    3170                3175                3180
Thr Thr Thr Pro Ile Thr Thr Thr Thr Thr Val Thr Pro Thr Pro Thr
3185                3190                3195                3200
Pro Thr Gly Thr Gln Thr Pro Thr Thr Thr Pro Ile Thr Thr Thr Thr
                3205                3210                3215
Thr Val Thr Pro Thr Pro Thr Pro Thr Gly Thr Gln Thr Pro Thr Thr
            3220                3225                3230
Thr Pro Ile Thr Thr Thr Thr Thr Val Thr Pro Thr Pro Thr Pro Thr
        3235                3240                3245
Gly Thr Gln Thr Pro Thr Thr Thr Pro Ile Thr Thr Thr Thr Thr Val
    3250                3255                3260
Thr Pro Thr Pro Thr Pro Thr Gly Thr Gln Thr Pro Thr Thr Thr Pro
3265                3270                3275                3280

Ile Thr Thr Thr Thr Thr Val Thr Pro Thr Pro Thr Pro Thr Gly Thr
                3285                3290                3295
Gln Thr Pro Thr Thr Thr Pro Ile Thr Thr Thr Thr Thr Val Thr Pro
            3300                3305                3310
Thr Pro Thr Pro Thr Gly Thr Gln Thr Pro Thr Thr Thr Pro Ile Thr
        3315                3320                3325
Thr Thr Thr Thr Val Thr Pro Thr Pro Thr Pro Thr Gly Thr Gln Thr
    3330                3335                3340
Pro Thr Thr Thr Pro Ile Thr Thr Thr Thr Thr Val Thr Pro Thr Pro
3345                3350                3355                3360
Thr Pro Thr Gly Thr Gln Thr Pro Thr Thr Thr Pro Ile Thr Thr Thr
                3365                3370                3375
Thr Thr Val Thr Pro Thr Pro Thr Pro Thr Gly Thr Gln Thr Pro Thr
            3380                3385                3390
Thr Thr Pro Ile Thr Thr Thr Thr Thr Val Thr Pro Thr Pro Thr Pro
        3395                3400                3405
Thr Gly Thr Gln Thr Pro Thr Thr Thr Pro Ile Thr Thr Thr Thr Thr
    3410                3415                3420
Val Thr Pro Thr Pro Thr Pro Thr Gly Thr Gln Thr Pro Thr Thr Thr
3425                3430                3435                3440
Pro Ile Thr Thr Thr Thr Thr Val Thr Pro Thr Pro Thr Pro Thr Gly
                3445                3450                3455
Thr Gln Thr Pro Thr Thr Thr Pro Ile Thr Thr Thr Thr Thr Val Thr
            3460                3465                3470
Pro Thr Pro Thr Pro Thr Gly Thr Gln Thr Pro Thr Thr Thr Pro Ile
        3475                3480                3485
Thr Thr Thr Thr Thr Val Thr Pro Thr Pro Thr Pro Thr Gly Thr Gln
    3490                3495                3500
Thr Pro Thr Thr Thr Pro Ile Thr Thr Thr Thr Thr Val Thr Pro Thr

3505                3510                3515                3520

Pro Thr Pro Thr Gly Thr Gln Thr Pro Thr Thr Thr Pro Ile Thr Thr

                3525                3530                3535

Thr Thr Thr Val Thr Pro Thr Pro Thr Pro Thr Gly Thr Gln Thr Pro

            3540                3545                3550

Thr Thr Thr Pro Ile Thr Thr Thr Thr Thr Val Thr Pro Thr Pro Thr

        3555                3560                3565

Pro Thr Gly Thr Gln Thr Pro Thr Thr Thr Pro Ile Thr Thr Thr Thr

    3570                3575                3580

Thr Val Thr Pro Thr Pro Thr Pro Thr Gly Thr Gln Thr Pro Thr Thr

3585                3590                3595                3600

Thr Pro Ile Thr Thr Thr Thr Thr Val Thr Pro Thr Pro Thr Pro Thr

                3605                3610                3615

Gly Thr Gln Thr Pro Thr Thr Thr Pro Ile Thr Thr Thr Thr Thr Val

            3620                3625                3630

Thr Pro Thr Pro Thr Pro Thr Gly Thr Gln Thr Pro Thr Thr Thr Pro

        3635                3640                3645

Ile Thr Thr Thr Thr Thr Val Thr Pro Thr Pro Thr Pro Thr Gly Thr

    3650                3655                3660

Gln Thr Pro Thr Thr Thr Pro Ile Thr Thr Thr Thr Thr Val Thr Pro

3665                3670                3675                3680

Thr Pro Thr Pro Thr Gly Thr Gln Thr Pro Thr Thr Thr Pro Ile Thr

                3685                3690                3695

Thr Thr Thr Thr Val Thr Pro Thr Pro Thr Pro Thr Gly Thr Gln Thr

            3700                3705                3710

Pro Thr Thr Thr Pro Ile Thr Thr Thr Thr Thr Val Thr Pro Thr Pro

        3715                3720                3725

Thr Pro Thr Gly Thr Gln Thr Pro Thr Thr Thr Pro Ile Thr Thr Thr

    3730                3735                3740

Thr Thr Val Thr Pro Thr Pro Thr Pro Thr Gly Thr Gln Thr Pro Thr
3745                3750                3755                3760
Thr Thr Pro Ile Thr Thr Thr Thr Thr Val Thr Pro Thr Pro Thr Pro
                3765                3770                3775
Thr Gly Thr Gln Thr Pro Thr Thr Thr Pro Ile Thr Thr Thr Thr Thr
            3780                3785                3790
Val Thr Pro Thr Pro Thr Pro Thr Gly Thr Gln Thr Pro Thr Thr Thr
        3795                3800                3805
Pro Ile Thr Thr Thr Thr Thr Val Thr Pro Thr Pro Thr Pro Thr Gly
    3810                3815                3820
Thr Gln Thr Pro Thr Thr Thr Pro Ile Thr Thr Thr Thr Thr Val Thr
3825                3830                3835                3840
Pro Thr Pro Thr Pro Thr Gly Thr Gln Thr Pro Thr Thr Thr Pro Ile
                3845                3850                3855
Thr Thr Thr Thr Thr Val Thr Pro Thr Pro Thr Pro Thr Gly Thr Gln
            3860                3865                3870
Thr Pro Thr Thr Thr Pro Ile Thr Thr Thr Thr Thr Val Thr Pro Thr
        3875                3880                3885
Pro Thr Pro Thr Gly Thr Gln Thr Pro Thr Thr Thr Pro Ile Thr Thr
    3890                3895                3900
Thr Thr Thr Val Thr Pro Thr Pro Thr Pro Thr Gly Thr Gln Thr Pro
3905                3910                3915                3920
Thr Thr Thr Pro Ile Thr Thr Thr Thr Thr Val Thr Pro Thr Pro Thr
                3925                3930                3935
Pro Thr Gly Thr Gln Thr Pro Thr Thr Thr Pro Ile Thr Thr Thr Thr
            3940                3945                3950
Thr Val Thr Pro Thr Pro Thr Pro Thr Gly Thr Gln Thr Pro Thr Thr
        3955                3960                3965
Thr Pro Ile Thr Thr Thr Thr Thr Val Thr Pro Thr Pro Thr Pro Thr

3970                3975                3980

Gly Thr Gln Thr Pro Thr Thr Thr Pro Ile Thr Thr Thr Thr Thr Val

3985                3990                3995                4000

Thr Pro Thr Pro Thr Pro Thr Gly Thr Gln Thr Pro Thr Thr Thr Pro

                4005                4010                4015

Ile Thr Thr Thr Thr Thr Val Thr Pro Thr Pro Thr Pro Thr Gly Thr

            4020                4025                4030

Gln Thr Pro Thr Thr Thr Pro Ile Thr Thr Thr Thr Thr Val Thr Pro

        4035                4040                4045

Thr Pro Thr Pro Thr Gly Thr Gln Thr Pro Thr Thr Thr Pro Ile Thr

    4050                4055                4060

Thr Thr Thr Thr Val Thr Pro Thr Pro Thr Pro Thr Gly Thr Gln Thr

4065                4070                4075                4080

Pro Thr Thr Thr Pro Ile Thr Thr Thr Thr Thr Val Thr Pro Thr Pro

                4085                4090                4095

Thr Pro Thr Gly Thr Gln Thr Pro Thr Thr Thr Pro Ile Thr Thr Thr

            4100                4105                4110

Thr Thr Val Thr Pro Thr Pro Thr Pro Thr Gly Thr Gln Thr Pro Thr

        4115                4120                4125

Thr Thr Pro Ile Thr Thr Thr Thr Thr Val Thr Pro Thr Pro Thr Pro

    4130                4135                4140

Thr Gly Thr Gln Thr Pro Thr Thr Thr Pro Ile Thr Thr Thr Thr Thr

4145                4150                4155                4160

Val Thr Pro Thr Pro Thr Pro Thr Gly Thr Gln Thr Pro Thr Thr Thr

                4165                4170                4175

Pro Ile Thr Thr Thr Thr Thr Val Thr Pro Thr Pro Thr Pro Thr Gly

            4180                4185                4190

Thr Gln Thr Gly Pro Pro Thr His Thr Ser Thr Ala Pro Ile Ala Glu

        4195                4200                4205

Leu Thr Thr Ser Asn Pro Pro Pro Glu Ser Ser Thr Pro Gln Thr Ser
    4210                4215                4220
Arg Ser Thr Ser Ser Pro Leu Thr Glu Ser Thr Thr Leu Leu Ser Thr
4225                4230                4235                4240
Leu Pro Pro Ala Ile Glu Met Thr Ser Thr Ala Pro Pro Ser Thr Pro
                4245                4250                4255
Thr Ala Pro Thr Thr Thr Ser Gly Gly His Thr Leu Ser Pro Pro Pro
            4260                4265                4270
Ser Thr Thr Thr Ser Pro Pro Gly Thr Pro Thr Arg Gly Thr Thr Thr
        4275                4280                4285
Gly Ser Ser Ser Ala Pro Thr Pro Ser Thr Val Gln Thr Thr Thr Thr
    4290                4295                4300
Ser Ala Trp Thr Pro Thr Pro Thr Pro Leu Ser Thr Pro Ser Ile Ile
4305                4310                4315                4320
Arg Thr Thr Gly Leu Arg Pro Tyr Pro Ser Ser Val Leu Ile Cys Cys
                4325                4330                4335
Val Leu Asn Asp Thr Tyr Tyr Ala Pro Gly Glu Glu Val Tyr Asn Gly
            4340                4345                4350
Thr Tyr Gly Asp Thr Cys Tyr Phe Val Asn Cys Ser Leu Ser Cys Thr
        4355                4360                4365
Leu Glu Phe Tyr Asn Trp Ser Cys Pro Ser Thr Pro Ser Pro Thr Pro
    4370                4375                4380
Thr Pro Ser Lys Ser Thr Pro Thr Pro Ser Lys Pro Ser Ser Thr Pro
4385                4390                4395                4400
Ser Lys Pro Thr Pro Gly Thr Lys Pro Pro Glu Cys Pro Asp Phe Asp
                4405                4410                4415
Pro Pro Arg Gln Glu Asn Glu Thr Trp Trp Leu Cys Asp Cys Phe Met
            4420                4425                4430
Ala Thr Cys Lys Tyr Asn Asn Thr Val Glu Ile Val Lys Val Glu Cys

4435                4440                4445

Glu Pro Pro Pro Met Pro Thr Cys Ser Asn Gly Leu Gln Pro Val Arg

    4450                4455                4460

Val Glu Asp Pro Asp Gly Cys Cys Trp His Trp Glu Cys Asp Cys Tyr

4465                4470                4475                4480

Cys Thr Gly Trp Gly Asp Pro His Tyr Val Thr Phe Asp Gly Leu Tyr

                4485                4490                4495

Tyr Ser Tyr Gln Gly Asn Cys Thr Tyr Val Leu Val Glu Glu Ile Ser

            4500                4505                4510

Pro Ser Val Asp Asn Phe Gly Val Tyr Ile Asp Asn Tyr His Cys Asp

        4515                4520                4525

Pro Asn Asp Lys Val Ser Cys Pro Arg Thr Leu Ile Val Arg His Glu

    4530                4535                4540

Thr Gln Glu Val Leu Ile Lys Thr Val His Met Met Pro Met Gln Val

4545                4550                4555                4560

Gln Val Gln Val Asn Arg Gln Ala Val Ala Leu Pro Tyr Lys Lys Tyr

                4565                4570                4575

Gly Leu Glu Val Tyr Gln Ser Gly Ile Asn Tyr Val Val Asp Ile Pro

            4580                4585                4590

Glu Leu Gly Val Leu Val Ser Tyr Asn Gly Leu Ser Phe Ser Val Arg

        4595                4600                4605

Leu Pro Tyr His Arg Phe Gly Asn Asn Thr Lys Gly Gln Cys Gly Thr

    4610                4615                4620

Cys Thr Asn Thr Thr Ser Asp Asp Cys Ile Leu Pro Ser Gly Glu Ile

4625                4630                4635                4640

Val Ser Asn Cys Glu Ala Ala Ala Asp Gln Trp Leu Val Asn Asp Pro

                4645                4650                4655

Ser Lys Pro His Cys Pro His Ser Ser Ser Thr Thr Lys Arg Pro Ala

            4660                4665                4670

Val Thr Val Pro Gly Gly Gly Lys Thr Thr Pro His Lys Asp Cys Thr
        4675                4680                4685
Pro Ser Pro Leu Cys Gln Leu Ile Lys Asp Ser Leu Phe Ala Gln Cys
    4690                4695                4700
His Ala Leu Val Pro Pro Gln His Tyr Tyr Asp Ala Cys Val Phe Asp
4705                4710                4715                4720
Ser Cys Phe Met Pro Gly Ser Ser Leu Glu Cys Ala Ser Leu Gln Ala
                4725                4730                4735
Tyr Ala Ala Leu Cys Ala Gln Gln Asn Ile Cys Leu Asp Trp Arg Asn
            4740                4745                4750
His Thr His Gly Ala Cys Leu Val Glu Cys Pro Ser His Arg Glu Tyr
        4755                4760                4765
Gln Ala Cys Gly Pro Ala Glu Glu Pro Thr Cys Lys Ser Ser Ser Ser
    4770                4775                4780
Gln Gln Asn Asn Thr Val Leu Val Glu Gly Cys Phe Cys Pro Glu Gly
4785                4790                4795                4800
Thr Met Asn Tyr Ala Pro Gly Phe Asp Val Cys Val Lys Thr Cys Gly
                4805                4810                4815
Cys Val Gly Pro Asp Asn Val Pro Arg Glu Phe Gly Glu His Phe Glu
            4820                4825                4830
Phe Asp Cys Lys Asn Cys Val Cys Leu Glu Gly Gly Ser Gly Ile Ile
        4835                4840                4845
Cys Gln Pro Lys Arg Cys Ser Gln Lys Pro Val Thr His Cys Val Glu
    4850                4855                4860
Asp Gly Thr Tyr Leu Ala Thr Glu Val Asn Pro Ala Asp Thr Cys Cys
4865                4870                4875                4880
Asn Ile Thr Val Cys Lys Cys Asn Thr Ser Leu Cys Lys Glu Lys Pro
                4885                4890                4895
Ser Val Cys Pro Leu Gly Phe Glu Val Lys Ser Lys Met Val Pro Gly

4900                4905                4910
Arg Cys Cys Pro Phe Tyr Trp Cys Glu Ser Lys Gly Val Cys Val His
        4915                4920                4925
Gly Asn Ala Glu Tyr Gln Pro Gly Ser Pro Val Tyr Ser Ser Lys Cys
    4930                4935                4940
Gln Asp Cys Val Cys Thr Asp Lys Val Asp Asn Asn Thr Leu Leu Asn
4945                4950                4955                4960
Val Ile Ala Cys Thr His Val Pro Cys Asn Thr Ser Cys Ser Pro Gly
                4965                4970                4975
Phe Glu Leu Met Glu Ala Pro Gly Glu Cys Cys Lys Lys Cys Glu Gln
            4980                4985                4990
Thr His Cys Ile Ile Lys Arg Pro Asp Asn Gln His Val Ile Leu Lys
        4995                5000                5005
Pro Gly Asp Phe Lys Ser Asp Pro Lys Asn Asn Cys Thr Phe Phe Ser
    5010                5015                5020
Cys Val Lys Ile His Asn Gln Leu Ile Ser Ser Val Ser Asn Ile Thr
5025                5030                5035                5040
Cys Pro Asn Phe Asp Ala Ser Ile Cys Ile Pro Gly Ser Ile Thr Phe
                5045                5050                5055
Met Pro Asn Gly Cys Cys Lys Thr Cys Thr Pro Arg Asn Glu Thr Arg
            5060                5065                5070
Val Pro Cys Ser Thr Val Pro Val Thr Thr Glu Val Ser Tyr Ala Gly
        5075                5080                5085
Cys Thr Lys Thr Val Leu Met Asn His Cys Ser Gly Ser Cys Gly Thr
    5090                5095                5100
Phe Val Met Tyr Ser Ala Lys Ala Gln Ala Leu Asp His Ser Cys Ser
5105                5110                5115                5120
Cys Cys Lys Glu Glu Lys Thr Ser Gln Arg Glu Val Val Leu Ser Cys
                5125                5130                5135

Pro Asn Gly Gly Ser Leu Thr His Thr Tyr Thr His Ile Glu Ser Cys
            5140                5145                5150
Gln Cys Gln Asp Thr Val Cys Gly Leu Pro Thr Gly Thr Ser Arg Arg
        5155                5160                5165
Ala Arg Arg Ser Pro Arg His Leu Gly Ser Gly
    5170                5175

<210> 122
<211> 1217
<212> PRT
<213> Homo sapiens

<400> 122
Ile Thr Ile Thr Glu Thr Thr Ser His Ser Thr Pro Ser Tyr Thr Thr
1               5                   10                  15
Ser Ile Thr Thr Thr Glu Thr Pro Ser His Ser Thr Pro Ser Tyr Thr
            20                  25                  30
Thr Ser Ile Thr Thr Thr Glu Thr Pro Ser His Ser Thr Pro Ser Phe
        35                  40                  45
Thr Ser Ser Ile Thr Thr Thr Glu Thr Thr Ser His Ser Thr Pro Ser
    50                  55                  60
Phe Thr Ser Ser Ile Arg Thr Thr Glu Thr Thr Ser Tyr Ser Thr Pro
65                  70                  75                  80
Ser Phe Thr Ser Ser Asn Thr Ile Thr Glu Thr Thr Ser His Ser Thr
                85                  90                  95
Pro Ser Tyr Ile Thr Ser Ile Thr Thr Thr Glu Thr Pro Ser Ser Ser

            100                 105                 110
Thr Pro Ser Phe Ser Ser Ser Ile Thr Thr Thr Glu Thr Thr Ser His
        115                 120                 125
Ser Thr Pro Gly Phe Thr Ser Ser Ile Thr Thr Thr Glu Thr Thr Ser
    130                 135                 140
His Ser Thr Pro Ser Phe Thr Ser Ser Ile Thr Thr Thr Glu Thr Thr
145                 150                 155                 160
Ser His Asp Thr Pro Ser Phe Thr Ser Ser Ile Thr Thr Ser Glu Thr
                165                 170                 175
Pro Ser His Ser Thr Pro Ser Ser Thr Ser Leu Ile Thr Thr Thr Lys
            180                 185                 190
Thr Thr Ser His Ser Thr Pro Ser Phe Thr Ser Ser Ile Thr Thr Thr
        195                 200                 205
Glu Thr Thr Ser His Ser Ala Arg Ser Phe Thr Ser Ser Ile Thr Thr
    210                 215                 220
Thr Glu Thr Thr Ser His Asn Thr Arg Ser Phe Thr Ser Ser Ile Thr
225                 230                 235                 240
Thr Thr Glu Thr Asn Ser His Ser Thr Thr Ser Phe Thr Ser Ser Ile
                245                 250                 255
Thr Thr Thr Glu Thr Thr Ser His Ser Thr Pro Ser Phe Ser Ser Ser
            260                 265                 270
Ile Thr Thr Thr Glu Thr Pro Leu His Ser Thr Pro Gly Leu Thr Ser
        275                 280                 285
Trp Val Thr Thr Thr Lys Thr Thr Ser His Ile Thr Pro Gly Leu Thr
    290                 295                 300
Ser Ser Ile Thr Thr Thr Glu Thr Thr Ser His Ser Thr Pro Gly Phe
305                 310                 315                 320
Thr Ser Ser Ile Thr Thr Thr Glu Thr Thr Ser Glu Ser Thr Pro Ser
                325                 330                 335

Leu Ser Ser Ser Thr Ile Tyr Ser Thr Val Ser Thr Ser Thr Thr Ala
            340                 345                 350
Ile Thr Ser His Phe Thr Thr Ser Glu Thr Ala Val Thr Pro Thr Pro
        355                 360                 365
Val Thr Pro Ser Ser Leu Ser Thr Asp Ile Pro Thr Thr Ser Leu Arg
    370                 375                 380
Thr Leu Thr Pro Ser Ser Val Gly Thr Ser Thr Ser Leu Thr Thr Thr
385                 390                 395                 400
Thr Asp Phe Pro Ser Ile Pro Thr Asp Ile Ser Thr Leu Pro Thr Arg
                405                 410                 415
Thr His Ile Ile Ser Ser Ser Pro Ser Ile Gln Ser Thr Glu Thr Ser
            420                 425                 430
Ser Leu Val Gly Thr Thr Ser Pro Thr Met Ser Thr Val Arg Met Thr
        435                 440                 445
Leu Arg Ile Thr Glu Asn Thr Pro Ile Ser Ser Phe Ser Thr Ser Ile
    450                 455                 460
Val Val Ile Pro Glu Thr Pro Thr Gln Thr Pro Pro Val Leu Thr Ser
465                 470                 475                 480
Ala Thr Gly Thr Gln Thr Ser Pro Ala Pro Thr Thr Val Thr Phe Gly
                485                 490                 495
Ser Thr Asp Ser Ser Thr Ser Thr Leu His Thr Leu Thr Pro Ser Thr
            500                 505                 510
Ala Leu Ser Thr Ile Val Ser Thr Ser Gln Val Pro Ile Pro Ser Thr
        515                 520                 525
His Ser Ser Thr Leu Gln Thr Thr Pro Ser Thr Pro Ser Leu Gln Thr
    530                 535                 540
Ser Leu Thr Ser Thr Ser Glu Phe Thr Thr Glu Ser Phe Thr Arg Gly
545                 550                 555                 560
Ser Thr Ser Thr Asn Ala Ile Leu Thr Ser Phe Ser Thr Ile Ile Trp

                565                 570                 575
Ser Ser Thr Pro Thr Ile Ile Met Ser Ser Ser Pro Ser Ser Ala Ser
            580                 585                 590
Ile Thr Pro Val Phe Ser Thr Thr Ile His Ser Val Pro Ser Ser Pro
        595                 600                 605
Tyr Ile Phe Ser Thr Glu Asn Val Gly Ser Ala Ser Ile Thr Gly Phe
    610                 615                 620
Pro Ser Leu Ser Ser Ser Ala Thr Thr Ser Thr Ser Ser Thr Ser Ser
625                 630                 635                 640
Ser Leu Thr Thr Ala Leu Thr Glu Ile Thr Pro Phe Ser Tyr Ile Ser
                645                 650                 655
Leu Pro Ser Thr Thr Pro Cys Pro Gly Thr Ile Thr Ile Thr Ile Val
            660                 665                 670
Pro Ala Ser Pro Thr Asp Pro Cys Val Glu Met Asp Pro Ser Thr Glu
        675                 680                 685
Ala Thr Ser Pro Pro Thr Thr Pro Leu Thr Val Phe Pro Phe Thr Thr
    690                 695                 700
Glu Met Val Thr Cys Pro Thr Ser Ile Ser Ile Gln Thr Thr Leu Thr
705                 710                 715                 720
Thr Tyr Met Asp Thr Ser Ser Met Met Pro Glu Ser Glu Ser Ser Ile
                725                 730                 735
Ser Pro Asn Ala Ser Ser Ser Thr Gly Thr Gly Thr Val Pro Thr Asn
            740                 745                 750
Thr Val Phe Thr Ser Thr Arg Leu Pro Thr Ser Glu Thr Trp Leu Ser
        755                 760                 765
Asn Ser Ser Val Ile Pro Leu Pro Leu Pro Gly Val Ser Thr Ile Pro
    770                 775                 780
Leu Thr Met Lys Pro Ser Ser Ser Leu Pro Thr Ile Leu Arg Thr Ser
785                 790                 795                 800

Ser Lys Ser Thr His Pro Ser Pro Pro Thr Thr Arg Thr Ser Glu Thr
                805                 810                 815
Pro Val Ala Thr Thr Gln Thr Pro Thr Thr Leu Thr Ser Arg Arg Thr
            820                 825                 830
Thr Arg Ile Thr Ser Gln Met Thr Thr Gln Ser Thr Leu Thr Thr Thr
        835                 840                 845
Ala Gly Thr Cys Asp Asn Gly Gly Thr Trp Glu Gln Gly Gln Cys Ala
    850                 855                 860
Cys Leu Pro Gly Phe Ser Gly Asp Arg Cys Gln Leu Gln Thr Arg Cys
865                 870                 875                 880
Gln Asn Gly Gly Gln Trp Asp Gly Leu Lys Cys Gln Cys Pro Ser Thr
                885                 890                 895
Phe Tyr Gly Ser Ser Cys Glu Phe Ala Val Glu Gln Val Asp Leu Asp
            900                 905                 910
Val Val Glu Thr Glu Val Gly Met Glu Val Ser Val Asp Gln Gln Phe
        915                 920                 925
Ser Pro Asp Leu Asn Asp Asn Thr Ser Gln Ala Tyr Arg Asp Phe Asn
    930                 935                 940
Lys Thr Phe Trp Asn Gln Met Gln Lys Ile Phe Ala Asp Met Gln Gly
945                 950                 955                 960
Phe Thr Phe Lys Gly Val Glu Ile Leu Ser Leu Arg Asn Gly Ser Ile
                965                 970                 975
Val Val Asp Tyr Leu Val Leu Leu Glu Met Pro Phe Ser Pro Gln Leu
            980                 985                 990
Glu Ser Glu Tyr Glu Gln Val Lys Thr Thr Leu Lys Glu Gly Leu Gln
        995                 1000                1005
Asn Ala Ser Gln Asp Val Asn Ser Cys Gln Asp Ser Gln Thr Leu Cys
    1010                1015                1020
Phe Lys Pro Asp Ser Ile Lys Val Asn Asn Asn Ser Lys Thr Glu Leu

1025                1030                1035                1040

Thr Pro Ala Ala Ile Cys Arg Arg Ala Ala Pro Thr Gly Tyr Glu Glu

                1045                1050                1055

Phe Tyr Phe Pro Leu Val Glu Ala Thr Arg Leu Arg Cys Val Thr Lys

            1060                1065                1070

Cys Thr Ser Gly Val Asp Asn Ala Ile Asp Cys His Gln Gly Gln Cys

        1075                1080                1085

Val Leu Glu Thr Ser Gly Pro Thr Cys Arg Cys Tyr Ser Thr Asp Thr

    1090                1095                1100

His Trp Phe Ser Gly Pro Arg Cys Glu Val Ala Val His Trp Arg Ala

1105                1110                1115                1120

Leu Val Gly Gly Leu Thr Ala Gly Ala Ala Leu Leu Val Leu Leu Leu

                1125                1130                1135

Leu Ala Leu Gly Val Arg Ala Val Arg Ser Gly Trp Trp Gly Gly Gln

            1140                1145                1150

Arg Arg Gly Arg Ser Trp Asp Gln Asp Arg Lys Trp Phe Glu Thr Trp

        1155                1160                1165

Asp Glu Glu Val Val Gly Thr Phe Ser Asn Trp Gly Phe Glu Asp Asp

    1170                1175                1180

Gly Thr Asp Lys Asp Thr Asn Phe Tyr Val Ala Leu Glu Asn Val Asp

1185                1190                1195                1200

Thr Thr Met Lys Val His Ile Lys Arg Pro Glu Met Thr Ser Ser Ser

                1205                1210                1215

Val

<210> 123

<211> 1373

<212> PRT

<213> Homo sapiens

<400> 123

Met Ser Val Gly Arg Arg Lys Leu Ala Leu Leu Trp Ala Leu Ala Leu

1               5                   10                  15

Ala Leu Ala Cys Thr Arg His Thr Gly His Ala Gln Asp Gly Ser Ser

            20                  25                  30

Glu Ser Ser Tyr Lys His His Pro Ala Leu Ser Pro Ile Ala Arg Gly

        35                  40                  45

Pro Ile Gly Val Pro Leu Arg Gly Ala Thr Val Phe Pro Ser Leu Arg

    50                  55                  60

Thr Ile Pro Val Val Arg Ala Ser Asn Pro Ala His Asn Gly Arg Val

65                  70                  75                  80

Cys Ser Thr Trp Gly Ser Phe His Tyr Lys Thr Phe Asp Gly Asp Val

                85                  90                  95

Phe Arg Phe Pro Gly Leu Cys Asn Tyr Val Phe Ser Glu His Cys Gly

            100                 105                 110

Ala Ala Tyr Glu Asp Phe Asn Ile Pro Ala Thr Pro Gln Pro Gly Val

        115                 120                 125

Ser Gly Pro His Ala Glu Gln Gly Pro His Glu Gly Gly Trp Arg Gly

    130                 135                 140

His Pro Ala Asp Gln Gly Leu Arg Pro Gly Gln Arg Pro Pro Gly Pro

145                 150                 155                 160

Ala Ala Leu Gln Pro Val Trp Gly Pro His Ser Ala Arg Ala Ala Ala

                165                 170                 175

Thr Pro Arg Trp Lys Pro Gly Trp Ala Leu Ser Ser Cys Gly Thr Thr

            180                 185                 190

Met Thr Ala Cys Cys Trp Lys Leu Asp Thr Lys Tyr Ala Asn Lys Asn

        195                 200                 205

Leu Trp Ala Leu Trp Gly Leu Gln Arg Asp Ala Arg Gly Gln Arg Ala

    210                 215                 220

Pro Leu Pro Gln His Gln Ala Asp Thr His Gly Ile Arg Glu Pro Ala

225                 230                 235                 240

Glu Arg Trp Thr Asn Pro Arg Ser Ser Val Arg Thr Leu Ser Leu Asn

                245                 250                 255

Pro Arg Arg Thr Ala Pro Leu Ala Leu Ala Ser Cys Glu Glu Leu Leu

            260                 265                 270

His Gly Gln Leu Phe Ser Gly Cys Val Ala Leu Val Asp Val Gly Ser

        275                 280                 285

Tyr Leu Glu Ala Cys Arg Gln Asp Leu Cys Phe Cys Glu Asp Thr Asp

    290                 295                 300

Leu Leu Ser Cys Val Cys His Thr Leu Ala Glu Tyr Ser Arg Gln Cys

305                 310                 315                 320

Thr His Ala Gly Gly Leu Pro Gln Asp Trp Arg Gly Pro Asp Phe Cys

                325                 330                 335

Pro Gln Lys Cys Pro Asn Asn Met Gln Tyr His Glu Cys Arg Ser Pro

            340                 345                 350

Cys Ala Asp Thr Cys Ser Asn Gln Glu His Ser Arg Ala Cys Glu Asp

        355                 360                 365

His Cys Val Ala Gly Cys Phe Cys Pro Glu Gly Thr Val Leu Asp Asp

    370                 375                 380

Ile Gly Gln Thr Gly Cys Val Pro Val Ser Lys Cys Ala Cys Val Tyr

385                 390                 395                 400

Asn Gly Ala Ala Tyr Ala Pro Gly Ala Thr Tyr Ser Thr Asp Cys Thr

                405                 410                 415

Asn Cys Thr Cys Ser Gly Gly Arg Trp Ser Cys Gln Glu Val Pro Cys

            420                 425                 430

Pro Gly Thr Cys Ser Val Leu Gly Gly Ala His Phe Ser Thr Phe Asp
        435                 440                 445
Gly Lys Gln Tyr Thr Val His Gly Asp Cys Ser Tyr Val Leu Thr Lys
    450                 455                 460
Pro Cys Asp Ser Ser Ala Phe Thr Val Leu Ala Glu Leu Arg Arg Cys
465                 470                 475                 480
Gly Leu Thr Asp Ser Glu Thr Cys Leu Lys Ser Val Thr Leu Ser Leu
                485                 490                 495
Asp Gly Ala Gln Thr Val Val Val Ile Lys Ala Ser Gly Glu Val Phe
            500                 505                 510
Leu Asn Gln Ile Tyr Thr Gln Leu Pro Ile Ser Ala Ala Asn Val Thr
        515                 520                 525
Ile Phe Arg Pro Ser Thr Phe Phe Ile Ile Ala Gln Thr Ser Leu Gly
    530                 535                 540
Leu Gln Leu Asn Leu Gln Leu Val Pro Thr Met Gln Leu Phe Met Gln
545                 550                 555                 560
Leu Ala Pro Lys Leu Arg Gly Gln Thr Cys Gly Leu Cys Gly Asn Phe
                565                 570                 575
Asn Ser Ile Gln Ala Asp Asp Phe Arg Thr Leu Ser Gly Val Val Glu
            580                 585                 590
Ala Thr Ala Ala Ala Phe Phe Asn Thr Phe Lys Thr Gln Ala Ala Cys
        595                 600                 605
Pro Asn Ile Arg Asn Ser Phe Glu Asp Pro Cys Ser Leu Ser Val Glu
    610                 615                 620
Asn Glu Lys Tyr Ala Gln His Trp Cys Ser Gln Leu Thr Asp Ala Asp
625                 630                 635                 640
Gly Pro Phe Gly Arg Cys His Ala Ala Val Lys Pro Gly Thr Tyr Tyr
                645                 650                 655
Ser Asn Cys Met Phe Asp Thr Cys Asn Cys Glu Arg Ser Glu Asp Cys

            660                 665                 670
Leu Cys Ala Ala Leu Ser Ser Tyr Val His Ala Cys Ala Ala Lys Gly
        675                 680                 685
Val Gln Leu Gly Gly Trp Arg Asp Gly Val Cys Thr Lys Pro Met Thr
    690                 695                 700
Thr Cys Pro Lys Ser Met Thr Tyr His Tyr His Val Ser Thr Cys Gln
705                 710                 715                 720
Pro Thr Cys Arg Ser Leu Ser Glu Gly Asp Ile Thr Cys Ser Val Gly
                725                 730                 735
Phe Ile Pro Val Asp Gly Cys Ile Cys Pro Lys Gly Thr Phe Leu Asp
            740                 745                 750
Asp Thr Gly Lys Cys Val Gln Ala Ser Asn Cys Pro Cys Tyr His Arg
        755                 760                 765
Gly Ser Met Ile Pro Asn Gly Glu Ser Val His Asp Ser Gly Ala Ile
    770                 775                 780
Cys Thr Cys Thr His Gly Lys Leu Ser Cys Ile Gly Gly Gln Ala Pro
785                 790                 795                 800
Ala Pro Val Cys Ala Ala Pro Met Val Phe Phe Asp Cys Arg Asn Ala
                805                 810                 815
Thr Pro Gly Asp Thr Gly Ala Gly Cys Gln Lys Ser Cys His Thr Leu
            820                 825                 830
Asp Met Thr Cys Tyr Ser Pro Gln Cys Val Pro Gly Cys Val Cys Pro
        835                 840                 845
Asp Gly Leu Val Ala Asp Gly Glu Gly Gly Cys Ile Thr Ala Glu Asp
    850                 855                 860
Cys Pro Cys Val His Asn Glu Ala Ser Tyr Arg Ala Gly Gln Thr Ile
865                 870                 875                 880
Arg Val Gly Cys Asn Thr Cys Thr Cys Asp Ser Arg Met Trp Arg Cys
                885                 890                 895

Thr Asp Asp Pro Cys Leu Ala Thr Cys Ala Val Tyr Gly Asp Gly His
            900                 905                 910
Tyr Leu Thr Phe Asp Gly Gln Ser Tyr Ser Phe Asn Glu Glu Thr Ala
        915                 920                 925
Ser Thr Arg Trp Cys Arg Thr Ala Val Ala Gly Lys Thr Ala Pro Arg
    930                 935                 940
Thr Pro Phe Val Leu Ser Pro Arg Thr Ser Pro Ala Ala Pro Gln Gly
945                 950                 955                 960
Pro Pro Ala Pro Arg Pro Ser Arg Phe Ser Trp Gly Asn Phe Glu Leu
                965                 970                 975
Lys Leu Ser His Gly Lys Val Glu Val Ile Gly Thr Asp Glu Ser Gln
            980                 985                 990
Glu Val Pro Tyr Thr Ile Arg Gln Met Gly Ile Tyr Leu Val Val Asp
        995                 1000                1005
Thr Asp Ile Gly Leu Val Leu Leu Trp Asp Lys Lys Thr Ser Ile Phe
    1010                1015                1020
Ile Asn Leu Ser Pro Glu Phe Lys Gly Arg Val Cys Gly Leu Cys Gly
1025                1030                1035                1040
Asn Phe Asp Asp Ile Ala Val Asn Asp Phe Ala Thr Arg Ser Arg Ser
                1045                1050                1055
Val Val Gly Asp Val Leu Glu Phe Gly Asn Ser Trp Lys Leu Ser Pro
            1060                1065                1070
Ser Cys Pro Asp Ala Leu Ala Pro Lys Asp Pro Cys Thr Ala Asn Pro
        1075                1080                1085
Phe Arg Lys Ser Trp Ala Gln Lys Gln Cys Ser Ile Leu His Gly Pro
    1090                1095                1100
Thr Phe Ala Ala Cys His Ala His Val Glu Pro Ala Arg Tyr Tyr Glu
1105                1110                1115                1120
Ala Cys Val Asn Asp Ala Cys Ala Cys Asp Ser Gly Gly Asp Cys Glu

1125                1130                1135

Cys Phe Cys Thr Ala Val Ala Arg Tyr Ala Gln Ala Cys His Glu Val

            1140                1145                1150

Gly Thr Cys Val Cys Leu Arg Thr Pro Ser Ile Cys Pro Leu Phe Cys

        1155                1160                1165

Asp Tyr Tyr Asn Pro Glu Gly Gln Cys Glu Trp His Tyr Gln Pro Cys

    1170                1175                1180

Gly Val Pro Cys Leu Arg Thr Cys Arg Asn Pro Arg Gly Asp Cys Leu

1185                1190                1195                1200

Arg Asp Val Arg Gly Leu Glu Gly Cys Tyr Pro Lys Cys Pro Pro Glu

                1205                1210                1215

Ala Pro Ile Phe Asp Glu Asp Lys Met Gln Cys Val Ala Thr Cys Pro

            1220                1225                1230

Thr Pro Pro Leu Pro Pro Arg Cys His Val His Gly Lys Ser Tyr Arg

        1235                1240                1245

Pro Gly Ala Val Val Pro Ser Asp Lys Asn Cys Gln Ser Cys Leu Cys

    1250                1255                1260

Thr Glu Arg Gly Val Glu Cys Thr Tyr Lys Ala Glu Ala Cys Val Cys

1265                1270                1275                1280

Thr Tyr Asn Gly Gln Arg Phe His Pro Gly Asp Val Ile Tyr His Thr

                1285                1290                1295

Thr Asp Gly Thr Gly Gly Cys Ile Ser Ala Arg Cys Gly Ala Asn Gly

            1300                1305                1310

Thr Ile Glu Arg Arg Val Tyr Pro Cys Ser Pro Thr Thr Pro Val Pro

        1315                1320                1325

Pro Thr Thr Phe Ser Phe Ser Thr Pro Pro Leu Val Val Ser Ser Thr

    1330                1335                1340

His Thr Pro Ser Asn Gly Pro Ser Ser Ala His Thr Gly Pro Pro Ser

1345                1350                1355                1360

Ser Ala Trp Pro Thr Thr Ala Gly Thr Ser Pro Arg Thr
                1365                1370

<210> 124
<211> 165
<212> PRT
<213> Homo sapiens

<400> 124
Met Glu Met Phe Gln Gly Leu Leu Leu Leu Leu Leu Leu Ser Met Gly
1               5                   10                  15
Gly Thr Trp Ala Ser Lys Glu Pro Leu Arg Pro Arg Cys Arg Pro Ile
            20                  25                  30
Asn Ala Thr Leu Ala Val Glu Lys Glu Gly Cys Pro Val Cys Ile Thr
        35                  40                  45
Val Asn Thr Thr Ile Cys Ala Gly Tyr Cys Pro Thr Met Thr Arg Val
    50                  55                  60
Leu Gln Gly Val Leu Pro Ala Leu Pro Gln Val Val Cys Asn Tyr Arg
65                  70                  75                  80
Asp Val Arg Phe Glu Ser Ile Arg Leu Pro Gly Cys Pro Arg Gly Val
                85                  90                  95
Asn Pro Val Val Ser Tyr Ala Val Ala Leu Ser Cys Gln Cys Ala Leu
            100                 105                 110
Cys Arg Arg Ser Thr Thr Asp Cys Gly Gly Pro Lys Asp His Pro Leu
        115                 120                 125
Thr Cys Asp Asp Pro Arg Phe Gln Asp Ser Ser Ser Ser Lys Ala Pro
    130                 135                 140
Pro Pro Ser Leu Pro Ser Pro Ser Arg Leu Pro Gly Pro Ser Asp Thr

145                 150                 155                 160

Pro Ile Leu Pro Gln

                165

<210> 125

<211> 1210

<212> PRT

<213> Homo sapiens

<400> 125

Met Arg Pro Ser Gly Thr Ala Gly Ala Ala Leu Leu Ala Leu Leu Ala

 1               5                  10                  15

Ala Leu Cys Pro Ala Ser Arg Ala Leu Glu Glu Lys Lys Val Cys Gln

            20                  25                  30

Gly Thr Ser Asn Lys Leu Thr Gln Leu Gly Thr Phe Glu Asp His Phe

        35                  40                  45

Leu Ser Leu Gln Arg Met Phe Asn Asn Cys Glu Val Val Leu Gly Asn

    50                  55                  60

Leu Glu Ile Thr Tyr Val Gln Arg Asn Tyr Asp Leu Ser Phe Leu Lys

65                  70                  75                  80

Thr Ile Gln Glu Val Ala Gly Tyr Val Leu Ile Ala Leu Asn Thr Val

                85                  90                  95

Glu Arg Ile Pro Leu Glu Asn Leu Gln Ile Ile Arg Gly Asn Met Tyr

            100                 105                 110

Tyr Glu Asn Ser Tyr Ala Leu Ala Val Leu Ser Asn Tyr Asp Ala Asn

        115                 120                 125

Lys Thr Gly Leu Lys Glu Leu Pro Met Arg Asn Leu Gln Glu Ile Leu

    130                 135                 140

His Gly Ala Val Arg Phe Ser Asn Asn Pro Ala Leu Cys Asn Val Glu
145                 150                 155                 160
Ser Ile Gln Trp Arg Asp Ile Val Ser Ser Asp Phe Leu Ser Asn Met
                165                 170                 175
Ser Met Asp Phe Gln Asn His Leu Gly Ser Cys Gln Lys Cys Asp Pro
            180                 185                 190
Ser Cys Pro Asn Gly Ser Cys Trp Gly Ala Gly Glu Glu Asn Cys Gln
        195                 200                 205
Lys Leu Thr Lys Ile Ile Cys Ala Gln Gln Cys Ser Gly Arg Cys Arg
    210                 215                 220
Gly Lys Ser Pro Ser Asp Cys Cys His Asn Gln Cys Ala Ala Gly Cys
225                 230                 235                 240
Thr Gly Pro Arg Glu Ser Asp Cys Leu Val Cys Arg Lys Phe Arg Asp
                245                 250                 255
Glu Ala Thr Cys Lys Asp Thr Cys Pro Pro Leu Met Leu Tyr Asn Pro
            260                 265                 270
Thr Thr Tyr Gln Met Asp Val Asn Pro Glu Gly Lys Tyr Ser Phe Gly
        275                 280                 285
Ala Thr Cys Val Lys Lys Cys Pro Arg Asn Tyr Val Val Thr Asp His
    290                 295                 300
Gly Ser Cys Val Arg Ala Cys Gly Ala Asp Ser Tyr Glu Met Glu Glu
305                 310                 315                 320
Asp Gly Val Arg Lys Cys Lys Lys Cys Glu Gly Pro Cys Arg Lys Val
                325                 330                 335
Cys Asn Gly Ile Gly Ile Gly Glu Phe Lys Asp Ser Leu Ser Ile Asn
            340                 345                 350
Ala Thr Asn Ile Lys His Phe Lys Asn Cys Thr Ser Ile Ser Gly Asp
        355                 360                 365
Leu His Ile Leu Pro Val Ala Phe Arg Gly Asp Ser Phe Thr His Thr

    370                 375                 380

Pro Pro Leu Asp Pro Gln Glu Leu Asp Ile Leu Lys Thr Val Lys Glu

385                 390                 395                 400

Ile Thr Gly Phe Leu Leu Ile Gln Ala Trp Pro Glu Asn Arg Thr Asp

                405                 410                 415

Leu His Ala Phe Glu Asn Leu Glu Ile Ile Arg Gly Arg Thr Lys Gln

            420                 425                 430

His Gly Gln Phe Ser Leu Ala Val Val Ser Leu Asn Ile Thr Ser Leu

        435                 440                 445

Gly Leu Arg Ser Leu Lys Glu Ile Ser Asp Gly Asp Val Ile Ile Ser

    450                 455                 460

Gly Asn Lys Asn Leu Cys Tyr Ala Asn Thr Ile Asn Trp Lys Lys Leu

465                 470                 475                 480

Phe Gly Thr Ser Gly Gln Lys Thr Lys Ile Ile Ser Asn Arg Gly Glu

                485                 490                 495

Asn Ser Cys Lys Ala Thr Gly Gln Val Cys His Ala Leu Cys Ser Pro

            500                 505                 510

Glu Gly Cys Trp Gly Pro Glu Pro Arg Asp Cys Val Ser Cys Arg Asn

        515                 520                 525

Val Ser Arg Gly Arg Glu Cys Val Asp Lys Cys Lys Leu Leu Glu Gly

    530                 535                 540

Glu Pro Arg Glu Phe Val Glu Asn Ser Glu Cys Ile Gln Cys His Pro

545                 550                 555                 560

Glu Cys Leu Pro Gln Ala Met Asn Ile Thr Cys Thr Gly Arg Gly Pro

                565                 570                 575

Asp Asn Cys Ile Gln Cys Ala His Tyr Ile Asp Gly Pro His Cys Val

            580                 585                 590

Lys Thr Cys Pro Ala Gly Val Met Gly Glu Asn Asn Thr Leu Val Trp

        595                 600                 605

Lys Tyr Ala Asp Ala Gly His Val Cys His Leu Cys His Pro Asn Cys
    610                 615                 620
Thr Tyr Gly Cys Thr Gly Pro Gly Leu Glu Gly Cys Pro Thr Asn Gly
625                 630                 635                 640
Pro Lys Ile Pro Ser Ile Ala Thr Gly Met Val Gly Ala Leu Leu Leu
                645                 650                 655
Leu Leu Val Val Ala Leu Gly Ile Gly Leu Phe Met Arg Arg Arg His
            660                 665                 670
Ile Val Arg Lys Arg Thr Leu Arg Arg Leu Leu Gln Glu Arg Glu Leu
        675                 680                 685
Val Glu Pro Leu Thr Pro Ser Gly Glu Ala Pro Asn Gln Ala Leu Leu
    690                 695                 700
Arg Ile Leu Lys Glu Thr Glu Phe Lys Lys Ile Lys Val Leu Gly Ser
705                 710                 715                 720
Gly Ala Phe Gly Thr Val Tyr Lys Gly Leu Trp Ile Pro Glu Gly Glu
                725                 730                 735
Lys Val Lys Ile Pro Val Ala Ile Lys Glu Leu Arg Glu Ala Thr Ser
            740                 745                 750
Pro Lys Ala Asn Lys Glu Ile Leu Asp Glu Ala Tyr Val Met Ala Ser
        755                 760                 765
Val Asp Asn Pro His Val Cys Arg Leu Leu Gly Ile Cys Leu Thr Ser
    770                 775                 780
Thr Val Gln Leu Ile Thr Gln Leu Met Pro Phe Gly Cys Leu Leu Asp
785                 790                 795                 800
Tyr Val Arg Glu His Lys Asp Asn Ile Gly Ser Gln Tyr Leu Leu Asn
                805                 810                 815
Trp Cys Val Gln Ile Ala Lys Gly Met Asn Tyr Leu Glu Asp Arg Arg
            820                 825                 830
Leu Val His Arg Asp Leu Ala Ala Arg Asn Val Leu Val Lys Thr Pro

        835                 840                 845
Gln His Val Lys Ile Thr Asp Phe Gly Leu Ala Lys Leu Leu Gly Ala
    850                 855                 860
Glu Glu Lys Glu Tyr His Ala Glu Gly Gly Lys Val Pro Ile Lys Trp
865                 870                 875                 880
Met Ala Leu Glu Ser Ile Leu His Arg Ile Tyr Thr His Gln Ser Asp
                885                 890                 895
Val Trp Ser Tyr Gly Val Thr Val Trp Glu Leu Met Thr Phe Gly Ser
            900                 905                 910
Lys Pro Tyr Asp Gly Ile Pro Ala Ser Glu Ile Ser Ser Ile Leu Glu
        915                 920                 925
Lys Gly Glu Arg Leu Pro Gln Pro Pro Ile Cys Thr Ile Asp Val Tyr
    930                 935                 940
Met Ile Met Val Lys Cys Trp Met Ile Asp Ala Asp Ser Arg Pro Lys
945                 950                 955                 960
Phe Arg Glu Leu Ile Ile Glu Phe Ser Lys Met Ala Arg Asp Pro Gln
                965                 970                 975
Arg Tyr Leu Val Ile Gln Gly Asp Glu Arg Met His Leu Pro Ser Pro
            980                 985                 990
Thr Asp Ser Asn Phe Tyr Arg Ala Leu Met Asp Glu Glu Asp Met Asp
        995                 1000                1005
Asp Val Val Asp Ala Asp Glu Tyr Leu Ile Pro Gln Gln Gly Phe Phe
    1010                1015                1020
Ser Ser Pro Ser Thr Ser Arg Thr Pro Leu Leu Ser Ser Leu Ser Ala
1025                1030                1035                1040
Thr Ser Asn Asn Ser Thr Val Ala Cys Ile Asp Arg Asn Gly Leu Gln
                1045                1050                1055
Ser Cys Pro Ile Lys Glu Asp Ser Phe Leu Gln Arg Tyr Ser Ser Asp
            1060                1065                1070

Pro Thr Gly Ala Leu Thr Glu Asp Ser Ile Asp Asp Thr Phe Leu Pro
        1075                1080                1085
Val Pro Glu Tyr Ile Asn Gln Ser Val Pro Lys Arg Pro Ala Gly Ser
    1090                1095                1100
Val Gln Asn Pro Val Tyr His Asn Gln Pro Leu Asn Pro Ala Pro Ser
1105                1110                1115                1120
Arg Asp Pro His Tyr Gln Asp Pro His Ser Thr Ala Val Gly Asn Pro
                1125                1130                1135
Glu Tyr Leu Asn Thr Val Gln Pro Thr Cys Val Asn Ser Thr Phe Asp
            1140                1145                1150
Ser Pro Ala His Trp Ala Gln Lys Gly Ser His Gln Ile Ser Leu Asp
        1155                1160                1165
Asn Pro Asp Tyr Gln Gln Asp Phe Phe Pro Lys Glu Ala Lys Pro Asn
    1170                1175                1180
Gly Ile Phe Lys Gly Ser Thr Ala Glu Asn Ala Glu Tyr Leu Arg Val
1185                1190                1195                1200
Ala Pro Gln Ser Ser Glu Phe Ile Gly Ala
                1205                1210

<210> 126
<211> 1255
<212> PRT
<213> Homo sapiens

<400> 126
Met Glu Leu Ala Ala Leu Cys Arg Trp Gly Leu Leu Leu Ala Leu Leu
 1               5                  10                  15
Pro Pro Gly Ala Ala Ser Thr Gln Val Cys Thr Gly Thr Asp Met Lys

20                  25                  30

Leu Arg Leu Pro Ala Ser Pro Glu Thr His Leu Asp Met Leu Arg His

        35                  40                  45

Leu Tyr Gln Gly Cys Gln Val Val Gln Gly Asn Leu Glu Leu Thr Tyr

    50                  55                  60

Leu Pro Thr Asn Ala Ser Leu Ser Phe Leu Gln Asp Ile Gln Glu Val

65                  70                  75                  80

Gln Gly Tyr Val Leu Ile Ala His Asn Gln Val Arg Gln Val Pro Leu

                85                  90                  95

Gln Arg Leu Arg Ile Val Arg Gly Thr Gln Leu Phe Glu Asp Asn Tyr

            100                 105                 110

Ala Leu Ala Val Leu Asp Asn Gly Asp Pro Leu Asn Asn Thr Thr Pro

        115                 120                 125

Val Thr Gly Ala Ser Pro Gly Gly Leu Arg Glu Leu Gln Leu Arg Ser

    130                 135                 140

Leu Thr Glu Ile Leu Lys Gly Gly Val Leu Ile Gln Arg Asn Pro Gln

145                 150                 155                 160

Leu Cys Tyr Gln Asp Thr Ile Leu Trp Lys Asp Ile Phe His Lys Asn

                165                 170                 175

Asn Gln Leu Ala Leu Thr Leu Ile Asp Thr Asn Arg Ser Arg Ala Cys

            180                 185                 190

His Pro Cys Ser Pro Met Cys Lys Gly Ser Arg Cys Trp Gly Glu Ser

        195                 200                 205

Ser Glu Asp Cys Gln Ser Leu Thr Arg Thr Val Cys Ala Gly Gly Cys

    210                 215                 220

Ala Arg Cys Lys Gly Pro Leu Pro Thr Asp Cys Cys His Glu Gln Cys

225                 230                 235                 240

Ala Ala Gly Cys Thr Gly Pro Lys His Ser Asp Cys Leu Ala Cys Leu

                245                 250                 255

His Phe Asn His Ser Gly Ile Cys Glu Leu His Cys Pro Ala Leu Val
            260                 265                 270
Thr Tyr Asn Thr Asp Thr Phe Glu Ser Met Pro Asn Pro Glu Gly Arg
        275                 280                 285
Tyr Thr Phe Gly Ala Ser Cys Val Thr Ala Cys Pro Tyr Asn Tyr Leu
    290                 295                 300
Ser Thr Asp Val Gly Ser Cys Thr Leu Val Cys Pro Leu His Asn Gln
305                 310                 315                 320
Glu Val Thr Ala Glu Asp Gly Thr Gln Arg Cys Glu Lys Cys Ser Lys
                325                 330                 335
Pro Cys Ala Arg Val Cys Tyr Gly Leu Gly Met Glu His Leu Arg Glu
            340                 345                 350
Val Arg Ala Val Thr Ser Ala Asn Ile Gln Glu Phe Ala Gly Cys Lys
        355                 360                 365
Lys Ile Phe Gly Ser Leu Ala Phe Leu Pro Glu Ser Phe Asp Gly Asp
    370                 375                 380
Pro Ala Ser Asn Thr Ala Pro Leu Gln Pro Glu Gln Leu Gln Val Phe
385                 390                 395                 400
Glu Thr Leu Glu Glu Ile Thr Gly Tyr Leu Tyr Ile Ser Ala Trp Pro
                405                 410                 415
Asp Ser Leu Pro Asp Leu Ser Val Phe Gln Asn Leu Gln Val Ile Arg
            420                 425                 430
Gly Arg Ile Leu His Asn Gly Ala Tyr Ser Leu Thr Leu Gln Gly Leu
        435                 440                 445
Gly Ile Ser Trp Leu Gly Leu Arg Ser Leu Arg Glu Leu Gly Ser Gly
    450                 455                 460
Leu Ala Leu Ile His His Asn Thr His Leu Cys Phe Val His Thr Val
465                 470                 475                 480
Pro Trp Asp Gln Leu Phe Arg Asn Pro His Gln Ala Leu Leu His Thr

                485                 490                 495
Ala Asn Arg Pro Glu Asp Glu Cys Val Gly Glu Gly Leu Ala Cys His
            500                 505                 510
Gln Leu Cys Ala Arg Gly His Cys Trp Gly Pro Gly Pro Thr Gln Cys
        515                 520                 525
Val Asn Cys Ser Gln Phe Leu Arg Gly Gln Glu Cys Val Glu Glu Cys
    530                 535                 540
Arg Val Leu Gln Gly Leu Pro Arg Glu Tyr Val Asn Ala Arg His Cys
545                 550                 555                 560
Leu Pro Cys His Pro Glu Cys Gln Pro Gln Asn Gly Ser Val Thr Cys
                565                 570                 575
Phe Gly Pro Glu Ala Asp Gln Cys Val Ala Cys Ala His Tyr Lys Asp
            580                 585                 590
Pro Pro Phe Cys Val Ala Arg Cys Pro Ser Gly Val Lys Pro Asp Leu
        595                 600                 605
Ser Tyr Met Pro Ile Trp Lys Phe Pro Asp Glu Glu Gly Ala Cys Gln
    610                 615                 620
Pro Cys Pro Ile Asn Cys Thr His Ser Cys Val Asp Leu Asp Asp Lys
625                 630                 635                 640
Gly Cys Pro Ala Glu Gln Arg Ala Ser Pro Leu Thr Ser Ile Val Ser
                645                 650                 655
Ala Val Val Gly Ile Leu Leu Val Val Val Leu Gly Val Val Phe Gly
            660                 665                 670
Ile Leu Ile Lys Arg Arg Gln Gln Lys Ile Arg Lys Tyr Thr Met Arg
        675                 680                 685
Arg Leu Leu Gln Glu Thr Glu Leu Val Glu Pro Leu Thr Pro Ser Gly
    690                 695                 700
Ala Met Pro Asn Gln Ala Gln Met Arg Ile Leu Lys Glu Thr Glu Leu
705                 710                 715                 720

Arg Lys Val Lys Val Leu Gly Ser Gly Ala Phe Gly Thr Val Tyr Lys
                725                 730                 735
Gly Ile Trp Ile Pro Asp Gly Glu Asn Val Lys Ile Pro Val Ala Ile
            740                 745                 750
Lys Val Leu Arg Glu Asn Thr Ser Pro Lys Ala Asn Lys Glu Ile Leu
        755                 760                 765
Asp Glu Ala Tyr Val Met Ala Gly Val Gly Ser Pro Tyr Val Ser Arg
    770                 775                 780
Leu Leu Gly Ile Cys Leu Thr Ser Thr Val Gln Leu Val Thr Gln Leu
785                 790                 795                 800
Met Pro Tyr Gly Cys Leu Leu Asp His Val Arg Glu Asn Arg Gly Arg
                805                 810                 815
Leu Gly Ser Gln Asp Leu Leu Asn Trp Cys Met Gln Ile Ala Lys Gly
            820                 825                 830
Met Ser Tyr Leu Glu Asp Val Arg Leu Val His Arg Asp Leu Ala Ala
        835                 840                 845
Arg Asn Val Leu Val Lys Ser Pro Asn His Val Lys Ile Thr Asp Phe
    850                 855                 860
Gly Leu Ala Arg Leu Leu Asp Ile Asp Glu Thr Glu Tyr His Ala Asp
865                 870                 875                 880
Gly Gly Lys Val Pro Ile Lys Trp Met Ala Leu Glu Ser Ile Leu Arg
                885                 890                 895
Arg Arg Phe Thr His Gln Ser Asp Val Trp Ser Tyr Gly Val Thr Val
            900                 905                 910
Trp Glu Leu Met Thr Phe Gly Ala Lys Pro Tyr Asp Gly Ile Pro Ala
        915                 920                 925
Arg Glu Ile Pro Asp Leu Leu Glu Lys Gly Glu Arg Leu Pro Gln Pro
    930                 935                 940
Pro Ile Cys Thr Ile Asp Val Tyr Met Ile Met Val Lys Cys Trp Met

945                 950                 955                 960

Ile Asp Ser Glu Cys Arg Pro Arg Phe Arg Glu Leu Val Ser Glu Phe

                965                 970                 975

Ser Arg Met Ala Arg Asp Pro Gln Arg Phe Val Val Ile Gln Asn Glu

            980                 985                 990

Asp Leu Gly Pro Ala Ser Pro Leu Asp Ser Thr Phe Tyr Arg Ser Leu

        995                 1000                1005

Leu Glu Asp Asp Asp Met Gly Asp Leu Val Asp Ala Glu Glu Tyr Leu

    1010                1015                1020

Val Pro Gln Gln Gly Phe Phe Cys Pro Asp Pro Ala Pro Gly Ala Gly

1025                1030                1035                1040

Gly Met Val His His Arg His Arg Ser Ser Ser Thr Arg Ser Gly Gly

                1045                1050                1055

Gly Asp Leu Thr Leu Gly Leu Glu Pro Ser Glu Glu Glu Ala Pro Arg

            1060                1065                1070

Ser Pro Leu Ala Pro Ser Glu Gly Ala Gly Ser Asp Val Phe Asp Gly

        1075                1080                1085

Asp Leu Gly Met Gly Ala Ala Lys Gly Leu Gln Ser Leu Pro Thr His

    1090                1095                1100

Asp Pro Ser Pro Leu Gln Arg Tyr Ser Glu Asp Pro Thr Val Pro Leu

1105                1110                1115                1120

Pro Ser Glu Thr Asp Gly Tyr Val Ala Pro Leu Thr Cys Ser Pro Gln

                1125                1130                1135

Pro Glu Tyr Val Asn Gln Pro Asp Val Arg Pro Gln Pro Pro Ser Pro

            1140                1145                1150

Arg Glu Gly Pro Leu Pro Ala Ala Arg Pro Ala Gly Ala Thr Leu Glu

        1155                1160                1165

Arg Ala Lys Thr Leu Ser Pro Gly Lys Asn Gly Val Val Lys Asp Val

    1170                1175                1180

Phe Ala Phe Gly Gly Ala Val Glu Asn Pro Glu Tyr Leu Thr Pro Gln
1185                1190                1195                1200
Gly Gly Ala Ala Pro Gln Pro His Pro Pro Pro Ala Phe Ser Pro Ala
                1205                1210                1215
Phe Asp Asn Leu Tyr Tyr Trp Asp Gln Asp Pro Pro Glu Arg Gly Ala
            1220                1225                1230
Pro Pro Ser Thr Phe Lys Gly Thr Pro Thr Ala Glu Asn Pro Glu Tyr
        1235                1240                1245
Leu Gly Leu Asp Val Pro Val
    1250                1255

<210> 127
<211> 190
<212> PRT
<213> Homo sapiens

<400> 127
Met Thr Thr Ala Ser Thr Ser Gln Val Arg Gln Asn Tyr His Gln Asp
1               5                   10                  15
Ser Glu Ala Ala Ile Asn Arg Gln Ile Asn Leu Glu Leu Tyr Ala Ser
            20                  25                  30
Tyr Val Tyr Leu Ser Met Ser Tyr Tyr Phe Asp Arg Asp Asp Val Ala
        35                  40                  45
Leu Lys Asn Phe Ala Lys Tyr Phe Leu His Gln Ser His Glu Glu Arg
    50                  55                  60
Glu His Ala Glu Lys Leu Met Lys Leu Gln Asn Gln Arg Gly Gly Arg
65                  70                  75                  80
Ile Phe Leu Gln Asp Ile Lys Lys Pro Asp Cys Asp Asp Trp Glu Ser

85                  90                  95

Gly Leu Asn Ala Met Glu Cys Ala Leu His Leu Glu Lys Asn Val Asn

            100                 105                 110

Gln Ser Leu Leu Glu Leu His Lys Leu Ala Thr Asp Lys Asn Asp Pro

        115                 120                 125

His Leu Cys Asp Phe Ile Glu Thr His Tyr Leu Asn Glu Gln Val Lys

    130                 135                 140

Ala Ile Lys Glu Leu Gly Asp His Val Thr Asn Leu Arg Lys Met Gly

145                 150                 155                 160

Ala Pro Glu Ser Gly Leu Ala Glu Tyr Leu Phe Asp Lys His Thr Trp

                165                 170                 175

Glu Thr Val Ile Met Lys Ala Lys Pro Arg Ala Asn Phe Pro

            180                 185                 190

<210> 128

<211> 175

<212> PRT

<213> Homo sapiens

<400> 128

<210> 129
<211> 535
<212> PRT
<213> Homo sapiens

<400> 129
Met Leu Gly Pro Cys Met Leu Leu Leu Leu Leu Leu Leu Gly Leu Arg
1               5                   10                  15
Leu Gln Leu Ser Leu Gly Ile Ile Leu Val Glu Glu Glu Asn Pro Asp
            20                  25                  30
Phe Trp Asn Arg Glu Ala Ala Glu Ala Leu Gly Ala Ala Lys Lys Leu
        35                  40                  45
Gln Pro Ala Gln Thr Ala Ala Lys Asn Leu Ile Ile Phe Leu Gly Asp

50                  55                  60

Gly Met Gly Val Ser Thr Val Thr Ala Ala Arg Ile Leu Lys Gly Gln

65                  70                  75                  80

Lys Lys Asp Lys Leu Gly Pro Glu Ile Pro Leu Ala Met Asp Arg Phe

                85                  90                  95

Pro Tyr Val Ala Leu Ser Lys Thr Tyr Asn Val Asp Lys His Val Pro

            100                 105                 110

Asp Ser Gly Ala Thr Ala Thr Ala Tyr Leu Cys Gly Val Lys Gly Asn

        115                 120                 125

Phe Gln Thr Ile Gly Leu Ser Ala Ala Ala Arg Phe Asn Gln Cys Asn

    130                 135                 140

Thr Thr Arg Gly Asn Glu Val Ile Ser Val Met Asn Arg Ala Lys Lys

145                 150                 155                 160

Ala Gly Lys Ser Val Gly Val Val Thr Thr Thr Arg Val Gln His Ala

                165                 170                 175

Ser Pro Ala Gly Thr Tyr Ala His Thr Val Asn Arg Asn Trp Tyr Ser

            180                 185                 190

Asp Ala Asp Val Pro Ala Ser Ala Arg Gln Glu Gly Cys Gln Asp Ile

        195                 200                 205

Ala Thr Gln Leu Ile Ser Asn Met Asp Ile Asp Val Ile Leu Gly Gly

    210                 215                 220

Gly Arg Lys Tyr Met Phe Arg Met Gly Thr Pro Asp Pro Glu Tyr Pro

225                 230                 235                 240

Asp Asp Tyr Ser Gln Gly Gly Thr Arg Leu Asp Gly Lys Asn Leu Val

                245                 250                 255

Gln Glu Trp Leu Ala Lys Arg Gln Gly Ala Arg Tyr Val Trp Asn Arg

            260                 265                 270

Thr Glu Leu Met Gln Ala Ser Leu Asp Pro Ser Val Ala His Leu Met

        275                 280                 285

Gly Leu Phe Glu Pro Gly Asp Met Lys Tyr Glu Ile His Arg Asp Ser
    290                 295                 300
Thr Leu Asp Pro Ser Leu Met Glu Met Thr Glu Ala Ala Leu Arg Leu
305                 310                 315                 320
Leu Ser Arg Asn Pro Arg Gly Phe Phe Leu Phe Val Glu Gly Gly Arg
                325                 330                 335
Ile Asp His Gly His His Glu Ser Arg Ala Tyr Arg Ala Leu Thr Glu
            340                 345                 350
Thr Ile Met Phe Asp Asp Ala Ile Glu Arg Ala Gly Gln Leu Thr Ser
        355                 360                 365
Glu Glu Asp Thr Leu Ser Leu Val Thr Ala Asp His Ser His Val Phe
    370                 375                 380
Ser Phe Gly Gly Tyr Pro Leu Arg Gly Ser Ser Ile Phe Gly Leu Ala
385                 390                 395                 400
Pro Gly Lys Ala Arg Asp Arg Lys Ala Tyr Thr Val Leu Leu Tyr Gly
                405                 410                 415
Asn Gly Pro Gly Tyr Val Leu Lys Asp Gly Ala Arg Pro Asp Val Thr
            420                 425                 430
Glu Ser Glu Ser Gly Ser Pro Glu Tyr Arg Gln Gln Ser Ala Val Pro
        435                 440                 445
Leu Asp Glu Glu Thr His Ala Gly Glu Asp Val Ala Val Phe Ala Arg
    450                 455                 460
Gly Pro Gln Ala His Leu Val His Gly Val Gln Glu Gln Thr Phe Ile
465                 470                 475                 480
Ala His Val Met Ala Phe Ala Ala Cys Leu Glu Pro Tyr Thr Ala Cys
                485                 490                 495
Asp Leu Ala Pro Pro Ala Gly Thr Thr Asp Ala Ala His Pro Gly Arg
            500                 505                 510
Ser Val Val Pro Ala Leu Leu Pro Leu Leu Ala Gly Thr Leu Leu Leu

        515                 520                 525

Leu Glu Thr Ala Thr Ala Pro

    530                 535

<210> 130

<211> 461

<212> PRT

<213> Homo sapiens

<400> 130

Met Asn Asn Phe Gly Asn Glu Glu Phe Asp Cys His Phe Leu Asp Glu

1               5                   10                  15

Gly Phe Thr Ala Lys Asp Ile Leu Asp Gln Lys Ile Asn Glu Val Ser

            20                  25                  30

Ser Ser Asp Asp Lys Asp Ala Phe Tyr Val Ala Asp Leu Gly Asp Ile

        35                  40                  45

Leu Lys Lys His Leu Arg Trp Leu Lys Ala Leu Pro Arg Val Thr Pro

    50                  55                  60

Phe Tyr Ala Val Lys Cys Asn Asp Ser Lys Ala Ile Val Lys Thr Leu

65                  70                  75                  80

Ala Ala Thr Gly Thr Gly Phe Asp Cys Ala Ser Lys Thr Glu Ile Gln

                85                  90                  95

Leu Val Gln Ser Leu Gly Val Pro Pro Glu Arg Ile Ile Tyr Ala Asn

            100                 105                 110

Pro Cys Lys Gln Val Ser Gln Ile Lys Tyr Ala Ala Asn Asn Gly Val

        115                 120                 125

Gln Met Met Thr Phe Asp Ser Glu Val Glu Leu Met Lys Val Ala Arg

    130                 135                 140

Ala His Pro Lys Ala Lys Leu Val Leu Arg Ile Ala Thr Asp Asp Ser
145                 150                 155                 160
Lys Ala Val Cys Arg Leu Ser Val Lys Phe Gly Ala Thr Leu Arg Thr
                165                 170                 175
Ser Arg Leu Leu Leu Glu Arg Ala Lys Glu Leu Asn Ile Asp Val Val
            180                 185                 190
Gly Val Ser Phe His Val Gly Ser Gly Cys Thr Asp Pro Glu Thr Phe
        195                 200                 205
Val Gln Ala Ile Ser Asp Ala Arg Cys Val Phe Asp Met Gly Ala Glu
    210                 215                 220
Val Gly Phe Ser Met Tyr Leu Leu Asp Ile Gly Gly Gly Phe Pro Gly
225                 230                 235                 240
Ser Glu Asp Val Lys Leu Lys Phe Glu Glu Ile Thr Gly Val Ile Asn
                245                 250                 255
Pro Ala Leu Asp Lys Tyr Phe Pro Ser Asp Ser Gly Val Arg Ile Ile
            260                 265                 270
Ala Glu Pro Gly Arg Tyr Tyr Val Ala Ser Ala Phe Thr Leu Ala Val
        275                 280                 285
Asn Ile Ile Ala Lys Lys Ile Val Leu Lys Glu Gln Thr Gly Ser Asp
    290                 295                 300
Asp Glu Asp Glu Ser Ser Glu Gln Thr Phe Met Tyr Tyr Val Asn Asp
305                 310                 315                 320
Gly Val Tyr Gly Ser Phe Asn Cys Ile Leu Tyr Asp His Ala His Val
                325                 330                 335
Lys Pro Leu Leu Gln Lys Arg Pro Lys Pro Asp Glu Lys Tyr Tyr Ser
            340                 345                 350
Ser Ser Ile Trp Gly Pro Thr Cys Asp Gly Leu Asp Arg Ile Val Glu
        355                 360                 365
Arg Cys Asp Leu Pro Glu Met His Val Gly Asp Trp Met Leu Phe Glu

370                 375                 380

Asn Met Gly Ala Tyr Thr Val Ala Ala Ala Ser Thr Phe Asn Gly Phe

385                 390                 395                 400

Gln Arg Pro Thr Ile Tyr Tyr Val Met Ser Gly Pro Ala Trp Gln Leu

                405                 410                 415

Met Gln Gln Phe Gln Asn Pro Asp Phe Pro Pro Glu Val Glu Glu Gln

            420                 425                 430

Asp Ala Ser Thr Leu Pro Val Ser Cys Ala Trp Glu Ser Gly Met Lys

        435                 440                 445

Arg His Arg Ala Ala Cys Ala Ser Ala Ser Ile Asn Val

    450                 455                 460

<210> 131

<211> 1148

<212> PRT

<213> Homo sapiens

<400> 131

Met Pro Leu Phe Lys Asn Thr Ser Val Ser Ser Leu Tyr Ser Gly Cys

 1               5                  10                  15

Arg Leu Thr Leu Leu Arg Pro Glu Lys Asp Gly Ala Ala Thr Arg Val

            20                  25                  30

Asp Ala Val Cys Thr His Arg Pro Asp Pro Lys Ser Pro Gly Leu Asp

        35                  40                  45

Arg Glu Arg Leu Tyr Trp Lys Leu Ser Gln Leu Thr His Gly Ile Thr

    50                  55                  60

Glu Leu Gly Pro Tyr Thr Leu Asp Arg His Ser Leu Tyr Val Asn Gly

65                  70                  75                  80

Phe Thr His Gln Ser Ser Met Thr Thr Thr Arg Thr Pro Asp Thr Ser
                85                  90                  95
Thr Met His Leu Ala Thr Ser Arg Thr Pro Ala Ser Leu Ser Gly Pro
            100                 105                 110
Thr Thr Ala Ser Pro Leu Leu Val Leu Phe Thr Ile Asn Phe Thr Ile
        115                 120                 125
Thr Asn Leu Arg Tyr Glu Glu Asn Met His His Pro Gly Ser Arg Lys
    130                 135                 140
Phe Asn Thr Thr Glu Arg Val Leu Gln Gly Leu Leu Arg Pro Val Phe
145                 150                 155                 160
Lys Asn Thr Ser Val Gly Pro Leu Tyr Ser Gly Cys Arg Leu Thr Leu
                165                 170                 175
Leu Arg Pro Lys Lys Asp Gly Ala Ala Thr Lys Val Asp Ala Ile Cys
            180                 185                 190
Thr Tyr Arg Pro Asp Pro Lys Ser Pro Gly Leu Asp Arg Glu Gln Leu
        195                 200                 205
Tyr Trp Glu Leu Ser Gln Leu Thr His Ser Ile Thr Glu Leu Gly Pro
    210                 215                 220
Tyr Thr Leu Asp Arg Asp Ser Leu Tyr Val Asn Gly Phe Thr Gln Arg
225                 230                 235                 240
Ser Ser Val Pro Thr Thr Ser Ile Pro Gly Thr Pro Thr Val Asp Leu
                245                 250                 255
Gly Thr Ser Gly Thr Pro Val Ser Lys Pro Gly Pro Ser Ala Ala Ser
            260                 265                 270
Pro Leu Leu Val Leu Phe Thr Leu Asn Phe Thr Ile Thr Asn Leu Arg
        275                 280                 285
Tyr Glu Glu Asn Met Gln His Pro Gly Ser Arg Lys Phe Asn Thr Thr
    290                 295                 300
Glu Arg Val Leu Gln Gly Leu Leu Arg Ser Leu Phe Lys Ser Thr Ser

305                 310                 315                 320

Val Gly Pro Leu Tyr Ser Gly Cys Arg Leu Thr Leu Leu Arg Pro Glu

                325                 330                 335

Lys Asp Gly Thr Ala Thr Gly Val Asp Ala Ile Cys Thr His His Pro

            340                 345                 350

Asp Pro Lys Ser Pro Arg Leu Asp Arg Glu Gln Leu Tyr Trp Glu Leu

        355                 360                 365

Ser Gln Leu Thr His Asn Ile Thr Glu Leu Gly His Tyr Ala Leu Asp

    370                 375                 380

Asn Asp Ser Leu Phe Val Asn Gly Phe Thr His Arg Ser Ser Val Ser

385                 390                 395                 400

Thr Thr Ser Thr Pro Gly Thr Pro Thr Val Tyr Leu Gly Ala Ser Lys

                405                 410                 415

Thr Pro Ala Ser Ile Phe Gly Pro Ser Ala Ala Ser His Leu Leu Ile

            420                 425                 430

Leu Phe Thr Leu Asn Phe Thr Ile Thr Asn Leu Arg Tyr Glu Glu Asn

        435                 440                 445

Met Trp Pro Gly Ser Arg Lys Phe Asn Thr Thr Glu Arg Val Leu Gln

    450                 455                 460

Gly Leu Leu Arg Pro Leu Phe Lys Asn Thr Ser Val Gly Pro Leu Tyr

465                 470                 475                 480

Ser Gly Ser Arg Leu Thr Leu Leu Arg Pro Glu Lys Asp Gly Glu Ala

                485                 490                 495

Thr Gly Val Asp Ala Ile Cys Thr His Arg Pro Asp Pro Thr Gly Pro

            500                 505                 510

Gly Leu Asp Arg Glu Gln Leu Tyr Leu Glu Leu Ser Gln Leu Thr His

        515                 520                 525

Ser Ile Thr Glu Leu Gly Pro Tyr Thr Leu Asp Arg Asp Ser Leu Tyr

    530                 535                 540

Val Asn Gly Phe Thr His Arg Ser Ser Val Pro Thr Thr Ser Thr Gly
545                 550                 555                 560
Val Val Ser Glu Glu Pro Phe Thr Leu Asn Phe Thr Ile Asn Asn Leu
                565                 570                 575
Arg Tyr Met Ala Asp Met Gly Gln Pro Gly Ser Leu Lys Phe Asn Ile
            580                 585                 590
Thr Asp Asn Val Met Lys His Leu Leu Ser Pro Leu Phe Gln Arg Ser
        595                 600                 605
Ser Leu Gly Ala Arg Tyr Thr Gly Cys Arg Val Ile Ala Leu Arg Ser
    610                 615                 620
Val Lys Asn Gly Ala Glu Thr Arg Val Asp Leu Leu Cys Thr Tyr Leu
625                 630                 635                 640
Gln Pro Leu Ser Gly Pro Gly Leu Pro Ile Lys Gln Val Phe His Glu
                645                 650                 655
Leu Ser Gln Gln Thr His Gly Ile Thr Arg Leu Gly Pro Tyr Ser Leu
            660                 665                 670
Asp Lys Asp Ser Leu Tyr Leu Asn Gly Tyr Asn Glu Pro Gly Leu Asp
        675                 680                 685
Glu Pro Pro Thr Thr Pro Lys Pro Ala Thr Thr Phe Leu Pro Pro Leu
    690                 695                 700
Ser Glu Ala Thr Thr Ala Met Gly Tyr His Leu Lys Thr Leu Thr Leu
705                 710                 715                 720
Asn Phe Thr Ile Ser Asn Leu Gln Tyr Ser Pro Asp Met Gly Lys Gly
                725                 730                 735
Ser Ala Thr Phe Asn Ser Thr Glu Gly Val Leu Gln His Leu Leu Arg
            740                 745                 750
Pro Leu Phe Gln Lys Ser Ser Met Gly Pro Phe Tyr Leu Gly Cys Gln
        755                 760                 765
Leu Ile Ser Leu Arg Pro Glu Lys Asp Gly Ala Ala Thr Gly Val Asp

    770                 775                 780

Thr Thr Cys Thr Tyr His Pro Asp Pro Val Gly Pro Gly Leu Asp Ile

785                 790                 795                 800

Gln Gln Leu Tyr Trp Glu Leu Ser Gln Leu Thr His Gly Val Thr Gln

                805                 810                 815

Leu Gly Phe Tyr Val Leu Asp Arg Asp Ser Leu Phe Ile Asn Gly Tyr

            820                 825                 830

Ala Pro Gln Asn Leu Ser Ile Arg Gly Glu Tyr Gln Ile Asn Phe His

        835                 840                 845

Ile Val Asn Trp Asn Leu Ser Asn Pro Asp Pro Thr Ser Ser Glu Tyr

    850                 855                 860

Ile Thr Leu Leu Arg Asp Ile Gln Asp Lys Val Thr Thr Leu Tyr Lys

865                 870                 875                 880

Gly Ser Gln Leu His Asp Thr Phe Arg Phe Cys Leu Val Thr Asn Leu

                885                 890                 895

Thr Met Asp Ser Val Leu Val Thr Val Lys Ala Leu Phe Ser Ser Asn

            900                 905                 910

Leu Asp Pro Ser Leu Val Glu Gln Val Phe Leu Asp Lys Thr Leu Asn

        915                 920                 925

Ala Ser Phe His Trp Leu Gly Ser Thr Tyr Gln Leu Val Asp Ile His

    930                 935                 940

Val Thr Glu Met Glu Ser Ser Val Tyr Gln Pro Thr Ser Ser Ser Ser

945                 950                 955                 960

Thr Gln His Phe Tyr Pro Asn Phe Thr Ile Thr Asn Leu Pro Tyr Ser

                965                 970                 975

Gln Asp Lys Ala Gln Pro Gly Thr Thr Asn Tyr Gln Arg Asn Lys Arg

            980                 985                 990

Asn Ile Glu Asp Ala Leu Asn Gln Leu Phe Arg Asn Ser Ser Ile Lys

        995                 1000                1005

Ser Tyr Phe Ser Asp Cys Gln Val Ser Thr Phe Arg Ser Val Pro Asn
    1010                1015                1020
Arg His His Thr Gly Val Asp Ser Leu Cys Asn Phe Ser Pro Leu Ala
1025                1030                1035                1040
Arg Arg Val Asp Arg Val Ala Ile Tyr Glu Glu Phe Leu Arg Met Thr
                1045                1050                1055
Arg Asn Gly Thr Gln Leu Gln Asn Phe Thr Leu Asp Arg Ser Ser Val
            1060                1065                1070
Leu Val Asp Gly Tyr Ser Pro Asn Arg Asn Glu Pro Leu Thr Gly Asn
        1075                1080                1085
Ser Asp Leu Pro Phe Trp Ala Val Ile Phe Ile Gly Leu Ala Gly Leu
    1090                1095                1100
Leu Gly Leu Ile Thr Cys Leu Ile Cys Gly Val Leu Val Thr Thr Arg
1105                1110                1115                1120
Arg Arg Lys Lys Glu Gly Glu Tyr Asn Val Gln Gln Gln Cys Pro Gly
                1125                1130                1135
Tyr Tyr Gln Ser His Leu Asp Leu Glu Asp Leu Gln
            1140                1145

<210> 132
<211> 526
<212> PRT
<213> Homo sapiens

<400> 132
Met Gly His Leu Ser Ala Pro Leu His Arg Val Arg Val Pro Trp Gln
1               5                   10                  15
Gly Leu Leu Leu Thr Ala Ser Leu Leu Thr Phe Trp Asn Pro Pro Thr

            20                  25                  30

Thr Ala Gln Leu Thr Thr Glu Ser Met Pro Phe Asn Val Ala Glu Gly

        35                  40                  45

Lys Glu Val Leu Leu Leu Val His Asn Leu Pro Gln Gln Leu Phe Gly

    50                  55                  60

Tyr Ser Trp Tyr Lys Gly Glu Arg Val Asp Gly Asn Arg Gln Ile Val

65                  70                  75                  80

Gly Tyr Ala Ile Gly Thr Gln Gln Ala Thr Pro Gly Pro Ala Asn Ser

                85                  90                  95

Gly Arg Glu Thr Ile Tyr Pro Asn Ala Ser Leu Leu Ile Gln Asn Val

            100                 105                 110

Thr Gln Asn Asp Thr Gly Phe Tyr Thr Leu Gln Val Ile Lys Ser Asp

        115                 120                 125

Leu Val Asn Glu Glu Ala Thr Gly Gln Phe His Val Tyr Pro Glu Leu

    130                 135                 140

Pro Lys Pro Ser Ile Ser Ser Asn Asn Ser Asn Pro Val Glu Asp Lys

145                 150                 155                 160

Asp Ala Val Ala Phe Thr Cys Glu Pro Glu Thr Gln Asp Thr Thr Tyr

                165                 170                 175

Leu Trp Trp Ile Asn Asn Gln Ser Leu Pro Val Ser Pro Arg Leu Gln

            180                 185                 190

Leu Ser Asn Gly Asn Arg Thr Leu Thr Leu Leu Ser Val Thr Arg Asn

        195                 200                 205

Asp Thr Gly Pro Tyr Glu Cys Glu Ile Gln Asn Pro Val Ser Ala Asn

    210                 215                 220

Arg Ser Asp Pro Val Thr Leu Asn Val Thr Tyr Gly Pro Asp Thr Pro

225                 230                 235                 240

Thr Ile Ser Pro Ser Asp Thr Tyr Tyr Arg Pro Gly Ala Asn Leu Ser

                245                 250                 255

Leu Ser Cys Tyr Ala Ala Ser Asn Pro Pro Ala Gln Tyr Ser Trp Leu
            260                 265                 270
Ile Asn Gly Thr Phe Gln Gln Ser Thr Gln Glu Leu Phe Ile Pro Asn
        275                 280                 285
Ile Thr Val Asn Asn Ser Gly Ser Tyr Thr Cys His Ala Asn Asn Ser
    290                 295                 300
Val Thr Gly Cys Asn Arg Thr Thr Val Lys Thr Ile Ile Val Thr Glu
305                 310                 315                 320
Leu Ser Pro Val Val Ala Lys Pro Gln Ile Lys Ala Ser Lys Thr Thr
                325                 330                 335
Val Thr Gly Asp Lys Asp Ser Val Asn Leu Thr Cys Ser Thr Asn Asp
            340                 345                 350
Thr Gly Ile Ser Ile Arg Trp Phe Phe Lys Asn Gln Ser Leu Pro Ser
        355                 360                 365
Ser Glu Arg Met Lys Leu Ser Gln Gly Asn Thr Thr Leu Ser Ile Asn
    370                 375                 380
Pro Val Lys Arg Glu Asp Ala Gly Thr Tyr Trp Cys Glu Val Phe Asn
385                 390                 395                 400
Pro Ile Ser Lys Asn Gln Ser Asp Pro Ile Met Leu Asn Val Asn Tyr
                405                 410                 415
Asn Ala Leu Pro Gln Glu Asn Gly Leu Ser Pro Gly Ala Ile Ala Gly
            420                 425                 430
Ile Val Ile Gly Val Val Ala Leu Val Ala Leu Ile Ala Val Ala Leu
        435                 440                 445
Ala Cys Phe Leu His Phe Gly Lys Thr Gly Arg Ala Ser Asp Gln Arg
    450                 455                 460
Asp Leu Thr Glu His Lys Pro Ser Val Ser Asn His Thr Gln Asp His
465                 470                 475                 480
Ser Asn Asp Pro Pro Asn Lys Met Asn Glu Val Thr Tyr Ser Thr Leu

485                    490                    495

Asn Phe Glu Ala Gln Gln Pro Thr Gln Pro Thr Ser Ala Ser Pro Ser

            500                    505                    510

Leu Thr Ala Thr Glu Ile Ile Tyr Ser Glu Val Lys Lys Gln

        515                    520                    525

<210> 133

<211> 212

<212> PRT

<213> Homo sapiens

<400> 133

Met Gly Pro Pro Ser Ala Pro Pro His Arg Glu Cys Ile Pro Trp Gln

 1               5                   10                  15

Gly Leu Leu Leu Thr Ala Ser Leu Leu Asn Phe Trp Asn Pro Pro Thr

            20                  25                  30

Thr Ala Lys Leu Thr Ile Glu Ser Met Pro Leu Ser Val Ala Glu Gly

        35                  40                  45

Lys Glu Val Leu Leu Leu Val His Asn Leu Pro Gln His Leu Phe Gly

    50                  55                  60

Tyr Ser Trp Tyr Lys Gly Glu Arg Val Asp Gly Asn Ser Leu Ile Val

65                  70                  75                  80

Gly Tyr Val Ile Gly Thr Gln Gln Ala Thr Pro Gly Ala Ala Tyr Ser

                85                  90                  95

Gly Arg Glu Thr Ile Tyr Thr Asn Ala Ser Leu Leu Ile Gln Asn Val

            100                 105                 110

Thr Gln Asn Asp Ile Gly Phe Tyr Thr Leu Gln Val Ile Lys Ser Asp

        115                 120                 125

Leu Val Asn Glu Glu Ala Thr Gly Gln Phe His Val Tyr Gln Glu Asn
    130                 135                 140
Ala Pro Gly Leu Pro Val Gly Ala Val Ala Gly Ile Val Thr Gly Val
145                 150                 155                 160
Leu Val Gly Val Ala Leu Val Ala Ala Leu Val Cys Phe Leu Leu Leu
                165                 170                 175
Ala Lys Thr Gly Arg Pro Trp Ser Leu Pro Gln Leu Cys Leu Leu Asp
            180                 185                 190
Val Pro Ser Leu His Cys Leu Gly Pro Pro Thr Gln Pro Gln Asp Ser
        195                 200                 205
Ser Phe His Leu
    210

<210> 134
<211> 244
<212> PRT
<213> Homo sapiens

<400> 134
Met Gly Pro Pro Ser Ala Ala Pro Arg Gly Gly His Arg Pro Trp Gln
1               5                   10                  15
Gly Leu Leu Ile Thr Ala Ser Leu Leu Thr Phe Trp Asp Pro Pro Thr
            20                  25                  30
Thr Val Gln Phe Thr Ile Glu Ala Leu Pro Ser Ser Ala Ala Glu Gly
        35                  40                  45
Lys Asp Val Leu Leu Leu Ala Cys Asn Ile Ser Glu Thr Ile Gln Ala
    50                  55                  60
Tyr Tyr Trp His Lys Gly Lys Thr Ala Glu Gly Ser Pro Leu Ile Ala

65                  70                  75                  80

Gly Tyr Ile Thr Asp Ile Gln Ala Asn Ile Pro Gly Ala Ala Tyr Ser

                85                  90                  95

Gly Arg Glu Gln Val Tyr Pro Asn Gly Ser Leu Leu Phe Gln Asn Ile

            100                 105                 110

Thr Leu Glu Asp Ala Gly Ser Tyr Thr Leu Arg Thr Ile Asn Ala Ser

        115                 120                 125

Tyr Asp Ser Asp Gln Ala Thr Gly Gln Leu His Val His Gln Asn Asn

    130                 135                 140

Val Pro Gly Leu Pro Val Gly Ala Val Ala Gly Ile Val Thr Gly Val

145                 150                 155                 160

Leu Val Gly Val Ala Leu Val Ala Ala Leu Val Cys Phe Leu Leu Leu

                165                 170                 175

Ser Arg Thr Gly Arg Ala Ser Ile Gln Arg Asp Leu Arg Glu Gln Pro

            180                 185                 190

Pro Pro Ala Ser Thr Pro Gly His Gly Pro Ser His Arg Ser Thr Phe

        195                 200                 205

Ser Ala Pro Leu Pro Ser Pro Arg Thr Ala Thr Pro Ile Tyr Val Glu

    210                 215                 220

Phe Leu Tyr Ser Asp Ala Asn Ile Tyr Cys Gln Ile Asp His Lys Ala

225                 230                 235                 240

Asp Val Val Ser

<210> 135

<211> 344

<212> PRT

<213> Homo sapiens

<400> 135

Met Gly Pro Pro Ser Ala Pro Pro Cys Arg Leu His Val Pro Trp Lys
1               5                   10                  15

Glu Val Leu Leu Thr Ala Ser Leu Leu Thr Phe Trp Asn Pro Pro Thr
            20                  25                  30

Thr Ala Lys Leu Thr Ile Glu Ser Thr Pro Phe Asn Val Ala Glu Gly
        35                  40                  45

Lys Glu Val Leu Leu Leu Ala His Asn Leu Pro Gln Asn Arg Ile Gly
    50                  55                  60

Tyr Ser Trp Tyr Lys Gly Glu Arg Val Asp Gly Asn Ser Leu Ile Val
65                  70                  75                  80

Gly Tyr Val Ile Gly Thr Gln Gln Ala Thr Pro Gly Pro Ala Tyr Ser
                85                  90                  95

Gly Arg Glu Thr Ile Tyr Pro Asn Ala Ser Leu Leu Ile Gln Asn Val
            100                 105                 110

Thr Gln Asn Asp Thr Gly Phe Tyr Thr Leu Gln Val Ile Lys Ser Asp
        115                 120                 125

Leu Val Asn Glu Glu Ala Thr Gly Gln Phe His Val Tyr Pro Glu Leu
    130                 135                 140

Pro Lys Pro Ser Ile Ser Ser Asn Asn Ser Asn Pro Val Glu Asp Lys
145                 150                 155                 160

Asp Ala Val Ala Phe Thr Cys Glu Pro Glu Val Gln Asn Thr Thr Tyr
                165                 170                 175

Leu Trp Trp Val Asn Gly Gln Ser Leu Pro Val Ser Pro Arg Leu Gln
            180                 185                 190

Leu Ser Asn Gly Asn Met Thr Leu Thr Leu Leu Ser Val Lys Arg Asn
        195                 200                 205

Asp Ala Gly Ser Tyr Glu Cys Glu Ile Gln Asn Pro Ala Ser Ala Asn

210                 215                 220

Arg Ser Asp Pro Val Thr Leu Asn Val Leu Tyr Gly Pro Asp Val Pro

225                 230                 235                 240

Thr Ile Ser Pro Ser Lys Ala Asn Tyr Arg Pro Gly Glu Asn Leu Asn

                245                 250                 255

Leu Ser Cys His Ala Ala Ser Asn Pro Pro Ala Gln Tyr Ser Trp Phe

            260                 265                 270

Ile Asn Gly Thr Phe Gln Gln Ser Thr Gln Glu Leu Phe Ile Pro Asn

        275                 280                 285

Ile Thr Val Asn Asn Ser Gly Ser Tyr Met Cys Gln Ala His Asn Ser

    290                 295                 300

Ala Thr Gly Leu Asn Arg Thr Thr Val Thr Met Ile Thr Val Ser Gly

305                 310                 315                 320

Ser Ala Pro Val Leu Ser Ala Val Ala Thr Val Gly Ile Thr Ile Gly

                325                 330                 335

Val Leu Ala Arg Val Ala Leu Ile

            340

<210> 136

<211> 265

<212> PRT

<213> Homo sapiens

<400> 136

Met Gly Ser Pro Ser Ala Cys Pro Tyr Arg Val Cys Ile Pro Trp Gln

 1               5                  10                  15

Gly Leu Leu Leu Thr Ala Ser Leu Leu Thr Phe Trp Asn Leu Pro Asn

            20                  25                  30

Ser Ala Gln Thr Asn Ile Asp Val Val Pro Phe Asn Val Ala Glu Gly
        35                  40                  45
Lys Glu Val Leu Leu Val Val His Asn Glu Ser Gln Asn Leu Tyr Gly
    50                  55                  60
Tyr Asn Trp Tyr Lys Gly Glu Arg Val His Ala Asn Tyr Arg Ile Ile
65                  70                  75                  80
Gly Tyr Val Lys Asn Ile Ser Gln Glu Asn Ala Pro Gly Pro Ala His
                85                  90                  95
Asn Gly Arg Glu Thr Ile Tyr Pro Asn Gly Thr Leu Leu Ile Gln Asn
            100                 105                 110
Val Thr His Asn Asp Ala Gly Phe Tyr Thr Leu His Val Ile Lys Glu
        115                 120                 125
Asn Leu Val Asn Glu Glu Val Thr Arg Gln Phe Tyr Val Phe Ser Glu
    130                 135                 140
Pro Pro Lys Pro Ser Ile Thr Ser Asn Asn Phe Asn Pro Val Glu Asn
145                 150                 155                 160
Lys Asp Ile Val Val Leu Thr Cys Gln Pro Glu Thr Gln Asn Thr Thr
                165                 170                 175
Tyr Leu Trp Trp Val Asn Asn Gln Ser Leu Leu Val Ser Pro Arg Leu
            180                 185                 190
Leu Leu Ser Thr Asp Asn Arg Thr Leu Val Leu Leu Ser Ala Thr Lys
        195                 200                 205
Asn Asp Ile Gly Pro Tyr Glu Cys Glu Ile Gln Asn Pro Val Gly Ala
    210                 215                 220
Ser Arg Ser Asp Pro Val Thr Leu Asn Val Arg Tyr Glu Ser Val Gln
225                 230                 235                 240
Ala Ser Ser Pro Asp Leu Ser Ala Gly Thr Ala Val Ser Ile Met Ile
                245                 250                 255
Gly Val Leu Ala Gly Met Ala Leu Ile

            260                 265

<210> 137

<211> 349

<212> PRT

<213> Homo sapiens

<400> 137

Met Gly Pro Ile Ser Ala Pro Ser Cys Arg Trp Arg Ile Pro Trp Gln

1               5                   10                  15

Gly Leu Leu Leu Thr Ala Ser Leu Phe Thr Phe Trp Asn Pro Pro Thr

            20                  25                  30

Thr Ala Gln Leu Thr Ile Glu Ala Val Pro Ser Asn Ala Ala Glu Gly

        35                  40                  45

Lys Glu Val Leu Leu Leu Val His Asn Leu Pro Gln Asp Pro Arg Gly

    50                  55                  60

Tyr Asn Trp Tyr Lys Gly Glu Thr Val Asp Ala Asn Arg Arg Ile Ile

65                  70                  75                  80

Gly Tyr Val Ile Ser Asn Gln Gln Ile Thr Pro Gly Pro Ala Tyr Ser

                85                  90                  95

Asn Arg Glu Thr Ile Tyr Pro Asn Ala Ser Leu Leu Met Arg Asn Val

            100                 105                 110

Thr Arg Asn Asp Thr Gly Ser Tyr Thr Leu Gln Val Ile Lys Leu Asn

        115                 120                 125

Leu Met Ser Glu Glu Val Thr Gly Gln Phe Ser Val His Pro Glu Thr

    130                 135                 140

Pro Lys Pro Ser Ile Ser Ser Asn Asn Ser Asn Pro Val Glu Asp Lys

145                 150                 155                 160

Asp Ala Val Ala Phe Thr Cys Glu Pro Glu Thr Gln Asn Thr Thr Tyr
                165                 170                 175
Leu Trp Trp Val Asn Gly Gln Ser Leu Pro Val Ser Pro Arg Leu Gln
            180                 185                 190
Leu Ser Asn Gly Asn Arg Thr Leu Thr Leu Leu Ser Val Thr Arg Asn
        195                 200                 205
Asp Val Gly Pro Tyr Glu Cys Glu Ile Gln Asn Pro Ala Ser Ala Asn
    210                 215                 220
Phe Ser Asp Pro Val Thr Leu Asn Val Leu Tyr Gly Pro Asp Ala Pro
225                 230                 235                 240
Thr Ile Ser Pro Ser Asp Thr Tyr Tyr His Ala Gly Val Asn Leu Asn
                245                 250                 255
Leu Ser Cys His Ala Ala Ser Asn Pro Pro Ser Gln Tyr Ser Trp Ser
            260                 265                 270
Val Asn Gly Thr Phe Gln Gln Tyr Thr Gln Lys Leu Phe Ile Pro Asn
        275                 280                 285
Ile Thr Thr Lys Asn Ser Gly Ser Tyr Ala Cys His Thr Thr Asn Ser
    290                 295                 300
Ala Thr Gly Arg Asn Arg Thr Thr Val Arg Met Ile Thr Val Ser Asp
305                 310                 315                 320
Ala Leu Val Gln Gly Ser Ser Pro Gly Leu Ser Ala Arg Ala Thr Val
                325                 330                 335
Ser Ile Met Ile Gly Val Leu Ala Arg Val Ala Leu Ile
            340                 345

<210> 138
<211> 459
<212> PRT

<213> Homo sapiens

<400> 138

Met Ala Pro Leu Cys Pro Ser Pro Trp Leu Pro Leu Leu Ile Pro Ala
1               5                   10                  15

Pro Ala Pro Gly Leu Thr Val Gln Leu Leu Leu Ser Leu Leu Leu Leu
            20                  25                  30

Met Pro Val His Pro Gln Arg Leu Pro Arg Met Gln Glu Asp Ser Pro
        35                  40                  45

Leu Gly Gly Gly Ser Ser Gly Glu Asp Asp Pro Leu Gly Glu Glu Asp
    50                  55                  60

Leu Pro Ser Glu Glu Asp Ser Pro Arg Glu Glu Asp Pro Pro Gly Glu
65                  70                  75                  80

Glu Asp Leu Pro Gly Glu Glu Asp Leu Pro Gly Glu Glu Asp Leu Pro
                85                  90                  95

Glu Val Lys Pro Lys Ser Glu Glu Glu Gly Ser Leu Lys Leu Glu Asp
            100                 105                 110

Leu Pro Thr Val Glu Ala Pro Gly Asp Pro Gln Glu Pro Gln Asn Asn
        115                 120                 125

Ala His Arg Asp Lys Glu Gly Asp Asp Gln Ser His Trp Arg Tyr Gly
    130                 135                 140

Gly Asp Pro Pro Trp Pro Arg Val Ser Pro Ala Cys Ala Gly Arg Phe
145                 150                 155                 160

Gln Ser Pro Val Asp Ile Arg Pro Gln Leu Ala Ala Phe Cys Pro Ala
                165                 170                 175

Leu Arg Pro Leu Glu Leu Leu Gly Phe Gln Leu Pro Pro Leu Pro Glu
            180                 185                 190

Leu Arg Leu Arg Asn Asn Gly His Ser Val Gln Leu Thr Leu Pro Pro
        195                 200                 205

Gly Leu Glu Met Ala Leu Gly Pro Gly Arg Glu Tyr Arg Ala Leu Gln
    210                 215                 220
Leu His Leu His Trp Gly Ala Ala Gly Arg Pro Gly Ser Glu His Thr
225                 230                 235                 240
Val Glu Gly His Arg Phe Pro Ala Glu Ile His Val Val His Leu Ser
                245                 250                 255
Thr Ala Phe Ala Arg Val Asp Glu Ala Leu Gly Arg Pro Gly Gly Leu
            260                 265                 270
Ala Val Leu Ala Ala Phe Leu Glu Glu Gly Pro Glu Glu Asn Ser Ala
        275                 280                 285
Tyr Glu Gln Leu Leu Ser Arg Leu Glu Glu Ile Ala Glu Glu Gly Ser
    290                 295                 300
Glu Thr Gln Val Pro Gly Leu Asp Ile Ser Ala Leu Leu Pro Ser Asp
305                 310                 315                 320
Phe Ser Arg Tyr Phe Gln Tyr Glu Gly Ser Leu Thr Thr Pro Pro Cys
                325                 330                 335
Ala Gln Gly Val Ile Trp Thr Val Phe Asn Gln Thr Val Met Leu Ser
            340                 345                 350
Ala Lys Gln Leu His Thr Leu Ser Asp Thr Leu Trp Gly Pro Gly Asp
        355                 360                 365
Ser Arg Leu Gln Leu Asn Phe Arg Ala Thr Gln Pro Leu Asn Gly Arg
    370                 375                 380
Val Ile Glu Ala Ser Phe Pro Ala Gly Val Asp Ser Ser Pro Arg Ala
385                 390                 395                 400
Ala Glu Pro Val Gln Leu Asn Ser Cys Leu Ala Ala Gly Asp Ile Leu
                405                 410                 415
Ala Leu Val Phe Gly Leu Leu Phe Ala Val Thr Ser Val Ala Phe Leu
            420                 425                 430
Val Gln Met Arg Arg Gln His Arg Arg Gly Thr Lys Gly Gly Val Ser

        435                 440                 445

Tyr Arg Pro Ala Glu Val Ala Glu Thr Gly Ala

    450                 455

attactgtgt gtgagccaga gggagctgtg gtaagggttg ggcccccagc ctgtagggaa
4320

ctttctggac tcccactctt tgaatcgata taggcatttg gtctcactac ttgaccattc
4380

tcaccctgtg aaacgtccca cactttgaag caaatacaat tcacagcaca gtacacacaa
4440

aaaccttggc ataagacaga gaaggttctt cttattttgt gggctggttg ctgtagaaac
4500

atataacaaa gggcagccct ccacttctgg tataattgtg tagccccttt tctttgggct
4560

tgacacctgt cttgaataag agtgattaga gctgcataat gtccctctct tggctattga
4620

ccatgtggtt cacgtacaaa actctgtata agttgaagga aaatgttcat gttcatatgt
4680

acttgtttgc tatgactaca ttttgaggtt ttgtaaaact gttatttttt tttttttcac
4740

aatgtgaaac tgaaggtcaa taaattatta gagattttct cttcaaaaaa aaaaaaaaaa
4800

aaaaaaaaaa aaaaaaaaaa aaaaaaa
4827

<210> 15

<211> 1098

<212> DNA

<213> Homo sapiens

<400> 15
   cggcacgagg gtcccgcgcg ctcctccgac ccgctccgct ccgctccgct cggccccgcg 60
   ccgcccgtca acatgatccg ctgcggcctg gcctgcgagc gctgccgctg gatcctgccc
   120
   ctgctcctac tcagcgccat cgccttcgac atcatcgcgc tggccggccg cggctggttg
   180
   cagtctagcg accacggcca gacgtcctcg ctgtggtgga aatgctccca agagggcggc
   240
   ggcagcgggt cctacgagga gggctgtcag agcctcatgg agtacgcgtg gggtagagca
   300
   gcggctgcca tgctcttctg tggcttcatc atcctggtga tctgtttcat cctctccttc
   360
   ttcgccctct gtggacccca gatgcttgtc ttcctgagag tgattggagg tctccttgcc
   420
   ttggctgctg tgttccagat catctccctg gtaatttacc ccgtgaagta cacccagacc
   480
   ttcacccttc atgccaaccc tgctgtcact tacatctata actgggccta cggctttggg
   540
   tgggcagcca cgattatcct gattggctgt gccttcttct tctgctgcct cctcaactac
   600
   gaagatgacc ttctgggcaa tgccaagccc aggtacttct acacatctgc ctaacttggg
   660
   aatgaatgtg ggagaaaatc gctgctgctg agatggactc cagaagaaga aactgtttct
   720
   ccaggcgact ttgaacccat tttttggcag tgttcatatt attaaactag tcaaaaatgc
   780
   taaaataatt tgggagaaaa tattttttaa gtagtgttat agtttcatgt ttatctttta
   840
   ttatgttttg tgaagttgtg tcttttcact aattacctat actatgccaa tatttcctta
   900
   tatctatcca taacatttat actacatttg taagagaata tgcacgtgaa acttaacact
   960
   ttataaggta aaaatgaggt ttccaagatt taataatctg atcaagttct tgttatttcc
   1020
   aaatagaatg gactcggtct gttaagggct aaggagaaga ggaagataag gttaaaagtt
   1080
   gttaatgacc aaacattc
   1098

<210> 16

<211> 1381

<212> DNA

<213> Homo sapiens

gcatgctgtt gtacatgatc ctgacaagaa gaaaatgaag ctcaaagtca aaaaatctcg
1140

tgaaaaacgg gagtttggcc tctcatctca gtggatatat cctcccaaaa ggaaacaagg
1200

gcaaggctta tctttgtgtc aaaacggaga gtcacccaac tgtgtggaag acaagatgct
1260

ctcgacagtt gcagtactta cccttggcta agaactgcac tgctttgttt aaaggactgc
1320

agaccaagga gtcgagcttt ctctcagagc atgcttttct ttattaaaat tactgatgca
1380

g
1381

<210> 17

<211> 1978

<212> DNA

<213> Homo sapiens

<400> 17

gggagggtac ttagggccgg ggctggccca ggctacggcg gctgcagggc tccggcaacc 60

gctccggcaa cgccaaccgc tccgctgcgc gcaggctggg ctgcaggctc tcggctgcag
120

cgctgggtgg atctaggatc cggcttccaa catgtggcag ctctgggcct ccctctgctg
180

cctgctggtg ttggccaatg cccggagcag gccctctttc catcccctgt cggatgagct
240

ggtcaactat gtcaacaaac ggaataccac gtggcaggcc gggcacaact tctacaacgt
300

ggacatgagc tacttgaaga ggctatgtgg taccttcctg ggtgggccca agccacccca
360

gagagttatg tttaccgagg acctgaagct gcctgcaagc ttcgatgcac gggaacaatg
420

gccacagtgt cccaccatca aagagatcag agaccagggc tcctgtggct cctgctgggc
480

cttcggggct gtggaagcca tctctgaccg gatctgcatc cacaccaatg cgcacgtcag
540
cgtggaggtg tcggcggagg acctgctcac atgctgtggc agcatgtgtg gggacggctg
600
taatggtggc tatcctgctg aagcttggaa cttctggaca agaaaaggcc tggtttctgg
660
tggcctctat gaatcccatg tagggtgcag accgtactcc atccctccct gtgagcacca
720
cgtcaacggc tcccggcccc catgcacggg ggagggagat acccccaagt gtagcaagat
780
ctgtgagcct ggctacagcc cgacctacaa acaggacaag cactacggat acaattccta
840
cagcgtctcc aatagcgaga aggacatcat ggccgagatc tacaaaaacg gccccgtgga
900
gggagctttc tctgtgtatt cggacttcct gctctacaag tcaggagtgt accaacacgt
960
caccggagag atgatgggtg gccatgccat ccgcatcctg ggctggggag tggagaatgg
1020
cacaccctac tggctggttg ccaactcctg gaacactgac tggggtgaca atggcttctt
1080
taaaatactc agaggacagg atcactgtgg aatcgaatca gaagtggtgg ctggaattcc
1140
acgcaccgat cagtactggg aaaagatcta atctgccgtg ggcctgtcgt gccagtcctg
1200
ggggcgagat cggggtagaa atgcatttta ttctttaagt tcacgtaaga tacaagtttc
1260
agacagggtc tgaaggactg gattggccaa acatcagacc tgtcttccaa ggagaccaag
1320
tcctggctac atcccagcct gtggttacag tgcagacagg ccatgtgagc caccgctgcc
1380
agcacagagc gtccttcccc ctgtagacta gtgccgtagg gagtacctgc tgccccagct
1440
gactgtggcc ccctccgtga tccatccatc tccagggagc aagacagaga cgcaggaatg
1500
gaaagcggag ttcctaacag gatgaaagtt cccccatcag ttcccccagt acctccaagc
1560
aagtagcttt ccacatttgt cacagaaatc agaggagaga tggtgttggg agccctttgg
1620

agaacgccag tctcccaggc cccctgcatc tatcgagttt gcaatgtcac aacctctctg
1680

atcttgtgct cagcatgatt ctttaataga agttttattt tttcgtgcac tctgctaatc
1740

atgtgggtga gccagtggaa cagcgggaga cctgtgctag ttttacagat tgcctcctaa
1800

tgacgcggct caaaaggaaa ccaagtggtc aggagttgtt tctgacccac tgatctctac
1860

taccacaagg aaaatagttt aggagaaacc agcttttact gtttttgaaa aattacagct
1920

tcaccctgtc aagttaacaa ggaatgcctg tgccaataaa agttttctcc aacttgaa
1978

<210> 18

<211> 1074

<212> DNA

<213> Homo sapiens

<400> 18

ggtcaggaaa gctcaggcaa gcccaccctc aggcattaca gctagactcc gagcttactg 60

ggcagtcatc tgattcgacc aacatcagtt cgcagggctt aagcccagtc ccttacggcg
120

gctggggagg gaccaggccc aagtatataa agctccctga gggtccgcgt tggctttgcg
180

cctgtgagtg tgattcaaga acgtcccagt gcccttggct cctttcggag tgtgaccccg
240

tgcttgcacg ggacacgtta cccagctcgg gtgagaaggg tatcttccgg gaacctcgcc
300

tttaatagca caacgagcgc agagtccact ggatctgcga gaagaaaccg cgctaactag
360

tttgtcccta cggccgcctc gtagtcactg ccgcggcgcc ttgagtctcc gggccgcctt
420

gccatggctg cccgtggtgt catcgctcca gttggcgaga gtttgcgcta cgctgagtac
480

ttgcagccct cggccaaacg gccagacgcc gacgtcgacc agcagggact ggtaagaagt
540

ttgatagctg taggactggg tgttgcagct cttgcatttg caggtcgcta cgcatttcgg
600

atctggaaac ctctagaaca agttatcaca gaaactgcaa agaagatttc aactcctagc
660

ttttcatcct actataaagg aggatttgaa cagaaaatga gtaggcgaga agctggtctt
720

attttaggtg taagcccatc tgctggcaag gctaagatta gaacagctca taggagagtc
780

atgattttga atcacccaga taaaggtgga tctccttacg tagcagccaa aataaatgaa
840

gcaaaagact tgctagaaac aaccaccaaa cattgatgct taaggaccac actgaaggaa
900

aaaaaaagag gggacttcga aaaaaaaaaa agccctgcaa aatattctaa aacatggtct
960

tcttaatttt ctatatggat tgaccacagt cttatcttcc accattaagc tgtataacaa
1020

taaaatgtta atagtcttgc tttttattat cttttaaaga tctccttaaa ttct
1074

<210> 19

<211> 4098

<212> DNA

<213> Homo sapiens

<400> 19

ggtggcctct gtggccgtcc aggctagcgg cggcccgcag gcggcgggga gaaagactct 60

ctcacctggt cttgcggctg tggccaccgc cggccagggg tgtggagggc gtgctgccgg
120

agacgtccgc cgggctctgc agttccgccg ggggtcgggc agctatggag ccgcggccca
180

cggcgccctc ctccggcgcc ccgggactgg ccggggtcgg ggagacgccg tcagccgctg
240

cgctggccgc agccagggtg gaactgcccg gcacggctgt gccctcggtg ccggaggatg
300

ctgcgcccgc gagccgggac ggcggcgggg tccgcgatga gggccccgcg gcggccgggg
360

acgggctggg cagacccttg gggcccaccc cgagccagag ccgtttccag gtggacctgg
420

tttccgagaa cgccgggcgg gccgctgctg cggcggcggc ggcggcggcg gcagcggcgg
480

cggctggtgc tggggcgggg gccaagcaga cccccgcgga cggggaagcc agcggcgaga
540

gcgagccagc taaaggcagc gaggaagcca agggccgctt ccgcgtgaac ttcgtggacc
600

cagctgcctc ctcgtcggct gaagacagcc tgtcagatgc tgccggggtc ggagtcgacg
660

ggcccaacgt gagcttccag aacggcgggg acacggtgct gagcgagggc agcagcctgc
720

actccggcgg cggcggcggc agtgggcacc accagcacta ctattatgat acccacacca
780

acacctacta cctgcgcacc ttcggccaca acaccatgga cgctgtgccc aggatcgatc
840

actaccggca cacagccgcg cagctgggcg agaagctgct ccggcctagc ctggcggagc
900

tccacgacga gctggaaaag gaaccttttg aggatggctt tgcaaatggg gaagaaagta
960

ctccaaccag agatgctgtg gtcacgtata ctgcagaaag taaaggagtc gtgaagtttg
1020

gctggatcaa gggtgtatta gtacgttgta tgttaaacat ttggggtgtg atgcttttca
1080

ttagattgtc atggattgtg ggtcaagctg gaataggtct atcagtcctt gtaataatga
1140

tggccactgt tgtgacaact atcacaggat tgtctacttc agcaatagca actaatggat
1200

ttgtaagagg aggaggagca tattatttaa tatctagaag tctagggcca gaatttggtg
1260

gtgcaattgg tctaatcttc gcctttgcca acgctgttgc agttgctatg tatgtggttg
1320

gatttgcaga aaccgtggtg gagttgctta aggaacattc catacttatg atagatgaaa
1380

tcaatgatat ccgaattatt ggagccatta cagtcgtgat tcttttaggt atctcagtag
1440

ctggaatgga gtgggaagca aaagctcaga ttgttctttt ggtgatccta cttcttgcta
1500

ttggtgattt cgtcatagga acatttatcc cactggagag caagaagcca aaaggttttt
1560

ttggttataa atctgaaata tttaatgaga actttgggcc cgattttcga gaggaagaga
1620

ctttcttttc tgtatttgcc atcttttttc ctgctgcaac tggtattctg gctggagcaa
1680

atatctcagg tgatcttgca gatcctcagt cagccatacc caaaggaaca ctcctagcca
1740

ttttaattac tacattggtt tacgtaggaa ttgcagtatc tgtaggttct tgtgttgttc
1800

gagatgccac tggaaacgtt aatgacacta tcgtaacaga gctaacaaac tgtacttctg
1860

cagcctgcaa attaaacttt gatttttcat cttgtgaaag cagtccttgt tcctatggcc
1920

taatgaacaa cttccaggta atgagtatgg tgtcaggatt tacaccacta atttctgcag
1980

gtatattttc agccactctt tcttcagcat tagcatccct agtgagtgct cccaaaatat
2040

ttcaggctct atgtaaggac aacatctacc cagctttcca gatgtttgct aaaggttatg
2100

ggaaaaataa tgaacctctt cgtggctaca tcttaacatt cttaattgca cttggattca
2160

tcttaattgc tgaactgaat gttattgcac caattatctc aaacttcttc cttgcatcat
2220

atgcattgat caatttttca gtattccatg catcacttgc aaaatctcca ggatggcgtc
2280

ctgcattcaa atactacaac atgtggatat cacttcttgg agcaattctt tgttgcatag
2340

taatgttcgt cattaactgg tgggctgcat tgctaacata tgtgatagtc cttgggctgt
2400

atatttatgt tacctacaaa aaaccagatg tgaattgggg atcctctaca caagccctga
2460

cttacctgaa tgcactgcag cattcaattc gtctttctgg agtggaagac cacgtgaaaa
2520

ccagccccca ttggcgagtt tgagaaggtg tgcagcaatg acaacaagac cttcgactct
420

tcctgccact tctttgccac aaagtgcacc ctggagggca ccaagaaggg ccacaagctc
480

cacctggact acatcgggcc ttgcaaatac atcccccctt gcctggactc tgagctgacc
540

gaattccccc tgcgcatgcg ggactggctc aagaacgtcc tggtcaccct gtatgagagg
600

gatgaggaca acaaccttct gactgagaag cagaagctgc gggtgaagaa gatccatgag
660

aatgagaagc gcctggaggc aggagaccac cccgtggagc tgctggcccg ggacttcgag
720

aagaactata acatgtacat cttccctgta cactggcagt tcggccagct ggaccagcac
780

cccattgacg ggtacctctc ccacaccgag ctggctccac tgcgtgctcc cctcatcccc
840

atggagcatt gcaccacccg ctttttcgag acctgtgacc tggacaatga caagtacatc
900

gccctggatg agtgggccgg ctgcttcggc atcaagcaga aggatatcga caaggatctt
960

gtgatctaaa tccactcctt ccacagtacc ggattctctc tttaaccctc cccttcgtgt
1020

ttcccccaat gtttaaaatg tttggatggt ttgttgttct gcctggagac aaggtgctaa
1080

catagattta agtgaataca ttaacggtgc taaaaatgaa aattctaacc caagacatga
1140

cattcttagc tgtaacttaa ctattaaggc cttttccaca cgcattaata gtcccatttt
1200

tctcttgcca tttgtagctt tgcccattgt cttattggca catgggtgga cacggatctg
1260

ctgggctctg ccttaaacac acattgcagc ttcaactttt ctctttagtg ttctgtttga
1320

aactaatact taccgagtca gactttgtgt tcatttcatt tcagggtctt ggctgcctgt
1380

gggcttcccc aggtggcctg gaggtgggca aagggaagta acagacacac gatgttgtca
1440

aggatggttt tgggactaga ggctcagtgg tgggagagat ccctgcagaa tccaccaacc
1500

agaacgtggt ttgcctgagg ctgtaactga gagaaagatt ctggggctgt cttatgaaaa
1560

tatagacatt ctcacataag cccagttcat caccatttcc tcctttacct ttcagtgcag
1620

tttcttttca cattaggctg ttggttcaaa cttttgggag cacggactgt cagttctctg
1680

ggaagtggtc agcgcatcct gcagggcttc tcctcctctg tcttttggag aaccagggct
1740

cttctcaggg gctctaggga ctgccaggct gtttcagcca ggaaggccaa aatcaagagt
1800

gagatgtaga aagttgtaaa atagaaaaag tggagttggt gaatcggttg ttctttcctc
1860

acatttggat gattgtcata aggtttttag catgttcctc cttttcttca ccctcccctt
1920

tgttcttcta ttaatcaaga gaaacttcaa agttaatggg atggtcggat ctcacaggct
1980

gagaactcgt tcacctccaa gcatttcatg aaaaagctgc ttcttattaa tcatacaaac
2040

tctcaccatg atgtgaagag tttcacaaat ctttcaaaat aaaaagtaat gacttagaaa
2100

ctgaaaaaaa aaaaaaaaaa aaaaaaaaaa aaa
2133

<210> 26

<211> 2691

<212> DNA

<213> Homo sapiens

<400> 26

gcttgcccgt cggtcgctag ctcgctcggt gcgcgtcgtc ccgctccatg gcgctcttcg 60

tgcggctgct ggctctcgcc ctggctctgg ccctgggccc cgccgcgacc ctggcgggtc
120

ccgccaagtc gccctaccag ctggtgctgc agcacagcag gctccggggc cgccagcacg
180

gggtcagcac tacaacatct ccccccagga tttggagact gtatttcccc atggccttcc
420

tcctcgcttt gtgatgcagg tgaagacatt cagtgaagct tgcctgatgg taaggaaacc
480

agccctagaa cttctgcatt acctgaaaaa caccagtttt gcttatccag ctatacgata
540

tcttctgtat ggagagaagg gaacaggaaa aaccctaagt ctttgccatg ttattcattt
600

ctgtgcaaaa caggactggc tgatactaca tattccagat gctcatcttt gggtgaaaaa
660

ttgtcgggat cttctgcagt ccagctacaa caaacagcgc tttgatcaac ctttagaggc
720

ttcaacctgg ctgaagaatt tcaaaactac aaatgagcgc ttcctgaacc agataaaagt
780

tcaagagaag tatgtctgga ataagagaga aagcactgag aaagggagtc ctctgggaga
840

agtggttgaa cagggcataa cacgggtgag gaacgccaca gatgcagttg gaattgtgct
900

gaaagagcta aagaggcaaa gttctttggg tatgtttcac ctcctagtgg ccgtggatgg
960

aatcaatgct ctttggggaa gaaccactct gaaaagagaa gataaaagcc cgattgcccc
1020

cgaggaatta gcacttgttc acaacttgag gaaaatgatg aaaaatgatt ggcatggagg
1080

cgccattgtg tcggctttga gccagactgg gtctctcttt aagccccgga aagcctatct
1140

gccccaggag ttgctgggaa aggaaggatt tgatgccctg gatcccttta ttcccatcct
1200

ggtttccaac tataacccaa aggaatttga aagttgtatt cagtattatt tggaaaacaa
1260

ttggcttcaa catgagaaag ctcctacaga agaagggaaa aaagagctgc tgttcctaag
1320

taacgcgaac ccctcgctgc tggagcggca ctgtgcctac ctctaagcca agatcacagc
1380

atgtgaggaa gacagtggac atctgcttta tgctggaccc agtaagatga ggaagtcggg
1440

cagtacacag gaagaggagc caggcccttg tacctatggg attggacagg actgcagttg
1500

tggaggcttg caggcaagac ctctgcttct gtgaagacac cgacctgctc agctgcgtct
960

gccacaccct tgccgagtac tcccggcagt gcacccatgc aggggggttg ccccaggact
1020

ggcggggccc tgacttctgc ccccagaagt gccccaacaa catgcagtac cacgagtgcc
1080

gctccccctg cgcagacacc tgctccaacc aggagcactc ccgggcctgt gaggaccact
1140

gtgtggccgg ctgcttctgc cctgagggga cggtgcttga cgacatcggc cagaccggct
1200

gtgtccctgt gtcaaagtgt gcctgcgtct acaacggggc tgcctatgcc ccaggggcca
1260

cctactccac agactgcacc aactgcacct gctccggagg ccggtggagc tgccaggaag
1320

ttccatgccc gggtacctgc tctgtgcttg gaggtgccca cttctcaacg tttgacggga
1380

agcaatacac ggtgcacggc gactgcagct atgtgctgac caagccctgt gacagcagtg
1440

ccttcactgt actggctgag ctgcgcaggt gcgggctgac ggacagcgag acctgcctga
1500

agagcgtgac actgagcctg gatggggcgc agacggtggt ggtgatcaag gccagtgggg
1560

aagtgttcct gaaccagatc tacacccagc tgcccatctc tgcagccaac gtcaccatct
1620

tcagaccctc aaccttcttc atcatcgccc agaccagcct gggcctgcag ctgaacctgc
1680

agctggtgcc caccatgcag ctgttcatgc agctggcgcc caagctccgt gggcagacct
1740

gcggtctctg tgggaacttc aacagcatcc aggccgatga cttccggacc ctcagtgggg
1800

tggtggaggc caccgctgcg gccttcttca acaccttcaa gacccaggcc gcctgcccca
1860

acatcaggaa cagcttcgag gacccctgct ctctgagcgt ggagaatgag aagtatgctc
1920

agcactggtg ctcgcagctg accgatgccg acggcccctt cggccggtgc catgctgccg
1980

tgaagccggg aacctactac tcgaactgca tgtttgacac ctgcaactgt gagcggagcg
2040

aggactgcct gtgcgccgcg ctgtcctcct acgtgcacgc ctgtgccgcc aagggcgtgc
2100

agctcggcgg ctggagggac ggcgtctgca cgaagcctat gaccacttgc cccaagtcaa
2160

tgacgtacca ctaccatgtc agcacctgcc agcccacctg ccgctccctg agcgaggggg
2220

acatcacctg cagtgttggc ttcatccccg tggatggctg catctgtccc aagggcacct
2280

tcctggacga cacgggcaag tgtgtgcagg ccagcaactg tccctgctac cacagaggct
2340

ccatgatccc caatggggag tcggtgcacg acagcggggc tatctgcacc tgcacacatg
2400

ggaagctgag ctgcatcgga ggccaagccc ccgccccagt gtgtgctgcg cccatggtgt
2460

tctttgactg ccgaaatgcc acgcccgggg acacaggggc tggctgtcag aagagctgcc
2520

acacactgga catgacctgt tacagccccc aatgtgtgcc tggctgcgtg tgccccgacg
2580

ggctggtggc ggacggcgag ggcggctgca tcactgcgga ggactgcccc tgcgtgcaca
2640

atgaggccag ctaccgggcc ggccagacca tccgggtggg ctgcaacacc tgcacctgtg
2700

acagcaggat gtggcggtgc acagatgacc cctgcctggc cacctgcgcc gtgtacgggg
2760

acggccacta cctcaccttc gacggacaga gctacagctt caacgaggag actgcgagta
2820

ctcgctggtg cagaaccgct gtggcgggaa agacagcacc caggactcct ttcgtgttgt
2880

caccgagaac gtcccctgcg gcaccacagg gaccacctgc tccaaggcca tcaagatttt
2940

cctgggggaa cttcgagctg aagctaagcc atgggaaggt ggaggtgatc gggacggacg
3000

agagccagga ggtgccatac accatccggc agatgggcat ctacctggtg gtggacaccg
3060

acattggcct ggtgctgctg tgggacaaga agaccagcat cttcatcaac ctcagccccg
3120

agttcaaggg cagggtctgc ggcctgtgtg ggaacttcga cgacatcgcc gttaatgact
3180

<212> DNA

<213> Homo sapiens

<400> 57

tccagcactt tgctcgggtc acggcctcct cctggctccc aggaccccac cataggcaga 60

ggcaggcctt cctacaccct actccctgtg cctccaggct cgactagtcc ctagcactcg
120

acgactgagt ctctgagatc acttcaccgt ggtctccgcc tcacccttgg cgctggacca
180

gtgagaggag agggctgggg cgctccgctg agccactcct gcgcccccct ggccttgtct
240

acctcttgcc ccccgaaggg ttagtgtcga gctcacccca gcatcctaca acctcctggt
300

ggccttgccg cccccacaac cccgaggtat aaagccaggt acacgaggca ggggacgcac
360

caaggatgga gatgttccag gggctgctgc tgttgctgct gctgagcatg ggcgggacat
420

gggcatccaa ggagccgctt cggccacggt gccgccccat caatgccacc ctggctgtgg
480

agaaggaggg ctgccccgtg tgcatcaccg tcaacaccac catctgtgcc ggctactgcc
540

ccaccatgac ccgcgtgctg cagggggtcc tgccggccct gcctcaggtg gtgtgcaact
600

accgcgatgt gcgcttcgag tccatccggc tccctggctg cccgcgcggc gtgaaccccg
660

tggtctccta cgccgtggct ctcagctgtc aatgtgcact ctgccgccgc agcaccactg
720

actgcggggg tcccaaggac caccccttga cctgtgatga cccccgcttc caggactcct
780

cttcctcaaa ggcccctccc cccagccttc caagtccatc ccgactcccg gggccctcgg
840

acaccccgat cctcccacaa taaaggcttc tcaatccgca
880

<210> 58

<210> 75

<211> 300

<212> PRT

<213> Homo sapiens

<400> 75

Met Arg Ile Ala Val Ile Cys Phe Cys Leu Leu Gly Ile Thr Cys Ala
1               5                   10                  15

Ile Pro Val Lys Gln Ala Asp Ser Gly Ser Ser Glu Glu Lys Gln Leu
            20                  25                  30

Tyr Asn Lys Tyr Pro Asp Ala Val Ala Thr Trp Leu Asn Pro Asp Pro
        35                  40                  45

Ser Gln Lys Gln Asn Leu Leu Ala Pro Gln Thr Leu Pro Ser Lys Ser
    50                  55                  60

Asn Glu Ser His Asp His Met Asp Asp Met Asp Asp Glu Asp Asp Asp
65                  70                  75                  80

Asp His Val Asp Ser Gln Asp Ser Ile Asp Ser Asn Asp Ser Asp Asp
                85                  90                  95

Val Asp Asp Thr Asp Asp Ser His Gln Ser Asp Glu Ser His His Ser
            100                 105                 110

Asp Glu Ser Asp Glu Leu Val Thr Asp Phe Pro Thr Asp Leu Pro Ala
        115                 120                 125

Thr Glu Val Phe Thr Pro Val Val Pro Thr Val Asp Thr Tyr Asp Gly
    130                 135                 140

Arg Gly Asp Ser Val Val Tyr Gly Leu Arg Ser Lys Ser Lys Lys Phe
145                 150                 155                 160

Arg Arg Pro Asp Ile Gln Tyr Pro Asp Ala Thr Asp Glu Asp Ile Thr

<210> 78

<211> 165

<212> PRT

<213> Homo sapiens

<400> 78

Met Ala Pro Asn Ala Ser Cys Leu Cys Val His Val Arg Ser Glu Glu
1               5                   10                  15

Trp Asp Leu Met Thr Phe Asp Ala Asn Pro Tyr Asp Ser Val Lys Lys
            20                  25                  30

Ile Lys Glu His Val Arg Ser Lys Thr Lys Val Pro Val Gln Asp Gln
        35                  40                  45

Val Leu Leu Leu Gly Ser Lys Ile Leu Lys Pro Arg Arg Ser Leu Ser
    50                  55                  60

Ser Tyr Gly Ile Asp Lys Glu Lys Thr Ile His Leu Thr Leu Lys Val
65                  70                  75                  80

Val Lys Pro Ser Asp Glu Glu Leu Pro Leu Phe Leu Val Glu Ser Gly
                85                  90                  95

Asp Glu Ala Lys Arg His Leu Leu Gln Val Arg Arg Ser Ser Ser Val
            100                 105                 110

Ala Gln Val Lys Ala Met Ile Glu Thr Lys Thr Gly Ile Ile Pro Glu
        115                 120                 125

Thr Gln Ile Val Thr Cys Asn Gly Lys Arg Leu Glu Asp Gly Lys Met
    130                 135                 140

Met Ala Asp Tyr Gly Ile Arg Lys Gly Asn Leu Leu Phe Leu Ala Ser
145                 150                 155                 160

Tyr Cys Ile Gly Gly

                165

<210> 79

<211> 1464

<212> PRT

<213> Homo sapiens

<400> 79

Met Phe Ser Phe Val Asp Leu Arg Leu Leu Leu Leu Leu Ala Ala Thr
1               5                   10                  15

Ala Leu Leu Thr His Gly Gln Glu Glu Gly Gln Val Glu Gly Gln Asp
            20                  25                  30

Glu Asp Ile Pro Pro Ile Thr Cys Val Gln Asn Gly Leu Arg Tyr His
        35                  40                  45

Asp Arg Asp Val Trp Lys Pro Glu Pro Cys Arg Ile Cys Val Cys Asp
    50                  55                  60

Asn Gly Lys Val Leu Cys Asp Asp Val Ile Cys Asp Glu Thr Lys Asn
65                  70                  75                  80

Cys Pro Gly Ala Glu Val Pro Glu Gly Glu Cys Cys Pro Val Cys Pro
                85                  90                  95

Asp Gly Ser Glu Ser Pro Thr Asp Gln Glu Thr Thr Gly Val Glu Gly
            100                 105                 110

Pro Lys Gly Asp Thr Gly Pro Arg Gly Pro Arg Gly Pro Ala Gly Pro
        115                 120                 125

Pro Gly Arg Asp Gly Ile Pro Gly Gln Pro Gly Leu Pro Gly Pro Pro
    130                 135                 140

Gly Pro Pro Gly Pro Pro Gly Pro Pro Gly Leu Gly Gly Asn Phe Ala
145                 150                 155                 160

385                 390                 395                 400

Gly Ala Pro Gly Ile Ala Gly Ala Pro Gly Phe Pro Gly Ala Arg Gly

                405                 410                 415

Pro Ser Gly Pro Gln Gly Pro Gly Gly Pro Pro Gly Pro Lys Gly Asn

            420                 425                 430

Ser Gly Glu Pro Gly Ala Pro Gly Ser Lys Gly Asp Thr Gly Ala Lys

        435                 440                 445

Gly Glu Pro Gly Pro Val Gly Val Gln Gly Pro Pro Gly Pro Ala Gly

    450                 455                 460

Glu Glu Gly Lys Arg Gly Ala Arg Gly Glu Pro Gly Pro Thr Gly Leu

465                 470                 475                 480

Pro Gly Pro Pro Gly Glu Arg Gly Gly Pro Gly Ser Arg Gly Phe Pro

                485                 490                 495

Gly Ala Asp Gly Val Ala Gly Pro Lys Gly Pro Ala Gly Glu Arg Gly

            500                 505                 510

Ser Pro Gly Pro Ala Gly Pro Lys Gly Ser Pro Gly Glu Ala Gly Arg

        515                 520                 525

Pro Gly Glu Ala Gly Leu Pro Gly Ala Lys Gly Leu Thr Gly Ser Pro

    530                 535                 540

Gly Ser Pro Gly Pro Asp Gly Lys Thr Gly Pro Pro Gly Pro Ala Gly

545                 550                 555                 560

Gln Asp Gly Arg Pro Gly Pro Pro Gly Pro Pro Gly Ala Arg Gly Gln

                565                 570                 575

Ala Gly Val Met Gly Phe Pro Gly Pro Lys Gly Ala Ala Gly Glu Pro

            580                 585                 590

Gly Lys Ala Gly Glu Arg Gly Val Pro Gly Pro Pro Gly Ala Val Gly

        595                 600                 605

Pro Ala Gly Lys Asp Gly Glu Ala Gly Ala Gln Gly Pro Pro Gly Pro

    610                 615                 620

Ala Gly Pro Ala Gly Glu Arg Gly Glu Gln Gly Pro Ala Gly Ser Pro
625                 630                 635                 640
Gly Phe Gln Gly Leu Pro Gly Pro Ala Gly Pro Pro Gly Glu Ala Gly
                645                 650                 655
Lys Pro Gly Glu Gln Gly Val Pro Gly Asp Leu Gly Ala Pro Gly Pro
            660                 665                 670
Ser Gly Ala Arg Gly Glu Arg Gly Phe Pro Gly Glu Arg Gly Val Gln
        675                 680                 685
Gly Pro Pro Gly Pro Ala Gly Pro Arg Gly Ala Asn Gly Ala Pro Gly
    690                 695                 700
Asn Asp Gly Ala Lys Gly Asp Ala Gly Ala Pro Gly Ala Pro Gly Ser
705                 710                 715                 720
Gln Gly Ala Pro Gly Leu Gln Gly Met Pro Gly Glu Arg Gly Ala Ala
                725                 730                 735
Gly Leu Pro Gly Pro Lys Gly Asp Arg Gly Asp Ala Gly Pro Lys Gly
            740                 745                 750
Ala Asp Gly Ser Pro Gly Lys Asp Gly Val Arg Gly Leu Thr Gly Pro
        755                 760                 765
Ile Gly Pro Pro Gly Pro Ala Gly Ala Pro Gly Asp Lys Gly Glu Ser
    770                 775                 780
Gly Pro Ser Gly Pro Ala Gly Pro Thr Gly Ala Arg Gly Ala Pro Gly
785                 790                 795                 800
Asp Arg Gly Glu Pro Gly Pro Pro Gly Pro Ala Gly Phe Ala Gly Pro
                805                 810                 815
Pro Gly Ala Asp Gly Gln Pro Gly Ala Lys Gly Glu Pro Gly Asp Ala
            820                 825                 830
Gly Ala Lys Gly Asp Ala Gly Pro Pro Gly Pro Ala Gly Pro Ala Gly
        835                 840                 845
Pro Pro Gly Pro Ile Gly Asn Val Gly Ala Pro Gly Ala Lys Gly Ala

Ser Gly Gln Tyr Tyr Asp Tyr Asp Phe Pro Leu Ser Ile Tyr Gly Gln
20 25 30
Ser Ser Pro Asn Cys Ala Pro Glu Cys Asn Cys Pro Glu Ser Tyr Pro
35 40 45
Ser Ala Met Tyr Cys Asp Glu Leu Lys Leu Lys Ser Val Pro Met Val
50 55 60
Pro Pro Gly Ile Lys Tyr Leu Tyr Leu Arg Asn Asn Gln Ile Asp His
65 70 75 80
Ile Asp Glu Lys Ala Phe Glu Asn Val Thr Asp Leu Gln Trp Leu Ile
85 90 95
Leu Asp His Asn Leu Leu Glu Asn Ser Lys Ile Lys Gly Arg Val Phe
100 105 110
Ser Lys Leu Lys Gln Leu Lys Lys Leu His Ile Asn His Asn Asn Leu
115 120 125
Thr Glu Ser Val Gly Pro Leu Pro Lys Ser Leu Glu Asp Leu Gln Leu
130 135 140
Thr His Asn Lys Ile Thr Lys Leu Gly Ser Phe Glu Gly Leu Val Asn
145 150 155 160
Leu Thr Phe Ile His Leu Gln His Asn Arg Leu Lys Glu Asp Ala Val
165 170 175
Ser Ala Ala Phe Lys Gly Leu Lys Ser Leu Glu Tyr Leu Asp Leu Ser
180 185 190
Phe Asn Gln Ile Ala Arg Leu Pro Ser Gly Leu Pro Val Ser Leu Leu
195 200 205
Thr Leu Tyr Leu Asp Asn Asn Lys Ile Ser Asn Ile Pro Asp Glu Tyr
210 215 220
Phe Lys Arg Phe Asn Ala Leu Gln Tyr Leu Arg Leu Ser His Asn Glu
225 230 235 240
Leu Ala Asp Ser Gly Ile Pro Gly Asn Ser Phe Asn Val Ser Ser Leu

Ala Ala Cys Ser Arg Tyr Phe Glu Ala Met Phe Ser Gly Gly Leu Lys
65                  70                  75                  80
Glu Ser Gln Asp Ser Glu Val Asn Phe Asp Asn Ser Ile His Pro Glu
                85                  90                  95
Val Leu Glu Leu Leu Leu Asp Tyr Ala Tyr Ser Ser Arg Val Ile Ile
            100                 105                 110
Asn Glu Glu Asn Ala Glu Ser Leu Leu Glu Ala Gly Asp Met Leu Glu
        115                 120                 125
Phe Gln Asp Ile Arg Asp Ala Cys Ala Glu Phe Leu Glu Lys Asn Leu
    130                 135                 140
His Pro Thr Asn Cys Leu Gly Met Leu Leu Leu Ser Asp Ala His Gln
145                 150                 155                 160
Cys Thr Lys Leu Tyr Glu Leu Ser Trp Arg Met Cys Leu Ser Asn Phe
                165                 170                 175
Gln Thr Ile Arg Lys Asn Glu Asp Phe Leu Gln Leu Pro Gln Asp Met
            180                 185                 190
Val Val Gln Leu Leu Ser Ser Glu Glu Leu Glu Thr Glu Asp Glu Arg
        195                 200                 205
Leu Val Tyr Glu Ser Ala Ile Asn Trp Ile Ser Tyr Asp Leu Lys Lys
    210                 215                 220
Arg Tyr Cys Tyr Leu Pro Glu Leu Leu Gln Thr Val Arg Leu Ala Leu
225                 230                 235                 240
Leu Pro Ala Ile Tyr Leu Met Glu Asn Val Ala Met Glu Glu Leu Ile
                245                 250                 255
Thr Lys Gln Arg Lys Ser Lys Glu Ile Val Glu Glu Ala Ile Arg Cys
            260                 265                 270
Lys Leu Lys Ile Leu Gln Asn Asp Gly Val Val Thr Ser Leu Cys Ala
        275                 280                 285
Arg Pro Arg Lys Thr Gly His Ala Leu Phe Leu Leu Gly Gly Gln Thr

2245 2250 2255

Arg Gly Ala Pro Gly Glu Arg Gly Arg Thr Gly Pro Leu Gly Arg Lys

2260 2265 2270

Gly Glu Pro Gly Glu Pro Gly Pro Lys Gly Gly Ile Gly Asn Pro Gly

2275 2280 2285

Pro Arg Gly Glu Thr Gly Asp Asp Gly Arg Asp Gly Val Gly Ser Glu

2290 2295 2300

Gly Arg Arg Gly Lys Lys Gly Glu Arg Gly Phe Pro Gly Tyr Pro Gly

2305 2310 2315 2320

Pro Lys Gly Asn Pro Gly Glu Pro Gly Leu Asn Gly Thr Thr Gly Pro

2325 2330 2335

Lys Gly Ile Arg Gly Arg Arg Gly Asn Ser Gly Pro Pro Gly Ile Val

2340 2345 2350

Gly Gln Lys Gly Arg Pro Gly Tyr Pro Gly Pro Ala Gly Pro Arg Gly

2355 2360 2365

Asn Arg Gly Asp Ser Ile Asp Gln Cys Ala Leu Ile Gln Ser Ile Lys

2370 2375 2380

Asp Lys Cys Pro Cys Cys Tyr Gly Pro Leu Glu Cys Pro Val Phe Pro

2385 2390 2395 2400

Thr Glu Leu Ala Phe Ala Leu Asp Thr Ser Glu Gly Val Asn Gln Asp

2405 2410 2415

Thr Phe Gly Arg Met Arg Asp Val Val Leu Ser Ile Val Asn Val Leu

2420 2425 2430

Thr Ile Ala Glu Ser Asn Cys Pro Thr Gly Ala Arg Val Ala Val Val

2435 2440 2445

Thr Tyr Asn Asn Glu Val Thr Thr Glu Ile Arg Phe Ala Asp Ser Lys

2450 2455 2460

Arg Lys Ser Val Leu Leu Asp Lys Ile Lys Asn Leu Gln Val Ala Leu

2465 2470 2475 2480

## Description

Colorectal carcinoma is a malignant neoplastic disease. There is a high incidence of colorectal carcinoma in the Western world, particularly in the United States. Tumors of this type often metastasize through lymphatic and vascular channels. Many patients with colorectal carcinoma eventually die from this disease. In fact, it is estimated that 62,000 persons in the United States alone die of colorectal carcinoma annually.

However, if diagnosed early, colorectal cancer may be treated effectively by surgical removal of the cancerous tissue. Colorectal cancers originate in the colorectal epithelium and typically are not extensively vascularized (and therefore not invasive) during the early stages of development. Colorectal cancer is thought to result from the clonal expansion of a single mutant cell in the epithelial lining of the colon or rectum. The transition to a highly vascularized, invasive and ultimately metastatic cancer which spreads throughout the body commonly takes ten years or longer. If the cancer is detected prior to invasion, surgical removal of the cancerous tissue is an effective cure. However, colorectal cancer is often detected only upon manifestation of clinical symptoms, such as pain and black tarry stool. Generally, such symptoms are present only when the disease is well established, often after metastasis has occurred, and the prognosis for the patient is poor, even after surgical resection of the cancerous tissue. Early detection of colorectal cancer therefore is important in that detection may significantly reduce its morbidity.

Invasive diagnostic methods such as endoscopic examination allow for direct visual identification, removal, and biopsy of potentially cancerous growths such as polyps. Endoscopy is expensive, uncomfortable, inherently risky, and therefore not a practical tool for screening populations to identify those with colorectal cancer. Non-invasive analysis of stool samples for characteristics indicative of the presence of colorectal cancer or precancer is a preferred alternative for early diagnosis, but no known diagnostic method is available which reliably achieves this goal. A reliable, non-invasive, and accurate technique for diagnosing colorectal cancer at an early stage would help save many lives.

Ectopic expression of the pancreatic regenerating gene (RegI) has been identified previously in colorectal tumors, and suggested as a potential marker for colorectal cancer (Zenilman et al., (1997) *J*. *Gastrointest*. *Surg*., 1: 194; Watanabe et al., (1990) *J. Biol. Chem*., 265: 7432; Birse and Rosen, WO01/12781). At present, there is no reliable method known to those of skill in the art for the rapid and accurate detection of Reg1α in the serum of colorectal cancer patients (Satomura et al., (1995) *J. Gastroenterol.* 30: 643). There is thus a need in the art for a method of detecting, and/or monitoring colorectal cancer in a patient utilizing the expression of Reg1α in serum.

The present invention provides a method of detecting, monitoring or determining the therapeutic response of colorectal cancer in an individual as well as compositions, and kits for performing the method. In its most general aspect, the method comprises: obtaining a clinical sample from the individual and detecting the presence of one or more of the nucleic acid sequences of SEQ ID Nos. 1, 3, or 5-71, or the amino acid sequences of SEQ ID Nos. 2, 4, or 72-138.

The invention also provides a method of detecting, monitoring or determining the therapeutic response of colorectal cancer in an individual as well as compositions, and kits for performing the method, which, in its preferred aspect, comprises: obtaining a clinical sample from the individual and detecting the presence of Reg1α or TIMP1 in said sample, wherein the presence of Reg1α or TIMP1 in the sample is indicative of the presence or stage of colorectal cancer in the individual.

In one embodiment, the step of detecting comprises: contacting said clinical sample with a ligand which is capable of binding to Reg1α or TIMP1 under conditions which permit the ligand to bind to Reg1α or TIMP1; and detecting the binding of the ligand to Reg1α or TIMP1, wherein detection of binding is indicative of the presence of Reg1α or TIMP1 in the sample. The polypeptide ligand may comprise, for example, an antibody, peptide, oligonucleotide, or other molecule that specifically binds Reg1α or TIMP1. In a currently preferred embodiment, the clinical sample comprises serum.

The present invention further provides a method of detecting, monitoring, or determining the presence of colorectal cancer in an individual comprising: obtaining a clinical sample from said individual; and detecting the presence of Reg1α or TIMP1 and at least one other colorectal cancer associated marker in the sample, wherein the presence of Reg1α or TIMP1 and the at least one other colorectal cancer associated marker is indicative of colorectal cancer in the individual. The colorectal cancer associated marker may comprise, for example, one or more of the nucleic acid sequences of SEQ ID Nos 1, 3, or 5-71, or the amino acid sequences of SEQ ID Nos 2, 4, or 72-138, or derivatives or homologs thereof having substantially the same binding specificity.

In a preferred embodiment embodiment, the above step of detecting comprises contacting a serum sample with a first ligand which is capable of binding to Reg1α or TIMP1 and a second ligand which is capable of binding to the colorectal cancer associated marker, under conditions which permit the first and second ligands to bind to Reg1α or TIMP1 and the colorectal cancer associated marker, respectively; and detecting the binding of the first ligand to Reg1α or TIMP1 and the second ligand to the colorectal cancer associated marker, wherein detection of binding is indicative of the presence of Reg1α or TIMP1 and the colorectal cancer associated marker in said sample. The polypeptide ligand may comprise, for example, an antibody, peptide, oligonucleotide, or other molecule that specifically binds Reg1α or TIMP1.

The present invention also provides a method of detecting, monitoring or determining the presence of colorectal cancer in an individual comprising: obtaining a clinical sample from an individual; and detecting the presence of a nucleic acid molecule which encodes Reg1α or TIMP 1 in said sample, wherein the presence of the nucleic acid molecule in the sample is indicative of colorectal cancer in the individual.

The invention still further provides a method of detecting, monitoring or determining the presence of colorectal cancer in an individual comprising: obtaining a clinical sample from an individual; and detecting the presence of a nucleic acid molecule which encodes Reg1α or TIMP1 and at least one other nucleic acid molecule which encodes at least one other colorectal cancer associated marker in the sample, wherein the presence of the nucleic acid sequence encoding Reg1α or TIMP1 and the nucleic acid sequence encoding the at least one other colorectal cancer associated marker is indicative of colorectal cancer in the individual. In a preferred embodiment, the colorectal cancer associated marker is one or more of the nucleic acid sequences of SEQ ID Nos 1, 3, or 5-71, or the amino acid sequences of SEQ ID Nos 2, 4, or 72-138, or derivatives or homologs thereof having substantially the same binding specificity.

Figure 1 shows the level of Reg1α polypeptide present in serum obtained from normal control patients (n=35), patients diagnosed with inflammatory bowel disease (IBD; n=7), patients diagnosed with cirrhosis (n=7), and patients diagnosed with colorectal cancer (n=63).

Figure 2 shows the level of Reg1α polypeptide measured in the colorectal cancer patient group (n=63) differentiated based on cancer severity. The degree of cancer has been established by Dukes'-type staging, and data from patients with Dukes'-type A, B, C, and D is shown.

Figure 3 shows a graphical representation of the plasma level of TIMP 1 polypeptides, along with one or more other colorectal cancer associated markers obtained from patients with colorectal cancer.

The present invention is based, in part, on the discovery that the expression of they human islet regenerating protein, Reg1α, is increased in patients with colorectal cancer, and as such is a valuable marker for the identification of colorectal cancer in humans. The present invention further provides for the early detection of colorectal cancer by detecting the presence of Reg1α or TIMP1 (and optionally, one or more additional colorectal cancer associated markers) in a clinical sample from an individual. The invention provides further, the ability to monitor the recurrence of colorectal cancer in a patient wherein colorectal cancer has been previously detected, by monitoring the levels of Reg1α or TIMP1 polypeptide or polynucleotide sequences present in a clinical sample from the patient, wherein an increase in Reg1α or TIMP1 in the sample is indicative of the recurrence of colorectal cancer. The invention provides still further, the ability to monitor the decrease in colorectal cancer in response to a therapeutic agent, whereby the levels of Reg1α or TIMP1 are measured in a clinical sample obtained from a patient who has received therapeutic treatment for colorectal cancer, wherein a decrease in the levels of Reg1α or TIMP1 detected in the clinical sample from the patient is indicative of the efficacy of the therapeutic treatment. In any of the preceding embodiments, Reg1α or TIMP1 polynucleotide or polypeptide expression levels are measured in concert with at least one additional colorectal cancer associated marker.

Accordingly, the present invention relates in part to novel methods for identifying cancer in an individual, particularly colorectal cancer, by screening for genes or gene products, which are over or underexpressed in cancer relative to the level of expression in normal tissue, such as colon tissue. Alternatively, the invention provides a method for the identification of cancer in an individual by screening for genes or gene products which are over- or underexpressed in colorectal cancer, and which are detectable in a clinical sample of an individual with colorectal cancer.

In a preferred embodiment, the present invention relates to methods useful for the detection of colorectal cancer in an individual, preferably a human patient by detecting serum levels of Reg1α or TIMP1. The invention relates to methods for colorectal cancer detection that utilize either or both techniques of detecting the presence of the Reg1α or TIMP1 gene or detecting the Reg1α or TIMP1 encoded polypeptide product in the serum of an individual, or alternatively in a clinical sample from an individual.

The present invention further provides methods for the identification of colorectal cancer wherein cancer is detected by the identification of Reg1α or TIMP1 expression in a patient clinical sample, in combination with the expression in the same sample of at least one other colorectal cancer associated marker. This combination of Reg1α or TIMP1 detection analysis, in concert with the detection of additional colon-cancer markers provides an efficient and reliable method for detecting the presence of colorectal cancer.

The methods described herein which specifically refer to the detection of Reg1α, may equally be applied to the detection of TIMP1 by one of skill in the art, based on the disclosure of the present specification:

As used herein, "Reg1α" refers to a polypeptide molecule having the sequence of either of SEQ ID Nos 2 or 4. Reg1α as used herein, also refers to a polypeptide which is encoded by either of the sequences of SEQ ID Nos. 1 or 3. The sequences of SEQ ID Nos 2 and 4 each represent a functional Reg1α protein, but differ from each other by four amino acids in the leader sequence which is cleaved off during protein synthesis.

As used herein, "TIMP 1" refers to a polypeptide molecule having the sequence of SEQ ID NO: 100. TIMP1 as used herein, also refers to a nucleotide which is encoded by the sequence of SEQ ID NO: 33, or a functional homolog thereof.

As used herein, a "clinical sample" refers to a tissue, cellular, or fluid sample obtained from an individual. A "clinical sample", as used herein, can refer to a cells, circulating cells (e.g., circulating cells in blood), cells obtained from specific anatomical locations, or specific cell types (e.g., colon cell, gastrointestinal cell, cancerous cell, etc.), a tissue sample, or physiological fluids such as lymph, bile, serum, plasma, urine, synovial fluid, blood, CSF, mucus membrane secretions, or other physiological samples such as stool. Preferably, the clinical sample is serum or plasma. A colorectal cancer associated marker of the invention, such as TIMP1, may be detected in a suitable "clinical sample" where the suitability of a particular type of clinical sample for the detection of a specific colorectal cancer associated marker may be readily determined by one of skill in the art.

As used herein, "detecting" refers to the identification of the presence or absence of a molecule in a sample. Where the molecule to be detected is a polypeptide, the step of detecting can be performed by binding the polypeptide with an antibody that is detectably labeled. A detectable label is a molecule which is capable of generating, either independently, or in response to a stimulus, an observable signal. A detectable label can be, but is not limited to a fluorescent label, a chromogenic label, a luminescent label, or a radioactive label. Methods for "detecting" a label include quantitative and qualitative methods adapted for standard or confocal microscopy, FACS analysis, and those adapted for high throughput methods involving multiwell plates, arrays or microarrays. One of skill in the art can select appropriate filter sets and excitation energy sources for the detection of fluorescent emission from a given fluorescent polypeptide or dye. "Detecting" as used herein can also include the use of multiple antibodies to a polypeptide to be detected, wherein the multiple antibodies bind to different epitopes on the polypeptide to be detected. Antibodies used in this manner can employ two or more detectable labels, and can include, for example a FRET pair. A polypeptide molecule, such as Reg1α, is "detected" according to the present invention when the level of detectable signal is at all greater than the background level of the detectable label, or where the level of measured nucleic acid is at all greater than the level measured in a control sample.

As used herein, "detecting" as it refers to detecting the presence of a target nucleic acid molecule (e.g., a nucleic acid molecule encoding Reg1α, or other colorectal cancer-specific sequence) refers to a process wherein the signal generated by a directly or indirectly labeled probe nucleic acid molecule (capable of hybridizing to a target, e.g., a sequence encoding Reg1α, in a serum sample) is measured or observed. Thus, detection of the probe nucleic acid is directly indicative of the presence, and thus the detection, of a target nucleic acid, such as a sequence encoding Reg1α. For example, if the detectable label is a fluorescent label, the target nucleic acid (e.g., the nucleic acid molecule encoding Reg1α) is "detected" by observing or measuring the light emitted by the fluorescent label on the probe nucleic acid when it is excited by the appropriate wavelength, or if the detectable label is a fluorescence/quencher pair, the target nucleic acid is "detected" by observing or measuring the light emitted upon association or dissociation of the fluorescence/quencher pair present on the probe nucleic acid, wherein detection of the probe nucleic acid indicates detection of the target nucleic acid. If the detectable label is a radioactive label, the target nucleic acid, following hybridization with a radioactively labeled probe is "detected" by, for example, autoradiography. Methods and techniques for "detecting" fluorescent, radioactive, and other chemical labels may be found in Ausubel et al. (1995, Short Protocols in Molecular Biology, 3^{rd} Ed. John Wiley and Sons, Inc.). Alternatively, a nucleic acid may be "indirectly detected" wherein a moiety is attached to a probe nucleic acid which will hybridize with the target, such as an enzyme activity, allowing detection in the presence of an appropriate substrate, or a specific antigen or other marker allowing detection by addition of an antibody or other specific indicator. Alternatively, a target nucleic acid molecule can be detected by amplifying a nucleic acid sample prepared from a patient clinical sample, using oligonucleotide primers which are specifically designed to hybridize with a portion of the target nucleic acid sequence. Quantative amplification methods, such as, but not limited to TaqMan, may also be used to "detect" a target nucleic acid according to the invention. A nucleic acid molecule is "detected" as used herein where the level of nucleic acid measured (such as by quantitative PCR), or the level of detectable signal provided by the detectable label is at all above the background level.

As used herein, "detecting" refers further to the early detection of colorectal cancer in a patient, wherein "early" detection refers to the detection of colorectal cancer at Dukes stage A or preferably, prior to a time when the colorectal cancer is morphologically able to be classified in a particular Dukes stage. "Detecting" as used herein further refers to the detection of colorectal cancer recurrence in an individual, using the same detection criteria as indicated above. "Detecting" as used herein still further refers to the measuring of a change in the degree of colorectal cancer before and/or after treatment with a therapeutic agent. In this case, a change in the degree of colorectal cancer in response to a therapeutic agent refers to an increase or decrease in the expression of Reg1α (and optionally, one or more additional colorectal cancer associated markers), or alternatively, in the amount of Reg1α polypeptide (and optionally, one or more additional colorectal cancer associated markers) present in a clinical sample by at least 10% in response to the presence of a therapeutic agent relative to the expression level in the absence of the therapeutic agent.

As used herein, "individual" refers to a mammal, preferably a human.

As used herein, a "ligand" refers to a molecule which is capable of binding a polypeptide. A "polypeptide ligand" useful in the present invention includes, but is not limited to an antibody, a monoclonal antibody, a polyclonal antibody, an antibody fragment (e.g., Fv, scFV, or Fab), a small molecule, or a nucleic acid aptamer. A "ligand" as used herein can also refer to a "nucleic acid ligand", such as an oligonucleotide, polynucleotide, DNA, RNA, mRNA, or cDNA, which is capable of binding to a complementary nucleic acid molecule, or polypeptide molecule.

The term "antibody" as used herein is intended to include whole antibodies, e.g., of any isotype (IgG, IgA, IgM, IgE, etc), and includes fragments thereof, and single-chain antibodies, which also are specifically reactive with a vertebrate, e.g., mammalian, protein. Antibodies can be fragmented using conventional techniques and the fragments screened for utility in the same manner as described above for whole antibodies. Thus, the term includes segments of proteolytically-cleaved or recombinantly-prepared portions of an antibody molecule that are capable of selectively reacting with a certain protein. Nonlimiting examples of such proteolytic and/or recombinant fragments include Fab, F(ab')2, Fab' , Fv, and single chain antibodies (scFv) containing a V[L] and/or V[H] domain joined by a peptide linker. The scFv's may be covalently or non-covalently linked to form antibodies having two or more binding sites. The subject invention includes polyclonal, monoclonal, or other purified preparations of antibodies and recombinant antibodies.

As used herein, a "colorectal cancer associated marker" refers to a polypeptide or nucleic acid sequence which exhibits over- or underexpression of at least 10% in colorectal cancer cells, tissue, or serum obtained from an individual having colorectal cancer, relative to the level of expression in cells, tissue, or serum obtained from an individual that does not have colorectal cancer. Non-limiting examples of colorectal cancer associated markers useful in the present invention include the nucleic acid molecules of SEQ ID Nos 1, 3, 5-71, and/or the polypeptide molecules of SEQ ID Nos 2, 4, 72-138. In one embodiment, the polypeptide sequences of SEQ ID Nos 2, 4, 72-138 are encoded by the nucleic acid sequences of 1, 3, 5-71, respectively. A "colorectal cancer specific marker" useful in the invention may be a polypeptide or nucleic acid sequence which exhibits over- or underexpression in colorectal cancer as described above, but which may also be over or underexpressed in other, non-colorectal types of cancer. Alternatively, a "colorectal cancer associated marker", as used herein, may refer to a carbohydrate epitope present on a polypeptide or nucleic acid molecule and/or an antibody molecule which recognizes and is capable of binding to such an epitope, wherein the carbohydrate epitope is known to be associated with the presence of colorectal cancer in an individual. Such carbohydrate epitopes may be present on more than one unrelated protein or polypeptide. In one embodiment, such a carbohydrate epitope is CA 19-9, also known as sialyl-Lewis^{a}, is a tumor marker defined by a monoclonal antibody as a carbohydrate epitope, related to the blood group antigens, composed of a branching, 5-sugar structure covalently bound to a variety of glycoproteins or glycolipids. The proteins primarily belong to the mucin family and the lipids are usually membrane associated. The CA 19-9 epitope is typically the terminal moiety of a complex, O-linked carbohydrate structure on either macromolecule. Other tumor markers also defined as various carbohydrate epitopes useful in the present invention as a "colorectal cancer associated marker" include CA 72-4, TF, sTn, Tn, CA 50, CA 549, CA 242, LASA, and the Du-PAN's 1 -5.

The term "interact" as used herein is meant to include detectable interactions (e.g., biochemical interactions) between molecules, such as interaction between protein-protein, protein-nucleic acid, nucleic acid-nucleic acid, and protein-small molecule or nucleic acid-small molecule in nature.

As used herein, the term "nucleic acid" refers to polynucleotides such as deoxyribonucleic acid (DNA), and, where appropriate, ribonucleic acid (RNA). The term should also be understood to include, as equivalents, analogs of either RNA or DNA made from nucleotide analogs, and, as applicable to the embodiment being described, single (sense or antisense) and double-stranded polynucleotides. ESTs, chromosomes, cDNAs, mRNAs, and rRNAs are representative examples of molecules that may be referred to as nucleic acids.

The terms "protein", "polypeptide", and "peptide" are used interchangeably herein when referring to a gene product. As used herein, "polypeptide" refers to any kind of polypeptide such as peptides, human proteins, fragments of human proteins, proteins or fragments of proteins from non-human sources, engineered versions proteins or fragments of proteins, enzymes, antigens, drugs, molecules involved in cell signaling, such as receptor molecules, antibodies, including polypeptides of the immunoglobulin superfamily, such as antibody polypeptides or T-cell receptor polypeptides.

As used herein, the term "level of expression" refers to the measurable expression level of a given nucleic acid. The level of expression of a nucleic acid is determined by methods well known in the art. The "level of expression" may measured by hybridization analysis using labeled target nucleic acids according to methods well known in the art (see, for example, Ausubel et al., Short Protocols in Molecular Biology, 3^{rd} Ed. 1995, John Wiley and Sons, Inc.). The label on the target nucleic acid is a luminescent label, an enzymatic label, a radioactive label, a chemical label or a physical label. Preferably, the target nucleic acids are labeled with a fluorescent molecule. Preferred fluorescent labels include fluorescein, amino coumarin acetic acid, tetramethylrhodamine isothiocyanate (TRITC), Texas Red, Cy3 and Cy5. Alternatively, the "level of expression" can be measured by quantitative amplification protocols, such as TaqMan, known to those of skill in the art.

The term "vector" refers to a nucleic acid molecule capable of transporting another nucleic acid to which it has been linked. One type of preferred vector is an episome, i. e., a nucleic acid capable of extra-chromosomal replication. Preferred vectors are those capable of autonomous replication and/or expression of nucleic acids to which they are linked. Vectors capable of directing the expression of genes to which they are operatively linked are referred to herein as "expression vectors". In general, expression vectors of utility in recombinant DNA techniques are often in the form of "plasmids" which refer generally to circular double stranded DNA loops which, in their vector form are not bound to the chromosome. In the present specification, "plasmid" and "vector" are used interchangeably as the plasmid is the most commonly used form of vector. However, the invention is intended to include such other forms of expression vectors which serve equivalent functions and which become known in the art subsequently hereto.

### Reg1α and TIMP1 nucleic acid

As described above, the present invention relates to the detection of Reg1α or TIMP1 polypeptide in a clinical sample from an individual, preferably a serum or plasma sample, thus permitting the detection of colorectal cancer. The present invention, however, equally relates to the identification of the nucleic acid sequence which encodes Reg1α or TIMP1 as a marker for colorectal cancer.

Nucleic acid and amino acid sequences of Reg1α are shown in SEQ ID Nos 1 or 3, and 2 or 4, respectively. Nucleic acid and amino acid sequences of TIMP1 are shown in SEQ ID NO: 33 and 100 respectively. While the invention relates to the direct detection of either of the sequences of Reg 1α or TIMP1 in a method for detecting colorectal cancer, the invention further relates to the detection of sequences complementary thereto, or a sequence which specifically hybridizes to a sequence of SEQ ID Nos. 1, 3, or 33. The present invention also relates to the detection of colorectal cancer by detecting the presence, in a clinical sample, of a nucleic acid molecule which encodes the sequence of SEQ ID Nos. 2, 4, or 100, or a fragment thereof.

Another aspect of the invention provides the detection of colorectal cancer by the detection of a nucleic acid which hybridizes under low, medium, or high stringency conditions to a nucleic acid sequence represented by one or more of SEQ ID Nos. 1, 3, or 33, or a sequence complementary thereto. Appropriate stringency conditions which promote DNA hybridization, for example, 6.0 x sodium chloride/sodium citrate (SSC) at about 45 °C, followed by a wash of 2.0 x SSC at 50°C, are known to those skilled in the art or can be found in Current Protocols in Molecular Biology, John Wiley & Sons, N.Y. (1989), 6.3.1-12.3.6. For example, the salt concentration in the wash step can be selected from a low stringency of about 2.0 x SSC at 50°C to a high stringency of about 0.2 x SSC at 50°C. In addition, the temperature in the wash step can be increased from low stringency conditions at room temperature, about 22 °C, to high stringency conditions at about 65 °C. Both temperature and salt may be varied, or temperature or salt concentration may be held constant while the other variable is changed. In a preferred embodiment, a nucleic acid encoding Reg1α or TIMP1 will bind to SEQ ID Nos. 1, 3 or 33, or a sequence complementary thereto, or a fragment thereof, under moderately stringent conditions, for example at about 2.0 x SSC and about 40°C. In a particularly preferred embodiment, a Reg1α or TIMP1 nucleic acid sequence present in a patient clinical sample will bind of SEQ ID Nos. 1, 3, or 33, respectively, or a sequence complementary thereto, or fragment thereof, under high stringency conditions.

In one embodiment, the invention provides nucleic acids which hybridize under low stringency conditions of 6 x SSC at room temperature followed by a wash at 2 x SSC at room temperature.

In another embodiment, the invention provides nucleic acids which hybridize under high stringency conditions of 2 x SSC at about 65 °C followed by a wash at 0.2 x SSC at about 65 °C.

Detection of Reg1α nucleic acids having a sequence that differs from the nucleotide sequences shown in SEQ ID Nos. 1 or 3, or a sequence complementary thereto, due to degeneracy in the genetic code, are also within the scope of the invention. Such nucleic acids encode functionally equivalent peptides (i.e., a peptide having equivalent or similar biological activity) but differ in sequence from the sequence shown in the sequence listing due to degeneracy in the genetic code. For example, a number of amino acids are designated by more than one triplet. Codons that specify the same amino acid, or synonyms (for example, CAU and CAC each encode histidine) may result in "silent" mutations which do not affect the amino acid sequence of a polypeptide. However, it is expected that DNA sequence polymorphisms that do lead to changes in the amino acid sequences of the subject polypeptides will exist among mammals. One skilled in the art will appreciate that these variations in one or more nucleotides (e.g., up to about 3-5% of the nucleotides) of the nucleic acids encoding polypeptides having an activity of a polypeptide may exist among individuals of a given species due to natural allelic variation.

The invention also includes within its scope a polynucleotide which hybridizes under stringent conditions (at least about 4 x SSC at 65 °C, or at least about 4 x SSC at 42 °C; see, for example, U.S. Patent No. 5,707,829, incorporated herein by reference) with at least 15 contiguous nucleotides of SEQ ID Nos. 1 or 3. By this is intended that when at least 15 contiguous nucleotides of SEQ ID Nos. 1 or 3 is used as a probe, the probe will preferentially hybridize with a gene or mRNA (of the biological material) comprising the complementary sequence, allowing the identification and retrieval of the nucleic acids (i.e., Reg1α) of the biological material that uniquely hybridize to the selected probe. Probes of more than 15 nucleotides can be used, but 15 nucleotides represents enough sequence for unique identification.

Constructs of polynucleotides having the sequence of SEQ ID Nos. 1 or 3, a portion thereof, or a sequence complementary thereto, and useful, for example for generating a probe, can be produced synthetically, or obtained from natural sources (e.g., human cells) using methods well known to those of skill in the art (see, for example, Sambrook et al., Molecular Cloning: A Laboratory Manual, 2nd Ed. (Cold Spring Harbor Press, Cold Spring Harbor, NY 1989).

### Calculation of Sequence Homology

In one embodiment, the present invention relates to the detection of colorectal cancer in an individual by detecting the presence of Reg1α or TIMP1 or a sequence homologous thereto, by using probes and/or primers which are complementary to portions of the Reg1α or TIMP1 sequence, or are sufficiently homologous to portions of the Reg1α or TIMP1 sequence to permit hybridization of the probes and/or primers to Reg1α or TIMP1 under high stringency conditions. Sequences of the invention are at least 50% homologous to Reg1α or TIMP1, and are preferably 60%, 70%, 80%, 90% homologous up to complete sequence identity with Reg1α or TIMP1 (or optionally to a sequence encoding one or more additional colorectal cancer associated markers).

Sequence identity with respect to any of the sequences presented herein can be determined by a simple "eyeball" comparison (i.e. a strict comparison) of any one or more of the sequences with another sequence to see if that other sequence has, for example, at least 80% sequence identity to the sequence(s).

Relative sequence identity can also be determined by commercially available computer programs that can calculate % identity between two or more sequences using any suitable algorithm for determining identity, using for example default parameters. A typical example of such a computer program is CLUSTAL. Other computer program methods to determine identity and similarity between two sequences include but are not limited to the GCG program package (Devereux *et al* 1984 Nucleic Acids Research 12: 387) and FASTA (Atschul *et al* 1990 J Molec Biol 403-410).

% homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimized alignments with fewer gaps. Most alignment programs allow the gap penalties to be modified. However, it is preferred to use the default values when using such software for sequence comparisons. For example, when using the GCG Wisconsin Bestfit package the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (University of Wisconsin, U.S.A.; Devereux *et al*., 1984, Nucleic Acids Research 12:387). Examples of other software that can perform sequence comparisons include, but are not limited to, the BLAST package (Ausubel et al., 1995, Short Protocols in Molecular Biology, 3rd Edition, John Wiley & Sons), FASTA (Atschul *et al*., 1990, J. Mol. Biol., 403-410) and the GENEWORKS suite of comparison tools. Both BLAST and FASTA are available for offline and online searching (Ausubel *et al*., 1999 *supra*, pages 7-58 to 7-60).

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied. It is preferred to use the public default values for the GCG package, or in the case of other software, the default matrix, such as BLOSUM62.

Advantageously, the BLAST algorithm is employed, with parameters set to default values. The BLAST algorithm is described in detail on the World Wide Web at ncbi.nih.gov/BLAST/blast_help.html, which is incorporated herein by reference. The search parameters are defined as follows, and can be advantageously set to the defined default parameters.

Advantageously, "substantial identity" when assessed by BLAST equates to sequences which match with an EXPECT value of at least about 7, preferably at least about 9 and most preferably 10 or more. The default threshold for EXPECT in BLAST searching is usually 10.

BLAST (Basic Local Alignment Search Tool) is the heuristic search algorithm employed by the programs blastp, blastn, blastx, tblastn, and tblastx; these programs ascribe significance to their findings using the statistical methods of Karlin and Altschul (Karlin and Altschul 1990, *Proc. Natl. Acad. Sci. USA* 87:2264-68; Karlin and Altschul, 1993, *Proc. Natl. Acad. Sci. USA* 90:5873-7; see http://www.ncbi.nih.gov/BLAST/blast_help.html) with a few enhancements. The BLAST programs are tailored for sequence similarity searching, for example to identify homologues to a query sequence. For a discussion of basic issues in similarity searching of sequence databases, see Altschul *et al* (1994) Nature Genetics 6:119-129.

The five BLAST programs available on the World Wide Web at ncbi.nlm.nih.gov perform the following tasks: **blastp -** compares an amino acid query sequence against a protein sequence database; **blastn -** compares a nucleotide query sequence against a nucleotide sequence database; **blastx -** compares the six-frame conceptual translation products of a nucleotide query sequence (both strands) against a protein sequence database; **tblastn -** compares a protein query sequence against a nucleotide sequence database dynamically translated in all six reading frames (both strands); **tblastx -** compares the six-frame translations of a nucleotide query sequence against the six-frame translations of a nucleotide sequence database.

BLAST uses the following search parameters:

HISTOGRAM - Display a histogram of scores for each search; default is yes. (See parameter H in the BLAST Manual).

DESCRIPTIONS - Restricts the number of short descriptions of matching sequences reported to the number specified; default limit is 100 descriptions. (See parameter V in the manual page).

EXPECT - The statistical significance threshold for reporting matches against database sequences; the default value is 10, such that 10 matches are expected to be found merely by chance, according to the stochastic model of Karlin and Altschul (1990). If the statistical significance ascribed to a match is greater than the EXPECT threshold, the match will not be reported. Lower EXPECT thresholds are more stringent, leading to fewer chance matches being reported. Fractional values are acceptable. (See parameter E in the BLAST Manual).

CUTOFF - Cutoff score for reporting high-scoring segment pairs. The default value is calculated from the EXPECT value (see above). HSPs are reported for a database sequence only if the statistical significance ascribed to them is at least as high as would be ascribed to a lone HSP having a score equal to the CUTOFF value. Higher CUTOFF values are more stringent, leading to fewer chance matches being reported. (See parameter S in the BLAST Manual). Typically, significance thresholds can be more intuitively managed using EXPECT.

ALIGNMENTS - Restricts database sequences to the number specified for which high-scoring segment pairs (HSPs) are reported; the default limit is 50. If more database sequences than this happen to satisfy the statistical significance threshold for reporting (see EXPECT and CUTOFF below), only the matches ascribed the greatest statistical significance are reported. (See parameter B in the BLAST Manual).

MATRIX - Specify an alternate scoring matrix for BLASTP, BLASTX, TBLASTN and TBLASTX. The default matrix is BLOSUM62 (Henikoff & Henikoff, 1992). The valid alternative choices include: PAM40, PAM120, PAM250 and IDENTITY. No alternate scoring matrices are available for BLASTN; specifying the MATRIX directive in BLASTN requests returns an error response.

STRAND - Restrict a TBLASTN search to just the top or bottom strand of the database sequences; or restrict a BLASTN, BLASTX or TBLASTX search to just reading frames on the top or bottom strand of the query sequence.

FILTER - Mask off segments of the query sequence that have low compositional complexity, as determined by the SEG program of Wootton & Federhen (1993) Computers and Chemistry 17:149-163, or segments consisting of short-periodicity internal repeats, as determined by the XNU program of Claverie & States (1993) Computers and Chemistry 17:191-201, or, for BLASTN, by the DUST program of Tatusov and Lipman (see http://www.ncbi.nlm.nih.gov). Filtering can eliminate statistically significant but biologically uninteresting reports from the blast output (e.g., hits against common acidic-, basic- or proline-rich regions), leaving the more biologically interesting regions of the query sequence available for specific matching against database sequences.

Low complexity sequence found by a filter program is substituted using the letter "N" in nucleotide sequence (e.g., "NNNNNNNNNNNNN") and the letter "X" in protein sequences (e.g., "XXXXXXXXX").

Filtering is only applied to the query sequence (or its translation products), not to database sequences. Default filtering is DUST for BLASTN, SEG for other programs.

It is not unusual for nothing at all to be masked by SEG, XNU, or both, when applied to sequences in SWISS-PROT, so filtering should not be expected to always yield an effect. Furthermore, in some cases, sequences are masked in their entirety, indicating that the statistical significance of any matches reported against the unfiltered query sequence should be suspect.

NCBI-gi - Causes NCBI gi identifiers to be shown in the output, in addition to the accession and/or locus name.

Most preferably, sequence comparisons are conducted using the simple BLAST search algorithm provided on the World Wide Web at ncbi.nlm.nih.gov/BLAST. In some embodiments of the present invention, no gap penalties are used when determining sequence identity.

### Probes and Primers

The nucleotide sequence of Reg1α or TIMP1 is useful in the present invention for the generation of probes and primers designed for identifying the Reg1α or TIMP1 nucleic acid sequence in a patient sample such as serum, colon cells or tissue. Nucleotide sequences useful as probes/primers may include all or a portion of SEQ ID Nos. 1, 3 or 33, or a sequence complementary thereto, or sequences which hybridize under stringent conditions to all or a portion of SEQ ID No. 1, 3 or 33. For instance, the present invention also provides a probe/primer comprising a substantially purified oligonucleotide, which oligonucleotide comprising a nucleotide sequence that hybridizes under stringent conditions to at least approximately 8, preferably about 12, preferably about 15, preferably about 25 , more preferably about 40 consecutive nucleotides up to the full length of the sense or anti-sense sequence of SEQ ID Nos. 1, 3 or 33, or a sequence complementary thereto, or a naturally occurring mutant thereof. For instance, primers based on the nucleic acid represented in SEQ ID No. 1, 3 or 33, or a sequence complementary thereto, can be used in a reaction to amplify a template nucleic acid (e.g., Reg1α) contained within an mRNA sample derived from a patient clinical sample.

Not only are probes based on the nucleic acid sequence encoding Reg1α or TIMP1 useful for detecting Reg1α or TIMP1, but they can also provide a method for detecting mutations in wild-type Reg1α or TIMP1 in a patient. Nucleic acid probes which are complementary to a wild-type Reg1α or TIMP1 and can form mismatches with mutant genes are provided, allowing for detection by enzymatic or chemical cleavage or by shifts in electrophoretic mobility. Likewise, probes based on the subject sequences can be used to detect transcripts or genomic sequences encoding the same or homologous proteins, for use, for example, in prognostic or diagnostic assays. In preferred embodiments, the nucleic acid probe further comprises a label group attached thereto and able to be detected, e.g., the label group is selected from a radioisotope, a fluorescent compound, a chemiluminescent compound, a chromagenic compound, an enzyme, and enzyme co-factor.

Full-length cDNA molecules comprising the disclosed nucleic acids, useful for the generation of probes, primers, or for transcription to produce the Reg1α or TIMP1 protein itself, or antibodies thereto may be obtained as follows. The nucleic acid sequence of Reg1α or TIMP1 or a portion thereof comprising at least approximately 8, preferably about 12, preferably about 15, preferably about 25, more preferably about 40 nucleotides up to the full length of the sequence of SEQ ID Nos. 1, 3 or 33, or a sequence complementary thereto, may be used as a hybridization probe to detect hybridizing members of a cDNA library using probe design methods, cloning methods, and clone selection techniques as described in U.S. Patent No. 5,654,173, "Secreted Proteins and Polynucleotides Encoding Them," incorporated herein by reference. Libraries of cDNA may be made from selected tissues, such as normal or tumor tissue, or from tissues of a mammal treated with, for example, a pharmaceutical agent. Preferably, the tissue is the same as that used to generate the nucleic acids, as both the nucleic acid and the cDNA represent expressed genes. Alternatively, many cDNA libraries are available commercially. (Sambrook et al., Molecular Cloning: A Laboratory Manual, 2nd Ed. (Cold Spring Harbor Press, Cold Spring Harbor, NY 1989). The choice of cell type for library construction may be made after the identity of the protein encoded by the nucleic acid-related gene is known. This will indicate which tissue and cell types are likely to express the related gene, thereby containing the mRNA for generating the cDNA.

Members of the library that are larger than the nucleic acid, and preferably that contain the whole sequence of the native message, may be obtained. To confirm that the entire cDNA has been obtained, RNA protection experiments may be performed as follows. Hybridization of a full-length cDNA to an mRNA may protect the RNA from RNase degradation. If the cDNA is not full length, then the portions of the mRNA that arc not hybridized may be subject to RNase degradation. This may be assayed, as is known in the art, by changes in electrophoretic mobility on polyacrylamide gels, or by detection of released monoribonucleotides. Sambrook et al., Molecular Cloning: A Laboratory Manual, 2nd Ed. (Cold Spring Harbor Press, Cold Spring Harbor, NY 1989). In order to obtain additional sequences 5' to the end of a partial cDNA, 5' RACE (PCR Protocols: A Guide to Methods and Applications (Academic Press, Inc. 1990)) may be performed.

Genomic DNA (e.g., Reg1α genomic DNA) may be isolated using nucleic acids in a manner similar to the isolation of full-length cDNAs. Briefly, the nucleic acids, or portions thereof, may be used as probes to libraries of genomic DNA. Preferably, the library is obtained from the cell type that was used to generate the nucleic acids. Most preferably, the genomic DNA is obtained from the biological material described herein in the Example. Such libraries may be in vectors suitable for carrying large segments of a genome, such as P1 or YAC, as described in detail in Sambrook et al.,pages 9.4-9.30. In addition, genomic sequences can be isolated from human BAC libraries, which are commercially available from Research Genetics, Inc., Huntville, Alabama, USA, for example. In order to obtain additional 5' or 3' sequences, chromosome walking may be performed, as described in Sambrook et al., such that adjacent and overlapping fragments of genomic DNA are isolated. These may be mapped and pieced together, as is known in the art, using restriction digestion enzymes and DNA ligase.

Using the nucleic acids of the invention, corresponding full length genes can be isolated using both classical and PCR methods to construct and probe cDNA libraries. Using either method, Northern blots, preferably, may be performed on a number of cell types to determine which cell lines express the gene of interest at the highest rate.

Classical methods of constructing cDNA libraries in Sambrook et al., supra. With these methods, cDNA can be produced from mRNA and inserted into viral or expression vectors. Typically, libraries of mRNA comprising poly(A) tails can be produced with poly(T) primers. Similarly, cDNA libraries can be produced using the instant Reg1α sequence or portions thereof as primers.

PCR methods may be used to amplify the members of a cDNA library that comprise the desired insert. In this case, the desired insert may contain sequence from the full length cDNA that corresponds to the sequence encoding Reg1α. Such PCR methods include gene trapping and RACE methods.

Gene trapping may entail inserting a member of a cDNA library into a vector. The vector then may be denatured to produce single stranded molecules. Next, a substrate-bound probe, such a biotinylated oligo, may be used to trap cDNA inserts of interest. Biotinylated probes can be linked to an avidin-bound solid substrate. PCR methods can be used to amplify the trapped cDNA. To trap sequences corresponding to the full length genes, the labeled probe sequence may be based on the nucleic acid of SEQ ID Nos. 1 or 3, or a sequence complementary thereto. Random primers or primers specific to the library vector can be used to amplify the trapped cDNA. Such gene trapping techniques are described in Gruber et al., PCT WO 95/04745 and Gruber et al., U.S. Pat. No. 5,500,356. Kits are commercially available to perform gene trapping experiments from, for example, Life Technologies, Gaithersburg, Maryland, USA.

"Rapid amplification of cDNA ends," or RACE, is a PCR method of amplifying cDNAs from a number of different RNAs. The cDNAs may be ligated to an oligonucleotide linker and amplified by PCR using two primers. One primer may be based on sequence from the instant nucleic acids, for which full length sequence is desired, and a second primer may comprise a sequence that hybridizes to the oligonucleotide linker to amplify the cDNA. A description of this method is reported in PCT Pub. No. WO 97/19110.

In preferred embodiments of RACE, a common primer may be designed to anneal to an arbitrary adaptor sequence ligated to cDNA ends (Apte and Siebert, Biotechniques 15:890-893, 1993; Edwards et al., Nuc. Acids Res. 19:5227-5232, 1991). When a single gene-specific RACE primer is paired with the common primer, preferential amplification of sequences between the single gene specific primer and the common primer occurs. Commercial cDNA pools modified for use in RACE are available.

Once the full-length cDNA or gene is obtained, DNA encoding variants can be prepared by site-directed mutagenesis, described in detail in Sambrook 15.3-15.63. The choice of codon or nucleotide to be replaced can be based on the disclosure herein on optional changes in amino acids to achieve altered protein structure and/or function.

As an alternative method to obtaining DNA or RNA from a biological material, such as serum, nucleic acid comprising nucleotides having the sequence of one or more nucleic acids of the invention can be synthesized. Thus, the invention encompasses nucleic acid molecules ranging in length from about 8 nucleotides (corresponding to at least 12 contiguous nucleotides which hybridize under stringent conditions to or are at least 80% identical to the nucleic acid sequence of SEQ ID Nos. 1 or 3, or a sequence complementary thereto) up to a maximum length suitable for one or more biological manipulations, including replication and expression, of the nucleic acid molecule. The invention includes but is not limited to (a) nucleic acid having the size of the full Reg1α gene, or a sequence complementary thereto; (b) the nucleic acid of(a) also comprising at least one additional gene, operably linked to permit expression of a fusion protein; (c) an expression vector comprising (a) or (b); (d) a plasmid comprising (a) or (b); and (e) a recombinant viral particle comprising (a) or (b).

The sequence of a nucleic acid of the present invention is not limited and can be any sequence of A, T, G, and/or C (for DNA) and A, U, G, and/or C (for RNA) or modified bases thereof, including inosine and pseudouridine. The choice of sequence will depend on the desired function and can be dictated by coding regions desired, the intron-like regions desired, and the regulatory regions desired.

### Probe preparation

Prior to hybridization of a probe nucleic acid to a patient sample, the nucleic acid samples must be prepared to facilitate subsequent detection of hybridization. The nucleic acid samples obtained from an individual (including nucleic acid sequences encoding Reg1α, and optionally, at least one other colorectal cancer associated marker) to be screened for colorectal cancer are capable of being bound by a nucleic acid probe of complementary sequence through one or more types of chemical bonds, usually through complementary base pairing, usually through hydrogen bond formation.

Probes useful in the invention for hybridizing to and thus identifying the presence of Reg1α or TIMP1, and optionally, at least one additional colorectal cancer associated marker may be designed to hybridize to a polynucleotide molecule derived from an mRNA transcript coding for Reg1α, or optionally, at least one additional colorectal cancer associated marker. As used herein, a "polynucleotide derived from an mRNA transcript" refers to a polynucleotide for which synthesis of the mRNA transcript or a subsequence thereof has ultimately served as a template. Thus, a cDNA reverse transcribed from an mRNA, an RNA transcribed from that cDNA, a DNA amplified from the cDNA, an RNA transcribed from the amplified DNA, etc., are all derived from the mRNA transcript and detection of such derived products is indicative of the presence and/or abundance of the original transcript in a sample. Thus, suitable target nucleic acid samples include, but are not limited to, mRNA transcripts of a gene or genes (i.e., Reg1α or a colorectal cancer associated marker), cDNA reverse transcribed from the mRNA, cRNA transcribed from the cDNA, DNA amplified from a gene or genes, RNA transcribed from amplified DNA, and the like. The polynucleotide probes used herein are preferably designed to hybridize to Reg1α, or optionally to a sequence encoding at least one other colorectal cancer associated marker.

Nucleic acid probes may be generated using techniques which are well known to those of skill in the art (see, e.g., Sambrook et al., Molecular Cloning: A Laboratory Manual (2nd ed.), Vols. 1-3, Cold Spring Harbor Laboratory, (1989), or Current Protocols in Molecular Biology, F. Ausubel et al., ed. Greene Publishing and Wiley-Interscience, New York (1987).

In order to measure the hybridization of a probe nucleic acid to a target sequence in a sample, the probe nucleic acid is preferably labeled with a detectable label. Any analytically detectable marker that is attached to or incorporated into a molecule may be used in the invention. An analytically detectable marker refers to any molecule, moiety or atom which is analytically detected and quantified.

Detectable labels suitable for use in the present invention include any composition detectable by spectroscopic, photochemical, biochemical, immunochemical, electrical, optical or chemical means. Useful labels in the present invention include biotin for staining with labeled streptavidin conjugate, magnetic beads (e.g., DynabeadsTM), fluorescent dyes (e.g., fluorescein, texas red, rhodamine, green fluorescent protein, and the like), radiolabels (e.g., ³H, ¹²⁵I, 35S, ¹⁴C, or ³²P), enzymes (e.g., horse radish peroxidase, alkaline phosphatase and others commonly used in an ELISA), and colorimetric labels such as colloidal gold or colored glass or plastic (e.g., polystyrene, polypropylene, latex, etc.) beads. Patents teaching the use of such labels include U.S. Pat. Nos. 3,817,837; 3,850,752; 3,939,350; 3,996,345; 4,277,437; 4,275,149; and 4,366,241.

Means of detecting such labels are well known to those of skill in the art. Thus, for example, radiolabels may be detected using photographic film or scintillation counters, fluorescent markers may be detected using a photodetector to detect emitted light. Enzymatic labels are typically detected by providing the enzyme with a substrate and detecting the reaction product produced by the action of the enzyme on the substrate, and colorimetric labels are detected by simply visualizing the colored label.

The labels may be incorporated into a nucleic acid probe by any of a number of means well known to those of skill in the art. However, in a preferred embodiment, the label is simultaneously incorporated into the probe during an amplification step in the preparation of the probe polynucleotides. Thus, for example, polymerase chain reaction (PCR), or other amplification reaction, with labeled primers or labeled nucleotides will provide a labeled amplification product, and thus a labeled probe.

Alternatively, a label may be added directly to the probe. Means of attaching labels to polynucleotides are well known to those of skill in the art and include, for example nick translation or end-labeling (e.g. with a labeled RNA) and subsequent attachment (ligation) of a polynucleotide linker joining the sample polynucleotide to a label (e.g., a fluorophore).

In a preferred embodiment, the fluorescent modifications are by cyanine dyes e.g. Cy-3/Cy-5 dUTP, Cy-3/Cy-5 dCTP (Amersham Pharmacia) or alexa dyes (Khan, J., Simon, R., Bittner, M., Chen, Y., Leighton, S. B., Pohida, T., Smith, P. D., Jiang, Y., Gooden, G. C., Trent, J. M. & Meltzer, P. S. (1998) *Cancer Res.* 58, 50095013.).

In a preferred embodiment, a probe nucleic acid which is capable of hybridizing to Reg 1α and a probe nucleic acid which is capable of hybridizing to a nucleic acid sequence encoding at least one additional colorectal cancer associated marker, are co-hybridized to a test sample (e.g., a serum sample). In this embodiment, the two probe samples used for comparison are labeled with different fluorescent dyes which produce distinguishable detection signals, for example, probes hybridizable with Reg1α are labeled with Cy5 and probes hybridizable with another colorectal cancer associated marker are labeled with Cy3. The differently labeled target samples are hybridized to the same microarray simultaneously.

In a preferred embodiment, a control probe may be co-hybridized to a sample along with a probe for Reg1α and/or a probe for an additional colorectal cancer associated marker, wherein the control probe is capable of hybridizing to a nucleic acid sequence known to be found in the clinical sample, for example, where the clinical sample is a serum sample, a control sequence may be a sequence encoding serum albumin, or fibrinogen.

### Vectors and Host Cells

The present invention further provides vectors and plasmids useful for directing the expression of Reg1α or TIMP1 or other colorectal cancer associated markers, and further provides host cells which express the vectors and plasmids provided herein. Nucleic acid sequences useful for the expression from a vector or plasmid as described below include, but are not limited to any nucleic acid or gene sequence identified as being differentially regulated by the methods described above, and further include therapeutic nucleic acid molecules, such as antisense molecules. The host cell may be any prokaryotic or eukaryotic cell. Ligating the polynucleotide sequence into a gene construct, such as an expression vector, and transforming or transfecting into hosts, either eukaryotic (yeast, avian, insect or mammalian) or prokaryotic (bacterial cells), are standard procedures well known in the art.

### Vectors

There is a wide array of vectors known and available in the art that are useful for the expression of differentially expressed nucleic acid molecules according to the invention. The selection of a particular vector clearly depends upon the intended use the polypeptide encoded by the differentially expressed nucleic acid. For example, the selected vector must be capable of driving expression of the polypeptide in the desired cell type, whether that cell type be prokaryotic or eukaryotic. Many vectors comprise sequences allowing both prokaryotic vector replication and eukaryotic expression of operably linked gene sequences.

Vectors useful according to the invention may be autonomously replicating, that is, the vector, for example, a plasmid, exists extrachromosomally and its replication is not necessarily directly linked to the replication of the host cell's genome. Alternatively, the replication of the vector may be linked to the replication of the host's chromosomal DNA, for example, the vector may be integrated into the chromosome of the host cell as achieved by retroviral vectors.

Vectors useful according to the invention preferably comprise sequences operably linked to the sequence of interest (e.g., Reg1α) that permit the transcription and translation of the sequence. Sequences that permit the transcription of the linked sequence of interest include a promoter and optionally also include an enhancer element or elements permitting the strong expression of the linked sequences. The term "transcriptional regulatory sequences" refers to the combination of a promoter and any additional sequences conferring desired expression characteristics (e.g., high level expression, inducible expression, tissue- or cell-type-specific expression) on an operably linked nucleic acid sequence.

The selected promoter may be any DNA sequence that exhibits transcriptional activity in the selected host cell, and may be derived from a gene normally expressed in the host cell or from a gene normally expressed in other cells or organisms. Examples of promoters include, but are not limited to the following: A) prokaryotic promoters - *E. coli* lac, tac, or trp promoters, lambda phage P_{R} or P_{L} promoters, bacteriophage T7, T3, Sp6 promoters, *B. subtilis* alkaline protease promoter, and the *B. stearothermophilus* maltogenic amylase promoter, etc.; B) eukaryotic promoters - yeast promoters, such as GAL1, GAL4 and other glycolytic gene promoters (see for example, Hitzeman et al., 1980, J. Biol. Chem. 255: 12073 -12080; Alber & Kawasaki, 1982, J. Mol. Appl. Gen. 1: 419-434), LEU2 promoter (Martinez-Garcia et al., 1989, Mol Gen Genet. 217: 464-470), alcohol dehydrogenase gene promoters (Young et al., 1982, in Genetic Engineering of Microorganisms for Chemicals, Hollaender et al., eds., Plenum Press, NY), or the TPI1 promoter (U.S. Pat. No. 4,599,311); insect promoters, such as the polyhedrin promoter (U.S. Pat. No. 4,745,051; Vasuvedan et al., 1992, FEBS Lett. 311: 7-11), the P10 promoter (Vlak et al., 1988, J. Gen. Virol. 69: 765-776), the *Autographa californica* polyhedrosis virus basic protein promoter (EP 397485), the baculovirus immediate-early gene promoter gene 1 promoter (U.S. Pat. Nos. 5,155,037 and 5,162,222), the baculovirus 39K delayed-early gene promoter (also U.S. Pat. Nos. 5,155,037 and 5,162,222) and the OpMNPV immediate early promoter 2; mammalian promoters - the SV40 promoter (Subramani et al., 1981, Mol. Cell. Biol. 1: 854-864), metallothionein promoter (MT-1; Palmiter et al., 1983, Science 222: 809-814), adenovirus 2 major late promoter (Yu et al.,1984, Nucl. Acids Res. 12: 9309-21), cytomegalovirus (CMV) or other viral promoter (Tong et al., 1998, Anticancer Res. 18: 719-725), or even the endogenous promoter of a gene of interest in a particular cell type.

A selected promoter may also be linked to sequences rendering it inducible or tissue-specific. For example, the addition of a tissue-specific enhancer element upstream of a selected promoter may render the promoter more active in a given tissue or cell type. Alternatively, or in addition, inducible expression may be achieved by linking the promoter to any of a number of sequence elements permitting induction by, for example, thermal changes (temperature sensitive), chemical treatment (for example, metal ion- or IPTG-inducible), or the addition of an antibiotic inducing agent (for example, tetracycline).

Regulatable expression is achieved using, for example, expression systems that are drug inducible (e.g., tetracycline, rapamycin or hormone-inducible). Drug -regulatable promoters that are particularly well suited for use in mammalian cells include the tetracycline regulatable promoters, and glucocorticoid steroid-, sex hormone steroid-, ecdysone-, lipopolysaccharide (LPS)- and isopropylthiogalactoside (IPTG)-regulatable promoters. A regulatable expression system for use in mammalian cells should ideally, but not necessarily, involve a transcriptional regulator that binds (or fails to bind) nonmammalian DNA motifs in response to a regulatory agent, and a regulatory sequence that is responsive only to this transcriptional regulator.

Tissue-specific promoters may also be used to advantage in differentially expressed sequence-encoding constructs of the invention. A wide variety of tissue-specific promoters is known. As used herein, the term "tissue-specific" means that a given promoter is transcriptionally active (i.e., directs the expression of linked sequences sufficient to permit detection of the polypeptide product of the promoter) in less than all cells or tissues of an organism. A tissue specific promoter is preferably active in only one cell type, but may, for example, be active in a particular class or lineage of cell types (e.g., hematopoietic cells). A tissue specific promoter useful according to the invention comprises those sequences necessary and sufficient for the expression of an operably linked nucleic acid sequence in a manner or pattern that is essentially the same as the manner or pattern of expression of the gene linked to that promoter in nature. The following is a non-exclusive list of tissue specific promoters and literature references containing the necessary sequences to achieve expression characteristic of those promoters in their respective tissues; the entire content of each of these literature references is incorporated herein by reference. Examples of tissue specific promoters useful in the present invention are as follows:

Bowman et al., 1995 Proc. Natl. Acad. Sci. USA 92,12115-12119 describe a brain-specific transferrin promoter; the synapsin I promoter is neuron specific (Schoch et al., 1996 J. Biol. Chem. 271, 3317-3323); the nestin promoter is post-mitotic neuron specific (Uetsuki et al., 1996 J. Biol. Chem. 271, 918-924); the neurofilament light promoter is neuron specific (Charron et al., 1995 J. Biol. Chem. 270, 30604-30610); the acetylcholine receptor promoter is neuron specific (Wood et al., 1995 J. Biol. Chem. 270, 30933-30940); and the potassium channel promoter is high-frequency firing neuron specific (Gan et al., 1996 J. Biol. Chem 271, 5859-5865). Any tissue specific transcriptional regulatory sequence known in the art may be used to advantage with a vector encoding a differentially expressed nucleic acid sequence obtained from an animal subjected to pain.

In addition to promoter/enhancer elements, vectors useful according to the invention may further comprise a suitable terminator. Such terminators include, for example, the human growth hormone terminator (Palmiter et al., 1983, supra), or, for yeast or fungal hosts, the TPI1 (Alber & Kawasaki, 1982, supra) or ADH3 terminator (McKnight et al., 1985, EMBO J. 4: 2093-2099).

Vectors useful according to the invention may also comprise polyadenylation sequences (e.g., the SV40 or Ad5E1b poly(A) sequence), and translational enhancer sequences (e.g., those from Adenovirus VA RNAs). Further, a vector useful according to the invention may encode a signal sequence directing the recombinant polypeptide to a particular cellular compartment or, alternatively, may encode a signal directing secretion of the recombinant polypeptide.

### a. Plasmid vectors.

Any plasmid vector that allows expression of a coding sequence of interest (e.g., the coding sequence of Reg1α)in a selected host cell type is acceptable for use according to the invention. A plasmid vector useful in the invention may have any or all of the above-noted characteristics of vectors useful according to the invention. Plasmid vectors useful according to the invention include, but are not limited to the following examples: Bacterial - pQE70, pQE60, pQE-9 (Qiagen) pBs, phagescript, psiX174, pBluescript SK, pBsKS, pNH8a, pNH16a, pNH18a, pNH46a (Stratagene); pTrc99A, pKK223-3, pKK233-3, pDR540, and pRIT5 (Pharmacia); Eukaryotic - pWLneo, pSV2cat, pOG44, pXT1, pSG (Stratagene) pSVK3, pBPV, pMSG, and pSVL (Pharmacia). However, any other plasmid or vector may be used as long as it is replicable and viable in the host.

### b. Bacteriophage vectors.

There are a number of well known bacteriophage- derived vectors useful according to the invention. Foremost among these are the lambda-based vectors, such as Lambda Zap II or Lambda-Zap Express vectors (Stratagene) that allow inducible expression of the polypeptide encoded by the insert. Others include filamentous bacteriophage such as the M13-based family of vectors.

### c. Viral vectors.

A number of different viral vectors are useful according to the invention, and any viral vector that permits the introduction and expression of one or more of the polynucleotides of the invention in cells is acceptable for use in the methods of the invention. Viral vectors that can be used to deliver foreign nucleic acid into cells include but are not limited to retroviral vectors, adenoviral vectors, adeno-associated viral vectors, herpesviral vectors, and Semliki forest viral (alphaviral) vectors. Defective retroviruses are well characterized for use in gene transfer (for a review see Miller, A.D. (1990) *Blood* 76:271). Protocols for producing recombinant retroviruses and for infecting cells *in vitro* or *in vivo* with such viruses can be found in Current Protocols in Molecular Biology, Ausubel, F.M. et al. (eds.) Greene Publishing Associates, (1989), Sections 9.10-9.14, and other standard laboratory manuals.

In addition to retroviral vectors, Adenovirus can be manipulated such that it encodes and expresses a gene product of interest but is inactivated in terms of its ability to replicate in a normal lytic viral life cycle (see for example Berkner et al., 1988, BioTechniques 6:616; Rosenfeld et al., 1991, Science 252:431-434; and Rosenfeld et al., 1992, Cell 68:143-155). Suitable adenoviral vectors derived from the adenovirus strain Ad type 5 dl324 or other strains of adenovirus (e.g., Ad2, Ad3, Ad7 etc.) are well known to those skilled in the art. Adeno-associated virus (AAV) is a naturally occurring defective virus that requires another virus, such as an adenovirus or a herpes virus, as a helper virus for efficient replication and a productive life cycle. (For a review see Muzyczka et al., 1992, Curr. Topics in Micro. and Immunol. 158:97-129). An AAV vector such as that described in Traschin et al. (1985, Mol. Cell. Biol. 5:3251-3260) can be used to introduce nucleic acid into cells. A variety of nucleic acids have been introduced into different cell types using AAV vectors. (see, for example, Hermonat et al., 1984, Proc. Natl. Acad. Sci. USA 81: 6466-6470; and Traschin et al., 1985, Mol. Cell. Biol. 4: 2072-2081).

### Host cells

Any cell into which a recombinant vector carrying a gene of interest (e.g., a sequence encoding Reg1α) may be introduced and wherein the vector is permitted to drive the expression of the peptide encoded by the differentially expressed sequence is useful according to the invention. Any cell in which a differentially expressed molecule of the invention may be expressed and preferably detected is a suitable host, wherein the host cell is preferably a mammalian cell and more preferably a human cell. Vectors suitable for the introduction of nucleic acid sequences to host cells from a variety of different organisms, both prokaryotic and eukaryotic, are described herein above or known to those skilled in the art.

Host cells may be prokaryotic, such as any of a number of bacterial strains, or may be eukaryotic, such as yeast or other fungal cells, insect or amphibian cells, or mammalian cells including, for example, rodent, simian or human cells. Cells may be primary cultured cells, for example, primary human fibroblasts or keratinocytes, or may be an established cell line, such as NIH3T3, 293T or CHO cells. Further, mammalian cells useful in the present invention may be phenotypically normal or oncogenically transformed. It is assumed that one skilled in the art can readily establish and maintain a chosen host cell type in culture.

### Introduction of vectors to host cells.

Vectors useful in the present invention may be introduced to selected host cells by any of a number of suitable methods known to those skilled in the art. For example, vector constructs may be introduced to appropriate bacterial cells by infection, in the case of E. coli bacteriophage vector particles such as lambda or M 13, or by any of a number of transformation methods for plasmid vectors or for bacteriophage DNA. For example, standard calcium-chloride-mediated bacterial transformation is still commonly used to introduce naked DNA to bacteria (Sambrook et al., 1989, Molecular Cloning, A Laboratory Manual, Cold Spring Harbor Laboratory Press, Cold Spring Harbor, NY), but electroporation may also be used (Ausubel et al., 1988, Current Protocols in Molecular Biology, (John Wiley & Sons, Inc., NY, NY)).

For the introduction of vector constructs to yeast or other fungal cells, chemical transformation methods are generally used (e.g. as described by Rose et al., 1990, Methods in Yeast Genetics, Cold Spring Harbor Laboratory Press, Cold Spring Harbor, NY). For transformation of S. cerevisiae, for example, the cells are treated with lithium acetate to achieve transformation efficiencies of approximately 10⁴ colony-forming units (transformed cells)/µg of DNA. Transformed cells are then isolated on selective media appropriate to the selectable marker used. Alternatively, or in addition, plates or filters lifted from plates may be scanned for GFP fluorescence to identify transformed clones.

For the introduction of vectors comprising a sequence of interest to mammalian cells, the method used will depend upon the form of the vector. Plasmid vectors may be introduced by any of a number of transfection methods, including, for example, lipid-mediated transfection ("lipofection"), DEAE-dextran-mediated transfection, electroporation or calcium phosphate precipitation. These methods are detailed, for example, in Current Protocols in Molecular Biology (Ausubel et al., 1988, John Wiley & Sons, Inc., NY, NY).

Lipofection reagents and methods suitable for transient transfection of a wide variety of transformed and non-transformed or primary cells are widely available, making lipofection an attractive method of introducing constructs to eukaryotic, and particularly mammalian cells in culture. For example, LipofectAMINE™ (Life Technologies) or LipoTaxi™ (Stratagene) kits are available. Other companies offering reagents and methods for lipofection include Bio-Rad Laboratories, CLONTECH, Glen Research, In Vitrogen, JBL Scientific, MBI Fermentas, PanVera, Promega, Quantum Biotechnologies, Sigma-Aldrich, and Wako Chemicals USA.

Following transfection with a vector of the invention, eukaryotic (e.g., human) cells successfully incorporating the construct (intra- or extrachromosomally) may be selected, as noted above, by either treatment of the transfected population with a selection agent, such as an antibiotic whose resistance gene is encoded by the vector, or by direct screening using, for example, FACS of the cell population or fluorescence scanning of adherent cultures. Frequently, both types of screening may be used, wherein a negative selection is used to enrich for cells taking up the construct and FACS or fluorescence scanning is used to further enrich for cells expressing differentially expressed polynucleotides or to identify specific clones of cells, respectively. For example, a negative selection with the neomycin analog G418 (Life Technologies, Inc.) may be used to identify cells that have received the vector, and fluorescence scanning may be used to identify those cells or clones of cells that express the vector construct to the greatest extent.

### Reg1α and TIMP1 Polypeptides

The present invention provides a method for the detection of colorectal cancer in an individual by detecting the presence of Reg1α or TIMP1 in a clinical sample from an individual. In addition the invention encompasses the detection of cancer by identifying Reg1α or TIMP1 gene product in colon tissue or cells. Alternatively, the invention relates to a method for the detection of colorectal cancer in an individual wherein colorectal cancer is identified by detecting the presence of Reg1α or TIMP1 and at least one additional colorectal cancer associated marker in the clinical sample from an individual. Polypeptides of the present invention, the detection of which is indicative of colorectal cancer include those having the sequence shown in one or more of SEQ ID Nos. 2, 4, or 100, or alternatively, which are encoded by one or more of SEQ ID Nos. 1, 3 or 33.

Preferred polypeptides which can be detected and are thus indicative of colorectal cancer in an individual are those that are encoded by nucleic acid sequences at least about 70%, 75%, 80%, 90%, 95%, 97%, or 98% identical to a mRNA sequence complementary to the nucleic acid sequence of SEQ ID Nos. 1, 3 or 33. Particularly preferred polypeptides are those of SEQ ID Nos. 2, 4, or 99, or fragments thereof, or polypeptide sequences which are at least about 70%, 75%, 80%, 90%, 95%, 98% or 99% identical in sequence to the amino acid sequence of one or more of SEQ ID Nos. 2, 4, or 100.

In addition to a method for detecting colorectal cancer by identifying the presence of the Reg1α or TIMP1 polypeptide in a clinical sample from an individual, the invention further comprises a method of detecting cancer by identifying the presence of Reg1α or TIMP1 in addition to at least one other colorectal cancer associated marker in the same sample (e.g., in the same serum, tissue, or cell sample).

### Antibodies

The invention provides a method for colorectal cancer detection comprising the step of detecting the presence of Reg1α or TIMP1 (and optionally, at least one additional colorectal cancer associated marker) in a clinical sample from an individual. In one embodiment, the presence of Reg1α or TIMP1, or other marker, in such a sample is detected using a polypeptide ligand which is preferably detectably labeled, and is capable of binding to Reg1α or TIMP1 , and if present, the other marker, in the sample. In a preferred embodiment, the polypeptide ligand is an antibody. Antibodies of the invention include, but are not limited to, polyclonal, monoclonal, multispecific, human, humanized, or chimeric antibodies, single chain antibodies, Fab fragments, Fv fragments F(ab') fragments, fragments produced by a Fab expression library, anti -iodiotypic antibodies, or other epitope binding polypeptide. Preferably, an antibody, useful in the present invention for the detection of Reg1α or TIMP1 (and optionally at least one additional colorectal cancer associated marker), is a human antibody or fragment thereof, including scFv, Fab, Fab', F(ab'), Fd, single chain antibody, of Fv. Antibodies, useful in the invention may include a complete heavy or light chain constant region, or a portion thereof, or an absence thereof. An antibody, useful in the invention, may be obtained from an art recognized host, such as rabbit, mouse, rat, donkey, sheep, goat, guinea pig, camel, horse, or chicken. In one embodiment, an antibody, useful in the invention can be a humanized antibody, in which amino acids have been replaced in the non-antigen binding regions in order to more closely resemble a human antibody, while still retaining the original binding ability. Methods for making humanized antibodies are described in Teng et al., 1983, *Proc. Natl. Acad. Sci. USA 80:* 7308-7312; Kozbor et al., 1983, *Immunology Today 4*: 7279; Olsson et al., 1982, *Meth. Enzymol*. *92:* 3-16; WO 92/06193; EP 0239400.

Antibodies of the present invention may be monospecific, dispecific, trispecific, or of greater multispecificity. As such, Reg1α or TIMP1 and optionally an additional colorectal cancer associated marker useful for the detection of colorectal cancer may be detected with separate antibodies, or may be detected with the same antibody. Alternatively, a multispecific antibody may exhibit different specificities for different epitopes on the same protein (e.g., different epitopes on Reg1α). While specificity of an antibody useful in the present invention to either Reg1α or one or more additional colorectal cancer associated markers is preferred, antibodies that bind polypeptides with at least 95%, 90%, 85%, 75%, 65%, 55%, and at least 50% identity to a polypeptide useful in the present invention for the detection of colorectal cancer (i.e., Reg1α, and/or an additional colorectal cancer associated marker) are also included in the present invention. Also encompassed in the present invention are antibodies which bind to polypeptide molecules which are encoded by one or more nucleic acid sequences which are complementary to, or hybridize to the sequences of SEQ ID Nos. 1, 3 or 33, or one or more sequences which are complementary to, or hybridize to a nucleic acid sequence which encodes an additional colorectal cancer associated marker as described herein.

Antibodies of the present invention which are useful for the detection of colorectal cancer may further act as agonists or antagonists of the activity of the polypeptide molecules to which they bind, and may thus be useful as therapeutic molecules for the treatment or prevention of colorectal cancer.

An important, but not limiting, role of an antibody of the present invention is to provide for the purification, or detection of Reg 1α or TIMP1 or other colorectal cancer associated markers in a patient sample, including both in vitro and in vivo detection methods. Antibodies useful for the detection of colorectal cancer as described herein do not have to be used alone, and can be fused to other polypeptides, including a heterologous polypeptide at the N- or C-terminus of the antibody polypeptide sequence. For example, an antibody useful in the present invention may be fused with a detectable label to facilitate detection of the antibody when bound to a target polypeptide. Methods for detectably labeling an antibody polypeptide are known to those of skill in the art.

For the production of antibodies useful in the present invention, various hosts including goats, rabbits, rats, mice, etc., may be immunized by injection with the protein products (or any portion, fragment, or oligonucleotide thereof which retains immunogenic properties) of the candidate genes of the invention. Depending on the host species, various adjuvants may be used to increase the immunological response. Such adjuvants include but are not limited to Freund's, mineral gels such as aluminum hydroxide, and surface active substances such as lysolecithin, pluronic polyols, polyanions, peptides, oil emulsions, keyhole limpet hemocyanin, and dinitrophenol. BCG (bacilli Calmette-Guerin) and *Corynebacterium parvum* are potentially useful human adjuvants.

Polyclonal antisera or monoclonal antibodies can be made using methods known in the art. A mammal such as a mouse, hamster, or rabbit, can be immunized with an immunogenic form of a Reg1α or TIMP1 polypeptide, fragment, modified form thereof, or variant form thereof. Alternatively, an animal may be immunized with an immunogenic form of one or more additional colorectal cancer associated marker polypeptides. Techniques for conferring immunogenicity on such molecules include conjugation to carriers or other techniques well known in the art. For example, the immunogenic molecule can be administered in the presence of adjuvant as described above. Immunization can be monitored by detection of antibody titers in plasma or serum. Standard immunoassay procedures can be used with the immunogen as antigen to assess the levels and the specificity of antibodies. Following immunization, antisera can be obtained and, if desired, polyclonal antibodies isolated from the sera.

To produce monoclonal antibodies, antibody producing cells (lymphocytes) can be harvested from an immunized animal and fused with myeloma cells by standard somatic cell fusion procedures thus immortalizing these cells and yielding hybridoma cells. Such techniques are well known in the art (see, e.g., Kohler and Milstein, 1975, *Nature 256:* 495-497; Kozbor et al., 1983, *Immunol. Today 4*: 72, Cole et al., 1985, In *Monoclonal Antibodies in Cancer Therapy,* Allen R. Bliss, Inc., pages 77-96). Additionally, techniques described for the production of single-chain antibodies (U.S. Patent No. 4,946,778) can be adapted to produce antibodies according to the invention.

Antibody fragments which can specifically bind to a polypeptide of the invention such as Reg1α or TIMP1 or other colorectal cancer associated marker polypeptides, fragments thereof, modified forms thereof, and variants thereof, also may be generated by known techniques. For example, such fragments include, but are not limited to, F(ab')₂ fragments which can be produced by pepsin digestion of the antibody molecule and the Fab fragments which can be generated by reducing the disulfide bridges of the F(ab')₂ fragments. VH regions and FV regions can be expressed in bacteria using phage expression libraries (e.g., Ward et al., 1989, *Nature 341:* 544-546; Huse et al., 1989, *Science 246:* 1275-1281; McCafferty et al., 1990, *Nature 348:* 552-554).

Chimeric antibodies, i.e., antibody molecules that combine a non-human animal variable region and a human constant region also are within the scope of the invention. Chimeric antibody molecules include, for example, the antigen binding domain from an antibody of a mouse, rat, or other species, with human constant regions. Standard methods may be used to make chimeric antibodies containing the immunoglobulin variable region which recognizes the gene product of Reg1α antigens of the invention (see, e.g., Morrison et al., 1985, *Proc. Natl. Acad. Sci. USA 81:* 6851; Takeda et al., 1985, *Nature 314:* 452; U.S. Patent No. 4,816,567; U.S. Patent No. 4,816,397).

### Other Colorectal cancer Specific Analysis

In addition to the detection of colorectal cancer by identifying expression of Reg1α or TIMP1, or detecting Reg1α or TIMP1 polypeptides, the present invention further comprises a method for detecting colorectal cancer wherein a nucleic acid molecule encoding Reg1α or TIMP1, or Reg 1α or TIMP 1 polypeptide is identified in combination with at least one other nucleic acid sequence encoding a known colorectal cancer associated marker in a clinical sample from an individual. Alternatively, the presence of Reg1α or TIMP1 is detected in combination with at least one additional colorectal cancer marker amino acid sequence. Similar to the methods described above for Reg1α, a nucleic acid molecule which encodes at least one other colorectal cancer associated marker may be used to generate a nucleic acid probe for detection of the colorectal cancer associated marker sequence in a patient sample, or may be used to generate amplification primers to amplify the colorectal cancer associated marker sequence from a patient sample comprising the sequence, thus identifying the presence of the colorectal cancer associated marker in the sample, and thus indicating the detection of colorectal cancer. A colorectal cancer associated marker polypeptide sequence may be used, as described above for Reg1α to generate antibodies useful for detection of the colorectal cancer associated marker in a clinical sample. Methods for detecting a colorectal cancer associated marker nucleic acid or amino acid sequence are described below, and may be adapted from the methods for the detection of Reg1α nucleic acid or amino acid in a clinical sample.
A "colorectal cancer associated marker" useful in the present invention, refers to a polypeptide or nucleic acid sequence which exhibits over- or underexpression of at least 10% in colorectal cancer cells, tissue, or serum obtained from an individual having colorectal cancer, relative to the level of expression in cells, tissue, or serum obtained from an individual that does not have colorectal cancer. Non-limiting examples of colorectal cancer associated markers useful in the present invention include the nucleic acid molecules of SEQ ID Nos 1, 3, 5-71, and/or the polypeptide molecules of SEQ ID Nos 2,4, 72-138. In one embodiment, the polypeptide sequences of SEQ ID Nos 2, 4, 72-138 are encoded by the nucleic acid sequences of 1, 3, 5-71, respectively. It will be appreciated by one of skill in the art that, where the method of the invention relates to detection of Reg1α and at least one other colorectal cancer associated marker, TIMP1 may be included as a potential "other colorectal cancer associated marker". Likewise, where the detection method is based on the detection of TIMP1 and at least one other colorectal cancer associated marker, Reg1α may be included as a potential "other colorectal cancer associated marker". Alternatively, a colorectal cancer associated marker, as used in the present invention, may refer to a carbohydrate epitope present on a polypeptide or nucleic acid molecule and/or an antibody molecule which recognizes and is capable of binding to such an epitope, wherein the carbohydrate epitope is known to be associated with the presence of colorectal cancer in an individual. Such carbohydrate epitopes may be present on more than one unrelated protein or polypeptide. In one embodiment, such a carbohydrate epitope is CA 19-9, also known as sialyl-Lewis^{a}, is a tumor marker defined by a monoclonal antibody as a carbohydrate epitope, related to the blood group antigens, composed of a branching, 5-sugar structure covalently bound to a variety of glycoproteins or glycolipids. The proteins primarily belong to the mucin family and the lipids are usually membrane associated. The CA 19-9 epitope is typically the terminal moiety of a complex, O-linked carbohydrate structure on either macromolecule. Other tumor markers also defined as various carbohydrate epitopes useful in the present invention as a "colorectal cancer associated marker" include CA72-4 which is indicative of the presence of the Tag 72 antigen, which is a triply sialylated Tn antigen on varing protein backbones; Thomsen Freidenreich antigen (TF), which is a sialylated n-acetyl galactosamine moeity O-linked to various peptides; Tn and sialyated Tn (sTn) which is the backbone of the TF antigen without the terminal n-acetyl galactosamine moeity, O-linked to various peptides; CA 50 which is an epitope corresponding to sialylated Lewis A blood group antigen; CA 549 which is a CHO moiety on muc-1; CA 242 which is a sialylated CHO; LASA which is a lipid associated sialic acid, that is, a lipid without a protein associated to it; Du-PAN's 1-5, which are pancreatic associated mucin-like CHO antigens. These useful colon cancer specific antigens and others are known in the art and are described, for example, in "Serological Cancer Markers" Sell, S., Ed. 1992. Humana Press Inc., Totowa, NJ.

Table 1 below shows a list of "colorectal cancer associated markers" useful in the invention (although colorectal cancer associated markers useful in the invention are not limited to those shown in Table 1), and there correspondence with the sequences set forth in the "Sequence listing".

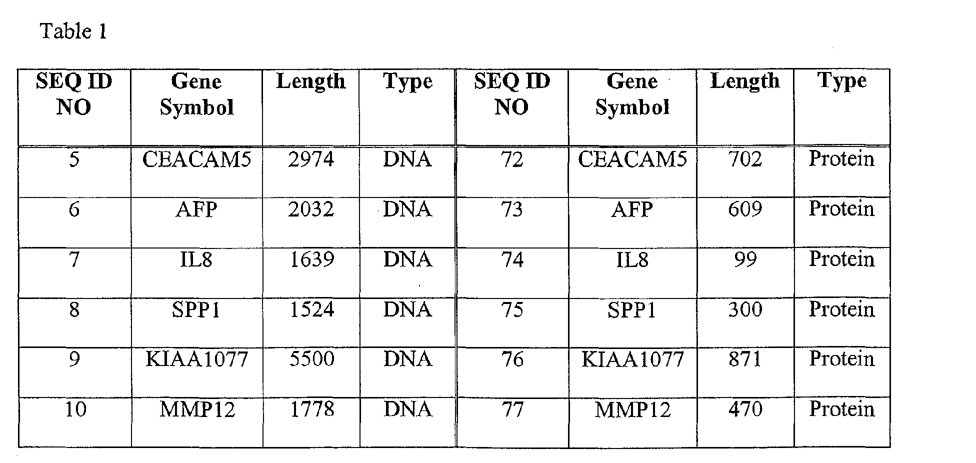

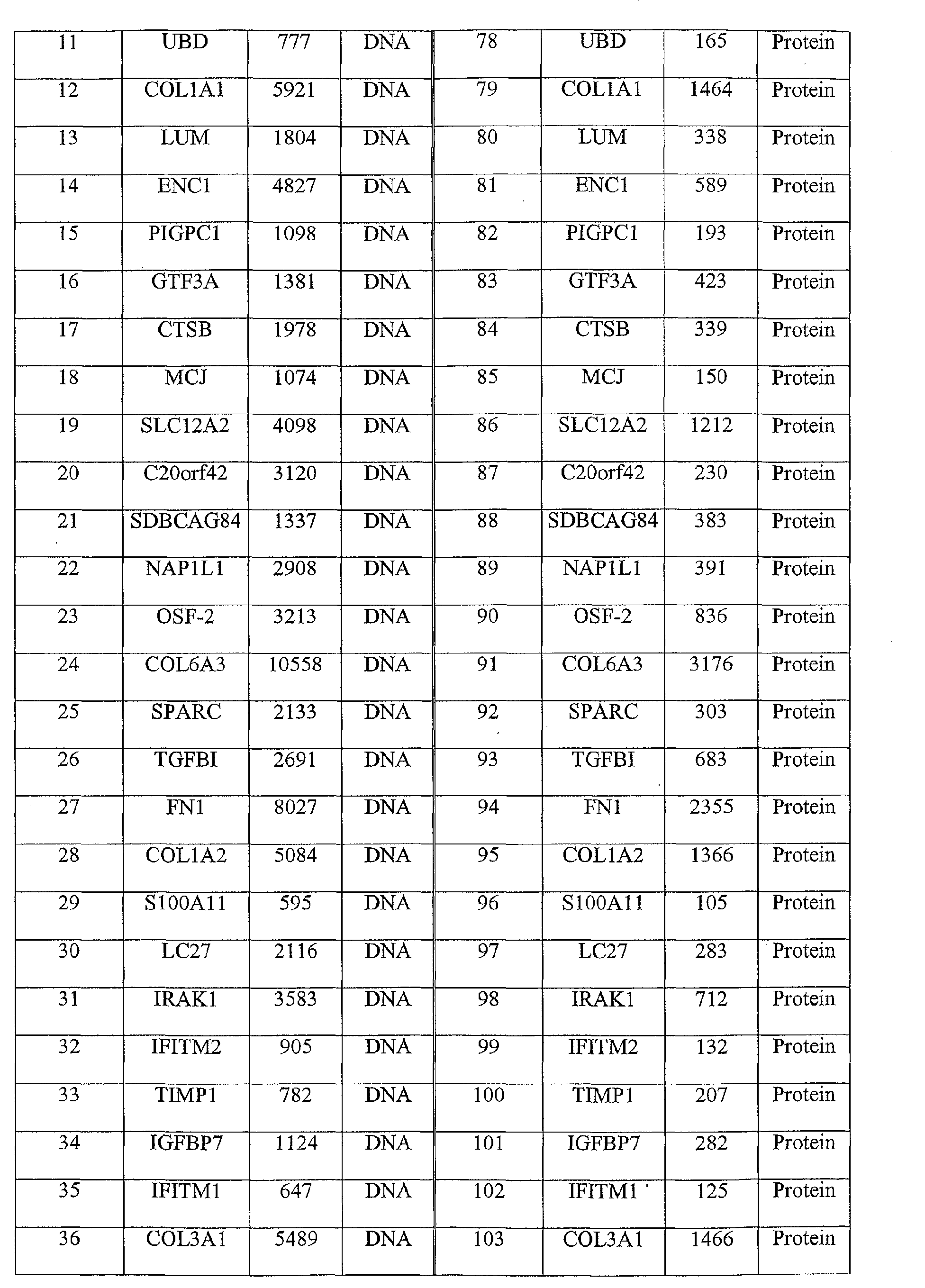

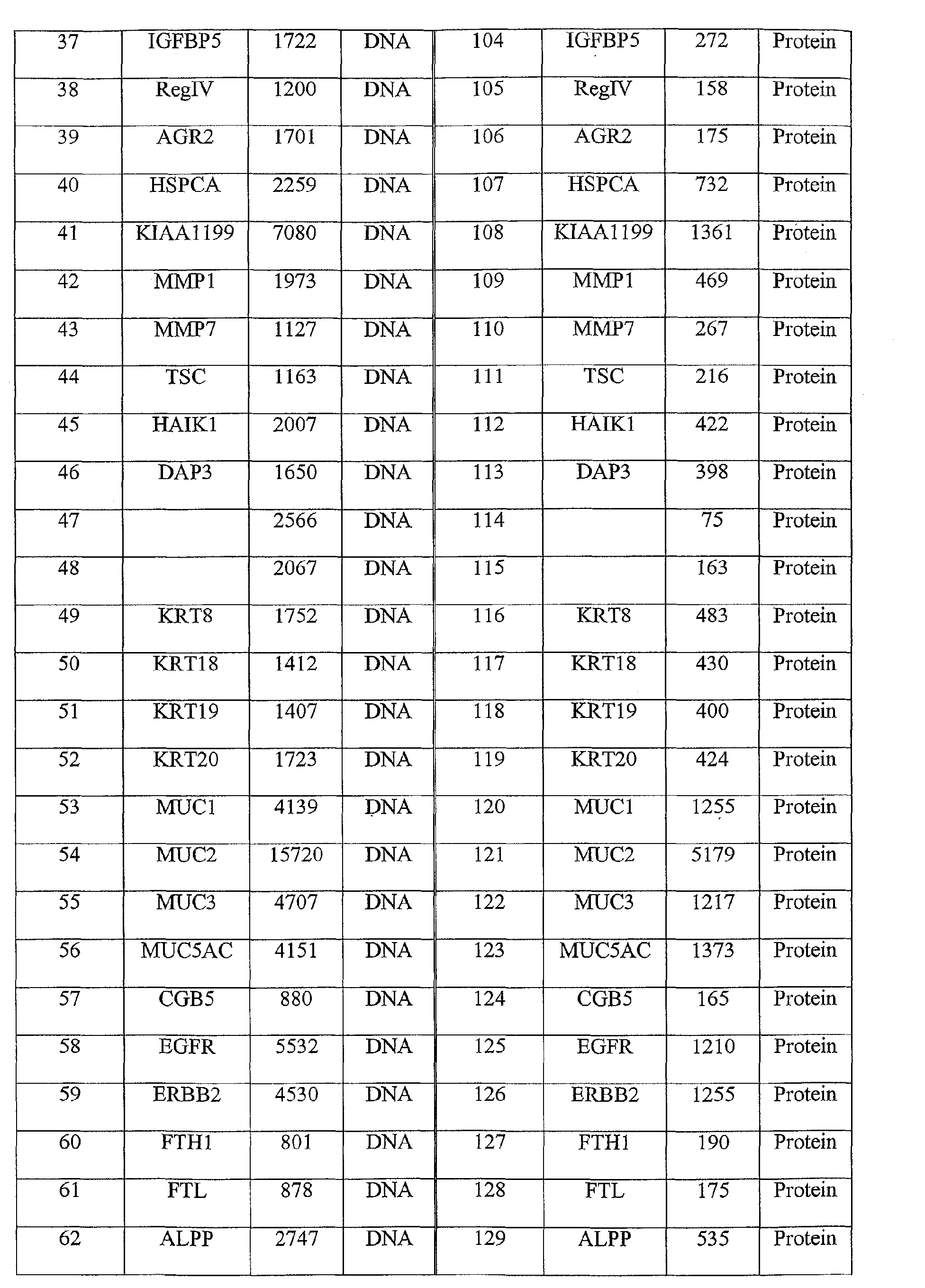

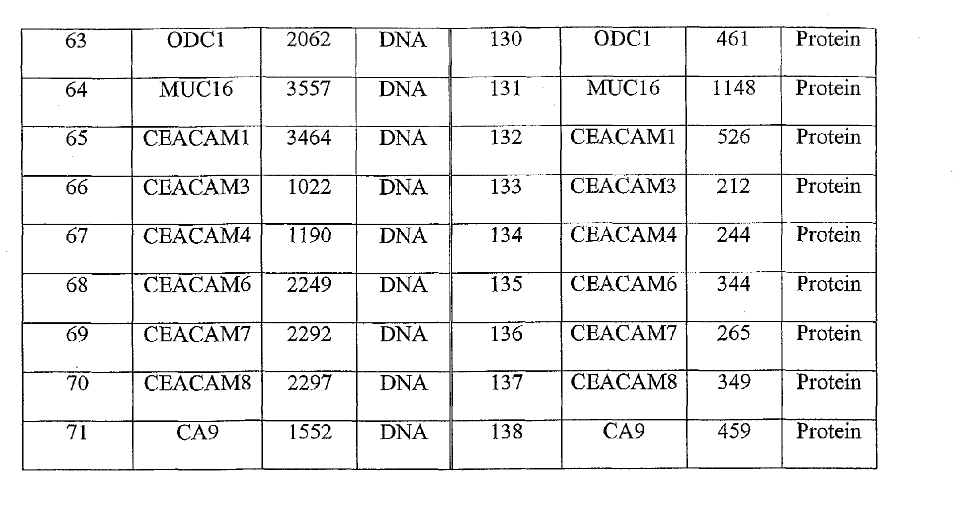

### Detection Assays

The present invention provides method for detecting colorectal cancer, or alternatively, determining whether a subject is at risk for developing colorectal cancer by detecting the disclosed biomarkers (i.e., the nucleic acid sequence of Reg1α or TIMP1 and optionally, one or more nucleic acid sequences encoding an additional colorectal cancer associated marker and/or polypeptide markers such as Reg1α or TIMP1 and optionally, at least one additional colorectal cancer associated marker) for the disease or condition encoded thereby.

In clinical applications, human tissue samples, preferably serum, can be screened for the presence and/or absence of Reg1α or TIMP1 and/or other colorectal cancer associated markers identified herein. Such samples may comprise tissue samples, whole cells, cell lysates, or isolated nucleic acids, including, for example, needle biopsy cores, surgical resection samples, lymph node tissue, or serum. A sample for analysis as described herein is preferably a serum sample. A serum sample may be obtained from an individual using methods which are well known to those of skill in the art. Briefly, a whole venous or arterial blood sample from an individual is collected into a test tube. The whole blood sample is permitted to incubate at room temperature for approximately 15-30 to allow the blood to clot. Once clotted, the sample is centrifuged at approximately 1500 to 3000 rpm for 5-30 minutes to completely separate the serum from the cellular components. This centrifugation may be repeated if necessary to achieve complete separation. The resulting serum sample may be subsequently screened for the presence of Reg1α nucleic acid or amino acid and/or one or more additional colorectal cancer associated markers as described herein.

### Screening for nucleic acid molecules

In one embodiment, the detection method of the present invention comprises determining whether a clinical sample from an individual contains mRNA of a colorectal cancer associated marker, preferably Reg1α or TIMP1, but also optionally including additional colorectal cancer associated markers as described herein. Techniques for determining the presence of a nucleic acid molecule of interest include Northern blot analysis, reverse transcription-polymerase chain reaction (RT-PCR), in situ hybridization, PCR, and quantitative amplification.

Prior to detection of target nucleic acid molecules in a clinical sample, it is preferred to first isolate the mRNA from the sample to facilitate detection of the target sequence (i.e., a sequence encoding Reg1α or TIMP1). Methods for isolation of mRNA from a biological sample are well known in the art. Briefly, where the sample is a serum sample, for example, 0.1 ml of 2 M sodium acetate, pH 4, 1 ml water-saturated phenol, and 0.2 ml of 49:1 chloroform/isoamyl alcohol are added to the serum sample sequentially. The sample is mixed after the addition of each component, and incubated for 15 min at 0-4°C after all components have been added. The sample is separated by centrifugation for 20 min at 10,000 x g, 4°C, precipitated by the addition of 1 ml of 100% isopropanol, incubated for 30 minutes at -20°C and pelleted by centrifugation for 10 minutes at 10,000 x g, 4°C. The resulting RNA pellet is dissolved in 0.3 ml denaturing solution, transferred to a microfuge tube, precipitated by the addition of 0.3 ml of 100% isopropanol for 30 minutes at -20°C, and centrifuged for 10 minutes at 10,000 x g at 4°C. The RNA pellet is washed in 70% ethanol, dried, and resuspended in 100-200µl DEPC-treated water or DEPC-treated 0.5% SDS (Chomczynski and Sacchi, 1987, Anal. Biochem., 162: 156).

Alternatively, total RNA may be extracted from a clinical sample according to the present invention using a commercially available RNA isolation reagent such as Trizol (Invitrogen, Carlsbad, CA), following the manufacturers instructions. Purity and integrity of RNA is assessed by absorbance at 260/280 nm and separation of RNA samples on a 1% agarose gel followed by inspection under ultraviolet light.

Following mRNA isolation, the mRNA may be reverse transcribed to provide a cDNA sample according to methods well known to those of skill in the art (see, e.g., Ausubel et al. (1995), Short Protocols in Molecular Biology, 3^{rd} Ed. John Wiley and Sons, Inc.)

Accordingly, in one aspect, the invention provides probes and primers that specifically hybridize to the Reg1α or TIMP1 nucleic acid sequences disclosed herein, or which can hybridize to a nucleic acid molecule encoding an additional colorectal cancer associated marker as described herein. Accordingly, the nucleic acid probes comprise a region of a nucleic acid sequence of SEQ ID Nos 1, 3, or 33 sufficient to hybridize with a nucleic acid substantially complementary to the sequence of SEQ ID Nos 1, 3 or 33. Preferred nucleic acid molecules for use as probes/primers can further comprise a region of nucleic acid sequence substantially complementary to the sequence of SEQ ID Nos. 1, 3 or 33 sufficient to hybridize with the sequence of SEQ ID Nos. 1, 3 or 33. In addition, nucleic acid sequences useful as probes/primers comprise a nucleotide sequence at least about 8 nucleotides in length, at least about 12 nucleotides in length, preferably at least about 15 nucleotides, more preferably about 25 nucleotides, and most preferably at least 40 nucleotides, and up to all or nearly all of the coding sequence which is complementary to a portion of the coding sequence of a marker nucleic acid sequence, which nucleic acid sequence is represented by SEQ ID Nos: 1, 3 or 33, or a sequence complementary thereto.

In one embodiment, the method comprises using a nucleic acid probe to determine the presence of a Reg1α or TIMP1 nucleic acid molecule in a clinical sample (such as a serum sample or a nucleic acid sample extracted therefrom). Specifically, the method comprises:
1. Providing a nucleic acid probe comprising a nucleotide sequence at least about 8 nucleotides in length, at least about 12 nucleotides in length, preferably at least about 15 nucleotides, more preferably about 25 nucleotides, and most preferably at least about 40 nucleotides, and up to all or nearly all of the coding sequence which is complementary to a portion of the coding sequence of a nucleic acid sequence represented by SEQ ID Nos: 1, 3 or 33, or a sequence complementary thereto;
2. Obtaining a clinical sample from a patient potentially comprising a Reg1α or TIMP1 nucleic acid sequence;
3. Providing a second clinical sample from an individual known to not have colorectal cancer;
4. Contacting the nucleic acid probe under stringent conditions with RNA of each of said first and second clinical samples (e.g., in a Northern blot or in situ hybridization assay); and
5. Comparing (a) the amount of hybridization of the probe with RNA of the first serum sample, with (b) the amount of hybridization of the probe with RNA of the second clinical sample; wherein a statistically significant difference in the amount of hybridization with the RNA of the first clinical sample as compared to the amount of hybridization with the RNA of the second clinical sample is indicative of the presence of Reg 1α or TIMP1 in the first clinical sample.

Although, primarily drawn to detection of Reg1α or TIMP1 in a clinical sample such as serum, in one aspect, the present invention provides a method comprising *in situ* hybridization detection of Reg1α or TIMP1 with a probe derived from a nucleic acid sequence represented by SEQ ID Nos: 1, 3 or 33, or a sequence complementary thereto. Preferably, the hybridization probe is detectably labeled. The method comprises contacting the labeled hybridization probe with a tissue or cell sample from an individual suspected of having colorectal cancer, washing off any unbound probe, and detecting the signal produced by the detectable label, wherein the detection of the detectable signal is indicative of the presence of Reg1α or TIMP1 in the sample, and thus permits the detection of colorectal cancer. Alternatively, the tissue or cell is additionally hybridized with a detectably labeled nucleic acid probe which is capable of specifically hybridizing with a nucleic acid sequence that encodes at least one additional colorectal cancer associated marker. Detection of the second detectably labeled probe is thus indicative of the presence of the additional colorectal cancer associated marker in the sample, and in conjunction with the detection of Reg1α or TIMP1, permits the detection of colorectal cancer in the individual. Specific methods for *in situ* hybridization are well known in the art.

Alternatively, methods such as PCR, Northern analysis, and Taqman may be used to detect and/or quantitate the expression of a nucleic acid sequence encoding Reg1α in a clinical sample. In one embodiment, reverse transcription PCR (RT-PCR) is performed using primers designed to specifically hybridize to a predetermined portion of the Reg1α mRNA sequence isolated from a clinical sample. Generation of a PCR product by such a reaction is thus indicative of the presence of the Reg1α or TIMP1 sequence in the sample. The technique of designing primers for PCR amplification is well known in the art. Oligonucleotide primers and probes are 5 to 100 nucleotides in length, ideally from 17 to 40 nucleotides, although primers and probes of different length are of use. Primers for amplification are preferably about 17 -25 nucleotides. Primers useful according to the invention are also designed to have a particular melting temperature (Tm) by the method of melting temperature estimation. Commercial programs, including Oligo™ (MBI, Cascade, CO), Primer Design and programs available on the internet, including Primer3 and Oligo Calculator can be used to calculate a Tm of a nucleic acid sequence useful according to the invention. Preferably, the Tm of an amplification primer useful according to the invention, as calculated for example by Oligo Calculator, is preferably between about 45 and 65° C and more preferably between about 50 and 60° C. Preferably, the Tm of a probe useful according to the invention is 7° C higher than the Tm of the corresponding amplification primers. It is preferred that, following generation of cDNA by RT-PCR, the cDNA fragment is cloned into an appropriate sequencing vector, such as a PCRII vector (TA cloning kit; Invitrogen). The identity of each cloned fragment is then confirmed by sequencing in both directions. It is expected that the sequence obtained from sequencing would be the same as the known sequence of Reg1α to TIMP1 as described herein.

Alternatively, the presence of an mRNA sequence encoding Reg1α or TIMP1 may be detected by Northern analysis. Sequence confirmed cDNAs, that is, cDNAs encoding Reg1α or TIMP1 (or alternatively an additional colorectal cancer associated marker) are used to produce ³²P-labeled cDNA probes using techniques well known in the art (see, for example, Ausubel, *supra*). Labeled probes for Northern analysis may also be produced using commercially available kits (Prime-It Kit, Stratagene, La Jolla, CA). Northern analysis of total RNA obtained from a clinical sample may be performed using classically described techniques. For example, total RNA samples are denatured with formaldehyde / formamide and run for two hours in a 1 % agarose, MOPS-acetate-EDTA gel. RNA is then transferred to nitrocellulose membrane by upward capillary action and fixed by UV cross-linkage. Membranes are pre -hybridized for at least 90 minutes and hybridized overnight at 42° C. Post hybridization washes are performed as known in the art (Ausubel, *supra*). The membrane is then exposed to x-ray film overnight with an intensifying screen at -80° C. Labeled membranes are then visualized after exposure to film. The signal produced on the x-ray film by the radiolabeled cDNA probes can then be quantified using any technique known in the art, such as scanning the film and quantifying the relative pixel intensity using a computer program such as NIH Image (National Institutes of Health, Bethesda, MD), wherein the detection of hybridization of a Reg1α-specific probe to the clinical sample is indicative of the presence of Reg1α or TIMP1 and thus may be used to detect colorectal cancer.

In an alternate embodiment, the presence and optionally the quantity of Reg1α or TIMP1 in a clinical sample may be determined using the Taqman™ (Perkin-Elmer, Foster City, CA) technique, which is performed with a transcript-specific antisense probe (i.e., a probe capable of specifically hybridizing to Reg1α). This probe is specific for a Reg1α or TIMP1 PCR product and is prepared with a quencher and fluorescent reporter probe complexed to the 5' end of the oligonucleotide. Different fluorescent markers can be attached to different reporters, allowing for measurement of two products in one reaction (e.g., measurement of Reg1α or TIMP1 and at least one additional colorectal cancer associated marker). When Taq DNA polymerase is activated, it cleaves off the fluorescent reporters by its 5'-to-3' nucleolytic activity. The reporters, now free of the quenchers, fluoresce. The color change is proportional to the amount of each specific product and is measured by fluorometer; therefore, the amount of each color can be measured and the RT-PCR product can be quantified. The PCR reactions can be performed in 96 well plates so that samples derived from many individuals can be processed and measured simultaneously. The Taqman™ system has the additional advantage of not requiring gel electrophoresis and allows for quantification when used with a standard curve.

### Screening for polypeptide molecules

The Reg1α- or TIMP1-specific and colorectal cancer marker-specific antibodies described above may be used to detect the presence of Reg1α or TIMP1 or an additional colorectal cancer associated marker in a clinical sample by any method known in the art. The immunoassays which can be used include but are not limited to competitive and non-competitive assay systems using techniques such as western blots, radioimmunoassays, ELISA (enzyme linked immunosorbent assay),"sandwich" immunoassays, immunoprecipitation assays, precipitation reactions, gel diffusion precipitin reactions, immunodiffusion assays, agglutination assays, complement-fixation assays, immunoradiometric assays, fluorescent immunoassays, protein A immunoassays, to name but a few. Such assays are routine and well known in the art (see, e. g., Ausubel et al, eds, 1994, Current Protocols in Molecular Biology, Vol. 1, John Wiley & Sons, Inc., New York, which is incorporated by reference herein in its entirety). Exemplary immunoassays are described briefly below (but are not intended by way of limitation).

Immunoprecipitation protocols generally comprise lysing a population of cells in a lysis buffer such as RIPA buffer (1% NP-40 or Triton X-100,1% sodium deoxycholate, 0.1% SDS, 0.15 M NaCI, 0.01 M sodium phosphate at pH 7.2,1% Trasylol) supplemented with protein phosphatase and/or protease inhibitors (e. g., EDTA, PMSF, aprotinin, sodium vanadate), adding the antibody of interest to the cell lysate, incubating for a period of time (e. g., 1-4 hours) at 4 C, adding protein A and/or protein G sepharose beads to the cell lysate, incubating for about an hour or more at 4 C, washing the beads in lysis buffer and resuspending the beads in SDS/sample buffer. In the case of immunonprecipitation of a serum sample, however the above protocol is carried out absent the cell lysis step. The ability of the antibody to immunoprecipitate Reg1α or TIMP 1 (or other colorectal cancer marker) antigen can be assessed by, e. g., western blot analysis. The parameters that can be modified to increase the binding of the antibody to an antigen and decrease the background (e. g., preclearing the cell lysate with sepharose beads) are well known to those of skill in the art (Ausubel et al, *supra*).

Reg1α or TIMP1 polypeptides, and optionally one or more additional colorectal cancer associated markers may be detected in a patient clinical sample using Western blot analysis. Briefly, Western blot analysis comprises preparing protein samples, electrophoresis of the protein samples in a polyacrylamide gel (e. g., 8%-20% SDS-PAGE), transferring the protein sample from the polyacrylamide gel to a membrane such as nitrocellulose, PVDF or nylon, blocking the membrane in blocking solution (e. g., PBS with 3% BSA or non-fat milk), washing the membrane in washing buffer (e. g., PBS-Tween 20), blocking the membrane with primary antibody (the antibody of interest) diluted in blocking buffer, washing the membrane in washing buffer, blocking the membrane with a secondary antibody (which recognizes the primary antibody, e. g., an antihuman antibody) conjugated to an enzymatic substrate (e. g., horseradish peroxidase or alkaline phosphatase) or radioactive molecule (e. g., 32P or 125I) diluted in blocking buffer, washing the membrane in wash buffer, and detecting the presence of the antigen. Methods for the optimization of such an analysis are well known in the art (Ausubel, et al., *supra*).

Alternatively, the presence of Reg1α or TIMP1 and optionally one or more additional colorectal cancer associated markers in a clinical sample may be detected by ELISA. ELISAs comprise preparing antigen, coating the well of a 96 well microtiter plate (or other suitable container) with the antigen, adding the antibody of interest conjugated to a detectable compound such as an enzymatic substrate (e. g., horseradish peroxidase or alkaline phosphatase) to the well and incubating for a period of time, and detecting the presence of the antigen. In ELISAs the antibody of interest does not have to be conjugated to a detectable compound; instead, a second antibody (which recognizes the antibody of interest, that is, the antibody which will bind to Reg1α or TIMP1 or a second colorectal cancer associated marker) conjugated to a detectable compound may be added to the well. Further, instead of coating the well with the antigen, the antibody may be coated to the well. In this case, a second antibody conjugated to a detectable compound may be added following the addition of the antigen of interest to the coated well. This method may be modified or optimized according techniques which are known to those of skill in the art.

The binding affinity of an antibody to an antigen and the off-rate of an antibodyantigen interaction can be determined by competitive binding assays. One example of such an assay is a radioimmunoassay comprising the incubation of labeled antigen (e. g., Reg1α labeled with 3H or 125I) with an anti-Reg1α or TIMP1 antibody in the presence of increasing amounts of unlabeled antigen, and the detection of the antibody bound to the labeled antigen. The affinity of the antibody of interest for a particular antigen and the binding off-rates can be determined from the data by scatchard plot analysis. Competition with a second antibody can also be determined using radioimmunoassays. In this case, the antigen is incubated with antibody of interest conjugated to a labeled compound (e. g., 3H or 125I) in the presence of increasing amounts of an unlabeled second antibody.

Preferably, the above detection assays re be carried out using antibodies to detect the protein product encoded by a nucleic acid having the sequence of SEQ ID Nos: 1, 3 or 33, or a sequence complementary thereto. Preferably, the protein product has the sequence of one or more of SEQ ID Nos. 2, 4, or 100. In addition, the above detection assays may be conducted using one or more antibodies which specifically recognize and bind to at least one additional colorectal cancer associated marker. Accordingly, in one embodiment, the assay would include contacting the proteins of the test cell with an antibody specific for the gene product of a nucleic acid represented by SEQ ID Nos: 1, 3 or 33, or a sequence complementary thereto, and determining the approximate amount of immunocomplex formation by the antibody and the proteins of the test cell, wherein a detection of such an immunocomplex is indicative of the presence of the antigen, and thus, permits the detection of colorectal cancer.

Immunoassays, useful in the present invention include those described above, and can also include both homogeneous and heterogeneous procedures such as fluorescence polarization immunoassay (FPIA), fluorescence immunoassay (FIA), enzyme immunoassay (EIA), and nephelometric inhibition immunoassay (NIA).

In another embodiment, the level of the encoded product, i.e., the product encoded by SEQ ID Nos 1, 3 or 33, or a sequence complementary thereto, in a biological fluid (e.g., blood or urine) of a patient may be determined as a way of monitoring the level of expression of the marker nucleic acid sequence in cells of that patient. Such a method would include the steps of obtaining a sample of a biological fluid from the patient, contacting the sample (or proteins from the sample) with an antibody specific for a encoded marker polypeptide, and determining the amount of immune complex formation by the antibody, with the amount of immune complex formation being indicative of the level of the marker encoded product in the sample. This determination is particularly instructive when compared to the amount of immune complex formation by the same antibody in a control sample taken from a normal individual or in one or more samples previously or subsequently obtained from the same person.

In another embodiment, the method can be used to determine the amount of marker polypeptide present in a cell, which in turn can be correlated with progression of a hyperproliferative disorder, e.g., colorectal cancer. The level of the marker polypeptide can be used predictively to evaluate whether a sample of cells contains cells which are, or are predisposed towards becoming, transformed cells. Moreover, the subject method can be used to assess the phenotype of cells which are known to be transformed, the phenotyping results being useful in planning a particular therapeutic regimen. For instance, very high levels of the marker polypeptide in sample cells is a powerful diagnostic and prognostic marker for a cancer, such as colorectal cancer. The observation of marker polypeptide level can be utilized in decisions regarding, e.g., the use of more aggressive therapies.

As set out above, one aspect of the present invention relates to detection assays for determining, in the context of cells isolated from a patient, if the level of a marker polypeptide is significantly reduced in the sample cells. The term "significantly reduced" refers to a cell phenotype wherein the cell possesses a reduced cellular amount of the marker polypeptide relative to a normal cell of similar tissue origin. For example, a cell may have less than about 50%, 25%, 10%, or 5% of the marker polypeptide that a normal control cell. In particular, the assay evaluates the level of marker polypeptide in the test cells, and, preferably, compares the measured level with marker polypeptide detected in at least one control cell, e.g., a normal cell and/or a transformed cell of known phenotype.

Of particular importance to the subject invention is the ability to quantitate the level of normal or abnormal Reg1α or TIMP1 expression. The expression of Reg1α or TIMP1, and/or the level of expression of Reg1α or TIMP1 can be used predictively to evaluate whether a patient is predisposed towards developing colorectal cancer, or for determining the severity of colorectal cancer.

In one embodiment, tissue samples may be used to measure Reg1α or TIMP1 expression by immunohistochemical staining which may be used to determine the number of cells (i.e., colon cells) expressing Reg1α or TIMP1. For such staining, a multiblock of tissue is taken from the biopsy or other tissue sample and subjected to proteolytic hydrolysis, employing such agents as protease K or pepsin. In certain embodiments, it may be desirable to isolate a nuclear fraction from the sample cells and detect the level of the marker polypeptide in the nuclear fraction.

The tissue samples are fixed by treatment with a reagent such as formalin, glutaraldehyde, methanol, or the like. The samples are then incubated with an antibody, preferably a monoclonal antibody, with binding specificity for Reg1α or TIMP1 and optionally an additional colorectal cancer associated marker. This antibody may be conjugated to a label for subsequent detection of binding. Samples are incubated for a time sufficient for formation of the immunocomplexes. Binding of the antibody is then detected by virtue of a label conjugated to this antibody. Where the antibody is unlabeled, a second labeled antibody may be employed, e.g., which is specific for the isotype of the anti -marker polypeptide antibody. Examples of labels which may be employed include radionuclides, fluorescers, chemiluniinescers, enzymes and the like.

Where enzymes are employed, the substrate for the enzyme may be added to the samples to provide a colored or fluorescent product. Examples of suitable enzymes for use in conjugates include horseradish peroxidase, alkaline phosphatase, malate dehydrogenase and the like. Where not commercially available, such antibody-enzyme conjugates are readily produced by techniques known to those skilled in the art. Other assays, known to those of skill in the art for determining the presence and/or quantity of a polypeptide in a sample (either serum or tissue) are also encompassed by the present invention.

### Drug screening

Several in vivo methods can be used to identify compounds that modulate expression of Reg1α or TIMP1 nucleic acids (SEQ ID Nos: 1, 3 or 33, or a sequence complementary thereto) and/or alter for example, inhibit the bioactivity of the encoded polypeptide (e.g., SEQ ID Nos: 2, 4, or 100).

Drug screening is performed by adding a test compound to a sample of cells, and monitoring the effect. A parallel sample which does not receive the test compound is also monitored as a control. The treated and untreated cells are then compared by any suitable phenotypic criteria, including but not limited to microscopic analysis, viability testing, ability to replicate, histological examination, the level of a particular RNA or polypeptide associated with the cells, the level of enzymatic activity expressed by the cells or cell lysates, and the ability of the cells to interact with other cells or compounds. Differences between treated and untreated cells indicates effects attributable to the test compound.

Desirable effects of a test compound include an effect on any phenotype that was conferred by the cancer-associated marker nucleic acid sequence. Examples include a test compound that limits the overabundance of mRNA, limits production of the encoded protein, or limits the functional effect of the protein. The effect of the test compound would be apparent when comparing results between treated and untreated cells.

The invention thus also encompasses methods of screening for agents which inhibit expression of Reg1α or TIMP1 nucleic acid (SEQ ID Nos: 1, 3 or 33, or a sequence complementary thereto) *in vitro*, comprising exposing either a cell or tissue in which Reg1α or TIMP1 nucleic acid mRNA is detectable or cultured cells comprising and capable of expressing Reg1α or TIMP1 nucleic acid to an agent in order to determine whether the agent is capable of inhibiting production of the mRNA; and determining the level of mRNA in the exposed cells or tissue, wherein a decrease in the level of the mRNA after exposure of the cell line to the agent is indicative of inhibition of the marker nucleic acid mRNA production.

Alternatively, the screening method may include in vitro screening of a cell or tissue in which Reg1α or TIMP1 is detectable, or cultured cells which express Reg1α or TIMP1, to an agent suspected of inhibiting production of Reg1α or TEMP1 protein; and determining the level of the Reg1α or TIMP1 protein in the cells or tissue, wherein a decrease in the level of marker protein after exposure of the cells or tissue to the agent is indicative of inhibition of marker protein production.

The invention also encompasses in vivo methods of screening for agents which inhibit expression of the marker nucleic acids, comprising exposing a mammal having tumor cells or serum in which Reg1α or TIMP1 mRNA or protein is detectable to an agent suspected of inhibiting production of marker mRNA or protein; and determining the level of marker mRNA or protein in serum or tumor cells of the exposed mammal. A decrease in the level of marker mRNA or protein after exposure of the mammal to the agent is indicative of inhibition of marker nucleic acid expression. Optionally, the effect of the candidate agent on the expression of at least one additional colorectal cancer associated marker may also be determined.

Accordingly, the invention provides a method comprising incubating a cell expressing the marker nucleic acids (SEQ ID Nos: 1, 3 or 33, or a sequence complementary thereto) with a test compound and measuring the mRNA or protein level. The invention further provides a method for quantitatively determining the level of expression of the marker nucleic acids in a cell population or clinical sample, and a method for determining whether an agent is capable of increasing or decreasing the level of expression of the Reg1α or TIMP1 nucleic acid in a cell population or clinical sample. The method for determining whether an agent is capable of increasing or decreasing the level of expression of Reg1α or TIMP1 nucleic acid in a cell population comprises the steps of (a) preparing cell extracts from control and agent-treated cell populations, (b) isolating the Reg1α or TIMP1 polypeptide from the cell extracts, (c) quantifying (e.g., in parallel) the amount of an immunocomplex formed between Reg1α or TIMP1 polypeptide and an antibody specific to said polypeptide. The Reg1α or TIMP1 polypeptide of this invention may also be quantified by assaying for its bioactivity. Agents that induce an increase in Reg1α or TIMP1 nucleic acid expression may be identified by their ability to increase the amount of immunocomplex formed in the treated cell as compared with the amount of the immunocomplex formed in the control cell. In a similar manner, agents that decrease expression of Reg1α or TIMP1 nucleic acid may be identified by their ability to decrease the amount of the immunocomplex formed in the treated cell extract as compared to the control cell.

mRNA levels can be determined by Northern blot hybridization. mRNA levels can also be determined by methods involving PCR. Other sensitive methods for measuring mRNA, which can be used in high throughput assays, e.g., a method using a DELFIA endpoint detection and quantification method, are described, e.g., in Webb and Hurskainen (1996) *Journal of Biomolecular Screening* 1:119. Reg1α protein levels can be determined by immunoprecipitations or immunohistochemistiy using an antibody that specifically recognizes the protein product of SEQ ID Nos: 2, 4, or 100.

Agents that are identified as active in the drug screening assay are candidates to be tested for their capacity to block cell proliferation activity. These agents would be useful for treating a disorder involving aberrant growth of cells, especially colon cells, especially colorectal cancer.

A variety of assay formats will suffice and, in light of the present disclosure, those not expressly described herein will nevertheless be comprehended by one of ordinary skill in the art. For instance, the assay can be generated in many different formats, and include assays based on cell-free systems, e.g., purified proteins or cell lysates, as well as cell-based assays which utilize intact cells.

In many drug screening programs which test libraries of compounds and natural extracts, high throughput assays are desirable in order to maximize the number of compounds surveyed in a given period of time. Assays of the present invention which are performed in cell -free systems, such as may be derived with purified or semi-purified proteins or with lysates, or with proteins purified or semi-purified from serum, are often preferred as "primary" screens in that they can be generated to permit rapid development and relatively easy detection of an alteration in a molecular target which is mediated by a test compound. Moreover, the effects of cellular toxicity and/or bioavailability of the test compound can be generally ignored in the *in vitro* system, the assay instead being focused primarily on the effect of the drug on the molecular target as may be manifest in an alteration of binding affinity with other proteins or changes in enzymatic properties of the molecular target.

### EXAMPLES

The examples below are non-limiting and are merely representative of various aspects and features of the present invention.

### Example 1: Generation of anti- Reg1α antibodies

To generate antibodies to Reg1α, the full-length open reading frame of Reg1α (shown in either SEQ ID NO: 1 or 3) was directionally cloned into a mammalian expression vector, such as pcDNA3.1/V5-His (Invitrogen), which includes C-terminal epitope and purification tags. The insert sequence was verified by dideoxy sequencing (see, for example, Ausubel et al., Current Protocols in Molecular Biology, John Wiley and Sons). Recombinant fusion protein was produced in a transient expression system in mammalian cells (e.g. CHO cells). The recombinant protein was purified from the cell culture supernatants by immobilized metal affinity chromatography (IMAC) by utilizing the C terminal His-tag. The sequence of the Reg1α protein used for the production of antibodies of the present invention is shown in either of SEQ ID Nos 2 or 4, all of which represent a functional Reg1α protein, and which are encoded by SEQ ID Nos 1 or 3, respectively. The purified, recombinant Reg1α protein was emulsified in Freund's adjuvant and injected into rabbits. The animals were periodically boosted until they elicited a reasonable serum titer of specific antibody to Reg1α. Methods for antibody production are well known to those of skill in the art and may be found, for example, in Harlow et al. Antibodies: A laboratory manual, 1988, Cold Spring Harbor Laboratory. The polyclonal antibodies, which recognized both native and denatured Reg1α, were utilized to develop a microtiter-based ELISA assay. Methods of performing an ELISA assay are well known to those of skill in the art (see, for example, Asusbel et al., *supra*).

### Example 2: Detection of Reg1α in Colorectal cancer Patient Serum Samples

The present invention relates to a method for the detection of colorectal cancer in an individual, which method includes the detection of Reg1α polypeptides in a serum sample from an individual with colorectal cancer, wherein the detection of Reg1α is indicative of the presence of colorectal cancer. Accordingly, Reg1α expression was measured in serum samples obtained from patients having been diagnosed with colorectal cancer.

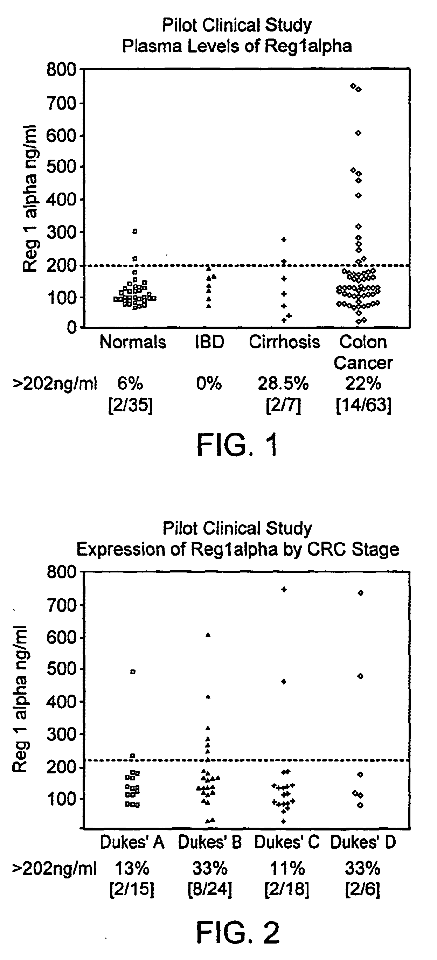

All patients used in this study were diagnosed at their respective medical institutions by qualified physicians using conventional diagnostic means, including physical exam, blood analysis, imaging, and endoscopy. Once identified, patients provided informed consent through an IRB approved protocol. The severity of colorectal cancer in each patient was graded using the Dukes staging scheme. Serum samples were subsequently collected from each patient using methods known to those of skill in the art. Samples were subsequently assessed for the presence of Reg1α by the ELISA assay described above. Figure 1 shows the levels of Reg1α protein measured in the colorectal cancer patients compared to samples obtained from naive patients and additional patients diagnosed with either inflammatory bowel disease (IBD) or cirrhosis of the liver. Figure 2 shows the levels of Reg1α expression in the colorectal cancer patients of Figure 1, identified at each stage of colorectal cancer severity. As can be seen in Figures 1 and 2, Reg1α expression is clearly elevated in serum samples obtained from patients diagnosed with colorectal cancer, and therefore may be used to detect the presence of colorectal cancer in a patient.

### Example 3: Detection of Reg1α Nucleic Acid Sequence in Colorectal cancer

In one embodiment, the present invention provides for a method of detecting the presence of colorectal cancer in a patient by detecting the presence of nucleic acid molecules encoding Reg1α in a serum sample obtained from a patient.

Serum may be obtained from a patient suspected of having colorectal cancer by methods described above and known to those of skill in the art. Nucleic acid molecules encoding Reg1α may be detected, for example, by Northern analysis. Briefly, probes for detection of Reg1α mRNA in a patient sample are derived by amplifying the Reg1α coding sequence by RT-PCR according to techniques known in the art. The cDNA fragments generated in this manner are subsequently cloned into a PCRII vector using the TA cloning kit (Invitrogen). The identity of each fragment can be verified by sequencing in each direction from the T3 and T7 polymerase sites present in the cloning vector. The cDNA molecules produced in this manner are then used to produce ³²P-labeled Reg1α cDNA probes using, for example, the Prime-It kit from Stratagene. Subsequently, 5 to 10 µg of total RNA isolated the serum of a patient suspected of having colorectal cancer is separated on an agarose/formaldehyde gel in 1X MOPS buffer. Methods of isolating RNA from a patient sample such as serum are well known in the art (see, for example, Ausubel et al., *supra*). Following staining with ethidium bromide and visualization under ultra violet light to determine the integrity of the RNA, the RNA is hydrolyzed by treatment with 0.05M NaOH/1.5MNaCl followed by incubation with 0.5M Tris-Cl (pH 7.4)/1.5M NaCl. The RNA is transferred to a commercially available nylon or nitrocellulose membrane (e.g. Hybond-N membrane, Amersham, Arlington Heights, IL) by methods well known in the art (Ausubel et al., supra, Sambrook et al., supra). Following transfer and UV cross linking, the membrane is hybridized with a ³²P-labeled Reg1α cDNA probe in hybridization solution (e.g. in 50% formamide/2.5% Denhardt's/100-200mg denatured salmon sperm DNA/0.1% SDS/5X SSPE) overnight at 65°C. The hybridization conditions can be varied as necessary as described in Ausubel et al., supra and Sambrook et al., supra. Following hybridization, the membrane is washed at room temperature in 2X SSC/0.1% SDS, at 42°C in 1X SSC/0.1% SDS, at 65°C in 0.2X SSC/0.1% SDS, and exposed to film overnight with an intensifying screen at -80° C. The stringency of the wash buffers can also be varied depending on the amount of background signal (Ausubel et al., supra). The film is subsequently developed and the intensity bands corresponding to the radiolabeled probe hybridized to RNA are quantified using methods known to those of skill in the art, for example, by digitizing the film and analyzing the band intensity with a computer software program such as NIH Image (NIH, Bethesda, MD).

Alternatively, Reg1α mRNA may be detected in a patient sample by real-time amplification using oligonucleotide primers capable of specifically hybridizing to the Reg1α sequence. For example, real-time PCR and TaqMan® probes may be used to detect and quantitate the presence of Reg1α mRNA in a patient sample. The technique of real-time PCR is well known in the art (see, for example, U.S. Pat. Nos. 5,691,146; 5,779,977; 5,866,336; and 5,914,230). Methods of designing primers useful for the amplification of Reg1α sequences are well known in the art (see, for example, Ausubel et al., *supra*)

cDNA samples, reverse transcribed from mRNA obtained from patient serum samples may be used to generate PCR products via an ABI 7700 sequence detection system (Applied Biosystems, Foster City, CA). A measurement may then be made of the level of expression of Reg1α in the patient sample to determine if Reg1α mRNA levels are elevated, thus, providing a means for the detection of colorectal cancer in the patient.

### Example 4: Detection of Reg1α in Other Patient Samples

In one embodiment of the present invention, colorectal cancer may be detected in a patient by detecting the expression of Reg1α in a clinical patient sample, which is not a serum sample. For example, a circulating cell sample may be obtained from a patient by collecting a sample such as blood, stool, or other bodily fluid. The sample is then subsequently treated to lyse the cells present therein, for example by treating the sample with a suitable lysis buffer, such as a buffer containing 30 mM Tris-Cl, pH 7.4, 100 mM NaCl, 5 mM EDTA, 1% (w/v) SDS, and 100 µg/ml proteinase K (for isolation of nucleic acid). The resulting sample is then analyzed for Reg1α expression either by isolating total RNA from the sample, as described above, and in Ausubel et al., *supra*, or the sample may be separated on a polyacrylamide gel for analysis by Western blot, or may be utilized in an ELISA-based assay as described above in Example 2.

### Example 5. Detection of TIMP1 in patient serum samples

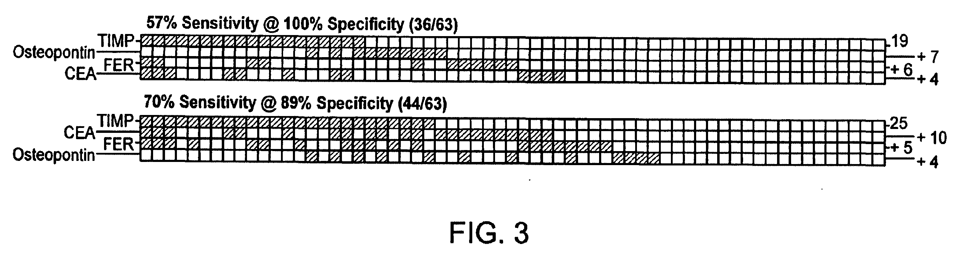

The present invention provides for the detection and monitoring of colorectal cancer in a patient by measuring the level of TIMP 1 polypeptide in a patient sample, preferably in a plasma sample. TIMP 1 expression was determined in 63 samples from patients diagnosed with colorectal cancer relative to the expression level of TIMP1 in 35 healthy individuals. The results demonstrate that TIMP1, in addition to one or more other colorectal cancer associated markers is overexpressed in colorectal cancer samples relative to normal samples, thus indicating that TIMP1 is a valuable marker for the detection of colorectal cancer in a patient (Figure 3).

To assess TIMP 1 polypeptide expression levels, 63 pre-treatment plasma samples from patients with colorectal cancer, and 35 samples from healthy donors were tested in either commercially available ELISAs (Osteopontin), ADVIA Centaur Immunoassays (CEA and Ferritin), or in-house developed ELISA (TIMP1). All patients used in this study were diagnosed at their respective medical institutions by qualified physicians using conventional diagnostic means, including physical exam, blood analysis, imaging, or endoscopy. Once identified, patients provided informed consent through an IRB approved protocol. The extent o f colorectal cancer in each patient was determined using the Dukes' staging scheme. Serum and plasma samples were subsequently collected from each patient using methods known to those of skill in the art.

Specificity at appropriate cutoff values was determined for each marker (e.g., TIMP1, osteopontin, CEA, and ferritin) by evaluating the normal samples. For example, the 100% specificity cutoff for any given marker is equal to the marker value of the highest normal sample. Using these values as the cutoffs, the levels of each marker in the 63 cancer samples were compared to their own respective cutoff values. If the level in the cancer sample was higher than the determined cutoff value, the sample was deemed "positive" and is represented by a shaded box (Figure 3). This same process was repeated at 97% specificity (using the second highest normal; e.g., 34 of the 35 samples were equal to or below this value). The overall specificity level for the entire panel is calculated by multiplying the specificity of each marker in the panel (e.g., 97% x 97% x 97% x 97% = 89% specificity for the panel). The markers were arranged on the graphs shown in Figure 3, according to the frequency of their overexpression in the cancer samples (TIMP1 was overexpressed in the highest number of cancer patients and is therefore listed first). The marker adding the most to the sensitivity of TIMP 1 is ranked second. For example, the 57% sensitivity/100% specificity graph shows that TIMP1 was elevated in 19 of the 63 colorectal cancer patient plasma samples, and is thus listed first on the graph. Evaluating the samples for osteopontin yielded seven additional positive patient samples, and osteopontin is thus listed second on the graph.

The sensitivity of the panel was determined by dividing the cumulative number of samples that were positive for at least one marker by the total number of cancer samples (63). Other embodiments will be evident to those of skill in the art. It should be understood that the foregoing detailed description is provided for clarity only and is merely exemplary. The spirit and scope of the present invention are not limited to the above examples, but are encompassed by the following claims.

## Claims

1. A method of diagnosing colon cancer in an individual comprising:
(a) obtaining a serum sample from said individual; and
(b) detecting the presence of TIMP 1 in said sample, wherein the presence of TIMP 1 in said sample is indicative of colon cancer in said individual.

2. The method of claim 1, wherein said step of detecting comprises:
(a) contacting said serum sample with a polypeptide ligand which is capable of binding to TIMP1under conditions which permit said polypeptide ligand to bind to TIMP1; and
(b) detecting the binding of said polypeptide ligand to TIMP1, wherein detection of binding is indicative of the presence of TIMP1 in said sample.

3. The method of claim 2, wherein said polypeptide ligand is an antibody.

4. The method of claim 2 or claim 3, wherein said polypeptide ligand comprises a detectable label.

5. The method of any one of the preceding claims, wherein said individual is a human.

6. The method of any one of the preceding claims, further comprising detecting at least one other colon cancer specific marker in said sample, wherein the presence of TIMP 1 and said at least one other colon cancer-specific marker is indicative of colon cancer in said individual.

7. The method of claim 6, wherein said colon cancer- specific marker is selected from the group consisting of the nucleic acid molecules of SEQ ID Nos 1, 3, 5-71, the polypeptide molecules of SEQ ID Nos 2, 4, 72-138, CA 19-9, CA 72-4, TF, sTn, Tn, CA 50, CA 549, CA 242, LASA, and Du-PAN 1 - 5.

8. The method of claim 6 or claim 7, wherein said step of detecting comprises:
(a) contacting said serum sample with a first polypeptide ligand which is capable of binding to TIMP1 and a second polypeptide ligand which is capable of binding to said colon cancer-specific marker, under conditions which permit said first and second polypeptide ligands to bind to TIMP 1 and said colon cancer-specific marker, respectively; and
(b) detecting the binding of said first polypeptide ligand to TIMP 1 and said second polypeptide ligand to said colon cancer-specific marker, wherein detection of binding is indicative of the presence of TIMP1 and said colon cancer-specific marker in said sample.

9. The method of claim 8, wherein said first and second polypeptide ligand are each an antibody.

10. The method of claim 8 or claim 9, wherein said first and second polypeptide ligand comprises a detectable label.

11. The method of any one of claims 1 to 10, further comprising the step of detecting the presence of REG1α in said sample, wherein the presence of REG1α in said sample is indicative of colon cancer in said individual.

12. A method of diagnosing colon cancer in an individual comprising:
(a) obtaining a serum sample from an individual; and
(b) detecting the presence of a nucleic acid molecule which encodes TIMP1 in said sample, wherein the presence of TIMP1 of said nucleic acid molecule in said sample is indicative of colon cancer in said individual.

13. The method of claim 12, further comprising detecting at least one other nucleic acid molecule which encodes at least one other colon cancer-specific marker in said sample, wherein the presence of said nucleic acid sequence encoding TIMP1and said nucleic acid sequence encoding said at least one other colon cancer-specific marker is indicative of colon cancer in said individual.

14. The method of claim 12 or claim 13, wherein said colon cancer specific marker is selected from the group consisting of SEQ ID Nos 1, 3, 5-71, the polypeptide molecules of SEQ ID Nos 2, 4, 72-138, CA 19-9, CA 72-4, TF, sTn, Tn, CA 50, CA 549, CA 242, LASA, and Du - PAN 1 - 5.

15. The method of any one of claims 12 to 14, further comprising the step of detecting presence of a nucleic acid molecule which encodes REG1α in said sample, wherein the presence of REG1α of said nucleic acid molecule in said sample is indicative of colon cancer in said individual.
